# EUROPEAN PATENT APPLICATION

(11) **EP 3 922 632 A1**
(43) Date of publication of application: **15.12.2021**
(21) Application number: 20753191.4
(22) Date of filing: 07.02.2020
(51) Int. Cl.: C07D 487/04, A61K 31/519, C07D 401/14, A61P 35/00

(54) **TARGET PROTEIN EED DEGRADATION-INDUCING DEGRADUCER, PREPARATION METHOD THEREOF, AND PHARMACEUTICAL COMPOSITION FOR PREVENTING OR TREATING DISEASES RELATED TO EED, EZH2, OR PRC2, COMPRISING SAME AS ACTIVE INGREDIENT**

(30) Priority: 07.02.2019 KR 20190014633
(71) Applicant: Korea Research Institute of Chemical Technology, Daejeon 34114 (KR); Korea Research Institute of Bioscience and Biotechnology, Daejeon 34141 (KR)
(72) Inventor: HWANG, Jong Yeon, Daejeon 34114 (KR); HA, Jae Du, Daejeon 34114 (KR); CHO, Sung Yun, Daejeon 34114 (KR); KIM, Pilho, Daejeon 34114 (KR); YUN, Chang Soo, Daejeon 34114 (KR); KIM, Hyun Jin, Daejeon 34114 (KR); PARK, Sung Goo, Daejeon 34141 (KR); PARK, Byoung Chul, Daejeon 34141 (KR); KIM, Jeong Hoon, Daejeon 34141 (KR); KIM, Sunhong, Daejeon 34141 (KR)
(74) Representative: Nony
(86) International application number: PCT/KR2020/001799
(87) International publication number: WO 2020/162725

(57) **Abstract**

The present invention relates to a target protein degradation-inducing Degraducer, a preparation method thereof, and a pharmaceutical composition for preventing or treating diseases related to EED, EZH2, or PRC2 comprising same as an active ingredient. A novel compound represented by formula 1, according to the present invention is a Degraducer compound that induces degradation of a target protein, i.e., embryonic ectoderm development (EED) or polycomb repressive complex 2 (PRC2), utilizing cereblon E3 ubiquitin ligase, von Hippel-Lindau tumor suppressor (VHL) E3 ubiquitin ligase, mouse double minute 2 homolog (MDM2) E3 ubiquitin ligase, and cellular inhibitor of apoptosis protein 1 (cIAP) E3 ubiquitin ligase, wherein the compound has an aspect of remarkably achieving target protein degradation-inducing activity through a ubiquitin proteasome system (UPS), and therefore there is a useful effect in that it is possible to provide a pharmaceutical composition for preventing or treating diseases or conditions related to a target protein, and a functional health food composition for preventing or improving same, comprising said compound as an active ingredient.

## Description

### [Technical Field]

The present invention relates to a target protein degradation-inducing degraducer, a method for preparing the same, and a pharmaceutical composition for preventing or treating EED, EZH2, or PRC2 related diseases comprising the same as an active ingredient. The present invention also relates to a method for preventing or treating EED, EZH2, or PRC2 related diseases, characterized in that the compound of the present invention is administered as an active ingredient, that is, to a medical use of the compounds of the present invention.

### [Background Art]

Polycomb family (PcG) proteins are chromatin modifying enzymes that are aberrantly regulated in many human cancers. Polycomb inhibitory complex 2 (PRC2) including SUZ12 (suppressor of zest 12), EED (embryonic ectoderm development), RbAp46/48 (Rb-binding protein 46/48) and the catalytic subunit EZH2 (enhancer of zest 2) represses gene expression by methylating the core histone H3 lysine 27 (H3K27me3) in and around the promoter region of the target gene.

PRC2 is a critical component of the cellular machinery involved in epigenetic regulation of gene transcription, and plays a critical function in development and tissue differentiation and regeneration. Although EZH2 is a catalytic subunit, PRC2 requires at least EED and SUZ12 for its methyltransferase activity. EED, SUZ12 and EZH2 are overexpressed in many cancers including, but not limited to, breast cancer, prostate cancer, hepatocellular carcinoma, hematological carcinoma, and the like.

Recently, somatic mutations in tyrosine 641 (Y641F, Y641N, Y641S and Y641H) of EZH2 was reported to be associated with germinal center B-cell like (GCB) subtypes of follicular lymphoma (FL) and diffuse large B-cell lymphoma (DLBCL). (Morin et al. (2010) Nat Genet 42:181-5).

In addition, studies in prostate and breast cancer cell lines and tissues showed a strong correlation between the levels of EZH2 and SUZ12 and the invasiveness of these cancers, indicating that aberrant functions of the PRC2 complex may contribute to cancer (Bracken et al. (2003) EMBO J 22:5323-35; Kirmizis et al. (2003) Mol Cancer Ther 2:113-21; Kleer et al. (2003) Proc Natl Acad Sci USA 100: 11606-11; and Varambally et al. (2002) Nature 419:624-9).

In all cases, the occurrence of the mutant EZH2 gene was shown to be heterozygous, and expression of both wild-type and mutant alleles was detected in mutant samples analyzed by transcriptome sequencing. In addition, it was also demonstrated that all mutants of EZH2 can be introduced into the multi-protein PRC2 complex, but the resulting complex has an improved ability to promote methylation of H3-K27 equivalent residues of the peptidic substrate. In addition, cell growth was inhibited by inhibition of EZH2 activity or EDD activity in certain lymphomas having this mutant EZH2. Therefore, it was reported that H3-K27 methylation increased by Tyr641 mutation of EZH2 is closely related to the development and progression of cancer.

Meanwhile, in patients with suspected Weaver syndrome, we identified a previously unknown *de novo* mutation in the partner protein EED of EZH2, excluding mutations in EZH2 and NSD1. Based on the similarity with the patient's phenotype for Weaver's syndrome confirmed by other evidence in addition to *de novo* mutations in EZH2 and mouse EED hypomorphs, this mutation is considered pathogenic in Weaver-like proliferative syndromes. This is the first report of a human EED mutation-related hyperproliferative phenotype (AS Cohen et al. (2015) Journal of Human Genetics 60: 339-342).

Under this background, the present inventors have endeavored to develop a drug that can achieve the advantages of more improved efficacy and reduced side effects through degraducer technology (target protein degradation agent) utilizing UPS (Ubiquitin Proteasome System) (South Korea Patent No. 10-1825065). Surprisingly, the present inventors have found that the compound represented by Chemical Formula 1 provided in the present invention can be used as a degraducer capable of exhibiting excellent degradation-inducing activity and inhibitory activity targeting EED. In addition, the present inventors have confirmed that they are useful as novel therapeutic agents for EED, EZH2, or PRC2 related diseases, thereby completing the present invention.

### [Disclosure]

### [Technical Problem]

An object of the present invention is to provide a novel degraducer drug that exhibits a more improved drug efficacy through induction of EED degradation, a target protein, and at the same time, that can reduce the side effects of existing therapeutic agents, not proteolysis-inducing drugs, and a composition comprising the same as an active ingredient.

Another object of the present invention is to provide a method for preparing the active ingredient compound.

Yet another object of the present invention is to provide a pharmaceutical composition for preventing or treating diseases or conditions related to EED, EZH2, or PRC. That is, another object of the present invention is to provide a method for preventing or treating EED, EZH2, or PRC2 related diseases.

Yet another object of the present invention is to provide a health functional food composition for preventing or improving EED, EZH2, or PRC-related diseases or conditions.

### [Technical Solution]

In order to solve the above object,
in one aspect of the present invention,
a compound represented by the following chemical formula 1, a stereoisomer thereof, or a pharmaceutically acceptable salt thereof is provided:

   [Chemical formula 1] **ULB—L—PTM**
(In chemical formula 1,
PTM is an EED (embryonic ectoderm development) targeting molecule,
L (Linker) is a single bond or a chemical linker, and
ULB is cereblon, MDM2 (Mouse double minute 2 homolog), cIAP (Cellular Inhibitor of Apoptosis Protein 1) or VHL (von Hippel-Lindau tumor suppressor) E3 ubiquitin ligase binding binder).

Also, in another aspect of the present invention,
as shown in scheme 1 below,
a method for preparing a compound represented by chemical formula 1 of claim 1 is provided, wherein the method comprises
a step of preparing a compound represented by the chemical formula ULB-L-H from the compound represented by ULB-X; and
a step of preparing a compound of ULB-L-PTM represented by chemical formula 1 from the compound represented by ULB-L-H prepared above. 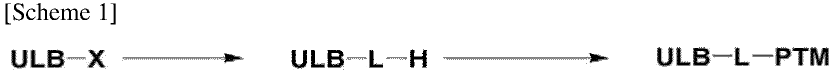
(In Scheme 1,
ULB, L, and PTM are as defined in claim 1, and
X is H or F).

Also, in another aspect of the present invention,
as shown in Scheme 2 below,
a method for preparing a compound represented by chemical formula 1 of claim 1 is provided, wherein the method comprises
a step of preparing a compound represented by the chemical formula PTM-L-H from the compound represented by PTM-X; and
a step of preparing a compound of ULB-L-PTM represented by chemical formula 1 from the compound represented by PTM-L-H prepared above: 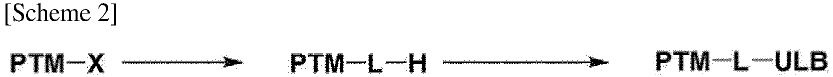
(In Scheme 2,
ULB, L, and PTM are as defined in claim 1, and
X is H).

Furthermore, in another aspect of the present invention,
a pharmaceutical composition for preventing or treating a disease or condition related to EED (embryonic ectoderm development), EZH2 (Enhancer of zeste homolog 2), or PRC2 (polycomb repressive complex 2) comprising the compound represented by chemical formula 1, a stereoisomer thereof, or a pharmaceutically acceptable salt thereof as an active ingredient is provided.

Also, in another aspect of the present invention,
a health functional food composition for preventing or improving a disease or condition related to EED (embryonic ectoderm development), EZH2 (Enhancer of zeste homolog 2), or PRC2 (polycomb repressive complex 2) comprising the compound represented by chemical formula 1, a stereoisomer thereof, or a pharmaceutically acceptable salt thereof as an active ingredient is provided.

### [Advantageous Effects]

The novel compound represented by chemical formula 1 according to the present invention is a degraducer compound that induces degradation of a target protein, that is, EED (embryonic ectoderm development) or PRC2 (polycomb repressive complex 2), and utilizes cereblon E3 ubiquitin ligase, VHL (von Hippel-Lindau tumor suppressor) E3 ubiquitin ligase, MDM2 (Mouse double minute 2 homolog) E3 ubiquitin ligase, or cIAP (Cellular Inhibitor of Apoptosis Protein 1) E3 ubiquitin ligase. The compound has an aspect of remarkably achieving target proteolysis-inducing activity through UPS (Ubiquitin Proteasome System). Thus, the present invention has a useful effect that can provide a pharmaceutical composition or a health functional food composition for preventing or treating a target protein-related disease or condition comprising the same as an active ingredient.

### [Brief Description of Drawings]

Figure 1 is a western blot showing the results of protein analysis of a-HA and α-GAPDH according to each concentration (0.04, 0.2, 1 µM) treatment of the compounds of Examples 58, 85, and 86 of the present invention.
Figure 2 is a western blot showing the results of protein analysis of α-HA and α-GAPDH according to each concentration (0.04, 0.2, 1 µM) treatment of the compounds of Examples 88, 89, 90, and 92 of the present invention.

### [Mode for Invention]

Hereinafter, the present invention will be described in more detail.

The following description should be understood as an example for helping the understanding of the present invention, and the spirit or scope of the present invention is not limited from the following description.

In one aspect of the present invention,
a compound represented by the following chemical formula 1, a stereoisomer thereof, or a pharmaceutically acceptable salt thereof is provided:

[Chemical formula 1] **ULB—L—PTM**

(In the chemical formula 1,
PTM is an embryonic ectoderm development (EED) targeting molecule,
L (Linker) is a single bond or a chemical linker,
ULB is cereblon, MDM2 (Mouse double minute 2 homolog), cIAP (Cellular Inhibitor of Apoptosis Protein 1) or VHL (von Hippel-Lindau tumor suppressor) E3 ubiquitin ligase binding binder).

Here, the PTM refers to a protein targeting molecule, but it can be understood that the protein targeted by the present invention is EED (embryonic ectoderm development), and the PTM is adjacent to, or binds to the protein, or can come to a position making a series of interactions with the protein.

Accordingly, the PTM may be understood to be a compound having a chemical structure capable of binding to EED (embryonic ectoderm development) known to date, which may be understood to be included in the present invention.

Meanwhile, the L (Linker) may be understood to mean a chemical bond without limitation connecting the PTM and the ULB, and is not particularly limited.

On the other hand, the ULB is an E3 ubiquitin ligase binding binder, and specifically can be understood to mean a compound of the chemical structure that is adjacent to, binds to, or has a series of interactions with cereblon E3 ubiquitin ligase, VHL (von Hippel-Lindau tumor suppressor) E3 ubiquitin ligase, MDM2 (Mouse double minute 2 homolog) E3 ubiquitin ligase, or cIAP (Cellular Inhibitor of Apoptosis Protein 1) E3 ubiquitin ligase.

In another aspect of the invention,
the cereblon E3 ubiquitin ligase binding binder may be one represented by one of the chemical formulas represented by the following (a) to (g). 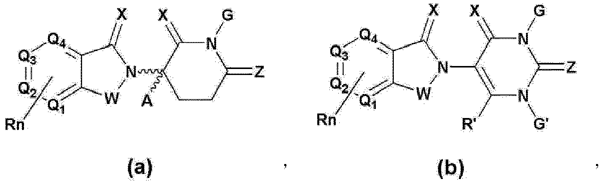 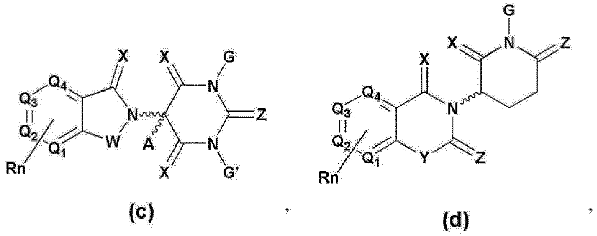 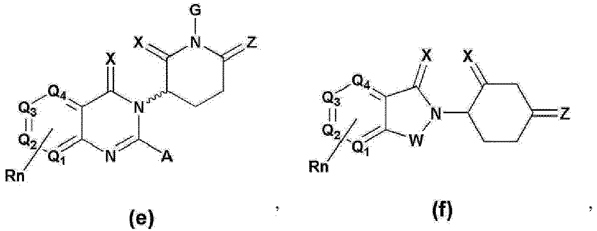 **or**

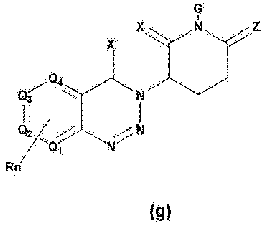

In the chemical formulas (a), (b), (c), (d), (e), (f), and (g),
W is CH₂, CHR, C=O, SO₂, NH, or N-C₁₋₁₀ straight or branched alkyl,
each X is independently O, S, or H₂,
Y is NH, N-C₁₋₁₀ straight or branched alkyl, N-C₆₋₁₀ aryl, N-heteroaryl (5-10-membered ring heteroaryl comprising one or more heteroatoms selected from the group consisting of N, O, and S), N-C₃₋₁₀ cycloalkyl, N-heterocycloalkyl (3-10 membered ring heterocycloalkyl comprising one or more heteroatoms selected from the group consisting of N, O, and S) , O, or S,
Z is O, S, or H₂,
G and G' are each independently H, C₁₋₁₀ straight or branched alkyl, OH, R'-substituted or unsubstituted CH₂-heterocyclyl (3-10 membered ring heterocyclyl comprising one or more heteroatoms selected from the group consisting of N, O, and S), or R'-substituted or unsubstituted benzyl,
Q₁, Q₂, Q₃, and Q₄ are each independently carbon substituted with R', or N,
A is C₁₋₁₀ straight or branched alkyl, C₃₋₁₀ cycloalkyl, or halogen,
R is -CONR'R", -OR', -NR'R", -SR', -SO₂R', -SO₂NR'R", -CR'R"-, -CR'NR'R"-, -C₆₋₁₀ aryl, -heteroaryl (5-10 membered heteroaryl comprising one or more heteroatoms selected from the group consisting of N, O, and S), -C₁₋₁₀ straight or branched alkyl, -C₃₋₁₀ cycloalkyl, -heterocyclyl (3-10 membered ring heterocyclyl comprising one or more heteroatoms selected from the group consisting of N, O, and S), -P(O)(OR')R", -P(O)R'R", -OP(O)(OR')R", -OP(O)R'R", -Cl, -F, -Br, -I, -CF₃, -CN, -NR'SO₂NR'R", -NR'CONR'R", -CONR'COR", -NR'C(=N-CN)NR'R", -C(=N-CN)NR'R", -NR'C(=N-CN)R", -NR'C(=CNO₂)NR'R", -SO₂NR'COR", -NO₂, -CO₂R', -C(C=NOR')R", -CR'=CR'R", -CCR', -S(C=O)(C=N-R')R", -SFs, or -OCF₃,
R' and R" are each independently selected from the group consisting of H, C₁₋₁₀ straight or branched alkyl, C₃₋₁₀ cycloalkyl, C₆₋₁₀ aryl, heteroaryl (5-10 membered ring heteroaryl comprising one or more heteroatoms selected from the group consisting of N, O, and S), and heterocyclyl (3-10 membered ring heterocyclyl comprising one or more heteroatoms selected from the group consisting of N, O, and S), 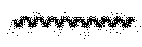 represents stereospecific (R or S) or non-stereospecific binding, and
Rₙ is -(CH₂)ₘ-NA-, -(CH₂)ₘ-O-, -O-(CH₂)ₘ-(C=O)-NA-, or -NA-(CH₂)ₘ-(C=O)-NA-, again, wherein m is an integer from 0 to 5,
the A is each H, or an unsubstituted or substituted C₁₋₁₀ straight or branched alkyl,
here again, the substituted alkyl is substituted with halogen, nitro, or cyano, and Rₙ is covalently bonded to the L (Linker).

In one embodiment of the present invention,
the ULB is 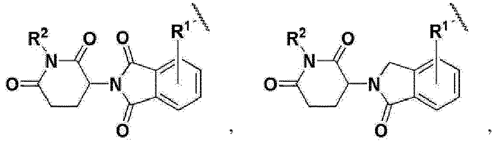 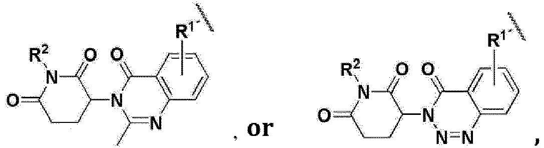
wherein R¹ is -(CH₂)ₙ-NR³-, -(CH₂)ₙ-O-, -O-(CH₂)ₙ-(C=O)-NR³-, or -NR³-(CH₂)ₙ-(C=O)-NR³-,
again, where n is an integer from 0 to 5,
the R² and R³ are each independently H, or unsubstituted or substituted C₁₋₁₀ straight or branched alkyl,
again, wherein the substituted alkyl is substituted with halogen, nitro, or cyano.

Preferably, the ULB is 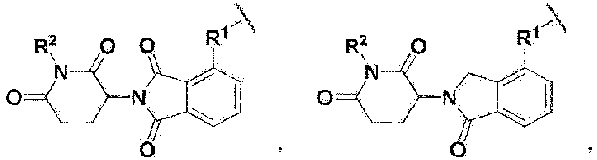 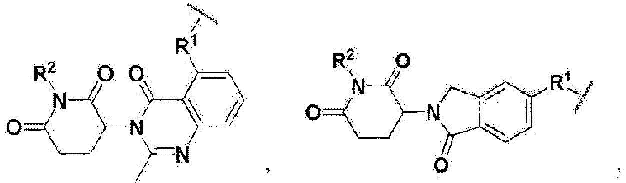 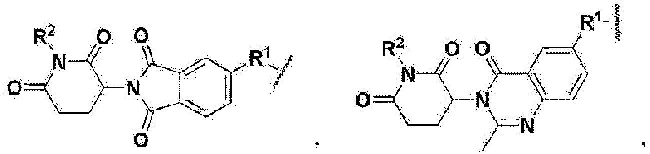 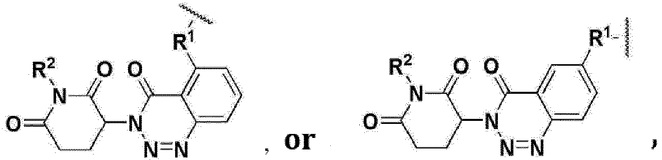
wherein R¹ is -(CH₂)ₙ-NR³-, -(CH₂)ₙ-O-, -O-(CH₂),-(C=O)-NR³-, or -NR³-(CH₂)ₙ-(C=O)-NR³-,
again, where n is an integer from 0 to 5,
the R² and R³ are each independently H, or unsubstituted or substituted C₁₋₁₀ straight or branched alkyl,
again, wherein the substituted alkyl is substituted with halogen, nitro, or cyano.

In yet another embodiment of the present invention, the ULB is a cereblon E3 ubiquitin ligase binding binder, wherein, the ULB is represented by the following chemical formula 1': 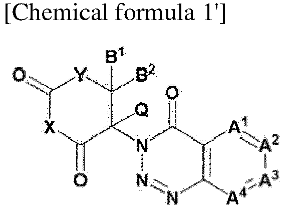 (In the chemical formula 1',
X and Y are each independently CR¹R², NR¹, or O,
here, R¹ and R² are each independently H, OH, halogen, substituted or unsubstituted C₁₋₁₀ straight or branched alkyl, or substituted or unsubstituted C₁₋₁₀ straight or branched alkoxy,
here, the substituted alkyl or substituted alkoxy is substituted with one or more substituents selected from the group consisting of halogen, oxo, -NR⁴R⁵, cyano, nitro, hydroxy, substituted or unsubstituted C₁₋₁₀ straight or branched alkyl, and substituted or unsubstituted C₁₋₁₀ straight or branched alkoxy,
here again, R⁴ and R⁵ are each independently H, or C₁₋₅ straight or branched alkyl, and the substituted alkyl or substituted alkoxy is substituted with one or more substituents selected from the group consisting of halogen, oxo, amino, cyano, nitro, hydroxy, C₁₋₁₀ straight or branched alkyl, C₁₋₁₀ straight or branched alkoxy, substituted or unsubstituted C₃₋₁₀ cycloalkyl, substituted or unsubstituted 3 to 10 membered heterocycloalkyl comprising one or more heteroatoms selected from the group consisting of N, O, and S, and substituted or unsubstituted C₆₋₁₀ aryl,
here again, wherein said substituted cycloalkyl, substituted heterocycloalkyl, or substituted aryl is substituted with one or more substituents selected from the group consisting of -Boc, halogen, amino, cyano, nitro, hydroxy, C₁₋₅ straight or branched alkyl, and C₁₋₅ straight or branched alkoxy;
B¹, B² and Q are each independently H, OH, halogen, amino, cyano, nitro, substituted or unsubstituted C₁₋₁₀ straight or branched alkyl, or substituted or unsubstituted C₁₋₁₀ straight or branched alkoxy,
here, the substituted alkyl or substituted alkoxy is substituted with one or more substituents selected from the group consisting of halogen, amino, cyano, nitro, hydroxy, C₁₋₅ straight or branched alkyl, and C₁₋₅ straight or branched alkoxy;
A¹, A², A³, and A⁴ are each independently N or CR³,
here, R³ is H, OH, halogen, amino, cyano, nitro, substituted or unsubstituted C₁₋₁₀ straight or branched alkyl, or substituted or unsubstituted C₁₋₁₀ straight or branched alkoxy,
here, the substituted alkyl or substituted alkoxy is substituted with one or more substituents selected from the group consisting of halogen, oxo, -NR⁴R⁵, cyano, nitro, hydroxy, C₁₋₅ straight or branched alkyl, and C₁₋₅ straight or branched alkoxy,
here again, R⁴ and R⁵ are each independently H, or C₁₋₅ straight or branched alkyl); and
at least one of X, Y, B¹, B², Q, A¹, A², A³, and A⁴ is
when L is a single bond, it is directly linked to the PTM by a covalent bond, or
when L is a chemical linker, it is modified to be linked to L by a covalent bond).

In one embodiment of the present invention,
the ULB may be a cereblon E3 ubiquitin ligase binding molecule represented by the following chemical formula 1": 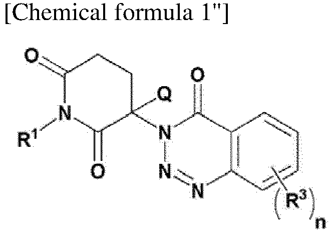 (In the chemical formula 1 ",
Q, R¹ and R³ are as defined in claim 1,
n is an integer from 0 to 4, wherein when n is from 2 to 4, each R³ is independently selected from those defined in claim 1), and
at least one of Q, R¹ and R³ is,
when L in chemical formula 1 is a single bond, it is directly linked to the PTM by a covalent bond, or
when L in chemical formula 1 is a chemical linker, it is covalently linked to L).

In one embodiment of the present invention, the ULB is a cereblon E3 ubiquitin ligase binding molecule which is any one selected from the group of compounds consisting of the followings, and as defined in chemical formula 1 above, at least one of X, Y, B¹, B², Q, A¹, A², A³, and A⁴ in the following compounds is directly linked to the PTM by a covalent bond when L is a single bond, or modified to be linked to L by a covalent bond when L is a chemical linker:
(1) 3-(5-fluoro-4-oxobenzo[d][1,2,3]triazin-3(4H)-yl)piperidin-2,6-dione;
(2) 3-(5-amino-4-oxobenzo[d][1,2,3]triazin-3(4H)-yl)piperidin-2,6-dione;
(3) 3-(5-nitro-4-oxobenzo[d][1,2,3]triazin-3(4H)-yl)piperidin-2,6-dione;
(4) 3-(5-chloro-4-oxobenzo[d][1,2,3]triazin-3(4H)-yl)piperidin-2,6-dione;
(5) 3-(5-methyl-4-oxobenzo[d][1,2,3]triazin-3(4H)-yl)piperidin-2,6-dione;
(6) 3-(6-chloro-4-oxobenzo[d][1,2,3]triazin-3(4H)-yl)piperidin-2,6-dione;
(7) 3-(6-bromo-4-oxobenzo[d][1,2,3]triazin-3(4H)-yl)piperidin-2,6-dione;
(8) 3-(6-iodo-4-oxobenzo[d][1,2,3]triazin-3(4H)-yl)piperidin-2,6-dione;
(9) 3-(8-bromo-6-methyl-4-oxobenzo[d][1,2,3]triazin-3(4H)-yl)piperidin-2,6-dione;
(10) 3-(2,6-dioxopiperidin-3-yl)-4-oxo-3,4-dihydrobenzo[d][1,2,3]triazin-6-carbonitrile;
(11) 3-(5-iodo-4-oxobenzo[d][1,2,3]triazin-3(4H)-yl)piperidin-2,6-dione;
(12) 3-(4-oxo-6-(trifluoromethoxy)benzo[d][1,2,3]triazin-3(4H)-yl)piperidin-2,6-dione;
(13) 3-(6-methoxy-4-oxobenzo[d][1,2,3]triazin-3(4H)-yl)piperidin-2,6-dione;
(14) N-(3-(2,6-dioxopiperidin-3-yl)-4-oxo-3,4-dihydrobenzo[d][1,2,3]triazin-5-yl)acetamide;
(15) 3-(8-nitro-4-oxobenzo[d][1,2,3]triazin-3(4H)-yl)piperidin-2,6-dione;
(16) 3-(8-amino-4-oxobenzo[d][1,2,3]triazin-3(4H)-yl)piperidin-2,6-dione;
(17) N-(3-(2,6-dioxopiperidin-3-yl)-4-oxo-3,4-dihydrobenzo[d][1,2,3]triazin-6-yl)acetamide;
(18) 3-(5-hydroxy-4-oxobenzo[d][1,2,3]triazin-3(4H)-yl)piperidin-2,6-dione;
(19) (3-(5-amino-4-oxobenzo[d][1,2,3]triazin-3(4H)-yl)-2,6-dioxopiperidin-1-yl)methyl pivalate;
(20) (3-(5-amino-4-oxobenzo[d][1,2,3]triazin-3(4H)-yl)-2,6-dioxopiperidin-1-yl)methyl benzoate;
(21) (3-(5-amino-4-oxobenzo[d][1,2,3]triazin-3(4H)-yl)-2,6-dioxopiperidin-1-yl)methyl piperidin-4-carboxylate hydrochloride;
(22) 2-((3-(5-amino-4-oxobenzo[d][1,2,3]triazin-3(4H)-yl)-2,6-dioxopiperidin-1-yl)methyl)1-(tert-butyl)(2R)-pyrrolidin-1,2-dicarboxylate;
(23) (3-(5-amino-4-oxobenzo[d][1,2,3]triazin-3(4H)-yl)-2,6-dioxopiperidin-1-yl)methyl D-prolinate hydrochloride;
(24) (3-(5-amino-4-oxobenzo[d][1,2,3]triazin-3(4H)-yl)-2,6-dioxopiperidin-1-yl)methyl L-isoleucinate hydrochloride;
(25) (3-(5-amino-4-oxobenzo[d][1,2,3]triazin-3(4H)-yl)-2,6-dioxopiperidin-1-yl)methyl butyrate;
(26) 3-(4-oxobenzo[d][1,2,3]triazin-3(4H)-yl)piperidin-2,6-dione;
(27) 3-(7-nitro-4-oxobenzo[d][1,2,3]triazin-3(4H)-yl)piperidin-2,6-dione;
(28) 3-(6-nitro-4-oxobenzo[d][1,2,3]triazin-3(4H)-yl)piperidin-2,6-dione;
(29) 3-(7-amino-4-oxobenzo[d][1,2,3]triazin-3(4H)-yl)piperidin-2,6-dione;
(30) 3-(2,6-dioxopiperidin-3-yl)-4-oxo-3,4-dihydrobenzo[d][1,2,3]triazin-5-carboxylic acid;
(31) 3-(5-amino-4-oxobenzo[d][1,2,3]triazin-3(4H)-yl)-3-methylpiperidin-2,6-dione;
(32) 3-(2,6-dioxopiperidin-3-yl)-4-oxo-3,4-dihydrobenzo[d][1,2,3]triazin-5-carboxamide;
(33) 3-(2,6-dioxopiperidin-3-yl)-N-methyl-4-oxo-3,4-dihydrobenzo[d][1,2,3]triazin-5-carboxamide;
(34) N-(3-(2,6-dioxopiperidin-3-yl)-4-oxo-3,4-dihydrobenzo[d][1,2,3]triazin-5-yl)benzamide; and
(35) 3-(6-amino-4-oxobenzo[d][1,2,3]triazin-3(4H)-yl)piperidin-2,6-dione.

In another aspect of the present invention,
the cereblon E3 ubiquitin ligase binder, ULB, is a moiety that binds to cereblon E3 ubiquitin ligase. The ULB provided in the present invention can be understood to be particularly excellent in binding to cereblon E3 ubiquitin ligase, and preferably, it exhibits superior binding activity compared to the conventional cereblon E3 ubiquitin ligase binder, and more preferably, it can be understood that the ubiquitination of the target protein or polypeptide is excellently induced from cereblon E3 ubiquitin ligase. Alternatively, the ULB provided in the present invention can be understood as a binder that can better exhibit effects such as degradation, activity inhibition, or activity regulation of target proteins or polypeptides from cereblon E3 ubiquitin ligase.

In another aspect of the invention,
the ULB is a VHL E3 ubiquitin ligase binding binder, and
the VHL (von Hippel-Lindau tumor suppressor) E3 ubiquitin ligase binding binder may be represented by the following chemical formula h:

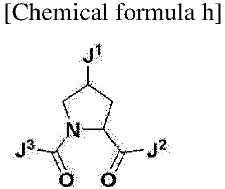

In the chemical formula h,
J¹ is OJ⁴,
J² is optionally substituted or unsubstituted-NJ⁵-(CH₂)o-C₆₋₁₀aryl-5-10 membered heteroaryl comprising one or more heteroatoms selected from the group consisting of N, O, and S,
the substituted-NJ⁵-(CH₂)o-C₆₋₁₀aryl-5-10 membered heteroaryl is substituted with C₁₋₁₀ straight or branched alkyl,
here, the J⁵ is H or C₁₋₅ straight or branched alkyl, and o is 0-5, and
J³ is -CJ⁶-NJ⁷-,
here, J⁶ is C₁₋₁₀ straight or branched alkyl, J⁷ is H or C₁₋₅ straight or branched alkyl, and J³ is covalently bonded to the L (Linker).

In one embodiment of the present invention,
the ULB may be 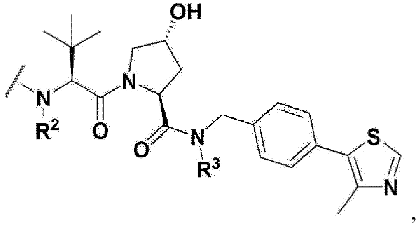
wherein R² and R³ are each independently H, or unsubstituted or substituted C₁₋₁₀ straight or branched alkyl,
here again, the substituted alkyl is substituted with a halogen, nitro, or cyano.

Meanwhile, the E3 cerebron ubiquitin ligase is used to describe the target enzyme(s) binding site of the ubiquitin ligase moiety in the bifunctional (chimeric) compound according to the present invention. E3 is a protein that, together with the E2 ubiquitin-conjugating enzyme, causes the attachment of ubiquitin to the lysine of the target protein.

E3 ubiquitin ligase targets specific protein substrates for degradation by the proteasome. Thus, E3 ubiquitin ligase, alone or in context, with the E2 ubiquitin conjugating enzyme is responsible for the delivery of ubiquitin to the target protein. In general, the ubiquitin ligase is involved in polyubiquitination such that a second ubiquitin is attached to a first ubiquitin, a third ubiquitin is attached to a second ubiquitin, and so on. Polyubiquitination marks proteins for degradation by the proteasome. However, there may be some ubiquitination events limited to mono-ubiquitination in which only a single ubiquitin is added to the substrate molecule by the ubiquitin ligase. Mono-ubiquitinated proteins are not targeted to the degradation proteasome, but instead their intracellular location or function may be altered, for example, through binding to other proteins having domains capable of binding ubiquitin. As a further complication, other lysines on ubiquitin can be targeted by E3 to make chains. The most common lysine is Lys48 of the ubiquitin chain. It is recognized by the proteasome and is a lysine used to make polyubiquitin.

In addition, in the compound represented by the chemical formula 1,
the L (Linker) may be a single bond, which means that the ULB and the PTM are directly covalently linked.

Here, when the ULB and the PTM are directly connected, it leads to at least one covalent bond, and the covalent bond may be a single bond, a double bond, or a triple bond, but is not particularly limited.

In one embodiment of the present invention,
the L is at least one selected from the group consisting of single bond, phenylene, -(CH₂)ᵢ-, -(CH₂)ᵢ-O-, -(CH₂-CH₂-O)ᵢ-, -(CH₂)ᵢ-S-, -(CH₂)ᵢ-NR-, -(CH₂)ᵢ-W₁U₁-, 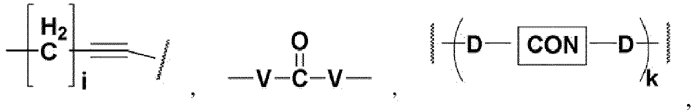 and combinations thereof.

Here,
D is each independently a single bond, 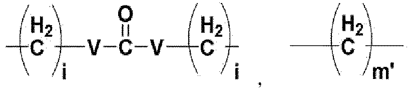 , **or** 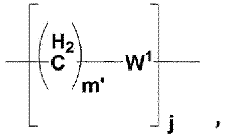 each i, m' or j is each independently 0 to 100,
V is independently a single bond, O, S, or N-R,
W₁U₁ forms an amide group, a urethane group, an ester or thioester group,
W¹ is O, S, or N-R,
R is H, C₁₋₃ alkyl, an alkanol group, or 3 to 10 membered heterocycloalkyl comprising one or more heteroatoms selected from the group consisting of N, O, and S,
CON is a single bond, piperazinyl, substituted or unsubstituted alkylene, 3 to 10 membered heterocycloalkylene comprising one or more heteroatoms selected from the group consisting of N, O, and S, 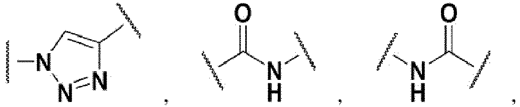 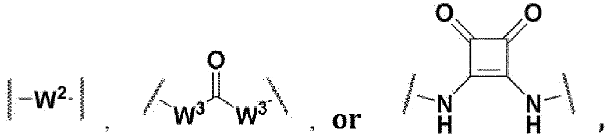
here again, W² is O, S, NR⁶, S(O), S(O)₂, -S(O)₂O, or -OS(O)₂O,
W³ is O, S, CHR⁶, or NR⁶, and
R⁶ may be H, or C₁₋₃ alkyl unsubstituted or substituted with one or two hydroxy.

In another aspect of the invention,
the L (Linker) may be any one selected from the group consisting of the followings, or their combination of 2 to 20. 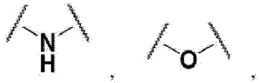 C₁₋₂₀ straight or branched alkylene, C₁₋₂₀ straight or branched alkenylene containing one or more double bonds, C₁₋₂₀ straight or branched alkynylene containing one or more triple bonds, phenylene, 5 to 6 membered heteroarylene comprising one or more heteroatoms selected from the group consisting of N, O, and S, C₃₋₁₀ cycloalkylene, and 3 to 10 membered heterocycloalkylene comprising one or more heteroatoms selected from the group consisting of N, O and S.

In one embodiment of the present invention,
the L (Linker) may be any one selected from the group consisting of the followings, or their combination of 2 to 10.

The L (Linker) may be any one among 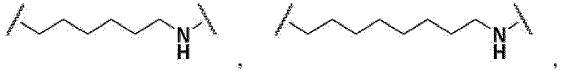 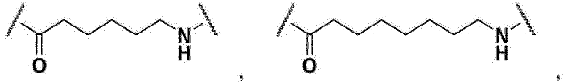 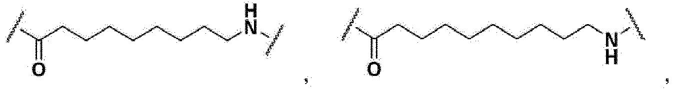 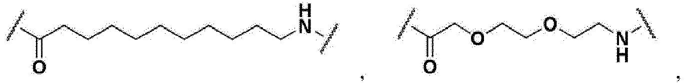 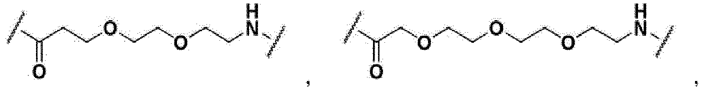 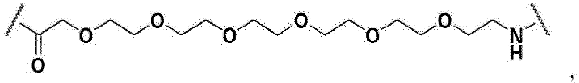 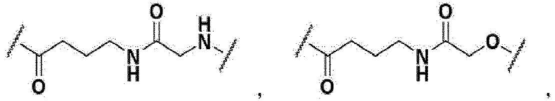 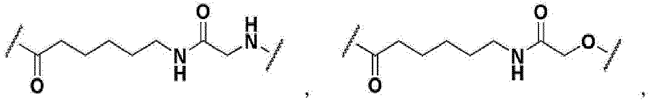 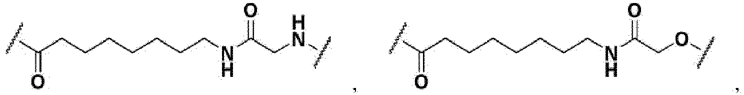 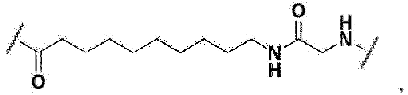 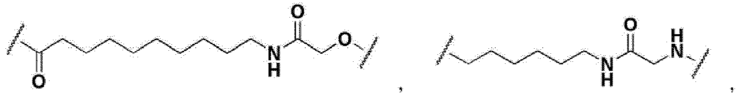 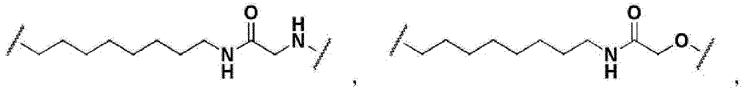 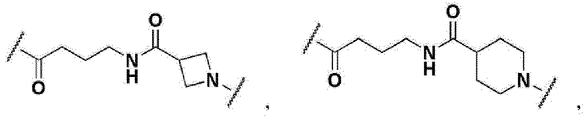 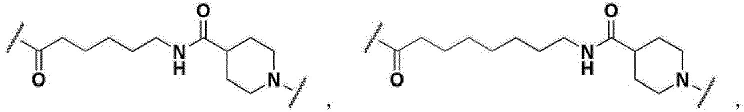 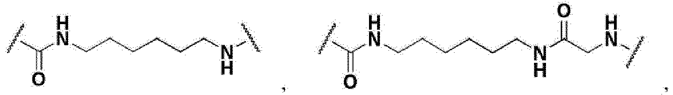 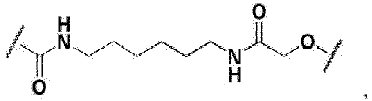 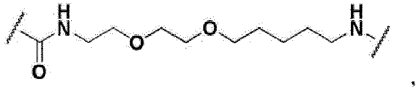 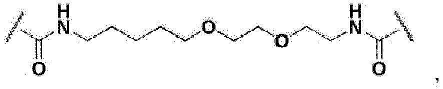 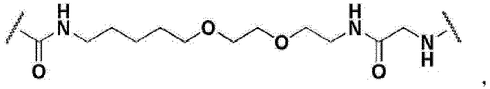 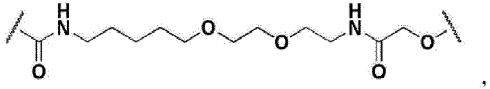 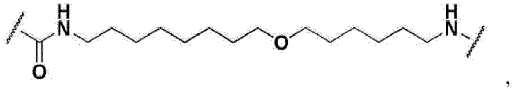 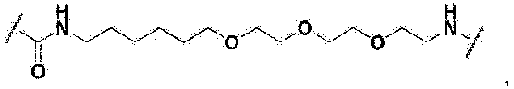 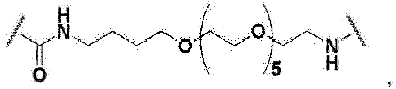 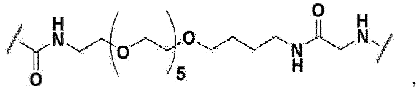 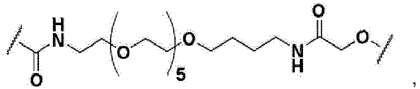 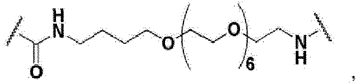 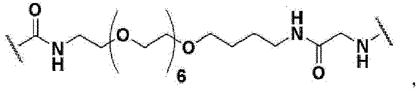 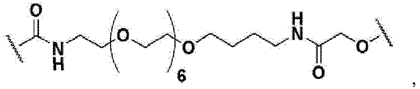 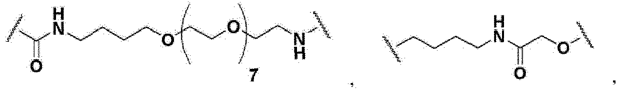 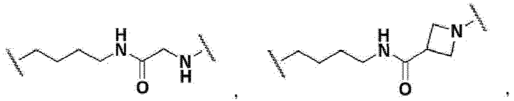 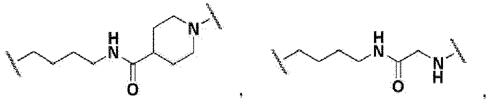 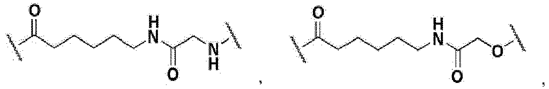 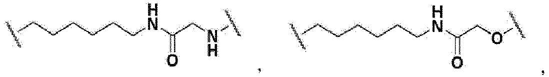 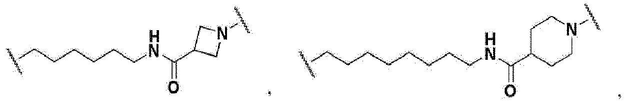 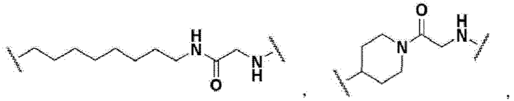 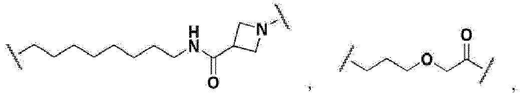 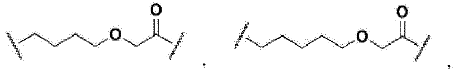 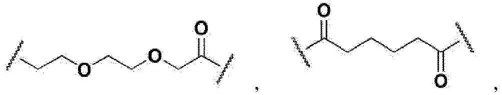 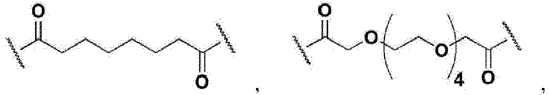 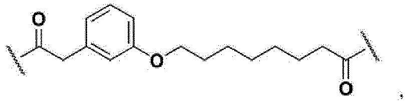 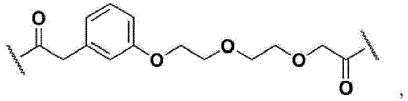 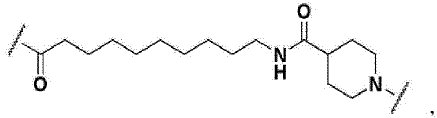 **and**

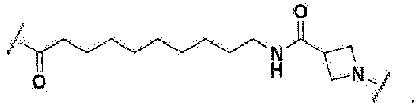

Furthermore, in the compound represented by the chemical formula 1,
the PTM is an EEM (embryonic ectoderm development) targeting molecule, and
the EEM (embryonic ectoderm development) targeting molecule may be represented by one of the chemical formulas represented by the following (i) to (j). 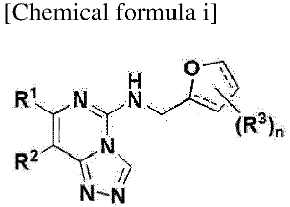
in the chemical formula i,
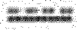 is a single bond or a double bond;
R¹ is independently selected from H, halogen and C1-C4 alkyl;
R² is independently selected from halogen, phenyl, and 5- to 6-membered heteroaryl comprising carbon atoms and 1-4 heteroatoms selected from N, NR^{a}, O, and S(O)ₚ; wherein said phenyl and heteroaryl are substituted with 0-3 R^{2A};
each R^{2A} is independently halogen, CN, -(O)ₘ-(C1-C6 alkyl substituted with 0-1 R^{2B}), C1-C6 haloalkyl, C1-C6 haloalkoxy, R^{2C}, -OR^{2C}, -C(=O)R^{2D}, NR^{2E}R^{2F}, -C(=O)NR^{2E}R^{2F},-NHC(=O)R^{2D}, -S(=O)₂R^{2D}, -S(=O)₂NR^{2E}R^{2F}, -NHS(=O)₂(C1-C4alkyl), and -CR^{2C}R^{2E}R^{2G};
R^{2B} is independently OH, NR^{e}R^{f}, C1-C4 alkoxy, -C(=O)NR^{e}R^{f}, -S(=O)₂(C1-C4 alkyl), - NHC(=O)(C1-C4 alkyl), and 5- to 6-membered heterocycloalkyl comprising carbon atoms and 1-2 heteroatoms selected from N, NR^{a}, O, and S(O)ₚ; wherein said heterocycloalkyl is substituted with 0-2 R^{c};
each R^{2C} is independently selected from C3-C6 cycloalkyl, phenyl, and 4- to 7-membered heterocycle comprising carbon atoms and 1-4 heteroatoms selected from N, NR^{a}, O, and S(O)ₚ; wherein each moiety is substituted with 0-2 R^{c};
each R^{2D} is independently selected from C1-C4 alkyl and R^{2C};
R^{2E} and R^{2G} at each occurrence are independently selected from H and C1-C4 alkyl;
each R^{2F} is independently selected from H and C1-C4 alkyl substituted with 0-1 R^{d};
R³ is independently selected from OH and C1-C4 alkyl;
each R^{a} is independently H, O, C1-C4 alkyl substituted with 0-1 R^{b}, -C(=O)H, -C(=O)(C1-C4 alkyl), -CO₂(C1-C4) alkyl), C3-C6 cycloalkyl, and benzyl;
R^{b} is independently selected from halogen, OH and C1-C4 alkoxy;
each R^{c} is independently selected from =O, halogen, OH, C1-C4 alkyl, C1-C4 haloalkyl, C1-C4 alkoxy, and C1-C4 haloalkoxy;
R^{d} is independently selected from OH and NR^{e}R^{f};
R^{e} and R^{f} at each occurrence are independently selected from H and C1-C4 alkyl;
each p is independently selected from 0, 1 and 2;
m and n at each occurrence are independently selected from 0 and 1. 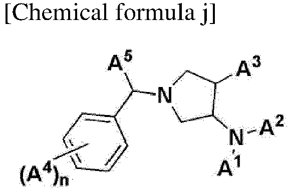
in the chemical formula j,
A¹ and A² are independently C1-C2 alkyl;
A³ is a substituted or unsubstituted C6-10 aryl, a 5- to 10-membered substituted or unsubstituted heteroaryl comprising at least one hetero atom selected from the group consisting of N, O, and S, or 3- to 10-membered substituted or unsubstituted heterocycloalkyl comprising one or more heteroatoms selected from the group consisting of N, O, and S,
wherein said substituted aryl, substituted heteroaryl, and substituted heterocycloalkyl are substituted with one or more substituents selected from the group consisting of R¹, OR¹, SR¹, S(O)R¹, SO₂R¹, C(O)R¹, CO(O)R¹, OC(O)R¹, OC(O)OR¹, NH₂, NHR¹, N(R¹)₂, NHC(O)R¹, NR¹C(O)R¹, NHS(O)₂R¹, NR¹S(O)₂R¹, NHC(O)OR¹, NR¹C(O)OR¹, NHC(O)NH₂, NHC(O)NHR¹, NHC(O)N(R¹)₂, NR1C(O)NHR¹, NR¹C(O)N(R¹)₂, C(O)NH₂, C(O)NHR¹, C(O)N(R¹)₂, C(O)NHOH, C(O)NHOR¹, C(O)NHSO₂R¹, C(O)NR¹SO₂R¹, SO₂NH₂, SO₂NHR¹, SO₂N(R¹)₂, C(O)H, C(O)OH, C(N)NH₂, C(N)NHR¹, C(N)N(R¹)₂, CNOH, CNOCH₃, OH, (O), CN, NO₂, CF₃, CF₂CF₃, OCF₃, OCF₂CF₃, F, Cl, Br and I;
A⁴ is each independently a substituent selected from the group consisting of straight or branched C1-C6 alkyl, OH and halogen, and n is 0 to 5;
A⁵ is a substituent selected from the group consisting of straight or branched C1-C6 alkyl, OH and halogen;
R¹ is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R¹ is straight or branched C1-C6 alkyl, straight or branched C2-C6 alkenyl, and straight or branched C2-C6 alkynyl, it may be optionally substituted with one or more substituents selected from the group consisting of R², OR², SR², S(O)R² , SO₂R², C(O)R², CO(O)R², OC(O)R², OC(O)OR², NH₂, NHR², N(R²)², NHC(O)R², NR²C(O)R², NHS(O)₂R², NR²S(O)₂R², NHC(O)OR², NR²C(O)OR², NHC(O)NH₂, NHC(O)NHR², NHC(O)N(R²)₂, NR²C(O)NHR², NR²C(O)N(R²)₂, C(O)NH₂, C(O)NHR², C(O)N(R²)₂, C(O)NHOH, C(O)NHOR₂, C(O)NHSO₂R², C(O)NR²SO₂R², SO₂NH₂, SO₂NHR², SO₂N(R²)₂, C(O)H, C(O)OH, OH, (O), CN, NO₂, CF₃, CF₂CF₃, OCF₃, OCF₂CF₃, and halogen,
on the other hand, when R¹ is aryl, heteroaryl, heterocycloalkyl, cycloalkyl, and cycloalkenyl, it may be optionally substituted with one or more substituents selected from the group consisting of R³, OR³, SR³, S(O)R³, SO₂R³, C(O)R³, CO(O)R³, OC(O)R³, OC(O)OR³, NH₂, NHR³, N(R³)₂, NHC(O)R³, NR³C(O)R³, NHS(O)₂R³, NR³S(O)₂R³, NHC(O)OR³, NR³C(O)OR³, NHC(O)NH₂, NHC(O)NHR³, NHC(O)N(R³)₂, NR³C(O)NHR³, NR³C(O)N(R³)₂, C(O)NH₂, C(O)NHR³, C(O)N(R³)₂, C(O)NHOH, C(O)NHOR³, C(O)NHSO₂R³, C(O)NR³SO₂R³, SO₂NH₂, SO₂NHR³, SO₂N(R³)₂, C(O)H, C(O)OH, C(O)C(O)NH₂, C(O)C(O)NHR³, C(O)C(O)N(R³)₂, C(N)NH₂, C(N)NHR³, C(N)N(R³)₂, CNOH, CNOCH₃, OH, (O), CN, NO₂, CF₃, CF₂CF₃, OCF₃, OCF₂CF₃, and halogen;
R² is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R² is straight or branched C1-C6 alkyl, straight or branched C2-C6 alkenyl, and straight or branched C2-C6 alkynyl, it may be optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen,
on the other hand, when R² is aryl, heteroaryl, heterocycloalkyl, cycloalkyl, and cycloalkenyl, it may be optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen;
R³ is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R³ is straight or branched C1-C6 alkyl, straight or branched C2-C6 alkenyl, and straight or branched C2-C6 alkynyl, it may be optionally substituted with one or more substituents selected from the group consisting of R⁴, OR⁴, SR⁴, S(O)R⁴, SO₂R⁴, C(O)R⁴, CO(O)R⁴, OC(O)R⁴, OC(O)OR⁴, NH₂, NHR⁴, N(R⁴)₂, NHC(O)R⁴, NR⁴C(O)R⁴, NHS(O)₂R⁴, NR⁴S(O)₂R⁴, NHC(O)OR₄, NR⁴C(O)OR⁴, NHC(O)NH₂, NHC(O)NHR⁴, NHC(O)N(R⁴)₂, NR⁴C(O)NHR⁴, NR⁴C(O)N(R)₂,⁴ C(O)NH₂, C(O)NHR⁴, C(O)N(R⁴)₂, C(O)NHOH, C(O)NHOR⁴, C(O)NHSO₂R⁴, C(O)NR⁴SO₂R⁴, SO₂NH₂, SO₂NHR⁴, SO₂N(R⁴)₂, C(O)H, C(O)OH, OH, (O), CN, NO₂, CF₃, CF₂CF₃, OCF₃, OCF₂CF₃, and halogen,
meanwhile, when R³ is aryl, heteroaryl, heterocycloalkyl, cycloalkyl, and cycloalkenyl, it may be optionally substituted with one or more substituents selected from the group consisting of R⁵, OR⁵, SR⁵, S(O)R⁵, SO₂R⁵, C(O)R⁵, CO(O)R⁵, OC(O)R⁵, OC(O)OR⁵, NH₂, NHR⁵, N(R⁵)₂, NHC(O)R⁵, NR⁵C(O)R⁵, NHS(O)₂R⁵, NR⁵S(O)₂R⁵, NHC(O)OR⁵, NR⁵C(O)OR⁵, NHC(O)NH₂, NHC(O)NHR⁵, NHC(O)N(R⁵)₂, NR⁵C(O)NHR⁵, NR⁵C(O)N(R⁵)₂, C(O)NH₂, C(O)NHR⁵, C(O)N(R⁵)₂, C(O)NHOH, C(O)NHOR⁵, C(O)NHSO₂R⁵, C(O)NR⁵SO₂R⁵, SO₂NH₂, SO₂NHR⁵, SO₂N(R⁵)₂, C(O)H, C(O)OH, C(N)NH₂, C(N)NHR⁵, C(N)N(R⁵)₂, CNOH, CNOCH₃, OH, (O), CN, NO₂, CF₃, CF₂CF₃, OCF³, OCF₂CF₃, and halogen;
R⁴ is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R⁴ is straight or branched C1-C6 alkyl, straight or branched C2-C6 alkenyl, and straight or branched C2-C6 alkynyl, it may be optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen,
meanwhile, when R⁴ is aryl, heteroaryl, heterocycloalkyl, cycloalkyl, and cycloalkenyl, it may be optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen;
R⁵ is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R⁵ is straight or branched C1-C6 alkyl, straight or branched C2-C6 alkenyl, and straight or branched C2-C6 alkynyl, it may be optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen,
meanwhile, when R⁵ is aryl, heteroaryl, heterocycloalkyl, cycloalkyl, and cycloalkenyl, it may be optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen.

In one embodiment of the present invention,
The PTM is 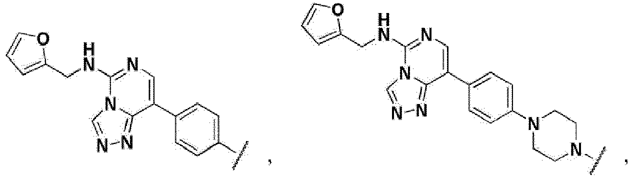 or

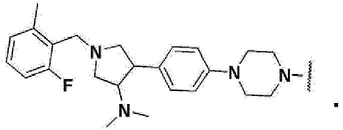

Alternatively, the EED (embryonic ectoderm development) targeting molecule may be a compound represented by the following (i'). 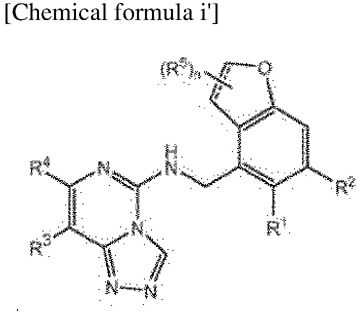 (In the chemical formula i',
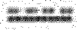 is a single bond or a double bond;
R¹ and R² are independently H or halogen;
R³ is independently selected from halogen, phenyl, and 5- to 6-membered heteroaryl comprising carbon atoms and 1-4 heteroatoms selected from N, NR^{a}, O, and S(O)ₚ; wherein said phenyl and heteroaryl are substituted with 0-3 R^{3A};
each R^{3A} is independently selected from halogen, CN, -(O)ₘ-(C₁-C₆ alkyl substituted with 0-1 R^{3B}), C₁-C₆ haloalkyl, C₁-C₆ haloalkoxy, R^{3C}, -OR^{3C}, -C(=O)R^{3D}, NR^{3E}R^{3F}, -C(=O)NR^{3E}R^{3F}, -NHC(=O)R^{3D}, -S(=O)₂R^{3D}, -S(=O)₂NR^{3E}R^{3F}, -NHS(=O)₂(C₁-C₄ alkyl), and -CR^{3C}R^{3E}R^{3G};
R^{3B} is independently selected from OH, NR^{e}R^{f}, C1-C4 alkoxy, -C(=O)NR^{e}R^{f}, -S(=O)₂(C₁-C₄ alkyl), -NHC(=O)(C₁-C₄ alkyl), and 5- to 6-membered heterocycloalkyl comprising carbon atoms and 1-2 heteroatoms selected from N, NR^{a}, O, and S(O)ₚ; wherein said heterocycloalkyl is substituted with 0-2 R^{c};
each R^{3C} is independently selected from C₃-C₆ cycloalkyl, phenyl, and 4- to 7-membered heterocycle comprising carbon atoms and 1-4 heteroatoms selected from N, NR^{a}, O, and S(O)ₚ; wherein each moiety is substituted with 0-2 R^{c};
each R^{3D} is independently selected from C₁-C₄ alkyl and R^{3C};
R^{3E} and R^{3G} at each occurrence are independently selected from H and C₁-C₄ alkyl;
each R^{3F} is independently selected from H and C₁-C₄ alkyl substituted with 0-1 R^{d};
R⁴ is independently selected from H, halogen and C₁-C₄ alkyl;
R⁵ is independently selected from OH and C₁-C₄ alkyl;
each R^{a} is independently H, →O, C₁-C₄ alkyl substituted with 0-1 R^{b}, -C(=O)H, - C(=O)(C₁-C₄ alkyl), -CO₂(C₁-C₄ alkyl), C3-C6 cycloalkyl, and benzyl;
R^{b} is independently selected from halogen, OH and C₁-C₄ alkoxy;
each R^{c} is independently selected from =O, halogen, OH, C₁-C₄ alkyl, C₁-C₄ haloalkyl, C₁-C₄ alkoxy, and C₁-C₄ haloalkoxy;
R^{d} is independently selected from OH and NR^{e}R^{f};
R^{e} and R^{f} at each occurrence are independently selected from H and C₁-C₄ alkyl;
each p is independently selected from 0, 1 and 2;
m and n at each occurrence are independently selected from 0 and 1).

Alternatively, the EED (embryonic ectoderm development) targeting molecule may be a compound represented by the following (j').

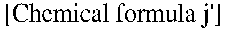

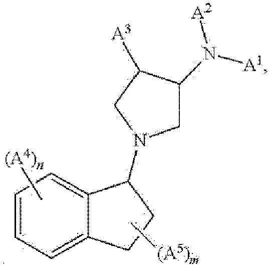

(In the chemical formula j',
A¹ and A² are each independently C₁-C₂ alkyl;
A³ is selected from the group consisting of aryl, heterocyclyl and heteroaryl, wherein the aryl, heterocyclyl and heteroaryl of A³ are optionally substituted with one or more substituents selected from the group consisting of R¹, OR¹, SR¹, S(O)R¹, SO₂R¹, C(O)R¹, CO(O)R¹, OC(O)R¹, OC(O)OR¹, NH₂, NHR¹, N(R¹)₂, NHC(O)R¹, NR¹C(O)R¹, NHS(O)₂R¹, NR¹S(O)₂R¹, NHC(O)OR¹, NR¹C(O)OR¹, NHC(O)NH₂, NHC(O)NHR¹, NHC(O)N(R¹)₂, NR¹C(O)NHR¹, NR¹C(O)N(R¹)₂, C(O)NH₂, C(O)NHR¹, C(O)N(R¹)₂, C(O)NHOH, C(O)NHOR¹, C(O)NHSO₂R¹, C(O)NR¹SO₂R¹, SO₂NH₂, SO₂NHR¹, SO₂N(R¹)₂, C(O)H, C(O)OH, C(N)NH₂, C(N)NHR¹, C(N)N(R¹)₂, CNOH, CNOCH₃, OH, (O), CN, NO₂, CF₃, CF₂CF₃, OCF₃, OCF₂CF₃, F, Cl, Br and I;
each A⁴ is a substituent independently selected from the group consisting of C1-C6 alkyl, OH, F, Cl, Br and I, which is a substituent on the substitutable position of the benzene ring of indane;
each A⁵ is a substituent independently selected from the group consisting of C1-C6 alkyl, OH, F, Cl, Br and I, and is a substituent on a substitutable position of the cyclopentane ring of indane;
each R¹ is independently selected from the group consisting of C₁-C₆ alkyl, C₂-C₆ alkenyl, C₂-C₆ alkynyl, aryl, heteroaryl, heterocyclyl, cycloalkyl and cycloalkenyl; wherein C₁-C₆ alkyl, C2-C6 alkenyl, and C2-C6 alkynyl of each R¹ are optionally substituted with one or more substituents selected from the group consisting of R², OR², SR², S(O)R² , SO₂R², C(O)R², CO(O)R², OC(O)R², OC(O)OR², NH₂, NHR², N(R²)₂, NHC(O)R², NR²C(O)R², NHS(O)₂R², NR²S(O)₂R², NHC(O)OR², NR²C(O)OR², NHC(O)NH₂, NHC(O)NHR², NHC(O)N(R²)₂, NR²C(O)NHR², NR²C(O)N(R²)₂, C(O)NH₂, C(O)NHR², C(O)N(R²)₂, C(O)NHOH, C(O)NHOR², C(O)NHSO₂R², C(O)NR²SO₂R², SO₂NH₂, SO₂NHR², SO₂N(R²)₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R¹ are optionally substituted with one or more substituents selected from the group consisting of R³, OR³, SR³, S(O)R³, SO₂R³, C(O)R³, CO(O)R³, OC(O)R³, OC(O)OR³, NH₂, NHR³, N(R³)₂, NHC(O)R³, NR³C(O)R³, NHS(O)₂R³, NR³S(O)₂R³, NHC(O)OR³, NR³C(O)OR³, NHC(O)NH₂, NHC(O)NHR³, NHC(O)N(R³)₂, NR³C(O)NHR³, NR³C(O)N(R³)₂, C(O)NH₂, C(O)NHR³, C(O)N(R³)₂, C(O)NHOH, C(O)NHOR³, C(O)NHSO₂R³, C(O)NR³SO₂R³, SO₂NH₂, SO₂NHR³, SO₂N(R³)₂, C(O)H, C(O)OH, C(O)C(O)NH₂, C(O)C(O)NHR³, C(O)C(O)N(R³)₂, C(N)NH₂, C(N)NHR³, C(N)N(R³)₂, CNOH, CNOCH₃, OH, (O), CN, NO₂, CF₃, CF₂CF₃, OCF₃, OCF₂CF₃, F, Cl, Br and I;
each R² is independently selected from the group consisting of C₁-C₆ alkyl, C₂-C₆ alkenyl, C₂-C₆ alkynyl, aryl, heteroaryl, heterocyclyl, cycloalkyl and cycloalkenyl; wherein C₁-C₆ alkyl, C₂-C₆ alkenyl, and C₂-C₆ alkynyl of each R² are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R² are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I;
each R3 is independently selected from the group consisting of C₁-C₆ alkyl, C₂-C₆ alkenyl, C₂-C₆ alkynyl, aryl, heteroaryl, heterocyclyl, cycloalkyl and cycloalkenyl; wherein C₁-C₆ alkyl, C₂-C₆ alkenyl, and C₂-C₆ alkynyl of each R³ are optionally substituted with one or more substituents selected from the group consisting of R⁴, OR⁴, SR⁴, S(O)R⁴, SO₂R⁴, C(O)R⁴, CO(O)R⁴, OC(O)R⁴, OC(O)OR⁴, NH₂, NHR⁴, N(R⁴)₂, NHC(O)R⁴, NR⁴C(O)R⁴, NHS(O)₂R⁴, NR⁴S(O)₂R⁴, NHC(O)OR⁴, NR⁴C(O)OR⁴, NHC(O)NH₂, NHC(O)NHR⁴, NHC(O)N(R⁴)₂, NR⁴C(O)NHR⁴, NR⁴C(O)N(R⁴)₂, C(O)NH₂, C(O)NHR⁴, C(O)N(R⁴)₂, C(O)NHOH, C(O)NHOR⁴, C(O)NHSO₂R⁴, C(O)NR⁴SO₂R⁴, SO₂NH₂, SO₂NHR⁴, SO₂N(R⁴)₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R³ are optionally substituted with one or more substituents selected from the group consisting of R⁵, OR⁵, SR⁵, S(O)R⁵, SO₂R⁵, C(O)R⁵, CO(O)R⁵, OC(O)R⁵, OC(O)OR⁵, NH₂, NHR⁵, N(R⁵)₂, NHC(O)R⁵, NR⁵C(O)R⁵, NHS(O)₂R⁵, NR⁵S(O)₂R⁵, NHC(O)OR⁵, NR⁵C(O)OR⁵, NHC(O)NH₂, NHC(O)NHR⁵, NHC(O)N(R⁵)₂, NR⁵C(O)NHR⁵, NR⁵C(O)N(R⁵)₂, C(O)NH₂, C(O)NHR⁵, C(O)N(R⁵)₂, C(O)NHOH, C(O)NHOR⁵, C(O)NHSO₂R⁵, C(O)NR⁵SO₂R⁵, SO₂NH₂, SO₂NHR⁵, SO₂N(R⁵)₂, C(O)H, C(O)OH, C(N)NH₂, C(N)NHR⁵, C(N)N(R⁵)₂, CNOH, CNOCH₃, OH, (O), CN, NO₂, CF₃, CF₂CF₃, OCF₃, OCF₂CF₃, F, Cl, Br and I;
each R⁴ is independently selected from the group consisting of C1-C6 alkyl, C2-C6 alkenyl, C₂-C₆ alkynyl, aryl, heteroaryl, heterocyclyl, cycloalkyl and cycloalkenyl; wherein C₁-C₆ alkyl, C₂-C₆ alkenyl, and C₂-C₆ alkynyl of each of R⁴ are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R⁴ are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I; and
each R⁵ is independently selected from the group consisting of C₁-C₆ alkyl, C₂-C₆ alkenyl, C₂-C₆ alkynyl, aryl, heteroaryl, heterocyclyl, cycloalkyl and cycloalkenyl; wherein C₁-C₆ alkyl, C₂-C₆ alkenyl, and C₂-C₆ alkynyl of each R⁵ are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R⁵ are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I;
n is 0, 1 or 2; and
m is 0 or 1).

As used herein, the term "alkyl" means a saturated straight chain or branched non-cyclic hydrocarbon, unless the context clearly dictates otherwise, having from 1 to 10 carbon atoms. "lower alkyl" means alkyl having from 1 to 4 carbon atoms. Representative saturated straight chain alkyls include -methyl, -ethyl, -n-propyl, -n-butyl, -n-pentyl, -n-hexyl, -n-heptyl, -n-octyl, -n-nonyl and -n-decyl; while saturated branched alkyls include -isopropyl, - sec-butyl, -isobutyl, -tert-butyl, -isopentyl, 2-methylbutyl, 3-methylbutyl, 2-methylpentyl, . 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like.

As used herein, if the term "C1-6", "C₁₋₆", or "C1-C6" is used, it means the number of carbon atoms is from 1 to 6. For example, C₁₋₆ alkyl means an alkyl which carbon number is any integer of from 1 to 6.

As used herein, the term "alkenyl" means a straight chain or branched non-cyclic hydrocarbon having from 2 to 10 carbon atoms and including at least one carbon-carbon double bond. Representative straight chain and branched (C₂-C₁₀)alkenyls include -vinyl, -allyl, -1-butenyl, -2-butenyl, -isobutylenyl, -1-pentenyl, -2-pentenyl, -3 -methyl- 1-butenyl, -2-methyl-2-butenyl, -2,3-dimethyl-2-butenyl, -1-hexenyl, -2-hexenyl, -3-hexenyl, -1-heptenyl, -2-heptenyl, - 3-heptenyl, -1-octenyl, -2-octenyl, -3-octenyl, -1-nonenyl, -2-nonenyl, -3-nonenyl, -1-decenyl, -2-decenyl, -3-decenyl and the like. An alkenyl group can be unsubstituted or substituted. A "cyclic alkylidene" is a ring having from 3 to 8 carbon atoms and including at least one carbon-carbon double bond, wherein the ring can have from 1 to 3 heteroatoms.

As used herein, the term "alkynyl" means a straight chain or branched non-cyclic hydrocarbon having from 2 to 10 carbon atoms and including at least one carbon-carbon triple bond. Representative straight chain and branched -(C₂-C₁₀)alkynyls include -acetylenyl, -propynyl, -1-butynyl, -2-butynyl, -1-pentynyl, -2-pentynyl, -3 -methyl- 1-butynyl, -4-pentynyl, -1-hexynyl, -2-hexynyl, -5-hexynyl, -1-heptynyl, -2-heptynyl, -6-heptynyl, -1-octynyl, -2-octynyl, -7- octynyl, -1-nonynyl, -2-nonynyl, -8-nonynyl, -1-decynyl, -2-decynyl, -9-decynyl, and the like. An alkynyl group can be unsubstituted or substituted.

The terms "halogen" and "halo" mean fluorine, chlorine, bromine or iodine.

As used herein, the term "haloalkyl", "haloalkoxy", "haloalkenyl" or "haloalkynyl" means an alkyl, alkoxy, alkenyl or alkynyl group, respectively, wherein one or more hydrogen atoms are substituted with halogen atoms. For example, the haloalkyl includes -CF3, -CHF2, -CH2F, -CBr3, -CHBr₂, -CH₂Br, -CCl₃, -CHCl₂, -CH₂CI, -CI₃, -CHI₂, -CH₂I, -CH₂-CF₃, -CH₂-CHF₂, -CH₂-CH₂F, -CH₂-CBr₃, -CH₂-CHBr₂, -CH₂-CH₂Br, -CH₂-CCl₃, -CH₂-CHCl₂, -CH₂-CH₂CI, -CH2-CI₃, -CH₂-CHI₂, -CH₂-CH₂I, and the like, wherein alkyl and halogen are as described above.

As used herein, the term "alkoxy" means an -O-(alkyl) group, wherein alkyl is defined above, including -OCH₃, -OCH₂CH₃, -O(CH₂)₂CH₃, -O(CH₂)₃CH₃, -O(CH₂)₄CH₃, -O(CH₂)₅CH₃, and the like. The term "lower alkoxy" means -O-(lower alkyl), wherein lower alkyl is as described above.

As used herein, the term "aryl" means a carbocyclic aromatic group containing from 5 to 10 ring atoms. Representative examples include, but are not limited to, phenyl, tolyl, xylyl, naphthyl, tetrahydronaphthyl, anthracenyl, fluorenyl, indenyl, and azulenyl. A carbocyclic aromatic group can be unsubstituted or optionally substituted.

As used herein, the term "cycloalkyl" means a monocyclic or polycyclic saturated ring having carbon and hydrogen atoms and having no carbon-carbon multiple bonds. Examples of cycloalkyl groups include, but are not limited to, (C₃-C₇)cycloalkyl groups, including cyclopropyl, cyclobutyl, cyclopentyl, cyclohexyl, and cycloheptyl. A cycloalkyl group can be unsubstituted or optinally substituted. In one embodiment, the cycloalkyl group is a monocyclic ring or bicyclic ring.

As used herein, the term "heteroaryl" means an aromatic heterocycle ring of 5- to 10 members and having at least one heteroatom selected from nitrogen, oxygen and sulfur, and containing at least 1 carbon atom, including both mono- and bicyclic ring systems. Representative heteroaryls are triazolyl, tetrazolyl, oxadiazolyl, pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, quinazolinyl, pyrimidyl, oxetanyl, azepinyl, piperazinyl, morpholinyl, dioxanyl, thietanyl and oxazolyl.

As used herein, the term "heterocycle" means a 5- to 7-membered monocyclic, or 7- to 10-membered bicyclic, heterocyclic ring which is either saturated, unsaturated, and which contains from 1 to 4 heteroatoms independently selected from nitrogen, oxygen and sulfur, and wherein the nitrogen and sulfur heteroatoms can be optionally oxidized, and the nitrogen heteroatom can be optionally quatemized, including bicyclic rings in which any of the above heterocycles are fused to a benzene ring. The heterocycle can be attached via any heteroatom or carbon atom. Heterocycles include heteroaryls as defined above. Representative heterocycles include morpholinyl, pyrrolidinonyl, pyrrolidinyl, piperidinyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, and tetrahydrothiopyranyl.

Furthermore, in another embodiment of the present invention,

The compound represented by chemical formula 1 may be any one selected from the group consisting of the following compounds.
(1) 2-(2,6-dioxopiperidin-3-yl)-4-(6-(4-(4-(5-(furan-2-ylmethylamino)-[1,2,4]triazolo[4,3-f]pyrimidin-8-yl)phenyl)piperazin-1-yl)hexylamino)isoindolin-1,3-dione;
(2) 2-(2,6-dioxopiperidin-3-yl)-4-(8-(4-(4-(5-(furan-2-ylmethylamino)-[1,2,4]triazolo[4,3-f]pyrimidin-8-yl)phenyl)piperazin-1-yl)octylamino)isoindolin-1,3-dione;
(3) 2-(2,6-dioxopiperidin-3-yl)-4-(6-(4-(4-(5-(furan-2-ylmethylamino)-[1,2,4]triazolo[4,3-f]pyrimidin-8-yl)phenyl)piperazin-1-yl)-6-oxohexylamino)isoindolin-1,3-dione;
(4) 2-(2,6-dioxopiperidin-3-yl)-4-(8-(4-(4-(5-(furan-2-ylmethylamino)-[1,2,4]triazolo[4,3-f]pyrimidin-8-yl)phenyl)piperazin-1-yl)-8-oxooctylamino)isoindolin-1,3-dione;
(5) 2-(2,6-dioxopiperidin-3-yl)-4-(9-(4-(4-(5-(furan-2-ylmethylamino)-[1,2,4]triazolo[4,3-f]pyrimidin-8-yl)phenyl)piperazin-1-yl)-9-oxononylamino)isoindolin-1,3-dione;
(6) 2-(2,6-dioxopiperidin-3-yl)-4-(10-(4-(4-(5-(furan-2-ylmethylamino)-[1,2,4]triazolo[4,3-f]pyrimidin-8-yl)phenyl)piperazin-1-yl)-10-oxodecylamino)isoindolin-1,3-dione;
(7) 2-(2,6-dioxopiperidin-3-yl)-4-(11-(4-(4-(5-(furan-2-ylmethylamino)-[1,2,4]triazolo[4,3-f]pyrimidin-8-yl)phenyl)piperazin-1-yl)-11-oxoundecylamino)isoindolin-1,3-dione;
(8) 2-(2,6-dioxopiperidin-3-yl)-4-(2-(2-(2-(4-(4-(5-(furan-2-ylmethylamino)-[1,2,4]triazolo[4,3-f]pyrimidin-8-yl)phenyl)piperazin-1-yl)-2-oxoethoxy)ethoxy)ethylamino)isoindolin-1,3-dione;
(9) 2-(2,6-dioxopiperidin-3-yl)-4-(2-(2-(3-(4-(4-(5-(furan-2-ylmethylamino)-[1,2,4]triazolo[4,3-f]pyrimidin-8-yl)phenyl)piperazin-1-yl)-3-oxopropoxy)ethoxy)ethylamino)isoindolin-1,3-dione;
(10) 2-(2,6-dioxopiperidin-3-yl)-4-(2-(2-(2-(2-(4-(4-(5-(furan-2-ylmethylamino)-[1,2,4]triazolo[4,3-f]pyrimidin-8-yl)phenyl)piperazin-1-yl)-2-oxoethoxy)ethoxy)ethoxy)ethylamino)isoindolin-1,3-dione;
(11) 2-(2,6-dioxopiperidin-3-yl)-4-(20-(4-(4-(5-(furan-2-ylmethylamino)-[1,2,4]triazolo[4,3-f]pyrimidin-8-yl)phenyl)piperazin-1-yl)-20-oxo-3,6,9,12,15,18-hexaoxicosylamino)isoindolin-1,3-dione;
(12) 2-(2-(2,6-dioxopiperidin-3-yl)-1,3-dioxoisoindolin-4-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide;
(13) 2-(2-(2,6-dioxopiperidin-3-yl)-1,3-dioxoisoindolin-4-yloxy)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide;
(14) 2-(2-(2,6-dioxopiperidin-3-yl)-1,3-dioxoisoindolin-4-ylamino)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide;
(15) 2-(2-(2,6-dioxopiperidin-3-yl)-1,3-dioxoisoindolin-4-yloxy)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide;
(16) 2-(2-(2,6-dioxopiperidin-3-yl)-1,3-dioxoisoindolin-4-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide:
(17) 2-(2-(2,6-dioxopiperidin-3-yl)-1,3-dioxoisoindolin-4-yloxy)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide;
(18) 2-(2-(2,6-dioxopiperidin-3-yl)-1,3-dioxoisoindolin-4-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide;
(19) 2-(2-(2,6-dioxopiperidin-3-yl)-1,3-dioxoisoindolin-4-yloxy)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide;
(20) 2-(2-(2,6-dioxopiperidin-3-yl)-1,3-dioxoisoindolin-4-ylamino)-N-(6-(4-(4-(5-(furan-2-ylmethylamino)-[1,2,4]triazolo[4,3-f]pyrimidin-8-yl)phenyl)piperazin-1-yl)hexyl)acetamide;
(21) 2-(2-(2,6-dioxopiperidin-3-yl)-1,3-dioxoisoindolin-4-ylamino)-N-(8-(4-(4-(5-(furan-2-ylmethylamino)-[1,2,4]triazolo[4,3-f]pyrimidin-8-yl)phenyl)piperazin-1-yl)octyl)acetamide;
(22) 2-(2,6-dioxopiperidin-3-yl)-5-(8-(4-(4-(5-(furan-2-ylmethylamino)-[1,2,4]triazolo[4,3-f]pyrimidin-8-yl)phenyl)piperazin-1-yl)-8-oxooctylamino)isoindolin-1,3-dione;
(23) 2-(2,6-dioxopiperidin-3-yl)-5-(10-(4-(4-(5-(furan-2-ylmethylamino)-[1,2,4]triazolo[4,3-f]pyrimidin-8-yl)phenyl)piperazin-1-yl)-10-oxodecylamino)isoindolin-1,3-dione;
(24) 2-(2-(2,6-dioxopiperidin-3-yl)-1,3-dioxoisoindolin-5-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide;
(25) 2-(2-(2,6-dioxopiperidin-3-yl)-1,3-dioxoisoindolin-5-ylamino)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide;
(26) 2-(2-(2,6-dioxopiperidin-3-yl)-1,3-dioxoisoindolin-5-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide;
(27) 2-(2-(2,6-dioxopiperidin-3-yl)-1,3-dioxoisoindolin-5-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide;
(28) 1-(2-(2,6-dioxopiperidin-3-yl)-1,3-dioxoisoindolin-4-yl)-N-(4-(4-(4-(5-(furan-2-ylmethylamino)-[1,2,4]triazolo[4,3-f]pyrimidin-8-yl)phenyl)piperazin-1-yl)-4-oxobutyl)azetidin-3-carboxamide;
(29) 1-(2-(2,6-dioxopiperidin-3-yl)-1,3-dioxoisoindolin-5-yl)-N-(4-(4-(4-(5-(furan-2-ylmethylamino)-[1,2,4]triazolo[4,3-f]pyrimidin-8-yl)phenyl)piperazin-1-yl)-4-oxobutyl)piperidin-4-carboxamide;
(30) 1-(2-(2,6-dioxopiperidin-3-yl)-1,3-dioxoisoindolin-5-yl)-N-(6-(4-(4-(5-(furan-2-ylmethylamino)-[1,2,4]triazolo[4,3-f]pyrimidin-8-yl)phenyl)piperazin-1-yl)-6-oxohexyl)piperidin-4-carboxamide;
(31) 1-(2-(2,6-dioxopiperidin-3-yl)-1,3-dioxoisoindolin-5-yl)-N-(8-(4-(4-(5-(furan-2-ylmethylamino)-[1,2,4]triazolo[4,3-f]pyrimidin-8-yl)phenyl)piperazin-1-yl)-8-oxooctyl)piperidin-4-carboxamide;
(32) N-(6-(2-(2,6-dioxopiperidin-3-yl)-1,3-dioxoisoindolin-4-ylamino)hexyl)-4-(5-(furan-2-ylmethylamino)-[1,2,4]triazolo[4,3-f]pyrimidin-8-yl)benzamide;
(33) N-(6-(2-(2-(2,6-dioxopiperidin-3-yl)-1,3-dioxoisoindolin-4-ylamino)acetamido)hexyl)-4-(5-(furan-2-ylmethylamino)-[1,2,4]triazolo[4,3-f]pyrimidin-8-yl)benzamide;
(34) N-(6-(2-(2-(2,6-dioxopiperidin-3-yl)-1,3-dioxoisoindolin-4-yloxy)acetamido)hexyl)-4-(5-(furan-2-ylmethylamino)-[1,2,4]triazolo[4,3-f]pyrimidin-8-yl)benzamide;
(35) 1-(2-(2,6-dioxopiperidin-3-yl)-1,3-dioxoisoindolin-5-yl)-N-(10-(4-(4-(5-(furan-2-ylmethylamino)-[1,2,4]triazolo[4,3-f]pyrimidin-8-yl)phenyl)piperazin-1-yl)-10-oxodecyl)piperidin-4-carboxamide;
(36) 1-(2-(2,6-dioxopiperidin-3-yl)-1,3-dioxoisoindolin-4-yl)-N-(10-(4-(4-(5-(furan-2-ylmethylamino)-[1,2,4]triazolo[4,3-f]pyrimidin-8-yl)phenyl)piperazin-1-yl)-10-oxodecyl)azetidin-3-carboxamide;
(37) N-(2-(2-(5-(2-(2,6-dioxopiperidin-3-yl)-1,3-dioxoisoindolin-4-ylamino)pentyloxy)ethoxy)ethyl)-4-(5-(furan-2-ylmethylamino)-[1,2,4]triazolo[4,3-f]pyrimidin-8-yl)benzamide;
(38) N-(5-(2-(2-(2-(2-(2,6-dioxopiperidin-3-yl)-1,3-dioxoisoindolin-4-ylamino)acetamido)ethoxy)ethoxy)pentyl)-4-(5-(furan-2-ylmethylamino)-[1,2,4]triazolo[4,3-f]pyrimidin-8-yl)benzamide;
(39) N-(5-(2-(2-(2-(2-(2,6-dioxopiperidin-3-yl)-1,3-dioxoisoindolin-4-yloxy)acetamido)ethoxy)ethoxy)pentyl)-4-(5-(furan-2-ylmethylamino)-[1,2,4]triazolo[4,3-f]pyrimidin-8-yl)benzamide;
(40) N-(8-(6-(2-(2,6-dioxopiperidin-3-yl)-1,3-dioxoisoindolin-4-ylamino)hexyloxy)octyl)-4-(5-(furan-2-ylmethylamino)-[1,2,4]triazolo[4,3-f]pyrimidin-8-yl)benzamide;
(41) N-(6-(2-(2-(2-(2-(2,6-dioxopiperidin-3-yl)-1,3-dioxoisoindolin-4-ylamino)ethoxy)ethoxy)ethoxy)hexyl)-4-(5-(furan-2-ylmethylamino)-[1,2,4]triazolo[4,3-f]pyrimidin-8-yl)benzamide;
(42) N-(1-(2-(2,6-dioxopiperidin-3-yl)-1,3-dioxoisoindolin-4-ylamino)-3,6,9,12,15,18-hexaoxadocosan-22-yl)-4-(5-(furan-2-ylmethylamino)-[1,2,4]triazolo[4,3-f]pyrimidin-8-yl)benzamide;
(43) N-(25-(2-(2,6-dioxopiperidin-3-yl)-1,3-dioxoisoindolin-4-ylamino)-24-oxo-3,6,9,12,15,18-hexaoxa-23-azapentacosyl)-4-(5-(furan-2-ylmethylamino)-[1,2,4]triazolo[4,3-f]pyrimidin-8-yl)benzamide;
(44) N-(25-(2-(2,6-dioxopiperidin-3-yl)-1,3-dioxoisoindolin-4-yloxy)-24-oxo-3,6,9,12,15,18-hexaoxa-23-azapentacosyl)-4-(5-(furan-2-ylmethylamino)-[1,2,4]triazolo[4,3-f]pyrimidin-8-yl)benzamide;
(45) N-(1-(2-(2,6-dioxopiperidin-3-yl)-1,3-dioxoisoindolin-4-ylamino)-3,6,9,12,15,18,21-heptaoxapentacosan-25-yl)-4-(5-(furan-2-ylmethylamino)-[1,2,4]triazolo[4,3-f]pyrimidin-8-yl)benzamide;
(46) N-(28-(2-(2,6-dioxopiperidin-3-yl)-1,3-dioxoisoindolin-4-ylamino)-27-oxo-3,6,9,12,15,18,21-heptaoxa-26-azaoctacosyl)-4-(5-(furan-2-ylmethylamino)-[1,2,4]triazolo[4,3-f]pyrimidin-8-yl)benzamide;
(47) N-(28-(2-(2,6-dioxopiperidin-3-yl)-1,3-dioxoisoindolin-4-yloxy)-27-oxo-3,6,9,12,15,18,21-heptaoxa-26-azaoctacosyl)-4-(5-(furan-2-ylmethylamino)-[1,2,4]triazolo[4,3-f]pyrimidin-8-yl)benzamide;
(48) N-(1-(2-(2,6-dioxopiperidin-3-yl)-1,3-dioxoisoindolin-4-ylamino)-3,6,9,12,15,18,21,24-octaoxaoctacosan-28-yl)-4-(5-(furan-2-ylmethylamino)-[1,2,4]triazolo[4,3-f]pyrimidin-8-yl)benzamide;
(49) N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-4-yloxy)acetamide;
(50) N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-4-ylamino)acetamide;
(51) N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-4-yl)azetidin-3-carboxamide;
(52) N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-4-yl)piperidin-4-carboxamide;
(53) N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-5-ylamino)acetamide;
(54) N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-5-yl)piperidin-4-carboxamide;
(55) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-(2-(2,6-dioxopiperidin-3-yl)-1,3-dioxoisoindolin-4-ylamino)acetamide;
(56) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-(2-(2,6-dioxopiperidin-3-yl)-1,3-dioxoisoindolin-4-yloxy)acetamide;
(57) N-(6-(4-(4-((3S,4R)-4-(dimethylamino)-1-(2-fluoro-6-methylbenzyl)pyrrolidin-3-yl)phenyl)piperazin-1-yl)hexyl)-2-(2-(2,6-dioxopiperidin-3-yl)-1,3-dioxoisoindolin-4-ylamino)acetamide;
(58) N-(6-(4-(4-((3S,4R)-4-(dimethylamino)-1-(2-fluoro-6-methylbenzyl)pyrrolidin-3-yl)phenyl)piperazin-1-yl)hexyl)-2-(2-(2,6-dioxopiperidin-3-yl)-1,3-dioxoisoindolin-4-yloxy)acetamide;
(59) N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-4-yl)azetidin-3-carboxamide;
(60) N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-4-yl)piperidin-4-carboxamide;
(61) N-(6-(4-(4-((3S,4R)-4-(dimethylamino)-1-(2-fluoro-6-methylbenzyl)pyrrolidin-3-yl)phenyl)piperazin-1-yl)hexyl)-2-((2-(2,6-dioxopiperidin-3-yl)-1,3-dioxoisoindolin-5-yl)amino)acetamide;
(62) N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-5-yl)piperidin-4-carboxamide;
(63) N-(8-(4-(4-((3S,4R)-4-(dimethylamino)-1-(2-fluoro-6-methylbenzyl)pyrrolidin-3-yl)phenyl)piperazin-1-yl)octyl)-1-(2-(2,6-dioxopiperidin-3-yl)-1,3-dioxoisoindolin-5-yl)piperidin-4-carboxamide;
(64) N-(8-(4-(4-((3S,4R)-4-(dimethylamino)-1-(2-fluoro-6-methylbenzyl)pyrrolidin-3-yl)phenyl)piperazin-1-yl)octyl)-2-((2-(2,6-dioxopiperidin-3-yl)-1,3-dioxoisoindolin-5-yl)amino)acetamide;
(65) 4-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethoxy)-2-(2,6-dioxopiperidin-3-yl)isoindolin-1,3-dione;
(66) 4-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-2-(2,6-dioxopiperidin-3-yl)isoindolin-1,3-dione;
(67) 3-(6-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-4-oxobenzo[d][1,2,3]triazin-3(4H)-yl)piperidin-2,6-dione;
(68) 3-(6-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethoxy)-4-oxobenzo[d][1,2,3]triazin-3(4H)-yl)piperidin-2,6-dione;
(69) 3-(6-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-4-oxobenzo[d][1,2,3]triazin-3(4H)-yl)piperidin-2,6-dione;
(70) 3-(5-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-4-oxobenzo[d][1,2,3]triazin-3(4H)-yl)piperidin-2,6-dione;
(71) 3-(6-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-4-oxobenzo[d][1,2,3]triazin-3(4H)-yl)piperidin-2,6-dione;
(72) 3-(6-(2-(4-((4-(4-(5-(furan-2-ylmethylamino)-[1,2,4]triazolo[4,3-f]pyrimidin-8-yl)phenyl)piperazin-1-yl)methyl)piperidin-1-yl)-2-oxoethylamino)-4-oxobenzo[d][1,2,3]triazin-3(4H)-yl)piperidin-2,6-dione;
(73) 2-(3-(2,6-dioxopiperidin-3-yl)-4-oxo-3,4-dihydrobenzo[d][1,2,3]triazin-6-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide;
(74) 1-(3-(2,6-dioxopiperidin-3-yl)-4-oxo-3,4-dihydrobenzo[d][1,2,3]triazin-6-yl)-N-(4-(4-(4-(5-(furan-2-ylmethylamino)-[1,2,4]triazolo[4,3-f]pyrimidin-8-yl)phenyl)piperazin-1-yl)-4-oxobutyl)piperidin-4-carboxamide;
(75) 3-(5-(6-(4-(4-(5-(furan-2-ylmethylamino)-[1,2,4]triazolo[4,3-f]pyrimidin-8-yl)phenyl)piperazin-1-yl)-6-oxohexylamino)-4-oxobenzo[d][1,2,3]triazin-3(4H)-yl)piperidin-2,6-dione;
(76) 3-(6-(6-(4-(4-(5-(furan-2-ylmethylamino)-[1,2,4]triazolo[4,3-f]pyrimidin-8-yl)phenyl)piperazin-1-yl)-6-oxohexylamino)-4-oxobenzo[d][1,2,3]triazin-3(4H)-yl)piperidin-2,6-dione;
(77) 3-(5-(8-(4-(4-(5-(furan-2-ylmethylamino)-[1,2,4]triazolo[4,3-f]pyrimidin-8-yl)phenyl)piperazin-1-yl)-8-oxooctylamino)-4-oxobenzo[d][1,2,3]triazin-3(4H)-yl)piperidin-2,6-dione;
(78) 3-(6-(8-(4-(4-(5-(furan-2-ylmethylamino)-[1,2,4]triazolo[4,3-f]pyrimidin-8-yl)phenyl)piperazin-1-yl)-8-oxooctylamino)-4-oxobenzo[d][1,2,3]triazin-3(4H)-yl)piperidin-2,6-dione;
(79) 2-(3-(2,6-dioxopiperidin-3-yl)-4-oxo-3,4-dihydrobenzo[d][1,2,3]triazin-6-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide;
(80) 1-(3-(2,6-dioxopiperidin-3-yl)-4-oxo-3,4-dihydrobenzo[d][1,2,3]triazin-6-yl)-N-(8-(4-(4-(5-(furan-2-ylmethylamino)-[1,2,4]triazolo[4,3-f]pyrimidin-8-yl)phenyl)piperazin-1-yl)-8-oxooctyl)piperidin-4-carboxamide;
(81) 3-(5-(10-(4-(4-(5-(furan-2-ylmethylamino)-[1,2,4]triazolo[4,3-f]pyrimidin-8-yl)phenyl)piperazin-1-yl)-10-oxodecylamino)-4-oxobenzo[d][1,2,3]triazin-3(4H)-yl)piperidin-2,6-dione;
(82) 3-(6-(10-(4-(4-(5-(furan-2-ylmethylamino)-[1,2,4]triazolo[4,3-f]pyrimidin-8-yl)phenyl)piperazin-1-yl)-10-oxodecylamino)-4-oxobenzo[d][1,2,3]triazin-3(4H)-yl)piperidin-2,6-dione;
(83) 2-(3-(2,6-dioxopiperidin-3-yl)-4-oxo-3,4-dihydrobenzo[d][1,2,3]triazin-6-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide;
(84) N-(4-(4-(4-((3S,4R)-4-(dimethylamino)-1-(2-fluoro-6-methylbenzyl)pyrrolidin-3-yl)phenyl)piperazin-1-yl)butyl)-2-(3-(2,6-dioxopiperidin-3-yl)-4-oxo-3,4-dihydrobenzo[d][1,2,3]triazin-6-ylamino)acetamide;
(85) N-(4-(4-(4-((3S,4R)-4-(dimethylamino)-1-(2-fluoro-6-methylbenzyl)pyrrolidin-3-yl)phenyl)piperazin-1-yl)butyl)-1-(3-(2,6-dioxopiperidin-3-yl)-4-oxo-3,4-dihydrobenzo[d][1,2,3]triazin-6-yl)azetidin-3-carboxamide;
(86) N-(4-(4-(4-((3S,4R)-4-(dimethylamino)-1-(2-fluoro-6-methylbenzyl)pyrrolidin-3-yl)phenyl)piperazin-1-yl)butyl)-1-(3-(2,6-dioxopiperidin-3-yl)-4-oxo-3,4-dihydrobenzo[d][1,2,3]triazin-6-yl)piperidin-4-carboxamide;
(87) N-(6-(4-(4-((3S,4R)-4-(dimethylamino)-1-(2-fluoro-6-methylbenzyl)pyrrolidin-3-yl)phenyl)piperazin-1-yl)hexyl)-2-(3-(2,6-dioxopiperidin-3-yl)-4-oxo-3,4-dihydrobenzo[d][1,2,3]triazin-6-ylamino)acetamide;
(88) N-(6-(4-(4-((3S,4R)-4-(dimethylamino)-1-(2-fluoro-6-methylbenzyl)pyrrolidin-3-yl)phenyl)piperazin-1-yl)hexyl)-1-(3-(2,6-dioxopiperidin-3-yl)-4-oxo-3,4-dihydrobenzo[d][1,2,3]triazin-6-yl)piperidin-4-carboxamide;
(89) N-(6-(4-(4-((3S,4R)-4-(dimethylamino)-1-(2-fluoro-6-methylbenzyl)pyrrolidin-3-yl)phenyl)piperazin-1-yl)hexyl)-1-(3-(2,6-dioxopiperidin-3-yl)-4-oxo-3,4-dihydrobenzo[d][1,2,3]triazin-6-yl)azetidin-3-carboxamide;
(90) N-(8-(4-(4-((3S,4R)-4-(dimethylamino)-1-(2-fluoro-6-methylbenzyl)pyrrolidin-3-yl)phenyl)piperazin-1-yl)octyl)-2-(3-(2,6-dioxopiperidin-3-yl)-4-oxo-3,4-dihydrobenzo[d][1,2,3]triazin-6-ylamino)acetamide;
(91) N-(8-(4-(4-((3S,4R)-4-(dimethylamino)-1-(2-fluoro-6-methylbenzyl)pyrrolidin-3-yl)phenyl)piperazin-1-yl)octyl)-1-(3-(2,6-dioxopiperidin-3-yl)-4-oxo-3,4-dihydrobenzo[d][1,2,3]triazin-6-yl)azetidin-3-carboxamide;
(92) N-(8-(4-(4-((3S,4R)-4-(dimethylamino)-1-(2-fluoro-6-methylbenzyl)pyrrolidin-3-yl)phenyl)piperazin-1-yl)octyl)-1-(3-(2,6-dioxopiperidin-3-yl)-4-oxo-3,4-dihydrobenzo[d][1,2,3]triazin-6-yl)piperidin-4-carboxamide;
(93) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-4-yl)oxy)acetamide;
(94) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-4-yl)amino)acetamide;
(95) (2S,4R)-1-((S)-2-(2-(3-(4-(4-(5-(furan-2-ylmethylamino)-[1,2,4]triazolo[4,3-f]pyrimidin-8-yl)phenyl)piperazin-l-yl)propoxy)acetamido)-3,3-dimethylbutanoyl)-4-hydroxy-N-(4-(4-methylthiazol-5-yl)benzyl)pyrrolidin-2-carboxamide;
(96) (2S,4R)-1-((S)-2-(2-(4-(4-(4-(5-(furan-2-ylmethylamino)-[1,2,4]triazolo[4,3-f]pyrimidin-8-yl)phenyl)piperazin-1-yl)butoxy)acetamido)-3,3-dimethylbutanoyl)-4-hydroxy-N-(4-(4-methylthiazol-5-yl)benzyl)pyrrolidin-2-carboxamide;
(97) (2S,4R)-1-((S)-2-(2-(2-(2-(4-(4-(5-(furan-2-ylmethylamino)-[1,2,4]triazolo[4,3-f]pyrimidin-8-yl)phenyl)piperazin-1-yl)ethoxy)ethoxy)acetamido)-3,3-dimethylbutanoyl)-4-hydroxy-N-(4-(4-methylthiazol-5-yl)benzyl)pyrrolidin-2-carboxamide;
(98) (2S,4R)-1-((S)-2-(6-(4-(4-(5-(furan-2-ylmethylamino)-[1,2,4]triazolo[4,3-f]pyrimidin-8-yl)phenyl)piperazin-1-yl)-6-oxohexanamido)-3,3-dimethylbutanoyl)-4-hydroxy-N-(4-(4-methylthiazol-5-yl)benzyl)pyrrolidin-2-carboxamide;
(99) (2S,4R)-1-((S)-2-(8-(4-(4-(5-(furan-2-ylmethylamino)-[1,2,4]triazolo[4,3-f]pyrimidin-8-yl)phenyl)piperazin-1-yl)-8-oxooctanamido)-3,3-dimethylbutanoyl)-4-hydroxy-N-(4-(4-methylthiazol-5-yl)benzyl)pyrrolidin-2-carboxamide;
(100) (2S,4R)-1-((S)-2-tert-butyl-23-(4-(4-(5-(furan-2-ylmethylamino)-[1,2,4]triazolo[4,3-f]pyrimidin-8-yl)phenyl)piperazin-1-yl)-4,23-dioxo-6,9,12,15,18,21-hexaoxa-3-azatricosan-1-oyl)-4-hydroxy-N-(4-(4-methylthiazol-5-yl)benzyl)pyrrolidin-2-carboxamide;
(101) (2S,4R)-1-((S)-2-(8-(3-(2-(4-(4-(5-(furan-2-ylmethylamino)-[1,2,4]triazolo[4,3-f]pyrimidin-8-yl)phenyl)piperazin-1-yl)-2-oxoethyl)phenoxy)octanamido)-3,3-dimethylbutanoyl)-4-hydroxy-N-(4-(4-methylthiazol-5-yl)benzyl)pyrrolidin-2-carboxamide;
(102) (2S,4R)-1-((S)-2-(2-(2-(2-(3-(2-(4-(4-(5-(furan-2-ylmethylamino)-[1,2,4]triazolo[4,3-f]pyrimidin-8-yl)phenyl)piperazin-1-yl)-2-oxoethyl)phenoxy)ethoxy)ethoxy)acetamido)-3,3-dimethylbutanoyl)-4-hydroxy-N-(4-(4-methylthiazol-5-yl)benzyl)pyrrolidin-2-carboxamide;
(103) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-5-yl)amino)acetamide;
(104) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((3-(2,6-dioxopiperidin-3-yl)-4-oxo-3,4-dihydrobenzo[d][1,2,3]triazin-6-yl)amino)acetamide;
(105) N-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)ethyl)-2-((2-(2,6-dioxopiperidin-3-yl)-1,3-dioxoisoindolin-5-yl)amino)acetamide;
(106) N-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)ethyl)-2-((3-(2,6-dioxopiperidin-3-yl)-4-oxo-3,4-dihydrobenzo[d][1,2,3]triazin-6-yl)amino)acetamide;
(107) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-5-yl)amino)acetamide;
(108) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((3-(2,6-dioxopiperidin-3-yl)-4-oxo-3,4-dihydrobenzo[d][1,2,3]triazin-6-yl)amino)acetamide;
(109) (2S,4R)-1-((R)-2-(2-(4-(4-(4-((3S,4R)-4-(dimethylamino)-1-(2-fluoro-6-methylbenzyl)pyrrolidin-3-yl)phenyl)piperazin-1-yl)butoxy)acetamido)-3,3-dimethylbutanoyl)-4-hydroxy-N-(4-(4-methylthiazol-5-yl)benzyl)pyrrolidin-2-carboxamide(carboxamide);
(110) (2S,4R)-1-((R)-2-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)acetamido)-3,3-dimethylbutanoyl)-4-hydroxy-N-(4-(4-methylthiazol-5-yl)benzyl)pyrrolidin-2-carboxamide;
(111) (2S,4R)-1-((R)-2-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)acetamido )-3,3-dimethylbutanoyl)-4-hydroxy-N-(4-(4-methylthiazol-5-yl)benzyl)pyrrolidin-2-carboxamide;
(112) 5-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethoxy)-2-(2,6-dioxopiperidin-3-yl)isoindolin-1,3-dione;
(113) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-5-yl)oxy)acetamide;
(114) N-(6-(4-(4-((3S,4R)-4-(dimethylamino)-1-(2-fluoro-6-methylbenzyl)pyrrolidin-3-yl)phenyl)piperazin-1-yl)hexyl)-2-((2-(2,6-dioxopiperidin-3-yl)-1,3-dioxoisoindolin-5-yl)oxy)acetamide;
(115) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-4-yl)oxy)acetamide;
(116) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-4-yl)amino)acetamide;
(117) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-5-yl)oxy)acetamide;
(118) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-5-yl)oxy)acetamide;
(119) N-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)ethyl)-2-((2-(2,6-dioxopiperidin-3-yl)-1,3-dioxoisoindolin-4-yl)amino)acetamide;
(120) N-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)ethyl)-2-((2-(2,6-dioxopiperidin-3-yl)-1,3-dioxoisoindolin-5-yl)oxy)acetamide;
(121) N-(2-(2-(2-(4-(4-((3S,4R)-4-(dimethylamino)-1-(2-fluoro-6-methylbenzyl)pyrrolidin-3-yl)phenyl)piperazin-1-yl)-2-oxoethoxy)ethoxy)ethyl)-2-((2-(2,6-dioxopiperidin-3-yl)-1,3-dioxoisoindolin-5-yl)amino)acetamide;
(122) N-(2-(2-(2-(2-(4-(4-((3S,4R)-4-(dimethylamino)-1-(2-fluoro-6-methylbenzyl)pyrrolidin-3-yl)phenyl)piperazin-1-yl)-2-oxoethoxy)ethoxy)ethoxy)ethyl)-2-((2-(2,6-dioxopiperidin-3-yl)-1,3-dioxoisoindolin-5-yl)amino)acetamide;
(123) 4-((4-(4-(4-((3S,4R)-4-(dimethylamino)-1-(2-fluoro-6-methylbenzyl)pyrrolidin-3-yl)phenyl)piperazin-1-yl)-4-oxobutyl)amino)-2-(2,6-dioxopiperidin-3-yl)isoindolin-1,3-dione;
(124) 5-((4-(4-(4-((3S,4R)-4-(dimethylamino)-1-(2-fluoro-6-methylbenzyl)pyrrolidin-3-yl)phenyl)piperazin-1-yl)-4-oxobutyl)amino)-2-(2,6-dioxopiperidin-3-yl)isoindolin-1,3-dione;
(125) 4-((8-(4-(4-((3S,4R)-4-(dimethylamino)-1-(2-fluoro-6-methylbenzyl)pyrrolidin-3-yl)phenyl)piperazin-1-yl)-8-oxooctyl)amino)-2-(2,6-dioxopiperidin-3-yl)isoindolin-1,3-dione;
(126) 5-((8-(4-(4-((3S,4R)-4-(dimethylamino)-1-(2-fluoro-6-methylbenzyl)pyrrolidin-3-yl)phenyl)piperazin-1-yl)-8-oxooctyl)amino)-2-(2,6-dioxopiperidin-3-yl)isoindolin-1,3-dione;
(127) 2-(2,6-dioxopiperidin-3-yl)-5-((2-(4-((4-(4-(5-((furan-2-ylmethyl)amino)-[1,2,4]triazolo[4,3-c]pyrimidin-8-yl)-1H-pyrazol-1-yl)piperidin-1-yl)methyl)piperidin-1-yl)-2-oxoethyl)amino)isomdolin-1,3-dione;
(128) 3-(6-((2-(4-((4-(4-(5-((furan-2-ylmethyl)amino)-[1,2,4]triazolo[4,3-c]pyrimidin-8-yl)-1H-pyrazol-1-yl)piperidin-1-yl)methyl)piperidin-1-yl)-2-oxoethyl)amino)-4-oxobenzo[d][1,2,3]triazin-3(4H)-yl)piperidin-2,6-dione;
(129) 2-((2-(2,6-dioxopiperidin-3-yl)-1,3-dioxoisoindolin-5-yl)amino)-N-(2-(2-(2-(4-(4-(5-((furan-2-ylmethyl)amino)-[1,2,4]triazolo[4,3-c]pyrimidin-8-yl)-1H-pyrazol-1-yl)piperidin-1-yl)ethoxy)ethoxy)ethyl)acetamide;
(130) 2-((3-(2,6-dioxopiperidin-3-yl)-4-oxo-3,4-dihydrobenzo[d][1,2,3]triazin-6-yl)amino)-N-(2-(2-(2-(4-(4-(5-((furan-2-ylmethyl)amino)-[1,2,4]triazolo[4,3-c]pyrimidin-8-yl)-1H-pyrazol-1-yl)piperidin-1-yl)ethoxy)ethoxy)ethyl)acetamide;
(131) 5-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-(2,6-dioxopiperidin-3-yl)isoindolin-1,3-dione;
(132) 5-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-(2,6-dioxopiperidin-3-yl)isoindolin-1,3-dione;
(133) 5-(4-(3-(4-(4-((3S,4S)-4-(dimethylamino)-1-(2-fluoro-6-methylbenzyl)pyrrolidin-3-yl)phenyl)piperazin-1-yl)-3-oxopropyl)piperidin-1-yl)-2-(2,6-dioxopiperidin-3-yl)isoindolin-1,3-dione;
(134) 5-(4-((4-((4-(4-((3S,4R)-4-(dimethylamino)-1-(2-fluoro-6-methylbenzyl)pyrrolidin-3-yl)phenyl)piperazin-1-yl)methyl)piperidin-1-yl)methyl)piperidin-1-yl)-2-(2,6-dioxopiperidin-3-yl)isoindolin-1,3-dione;
(135) N-(2-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)ethoxy)ethyl)-2-((2-(2,6-dioxopiperidin-3-yl)-1,3-dioxoisoindolin-5-yl)amino)acetamide;
(136) 5-((2-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-oxoethyl)amino)-2-(2,6-dioxopiperidin-3-yl)isoindolin-1,3-dione;
(137) 4-((6-(4-(4-((3S,4R)-4-(dimethylamino)-1-(2-fluoro-6-methylbenzyl)pyrrolidin-3-yl)phenyl)piperazin-1-yl)-6-oxohexyl)amino)-2-(2,6-dioxopiperidin-3-yl)isoindolin-1,3-dione;
(138) 5-((6-(4-(4-((3S,4R)-4-(dimethylamino)-1-(2-fluoro-6-methylbenzyl)pyrrolidin-3-yl)phenyl)piperazin-1-yl)-6-oxohexyl)amino)-2-(2,6-dioxopiperidin-3-yl)isoindolin-1,3-dione;
(139) 4-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-(2,6-dioxopiperidin-3-yl)isoindolin-1,3-dione;
(140) 5-((2-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-oxoethyl)amino)-2-(2,6-dioxopiperidin-3-yl)isoindolin-1,3-dione;
(141) 4-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-(2,6-dioxopiperidin-3-yl)isoindolin-1,3-dione;
(142) 4-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-(2,6-dioxopiperidin-3-yl)isoindolin-1,3-dione;
(143) 4-(2-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-oxoethoxy)-2-(2,6-dioxopiperidin-3-yl)isoindolin-1,3-dione;
(144) 4-((2-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-oxoethyl)amino)-2-(2,6-dioxopiperidin-3-yl)isoindolin-1,3-dione;
(145) 5-(2-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-oxoethoxy)-2-(2,6-dioxopiperidin-3-yl)isoindolin-1,3-dione;
(146) 5-((2-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-oxoethyl)amino)-2-(2,6-dioxopiperidin-3-yl)isoindolin-1,3-dione;
(147) 5-(2-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-oxoethoxy)-2-(2,6-dioxopiperidin-3-yl)isoindolin-1,3-dione;
(148) 4-(2-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-oxoethoxy)-2-(2,6-dioxopiperidin-3-yl)isoindolin-1,3-dione;
(149) 4-((2-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-oxoethyl)amino)-2-(2,6-dioxopiperidin-3-yl)isoindolin-1,3-dione;
(150) 5-(2-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-oxoethoxy)-2-(2,6-dioxopiperidin-3-yl)isoindolin-1,3-dione;
(151) 4-(2-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-oxoethoxy)-2-(2,6-dioxopiperidin-3-yl)isoindolin-1,3-dione;
(152) 4-((2-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-oxoethyl)amino)-2-(2,6-dioxopiperdin-3-yl)isoindolin-1,3-dione;
(153) 4-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-(2,6-dioxopiperidin-3-yl)isoindolin-1,3-dione;
(154) 5-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-(2,6-dioxopiperidin-3-yl)isoindolin-1,3-dione;
(155) 5-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-2-(2,6-dioxopiperidin-3-yl)isoindolin-1,3-dione;
(156) 5-(2-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-2-oxoethoxy)-2-(2,6-dioxopiperidin-3-yl)isoindolin-1,3-dione;
(157) 5-((2-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-2-oxoethyl)amino)-2-(2,6-dioxopiperidin-3-yl)isoindolin-1,3-dione;
(158) 5-(4-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-carbonyl)piperidin-1-yl)-2-(2,6-dioxopiperidin-3-yl)isoindolin-1,3-dione;
(159) 5-((8-(4-(4-((3S,4R)-4-(dimethylamino)-1-(7-fluoro-2,3-dihydro-1H-inden-1-yl)pyrrolidin-3-yl)phenyl)piperazin-1-yl)-8-oxooctyl)amino)-2-(2,6-dioxopiperidin-3-yl)isoindolin-1,3-dione;
(160) N-(8-(4-(4-((3S,4R)-4-(dimethylamino)-1-(7-fluoro-2,3-dihydro-1H-inden-1-yl)pyrroliin-3-yl)phenyl)piperazin-1-yl)-8-oxooctyl)-2-((2-(2,6-dioxopiperidin-3-yl)-1,3-dioxoisoindolin-5-yl)oxy)acetamide;
(161) N-(8-(4-(4-((3S,4R)-4-(dimethylamino)-1-(7-fluoro-2,3-dihydro-1H-mden-1-yl)pyrrolidin-3-yl)phenyl)piperazin-1-yl)-8-oxooctyl)-2-((2-(2,6-dioxopiperidin-3-yl)-1,3-dioxoisoindolin-5-yl)amino)acetamide;
(162) N-(8-(4-(4-((3S,4R)-4-(dimethylamino)-1-(7-fluoro-2,3-dihydro-1H-inden-1-yl)pyrrolidin-3-yl)phenyl)piperazin-1-yl)-8-oxooctyl)-1-(2-(2,6-dioxopiperidin-3-yl)-1,3-dioxoisoindolin-5-yl)piperidin-4-carboxamide;
(163) 5-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-2-(2,6-dioxopiperidin-3-yl)isoindolin-1,3-dione;
(164) 5-(2-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-2-oxoethoxy)-2-(2,6-dioxopiperidin-3-yl)isoindolin-1,3-dione;
(165) 5-((2-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-2-oxoethyl)amino)-2-(2,6-dioxopiperidin-3-yl)isoindolin-1,3-dione;
(166) 5-(4-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-carbonyl)piperidin-1-yl)-2-(2,6-dioxopiperidin-3-yl)isoindolin-1,3-dione;
(167) 5-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)-2-oxoethyl)piperidin-1-yl)-2-(2,6-dioxopiperidin-3-yl)isoindolin-1,3-dione;
(168) 5-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-2-(2,6-dioxopiperidin-3-yl)isoindolin-1,3-dione;
(169) 5-(2-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-2-oxoethoxy)-2-(2,6-dioxopiperidin-3-yl)isoindolin-1,3-dione;
(170) 5-((2-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-2-oxoethyl)amino)-2-(2,6-dioxopiperidin-3-yl)isoindolin-1,3-dione;
(171) 5-(4-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-carbonyl)piperidin-1-yl)-2-(2,6-dioxopiperidin-3-yl)isoindolin-1,3-dione;
(172) 3-(6-((8-(4-(4-((3S,4R)-4-(dimethylamino)-1-(7-fluoro-2,3-dihydro-1H-inden-1-yl)pyrrolidin-3-yl)phenyl)piperazin-1-yl)-8-oxooctyl)amino)-4-oxobenzo[d][1,2,3]triazin-3(4H)-yl)piperidin-2,6-dione;
(173) 3-(6-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-4-oxobenzo[d][1,2,3]triazin-3(4H)-yl)piperidin-2,6-dione;
(174) 3-(6-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)-2-oxoethyl)piperidin-1-yl)-4-oxobenzo[d] [1,2,3]triazin-3(4H)-yl)piperidin-2,6-dione;
(175) 3-(6-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-4-oxobenzo[d][1,2,3]triazin-3(4H)-yl)piperidin-2,6-dione;
(176) 3-(6-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-4-oxobenzo[d] [1,2,3]triazin-3(4H)-yl)piperidin-2,6-dione;
(177) 3-(6-((2-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-2-oxoethyl)amino)-4-oxobenzo[d] [1,2,3]triazin-3(4H)-yl)piperidin-2,6-dione;
(178) 3-(6-((2-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-2-oxoethyl)amino)-4-oxobenzo[d][1,2,3]triazin-3(4H)-yl)piperidin-2,6-dione;
(179) 3-(6-((2-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-2-oxoethyl)amino)-4-oxobenzo[d][1,2,3]triazin-3(4H)-yl)piperidin-2,6-dione; and
(180) 3-(6-(2-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-2-oxoethoxy)-4-oxobenzo[d] [1,2,3]triazin-3(4H)-yl)piperidin-2,6-dione.

The compound represented by chemical formula 1 of the present invention can be used in the form of a pharmaceutically acceptable salt, and as the salt, an acid addition salt formed by a pharmaceutically acceptable free acid is useful. Acid addition salts are obtained from inorganic acids such as hydrochloric acid, nitric acid, phosphoric acid, sulfuric acid, hydrobromic acid, hydroiodic acid, nitrous acid, phosphorous acid, aliphatic mono and dicarboxylates, phenyl-substituted alkanoates, hydroxy alkanoates and alkanoates, non-toxic organic acids such as aromatic acids, aliphatic and aromatic sulfonic acids, and organic acids such as acetic acid, benzoic acid, citric acid, lactic acid, maleic acid, gluconic acid, methanesulfonic acid, 4-toluenesulfonic acid, tartaric acid, fumaric acid and the like. Examples of such pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitro benzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate, mandelate, and the like.

The acid addition salt according to the present invention can be prepared by a conventional method, for example, by filtering and drying the resulting precipitate formed by dissolving a derivative of the chemical formula 1 compound in an organic solvent such as methanol, ethanol, acetone, dichloromethane, acetonitrile, etc. and adding an organic or inorganic acid, or by distilling the solvent and excess acid under reduced pressure followed by drying and crystallizing in an organic solvent.

In addition, pharmaceutically acceptable metal salts can be prepared with bases. The alkali metal or alkaline earth metal salt is obtained, for example, by dissolving the compound in an excess alkali metal hydroxide or alkaline earth metal hydroxide solution, filtering the undissolved compound salt, and evaporating and drying the filtrate. In this case, it is pharmaceutically suitable to prepare a sodium, potassium or calcium salt as the metal salt. In addition, the corresponding salt is obtained by reacting an alkali metal or alkaline earth metal salt with a suitable negative salt (e.g., silver nitrate).

Furthermore, the present invention includes not only the compound represented by chemical formula 1 and pharmaceutically acceptable salts thereof, but also polymorphs, solvates, optical isomers, hydrates, and the like that can be prepared therefrom.

And, even if the term "compound(s) of the present invention" does not mention its pharmaceutically acceptable sat, the term includes salts thereof. In one embodiment, the compounds of this present invention include stereo-chemically pure compounds, e.g., those substantially free (e.g., greater than 85% ee, greater than 90% ee, greater than 95% ee, greater than 97% ee, or greater than 99% ee) of other stereoisomers. That is, if the compounds of chemical formula 1 according to the present invention or salts thereof are tautomeric isomers and/or stereoisomers (e.g., geometrical isomers and conformational isomers), such isolated isomers and their mixtures also are included in the scope of this invention. If the compounds of the present invention or salts thereof have an asymmetric carbon in their structures, their active optical isomers and their racemic mixtures also are included in the scope of this invention.

As used herein, the term "polymorph" refers to solid crystalline forms of a compound of this invention or complex thereof. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

As used herein, the term "solvate" means a compound or its salt according to this invention that further includes a stoichiometric or non-stoichiometric amount of a solvent bound by non-covalent intermolecular forces. Preferred solvents are volatile, non-toxic, and/or acceptable for administration to humans in trace amounts.

As used herein, the term "hydrate" means a compound or its salt according to this invention that further includes a stoichiometric or non-stoichiometric amount of water bound by non-covalent intermolecular forces.

Also, in another aspect of the present invention,
as shown in Scheme 1 below,
a method for preparing a compound represented by claim 1 chemical formula 1 is provided, comprising
preparing a compound represented by the chemical formula ULB-L-H from the compound represented by ULB-X; and
preparing a compound of ULB-L-PTM represented by chemical formula 1 from the compound represented by ULB-L-H prepared above: 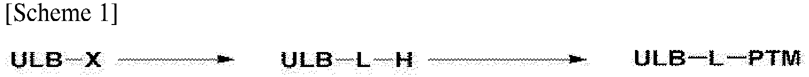
(In Scheme 1,
ULB, L, and PTM are as defined in claim 1, and
X is H or F).

Hereinafter, a method for preparing the compound represented by chemical formula 1 according to the present invention will be described in detail step by step.

In the method for preparing a compound represented by chemical formula 1 according to the present invention, step 1 is a step of preparing a compound represented by the chemical formula ULB-L-H from the compound represented by ULB-X.

In this case, when L of chemical formula 1 is not a single bond, the step is a step of introducing a linking group represented by L, and may be applied without limitation as long as it is a normal ligand introduction method into ULB.

In this case, the bond in which L and ULB are connected may be a chemical bond, for example, a covalent bond, preferably connected by a single bond, a double bond or a triple bond.

Here, X of ULB-X is halogen or hydrogen, and as an introduction site for chemical bonding with L, may be understood as a substituent that is replaced or omitted in a reaction for covalent bonding with L.

In one embodiment of the present invention,
a ligand can be introduced into ULB (ubiquitinated ligand binding molecule) by carrying out the same method as the preparation method of the compound of Examples below, and methods of easily changing/modifying the preparation method of the compound of Examples below are also included in the present invention.

On the other hand, the solvent usable in step 1 is not particularly limited, and for example, H₂O, ethanol, tetrahydrofuran (THF), dichloromethane, toluene, acetonitrile, dimethylformamide, mixtures thereof, etc. can be used.

In addition, the reaction temperature in the above step is not particularly limited, but it is preferably carried out between the boiling point of the solvent at 20-100 °C, and the reaction time is not particularly limited, but it is preferable to react for 30 minutes to 10 hours.

In the method for producing a compound represented by chemical formula 1 according to the present invention, step 2 is a step of preparing a compound of ULB-L-PTM represented by chemical formula 1 from the prepared ULB-L-H.

In this case, step 2 is a step of introducing a molecule (e.g., a molecule, a substituent or a compound) capable of binding to a target protein, and is chemically linked to L.

In this case, as the usable solvent, H₂O, ethanol, tetrahydrofuran (THF), dichloromethane, toluene, acetonitrile, dimethylformamide, mixtures thereof, and the like may be used.

In addition, the reaction temperature in the above step is not particularly limited, but it is preferably carried out between the boiling point of the solvent at 20-100 °C, and the reaction time is not particularly limited, but it is preferable to react for 30 minutes to 10 hours.

The above preparation method can be more preferably carried out in the same manner as the preparation method of the Example compound of the present invention.

In addition, in the preparation method described herein, a person skilled in the art may modify or change reaction conditions, for example, reaction temperature, reaction time, and reaction steps in consideration of the yield of the compound to be prepared, and the reaction steps may be repeated or changed in order.

Furthermore, it should be understood that the method for preparing the compound represented by chemical formula 1 to be provided in the present invention using a conventional synthetic method in the field of organic synthesis known in the prior art is included in the scope of the present invention without limitation.

In addition, if each linker and the molecules at both ends of the compound to be provided in the present invention can be designed, for example, any preparing method for forming a ligand capable of inducing a target protein-binding molecule on one side, and a ligand capable of inducing E3 ubiquitin ligase on the other side, and designing it by easily linking these should be understood to be included in the present invention.

Also, in another aspect of the present invention,
as shown in Scheme 2 below,
a method for preparing a compound represented by claim 1 chemical formula 1 is provided, comprising:
   preparing a compound represented by the chemical formula PTM-L-H from the compound represented by PTM-X; and
   preparing a compound of ULB-L-PTM represented by chemical formula 1 from the compound represented by PTM-L-H prepared above. 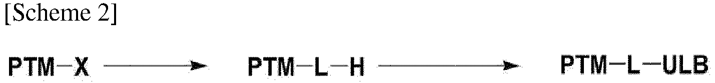
(In Scheme 2,
ULB, L, and PTM are as defined in claim 1, and
X is H).

Here, the preparation method of Scheme 2 is similar to the preparation method of Scheme 1, except that the order of introduction of ULB and PTM is inverted. Those skilled in the art can see that the level of inverting each step is easy based on the method for preparing the example compounds provided herein, and it can be understood that this is included herein without limitation.

Furthermore, in another aspect of the present invention,
there is provided a pharmaceutical composition for preventing or treating a disease or condition related to EED (embryonic ectoderm development), EZH2 (Enhancer of zeste homolog 2), or PRC2 (polycomb repressive complex 2), comprising the compound represented by chemical formula 1, its stereoisomer, or a pharmaceutically acceptable salt thereof as an active ingredient.

Here, the pharmaceutical composition provided in the present invention can show the effect of prevention or treatment without limitation, if disease or condition develops, progresses, or worsens from EED (embryonic ectoderm development), EZH2 (Enhancer of zeste homolog 2), or PRC2 (polycomb repressive complex 2).

That is, the compound represented by chemical formula 1 of the present invention is a novel degraducer, directly targets and binds to EED, induces the operation of the ubiquitination-proteasome system (UPS), and can remove the protein by degrading it, thereby it exhibits the effect of effectively preventing or treating a disease or condition.

In one embodiment of the present invention,

As in the following Examples and Experimental Examples, it was identified that the compound of the present invention represented by chemical formula 1 was a proteolytic inducing agent through the UPS pathway, and it was identified that it could exhibit improved efficacy compared to conventional targeted therapeutic agents.

Furthermore, the compound represented by chemical formula 1 of the present invention, the degraducer, has the advantage of not causing drug resistance problems and side effects problems caused by conventional targeted therapeutics and cytotoxic therapeutics.

In another embodiment of the present invention,
the disease or condition includes, for example, without limitation, any disease or condition that develops, progresses, or worsens from EED, EZH2, or PRC2, and may be, for example, cancer.

In another embodiment of the present invention,
the EED, EZH2, or PRC2 related disease or condition may, for example, be at least one selected from the group consisting of
diffuse large B-cell lymphoma, follicular lymphoma, lymphoma, leukemia, multiple myeloma, mesothelioma, gastric cancer, rhabdomyosarcoma, hepatocellular carcinoma, prostate cancer, breast carcinoma, bile duct and gallbladder cancer, bladder cancer, neuroblastoma, schwannoma, glioma, brain tumors including glioblastoma and astrocytoma, cervical cancer, colon cancer, melanoma, endometrial cancer, esophageal cancer, head and neck cancer, lung cancer, nasopharyngeal carcinoma, ovarian cancer, pancreatic cancer, renal cell carcinoma, rectal cancer, thyroid cancer, parathyroid tumor, uterine tumor, and soft tissue sarcoma.

Furthermore, in another aspect of the present invention,
there is provided a pharmaceutical composition for preventing or treating cancer, comprising the compound represented by the chemical formula 1, a stereoisomer thereof, or a pharmaceutically acceptable salt thereof as an active ingredient.

Here, the pharmaceutical composition for preventing or treating cancer, as described herein, from decomposition of EED by the chemical formula 1 compound, can be understood as preventing or treating cancer, or regardless of this theory, as shown in the Experimental Examples below, it can be understood that the compounds of the present invention prevent or treat cancer based on the extinct effect shown in cancer cell lines (see Experimental Examples and Figures below).

On the other hand, the cancer is generally classified as a cancer recognized in the medical field, and is not particularly limited as cancer cells, cancer cell lines, or tissues having the same, for example, may be at least one selected from the group consisting of colorectal cancer, liver cancer, stomach cancer, breast cancer, colon cancer, bone cancer, pancreatic cancer, head or neck cancer, uterine cancer, ovarian cancer, rectal cancer, esophageal cancer, small intestine cancer, perianal cancer, colon cancer, fallopian tube carcinoma, endometrial carcinoma, cervical carcinoma, vaginal carcinoma, vulvar carcinoma, Hodgkin's disease, prostate cancer, bladder cancer, kidney cancer, ureter cancer, renal cell carcinoma, renal pelvic carcinoma, central nervous system tumor, and leukemia.

Furthermore, the present invention relates to a pharmaceutical composition comprising an effective amount of a compound as described hereinabove, in combination with a pharmaceutically acceptable carrier, excipient or diluent and optionally other biologically active agents.

In an alternative aspect, the invention comprises administering to a patient or subject at least one effective amount of a compound as described hereinabove, optionally in combination with an additional biologically active agent, thereby degrading a bromo domain-containing protein or polypeptide capable of controlling a disease state or condition, which treats the disease state or condition. The method according to the invention can be used to treat various disease states or symptoms, including cancer, by administration of at least one effective amount of a compound described herein.

The "disease state or symptom" herein is used to describe any disease state or symptom wherein a protein dysregulation (i.e., an increase in the amount of protein expressed in the patient) occurs, and degradation of one or more proteins in the patient can provide an advantageous treatment for the patient in need thereof, or alleviate symptoms. In certain instances, the disease state or symtom can be treated.

In the disease state of the condition which can be treated using the compound according to the invention,
"neoplasia" or "cancer" of the present invention is used to refer to a pathological process that results in the formation and growth of a cancerous or malignant neoplasm, that is, an abnormal tissue that often grows by cellular proliferation more rapidly than normal, and continues to grow after stimulation that initiates the arrest of new growth. Malignant neoplasms exhibit some or all lack of functional cooperation with structural and normal tissues, invade well surrounding tissues, metastasize to multiple sites, recur easily after removal attempts, and cause death in patients without appropriate treatment. As used herein, neoplasia is used to describe all malignant cancer disease states and comprises or encompasses pathological processes associated with malignant hematopoietic, ascites and solid tumors. Exemplary cancers that may be treated with a compound of the present invention alone or in combination with at least one additional anticancer agent include squamous cell carcinoma, basal cell carcinoma, adenocarcinoma, hepatocellular carcinoma and renal cell carcinoma, bladder cancer, bowel cancer, breast cancer, cervical cancer, colon cancer, esophageal cancer, head cancer, kidney cancer, liver cancer, lung cancer, cervical cancer, ovarian cancer, pancreatic cancer, prostate cancer and stomach cancer; leukemia; benign and malignant lymphomas, particularly Burkitt lymphoma and non-Hodgkin's lymphoma; benign and malignant melanoma; myeloproliferative diseases; Ewing's sarcoma, angiosarcoma, Kaposi's sarcoma, liposarcoma, myoma, neuroepithelial sarcoma, synovial sarcoma, neurosarcoma, astrocytoma, oligodendroglioma, cerebral sarcoma, glioblastoma, neuroblastoma, gangliocytoma, ganglioglioma, medulloblastoma, pineocytoma, meningioma, sarcoma including meningeal sarcoma, neurofibroma and schwannoma; bowel cancer, breast cancer, prostate cancer, cervical cancer, uterine cancer, lung cancer, ovarian cancer, testicular cancer, thyroid cancer, astrocytoma, esophageal cancer, pancreatic cancer, stomach cancer, liver cancer, colon cancer, melanoma; carcinosarcoma, Hodgkin's disease, Wilms' tumor and teratocarcinoma. Further cancers that can be treated using the compounds according to the invention include, for example, T-lineage acute lymphoblastic leukemia (T-ALL), T-lineage lymphoblastic lymphoma (T-LL), peripheral T-cell lymphoma, adult T-cell leukemia, Pre-B-ALL, Pre-B lymphoma, giant B-cell lymphoma, Burkitt lymphoma, B-cell all, Philadelphia positive ALL and Philadelphia positive CML.

A "biooactive agent" as used herein is used to describe an agent used in combination with a compound of the present invention as an agent having a biological activity that can aid in inducing the intended therapy, inhibition and/or prevention/prophylaxis in which the compound of the present invention is used. Preferred bioactive agents that can be used in the present invention include agents having a similar pharmaceutical activity as when the compounds of the invention are used or administered, for example, in particular anti-HIV agents and anti-HCV agents, antibacterial agents, and anticancer agents, including antifungal agents, and antiviral agents.

The "pharmaceutically acceptable salt" of the present invention, when applicable in the present invention, is used to describe one or more salt forms of the compound of the present invention, in which it is provided to increase the solubility of the compound in the gastric juice of the gastrointestinal tract of a patient to enhance the solubility and bioactivity of the compound. Pharmaceutically acceptable salts include those derivatized from pharmaceutically acceptable inorganic or organic bases and acids, where applicable. Suitable salts include those derivatized from alkali metals such as potassium and sodium, alkaline earth metals such as calcium, magnesium and ammonium salts, among a variety of other acids and bases well known in the pharmaceutical art. Particular preference is given to the sodium and potassium salts as neutralizing salts of the phosphate salts according to the invention.

A "pharmaceutically acceptable derivative" herein is used to refers throughout the present invention to a form of any pharmaceutically acceptable prodrug (e.g. ester, amide or other prodrug group) that, upon administration to a patient, provides, either directly or indirectly, an active metabolite of a compound of the present invention or a compound of the present invention.

The additional aspect of the present invention provides a pharmaceutical composition comprising a combination of at least one bifunctional compound according to the present invention and an effective amount of one or more otherwise described compounds, all in effective amounts in combination with a pharmaceutically effective amount of a carrier, additive or excipient.

Where applicable, the present invention includes compositions comprising pharmaceutically acceptable salts, in particular acid or base addition salts, of a compound of the present invention. The acids used to prepare the pharmaceutically acceptable acid addition salts of the aforementioned basic compounds useful in the present invention include, among a variety of others, non-toxic acid addition salts, which are salts containing pharmaceutically acceptable ions, such as hydrochloride, hydrobromide, hydroiodide, nitrate, sulfate, disulfate, phosphate, acid phosphate, acetate, lactate, citrate, acid citrate, tartrate, bitartrate, succinate, maleate, fumarate, glucoate, saccharate, benzoate, methanesulfonate, ethanesulfonate, benzenesulfonate, p-toluenesulfonates and pamoate [i.e., 1,1'-methylene-bis-(2-hydroxy-3naphthoate)] salts.

Pharmaceutically acceptable base addition salts may also be used to provide pharmaceutically acceptable salt forms of the compounds or derivatives according to the present invention. Chemical bases that can be used to provide pharmaceutically acceptable base salts of compounds of the present invention that are acidic in nature are those that form non-toxic base salts with such compounds. Such non-toxic base salts include, but are not limited to, such pharmaceutically acceptable cations such as alkali metal cations (e.g., potassium and sodium) and alkaline earth metal cations (e.g., calcium, zinc and magnesium), ammonium or water-soluble amine addition salts such as those derivatized from N-methylglucamine (meglumine) and lower alkanolammonium and other base salts of pharmaceutically acceptable organic amines.

The compound of the present invention may be administered in a single dose or divided dose by oral, transdermal or topical routes according to the present invention. Administration of the active compound may range from continuous (intravenous instillation) to oral administration several times a day (e.g. QID), among other routes of administration, oral, topical, parenteral, intramuscular, intravenous, subcutaneous, transdermal (which may contain a permeation enhancer), buccal, sublingual and suppository administration may be included. Enteric coated oral tablets may also be used to enhance the bioavailability of the compound in the oral route of administration. The most effective dosage form will depend on the severity of the patient's disease as well as the pharmacokinetics of the particular agent selected. The administration of the compounds according to the invention can also be used as a spray, mist or as an aerosol for intranasal, intratracheal or pulmonary administration. The present invention therefore also relates to pharmaceutical compositions comprising an effective amount of a compound according to the invention, optionally in combination with a pharmaceutically acceptable carrier, additive or excipient. The compounds according to the invention may be administered in the form of immediate release, spaced release or delayed or controlled release. The delayed or controlled release form is preferably administered orally as well as suppositories and subcutaneous or other topical forms. Intramuscular injection in the form of liposomes can also be used to control or delay the release of compounds at the injection site.

The composition of the present invention may be formulated in a conventional manner using one or more pharmaceutically acceptable carriers, and may also be administered as a controlled-release formulation. Pharmaceutically acceptable carriers that may be used in such pharmaceutical compositions include, but are not limited to, ion exchangers, alumina, aluminum stearate, lecithin, serum proteins such as human serum albumin, buffer substances such as phosphates, glycine, sorbic acid, potassium sorbate, partially glycerated xixtures of saturated vegetable fatty acids, water salts or electrolytes, e.g. prolamine sulfate, disodium hydrogen phosphate, potassium hydrogen phosphate, sodium chloride, zinc salt, colloidal silica, magnesium trisilicate, polyvinyl pyrrolidine, cellulose-based substances, polyethylene glycol, sodium carboxymethylcellulose, polyacrylates, waxes, polyethylene-polyoxypropylene-block polymers, polyethylene glycol and lanolin.

The composition of the present invention may be administered orally, parenterally, as an inhalation spray, topically, rectally, nasally, buccally, via a vaginal artery or an implantation reservoir. As used herein, "parenteral" herein includes subcutaneous, intravenous, intramuscular, intra-articular, into joint fluid, intrasternal, intrathecal, intrahepatic, intramuscular and intracranial infusion or infusion techniques. Preferably, the composition is administered orally, intraperitoneally or intravenously.

The sterile injectable form of the composition of the present invention may be an aqueous or oleaginous suspension. Such suspensions may be formulated according to techniques known in the art using suitable dispersing or wetting agents and suspending agents. The sterile injectable preparation may also be a sterile injectable solution or suspension in a non-toxic pharmaceutically acceptable diluent or solvent, for example, as a solution in 1,3-butanediol. Among the acceptable vehicles and solvents that can be used are water, Ringer's solution, and isotonic sodium chloride solution. Additionally, sterile, non-volatile oils are commonly used as solvents or suspending media. For this purpose, any brand of non-volatile oils may be used, including synthetic monoglycerides or diglycerides. Fatty acids, such as oleic acid and its glyceride derivatives, can be used in preparations for injection, especially in polyoxyethylated versions of natural pharmaceutically acceptable oils, such as oleic oil or castor oil. Such oil solutions or suspensions may also include long-chain alcohol diluents or dispersants, for example Ph. Helv or similar alcohols.

The pharmaceutical composition of the present invention may be orally administered in any oral dosage form, including, but not limited to, capsules, tablets, aqueous suspensions or aqueous solutions. In the case of tablets for oral use, carriers commonly used include lactose and corn starch. Lubricating agents such as magnesium stearate are also commonly added. For oral administration in capsule form, useful diluents include lactose and dry corn starch. When aqueous suspensions are desired for oral use, the active ingredient may be combined with emulsifying and suspending agents. If desired, certain sweetening, flavoring or coloring agents may also be added.

Alternatively, the pharmaceutical composition of the present invention may be administered in the form of a suppository for rectal administration. These are solid at room temperature but liquid at rectal temperature, and therefore can be prepared by mixing the formulation with a suitable non-irritating vehicle that dissolves in the rectum to release the drug. These materials include cocoa butter, beeswax and polyethylene glycol.

The pharmaceutical composition of the present invention may also be administered topically. Suitable topical formulations are readily prepared for each site or organ. Topical application for the colon may result in a rectal suppository formulation or an appropriate enema formulation. Topical subcutaneous patties may also be used.

For topical application, the pharmaceutical composition may be formulated in a suitable ointment comprising the active ingredient suspended or dissolved in one or more carriers. Carriers for topical administration of the compounds of the present invention include, but are not limited to, mineral oil, liquid petrolatum, white petrolatum, propylene glycol, polyoxyethylene, polyoxypropylene, emulsion waxes and emulsions. In certain preferred aspects of the invention, the compound may be coated on a stent to be implanted in a patient to inhibit or reduce the incidence of occlusion in the patient's stent.

Alternatively, the pharmaceutical composition may be formulated as a suitable lotion or cream comprising the active ingredient suspended or dissolved in one or more pharmaceutically acceptable carriers. Suitable carriers include, but are not limited to, mineral oil, sorbitan monostearate, polysorbate 60, cetyl ester wax, cetearyl alcohol, 2-octyldodecanol, benzyl alcohol and water.

For ophthalmic use, the pharmaceutical composition may be formulated as a finely divided suspension in isotonic pH-adjusted sterile saline with or without preservatives, for example benzylalkonium chloride, preferably as a solution in isotonic pH-adjusted sterile saline. Alternatively for ophthalmic use, the pharmaceutical composition may be formulated in an ointment, for example in petrolatum.

The pharmaceutical composition of the present invention may also be administered by nasal aerosol or inhalation. Such compositions are prepared according to techniques well known in the art of pharmaceutical formulation. The compositions can be prepared as a solution with benzyl alcohol or other suitable preservatives, bioavailability enhancing absorption enhancers, carbon fluoride and/or other conventional solubilizing or dispersing agents.

The amount of a compound in a pharmaceutical composition of the present invention that may be combined with a single-use, carrier material to produce a single dosage form will vary depending upon the host and disease treated, and the particular method of administration. Preferably, the composition, alone or in combination with at least one other compound according to the present invention, may be formulated to contain from about 0.05 milligrams to about 750 milligrams or more of the active ingredient, more preferably from about 1 milligram to about 600 milligrams, even more preferably from about 10 milligrams to about 500 milligrams.

In addition, the specific dosage and treatment regimen for any particular patient should be understood that it depends on a variety of factors, including the activity of the particular compound to be used, age, weight, general health, sex, diet habit, duration of administration, frequency of excretion, drug combination, and the judgment of the treating physician and the severity of the particular disease or condition.

A patient or subject in need of treatment with a compound according to the invention can be treated by administering to the patient (individual) an effective amount of a compound according to the present invention, including a pharmaceutically acceptable salt, solvate or polymorph alone or in combination with well-known erythropoietic factor preparations, optionally in a pharmaceutically acceptable carrier or diluent.

Such compounds may be administered by any suitable route, including liquid, cream, gel or solid form or transdermal administration in aerosol form, for example, orally, parenterally, intravenously, intradermally, subcutaneously or topically.

The active compound is included in a pharmaceutically acceptable carrier or diluent in an amount sufficient to deliver a therapeutically effective amount to the patient according to the instructions required, without causing serious toxic effects in the patient being treated. A preferred dose of the active compound in all of the above-mentioned conditions is about 10 ng/kg to 300 mg/kg per day, preferably 0.1 to 100 mg/kg, more usually 0.5 to about 25 mg per day per kilogram of body weight of the subject/patient administered. Typical topical administration will be in the range of 0.01-5 wt/wt% in a suitable carrier.

The compounds are conveniently administered in any suitable unit dosage form including, but not limited to, less than 1 mg, 1 mg to 3000 mg, preferably 5 to 500 mg of active ingredient per unit dosage form. Oral administration of about 25-250 mg is often convenient.

The active ingredient is preferably administered such that the highest plasma concentration of the active compound is about 0.00001-30 mM, preferably about 0.1-30 µM. This can be achieved, for example, by intravenous infusion of a solution or formulation of the active ingredient, optionally in saline or aqueous medium, or by bolus administration of the active ingredient. Oral administration is also adjusted to produce effective plasma concentrations of the active agent.

The concentration of active compound in the drug composition will depend on absorption, distribution, inactivation and excretion rates of the drug, as well as other factors known to those skilled in the art. It should also be understood that doses may vary depending on the severity of the condition to be alleviated. In any particular subject, the specific dosing regimen should be adjusted for a period of time according to the individual judgment and individual needs of the person supervising or administering the administration of the composition, and the concentration ranges described above are exemplary only. It should be further understood that it is not intended to limit implementation and the range of the claimed composition. The active ingredient may be administered at one time or may be divided into various small portions and administered at intervals for various periods of time.

Furthermore, in another aspect of the present invention,
a method is provided, wherein the method for treating EED, EZH2, or PRC2 related diseases or conditions comprising administering to a subject in a therapeutically effective amount a compound represented by the chemical formula 1, a stereoisomer or a pharmaceutically acceptable salt thereof.

At this time, the EED, EZH2, or PRC2 related disease or condition is, as described herein, any disease or condition that can be prevented or treated by inhibiting the activity of EED, EZH2, or PRC2 or inducing degradation of EED or PRC2, and specifically includes any disease or condition described herein.

The therapeutically effective amount refers to an amount capable of treating, preventing or ameliorating a symptom or condition of a subject when administered into the body, depending on the administration method. In addition, the amount may be different depending on the weight, age, sex, condition, and family history of the subject to be administered, and in the present invention, the treatment method may determine a different amount of dosage according to different conditions for each subject.

The "effective amount" is an amount effective to treat EED, EZH2, or PRC2 related diseases, for example, cancer. In other embodiments, an "effective amount" of a compound means at least an amount that is capable of inhibiting EED, EZH2, or PRC2 activity or degrading EED.

The compounds and compositions according to the methods of the present invention may be administered in any amount and by any route of administration effective to treat a disease. The exact amount required will vary from subject to subject, depending on the subject's species, age, and general condition, the severity of the infection, the particular agent, its mode of administration, and the like. The compounds of the present invention are frequently formulated in dosage unit form for ease of administration and uniformity of dosage. The expression "dosage unit form" as used herein means a physically discrete unit of agent suitable for the subject to be treated. It will also be understood that the total daily usage of the compounds and compositions of the present invention will be determined by the attending physician within the scope of sound medical judgment.

The particular effective dosage level for any particular subject or organism will depend on a variety of factors, including: the disease being treated and the severity of the disease; the activity of the particular compound employed; the specific composition used; age, weight, general health, subject's gender and diet; time of administration, route of administration, and rate of excretion of the particular compound administered; duration of treatment; the specific compound used alone or co-administered, and factors other than those well known in the medical art.

The "subject" means an animal in the onset, progression, or exacerbation of EED, EZH2, or PRC2 related disease or condition, but is not limited to, may be a non-mammalian mammal, livestock, etc., for example, a human.

Hereinafter, the present invention will be described in detail with reference to Preparation Examples, Examples and Experimental Examples.

However, the following Preparation Examples, Examples and Experimental Examples are merely illustrative of the present invention, and the content of the present invention is not limited thereto.

### <Example 1> Preparation of 2-(2,6-dioxopiperidin-3-yl)-4-(6-(4-(4-(5-(furan-2-ylmethylamino)-[1,2,4]triazolo[4,3-f]pyrimidin-8-yl)phenyl)piperazin-1-yl)hexylamino)isoindoline-1,3-dione

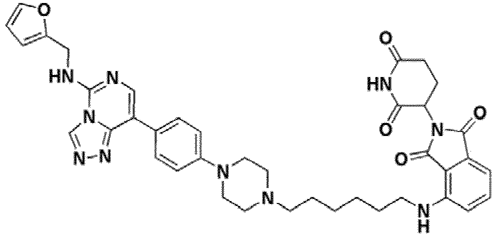

### Step 1: Preparation of tert-butyl (6-hydroxyhexyl)carbamate

To a solution of 6-aminohexane-1-ol (500 mg, 4.26 mmol) in THF (10 ml) was added TEA (0.89 ml, 6.39 mmol) and cooled to 5-10 °C. Then di-tert-butyldicarbonate (1.17 ml, 5.12 mmol) was added dropwise. The reaction mixture was stirred at room temperature for 1 hour. The reaction mixture was quenched with water and then extracted with EtOAc (50 mL×2). The combined organic layers were dried over MgSO4 and the solvent was removed in vacuo to give an oil. The crude mixture was purified by silica gel column chromatography using eluent (EtOAc / Hx = 38 %) to give the title compound (1.00 g, quant) as a clear oil.

¹H NMR (300 MHz, CDCl₃) δ 4.53 (s, 1H), 3.64 (d, J = 6.9 Hz, 2H), 3.12 (q, J = 6.6 Hz, 2H), 1.68-1.48 (m, 4H), 1.44 (s, 9H), 1.42-1.33 (m, 4H).

### Step 2: Preparation of 6-((tert-butoxycarbonyl)amino)hexyl 4-methylbenzenesulfonate

To a solution of the compound prepared in step 1 (1 g, 4.26 mmol) in DCM (20 ml) at room temperature, TEA (1.78 ml, 12.78 mmol) and DMAP (51 mg, 0.42 mmol) were added, followed by TsCl (1.62) g, 8.52 mmol) was added at 0 °C. The reaction mixture was stirred at room temperature overnight. The reaction mixture was diluted with water and then extracted with DCM (50 mL×2). The combined organic layers were dried over MgSO4 and the solvent was removed in vacuo. The crude mixture was purified by silica gel column chromatography using (EtOAc/Hx = 20 %) as eluent to give the title compound (1.47 g, 3.95 mmol, 92 %) as a clear oil.

1H NMR (300 MHz, CDC13) δ 7.86-7.75 (m, 2H), 7.37 (d, J = 8.0 Hz, 2H), 4.51 (s, 1H), 4.03 (t, J = 6.4 Hz, 2H), 3.08 (q, J = 6.7 Hz, 2H), 2.47 (s, 3H), 1.74-1.58 (m, 3H), 1.46 (s, 12H), 1.38-1.19 (m, 6H).

### Step 3: Preparation of tert-butyl (6-iodohexyl)carbamate

To a solution of the compound prepared in step 2 above (1.47 g, 3.95 mmol) in acetone (20 ml) was added sodium iodide (2.37 g, 15.8 mmol) at room temperature. The reaction mixture was stirred at reflux overnight. The reaction mixture was diluted with water and extracted with EtOAc (50 mL×2). The combined organic layers were dried over MgSO4 and the solvent was removed in vacuo to give the title compound (1.18 g, 3.60 mmol, 91 %) as a yellow transparent oil.

1H NMR (300MHz, CDC13) δ 4.51 (s, 1H), 3.18 (t, J = 7.0 Hz, 2H), 3.11 (q, J = 6.6 Hz, 2H), 1.82 (p, J = 7.0 Hz, 2H), 1.55-1.46 (m, 2H), 1.44 (s, 9H), 1.43-1.29 (m, 4H).

### Step 4: Preparation of tert-butyl (6-(4-(4-(5-((furan-2-ylmethyl)amino)-[1,2,4]triazolo[4,3-c]pyrimidin-8-yl)phenyl)piperazin-1-yl)hexyl)carbamate

N-(furan-2-ylmethyl)-8-(4-(piperazin-1-yl)phenyl)-[1,2,4]triazolo[4,3-f]pyrimidin-5-amine hydrochloride in DMF (40 mg, 0.097 mmol), the compound prepared in step 3 (54 mg, 0.145 mmol) and DIPEA (0.051 mL, 0.291 mmol) were added, and the mixture was stirred at 60° C. overnight. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 4 % to give the title compound (28 mg, 0.048 mmol, 50 %) as a yellow solid.

¹H NMR (300 MHz, CDCl₃) δ 8.31 (s, 1H), 8.12 (s, 1H), 7.88-7.80 (m, 2H), 7.43-7.38 (m, 1H), 7.09-6.99 (m, 2H), 6.46 (t, J = 5.8 Hz, 1H), 6.40-6.32 (m, 2H), 4.87 (d, J = 5.8 Hz, 2H), 4.54 (s, 1H), 3.29 (t, J = 5.0 Hz, 4H), 3.12 (q, J = 6.6 Hz, 2H), 2.63 (t, J = 5.0 Hz, 4H), 2.48-2.33 (m, 2H), 1.63-1.46 (m, 4H), 1.45 (s, 9H), 1.39-1.30 (m, 4H).

### Step 5: Preparation of 8-(4-(4-(6-aminohexyl)piperazin-1-yl)phenyl)-N-(furan-2-ylmethyl)-[1,2,4]triazolo[4,3-c]pyrimidin-5-amine

To a solution of the compound prepared in step 4 above (28 mg, 0.048 mmol) in DCM (1 ml) was added 4 M HCl in 1,4-dioxane (1 ml), and the resulting mixture was stirred at room temperature for 1 hour. stirred. The reaction mixture was concentrated in vacuo to give the title compound (29 mg, quant) as an oil.

¹H NMR (300 MHz, CD3OD) δ 9.01 (s, 1H), 8.32 (s, 1H), 7.86 (s, 1H), 7.75 (d, J = 8.5 Hz, 2H), 7.53-7.48 (m, 1H), 7.25 (d, J = 8.5 Hz, 2H), 6.50 (d, J = 3.3 Hz, 1H), 6.45-6.39 (m, 1H), 4.09-3.95 (m, 2H), 3.83-3.73 (m, 4H), 3.72-3.69 (m, 2H), 3.63-3.57 (m, 2H), 3.32-3.23 (m, 4H), 3.05-2.93 (m, 2H), 1.98-1.84 (m, 2H), 1.81-1.68 (m, 2H), 1.59-1.47 (m, 4H).

### Step 6: Preparation of 2-(2,6-dioxopiperidin-3-yl)-4-(6-(4-(4-(5-(furan-2-ylmethylamino)-[1,2,4]triazolo[4,3-f]pyrimidin-8-yl)phenyl)piperazin-1-yl)hexylamino)isoindolin-1,3-dione

2-(2,6-dioxopiperidin-3-yl)-4-fluoroisoindolin-1,3-dione (13 mg, 0.048 mmol) in a solution of the compound prepared in step 5 (24 mg, 0.048 mmol) in DMF, DIPEA (0.025 mL, 0.144 mmol) was added and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 7 % to give the final title compound (3 mg, 0.004 mmol, 8 %) as a yellow solid.

¹H NMR (500 MHz, chloroform-d) δ 8.36 (s, 1H), 8.31 (s, 1H), 8.13 (s, 1H), 7.88 (d, J = 8.1 Hz, 2H), 7.52 (t, J = 7.7 Hz, 1H), 7.43-7.39 (m, 1H), 7.11 (d, J = 7.0 Hz, 1H), 7.08-7.02 (m, 2H), 6.90 (d, J = 8.4 Hz, 1H), 6.48 (t, J = 5.8 Hz, 1H), 6.41-6.34 (m, 2H), 6.26-6.19 (m, 1H), 4.94-4.90 (m, 1H), 4.88 (d, J = 5.7 Hz, 2H), 3.81-3.54 (m, 8H), 3.34-3.25 (m, 2H), 3.07-2.97 (m, 4H), 2.88-2.67 (m, 3H), 2.19-2.12 (m, 1H), 2.07-1.91 (m, 6H).

### <Example 2> Preparation of 2-(2,6-dioxopiperidin-3-yl)-4-(8-(4-(4-(5-(furan-2-ylmethylamino)-[1,2,4]triazolo[4,3-f]pyrimidin-8-yl)phenyl)piperazin-1-yl)octylamino)isoindolin-1,3-dione

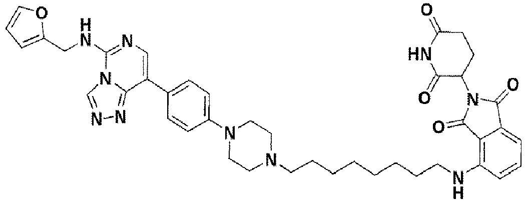

### Step 1: Preparation of tert-butyl (8-hydroxyoctyl)carbamate

To a solution of 8-aminooctane-1-ol (1 g, 6.88 mmol) in THF (20 ml) was added TEA (1.43 ml, 10.32 mmol) and the reaction mixture was cooled to 5-10 °C. Then, di-tert-butyldicarbonate (1.90 ml, 8.26 mmol) was added dropwise. The reaction mixture was stirred at room temperature for 1 hour. The reaction mixture was quenched with water and then extracted with EtOAc (50 mL×2). The combined organic layers were dried over MgSO4 and the solvent was removed in vacuo to give an oil. The crude mixture was purified by silica gel column chromatography using (EtOAc/Hx = 40 %) as eluent to give the title compound (1.48 g, 6.03 mmol, 87 %) as a white solid.

¹H NMR (300 MHz, CDCl₃) δ 4.50 (s, 1H), 3.64 (t, J = 6.6 Hz, 2H), 3.10 (q, J = 6.7 Hz, 2H), 1.64-1.47 (m, 4H), 1.44 (s, 9H), 1.39-1.28 (m, 8H).

### Step 2: Preparation of 8-((tert-butoxycarbonyl)amino)octyl 4-methylbenzensulfonate

To a solution of the compound prepared in step 1 (300 mg, 1.22 mmol) in DCM (10 ml) at room temperature, TEA (0.510 ml, 3.66 mmol) and DMAP (15 mg, 0.122 mmol) were added, followed by p-TsCl (466 mg, 2.44 mmol) was added at 0 °C. The reaction mixture was stirred at room temperature overnight. The reaction mixture was diluted with water and then extracted with DCM (50 mL×2). The combined organic layers were dried over MgSO4 and the solvent was removed in vacuo. The crude mixture was purified by silica gel column chromatography using eluent (EtOAc/Hx = 27 %) to give the title compound (484 mg, 1.21 mmol, 99 %) as a clear oil.

¹H NMR (300 MHz, CDCl₃) δ7.79 (d, J = 8.0 Hz, 2H), 7.35 (d, J = 8.0 Hz, 2H), 4.50 (s, 1H), 4.01 (t, *J* = 6.5 Hz, 2H), 3.08 (q, *J* = 6.8 Hz, 2H), 2.45 (s, 3H), 1.69-1.57 (m, 2H), 1.53-1.37 (m, 11H), 1.34-1.16 (m, 8H).

### Step 3: Preparation of tert-butyl (8-iodooctyl)carbamate

To a solution of the compound prepared in step 2 above (484 mg, 1.21 mmol) in acetone (20 ml) was added sodium iodide (725 mg, 4.84 mmol) at room temperature. The reaction mixture was stirred at reflux overnight. The reaction mixture was diluted with water and extracted with EtOAc (50 mL×2). The combined organic layers were dried over MgSO4 and the solvent was removed in vacuo to give the title compound (394 mg, 1.10 mmol, 91%) as a brown oil.

¹H NMR (300MHz, CDCl₃) δ4.50 (s, 1H), 3.19 (t, J = 7.0 Hz, 2H), 3.10 (q, J = 6.7 Hz, 2H), 1.82 (p, *J* = 7.0 Hz, 2H), 1.53-1.40 (m, 11H), 1.40-1.22 (m, 8H).

### Step 4: Preparation of tert-butyl (8-(4-(4-(5-((furan-2-ylmethyl)amino)-[1,2,4]triazolo[4,3-c]pyrimidin-8-yl)phenyl)piperazin-1-yl)octyl)carbamate

N-(furan-2-ylmethyl)-8-(4-(piperazin-1-yl)phenyl)-[1,2,4]triazolo[4,3-f]pyrimidin-5-amine hydrochloride in DMF (50 mg, 0.121 mmol), the compound prepared in step 3 (65 mg, 0.182 mmol) and DIPEA (0.063 mL, 0.363 mmol) were added, and the mixture was stirred at 60° C. overnight. The reaction mixture was diluted with water (20 ml) and extracted with EtOAc (50 ml×2). The combined organic layers were washed with brine, dried over MgSO4 and the solvent removed in vacuo to give an oil. The crude mixture was purified by silica gel column chromatography using MeOH/DCM = 4 % to give the title compound (32 mg, 0.053 mmol, 43 %) as an ivory solid.

¹H NMR (300 MHz, CDCl₃) δ 8.30 (s, 1H), 8.11 (s, 1H), 7.89-7.79 (m, 2H), 7.43-7.36 (m, 1H), 7.09-6.98 (m, 2H), 6.51 (t, *J* = 5.8 Hz, 1H), 6.40-6.32 (m, 2H), 4.86 (d, *J* = 5.7 Hz, 2H), 4.56 (s, 1H), 3.39-3.23 (m, 4H), 3.17-3.03 (m, 2H), 2.74-2.58 (m, 4H), 2.49-2.35 (m, 2H), 1.63-1.49 (m, 2H), 1.49-1.37 (m, 11H), 1.37-1.28 (m, 8H).

### Step 5: Preparation of 8-(4-(4-(8-aminooctyl)piperazin-1-yl)phenyl)-N-(furan-2-ylmethyl)-[1,2,4]triazolo[4,3-c]pyrimidin-5-amine

To a solution of the compound prepared in step 4 above (32 mg, 0.053 mmol) in DCM (2 mL) was added 4 M HCl in dioxane (1 ml, 0.53 mmol). The reaction mixture was stirred at room temperature for 1 hour. The reaction mixture was concentrated in vacuo to give the title compound (28 mg, crude) as an ivory solid.

### Step 6: Preparation of 2-(2,6-dioxopiperidin-3-yl)-4-(8-(4-(4-(5-(furan-2-ylmethylamino)-[1,2,4]triazolo[4,3-f]pyrimidin-8-yl)phenyl)piperazin-1-yl)octylamino)isoindolin-1,3-dione

To a solution of the compound prepared in step 5 above (28 mg, 0.053 mmol) in DMSO, 2-(2,6-dioxopiperidin-3-yl)-4-fluoroisoindolin-1,3-dione (15 mg, 0.053 mmol), DIPEA (0.029 mL, 0.158 mmol) was added and stirred at 90 °C overnight. The reaction mixture was diluted with water and extracted with EtOAc. The combined organic layers were washed with brine, dried over MgSO4 and the solvent removed in vacuo to give an oil. The crude mixture was purified using Prep TLC in MeOH/DCM = 5 % to give the title compound (3 mg, 0.003 mmol, impurity) as a yellow solid.

LC/MS (ESI) *m*/*z* 759.1[M+H]⁺, 757.1[M-H]⁻

### <Example 3> Preparation of 2-(2,6-dioxopiperidin-3-yl)-4-(6-(4-(4-(5-(furan-2-ylmethylamino)-[1,2,4]triazolo[4,3-f]pyrimidin-8-yl)phenyl)piperazin-1-yl)-6-oxohexylamino)isoindolin-1,3-dione

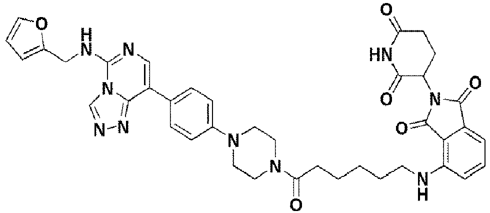

### Step 1: Preparation of tert-butyl 6-aminohexanoate

A solution of tert-butyl acetate (10 ml, 9.42 mmol) was cooled to 0 °C, and 6-aminohexanoic acid (500 mg, 3.14 mmol) was slowly added thereto. After 5 min, HClO4 (0.283 ml, 4.71 mmol) was added with stirring at 0°C. After 5 min, the solution was warmed to room temperature and stirred overnight. The reaction mixture was quenched by addition of NaHCO3 (aq). The combined organic layers were dried over MgSO4 and the solvent was removed in vacuo. The crude mixture was purified by silica gel column chromatography using (EtOAc/Hx = 70 %) as eluent to give the title compound (180 mg, 0.836 mmol, 26 %) as a yellow transparent oil.

### Step 2: Preparation of tert-butyl 6-((2-(2,6-dioxopiperidin-3-yl)-1,3-dioxoisoindolin-4-yl)amino)hexanoate

Compound prepared in step 1 above (85 mg, 0.323 mmol), 2-(2,6-dioxopiperidin-3-yl)-4-fluoroisoindolin-1,3-dione (107 mg, 0.387 mmol) in DMF (1 mL) ) was added DIPEA (0.084 mL, 0.485 mmol). The reaction mixture was stirred at 90 °C overnight. The reaction mixture was diluted with water and extracted with EtOAc (50 mL×2). The combined organic layers were washed with brine, dried over MgSO4 and the solvent removed in vacuo to give an oil. The crude mixture was purified by silica gel column chromatography using EtOAc/Hex 40 % as eluent to give the title compound (55 mg, 0.106 mmol, 33 %) as a yellow solid.

### Step 3: Preparation of 6-((2-(2,6-dioxopiperidin-3-yl)-1,3-dioxoisoindolin-4-yl)amino)hexanoate

To a solution of the compound prepared in step 2 above (55 mg, 0.323 mmol) in DCM (3 mL) was added TFA (2 mL). The reaction mixture was stirred at room temperature for 1 hour. The reaction mixture was concentrated in vacuo to give the title compound (50 mg, 0.106 mmol, quant.) as an oil.

### Step 4: Preparation of 2-(2,6-dioxopiperidin-3-yl)-4-(6-(4-(4-(5-(furan-2-ylmethylamino)-[1,2,4]triazolo[4,3-f]pyrimidin-8-yl)phenyl)piperazin-1-yl)-6-oxohexylamino)isoindolin-1,3-dione

N-(furan-2-ylmethyl)-8-(4-(piperazin-1-yl)phenyl)-[1,2,4]triazolo[4,3-f]pyrimidin-5-amine hydrochloride in DMF (16 mg , 0.039 mmol), the compound prepared in step 3 (15 mg, 0.039 mmol), HATU (22 mg, 0.059 mmol), and DIPEA (0.027 mL, 0.157 mmol) were added and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using eluent MeOH/DCM = 4 % to give the title compound (12 mg, 0.016 mmol, 41 %) as a yellow solid.

LC/MS (ESI) m/z 815.1 [M+H]+, 813.0 [M-H]-

### <Example 4> Preparation of 2-(2,6-dioxopiperidin-3-yl)-4-(8-(4-(4-(5-(furan-2-ylmethylamino)-[1,2,4]triazolo[4,3-f]pyrimidin-8-yl)phenyl)piperazin-1-yl)-8-oxooctylamino)isoindolin-1,3-dione

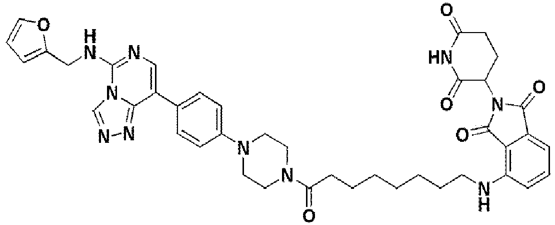

### Step 1: Preparation of tert-butyl 8-aminooctanoate

A solution of tert-butyl acetate (10 ml, 9.42 mmol) was cooled to 0 °C, and 8-aminooctane acid (500 mg, 3.14 mmol) was slowly added. After 5 min, HClO4 (0.283 ml, 4.71 mmol) was added with stirring at 0°C. After 5 min, the solution was warmed to room temperature and stirred overnight. The reaction mixture was quenched by addition of NaHCO3 (aq). The combined organic layers were dried over MgSO4 and the solvent was removed in vacuo. The crude mixture was purified by silica gel column chromatography using (EtOAc/Hx = 70 %) as eluent to give the title compound (180 mg, 0.836 mmol, 26 %) as a yellow transparent oil.

¹H NMR (300 MHz, CDCl₃) δ 3.15 (t, J = 7.7 Hz, 2H), 2.20 (t, J = 7.5 Hz, 2H), 2.02-1.47 (m, 8H), 1.44 (s, 9H), 1.40-1.20 (m, 6H).

### Step 2: Preparation of tert-butyl 8-((2-(2,6-dioxopiperidin-3-yl)-1,3-dioxoisoindolin-4-yl)amino)octanoate

To a solution of the compound of step 1 above (180 mg, 0.836 mmol) in DMF, 2-(2,6-dioxopiperidin-3-yl)-4-fluoroisoindolin-1,3-dione (231 mg, 0.836 mmol), DIPEA ( 0.218 mL, 1.25 mmol) was added and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc/Hx = 29 % to give the title compound (51 mg, 0.108 mmol, 13 %) as a yellow solid.

¹H NMR (300 MHz, CDCl₃) δ 7.99 (s, 1H), 7.55-7.44 (m, 1H), 7.13-7.05 (m, 1H), 6.92-6.83 (m, 1H), 6.22 (t, J = 5.6 Hz, 1H), 4.97-4.86 (m, 1H), 3.26 (q, J = 6.7 Hz, 2H), 2.96-2.69 (m, 3H), 2.21 (t, J = 7.4 Hz, 2H), 2.17-2.08 (m, 1H), 1.73-1.50 (m, 5H), 1.44 (s, 9H), 1.36 (m, 5H).

### Step 3: Preparation of 8-((2-(2,6-dioxopiperidin-3-yl)-1,3-dioxoisoindolin-4-yl)amino)octanoate

To a solution of the compound prepared in step 2 above (50 mg, 0.106 mmol) in DCM (1 ml) was added TFA (1 ml), and the resulting mixture was stirred at room temperature for 4 hours. The reaction mixture was concentrated in vacuo to give the desired compound (50 mg, crude) as an oil.

¹H NMR (300 MHz, DMSO-*d*₆) δ 11.10 (s, 1H), 7.58 (dd, J = 8.6, 7.0 Hz, 1H), 7.09 (d, J = 8.6 Hz, 1H), 7.02 (d, J = 7.0 Hz, 1H), 5.12-5.00 (m, 1H), 3.29 (t, J = 7.0 Hz, 2H), 2.97-2.79 (m, 1H), 2.65-2.53 (m, 2H), 2.19 (t, J = 7.3 Hz, 2H), 2.10-2.00 (m, 1H), 1.65-1.54 (m, 2H), 1.53-1.42 (m, 2H), 1.41-1.21 (m, 6H).

### Step 4: Preparation of 2-(2,6-dioxopiperidin-3-yl)-4-(8-(4-(4-(5-(furan-2-ylmethylamino)-[1,2,4]triazolo[4,3-f]pyrimidin-8-yl)phenyl)piperazin-1-yl)-8-oxooctylamino)isoindolin-1,3-dione

N-(furan-2-ylmethyl)-8-(4-(piperazin-1-yl)phenyl)-[1,2,4]triazolo[4,3-f]pyrimidin-5-amine hydrochloride in DMF (43 mg, 0.106 mmol), the compound prepared in step 3 (44 mg, 0.106 mmol), HATU (60 mg, 0.159 mmol), and DIPEA (0.074 mL, 0.424 mmol) were added and stirred at room temperature for 2 hours.. The reaction mixture was diluted with water and extracted with EtOAc. The combined organic layers were washed with brine, dried over MgSO4 and the solvent removed in vacuo to give an oil.

¹H NMR (500 MHz, CDCl₃) δ 8.39 (s, 1H), 8.31 (s, 1H), 8.12 (s, 1H), 7.89-7.82 (m, 2H), 7.49 (dd, J = 8.5, 7.1 Hz, 1H), 7.43-7.37 (m, 1H), 7.08 (d, J = 7.1 Hz, 1H), 7.06-7.01 (m, 2H), 6.87 (d, J = 8.5 Hz, 1H), 6.51 (t, J = 5.8 Hz, 1H), 6.40-6.34 (m, 2H), 6.23 (t, J = 5.6 Hz, 1H), 4.94-4.88 (m, 1H), 4.87 (d, J = 5.6 Hz, 2H), 3.88-3.73 (m, 2H), 3.71-3.59 (m, 2H), 3.32-3.16 (m, 6H), 2.93-2.66 (m, 3H), 2.38 (d, J = 7.5 Hz, 2H), 2.18-2.08 (m, 1H), 1.76-1.59 (m, 4H), 1.50-1.33 (m, 6H) ; LC/MS (ESI) m/z 773.1 [M+H]⁺, 771.1 [M-H]⁻

### <Example 5> Preparation of 2-(2,6-dioxopiperidin-3-yl)-4-(9-(4-(4-(5-(furan-2-ylmethylamino)-[1,2,4]triazolo[4,3-f]pyrimidin-8-yl)phenyl)piperazin-1-yl)-9-oxononylamino)isoindolin-1,3-dione

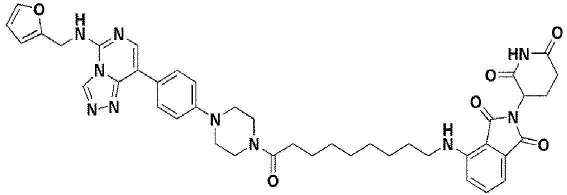

### Step 1: Preparation of 9-bromonanoate

To a solution of concentrated nitric acid (30 mL) was added 9-bromononanol (2.00 g, 8.96 mmol)) over 30 min maintaining the temperature of 25-30 °C. The solution was stirred at room temperature for 4 h, then heated to 80 °C and stirred for an additional 1 h. The reaction mixture was then cooled to room temperature and carefully diluted with 100 ml of distilled water. The aqueous layer was extracted with diethyl ether and the combined organics were washed with brine, dried over MgSO4 and concentrated under reduced pressure to give the title compound (1.36 g, 5.73 mmol, crude) as a clear yellow oil.

¹H NMR (300 MHz, DMSO-*d*₆) δ 3.53 (t, *J* = 6.7 Hz, 2H), 2.19 (t, *J* = 7.3 Hz, 2H), 1.79 (p, J = 6.8 Hz, 2H), 1.41-1.18 (m, 10H).

### Step 2: Preparation of tert-butyl 9-bromonanoate

A solution of the compound prepared in step 1 above (1.36 g, 5.73 mmol) in anhydrous THF (30 mL) was cooled to -40 °C and trifluoroacetic anhydride (1.59 mL, 11.47 mmol) was added slowly. At -40 °C After stirring for 30 min, t-BuOH (7.34 mL, 76.7 mmol) was added and the solution was allowed to warm to room temperature and stirred for 16 h. The reaction mixture was poured slowly onto a mixture of crushed ice and NaHCO3 (sat. aq.). The aqueous layer was extracted with EtOAc and the combined organics were washed with brine, dried over MgSO4 and concentrated under reduced pressure. The crude mixture was purified by silica gel column chromatography using EtOAc/Hex (5 %) as eluent to give the title compound (200 mg, 0.682 mmol, 7 %) as a colorless oil.

¹H NMR (300 MHz, CDCl₃) δ 3.39 (t, *J* = 6.8 Hz, 2H), 2.19 (t, J = 7.5 Hz, 2H), 1.84 (p, *J* = 7.0 Hz, 2H), 1.64-1.50 (m, 2H), 1.50-1.36 (m, 11H), 1.36-1.23 (m, 6H).

### Step 3: Preparation of tert-butyl 9-aminononanoate

To a solution of the compound prepared in step 2 (200 mg, 0.682 mmol) was added 7N NH3 in MeOH and stirred at 50°C for 24 hours. The reaction mixture was concentrated to give an oil. The crude mixture was purified by silica gel column chromatography using MeOH/DCM = 8 % to give the title compound (48 mg, 0.209 mmol, 30 %) as a yellow solid.

¹H NMR (300 MHz, CDCl₃) δ 6.92 (s, 2H), 3.04 (t, J = 7.7 Hz, 2H), 2.20 (t, J = 7.5 Hz, 2H), 1.91-1.73 (m, 2H), 1.63-1.50 (m, 2H), 1.50-1.37 (m, 11H), 1.37-1.21 (m, 6H).

### Step 4: Preparation of tert-butyl 9-((2-(2,6-dioxopiperidin-3-yl)-1,3-dioxoisoindolin-4-yl)amino)nonanoate

To a solution of the compound prepared in step 3 above (48 mg, 0.209 mmol) in DMSO, 2-(2,6-dioxopiperidin-3-yl)-4-fluoroisoindolin-1,3-dione (48 mg, 0.209 mmol), DIPEA (0.045 mL, 0.261 mmol) was added and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc/Hex = 33 % to give the title compound (20 mg, 0.041 mmol, 23 %) as a yellow solid.

¹H NMR (300 MHz, CDCl₃) δ 8.22 (s, 1H), 7.49 (dd, *J* = 8.5, 7.1 Hz, 1H), 7.08 (d, *J* = 7.1 Hz, 1H), 6.88 (d, *J* = 8.5 Hz, 1H), 6.23 (t, *J* = 5.6 Hz, 1H), 4.96-4.88 (m, 1H), 3.25 (q, *J* = 6.6 Hz, 2H), 2.93-2.69 (m, 3H), 2.24-2.17 (m, 2H), 2.17-2.10 (m, 1H), 1.71-1.63 (m, 2H), 1.62-1.53 (m, 2H), 1.44 (s, 9H), 1.43-1.38 (m, 2H), 1.38-1.29 (m, 6H).

### Step 5: Preparation of 9-((2-(2,6-dioxopiperidin-3-vl)-1,3-dioxoisoindolin-4-yl)amino)nonanoate

To a solution of the compound prepared in step 4 above (20 mg, 0.041 mmol) in DCM (2 ml) was added TFA (1 ml), and the resulting mixture was stirred at room temperature for 1 hour. The reaction mixture was concentrated in vacuo to give the title compound (263 mg, crude) as a yellow oil.

¹H NMR (300 MHz, DMSO-*d*₆) δ 11.10 (s, 1H), 7.58 (t, *J* = 8.2, 7.0 Hz, 1H), 7.09 (d, *J* = 8.2 Hz, 1H), 7.02 (d, J = 7.0 Hz, 1H), 6.61-6.47 (m, 1H), 5.11-5.00 (m, 1H), 3.36-3.23 (m, 2H), 2.97-2.71 (m, 2H), 2.66-2.52 (m, 2H), 2.18 (t, *J* = 7.3 Hz, 2H), 2.11-1.95 (m, 1H), 1.64-1.39 (m, 4H), 1.40-1.00 (m, 10H).

### Step 6: Preparation of 2-(2,6-dioxopiperidin-3-yl)-4-(9-(4-(4-(5-(furan-2-ylmethylamino)-[1,2,4]triazolo[4,3-f]pyrimidin-8-yl)phenyl)piperazin-1-yl)-9-oxononylamino)isoindolin-1,3-dione

N-(furan-2-ylmethyl)-8-(4-(piperazin-1-yl)phenyl)-[1,2,4]triazolo[4,3-f]pyrimidin-5-amine hydrochloride (17mg) in DMF , 0.041mmol), the compound prepared in step 5 (17mg, 0.041mmol), EDCI HCl (9mg, 0.045mmol), HOBt HO (7mg, 0.041mmol), DIPEA (0.028 mL, 0.164 mmol) was added and , and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 5 % to give the title compound (8 mg, 0.010 mmol, 24 %) as a yellow solid.

¹H NMR (500 MHz, CDCl₃) δ 8.31 (s, 1H), 8.29 (s, 1H), 8.12 (s, 1H), 7.89-7.82 (m, 2H), 7.49 (dd, *J* = 8.5, 7.1 Hz, 1H), 7.43-7.39 (m, 1H), 7.08 (d, *J* = 7.1 Hz, 1H), 7.07-7.02 (m, 2H), 6.88 (d, *J* = 8.5 Hz, 1H), 6.50 (t, *J* = 5.8 Hz, 1H), 6.41-6.35 (m, 2H), 6.22 (t, *J* = 5.6 Hz, 1H), 4.94-4.89 (m, 1H), 4.87 (d, *J* = 5.7 Hz, 2H), 3.86-3.76 (m, 2H), 3.71-3.61 (m, 2H), 3.31-3.18 (m, 6H), 2.92-2.68 (m, 3H), 2.38 (t, J = 7.6 Hz, 2H), 2.18-2.10 (m, 1H), 1.75-1.59 (m, 6H), 1.47-1.32 (m, 6H). LC/MS (ESI) *m*/*z* 787.1 [M+H]⁺, 785.0 [M-H]⁻

### <Example 6> Preparation of 2-(2,6-dioxopiperidin-3-yl)-4-(10-(4-(4-(5-(furan-2-ylmethylamino)-[1,2,4]triazolo[4,3-f]pyrimidin-8-yl)phenyl)piperazin-1-yl)-10-oxodecylamino)isoindolin-1,3-dione

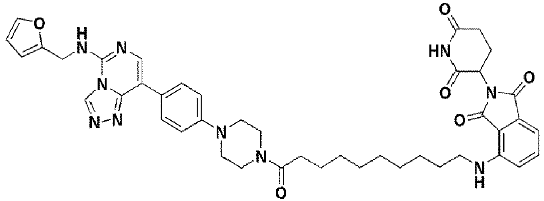

### Step 1: Preparation of 11-aminoundecanoate

In a solution of 11-bromoundecanoic acid (5.00 g, 18.9 mmol) in ammonium hydroxide (28 % NH3), the reaction mixture was stirred for 24 h. The solution was evaporated under reduced pressure to give a solid which was washed with DCM to give the title compound (1.90 g, 9.44 mmol, 50 %) as a brown solid.

¹H NMR (300 MHz, DMSO-*d*₆) δ 6.73 (s, 2H), 2.76 (t, *J* = 7.5 Hz, 1H), 2.15 (t, *J* = 7.3 Hz, 2H), 1.62-1.40 (m, 4H), 1.38-1.12 (m, 12H).

### Step 2: Preparation of tert-butyl 11-aminoundecanoate

A solution of the compound prepared in step 1 above (500 mg, 2.48 mmol) in thionyl chloride (24.80 ml, 24.8 mmol) was stirred at room temperature for 1.5 h, after which the excess thionyl chloride was removed in vacuo. A solution of NaHCO3 (458 mg, 5.45 mmol) in t-BuOH (6 ml) was added to the residue under stirring and the reaction mixture was stirred overnight at room temperature. The volatiles were removed in vacuo and the residue was dissolved in EtOAc (30 ml) and NaOH (1M, aq) (20 ml). The combined organic layers were washed with water (30 ml) and brine (20 ml × 3), dried over MgSO4 and the solvent removed in vacuo to give the title compound (521 mg, 2.02 mmol, 81%) as a yellow clear oil.

¹H NMR (300 MHz, CDCl₃) δ2.72 (t, *J* = 7.2 Hz, 2H), 2.20 (t, *J* = 7.5 Hz, 2H), 1.64-1.45 (m, 4H), 1.44 (s, 9H), 1.36-1.21 (m, 12H).

### Step 3: Preparation of tert-butyl 11-((2-(2,6-dioxopiperidin-3-yl)-1,3-dioxoisoindolin-4-yl)amino) undecanoate

To a solution of the compound prepared in step 2 (300 mg, 1.16 mmol) in DMF, 2-(2,6-dioxopiperidin-3-yl)-4-fluoroisoindolin-1,3-dione (320 mg, 1.16 mmol), DIPEA (0.304 mL, 1725 mmol) was added and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc/Hx = 33 % to give the title compound (100 mg, 0.195 mmol, 16 %) as a yellow solid.

¹H NMR (300 MHz, CDCl₃) δ 8.49 (s, 1H), 7.55-7.43 (m, 1H), 7.07 (d, *J* = 7.1 Hz, 1H), 6.87 (d, *J* = 8.6 Hz, 1H), 6.24 (t, *J* = 5.6 Hz, 1H), 4.99-4.86 (m, 1H), 3.25 (q, *J* = 6.6 Hz, 2H), 2.93-2.69 (m, 3H), 2.20 (t, *J* = 7.5 Hz, 2H), 2.17-2.03 (m, 1H), 1.73-1.62 (m, 2H), 1.62-1.50 (m, 2H), 1.44 (s, 9H), 1.40-1.16 (m, 12H).

### Step 4: Preparation of 11-((2-(2,6-dioxopiperidin-3-yl)-1,3-dioxoisoindolin-4-yl)amino)undecanoate

To a solution of the compound prepared in step 3 above (100 mg, 0.195 mmol) in DCM (2 ml) was added TFA (2 ml), and the resulting mixture was stirred at room temperature for 1 hour. The reaction mixture was concentrated in vacuo to give the title compound (100 mg, crude) as a yellow oil.

¹H NMR (300 MHz, DMSO-*d*₆) δ 11.10 (s, 1H), 7.58 (dd, *J* = 8.6, 7.1 Hz, 1H), 7.09 (d, *J* = 8.6 Hz, 1H), 7.02 (d, *J* = 7.1 Hz, 1H), 5.11-5.00 (m, 1H), 3.29 (t, *J* = 7.1 Hz, 2H), 2.97-2.78 (m, 1H), 2.65-2.46 (m, 2H), 2.18 (t, *J* = 7.3 Hz, 2H), 2.08-1.97 (m, 1H), 1.64-1.41 (m, 4H), 1.41-1.19 (m, 12H).

### Step 5: Preparation of 2-(2,6-dioxopiperidin-3-yl)-4-(10-(4-(4-(5-(furan-2-ylmethylamino)-[1,2,4]triazolo[4,3-f]pyrimidin-8-yl)phenyl)piperazin-1-yl)-10-oxodecylamino)isoindolin-1,3-dione

To a solution of the compound prepared in step 4 (30 mg, 0.072 mmol) in DMF, N-(furan-2-ylmethyl)-8-(4-(piperazin-1-yl)phenyl)-[1,2,4 ]triazolo[4,3-f]pyrimidin-5-amine hydrochloride (32 mg, 0.072 mmol), HATU (41 mg, 0.109 mmol), and DIPEA (0.051 mL, 0.291 mmol) were added and stirred at room temperature for 2 hours.. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 3 % to give the title compound (18 mg, 0.024 mmol, 34 %) as a yellow solid.

¹H NMR (500 MHz, CDC13) δ 8.31 (s, 1H), 8.12 (s, 1H), 7.89-7.83 (m, 2H), 7.51-7.46 (m, 1H), 7.43-7.38 (m, 1H), 7.08 (d, *J* = 7.1 Hz, 1H), 7.07-7.02 (m, 2H), 6.88 (d, *J* = 8.5 Hz, 1H), 6.48 (t, *J* = 5.8 Hz, 1H), 6.40-6.34 (m, 2H), 6.23 (t, *J* = 5.6 Hz, 1H), 4.94-4.89 (m, 1H), 4.87 (d, *J* = 5.7 Hz, 2H), 3.85-3.76 (m, 2H), 3.69-3.62 (m, 2H), 3.30-3.18 (m, 6H), 2.92-2.68 (m, 3H), 2.41-2.35 (m, 2H), 2.17-2.09 (m, 1H), 1.70-1.59 (m, 6H), 1.45-1.39 (m, 2H), 1.39-1.30 (m, 8H).

### <Example 7> Preparation of 2-(2,6-dioxopiperidin-3-yl)-4-(11-(4-(4-(5-(furan-2-ylmethylamino)-[1,2,4]triazolo[4,3-f]pyrimidin-8-yl)phenyl)piperazin-1-yl)-11-oxoundecylamino)isoindolin-1,3-dione

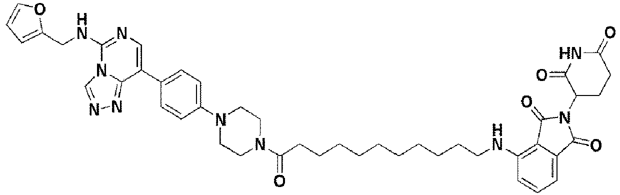

### Step 1: Preparation of tert-butyl 10-aminodecanoate

A solution of 8-aminooctane acid (300 mg, 1.60 mmol) in thionyl chloride (16 ml, 16.0 mmol) was stirred at room temperature for 1.5 h, after which the excess thionyl chloride was removed in vacuo. A solution of NaHCO3 (90 mg, 3.20 mmol) in t-BuOH (8 ml) was added to the residue under stirring and the reaction mixture was stirred overnight at room temperature. The volatiles were removed in vacuo and the residue was dissolved in EtOAc (30 ml) and NaOH (1M, aq) (20 ml). The combined organic layers were washed with water (30 ml) and brine (20 ml×3), dried over MgSO4 and the solvent was removed in vacuo to give the title compound (290 mg, 1.18 mmol, 74%) as a yellow clear oil.

### Step 2: Preparation of tert-butyl 10-((2-(2,6-dioxopiperidin-3-yl)-1,3-dioxoisoindolin-4-yl)amino)decanoate

To a solution of the compound prepared in step 1 (290 mg, 1.19 mmol) in DMSO, 2-(2,6-dioxopiperidin-3-yl)-4-fluoroisoindolin-1,3-dione (330 mg, 1.19 mmol), DIPEA (0.3 mL, 1.78 mmol) was added and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc/Hx = 33 % to give the title compound (190 mg, 0.380 mmol, 32 %) as a yellow solid.

¹H NMR (300 MHz, CDCl₃) δ 8.26 (s, 1H), 7.56-7.49 (m, 1H), 7.11-7.07 (m, 1H), 6.89 (d, *J* = 8.5 Hz, 1H), 6.25 (t, *J* = 5.6 Hz, 1H), 4.98-4.87 (m, 1H), 3.27 (q, *J* = 6.6 Hz, 2H), 2.94-2.73 (m, 1H), 2.22 (t, *J* = 7.5 Hz, 2H), 2.17-2.10 (m, 1H), 1.70-1.56 (m, 4H), 1.46 (s, 11H), 1.32 (s, 9H).

### Step 3: Preparation of 10-((2-(2,6-dioxopiperidin-3-yl)-1,3-dioxoisoindolin-4-yl)amino)decanoate

To a solution of the compound prepared in step 2 above (190 mg, 0.380 mmol) in DCM (2 ml) was added TFA (2 ml), and the resulting mixture was stirred at room temperature for 1 hour. The reaction mixture was concentrated in vacuo to give the desired compound (190 mg, crude) as a yellow oil.

### Step 4: Preparation of 2-(2,6-dioxopiperidin-3-yl)-4-(11-(4-(4-(5-(furan-2-ylmethylamino)-[1,2,4]triazolo[4,3-f]pyrimidin-8-yl)phenyl)piperazin-1-yl)-11-oxoundecylamino)isoindolin-1,3-dione

(2R,4R)-1-((R)-2-amino-3,3-dimethylbutanoyl)-4-hydroxy-N-(4-(4-methylthiazole-5-yl)benzyl)pyrrolidin-2- in DMF To a solution of carboxamide hydrochloride (30 mg, 0.072 mmol), the compound prepared in step 3 (34 mg, 0.072 mmol), HATU (41 mg, 0.109 mmol), and DIPEA (0.051 mL, 0.291 mmol) were added and stirred at room temperature for 2 stirred for hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 5 % to give the title compound (18 mg, 0.022 mmol, 30 %) as a yellow solid.

¹H NMR (500 MHz, CDCl₃) δ 8.31 (s, 1H), 8.12 (s, 1H), 7.89-7.82 (m, 2H), 7.48 (t, *J* = 7.8 Hz, 1H), 7.42-7.38 (m, 1H), 7.08 (d, *J* = 7.1 Hz, 1H), 7.06-7.00 (m, 2H), 6.87 (d, *J* = 8.5 Hz, 1H), 6.52 (t, *J* = 5.8 Hz, 1H), 6.40-6.33 (m, 2H), 6.22 (t, *J* = 5.5 Hz, 1H), 4.95-4.89 (m, 1H), 4.87 (d, *J* = 5.4 Hz, 2H), 3.85-3.75 (m, 2H), 3.69-3.63 (m, 2H), 3.30-3.17 (m, 6H), 2.94-2.71 (m, 3H), 2.38 (t, *J* = 7.7 Hz, 2H), 2.17-2.09 (m, 1H), 1.72-1.60 (m, 4H), 1.46-1.29 (m, 12H).

### <Example 8> Preparation of 2-(2,6-dioxopiperidin-3-yl)-4-(2-(2-(2-(4-(4-(5-(furan-2-ylmethylamino)-[1,2,4]triazolo[4,3-f]pyrimidin-8-yl)phenyl)piperazin-1-yl)-2-oxoethoxy)ethoxy)ethylamino)isoindolin-1,3-dione

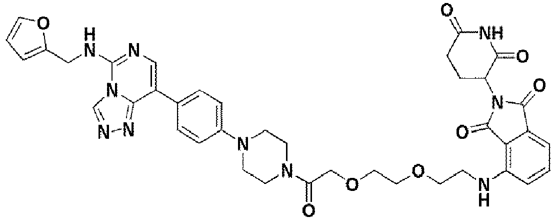

### Step 1: Preparation of tert-butyl 2-(2-(2-hydroxyethoxy)ethoxy)acetate

A solution of diethylene glycol (2.0 g, 18.86 mmol), tert-butyl bromoacetate (2.76 mL, 18.86 mmol) and NaH (225 mg, 5.66 mmol) in THF (30 mL) was stirred from 0 °C to room temperature for 18 h. The reaction mixture was diluted with brine and extracted with EtOAc. The combined organic phases were dried over Na2SO4, filtered, concentrated, and the resulting residue was purified by silica chromatography (0-70 % gradient of EtOAc/hexane) to give the title compound (946 mg, 9.42 mmol, 22 %).

¹H NMR (300 MHz, CDCl₃) δ 4.02 (d, J = 1.2 Hz, 2H), 3.80-3.67 (m, 7H), 3.65-3.60 (m, 2H), 1.48 (d, J = 1.2 Hz, 9H).

### Step 2: Preparation of tert-butyl 2-(2-(2-(tosyloxy)ethoxy)ethoxy)acetate

A solution of the compound prepared in step 1 above (946 mg, 4.29 mmol), TsCl (1.07 mg, 5.58 mmol) and TEA (66 mL, 4.72 mmol) in DCM (13 mL) was stirred at room temperature for 12 h. The reaction mixture was diluted with brine and extracted with EtOAc. The combined organic phases were dried over Na2SO4, filtered, concentrated, and the resulting residue was purified by silica chromatography (0-30 % gradient of EtOAc/hexane) to give the title compound (214 mg, 0.57 mmol, 41 %).

¹H NMR (300 MHz, CDCL₃) δ 7.83-7.78 (m, 2H), 7.34 (d, J = 8.6 Hz, 2H), 4.19-4.14 (m, 2H), 3.98 (s, 2H), 3.73-3.68 (m, 2H), 3.68-3.59 (m, 4H), 2.45 (s, 3H), 1.47 (s, 9H).

### Step 3: Preparation of tert-butyl 2-(2-(2-iodoethoxy)ethoxy)acetate

A solution of the compound prepared in step 2 above (654 mg, 1.75 mmol) and NaI (524 mg, 3.49 mmol) in acetone (10 mL) was refluxed with N2 for 2 h. The reaction solvent was removed. The reaction mixture was then diluted with water and extracted with DCM. The combined organic phases were combined, washed with brine, dried over Na2SO4 and the solvent removed in vacuo to give the desired compound (143 mg, 0.43 mmol, 76%).

¹H NMR (300 MHz, CDCl₃) δ 4.04 (s, 2H), 3.81-3.75 (m, 2H), 3.75-3.68 (m, 4H), 3.31-3.24 (m, 2H), 1.48 (s, 9H).

Step 4: Preparation of tert-butyl 2-(2-(2- 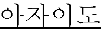 ethoxy)ethoxy)acetate

A solution of the compound prepared in step 3 above (600 mg, 1.82 mmol) and NaN3 (472 mg, 7.27 mmol) in DMF (5 mL) was stirred at room temperature for 16 h. The reaction was quenched with water and then extracted with EA. The organic layer was dried over Na2SO4 and the solvent was removed in vacuo to give the desired compound (304 mg, 1.23 mmol).

¹H NMR (300 MHz, CDCl₃) δ 4.03 (s, 2H), 3.76-3.71 (m, 4H), 3.71-3.66 (m, 2H), 3.41 (t, J = 5.1 Hz, 2H), 1.48 (s, 9H).

### Step 5: Preparation of tert-butyl 2-(2-(2-aminoethoxy)ethoxy)acetate

A solution of the compound prepared in step 4 above (304 mg, 1.24 mmol) and Pd(OH)2/C (70 mg) in EtOH (27 mL) was stirred at room temperature for 8 hours. The reaction mixture was stirred at room temperature under a hydrogen gas environment. The solution was filtered through Celite and the solvent was dried under vacuum to give the desired compound (200 mg, 0.912 mmol, 74 %).

¹H NMR (300 MHz, CDCl₃) δ 4.03 (s, 2H), 3.75-3.65 (m, 4H), 3.52 (t, J = 5.3 Hz, 2H), 2.87 (t, J = 5.2 Hz, 2H), 1.48 (s, 9H).

### Step 6: Preparation of tert-butyl 2-(2-(2-((2-(2,6-dioxopiperidin-3-yl)-1,3-dioxoisoindolin-4-yl)amino)ethoxy)ethoxy)acetate

The compound prepared in step 5 (510 mg, 0.88 mmol), 2-(2,6-dioxopiperidin-3-yl)-4-fluoroisoindolin-1,3-dione (243 mg, 0.88 mmol) and DIPEA (0.4 mL) , 2.20 mmol) in DMF (3.5 mL) was stirred at 90 °C for 16 hours. After cooling to room temperature, the reaction mixture was poured into H 2 O and the solid formed was filtered off. The solid was further purified by silica chromatography (EtOAc/hexane 0 to 50 % gradient) to obtain the target compound (220 mg, 0.462 mmol).

¹H NMR (300 MHz, CDCl₃) δ 8.12 (s, 1H), 7.49 (dd, J = 8.5, 7.1 Hz, 1H), 7.10 (dd, J = 7.1, 0.7 Hz, 1H), 6.94 (d, J = 8.2 Hz, 1H), 6.49 (t, J = 5.6 Hz, 1H), 4.94-4.85 (m, 1H), 4.03 (s, 2H), 3.81-3.62 (m, 6H), 3.49 (q, J = 5.6 Hz, 2H), 2.88-2.65 (m, 3H), 2.18-2.04 (m, 1H), 1.47 (s, 9H).

### Step 7: Preparation of 2-(2-(2-((2-(2,6-dioxopiperidin-3-yl)-1,3-dioxoisoindolin-4-yl)amino)ethoxy)ethoxy)acetate

A solution of the compound prepared in step 6 (220 0.46 mmol) and TFA (4 mL) in DCM (6 mL) was stirred for 3 hours. Then, the solvent was removed in vacuo to give the title compound (120 mg, 0.28 mmol).

¹H NMR (300 MHz, CDCl₃) δ 9.01 (s, 1H), 7.55-7.48 (m, 1H), 7.13 (d, J = 7.1 Hz, 1H), 6.93 (d, J = 8.1 Hz, 1H), 5.02-4.88 (m, 1H), 4.19 (d, J = 1.0 Hz, 2H), 3.80-3.73 (m, 6H), 3.50 (t, J = 5.3 Hz, 2H), 2.93-2.70 (m, 3H), 2.22-2.06 (m, 1H).

### Step 8: Preparation of 2-(2,6-dioxopiperidin-3-yl)-4-(2-(2-(2-(4-(4-(5-(furan-2-ylmethylamino)-[1,2,4]triazolo[4,3-f]pyrimidin-8-yl)phenyl)piperazin-1-yl)-2-oxoethoxy)ethoxy)ethylamino)isoindolin-1,3-dione

N-(furan-2-ylmethyl)-8-(4-(piperazin-1-yl)phenyl)-[1,2,4]triazolo[4,3-f]pyrimidin-5-amine hydrochloride in DMF (15mg , 0.036 mmol), the compound prepared in step 7 (15 mg, 0.036 mmol), HATU (20 mg, 0.054 mmol), and DIPEA (0.019 mL, 0.108 mmol) were added and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 6 % to give the final desired compound (4 mg, 0.005 mmol, 14 %) as a yellow solid.

¹H NMR (300 MHz, CDCl₃) δ 8.74 (s, 1H), 8.34 (s, 1H), 8.12 (s, 1H), 7.89-7.79 (m, 2H), 7.55-7.46 (m, 1H), 7.46-7.41 (m, 1H), 7.15-7.08 (m, 1H), 7.08-7.01 (m, 2H), 6.92 (d, J = 8.5 Hz, 1H), 6.60-6.45 (m, 2H), 6.44-6.35 (m, 2H), 4.93-4.88 (m, 3H), 4.86 (d, J = 5.7 Hz, 1H), 4.30 (d, J = 1.7 Hz, 2H), 3.84-3.66 (m, 10H), 3.49 (q, J = 5.3 Hz, 2H), 3.36-3.15 (m, 4H), 2.91-2.63 (m, 3H), 2.17-1.99 (m, 1H) ; LC/MS (ESI) m/z 777.0 [M+H]⁺, 775.0 [M-H]⁻

### <Example 9> Preparation of 2-(2,6-dioxopiperidin-3-yl)-4-(2-(2-(3-(4-(4-(5-(furan-2-ylmethylamino)-[1,2,4]triazolo[4,3-f]pyrimidin-8-yl)phenyl)piperazin-1-yl)-3-oxopropoxy)ethoxy)ethylamino)isoindolin-1,3-dione

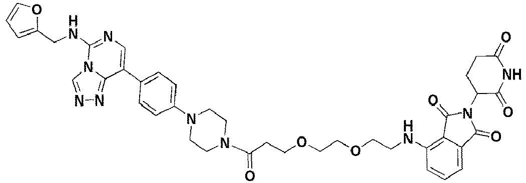

### Step 1: Preparation of tert-butyl 3-((5-hydroxypentyl)oxy)propanoate

A solution of diethylene glycol (2.0 g, 18.86 mmol), tert-butyl acrylate (2.76 mL, 18.86 mmol) and NaH (225 mg, 5.66 mmol) in THF (30 mL) was stirred at 0 °C for 18 h. The reaction mixture was diluted with brine and extracted with EtOAc. The combined organic phases were dried over Na2SO4, filtered, concentrated, and the resulting residue was purified by silica chromatography (0-70% gradient of EtOAc/hexane) to give the title compound (900 mg, mixture).

### Step 2: Preparation of tert-butyl 3-(2-(2-(tosyloxy)ethoxy)ethoxy)propane

To a solution of the compound prepared in step 1 above (946 mg, 4.29 mmol) in DCM (6 mL) was added TsCl (478 mg, 2.50 mmol) and TEA (0.30 mL, 2.11 mmol) and stirred at room temperature for 12 hours.. The reaction mixture was diluted with brine and extracted with EtOAc. The combined organic phases were dried over Na2SO4, filtered, concentrated, and the resulting residue was purified by silica chromatography (Gradient from 0 to 30% EtOAc/hexane) to the desired compound (900 mg, 2.08 mmol, 37% for 2 steps) ) was obtained.

¹H NMR (300 MHz, CDCl₃) δ 7.80 (d, J = 8.4 Hz, 2H), 7.34 (d, J = 8.0 Hz, 2H), 4.18-4.10 (m, 2H), 3.72-3.62 (m, 4H), 3.55 (q, J = 1.4 Hz, 4H), 2.48 (t, J = 6.5 Hz, 2H), 2.45 (s, 3H), 1.44 (s, 9H).

### Step 3: Preparation of tert-butyl 3-(2-(2-iodoethoxy)ethoxy)propanoate

A solution of the compound prepared in step 2 above (900 mg, 2.32 mmol) and NaI (2.1 g, 13.90 mmol) in acetone (40 mL) was refluxed with N2 for 4 h. The reaction solvent was removed. The reaction mixture was then diluted with water and extracted with DCM. The combined organic phases were combined, washed with brine, dried over Na2SO4 and the solvent removed in vacuo to give the title compound (700 mg, 2.03 mmol).

¹H NMR (300 MHz, CDCl₃) δ 3.79-3.68 (m, 4H), 3.68-3.59 (m, 4H), 3.29-3.21 (m, 2H), 2.51 (t, J = 6.5 Hz, 2H), 1.45 (s, 9H).

### Step 4: Preparation of tert-butyl 3-(2-(2-azidoethoxy)ethoxy)propanoate

A solution of the compound prepared in step 3 above (700 mg, 2.03 mmol) and NaN3 (529 mg, 8.14 mmol) in DMF (10 mL) was stirred under reflux with N2 for 16 h. The reaction solvent was removed. The reaction mixture was then diluted with water and extracted with DCM. The combined organic phases were combined, washed with brine, dried over Na2SO4 and the solvent removed in vacuo to give the title compound (478 mg, 1.84 mmol).

¹H NMR (300 MHz, CDCl₃) δ 3.73 (t, J = 6.5 Hz, 2H), 3.69-3.59 (m, 6H), 3.39 (t, J = 5.1 Hz, 2H), 2.51 (t, J = 6.5 Hz, 2H), 1.45 (s, 9H).

### Step 5: Preparation of tert-butyl 3-(2-(2-aminoethoxy)ethoxy)propanoate

A solution of the compound prepared in step 4 above (80 mg, 1.24 mmol) and Pd(OH)2/C (20 mg) in EtOH (2 mL) was stirred at room temperature for 8 hours. The reaction mixture was stirred at room temperature under a hydrogen gas environment. The solution was filtered through celite and the solvent was dried under vacuum to give the title compound (304 mg, 1.30 mmol, 71%).

¹H NMR (300 MHz, CDCl₃) δ 3.72 (t, J = 6.5 Hz, 2H), 3.61 (s, 4H), 3.50 (t, J = 5.3 Hz, 2H), 2.86 (t, J = 5.3 Hz, 2H), 2.51 (t, J = 6.5 Hz, 2H), 1.45 (s, 9H).

### Step 6: Preparation of tert-butyl 3-(2-(2-((2-(2,6-dioxopiperidin-3-yl)-1,3-dioxoisoindolin-4-yl)amino)ethoxy)ethoxy)propanoate

The compound prepared in step 5 (270 mg, 0.88 mmol), 2-(2,6-dioxopiperidin-3-yl)-4-fluoroisoindolin-1,3-dione (243 mg, 0.88 mmol) and DIPEA (0.4 mL) , 2.20 mmol) in DMF (3.5 mL) was stirred at 90 °C for 16 hours. After cooling to room temperature, the reaction mixture was poured into H 2 O and the solid formed was filtered off. The solid was further purified by silica chromatography (EtOAc/hexane 0 to 30 % gradient) to obtain the target compound (35 mg, 0.071 mmol).

¹H NMR (300 MHz, CDCl₃) δ 8.08 (s, 1H), 7.54-7.46 (m, 1H), 7.11 (dd, J = 7.1, 0.6 Hz, 1H), 6.92 (d, J = 8.5 Hz, 1H), 6.49 (t, J = 5.5 Hz, 1H), 4.92 (dd, J = 12.1, 5.4 Hz, 1H), 3.76-3.69 (m, 4H), 3.69-3.60 (m, 4H), 3.51-3.42 (m, 2H), 2.94-2.68 (m, 3H), 2.51 (t, J = 6.6 Hz, 2H), 2.16-2.09 (m, 1H), 1.44 (s, 9H).

### Step 7: Preparation of 3-(2-(2-((2-(2,6-dioxopiperidin-3-yl)-1,3-dioxoisoindolin-4-yl)amino)ethoxy)ethoxy)propanoate

A solution of the compound prepared in step 6 above (168 0.34 mmol) and TFA (4.0 mL) in DCM (6.0 mL) was stirred overnight. Then, the solvent was removed in vacuo to give the title compound (130 mg, 0.29 mmol).

¹H NMR (300 MHz, CDCl₃) δ 8.48 (s, 1H), 7.53-7.46 (m, 1H), 7.13-7.08 (m, 1H), 6.91 (d, J = 8.0 Hz, 1H), 4.97-4.88 (m, 1H), 3.98 (s, 1H), 3.82-3.64 (m, 9H), 3.52-3.41 (m, 3H), 2.91-2.69 (m, 3H), 2.64 (t, J = 6.3 Hz, 2H), 2.19-2.10 (m, 1H).

### Step 8: Preparation of 2-(2,6-dioxopiperidin-3-yl)-4-(2-(2-(3-(4-(4-(5-(furan-2-ylmethylamino)-[1,2,4]triazolo[4,3-f]pyrimidin-8-yl)phenyl)piperazin-1-yl)-3-oxopropoxy)ethoxy)ethylamino)isoindolin-1,3-dione

N-(furan-2-ylmethyl)-8-(4-(piperazin-1-yl)phenyl)-[1,2,4]triazolo[4,3-f]pyrimidin-5-amine hydrochloride in DMF (15mg , 0.036 mmol), the compound prepared in step 7 (15 mg, 0.036 mmol), HATU (20 mg, 0.054 mmol), and DIPEA (0.019 mL, 0.108 mmol) were added and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 6 % to give the final desired compound (2 mg, 0.003 mmol, 8 %) as a yellow solid.

¹H NMR (300 MHz, CDC13) δ 8.43 (s, 1H), 8.31 (s, 1H), 8.11 (s, 1H), 7.88-7.81 (m, 2H), 7.52-7.44 (m, 1H), 7.43-7.39 (m, 1H), 7.09 (d, J = 7.1 Hz, 1H), 7.08-6.97 (m, 2H), 6.90 (d, J = 8.4 Hz, 1H), 6.55-6.42 (m, 2H), 6.41-6.34 (m, 2H), 4.94-4.90 (m, 1H), 4.88 (d, J = 5.8 Hz, 2H), 3.89-3.77 (m, 4H), 3.68 (d, J = 14.1 Hz, 9H), 3.45 (q, J = 5.7 Hz, 3H), 3.30-3.16 (m, 4H), 2.92-2.59 (m, 6H), 2.19-2.07 (m, 1H) ; LC/MS (ESI) m/z 791.0 [M+H]⁺, 788.9 [M-H]⁻

### <Example 10> Preparation of 2-(2,6-dioxopiperidin-3-yl)-4-(2-(2-(2-(2-(4-(4-(5-(furan-2-ylmethylamino)-[1,2,4]triazolo[4,3-f]pyrimidin-8-yl)phenyl)piperazin-1-yl)-2-oxoethoxy)ethoxy)ethoxy)ethylamino)isoindolin-1,3-dione

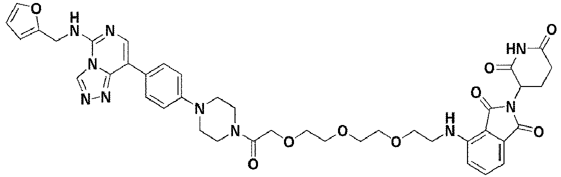

### Step 1: Preparation of tert-butyl 2-(2-(2-(2-chloroethoxy)ethoxy)ethoxy)acetate

Potassium tert-butoxide (5.01) in a solution of 2-(2-(2-chloroethoxy)ethoxy)ethanol (5 g, 29.8 mmol), tert-butylbromoacetate (4.40 mL, 29.8 mmol) in THF (50 mL) at 0 °C g, 44.6 mmol) and the reaction mixture was stirred at room temperature overnight. The reaction mixture was diluted with water and extracted with EtOAc (200 mL×2). The combined organic layers were dried over MgSO4 and the solvent was removed in vacuo to give a yellow oil. The crude mixture was purified by silica gel column chromatography using EA/Hx (20 %) as eluent to give the title compound (1.81 g, 6.40 mmol, 22 %) as a colorless oil.

¹H NMR (300 MHz, CDCl₃) δ 4.04 (s, 1H), 3.79-3.62 (m, J = 7.5 Hz, 12H), 1.49 (s, 9H)

### Step 2: Preparation of tert-butyl 2-(2-(2-(2-azidoethoxy)ethoxy)ethoxy)acetate

To a DMF (4 mL) solution of the compound (300 mg, 1.06 mmol) prepared in step 1 above, sodium azide (552 mg, 8.49 mmol) was added at room temperature, and the reaction mixture was stirred at 120 °C for 3 hours.. The reaction mixture was diluted with water and extracted with EtOAc (50 mL×2). The combined organic layers were dried over MgSO4 and the solvent was removed in vacuo to give a yellow oil. The crude mixture was purified by silica gel column chromatography using EA/Hx (20 %) as eluent to give the title compound (287 mg, 0.992 mmol, 93 %) as a colorless oil.

¹H NMR (300 MHz, CDCl₃) δ 4.03 (s, 2H), 3.78-3.60 (m, 10H), 3.39 (t, J = 5.1 Hz, 2H), 1.48 (s, 9H).

### Step 3: Preparation of tert-butyl 2-(2-(2-(2-aminoethoxy)ethoxy)ethoxy)acetate

To a solution of the compound prepared in step 2 above (200 mg, 0.691 mmol) in THF (3 mL)-H2O (0.03 mL) at room temperature was added triphenyl phosphine (272 mg, 1.04 mmol), and the reaction mixture was stirred at room temperature. stirred overnight. The reaction mixture was concentrated under reduced pressure to remove the solvent, and the residue was diluted with water and then extracted with DCM (50 mL×2). The combined organic layers were dried over MgSO4 and the solvent was removed in vacuo to give a yellow oil. The crude mixture was purified by silica gel column chromatography using MeOH/DCM (7 %) as eluent to give the title compound (85 mg, 0.323 mmol, 47 %) as a brown oil.

¹H NMR (300 MHz, CDC13) δ 3.92 (s, 2H), 3.68-3.49 (m, 8H), 3.43 (t, J = 5.2 Hz, 2H), 2.78 (t, J = 5.2 Hz, 2H), 2.43 (s, 2H), 1.36 (s, 9H).

### Step 4: Preparation of tert-butyl 2-(2-(2-(2-((2-(2,6-dioxopiperidin-3-yl)-1,3-dioxoisoindolin-4-yl)amino)ethoxy)ethoxy)ethoxy)acetate

Compound prepared in step 3 above (85 mg, 0.323 mmol), 2-(2,6-dioxopiperidin-3-yl)-4-fluoroisoindolin-1,3-dione (107 mg, 0.387 mmol) in DMF (1 mL)) was added DIPEA (0.084 mL, 0.485 mmol). The reaction mixture was stirred at 90 °C overnight. The reaction mixture was diluted with water and extracted with EtOAc (50 mL×2). The combined organic layers were washed with brine, dried over MgSO4 and the solvent removed in vacuo to give an oil. The crude mixture was purified by silica gel column chromatography using EtOAc/Hex 40 % as eluent to give the title compound (55 mg, 0.106 mmol, 33 %) as a yellow solid.

¹H NMR (300 MHz, CDCl₃) δ 8.48 (s, 1H), 7.49 (dd, J = 8.5, 7.2 Hz, 1H), 7.09 (d, J = 7.1 Hz, 1H), 6.92 (d, J = 8.5 Hz, 1H), 6.49 (t, J = 5.7 Hz, 1H), 4.97-4.87 (m, 1H), 4.02 (s, 2H), 3.77-3.60 (m, 10H), 3.52-3.42 (m, 2H), 2.93-2.66 (m, 3H), 2.18-2.07 (m, 1H), 1.47 (s, 9H).

### Step 5: Preparation of tert-butyl 2-(2-(2-(2-((2-(2,6-dioxopiperidin-3-yl)-1,3-dioxoisoindolin-4-yl)amino)ethoxy)ethoxy)ethoxy)acetate

To a solution of the compound prepared in step 4 above (55 mg, 0.323 mmol) in DCM (3 mL) was added TFA (2 mL). The reaction mixture was stirred at room temperature for 1 hour. The reaction mixture was concentrated in vacuo to give the desired compound (50 mg, 0.106 mmol, quant.) as an oil.

¹H NMR (300 MHz, CDCl₃) δ 8.63 (s, 1H), 7.51 (dd, J = 8.5, 7.2 Hz, 1H), 7.13 (d, J = 7.1 Hz, 1H), 6.94 (d, J = 8.6 Hz, 1H), 5.22 (s, 1H), 5.00-4.91 (m, 1H), 4.17 (s, 2H), 3.82-3.67 (m, 10H), 3.50 (t, J = 5.2 Hz, 2H), 2.97-2.74 (m, 3H), 2.20-2.11 (m, 1H).

### Step 6: Preparation of tert-butyl 2-(2-(2-(2-((2-(2,6-dioxopiperidin-3-yl)-1,3-dioxoisoindolin-4-yl)amino)ethoxy)ethoxy)ethoxy)acetate

N-(furan-2-ylmethyl)-8-(4-(piperazin-1-yl)phenyl)-[1,2,4]triazolo[4,3-f]pyrimidin-5-amine hydrochloride (11 mg) in DMF , 0.026 mmol), the compound prepared in step 5 (12 mg, 0.026 mmol), HATU (15 mg, 0.039 mmol), and DIPEA (0.018 mL, 0.104 mmol) were stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 6 % to give the final desired compound (4 mg, 0.005 mmol, 18 %) as a yellow solid.

¹H NMR (300 MHz, CDCl₃) δ 8.31 (s, 1H), 8.13-8.08 (m, 2H), 7.84 (d, J = 8.6 Hz, 2H), 7.51-7.44 (m, 1H), 7.42-7.40 (m, 1H), 7.09 (d, J = 7.0 Hz, 1H), 7.02 (d, J = 8.6 Hz, 2H), 6.93-6.81 (m, 1H), 6.55-6.43 (m, 2H), 6.41-6.33 (m, 2H), 4.95-4.90 (m, 1H), 4.87 (d, J = 6.0 Hz, 2H), 4.28 (d, J = 5.5 Hz, 2H), 3.77-3.65 (m, 14H), 3.42 (q, J = 5.6 Hz, 2H), 3.30-3.16 (m, 4H), 2.91-2.68 (m, 3H), 2.15-2.08 (m, 1H); LC/MS (ESI) m/z 821.0 [M+H]⁺, 818.9 [M-H]⁻

### <Example 11> Preparation of 2-(2,6-dioxopiperidin-3-yl)-4-(20-(4-(4-(5-(furan-2-ylmethylamino)-[1,2,4]triazolo[4,3-f]pyrimidin-8-yl)phenyl)piperazin-1-yl)-20-oxo-3,6,9,12,15,18-hexaoxicosylamino)isoindolin-1,3-dione

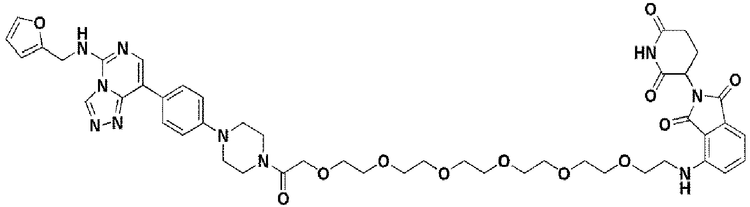

### Step 1: Preparation of 1-phenyl-2,5,8,11-tetraoxatridecane-13-yl-4-methylbenzensulfonate

To a solution of tetraethylene glycol monobenzyl ether (3 g, 10.55 mmol) in DCM (50 ml) was added TEA (4.41 ml, 31.65 mmol), DMAP (128 mg, 1.05 mmol) at room temperature, followed by p-TsCl (4.02) g, 21.10 mmol) was added at 0 °C. The reaction mixture was stirred at room temperature overnight. The reaction mixture was diluted with water and then extracted with DCM (50 mL×2). The combined organic layers were dried over MgSO4 and the solvent was removed in vacuo. The crude mixture was purified by silica gel column chromatography using (EtOAc/Hx = 45 %) as eluent to give the title compound (4.50 g, 10.26 mmol, 97 %) as a clear oil.

¹H NMR (300 MHz, CDCl₃) δ 7.83-7.76 (m, 2H), 7.34 (d, J = 4.4 Hz, 5H), 7.32-7.24 (m, 2H), 4.56 (s, 2H), 4.18-4.12 (m, 2H), 3.72-3.64 (m, 5H), 3.64-3.55 (m, 9H), 2.44 (s, 3H).

### Step 2: Preparation of 1-phenyl-2,5,8,11,14,17-hexaoxanonadecane-19-ol

To a solution of the compound (3 g, 10.26 mmol) prepared in step 1 in diethylene glycol (50 ml), NaOH (1.23 g, 30.78 mmol) was added at room temperature. The reaction mixture was stirred at 60 °C for 3 h. The reaction mixture was diluted with water and then extracted with DCM (50 mL×5). The combined organic layers were dried over MgSO4 and the solvent was removed in vacuo. The crude mixture was purified by silica gel column chromatography using EtOAc as eluent (DCM/MeOH = 5 %) to give the title compound (2.41 g, 6.47 mmol, 63 %) as a clear oil.

¹H NMR (300MHz, CDCl₃) δ 7.33 (d, J = 4.5 Hz, 4H), 7.31-7.22 (m, 1H), 4.55 (s, 2H), 3.76-3.57 (m, 22H), 3.60-3.54 (m, 2H), 3.20 (s, 1H).

### Step 3: Preparation of tert-butyl 1-phenyl-2,5,8,11,14,17,20-heptaoxadocosan-22-oate

To a solution of the compound prepared in step 2 above (2.60 g, 6.98 mmol) in DCM (15 ml) was added 37% aq. NaOH (15 ml), t-butylbromoacetate (4.12 ml, 27.9 mmol), TBAB (2.30 g, 7.12 mmol) were added at room temperature. The reaction mixture was stirred at room temperature overnight. The reaction mixture was diluted with water and extracted with EtOAc (50 mL×2). The combined organic layers were dried over MgSO4 and the solvent was removed in vacuo. The crude mixture was purified by silica gel column chromatography using (EtOAc/Hx = 60 %) as eluent to give the title compound (1.81 g, 3.72 mmol, 53 %) as a clear oil.

¹H NMR (300 MHz, CDCl₃) δ 7.34 (d, J = 4.4 Hz, 4H), 7.32-7.24 (m, 1H), 4.57 (s, 2H), 4.02 (s, 2H), 3.73-3.68 (m, 4H), 3.67-3.60 (m, 20H), 1.47 (s, 9H).

### Step 4: Preparation of tert-butyl 20-hydroxy-3,6,9,12,15,18-hexaoxicosanoate

To a solution of the compound prepared in step 3 above (1.81 g, 3.72 mmol) in EtOH (50 ml) was added Pd/C (10 wt %) (156 mg) and the resulting mixture was placed under hydrogen. The reaction mixture was stirred at room temperature overnight. The reaction mixture was filtered through Celite, and the Celite pad was washed several times with ethanol. The filtrate was concentrated in vacuo to give the desired compound (1.39 g, 3.50 mmol, 94 %) as an oil.

¹H NMR (300 MHz, CDCl₃) δ 4.02 (s, 2H), 3.77-3.58 (m, 24H), 2.65 (t, J = 6.2 Hz, 1H), 1.48 (s, 9H).

### Step 5: Preparation of tert-butyl 20-(tosyloxy)-3,6,9,12,15,18-hexaoxicosanoate

To a solution of the compound prepared in step 4 (500 mg, 1.26 mmol) in DCM (10 ml) at room temperature, TEA (0.527 ml, 3.78 mmol) and DMAP (15 mg, 0.13 mmol) were added, followed by TsCl ( 480 mg, 2.52 mmol) was added at 0 °C. The reaction mixture was stirred at room temperature overnight. The reaction mixture was diluted with water and then extracted with DCM (50 mL×2). The combined organic layers were dried over MgSO4 and the solvent was removed in vacuo. The crude mixture was purified by silica gel column chromatography using (MeOH/DCM = 5 %) as eluent to give the title compound (643 mg, 1.16 mmol, 92 %) as a brown oil.

¹H NMR (300 MHz, CDCl₃) δ 7.83-7.76 (m, 2H), 7.34 (d, J = 7.8 Hz, 2H), 4.16 (t, J = 4.8 Hz, 2H), 4.02 (s, 2H), 3.72-3.61 (m, 18H), 3.58 (s, 4H), 2.45 (s, 3H), 1.47 (s, 9H).

### Step 6: Preparation of tert-butyl 20-iodo-3,6,9,12,15,18-hexaoxicosanoate

To a solution of the compound prepared in step 5 above (643 mg, 1.17 mmol) in acetone (10 ml) was added sodium iodide (700 mg, 4.67 mmol) at room temperature. The reaction mixture was stirred at reflux overnight. The reaction mixture was diluted with water and extracted with EtOAc (50 mL×2). The combined organic layers were dried over MgSO4 and the solvent was removed in vacuo. The crude mixture was purified by silica gel column chromatography using (EtOAc/Hx = 55 %) as eluent to give the title compound (413 mg, 0.815 mmol, 70 %) as a yellow transparent oil.

¹H NMR (300MHz, CDCl₃) δ 4.02 (s, 2H), 3.76 (t, J = 6.9 Hz, 2H), 3.73-3.68 (m, 4H), 3.68-3.63 (m, 16H), 3.26 (t, J = 6.9 Hz, 2H), 1.48 (s, 9H).

### Step 7: Preparation of tert-butyl 20-azido-3,6,9,12,15,18-hexaoxicosanoate

To a solution of the compound prepared in step 6 above (400 mg, 0.790 mmol) in DMF (5 ml) was added sodium azide (77 mg, 1.18 mmol) at room temperature. The reaction mixture was stirred at 50 °C for 2 h. The reaction mixture was diluted with water and extracted with EtOAc (50 mL×2). The combined organic layers were dried over MgSO4 and the solvent was removed in vacuo to give the title compound (110 mg, 0.260 mmol, 33 %) as a yellow transparent oil.

¹H NMR (300MHz, CDCl₃) δ 4.03 (s, 2H), 3.76-3.70 (m, 4H), 3.70-3.60 (m, 18H), 3.40 (t, J = 5.1 Hz, 2H), 1.48 (s, 9H).

### Step 8: Preparation of tert-butyl 20-amino-3,6,9,12,15,18-hexaoxicosanoate

To a solution of the compound prepared in step 7 above (96 mg, 0.227 mmol) in EtOH (5 ml) was added Pd(OH)2 (24 mg), and the resulting mixture was placed under hydrogen. The reaction mixture was stirred at room temperature overnight. The reaction mixture was filtered through Celite, and the Celite pad was washed several times with ethanol. The filtrate was concentrated in vacuo to give the title compound (100 mg, crude) as an oil.

### Step 9: Preparation of tert-butyl 20-((2-(2,6-dioxopiperidin-3-yl)-1,3-dioxoisoindolin-4-yl)amino )-3,6,9,12,15, 18-hexaoxicosanoate

To a solution of the compound (40 mg, 0.101 mmol) prepared in step 8 above in DMF, 2-(2,6-dioxopiperidin-3-yl)-4-fluoroisoindolin-1,3-dione (25 mg, 0.050 mmol), DIPEA (0.026 mL, 0.151 mmol) was added and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 5 % to give the title compound (17 mg, 0.026 mmol, 25 %) as a yellow solid.

¹H NMR (300 MHz, CDCl₃) δ 8.32 (s, 1H), 7.54-7.46 (m, 1H), 7.11 (d, J = 7.1 Hz, 1H), 6.92 (d, J = 8.5 Hz, 1H), 6.50 (t, J = 5.6 Hz, 1H), 4.97-4.86 (m, 1H), 4.05-3.99 (m, 2H), 3.75-3.60 (m, 22H), 3.47 (q, J = 5.5 Hz, 2H), 2.97-2.70 (m, 3H), 2.19-2.07 (m, 1H), 1.47 (s, 9H).

### Step 10: Preparation of 20-((2-(2,6-dioxopiperidin-3-yl)-1,3-dioxoisoindolin-4-yl)amino)-3,6,9,12,15, 18-hexaoxaicosanoate

To a solution of the compound prepared in step 9 above (17 mg, 0.026 mmol) in DCM (1 ml) was added TFA (1 ml), and the resulting mixture was stirred at room temperature for 1 hour. The reaction mixture was concentrated in vacuo to give the title compound (20 mg, quant) as an oil.

¹H NMR (300 MHz, CDCl₃) δ 8.75 (s, 1H), 7.50 (t, J = 7.8 Hz, 1H), 7.11 (d, J = 7.1 Hz, 1H), 6.93 (d, J = 8.5 Hz, 1H), 5.00-4.85 (m, 1H), 4.18 (s, 2H), 3.81-3.57 (m, 22H), 3.47 (t, J = 5.4 Hz, 2H), 2.96-2.68 (m, 3H), 2.20-2.00 (m, 1H).

### Step 11: Preparation of 2-(2,6-dioxopiperidin-3-yl)-4-(20-(4-(4-(5-(furan-2-ylmethylamino)-[1,2,4]triazolo[4,3-f]pyrimidin-8-yl)phenyl)piperazin-1-yl)-20-oxo-3,6,9,12,15,18-hexaoxicosylamino)isoindolin-1,3-dione

N-(furan-2-ylmethyl)-8-(4-(piperazin-1-yl)phenyl)-[1,2,4]triazolo[4,3-f]pyrimidin-5-amine hydrochloride (11 mg) in DMF , 0.026mmol), the compound prepared in step 10 (15mg, 0.026mmol), HATU (15mg, 0.039mmol), and DIPEA (0.018mL, 0.104mmol) were added and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 5 % to give the title compound (1.6 mg, 0.001 mmol, 6 %) as a green solid.

¹H NMR (500 MHz, CDCl₃) δ 8.33 (s, 1H), 8.12 (s, 1H), 7.85 (d, J = 7.8 Hz, 2H), 7.52-7.45 (m, 1H), 7.43-7.38 (m, 1H), 7.13-7.07 (m, 2H), 6.90 (d, J = 8.4 Hz, 1H), 6.49 (t, J = 5.9 Hz, 1H), 6.41-6.34 (m, 2H), 4.95-4.90 (m, 1H), 4.88 (d, J = 5.7 Hz, 2H), 4.29 (s, 2H), 3.81 (s, 2H), 3.78-3.55 (m, 22H), 3.45 (s, 2H), 3.35-3.21 (m, 4H), 2.91-2.68 (m, 3H), 2.07-1.95 (m, 1H).

### <Example 12> Preparation of 2-(2-(2,6-dioxopiperidin-3-yl)-1,3-dioxoisoindolin-4-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide

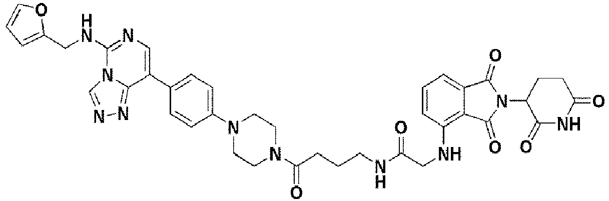

### Step 1: Preparation of ((tert-butoxycarbonyl)amino)butanoate

1,4-dioxane: To a solution of 4-aminobutane acid (500 mg, 4.84 mmol) in water (2:1) (12 mL) was added 2 M NaOH (aq). The reaction mixture was then cooled to 0° C. and di-tert-butyldicarbonate (1.22 mL, 5.33 mmol) was added dropwise. The reaction mixture was stirred at room temperature overnight. The solvent was evaporated in vacuo, acidified to pH 2 with 1M HCl and extracted with EtOAc. The combined organic layers were dried over MgSO4 and the solvent was removed in vacuo to give an oil. The crude mixture was purified by silica gel column chromatography using DCM / MeOH = 6 % to give the title compound (1.09 g, quant) as a clear oil.

¹H NMR (300 MHz, CDCl₃) δ10.29 (s, 1H), 4.77 (s, 1H), 3.31-3.03 (m, 2H), 2.39 (t, *J* = 7.2 Hz, 2H), 1.82 (p, *J* = 7.0 Hz, 2H), 1.44 (s, 9H).

### Step 2: Preparation of tert-butyl (4-(4-(4-(5-((furan-2-ylmethyl)amino)-[1,2,4]triazolo[4,3-c]pyrimidin-8-yl)phenyl)piperazin-1-yl)-4-oxobutyl)carbamate

N-(furan-2-ylmethyl)-8-(4-(piperazin-1-yl)phenyl)-[1,2,4]triazolo[4,3-f]pyrimidin-5-amine hydrochloride in DMF (212 mg, 0.514 mmol), the compound prepared in step 1 (105 mg, 0.514 mmol), HATU (293 mg, 0.772 mmol), and DIPEA (0.538 mL, 2.06 mmol) were added and stirred at room temperature overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc / Hex = 55 % to give the title compound (196 mg, 0.349 mmol, 68 %) as a beige solid.

¹H NMR (300 MHz, CDCl₃) δ 8.31 (s, 1H), 8.12 (s, 1H), 7.90-7.83 (m, 2H), 7.43-7.37 (m, 1H), 7.08-6.99 (m, 2H), 6.53 (t, *J* = 5.8 Hz, 1H), 6.40-6.32 (m, 2H), 4.91-4.77 (m, 3H), 3.84-3.75 (m, 2H), 3.68-3.60 (m, 2H), 3.28-3.14 (m, 6H), 2.43 (t, *J* = 7.3 Hz, 2H), 1.93-1.80 (m, 2H), 1.44 (s, 9H).

### Step 3: Preparation of 4-amino-1-(4-(4-(5-((puran-2-ylmethyl)amino)-[1,2,4]triazolo[4,3-c]pyrimidin-8-yl)phenyl)piperazine-1-yl)butane-1-on

To a solution of the compound prepared in step 2 above (196 mg, 0.349 mmol) in DCM (6 mL) was added 4 M HCl in dioxane (3 ml). The reaction mixture was stirred at room temperature for 1 hour. The reaction mixture was concentrated in vacuo to give the desired compound (150 mg, crude) as a beige solid.

¹H NMR (300 MHz, DMSO-*d₆*) δ 8.74 (t, *J* = 6.1 Hz, 1H), 8.60 (s, 1H), 8.24 (s, 1H), 8.03-7.95 (m, 2H), 7.60-7.56 (m, 1H), 7.18-7.07 (m, 2H), 6.42-6.37 (m, 1H), 6.32 (d, *J* = 3.2 Hz, 1H), 4.74 (d, *J* = 6.0 Hz, 2H), 4.38 (s, 3H), 3.73-3.59 (m, 4H), 3.33-3.16 (m, 4H), 2.88-2.78 (m, 2H), 1.80 (p, *J* = 7.4 Hz, 2H).

### Step 4: Preparation of 2-(2-(2,6-dioxopiperidin-3-yl)-1,3-dioxoisoindolin-4-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide

In a solution of 2-(2-(2,6-dioxopiperidin-3-yl)-1,3-dioxoisoindolin-4-ylamino)acetate (17 mg, 0.051 mmol) in DMF, the compound prepared in step 3 (25 mg , 0.051 mmol), EDCI HCl (11 mg, 0.057 mmol), HOBt HO (9 mg, 0.057 mmol), DIPEA (0.035 mL, 0.204 mmol) were added and stirred at room temperature for 2 h. The reaction mixture was diluted with water and extracted with EtOAc. The combined organic layers were washed with brine, dried over MgSO4 and the solvent removed in vacuo to give an oil. The crude mixture was purified on PREP TLC using MeOH / DCM = 5 % to give the title compound (10 mg, 0.013 mmol, 25 %) as a yellow solid.

¹H NMR (500 MHz, CDCl₃) δ8.65 (s, 1H), 8.31 (s, 1H), 8.11 (s, 1H), 7.84 (d, *J* = 8.3 Hz, 2H), 7.51 (t, *J* = 7.9 Hz, 1H), 7.41 (s, 1H), 7.32-7.27 (m, 1H), 7.19 (d, *J* = 7.2 Hz, 1H), 6.99 (d, *J* = 8.3Hz, 2H), 6.80 (d, *J* = 8.5 Hz, 1H), 6.72 (t, *J* = 6.2 Hz, 1H), 6.49 (t, *J* = 5.8 Hz, 1H), 6.41-6.34 (m, 2H), 4.98-4.92 (m, 1H), 4.87 (d, *J* = 5.7 Hz, 2H), 3.95 (d, *J* = 6.1 Hz, 2H), 3.72-3.63 (m, 2H), 3.59-3.50 (m, 2H), 3.33 (q, *J* = 6.6 Hz, 2H), 3.21-3.16 (m, 2H), 3.16-3.09 (m, 2H), 2.94-2.70 (m, 3H), 2.42-2.35 (m, 2H), 2.17-2.09 (m, 1H), 1.94-1.81 (m, 2H). LC/MS (ESI) m/z 774.1 [M+H]⁺, 772.1 [M-H]⁻

### <Example 13> Preparation of 2-(2-(2,6-dioxopiperidin-3-yl)-1,3-dioxoisoindolin-4-yloxy)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide

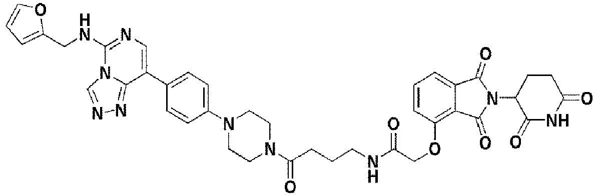

To 2-(2-(2,6-dioxopiperidin-3-yl)-1,3-dioxoisoindolin-4-yloxy)acetic acid (17 mg, 0.051 mmol) in DMF, EDCI HCl(11 mg, 0.057mmol), HOBt (9 mg, 0.057 mmol), DIPEA (0.035mL, 0.204 mmol), and 4-amino-1-(4-(4-(5-((puran-2-ylmethyl)amino)-[1,2,4]triazolo[4,3-c]pyrimidin-8-yl)phenyl)piperazine-1-yl)butane-1-on (25mg, 0.051 mmol, Prepared in example 12 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% X2), target chemical, 2-(2-(2,6-dioxopiperidin-3-yl)-1,3-dioxoisoindolin-4-yloxy)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide was obtained (7 mg, 0.009 mmol, 17% yield) as yellow solid.

¹H NMR (500 MHz, CDCl₃) δ9.26 (s, 1H), 8.31 (s, 1H), 8.11 (s, 1H), 7.88-7.82 (m, 2H), 7.75 (t, *J* = 8.0 Hz, 1H), 7.64 (t, *J* = 5.7 Hz, 1H), 7.56 (d, *J* = 7.3 Hz, 1H), 7.41 (s, 1H), 7.21 (d, *J* = 8.4 Hz, 1H), 7.10-7.03 (m, 2H), 6.48 (t, *J* = 5.9 Hz, 1H), 6.41-6.34 (m, 2H), 4.93-4.85 (m, 3H), 4.66 (q, *J* = 14.1 Hz, 2H), 4.02-3.94 (m, 1H), 3.73-3.57 (m, 3H), 3.57-3.48 (m, 1H), 3.45-3.37 (m, 1H), 3.38-3.27 (m, 2H), 3.20-3.08 (m, 2H), 2.90-2.83 (m, 1H), 2.83-2.64 (m, 2H), 2.54-2.43 (m, 2H), 2.20-2.11 (m, 1H), 2.06-1.94 (m, 2H). LC/MS (ESI) *m*/*z* 775.1 [M+H]⁺, 773.0 [M-H]⁻

### <Example 14> Preparation of 2-(2-(2,6-dioxopiperidin-3-yl)-1,3-dioxoisoindolin-4-ylamino)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide

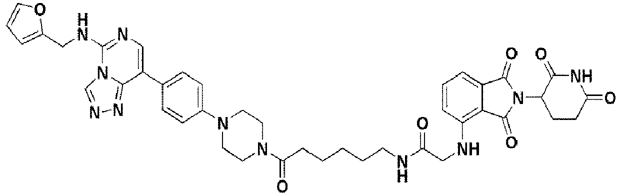

### Step 1: Preparation of tert-butyl 6-aminohexanoate

A solution of 6-aminohexane acid (1 g, 7.62 mmol) in thionyl chloride (76.2 ml, 76.2 mmol) was stirred at room temperature for 1.5 h, after which the excess thionyl chloride was removed in vacuo. A solution of NaHCO3 (1.40 g, 16.7 mmol) in t-BuOH (10 ml) was added to the residue under stirring and the reaction mixture was stirred overnight at room temperature. The volatiles were removed in vacuo and the residue was dissolved in EtOAc (30 ml) and NaOH (1M, aq) (20 ml). The combined organic layers were washed with water (30 ml) and brine (20 ml×3), dried over MgSO 4 and the solvent removed in vacuo to give the title compound (521 mg, 6.94 mmol, 91 %) as a yellow clear oil.

¹H NMR (300 MHz, CDCl₃) δ2.78 (t, *J* = 7.3 Hz, 2H), 2.21 (t, *J* = 7.4 Hz, 2H), 1.70-1.49 (m, 4H), 1.44 (s, 9H), 1.42-1.30 (m, 2H).

### Step 2: Preparation of tert-butyl 6-((2-(2,6-dioxopiperidin-3-yl)-1,3-dioxoisoindolin-4-yl)amino)hexanoate

tert-butyl 6-aminohexanoate (100 mg, 0.53 mmol), (2-(2,6-dioxopiperidin-3-yl)-1,3-dioxoisoindolin-4-yl)glycine (178 mg, 0.53 mmol), HATU (223 mg, 0.59 mmol) and DIPEA (0.23 mL, 1.33 mmol) were stirred at 90 °C for 16 h. After cooling to room temperature, the reaction mixture was poured into H 2 O and the solid formed was filtered off. This was further purified by chromatography on silica (0-10% gradient of MeOH/DCM) to give the title compound (136 mg, 0.271 mmol, 51%) as a yellow solid.

¹H NMR (300 MHz, CDCl₃) δ 8.37 (s, 1H), 7.61-7.52 (m, 1H), 7.23 (d, *J* = 6.7 Hz, 1H), 6.82 (d, *J* = 8.3 Hz, 1H), 6.68 (t, *J* = 6.0 Hz, 1H), 6.56 (t, *J* = 5.8 Hz, 1H), 4.95 (dd, *J* = 12.5, 5.3 Hz, 1H), 3.95 (d, *J* = 6.1 Hz, 2H), 3.25 (q, *J* = 6.8 Hz, 2H), 2.94-2.72 (m, 4H), 2.17 (t, *J* = 7.4 Hz, 3H), 1.60-1.46 (m, 4H), 1.43 (s, 9H).

### Step 3: Preparation of 6-(2-((2-(2,6-dioxopiperidin-3-yl)-1,3-dioxoisoindolin-4-yl)amino)acetamido)hexanoate

A solution of the compound prepared in step 2 above (136 mg, 0.271 mmol) and TFA (1 mL) in DCM (2 mL) was stirred at room temperature for 30 min. The reaction mixture was concentrated under reduced pressure to give the desired compound (130 mg, crude) as a yellow solid.

1H NMR (300MHz, CDC13) δ 4.51 (s, 1H), 3.18 (t, J = 7.0 Hz, 2H), 3.11 (q, J = 6.6 Hz, 2H), 1.82 (p, J = 7.0 Hz, 2H), 1.55-1.46 (m, 2H), 1.44 (s, 9H), 1.43-1.29 (m, 4H).

### Step 4: Preparation of 2-(2-(2,6-dioxopiperidin-3-yl)-1,3-dioxoisoindolin-4-ylamino)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide

N-(furan-2-ylmethyl)-8-(4-(piperazin-1-yl)phenyl)-[1,2,4]triazolo[4,3-f]pyrimidin-5-amine hydrochloride in DMF (20 mg, 0.048 mmol), the compound prepared in step 3 (22 mg, 0.048 mmol), HATU (27 mg, 0.073 mmol), and DIPEA (0.033 mL, 0.192 mmol) were added and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified on PREP TLC using MeOH / DCM = 5% to give the title compound (3 mg, 0.004 mmol, 12%) as a yellow solid.

¹H NMR (500 MHz, CDCl₃) δ 8.32 (s, 1H), 8.25 (s, 1H), 8.12 (s, 1H), 7.89-7.84 (m, 2H), 7.59-7.54 (m, 1H), 7.43-7.40 (m, 1H), 7.24 (d, *J* = 7.1 Hz, 1H), 7.06-7.02 (m, 2H), 6.83 (d, *J* = 8.5 Hz, 1H), 6.69 (t, *J* = 6.0 Hz, 1H), 6.61 (t, *J* = 6.0 Hz, 1H), 6.47 (t, *J* = 5.8 Hz, 1H), 6.41-6.34 (m, 2H), 4.98-4.91 (m, 1H), 4.88 (d, *J* = 5.7 Hz, 2H), 3.96 (d, *J* = 6.0 Hz, 2H), 3.82-3.75 (m, 2H), 3.68-3.59 (m, 2H), 3.31 (q, *J* = 6.7 Hz, 2H), 3.27-3.18 (m, 4H), 2.95-2.69 (m, 3H), 2.35 (t, *J* = 7.3 Hz, 2H), 2.19-2.11 (m, 1H), 1.69-1.61 (m, 2H), 1.57-1.46 (m, 2H), 1.39-1.30 (m, 2H). LC/MS (ESI) *m*/*z* 802.1 [M+H]⁺, 800.0 [M-H]⁻

### <Example 15> Preparation of 2-(2-(2,6-dioxopiperidin-3-yl)-1,3-dioxoisoindolin-4-yloxy)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide

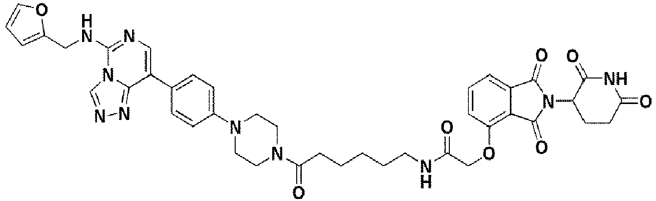

To N-(furan-2-ylmethyl)-8-(4-(piperazin-1-yl)phenyl)-[1,2,4]triazolo[4,3-f]pyrimidin-5-amine (20 mg, 0.048 mmol) in DMF, HATU (27 mg, 0.073 mmol), DIPEA (0.033mL, 0.192 mmol), and 6-(2-((2-(2,6-dioxopiperidin-3-yl)-1,3-dioxoisoindolin-4-yl)amino)acetamido)hexanoic acid (25mg, 0.051 mmol, Prepared in example 14 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by Silica Gel Column Chromatography (MeOH/DCM = 5%), target chemical, target chemical, 2-(2-(2,6-dioxopiperidin-3-yl)-1,3-dioxoisoindolin-4-yloxy)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide was obtained (17 mg, 0.021 mmol, 44% yield) as white solid.

¹H NMR (500 MHz, CDCl₃) δ8.31 (s, 1H), 8.12 (s, 1H), 7.88-7.83 (m, 2H), 7.74 (dd, *J* = 8.4, 7.4 Hz, 1H), 7.62 (t, *J* = 5.5 Hz, 1H), 7.58-7.54 (m, 1H), 7.42-7.39 (m, 1H), 7.20 (d, *J* = 8.4 Hz, 1H), 7.06-7.01 (m, 2H), 6.49 (t, *J* = 5.8 Hz, 1H), 6.40-6.34 (m, 2H), 5.00-4.94 (m, 1H), 4.87 (d, *J* = 5.7 Hz, 2H), 4.72-4.58 (m, 2H), 3.87-3.76 (m, 2H), 3.69-3.60 (m, 2H), 3.50-3.41 (m, 1H), 3.35-3.27 (m, 1H), 3.27-3.16 (m, 4H), 2.96-2.71 (m, 3H), 2.49-2.37 (m, 2H), 2.21-2.13 (m, 1H), 1.78-1.62 (m, 4H), 1.51-1.39 (m, 2H). LC/MS (ESI) *m*/*z* 803.0 [M+H]⁺, 801.0 [M-H]⁻

### <Example 16> Preparation of 2-(2-(2,6-dioxopiperidin-3-yl)-1,3-dioxoisoindolin-4-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide

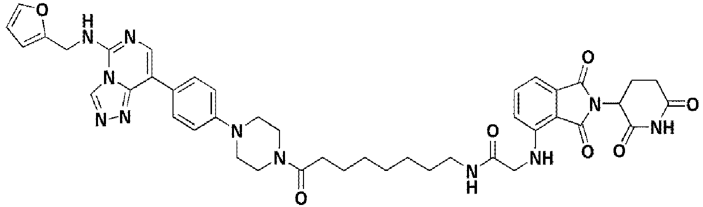

### Step 1: Preparation of 8-((tert-butoxycarbonyl)amino)octanoate

1,4-dioxane: To a solution of 8-aminooctanoate in water (2:1) (30ml) was added 2M NaOH (aq.). Then, the reaction mixture was cooled to 0 °C, and di-tert-butyldicarbonate was added dropwise. The reaction mixture was stirred at room temperature overnight. The solvent was evaporated in vacuo, acidified to pH 2 with 1M HCl and extracted with EtOAc. The combined organic layers were dried over MgSO4 and the solvent was concentrated in vacuo to afford the desired compound as an ivory solid.

¹H NMR (300 MHz, CDC13) δ 4.53 (s, 1H), 3.17-3.03 (m, 2H), 2.34 (t, *J* = 7.4 Hz, 2H), 1.69-1.58 (m, 2H), 1.52-1.40 (m, 11H), 1.39-1.26 (m, 6H).

### Step 2: Preparation of tert-butyl (8-(4-(4-(5-((furan-2-ylmethyl)amino)-[1,2,4]triazolo[4,3-c]pyrimidin-8-yl)phenyl)piperazin-1-yl)-8-oxooctyl)carbamate

N-(furan-2-ylmethyl)-8-(4-(piperazin-1-yl)phenyl)-[1,2,4]triazolo[4,3-f]pyrimidin-5-amine hydrochloride in DMF (150 mg, 0.364 mmol), the compound prepared in step 1 (95 mg, 0.364 mmol), HATU (208 mg, 0.546 mmol), and DIPEA (0.253 mL, 1.45 mmol) were added and stirred at room temperature overnight. The reaction mixture was diluted with water and extracted with EtOAc. The combined organic layers were washed with brine, dried over MgSO4 and the solvent removed in vacuo to give an oil. The crude mixture was purified by silica gel column chromatography using DCM / MeOH = 5 % to give the title compound (154 mg, 0.249 mmol, 68 %) as a pale yellow solid.

¹H NMR (300 MHz, CDCl₃) δ8.33-8.28 (m, 1H), 8.12 (s, 1H), 7.91-7.82 (m, 2H), 7.43-7.36 (m, 1H), 7.03 (d, *J* = 8.3 Hz, 2H), 6.66-6.55 (m, 1H), 6.40-6.31 (m, 2H), 4.86 (d, *J* = 5.8 Hz, 2H), 4.60 (s, 1H), 3.86-3.73 (m, 2H), 3.73-3.58 (m, 2H), 3.30-3.17 (m, 4H), 3.10 (q, *J* = 6.7 Hz, 2H), 2.37 (t, *J* = 7.6 Hz, 2H), 1.73-1.58 (m, 2H), 1.52-1.42 (m, 11H), 1.40-1.28 (m, 6H).

### Step 3: Preparation of 8-amino-1-(4-(4-(5-((puran-2-ylmethyl)amino-[1,2,4]triazolo[4,3-c]pyrimidin-8-yl)phenyl)piperazine-1-yl)octane-1-on

To a solution of the compound prepared in step 2 above (154 mg, 0.249 mmol) in DCM (3 mL) was added 4 M HCl in dioxane (1.5 ml). The reaction mixture was stirred at room temperature for 1 hour. The reaction mixture was concentrated in vacuo to give the title compound (150 mg, crude) as a pale yellow solid.

### Step 4: Preparation of 2-(2-(2,6-dioxopiperidin-3-yl)-1,3-dioxoisoindolin-4-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide

In a solution of 2-(2-(2,6-dioxopiperidin-3-yl)-1,3-dioxoisoindolin-4-ylamino)acetic acid (17 mg, 0.051 mmol) in DMF, the compound prepared in step 3 ( 28 mg, 0.051 mmol), EDCI HCl (11 mg, 0.057 mmol), HOBt HO (9 mg, 0.057 mmol), DIPEA (0.035 mL, 0.204 mmol) were added and stirred at room temperature for 2 h. The reaction mixture was diluted with water and extracted with EtOAc. The combined organic layers were washed with brine, dried over MgSO4 and the solvent removed in vacuo to give an oil. The crude mixture was purified on PREP TLC using MeOH / DCM = 5 % to give the title compound (12 mg, 0.014 mmol, 28 %) as a yellow solid.

¹H NMR (500 MHz, CDCl₃) δ 8.44 (s, 1H), 8.31 (d, *J* = 1.6 Hz, 1H), 8.12 (d, *J* = 1.6 Hz, 1H), 7.89-7.83 (m, 2H), 7.56 (t, *J* = 8.0 Hz, 1H), 7.41 (s, 1H), 7.24 (d, *J* = 7.2 Hz, 1H), 7.08-7.00 (m, 2H), 6.82 (d, *J* = 8.4 Hz, 1H), 6.67 (t, *J* = 5.9 Hz, 1H), 6.56-6.46 (m, 2H), 6.40-6.34 (m, 2H), 4.98-4.90 (m, 1H), 4.87 (d, *J* = 5.8 Hz, 2H), 3.95 (d, *J* = 5.9 Hz, 2H), 3.84-3.76 (m, 2H), 3.70-3.60 (m, 2H), 3.31-3.18 (m, 6H), 2.95-2.70 (m, 3H), 2.35 (t, *J* = 7.7 Hz, 2H), 2.17-2.11 (m, 1H), 1.64-1.58 (m, 2H), 1.53-1.42 (m, 2H), 1.37-1.25 (m, 6H).

### <Example 17> Preparation of 2-(2-(2,6-dioxopiperidin-3-yl)-1,3-dioxoisoindolin-4-yloxy)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide

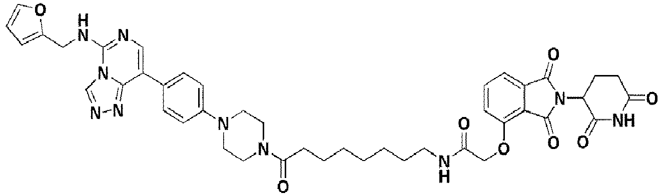

To 2-(2-(2,6-dioxopiperidin-3-yl)-1,3-dioxoisoindolin-4-yloxy)acetic acid (17 mg, 0.051 mmol) in DMF, EDCI HCI(11mg, 0.057 mmol), HOBt (9 mg, 0.057 mmol), DIPEA (0.035mL, 0.204 mmol), and 8-amino-1-(4-(4-(5-((puran-2-ylmethyl)amino-[1,2,4]triazolo[4,3-c]pyrimidin-8-yl)phenyl)piperazine-1-yl)octane-1-on (28mg, 0.051 mmol, Prepared in example 16 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5%), target chemical, 2-(2-(2,6-dioxopiperidin-3-yl)-1,3-dioxoisoindolin-4-yloxy)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide was obtained (6mg, 0.007 mmol, 14% yield) as beige solid.

¹H NMR (500 MHz, CDCl₃) δ 8.31 (d, *J* = 1.6 Hz, 1H), 8.12 (d, *J* = 1.6 Hz, 1H), 7.88-7.83 (m, 2H), 7.74 (t, *J* = 8.0 Hz, 1H), 7.55 (d, *J* = 7.4 Hz, 1H), 7.50 (t, *J* = 4.8 Hz, 1H), 7.41 (s, 1H), 7.18 (d, *J* = 8.4 Hz, 1H), 7.06-7.00 (m, 2H), 6.47 (t, *J* = 5.8 Hz, 1H), 6.40-6.35 (m, 2H), 5.03-4.95 (m, 1H), 4.87 (d, *J* = 5.7 Hz, 2H), 4.62 (s, 2H), 3.85-3.76 (m, 2H), 3.70-3.61 (m, 2H), 3.50-3.41 (m, 1H), 3.37-3.29 (m, 1H), 3.27-3.18 (m, 4H), 2.97-2.73 (m, 3H), 2.41 (t, *J* = 7.7 Hz, 2H), 2.19-2.13 (m, 1H), 1.64-1.57 (m, 4H), 1.47-1.34 (m, 6H).

### <Example 18> Preparation of 2-(2-(2,6-dioxopiperidin-3-yl)-1,3-dioxoisoindolin-4-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide

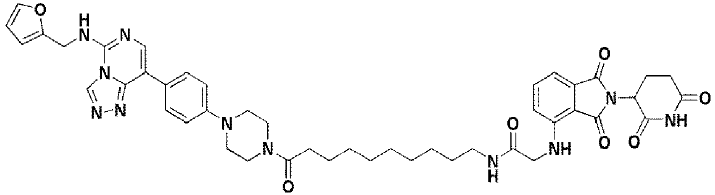

### Step 1: Preparation of 10-aminodecanoate

To a solution of 10-bromodecanoate (1.88 g, 7.48 mmol) in ammonium hydroxide (28 % NH3), the reaction mixture was stirred for 24 h. The solution was evaporated under reduced pressure to give the desired compound (1.54 g, quant) as a beige solid.

¹H NMR (300 MHz, DMSO-*d*₆) δ 7.00 (s, 2H), 2.83-2.69 (m, 2H), 2.18 (t, *J* = 7.4 Hz, 2H), 1.63-1.42 (m, 4H), 1.43-1.06 (m, 12H).

### Step 2: Preparation of 10-((tert-butoxycarbonyl)amino)decanoate

To a solution of the compound prepared in step 1 above (500 mg, 2.48 mmol) in 1,4-dioxane : water (2 : 1) (12 mL) was added 2 M NaOH (aq.). The reaction mixture was then cooled to 0° C. and di-tert-butyldicarbonate (0.627 mL, 2.73 mmol) was added dropwise. The reaction mixture was stirred at room temperature overnight. The solvent was evaporated in vacuo, acidified to pH 2 with 1M HCl and extracted with EtOAc. The combined organic layers were dried over MgSO4 and the solvent was concentrated in vacuo to give the title compound (482 mg, 1.67 mmol, 67%) as a clear oil.

¹H NMR (300 MHz, CDCl₃) δ4.63 (s, 1H), 3.17-2.98 (m, 2H), 2.33 (t, *J* = 7.5 Hz, 2H), 1.72-1.56 (m, 2H), 1.50-1.41 (m, 11H), 1.38-1.20 (m, 10H).

### Step 3: Preparation of tert-butyl (10-(4-(4-(5-((furan-2-ylmethyl)amino)-[1,2,4]triazolo[4,3-c]pyrimidin-8-yl)phenyl)piperazin-1-yl)-10-oxodecyl)carbamate

N-(furan-2-ylmethyl)-8-(4-(piperazin-1-yl)phenyl)-[1,2,4]triazolo[4,3-f]pyrimidin-5-amine hydrochloride in DMF (150 mg, 0.364 mmol), the compound prepared in step 2 (104 mg, 0.364 mmol), HATU (208 mg, 0.546 mmol), and DIPEA (0.254 mL, 1.45 mmol) were added and stirred at room temperature overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc/Hex = 55 % to give the title compound (160 mg, 0.248 mmol, 68 %) as a beige solid.

¹H NMR (300 MHz, CD₃OD) δ 8.40 (s, 1H), 8.10 (s, 1H), 7.86-7.78 (m, 2H), 7.49-7.43 (m, 1H), 7.16-7.07 (m, 2H), 6.56 (s, 1H), 6.41-6.35 (m, 2H), 4.84 (s, 2H), 3.83-3.67 (m, 4H), 3.31-3.19 (m, 4H), 3.08-2.98 (m, 2H), 2.47 (t, *J* = 7.6 Hz, 2H), 1.71-1.56 (m, 2H), 1.53-1.41 (m, 11H), 1.41-1.28 (m, 10H).

### Step 4: Preparation of 10-amino-1-(4-(4-(5-((furan-2-ylmethyl)amino-[1,2,4]triazolo[4,3-c]pirimidin-8-yl)phenyl)piperazin-1-yl)decane-1-on

To a solution of the compound prepared in step 3 above (160 mg, 0.248 mmol) in DCM (4 mL) was added 4 M HCl in dioxane (2 ml, 2.48 mmol). The reaction mixture was stirred at room temperature for 1 hour. The reaction mixture was concentrated in vacuo to give the desired compound (150 mg, crude) as a beige solid.

¹H NMR (300 MHz, DMSO-*d*₆) δ 8.78 (t, *J* = 6.1 Hz, 1H), 8.61 (s, 1H), 8.26 (s, 1H), 8.01 (d, *J* = 8.6 Hz, 2H), 7.85 (s, 2H), 7.61-7.56 (m, 1H), 7.20 (d, *J* = 8.6 Hz, 2H), 6.42-6.38 (m, 1H), 6.36-6.31 (m, 1H), 4.74 (d, *J* = 6.0 Hz, 2H), 3.76-3.63 (m, 4H), 3.38-3.18 (m, 4H), 2.82-2.68 (m, 2H), 2.36 (t, *J* = 7.4 Hz, 2H), 1.59-1.41 (m, 4H), 1.37-1.16 (m, 10H).

### Step 5: Preparation of 2-(2-(2,6-dioxopiperidin-3-yl)-1,3-dioxoisoindolin-4-ylamino)-N-(10-(4-(4-(5-(furan-2-ylmethylamino)-[1,2,4]triazolo[4,3-f]pyrimidin-8-yl)phenylpiperazin-1-yl)-10-oxodecyl)acetamide

In a solution of 2-(2-(2,6-dioxopiperidin-3-yl)-1,3-dioxoisoindolin-5-ylamino)acetate (17 mg, 0.051 mmol) in DMF, the compound prepared in step 4 (30 mg, 0.051 mmol), EDCI HCl (11 mg, 0.057 mmol), HOBt HO (9 mg, 0.057 mmol), DIPEA (0.035 mL, 0.204 mmol) were added and stirred at room temperature for 2 h. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 3 % to give the title compound (6 mg, 0.007 mmol, 13 %) as a yellow solid.

¹H NMR (500 MHz, CDCl₃) δ 8.31 (s, 1H), 8.12 (s, 1H), 7.89-7.83 (m, 2H), 7.56 (dd, *J* = 8.4, 7.2 Hz, 1H), 7.42-7.40 (m, 1H), 7.24 (d, *J* = 7.2 Hz, 1H), 7.07-7.01 (m, 2H), 6.82 (d, *J* = 8.4 Hz, 1H), 6.67 (t, *J* = 6.0 Hz, 1H), 6.48 (t, *J* = 5.9 Hz, 1H), 6.45 (t, *J* = 6.0 Hz, 2H), 6.40-6.34 (m, 2H), 4.97-4.91 (m, 1H), 4.88 (d, *J* = 5.7 Hz, 2H), 3.95 (d, *J* = 6.0 Hz, 2H), 3.84-3.75 (m, 2H), 3.70-3.62 (m, 2H), 3.31-3.18 (m, 6H), 2.94-2.71 (m, 3H), 2.37 (t, *J* = 7.5 Hz, 2H), 2.19-2.11 (m, 1H), 1.75-1.54 (m, 2H), 1.50-1.43 (m, 2H), 1.38-1.23 (m, 10H). LC/MS (ESI) *m*/*z* 858.1[M+H]⁺, 856.0 [M-H]⁻

### <Example 19> Preparation of 2-(2-(2,6-dioxopiperidin-3-yl)-1,3-dioxoisoindolin-4-yloxy)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide

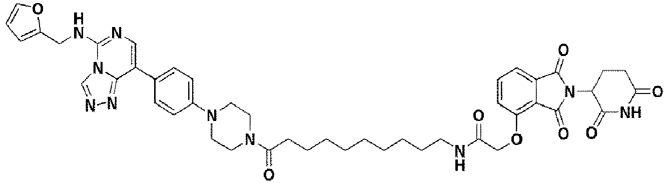

To 2-(2-(2,6-dioxopiperidin-3-yl)-1,3-dioxoisoindolin-4-yloxy)acetic acid (17 mg, 0.051 mmol) in DMF, EDCI HCI(11mg, 0.057 mmol), HOBt (9 mg, 0.057 mmol), DIPEA (0.035mL, 0.204 mmol), and 10-amino-1-(4-(4-(5-((furan-2-ylmethyl)amino-[1,2,4]triazolo[4,3-c]pirimidin-8-yl)phenyl)piperazin-1-yl)decane-1-on (30mg, 0.051 mmol, Prepared in example 18 step 4). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by Silica Gel Column Chromatography (MeOH/DCM = 3%), target chemical, 2-(2-(2,6-dioxopiperidin-3-yl)-1,3-dioxoisoindolin-4-yloxy)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide was obtained (16mg, 0.018 mmol, 14% yield) as yellow solid.

¹H NMR (500 MHz, CDCl₃) δ 8.96 (s, 1H), 8.31 (s, 1H), 8.12 (s, 1H), 7.89-7.83 (m, 2H), 7.73 (dd, *J* = 8.4, 7.4 Hz, 1H), 7.54 (d, *J* = 7.4 Hz, 1H), 7.45-7.37 (m, 2H), 7.18 (d, *J* = 8.4 Hz, 1H), 7.07-6.99 (m, 2H), 6.51 (t, *J* = 5.8 Hz, 1H), 6.41-6.33 (m, 2H), 5.01-4.93 (m, 1H), 4.87 (d, *J* = 5.7 Hz, 2H), 4.67-4.59 (m, 2H), 3.85-3.76 (m, 2H), 3.70-3.60 (m, 2H), 3.37 (q, *J* = 6.7 Hz, 2H), 3.27-3.17 (m, 4H), 2.96-2.71 (m, 3H), 2.38 (t, *J* = 7.6 Hz, 2H), 2.19-2.12 (m, 1H), 1.69-1.63 (m, 2H), 1.59 (p, *J* = 6.9 Hz, 2H), 1.43-1.28 (m, 10H). LC/MS (ESI) *m*/*z* 859.1 [M+H]⁺, 857.1 [M-H]⁻

### <Example 20> Preparation of 2-(2-(2,6-dioxopiperidin-3-yl)-1,3-dioxoisoindolin-4-ylamino)-N-(6-(4-(4-(5-(furan-2-ylmethylamino)-[1,2,4]triazolo[4,3-f]pyrimidin-8-yl)phenyl)piperazin-1-yl)hexyl)acetamide

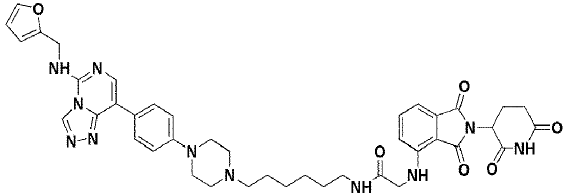

To 2-(2-(2,6-dioxopiperidin-3-yl)-1,3-dioxoisoindolin-4-ylamino)acetic acid (17 mg, 0.051 mmol) in DMF, EDCI HCl (11 mg, 0.057mmol), HOBt (9 mg, 0.057 mmol), DIPEA (0.035mL, 0.204 mmol), and 8-(4-(4-(6-aminohexyl)piperazin-1-yl)phenyl)-N-(furan-2-ylmethyl)-[1,2,4]triazolo[4,3-c]pyrimidin-5-amine (25mg, 0.051 mmol, Prepared in example 1 step 5). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 2-(2-(2,6-dioxopiperidin-3-yl)-1,3-dioxoisoindolin-4-ylamino)-N-(6-(4-(4-(5-(furan-2-ylmethylamino)-[1,2,4]triazolo[4,3-f]pyrimidin-8-yl)phenyl)piperazin-1-yl)hexyl)acetamide was obtained (12 mg, 0.015 mmol, 29% yield) as yellow solid.

¹H NMR (500 MHz, CDCl₃) δ 9.07 (s, 1H), 8.31 (s, 1H), 8.11 (s, 1H), 7.87-7.80 (m, 2H), 7.56 (t, *J* = 7.8 Hz, 1H), 7.40 (s, 1H), 7.24 (d, *J* = 7.1 Hz, 1H), 7.07-7.00 (m, 2H), 6.84 (d, *J* = 8.4 Hz, 1H), 6.64 (t, *J* = 6.2 Hz, 1H), 6.51 (t, *J* = 6.0 Hz, 1H), 6.46 (t, *J* = 6.0 Hz, 1H), 6.40-6.34 (m, 2H), 4.96-4.89 (m, 1H), 4.87 (d, *J* = 5.7 Hz, 2H), 4.04-3.90 (m, 2H), 3.37-3.18 (m, 6H), 2.92-2.69 (m, 3H), 2.66-2.57 (m, 4H), 2.42-2.32 (m, 2H), 2.19-2.11 (m, 1H), 1.55-1.43 (m, 4H), 1.37-1.28 (m, 4H). LC/MS (ESI) *m*/*z* 788.1 [M+H]⁺, 786.1 [M-H]⁻

### <Example 21> Preparation of 2-(2-(2,6-dioxopiperidin-3-yl)-1,3-dioxoisoindolin-4-ylamino)-N-(8-(4-(4-(5-(furan-2-ylmethylamino)-[1,2,4]triazolo[4,3-f]pyrimidin-8-yl)phenyl)piperazin-1-yl)octyl)acetamide

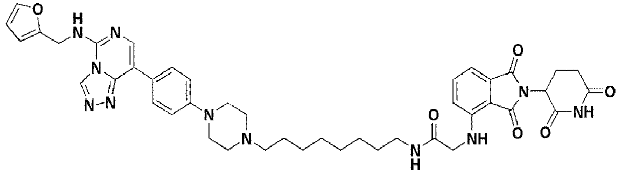

To 2-(2-(2,6-dioxopiperidin-3-yl)-1,3-dioxoisoindolin-4-ylamino)acetic acid (17 mg, 0.051 mmol) in DMF, EDCI HCl (11 mg, 0.057mmol), HOBt (9 mg, 0.057 mmol), DIPEA (0.035mL, 0.204 mmol), and 8-(4-(4-(8-aminooctyl)piperazin-1-yl)phenyl)-N-(furan-2-ylmethyl)-[1,2,4]triazolo[4,3-c]pyrimidin-5-amine (25mg, 0.051 mmol, Prepared in example 2 step 5). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 2-(2-(2,6-dioxopiperidin-3-yl)-1,3-dioxoisoindolin-4-ylamino)-N-(8-(4-(4-(5-(furan-2-ylmethylamino)-[1,2,4]triazolo[4,3-f]pyrimidin-8-yl)phenyl)piperazin-1-yl)octyl)acetamide was obtained (10 mg, 0.012 mmol, 24% yield) as yellow solid.

¹H NMR (500 MHz, CDCl₃) δ8.76 (s, 1H), 8.30 (s, 1H), 8.11 (s, 1H), 7.83 (d, *J* = 8.3 Hz, 2H), 7.55 (t, *J* = 7.8 Hz, 1H), 7.40 (s, 1H), 7.24 (d, *J* = 7.2 Hz, 1H), 7.03 (d, *J* = 8.3 Hz, 2H), 6.82 (d, *J* = 8.4 Hz, 1H), 6.69-6.63 (m, 1H), 6.52-6.43 (m, 2H), 6.41-6.34 (m, 2H), 4.97-4.91 (m, 1H), 4.87 (d, *J* = 5.8 Hz, 2H), 3.95 (d, *J* = 6.0 Hz, 2H), 3.33-3.20 (m, 6H), 2.97-2.69 (m, 3H), 2.67-2.58 (m, 4H), 2.38 (t, *J* = 7.8 Hz, 2H), 2.20-2.10 (m, 1H), 1.56-1.43 (m, 4H), 1.35-1.17 (m, 10H). LC/MS (ESI) m/z 816.1 [M+H]⁺, 814.1 [M-H]⁻

### <Example 22> Preparation of 2-(2,6-dioxopiperidin-3-yl)-5-(8-(4-(4-(5-(furan-2-ylmethylamino)-[1,2,4]triazolo[4,3-f]pyrimidin-8-yl)phenyl)piperazin-1-yl)-8-oxooctylamino)isoindolin-1,3-dione

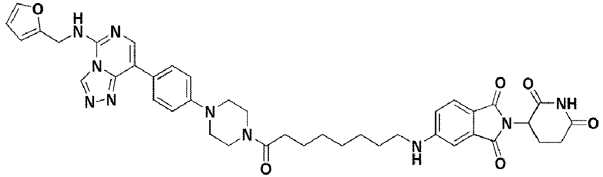

### Step 1: Preparation of tert-butyl 8-((2-(2,6-dioxopiperidin-3-yl)-1,3-dioxoisoindolin-5-yl)amino)octanoate

5-F-thalidomide (100 mg, 0.362 mmol) and DIPEA (0.094 mL, 0.543 mmol) were added to a solution of tert-butyl 8-aminooctanoate (117 mg, 0.543 mmol) in DMSO and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc / Hx = 30 % to give the title compound (36 mg, 0.076 mmol, 21 %) as a green solid.

¹H NMR (300 MHz, CDCl₃) δ 8.37 (s, 1H), 7.57 (d, *J* = 8.3 Hz, 1H), 6.92 (d, *J* = 2.1 Hz, 1H), 6.71 (dd, *J* = 8.3, 2.1 Hz, 1H), 5.00-4.89 (m, 1H), 4.69 (t, *J* = 5.4 Hz, 1H), 3.18 (q, *J* = 6.5 Hz, 2H), 2.93-2.70 (m, 3H), 2.22 (t, *J* = 7.4 Hz, 2H), 2.16-2.06 (m, 1H), 1.70-1.52 (m, 4H), 1.44 (s, 9H), 1.42-1.30 (m, 6H).

### Step 2: Preparation of 8-((2-(2,6-dioxopiperidin-3-yl)-1,3-dioxoisoindolin-5-yl)amino)octanoate

To a solution of the compound prepared in the previous step (36 mg, 0.076 mmol) in DCM (1 ml) was added TFA (1 ml), and the resulting mixture was stirred at room temperature for 3 hours. The reaction mixture was concentrated in vacuo to give the desired compound (36 mg, crude) as a yellow oil.

### Step 3: Preparation of 2-(2,6-dioxopiperidin-3-yl)-5-(8-(4-(4-(5-(furan-2-ylmethylamino)-[1,2,4]triazolo[4,3-f]pyrimidin-8-yl)phenyl)piperazin-1-yl)-8-oxooctylamino)isoindolin-1,3-dione

N-(furan-2-ylmethyl)-8-(4-(piperazin-1-yl)phenyl)-[1,2,4]triazolo[4,3-f]pyrimidin-5-amine hydrochloride (25mg) in DMF , 0.060mmol), the compound prepared in the previous step (25mg, 0.060mmol), EDCI HCl (12mg, 0.066mmol), HOBt HO (10mg, 0.066mmol ), DIPEA (0.042 mL, 0.240 mmol) was added, Stirred at room temperature for 2 hours. The reaction mixture was diluted with water and extracted with EtOAc. The combined organic layers were washed with brine, dried over MgSO4 and the solvent removed in vacuo to give an oil. The crude mixture was purified on PREP TLC using MeOH / DCM = 5% to give the title compound (7 mg, 0.009 mmol, 15%) as a yellow solid.

¹H NMR (500 MHz, CDC13) δ 8.31 (d, *J* = 1.4 Hz, 1H), 8.20-8.09 (m, 2H), 7.89-7.83 (m, 2H), 7.60 (d, *J* = 8.3 Hz, 1H), 7.41 (s, 1H), 7.07-7.01 (m, 2H), 6.95 (s, 1H), 6.73 (d, *J* = 8.4 Hz, 1H), 6.48 (t, *J* = 5.8 Hz, 1H), 6.41-6.34 (m, 2H), 4.97-4.90 (m, 1H), 4.88 (d, *J* = 5.8 Hz, 2H), 4.63 (t, *J* = 5.5 Hz, 1H), 3.86-3.77 (m, 2H), 3.71-3.60 (m, 2H), 3.29-3.17 (m, 6H), 2.94-2.67 (m, 3H), 2.39 (t, *J* = 7.5 Hz, 2H), 2.17-2.08 (m, 1H), 1.72-1.64 (m, 4H), 1.48-1.36 (m, 6H) ; LC/MS (ESI) *m*/*z* 773.1 [M+H]⁺, 771.0 [M-H]⁻

### <Example 23> Preparation of 2-(2,6-dioxopiperidin-3-yl)-5-(10-(4-(4-(5-(furan-2-ylmethylamino)-[1,2,4]triazolo[4,3-f]pyrimidin-8-yl)phenyl)piperazin-1-yl)-10-oxodecylamino)isoindolin-1,3-dione

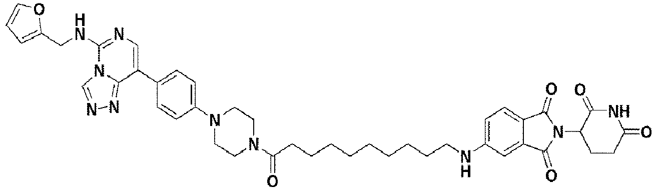

To N-(furan-2-ylmethyl)-8-(4-(piperazin-1-yl)phenyl)-[1,2,4]triazolo[4,3-f]pyrimidin-5-amine hydrochloride (13 mg, 0.031 mmol) in DMF, EDCI HCl (6 mg, 0.034mmol), HOBt (5 mg, 0.031 mmol), DIPEA (0.022mL, 0.124 mmol), and 10-amino-1-(4-(4-(5-((furan-2-ylmethyl)amino-[1,2,4]triazolo[4,3-c]pirimidin-8-yl)phenyl)piperazin-1-yl)decane-1-on (25mg, 0.051 mmol, Prepared in example 18 step 4). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP (MeOH/DCM = 5% ) and silica gel column chromatography, target chemical, 2-(2,6-dioxopiperidin-3-yl)-5-(10-(4-(4-(5-(furan-2-ylmethylamino)-[1,2,4]triazolo[4,3-f]pyrimidin-8-yl)phenyl)piperazin-1-yl)-10-oxodecylamino)isoindolin-1,3-dione was obtained (5 mg, 0.006 mmol, 20% yield) as yellow solid.

¹H NMR (500 MHz, CDCl₃) δ8.31 (s, 1H), 8.12 (s, 1H), 7.89-7.83 (m, 2H), 7.60 (d, *J* = 8.3 Hz, 1H), 7.43-7.38 (m, 1H), 7.08-7.01 (m, 2H), 6.95 (d, *J* = 2.2 Hz, 1H), 6.72 (dd, *J* = 8.3, 2.2 Hz, 1H), 6.48 (t, *J* = 5.8 Hz, 1H), 6.40-6.34 (m, 2H), 4.96-4.90 (m, 1H), 4.87 (d, *J* = 5.7 Hz, 2H), 3.84-3.77 (m, 2H), 3.70-3.62 (m, 2H), 3.29-3.17 (m, 6H), 2.92-2.68 (m, 3H), 2.39 (t, *J* = 7.5 Hz, 2H), 2.15-2.09 (m, 1H), 1.78-1.54 (m, 2H), 1.39-1.28 (m, 12H).

### <Example 24> Preparation of 2-(2-(2,6-dioxopiperidin-3-yl)-1,3-dioxoisoindolin-5-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide

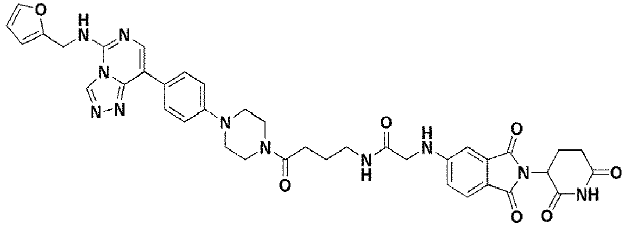

To 2-(2-(2,6-dioxopiperidin-3-yl)-1,3-dioxoisoindolin-5-ylamino)acetic acid (17 mg, 0.051 mmol) in DMF, EDCI HCl (11 mg, 0.057mmol), HOBt (9 mg, 0.057 mmol), DIPEA (0.035mL, 0.204 mmol), and 4-amino-1-(4-(4-(5-((puran-2-ylmethyl)amino)-[1,2,4]triazolo[4,3-c]pyrimidin-8-yl)phenyl)piperazine-1-yl)butane-1-on (25mg, 0.051 mmol, Prepared in example 12 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 2-(2-(2,6-dioxopiperidin-3-yl)-1,3-dioxoisoindolin-5-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide was obtained (7 mg, 0.009 mmol, 17% yield) as yellow solid.

¹H NMR (500 MHz, CDCl₃) δ8.81 (s, 1H), 8.33 (s, 1H), 8.12 (s, 1H), 7.84 (d, *J* = 8.3 Hz, 2H), 7.60 (d, *J* = 8.3 Hz, 1H), 7.41 (s, 1H), 7.40-7.33 (m, 1H), 7.00 (d, *J* = 8.3 Hz, 2H), 6.95 (s, 1H), 6.77 (d, *J* = 8.4 Hz, 1H), 6.50 (t, *J* = 6.1 Hz, 1H), 6.41-6.33 (m, 2H), 5.53 (t, *J* = 5.3 Hz, 1H), 4.97-4.90 (m, 1H), 4.87 (d, *J* = 5.6 Hz, 2H), 3.84 (d, *J* = 5.1 Hz, 2H), 3.80-3.67 (m, 2H), 3.67-3.55 (m, 2H), 3.44-3.28 (m, 2H), 3.25-3.19 (m, 2H), 3.19-3.13 (m, 2H), 2.92-2.66 (m, 3H), 2.45 (t, *J* = 6.4 Hz, 2H), 2.17-2.05 (m, 1H), 1.96-1.85 (m, 2H). LC/MS (ESI) *m*/*z* 774.1 [M+H]⁺, 772.1 [M-H]⁻

### <Example 25> Preparation of 2-(2-(2,6-dioxopiperidin-3-yl)-1,3-dioxoisoindolin-5-ylamino)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide

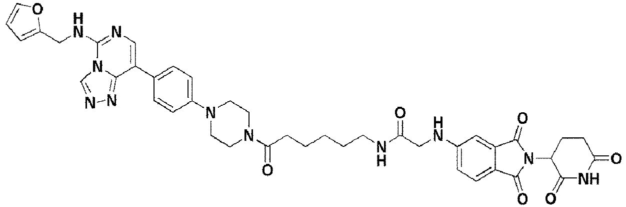

### Step 1: Preparation of tert-butyl (6-(4-(4-(5-((furan-2-ylmethyl)amino)-[1,2,4]triazolo[4,3-c]pyrimidin-8-yl)phenyl)piperazin-1-yl)-6-oxohexyl)carbamate

N-(furan-2-ylmethyl)-8-(4-(piperazin-1-yl)phenyl)-[1,2,4]triazolo[4,3-f]pyrimidin-5-amine hydrochloride in DMF (100 mg, 0.242 mmol) was added to a solution of 6-((tert-butoxycarbonyl)amino)hexane (56 mg, 0.242 mmol), HATU (138 mg, 0.363 mmol), and DIPEA (0.168 mL, 0.968 mmol) and stirred at room temperature overnight. did. The reaction mixture was diluted with water and extracted with EtOAc. The combined organic layers were washed with brine, dried over MgSO4 and the solvent removed in vacuo to give an oil. The crude mixture was purified by silica gel column chromatography using DCM / MeOH = 5 % to give the desired compound (102 mg, 0.173 mmol, 71 %) as a beige solid.

¹H NMR (500 MHz, CDCl₃) δ 8.31 (d, *J* = 1.4 Hz, 1H), 8.12 (d, *J* = 1.4 Hz, 1H), 7.89-7.84 (m, 2H), 7.41 (s, 1H), 7.07-7.01 (m, 2H), 6.49 (t, *J* = 5.9 Hz, 1H), 6.40-6.33 (m, 2H), 4.87 (d, *J* = 5.8 Hz, 2H), 4.59 (s, 1H), 3.84-3.76 (m, 2H), 3.69-3.60 (m, 2H), 3.29-3.20 (m, 4H), 3.17-3.06 (m, 2H), 2.38 (t, *J* = 7.6 Hz, 2H), 1.69 (p, *J* = 7.8 Hz, 2H), 1.55-1.49 (m, 2H), 1.44 (s, 9H), 1.43-1.35 (m, 2H).

### Step 2: Preparation of 6-amino-1-(4-(4-(5-((furan-2-ylmethyl)amino)-[1,2,4]triazolo[4,3-c]pyrimidin-8-yl)phenyl)piperazin-1-yl)hexane-1-on

To a solution of the compound prepared in the previous step (102 mg, 0.173 mmol) in DCM (3 mL) was added 4 M HCl in dioxane (1.5 ml). The reaction mixture was stirred at room temperature for 1 hour. The reaction mixture was concentrated in vacuo to give the desired compound (101 mg, crude) as a beige solid.

¹H NMR (300 MHz, DMSO-*d*₆) δ8.74 (t, *J* = 6.1 Hz, 1H), 8.60 (s, 1H), 8.24 (s, 1H), 7.98 (d, *J* = 8.7 Hz, 2H), 7.79 (s, 3H), 7.62-7.56 (m, 1H), 7.12 (d, *J* = 8.7 Hz, 2H), 6.43-6.37 (m, 1H), 6.36-6.30 (m, 1H), 4.74 (d, *J* = 6.0 Hz, 2H), 3.69-3.60 (m, 4H), 3.30-3.15 (m, 4H), 2.84-2.71 (m, 3H), 2.38 (t, *J* = 7.2 Hz, 2H), 1.58-1.48 (m, 2H), 1.41-1.21 (m, 6H).

### Step 3: Preparation of 2-(2-(2,6-dioxopiperidin-3-yl)-1,3-dioxoisoindolin-5-ylamino)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide

In a solution of 2-(2-(2,6-dioxopiperidin-3-yl)-1,3-dioxoisoindolin-5-ylamino)acetic acid (16 mg, 0.047 mmol) in DMF, the compound prepared in the previous step (25 mg , 0.047 mmol), EDCl HCl (10 mg, 0.052 mmol), HOBt HO (8 mg, 0.052 mmol), DIPEA (0.033 mL, 0.188 mmol) were added and stirred at room temperature for 2 h. The reaction mixture was diluted with water and extracted with EtOAc. The combined organic layers were washed with brine, dried over MgSO4 and the solvent removed in vacuo to give an oil. The crude mixture was purified on PREP TLC using MeOH / DCM = 5 % to give the title compound (14 mg, 0.017 mmol, 37 %) as a yellow solid.

¹H NMR (500 MHz, CDCl₃) δ 8.85 (s, 1H), 8.33 (d, *J* = 1.6 Hz, 1H), 8.12 (d, *J* = 1.6 Hz, 1H), 7.89-7.82 (m, 2H), 7.60 (d, *J* = 8.2 Hz, 1H), 7.40 (s, 1H), 7.07-7.00 (m, 2H), 6.98-6.94 (m, 1H), 6.91 (t, *J* = 5.9 Hz, 1H), 6.79 (d, *J* = 8.2 Hz, 1H), 6.51 (t, *J* = 6.1 Hz, 1H), 6.41-6.34 (m, 2H), 5.64 (t, *J* = 5.4 Hz, 1H), 4.98-4.90 (m, 1H), 4.87 (d, *J* = 5.8 Hz, 2H), 3.87 (d, *J* = 5.2 Hz, 2H), 3.87-3.74 (m, 2H), 3.70-3.59 (m, 2H), 3.35 (q, *J* = 6.6 Hz, 2H), 3.30-3.16 (m, 4H), 2.91-2.66 (m, 3H), 2.38 (t, *J* = 7.0 Hz, 2H), 2.16-2.07 (m, 1H), 1.64 (p, *J* = 7.4 Hz, 3H), 1.55 (p, *J* = 6.8 Hz, 2H), 1.42-1.32 (m, 2H) ; LC/MS (ESI) *m*/*z* 802.1 [M+H]⁺, 800.0 [M-H]⁻

### <Example 26> Preparation of 2-(2-(2,6-dioxopiperidin-3-yl)-1,3-dioxoisoindolin-5-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide

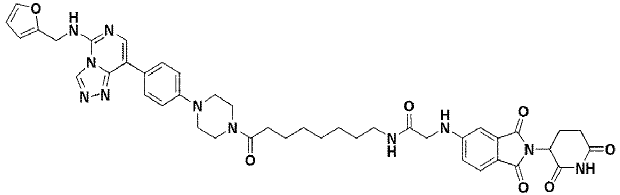

To 2-(2-(2,6-dioxopiperidin-3-yl)-1,3-dioxoisoindolin-5-ylamino)acetic acid (17 mg, 0.051 mmol) in DMF, EDCI HCl (11 mg, 0.057mmol), HOBt (9 mg, 0.057 mmol), DIPEA (0.035mL, 0.204 mmol), and 8-amino-1-(4-(4-(5-((puran-2-ylmethyl)amino-[1,2,4]triazolo[4,3-c]pyrimidin-8-yl)phenyl)piperazine-1-yl)octane-1-on (28mg, 0.051 mmol, Prepared in example 16 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 2-(2-(2,6-dioxopiperidin-3-yl)-1,3-dioxoisoindolin-5-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide was obtained (14 mg, 0.016 mmol, 33% yield) as yellow solid.

¹H NMR (500 MHz, CDCl₃) δ 8.92 (s, 1H), 8.32 (d, *J* = 1.7 Hz, 1H), 8.12 (d, *J* = 1.7 Hz, 1H), 7.90-7.80 (m, 2H), 7.59 (d, *J* = 8.3 Hz, 1H), 7.40 (s, 1H), 7.02 (d, *J* = 8.3 Hz, 2H), 6.94 (s, 1H), 6.78 (d, *J* = 8.3 Hz, 1H), 6.61-6.51 (m, 2H), 6.41-6.33 (m, 2H), 5.71 (t, *J* = 5.2 Hz, 1H), 4.99-4.90 (m, 1H), 4.87 (d, *J* = 5.8 Hz, 2H), 3.86 (d, *J* = 5.2 Hz, 2H), 3.84-3.76 (m, 2H), 3.70-3.60 (m, 2H), 3.35-3.26 (m, 2H), 3.26-3.17 (m, 4H), 2.91-2.68 (m, 3H), 2.37 (t, *J* = 7.5 Hz, 2H), 2.16-2.08 (m, 1H), 1.68-1.57 (m, 2H), 1.54-1.44 (m, 2H), 1.39-1.29 (m, 6H). LC/MS (ESI) *m*/*z* 830.1 [M+H]⁺, 828.0 [M-H]⁻

### <Example 27> Preparation of 2-(2-(2,6-dioxopiperidin-3-yl)-1,3-dioxoisoindolin-5-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide

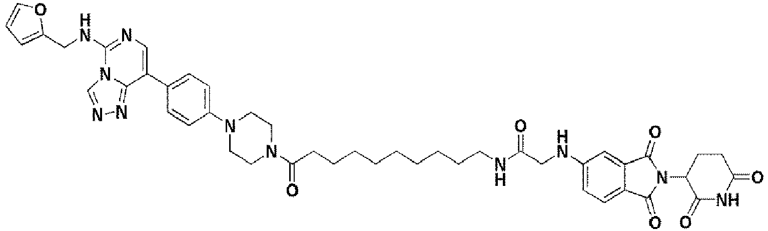

To 2-(2-(2,6-dioxopiperidin-3-yl)-1,3-dioxoisoindolin-5-ylamino)acetic acid (11 mg, 0.034 mmol) in DMF, EDCI HCl (7 mg, 0.038mmol), HOBt (6 mg, 0.038 mmol), DIPEA (0.024mL, 0.137 mmol), and 10-amino-1-(4-(4-(5-((furan-2-ylmethyl)amino-[1,2,4]triazolo[4,3-c]pirimidin-8-yl)phenyl)piperazin-1-yl)decane-1-on (20mg, 0.034 mmol, Prepared in example 18 step 4). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 2-(2-(2,6-dioxopiperidin-3-yl)-1,3-dioxoisoindolin-5-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide was obtained (7 mg, 0.008 mmol, 24% yield) as yellow solid.

¹H NMR (500 MHz, CDCl₃) δ 8.31 (s, 1H), 8.12 (s, 1H), 7.89-7.83 (m, 2H), 7.63 (d, *J* = 8.3 Hz, 1H), 7.43-7.39 (m, 1H), 7.07-7.01 (m, 2H), 6.97 (d, *J* = 2.2 Hz, 1H), 6.80 (dd, *J* = 8.3, 2.2 Hz, 1H), 6.49 (t, *J* = 5.8 Hz, 1H), 6.40-6.35 (m, 2H), 6.30 (t, *J* = 5.8 Hz, 1H), 5.53 (t, *J* = 5.2 Hz, 1H), 4.97-4.90 (m, 1H), 4.87 (d, *J* = 5.7 Hz, 2H), 3.87 (d, *J* = 5.1 Hz, 2H), 3.84-3.76 (m, 2H), 3.70-3.62 (m, 2H), 3.30 (q, *J* = 6.7 Hz, 2H), 3.27-3.18 (m, 4H), 2.92-2.68 (m, 3H), 2.38 (t, *J* = 7.5 Hz, 2H), 2.16-2.09 (m, 1H), 1.75-1.57 (m, 2H), 1.53-1.46 (m, 2H), 1.38-1.27 (m, 10H). LC/MS (ESI) *m*/*z* 858.1 [M+H]⁺, 856.1 [M-H]⁻

### <Example 28> Preparation of 1-(2-(2,6-dioxopiperidin-3-yl)-1,3-dioxoisoindolin-4-yl)-N-(4-(4-(4-(5-(furan-2-ylmethylamino)-[1,2,4]triazolo[4,3-f]pyrimidin-8-yl)phenyl)piperazin-1-yl)-4-oxobutyl)azetidin-3-carboxamide

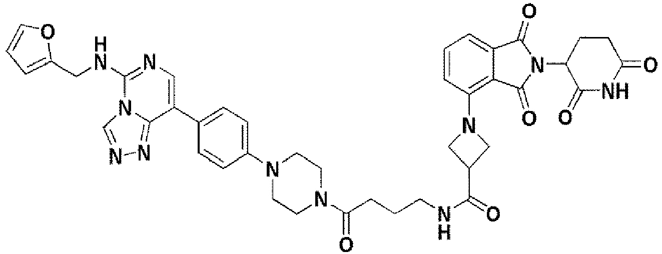

To 1-(2-(2,6-dioxopiperidin-3-yl)-1,3-dioxoisoindolin-4-yl)azetidin-3-carboxyl acid (18 mg, 0.051 mmol) in DMF, EDCI HCl (11 mg, 0.057 mmol), HOBt (9 mg, 0.057 mmol), DIPEA (0.035mL, 0.204 mmol), and 4-amino-1-(4-(4-(5-((puran-2-ylmethyl)amino)-[1,2,4]triazolo[4,3-c]pyrimidin-8-yl)phenyl)piperazine-1-yl)butane-1-on (25mg, 0.051 mmol, Prepared in example 12 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 2-(2-(2,6-dioxopiperidin-3-yl)-1,3-dioxoisoindolin-5-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide was obtained (1.7 mg, 0.002 mmol, 4% yield) as yellow solid.

¹H NMR (500 MHz, CDCl₃) δ 8.59 (s, 1H), 8.32 (s, 1H), 8.12 (s, 1H), 7.86 (d, *J* = 8.2 Hz, 2H), 7.46 (t, *J* = 7.6 Hz, 1H), 7.41 (s, 1H), 7.19 (d, *J* = 7.0 Hz, 1H), 7.09-7.04 (m, 2H), 6.70 (t, *J* = 5.2 Hz, 1H), 6.62 (d, *J* = 8.5 Hz, 1H), 6.46 (t, *J* = 5.7 Hz, 1H), 6.37 (d, *J* = 7.9 Hz, 2H), 4.88 (d, *J* = 5.7 Hz, 3H), 4.45-4.33 (m, 2H), 3.86-3.77 (m, 2H), 3.71-3.59 (m, 2H), 3.43-3.31 (m, 2H), 3.30-3.16 (m, 4H), 2.85-2.76 (m, 1H), 2.76-2.60 (m, 2H), 2.54-2.43 (m, 2H), 2.16-2.05 (m, 1H), 2.07-1.86 (m, 2H). LC/MS (ESI) *m*/*z* 800.1 [M+H]⁺, 798.1 [M-H]⁻

### <Example 29> Preparation of 1-(2-(2,6-dioxopiperidin-3-yl)-1,3-dioxoisoindolin-5-yl)-N-(4-(4-(4-(5-(furan-2-ylmethylamino)-[1,2,4]triazolo[4,3-f]pyrimidin-8-yl)phenyl)piperazin-1-yl)-4-oxobutyl)piperidin-4-carboxamide

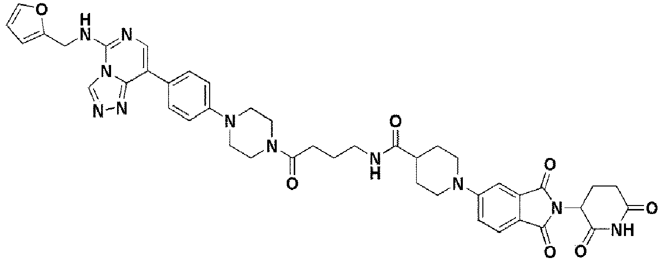

To 1-(2-(2,6-dioxopiperidin-3-yl)-1,3-dioxoisoindolin-5-yl)piperidin-4-carboxyl acid (19 mg, 0.051 mmol) in DMF, EDCI HCl (11 mg, 0.057 mmol), HOBt (9 mg, 0.057 mmol), DIPEA (0.035mL, 0.204 mmol), and 4-amino-1-(4-(4-(5-((puran-2-ylmethyl)amino)-[1,2,4]triazolo[4,3-c]pyrimidin-8-yl)phenyl)piperazine-1-yl)butane-1-on (25mg, 0.051 mmol, Prepared in example 12 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 1-(2-(2,6-dioxopiperidin-3-yl)-1,3-dioxoisoindolin-5-yl)-N-(4-(4-(4-(5-(furan-2-ylmethylamino)-[1,2,4]triazolo[4,3-f]pyrimidin-8-yl)phenyl)piperazin-1-yl)-4-oxobutyl)piperidin-4-carboxamide was obtained (14 mg, 0.017 mmol, 33% yield) as yellow solid.

¹H NMR (500 MHz, CDCl₃) δ 8.42 (s, 1H), 8.32 (d, *J* = 1.4 Hz, 1H), 8.13 (d, *J* = 1.4 Hz, 1H), 7.87 (d, *J* = 8.2 Hz, 2H), 7.67 (d, *J* = 8.5 Hz, 1H), 7.41 (s, 1H), 7.08-7.00 (m, 3H), 6.74-6.67 (m, 1H), 6.49 (t, *J* = 5.9 Hz, 1H), 6.41-6.33 (m, 2H), 4.98-4.91 (m, 1H), 4.88 (d, *J* = 5.8 Hz, 2H), 3.94 (d, *J* = 13.1 Hz, 2H), 3.84-3.76 (m, 2H), 3.71-3.61 (m, 2H), 3.33 (q, *J* = 6.1, 5.7 Hz, 2H), 3.29-3.16 (m, 4H), 3.05-2.94 (m, 2H), 2.93-2.67 (m, 3H), 2.49 (t, *J* = 6.4 Hz, 2H), 2.41-2.30 (m, 1H), 2.18-2.08 (m, 1H), 2.00-1.88 (m, 4H), 1.87-1.76 (m, 2H). LC/MS (ESI) *m*/*z* 828.2 [M+H]⁺, 826.1 [M-H]⁻

### <Example 30> Preparation of 1-(2-(2,6-dioxopiperidin-3-yl)-1,3-dioxoisoindolin-5-yl)-N-(6-(4-(4-(5-(furan-2-ylmethylamino)-[1,2,4]triazolo[4,3-f]pyrimidin-8-yl)phenyl)piperazin-1-yl)-6-oxohexyl)piperidin-4-carboxamide

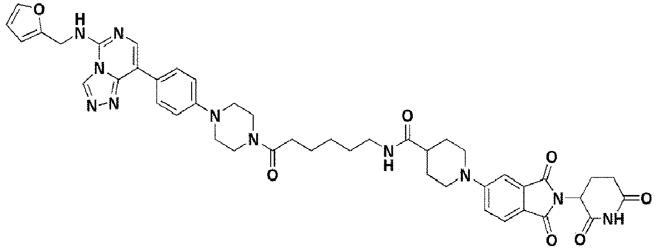

### Step 1: Preparation of 6-((tert-butoxycarbonyl)amino)hexanoate

To a solution of 6-amino acid (2.0 g, 15.25 mmol), Boc2O (4.9 mL, 21.34 mmol) and 2M NaOH (15.0 mL) in dioxane/HO (25.0 mL) was stirred at 25 °C for 1 h. Then conc. Adjust HCl to PH4.0 and inject EA. The combined organic phases were dried over NaSO4, filtered, concentrated and the resulting residue purified by silica chromatography (0-15%) gradient (MeOH/DCM gradient) to 6-((tert-butoxycarbonyl) amino) hexanoate, 13.4 mmol, 88%) was obtained as a white solid.

¹H NMR (300 MHz, CDCl₃) δ 11.54 (s, 1H), 4.95 (s, 1H), 3.10 (t, *J* = 6.7 Hz, 2H), 2.34 (t, *J* = 7.4 Hz, 2H), 1.72-1.59 (m, 2H), 1.54-1.34 (m, 13H).

### Step 2: Preparation of tert-butyl (6-(4-(4-(5-((furan-2-ylmethyl)amino)-[1,2,4]triazolo[4,3-c]pyrimidin-8-yl)phenyl)piperazin-1-yl)-6-oxohexyl)carbamate

N-(furan-2-ylmethyl)-8-(4-(piperazin-1-yl)phenyl)-[1,2,4]triazolo[4,3-f]pyrimidin-5-amine hydrochloride in DMF (100 mg, 0.242 mmol), HSJ-18-143 (56 mg, 0.242 mmol), HATU (138 mg, 0.363 mmol), DIPEA (0.168 mL, 0.968 mmol) were added and stirred at room temperature overnight. The reaction mixture was diluted with water and extracted with EtOAc. The combined organic layers were washed with brine, dried over MgSO4 and the solvent removed in vacuo to give an oil. The crude mixture was purified by silica gel column chromatography using DCM / MeOH = 5 % to give the desired compound (102 mg, 0.173 mmol, 71 %) as a beige solid.

¹H NMR (500 MHz, CDCl₃) δ 8.31 (d, *J* = 1.4 Hz, 1H), 8.12 (d, *J* = 1.4 Hz, 1H), 7.89-7.84 (m, 2H), 7.41 (s, 1H), 7.07-7.01 (m, 2H), 6.49 (t, *J* = 5.9 Hz, 1H), 6.40-6.33 (m, 2H), 4.87 (d, *J* = 5.8 Hz, 2H), 4.59 (s, 1H), 3.84-3.76 (m, 2H), 3.69-3.60 (m, 2H), 3.29-3.20 (m, 4H), 3.17-3.06 (m, 2H), 2.38 (t, *J* = 7.6 Hz, 2H), 1.69 (p, *J* = 7.8 Hz, 2H), 1.55-1.49 (m, 2H), 1.44 (s, 9H), 1.43-1.35 (m, 2H).

### Step 3: Preparation of 6-amino-1-(4-(4-(5-((furan-2-ylmethyl)amino)-[1,2,4]triazolo[4,3-c]pyrimidin-8-yl)phenyl)piperazin-1-yl)hexane-1-on

To a solution of the compound prepared in the previous step (102 mg, 0.173 mmol) in DCM (3 mL) was added 4 M HCl in dioxane (1.5 ml). The reaction mixture was stirred at room temperature for 1 hour. The reaction mixture was concentrated in vacuo to give the desired compound (101 mg, crude) as a beige solid.

¹H NMR (300 MHz, DMSO-*d*₆) δ8.74 (t, *J* = 6.1 Hz, 1H), 8.60 (s, 1H), 8.24 (s, 1H), 7.98 (d, *J* = 8.7 Hz, 2H), 7.79 (s, 3H), 7.62-7.56 (m, 1H), 7.12 (d, *J* = 8.7 Hz, 2H), 6.43-6.37 (m, 1H), 6.36-6.30 (m, 1H), 4.74 (d, *J* = 6.0 Hz, 2H), 3.69-3.60 (m, 4H), 3.30-3.15 (m, 4H), 2.84-2.71 (m, 3H), 2.38 (t, *J* = 7.2 Hz, 2H), 1.58-1.48 (m, 2H), 1.41-1.21 (m, 6H).

### Step 4: Preparation of 1-(2-(2,6-dioxopiperidin-3-yl)-1,3-dioxoisoindolin-5-yl)-N-(6-(4-(4-(5-(furan-2-ylmethylamino)-[1,2,4]triazolo[4,3-f]pyrimidin-8-yl)phenyl)piperazin-1-yl)-6-oxohexyl)piperidin-4-carboxamide

The compound prepared in the previous step in a solution of 1-(2-(2,6-dioxopiperidin-3-yl)-1,3-dioxoisoindolin-5-yl)piperidin-4-carboxylate (18 mg, 0.047 mmol) in DMF (25 mg, 0.047 mmol), EDCl HCl (10 mg, 0.052 mmol), HOBt HO (8 mg, 0.052 mmol), DIPEA (0.033 mL, 0.188 mmol) were added and stirred at room temperature for 2 h. The reaction mixture was diluted with water and extracted with EtOAc. The combined organic layers were washed with brine, dried over MgSO4 and the solvent removed in vacuo to give an oil. The crude mixture was purified on PREP TLC using MeOH / DCM = 5% to give the title compound (21 mg, 0.024 mmol, 52%) as a yellow solid.

¹H NMR (500 MHz, CDCl₃) δ8.35 (s, 1H), 8.31 (d, *J* = 1.5 Hz, 1H), 8.12 (d, *J* = 1.5 Hz, 1H), 7.90-7.84 (m, 2H), 7.70-7.64 (m, 1H), 7.41 (s, 1H), 7.08-7.00 (m, 3H), 6.52 (t, *J* = 5.9 Hz, 1H), 6.40-6.33 (m, 2H), 6.01 (t, *J* = 5.8 Hz, 1H), 4.97-4.91 (m, 1H), 4.87 (d, *J* = 5.8 Hz, 2H), 4.01-3.92 (m, 2H), 3.83-3.76 (m, 2H), 3.69-3.60 (m, 2H), 3.31 (q, *J* = 6.6 Hz, 2H), 3.27-3.18 (m, 4H), 3.05-2.97 (m, 2H), 2.92-2.67 (m, 3H), 2.40 (t, *J* = 7.1 Hz, 2H), 2.16-2.09 (m, 1H), 1.99-1.92 (m, 2H), 1.90-1.80 (m, 2H), 1.70-1.65 (m, 2H), 1.56 (p, *J* = 7.1 Hz, 2H), 1.40 (p, *J* = 7.7 Hz, 2H). LC/MS (ESI) m/z 856.1 [M+H]⁺, 854.0 [M-H]⁻

### <Example 31> Preparation of 1-(2-(2,6-dioxopiperidin-3-yl)-1,3-dioxoisoindolin-5-yl)-N-(8-(4-(4-(5-(furan-2-ylmethylamino)-[1,2,4]triazolo[4,3-f]pyrimidin-8-yl)phenyl)piperazin-1-yl)-8-oxooctyl)piperidin-4-carboxamide

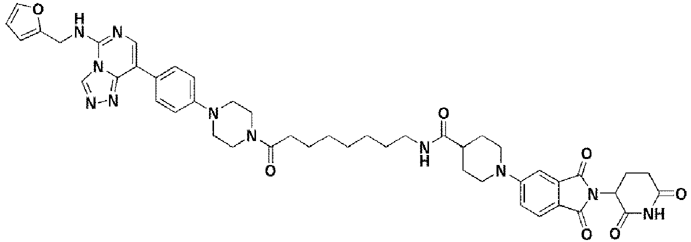

To 1-(2-(2,6-dioxopiperidin-3-yl)-1,3-dioxoisoindolin-5-yl)piperidin-4-carboxyl acid (19 mg, 0.051 mmol) in DMF, EDCI HCl (11 mg, 0.057 mmol), HOBt (9 mg, 0.057 mmol), DIPEA (0.035mL, 0.204 mmol), and 8-amino-1-(4-(4-(5-((puran-2-ylmethyl)amino-[1,2,4]triazolo[4,3-c]pyrimidin-8-yl)phenyl)piperazine-1-yl)octane-1-on (28mg, 0.051 mmol, Prepared in example 16 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 1-(2-(2,6-dioxopiperidin-3-yl)-1,3-dioxoisoindolin-5-yl)-N-(8-(4-(4-(5-(furan-2-ylmethylamino)-[1,2,4]triazolo[4,3-f]pyrimidin-8-yl)phenyl)piperazin-1-yl)-8-oxooctyl)piperidin-4-carboxamide was obtained (22 mg, 0.024 mmol, 48% yield) as yellow solid.

¹H NMR (500 MHz, CDCl₃) δ 8.31 (s, 1H), 8.21 (s, 1H), 8.12 (s, 1H), 7.86 (d, *J* = 8.2 Hz, 2H), 7.67 (d, *J* = 8.5 Hz, 1H), 7.41 (s, 1H), 7.08-7.02 (m, 3H), 6.50 (t, *J* = 6.0 Hz, 1H), 6.40-6.34 (m, 2H), 5.60 (t, *J* = 5.9 Hz, 1H), 4.98-4.90 (m, 1H), 4.87 (d, *J* = 5.7 Hz, 2H), 4.02-3.93 (m, 2H), 3.86-3.76 (m, 2H), 3.71-3.61 (m, 2H), 3.31-3.18 (m, 6H), 3.06-2.97 (m, 2H), 2.94-2.69 (m, 3H), 2.38 (t, *J* = 7.5 Hz, 2H), 2.36-2.30 (m, 1H), 2.17-2.09 (m, 1H), 2.00-1.92 (m, 2H), 1.90-1.79 (m, 2H), 1.66-1.62 (m, 2H), 1.56-1.45 (m, 2H), 1.42-1.29 (m, 6H). LC/MS (ESI) *m*/*z* 884.1 [M+H]⁺, 882.0 [M-H]⁻

### <Example 32> Preparation of N-(6-(2-(2,6-dioxopiperidin-3-yl)-1,3-dioxoisoindolin-4-ylamino)hexyl)-4-(5-(furan-2-ylmethylamino)-[1,2,4]triazolo[4,3-f]pyrimidin-8-yl)benzamide

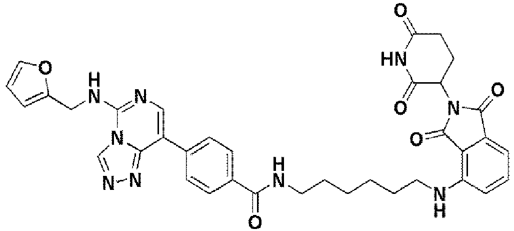

### Step 1: Preparation of tert-butyl (6-((2-(2,6-dioxopiperidin-3-yl)-1,3-dioxoisoindolin-4-yl)amino)hexyl)carbamate

In a solution of N-Boc-1,6-hexanediamine (100 mg, 0.46 mmol, 1 eq.) in DMSO, 4-fluoro-2-(2,6-dioxopiperidin-3-yl)isoindolin-1,3- Dione (128 mg, 0.46 mmol, 1 eq.), DIPEA (0.3 mL, 1.4 mmol, 3 eq.) were added. The reaction mixture was stirred at room temperature for 30 min and then at 90 oC for 12 h. The mixture was extracted with EtOAc. The collected organic layer was washed with brine solution. Then, the organic layer was dried over Na2SO4 and filtered. The solvent was removed in vacuo, and the product was purified through column chromatography using MeOH/DCM 6% as an eluent to prepare the title compound in the form of a yellow solid.

### Step 2: Preparation of 4-((6-aminohexyl)amino)-2-(2,6-dioxopiperidin-3-yl)isoindolin-1,3-dione

To a solution of the compound prepared in the previous step (156 mg, 0.34 mmol) dissolved in DCM (2 mL), 4 M HCl dissolved in dioxane was added. The reaction mixture was stirred at room temperature for 30 minutes. The reaction mixture was concentrated under reduced pressure to prepare the target compound (172 mg, crude) in the form of a yellow oil.

### Step 3: Preparation of N-(6-(2-(2,6-dioxopiperidin-3-yl)-1,3-dioxoisoindolin-4-ylamino)hexyl)-4-(5-(furan-2-ylmethylamino)-[1,2,4]triazolo[4,3-f]pyrimidin-8-yl)benzamide

A solution of 4-(5-((furan-2-yl)methylamino)-[1,2,4]triazolo[4,3-f]pyrimidin-8-yl)benzoate (20 mg, 0.059 mmol) in DMF To, the compound prepared in the previous step (24 mg, 0.059 mmol), EDCI HCl (12 mg, 0.065 mmol), HOBt HO (10 mg, 0.065 mmol), DIPEA (0.041 mL, 0.236 mmol) was added, and at room temperature Stirred for 2 hours. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 3% to prepare the title compound (3 mg, 0.004 mmol, 7%) as a yellow solid.

¹H NMR (500 MHz, CDCl₃) δ 8.34 (s, 1H), 8.25 (s, 1H), 8.24 (s, 1H), 8.06-8.01 (m, 2H), 7.92-7.86 (m, 2H), 7.49 (dd, *J* = 8.5, 7.1 Hz, 1H), 7.44-7.40 (m, 1H), 7.09 (d, *J* = 7.1 Hz, 1H), 6.88 (d, *J* = 8.5 Hz, 1H), 6.59 (t, *J* = 5.8 Hz, 1H), 6.42-6.35 (m, 2H), 6.25 (q, *J* = 5.1 Hz, 2H), 4.94-4.87 (m, 3H), 3.54-3.45 (m, 2H), 3.29 (q, *J* = 6.6 Hz, 2H), 2.91-2.67 (m, 3H), 2.16-2.09 (m, 1H), 1.75-1.64 (m, 4H), 1.54-1.43 (m, 4H) ; LC/MS (ESI) *m*/*z* 690.1 [M+H]⁺, 688.0 [M-H]⁻

### <Example 33> Preparation of N-(6-(2-(2-(2,6-dioxopiperidin-3-yl)-1,3-dioxoisoindolin-4-ylamino)acetamido)hexyl)-4-(5-(furan-2-ylmethylamino)-[1,2,4]triazolo[4,3-f]pyrimidin-8-yl)benzamide

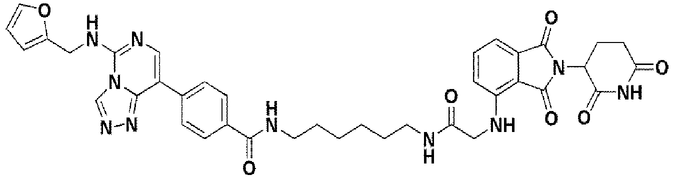

### Step 1: Preparation of tert-butyl (6-(4-(5-((furan-2-ylmethyl)amino)-[1,2,4]triazolo[4,3-c]pyrimidin-8-yl)benzamido)hexyl)carbamate

A solution of 4-(5-((furan-2-yl)methylamino)-[1,2,4]triazolo[4,3-f]pyrimidin-8-yl)benzoate (100 mg, 0.298 mmol) in DMF To, N-Boc-1,6-diaminohexane (0.066 ml, 0.298 mmol), HATU (170 mg, 0.447 mmol), and DIPEA (0.207 mL, 1.19 mmol) were added, and the mixture was stirred at room temperature for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using EtOAc / Hex = 60% to prepare the title compound (86 mg, 0.127 mmol, 42%) as a white solid.

¹H NMR (500 MHz, CDCl₃) δ 8.36-8.30 (m, 1H), 8.26-8.20 (m, 1H), 8.07-8.00 (m, 2H), 7.91 (d, *J* = 8.0 Hz, 2H), 7.43-7.38 (m, 1H), 6.64-6.58 (m, 1H), 6.45 (s, 1H), 6.41-6.34 (m, 2H), 4.89 (d, *J* = 5.8 Hz, 2H), 4.58 (s, 1H), 3.47 (q, *J* = 6.6 Hz, 2H), 3.14 (q, *J* = 6.8 Hz, 2H), 1.76-1.60 (m, 2H), 1.55-1.46 (m, 2H), 1.44 (s, 9H), 1.43-1.34 (m, 4H).

### Step 2: Preparation of N-(6-aminohexyl)-4-(5-((furan-2-ylmethyl)amino)-[1,2,4]triazolo[4,3-c]pyrimidin-8-yl)benzamide hydrochloride

To a solution of the compound prepared in the previous step (68 mg, 0.127 mmol) dissolved in DCM (2 mL), 4 N HCl dissolved in 1,4-dioxane (1 mL) was added, and the mixture was stirred at room temperature for 1 h. did. The reaction mixture was concentrated in vacuo. The solid was filtered with DCM under reduced pressure to prepare the target compound (60 mg, crude) in the form of an ivory solid.

¹H NMR (300 MHz, DMSO-*d*₆) δ 8.97 (t, *J* = 6.1 Hz, 1H), 8.66 (s, 1H), 8.55 (t, *J* = 5.6 Hz, 1H), 8.44 (s, 1H), 8.27-8.17 (m, 2H), 8.01-7.93 (m, 2H), 7.87 (s, 3H), 7.63-7.56 (m, 1H), 6.45-6.32 (m, 2H), 4.76 (d, *J* = 6.0 Hz, 2H), 3.53-3.43 (m, 2H), 3.29 (q, *J* = 6.6 Hz, 2H), 2.83-2.71 (m, 2H), 1.62-1.49 (m, 4H), 1.41-1.26 (m, 4H).

### Step 3: Preparation of N-(6-(2-(2-(2,6-dioxopiperidin-3-yl)-1,3-dioxoisoindolin-4-ylamino)acetamido)hexyl)-4-(5-(furan-2-ylmethylamino)-[1,2,4]triazolo[4,3-f]pyrimidin-8-yl)benzamide

In a solution of 2-(2-(2,6-dioxopiperidin-3-yl)-1,3-dioxoisoindolin-4-ylamino)acetic acid (18 mg, 0.055 mmol) dissolved in DMF, the compound prepared in the previous step ( 26 mg, 0.055 mmol), EDCI HCl (12 mg, 0.060 mmol), HOBt HO (9 mg, 0.060 mmol), DIPEA (0.038 mL, 0.220 mmol) were added and stirred at room temperature for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 5% to prepare the title compound (17 mg, 0.022 mmol, 41%) as a yellow solid.

¹H NMR (500 MHz, DMSO-*d*₆) δ 11.11 (s, 1H), 8.96 (t, *J* = 6.1 Hz, 1H), 8.65 (s, 1H), 8.48 (t, *J* = 5.6 Hz, 1H), 8.43 (s, 1H), 8.23-8.16 (m, 2H), 8.09 (t, *J* = 5.7 Hz, 1H), 7.96-7.90 (m, 2H), 7.62-7.55 (m, 2H), 7.06 (d, *J* = 7.0 Hz, 1H), 6.95 (t, *J* = 5.7 Hz, 1H), 6.85 (d, *J* = 8.5 Hz, 1H), 6.42-6.37 (m, 1H), 6.37-6.32 (m, 1H), 5.10-5.04 (m, 1H), 4.76 (d, *J* = 6.0 Hz, 2H), 3.92 (d, *J* = 5.7 Hz, 2H), 3.27 (d, *J* = 6.6 Hz, 2H), 3.10 (q, *J* = 6.6 Hz, 2H), 2.93-2.84 (m, 1H), 2.65-2.52 (m, 2H), 2.06-1.98 (m, 1H), 1.58-1.47 (m, 2H), 1.48-1.38 (m, 2H), 1.36-1.26 (m, 4H) ; LC/MS (ESI) *m*/*z* 747.1 [M+H]⁺, 745.0 [M-H]-

### <Example 34> Preparation of N-(6-(2-(2-(2,6-dioxopiperidin-3-yl)-1,3-dioxoisoindolin-4-yloxy)acetamido)hexyl)-4-(5-(furan-2-ylmethylamino)-[1,2,4]triazolo[4,3-f]pyrimidin-8-yl)benzamide

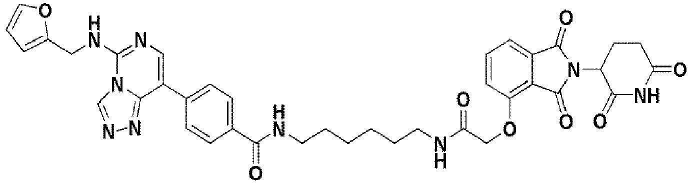

To 2-(2-(2,6-dioxopiperidin-3-yl)-1,3-dioxoisoindolin-4-yloxy)acetic acid (18 mg, 0.055 mmol) in DMF, EDCI HCl (12 mg, 0.060 mmol), HOBt (9 mg, 0.060 mmol), DIPEA (0.038mL, 0.220 mmol), and (N-(6-aminohexyl)-4-(5-((furan-2-ylmethyl)amino)-[1,2,4]triazolo[4,3-c]pyrimidin-8-yl)benzamid hydrochloride (26mg, 0.055 mmol, Prepared in example 33 step 2). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by silica gel column chromatography (MeOH/DCM = 5% ), target chemical, N-(6-(2-(2-(2,6-dioxopiperidin-3-yl)-1,3-dioxoisoindolin-4-yloxy)acetamido)hexyl)-4-(5-(furan-2-ylmethylamino)-[1,2,4]triazolo[4,3-f]pyrimidin-8-yl)benzamide was obtained (21 mg, 0.028 mmol, 51% yield) as yellow solid.

¹H NMR (500 MHz, CDCl₃) δ 9.09 (s, 1H), 8.33 (s, 1H), 8.22 (s, 1H), 8.04-7.98 (m, 2H), 7.91-7.85 (m, 2H), 7.73 (dd, *J* = 8.4, 7.4 Hz, 1H), 7.53 (d, *J* = 7.4 Hz, 1H), 7.50 (t, *J* = 5.7 Hz, 1H), 7.43-7.39 (m, 1H), 7.18 (d, *J* = 8.4 Hz, 1H), 6.59 (t, *J* = 5.8 Hz, 1H), 6.43 (t, *J* = 5.9 Hz, 1H), 6.41-6.34 (m, 2H), 5.00-4.93 (m, 1H), 4.89 (d, *J* = 5.8 Hz, 2H), 4.63 (s, 2H), 3.58-3.49 (m, 1H), 3.49-3.41 (m, 2H), 3.41-3.33 (m, 1H), 2.92-2.72 (m, 3H), 2.19-2.11 (m, 1H), 1.70-1.62 (m, 4H), 1.53-1.41 (m, 4H) ; LC/MS (ESI) *m*/*z* 748.0 [M+H]⁺, 746.0 [M-H]⁻

### <Example 35> Preparation of 1-(2-(2,6-dioxopiperidin-3-yl)-1,3-dioxoisoindolin-5-yl)-N-(10-(4-(4-(5-(furan-2-ylmethylamino)-[1,2,4]triazolo[4,3-f]pyrimidin-8-yl)phenyl)piperazin-1-yl)-10-oxodecyl)piperidin-4-carboxamide

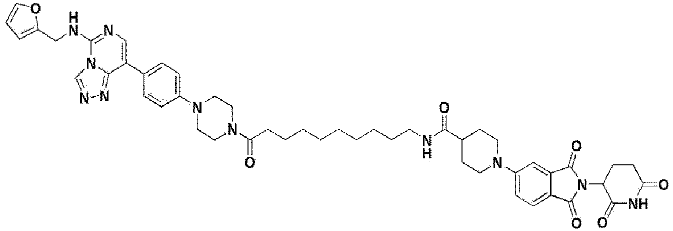

To 1-(2-(2,6-dioxopiperidin-3-yl)-1,3-dioxoisoindolin-5-yl)piperidin-4-carboxyl acid (13 mg, 0.034 mmol) in DMF, EDCI HCl (7 mg, 0.038 mmol), HOBt (6 mg, 0.038 mmol), DIPEA (0.024mL, 0.137 mmol), and 10-amino-1-(4-(4-(5-((furan-2-ylmethyl)amino-[1,2,4]triazolo[4,3-c]pirimidin-8-yl)phenyl)piperazin-1-yl)decane-1-on (20mg, 0.034 mmol, Prepared in example 18 step 4). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 1-(2-(2,6-dioxopiperidin-3-yl)-1,3-dioxoisoindolin-5-yl)-N-(10-(4-(4-(5-(furan-2-ylmethylamino)-[1,2,4]triazolo[4,3-f]pyrimidin-8-yl)phenyl)piperazin-1-yl)-10-oxodecyl)piperidin-4-carboxamide was obtained (5 mg, 0.005 mmol, 16% yield) as yellow solid.

¹H NMR (500 MHz, CDCl₃) δ 8.31 (s, 1H), 8.12 (s, 1H), 8.05 (s, 1H), 7.90-7.84 (m, 2H), 7.68 (d, *J* = 8.5 Hz, 1H), 7.44-7.38 (m, 1H), 7.28 (d, *J* = 2.4 Hz, 1H), 7.11-7.00 (m, 3H), 6.47 (t, *J* = 5.8 Hz, 1H), 6.41-6.35 (m, 2H), 5.52 (t, *J* = 6.2 Hz, 1H), 4.96-4.91 (m, 1H), 4.88 (d, *J =* 5.6 Hz, 2H), 4.02-3.94 (m, 2H), 3.86-3.78 (m, 2H), 3.70-3.62 (m, 2H), 3.29-3.19 (m, 6H), 3.06-2.98 (m, 2H), 2.92-2.78 (m, 1H), 2.77-2.68 (m, 2H), 2.38 (d, *J* = 7.5 Hz, 2H), 2.36-2.30 (m, 1H), 1.99-1.93 (m, 2H), 1.89-1.80 (m, 2H), 1.70-1.63 (m, 2H), 1.55-1.46 (m, 2H), 1.40-1.28 (m, 10H). LC/MS (ESI) *m*/*z* 912.2 [M+H]⁺, 910.0 [M-H]⁻

### <Example 36> Preparation of 1-(2-(2,6-dioxopiperidin-3-yl)-1,3-dioxoisoindolin-4-yl)-N-(10-(4-(4-(5-(furan-2-ylmethylamino)-[1,2,4]triazolo[4,3-f]pyrimidin-8-yl)phenyl)piperazin-1-yl)-10-oxodecyl)azetidin-3-carboxamide

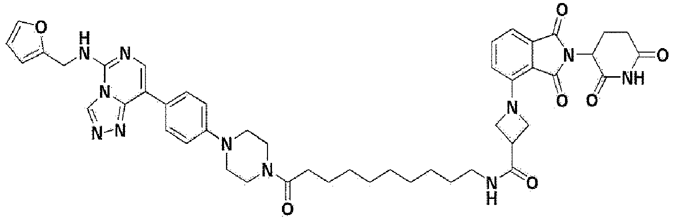

To 1-(2-(2,6-dioxopiperidin-3-yl)-1,3-dioxoisoindolin-4-yl)azetidin-3-carboxyl acid (13 mg, 0.034 mmol) in DMF, EDCI HCl (7 mg, 0.038 mmol), HOBt (6 mg, 0.038 mmol), DIPEA (0.024mL, 0.137 mmol), and 10-amino-1-(4-(4-(5-((furan-2-ylmethyl)amino-[1,2,4]triazolo[4,3-c]pirimidin-8-yl)phenyl)piperazin-1-yl)decane-1-on (20mg, 0.034 mmol, Prepared in example 18 step 4). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5% ), target chemical, 1-(2-(2,6-dioxopiperidin-3-yl)-1,3-dioxoisoindolin-4-yl)-N-(10-(4-(4-(5-(furan-2-ylmethylamino)-[1,2,4]triazolo[4,3-f]pyrimidin-8-yl)phenyl)piperazin-1-yl)-10-oxodecyl)azetidin-3-carboxamide was obtained (2 mg, 0.002 mmol, 6% yield) as yellow solid

¹H NMR (500 MHz, CDCl₃) δ8.31 (s, 1H), 8.20 (s, 1H), 8.12 (s, 1H), 7.90-7.84 (m, 2H), 7.47 (dd, *J* = 8.6, 7.0 Hz, 1H), 7.43-7.40 (m, 1H), 7.20 (d, *J* = 7.0 Hz, 1H), 7.06-7.02 (m, 2H), 6.64 (d, *J* = 8.6 Hz, 1H), 6.46 (t, *J* = 5.6 Hz, 1H), 6.40-6.35 (m, 2H), 5.72-5.66 (m, 1H), 4.94-4.89 (m, 1H), 4.87 (d, *J* = 5.7 Hz, 2H), 4.48-4.41 (m, 2H), 4.41-4.34 (m, 2H), 3.84-3.77 (m, 2H), 3.70-3.63 (m, 2H), 3.38 (d, *J* = 7.0 Hz, 1H), 3.31-3.19 (m, 6H), 2.91-2.69 (m, 3H), 2.38 (t, *J* = 7.7 Hz, 2H), 2.15-2.08 (m, 1H), 2.07-1.99 (m, 2H), 1.70-1.61 (m, 2H), 1.54-1.47 (m, 2H), 1.39-1.29 (m, 10H). LC/MS (ESI) *m*/*z* 884.1 [M+H]⁺, 882.0 [M-H]⁻

### <Example 37> Preparation of N-(2-(2-(5-(2-(2,6-dioxopiperidin-3-yl)-1,3-dioxoisoindolin-4-ylamino)pentyloxy)ethoxy)ethyl)-4-(5-(furan-2-ylmethylamino)-[1,2,4]triazolo[4,3-f]pyrimidin-8-yl)benzamide

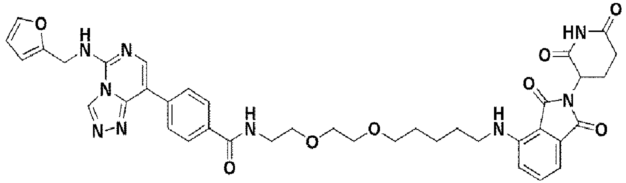

### Step 1: Preparation of 2-(2-((tert-butoxycarbonyl)amino)ethoxy)ethyl 4-methylbenzensulfonate

To a solution of tert-butyl 2-(2-hydroxyethoxy)ethylcarbamate (2.00 g, 9.74 mmol) in DCM (30 mL), TsCl (3.71 mL, 19.5 mmol), TEA (4.07 mL, 29.2 mmol) and DMAP (118) mg, 0.97 mmol) was added at 0 °C. Then, the solution was stirred at room temperature for 12 h. The reaction was quenched with water and extracted with DCM (2 X 25 mL). The collected organic layer was dried over Na2SO4 and the solvent was removed in vacuo. The crude mixture was purified through silica gel column chromatography using EtOAc/Hex (3/7) as an eluent to give the title compound (3.42 g, 9.51 mmol, 98%) as a brown oil.

¹H NMR (300 MHz, CDCl₃) δ 7.84-7.35 (m, 2H), 7.39-7.29 (m, 2H), 4.81 (s, 1H), 4.21-4.11 (m, 2H), 3.66-3.55 (m, 2H), 3.48-3.37 (m, 2H), 3.28-3.15 (m, 2H), 2.48-2.37 (m, 3H), 1.49-1.38 (m, 9H)

### Step 2: Preparation of tert-butyl (2-(2-((5-hydroxypantyl)oxy)ethoxy)ethyl)carbamate

To a solution of the compound prepared in the previous step (2.00 g, 5.56 mmol) in propane-1,3-diol (15.0 mL), NaOH (667 mg, 16.7 mmol) was added. The solution was stirred at 60 ° for 2 h. The reaction was quenched with water and extracted with DCM (2 X 25 mL). The collected organic layer was dried over Na2SO4 and the solvent was removed under vacuum. The crude mixture was purified through silica gel column chromatography using EtOAc/Hex (1/1) as an eluent to give the title compound (1.55 g, 5.32 mmol, 96%) as a colorless oil.

¹H NMR (300 MHz, CDCl₃) δ 5.24 (br s, 1H), 3.70-3.51 (m, 8H), 3.48 (t, *J* = 6.6 Hz, 2H), 3.35-3.26 (m, 2H), 2.08 (br s, 1H), 1.70-1.51 (m, 4H), 1.52-1.35 (m, 12H).

### Step 3: Preparation of 2,2-dimethyl-4-oxo-3,8,11-trioxa-5-azahexadecane-16-yl 4-methylbenzensulfonate

To a solution of the compound prepared in the previous step (1.55 g, 5.32 mmol) in DCM (10 mL), TsCl (2.03 g, 10.6 mmol), TEA (2.22 mL, 16.0 mmol) and DMAP (64 mg, 0.53 mmol) was added at 0 °C. Then, the solution was stirred at room temperature for 12 h. The reaction was quenched with water and extracted with DCM (2 X 25 mL). The collected organic layer was dried over Na2SO4 and the solvent was removed in vacuo. The crude mixture was purified by silica gel column chromatography using EtOAc/Hex (3/7) as an eluent to give the title compound (1.69 g, 3.79 mmol, 71%) as a colorless oil.

¹H NMR (300 MHz, CDCl₃) δ 7.78 (d, *J* = 7.8 Hz, 2H), 7.35 (d, *J* = 8.1 Hz, 2H), 5.02 (br s, 1H), 4.04 (t, *J* = 6.6 Hz, 2H), 3.67-3.50 (m, 6H), 3.49-3.37 (m, 2H), 3.67-3.25 (m, 2H), 2.45 (s, 3H), 1.78-1.61 (m, 2H), 1.61-1.51 (m, 2H), 1.51-1.41 (m, 9H), 1.42-1.32 (m, 2H).

### Step 4: Preparation of tert-butyl (2-(2-((5-iodopantyl)oxy)ethoxy)ethyl)carbamate

To a solution of the compound prepared in the previous step (1.69 g, 3.78 mmol) dissolved in acetone (20 mL), sodium iodide (2.84 mg, 19.0 mmol) was added and stirred at 80 ° for one day. The reaction mixture was concentrated to remove the solvent. The reaction was quenched with water and extracted with EtOAc (2 X 25 mL). The collected organic layer was dried over Na2SO4, and the solvent was removed under vacuum to prepare the title compound (1.41 g, 3.51 mmol, 92%) as a yellow oil.

¹H NMR (500 MHz, CDCl₃) δ 5.06 (s, 1H), 3.63-3.59 (m, 2H), 3.59-3.52 (m, 4H), 3.47 (t, *J* = 6.5 Hz, 2H), 3.35-3.29 (m, 2H), 3.19 (t, *J* = 7.0 Hz, 2H), 1..91-1.81 (m, 2H), 1.66-1.58 (m, 2H), 1.51-1.46 (m, 2H), 1.46-1.41 (m, 9H).

### Step 5: Preparation of tert-butyl (2-(2-((5-aminopantyl)oxy)ethoxy)ethyl)carbamate

A solution of the compound prepared in the previous step (1.41 g, 3.51 mmol) dissolved in MeOH (14.0 mL), 7 N NH 3 was stirred at 50 °C for 16 h. The reaction mixture was concentrated to remove the solvent. The crude mixture was purified through silica gel column chromatography using DCM/MeOH (9/1) as eluent to give the title compound (795 mg, 2.73 mmol, 78%) as a brown oil.

¹H NMR (300 MHz, CDCl₃) δ 7.64 (br s, 1H), 5.13 (s, 1H), 3.70-3.56 (m, 6H), 3.59-3.52 (m, 4H), 3.52 (t, *J* = 6.0 Hz, 2H), 3.39-3.27 (m, 2H), 3.13 (t, *J* = 7.2 Hz, 2H), 1..97-1.84 (m, 2H), 1.72-1.61 (m, 2H), 1.60-1.49 (m, 2H), 1.49-1.35 (m, 9H).

### Step 6: Preparation of tert-butyl (2-(2-((5-((2-(2,6-dioxopiperidin-3-yl)-1,3-dioxoisoindolin-4-yl)amino)pantyl)oxy)ethoxy)ethyl)carbamate

In a solution of the compound prepared in the previous step (500 mg, 1.72 mmol) in DMSO (5 mL), 4-fluoro-2-(2,6-dioxopiperidin-3-yl)isoindolin-1,3-dione (570 mg, 2.07 mmol) and DIPEA (0.60 mL, 3.44 mmol) were added. The reaction mixture was stirred at 90 ° for one day. The reaction was quenched with water and extracted with EtOAc (2 X 25 mL). The collected organic layer was dried over Na2SO4 and the solvent was removed under vacuum. The crude mixture was purified through silica gel column chromatography using EtOAc/Hex (1/1) as an eluent to prepare the title compound (183 mg, 0.33 mmol, 19%) as a yellow solid.

¹H NMR (300 MHz, CDCl₃) δ 8.54 (s, 1H), 7.53-7.44 (m, 1H), 7.08 (d, *J* = 6.9 Hz, 1H), 6.88 (d, *J* = 8.7 Hz, 1H), 6.24 (t, *J* = 5.7 Hz, 1H), 5.04 (br s, 1H), 4.98-4.88 (m, 1H), 3.65-3.52 (m, 6H), 3.48 (t, *J* = 6.6 Hz, 2H), 3.36-3.22 (m, 2H), 2.92-2.68 (m, 3H), 2.19-2.08 (m, 1H), 1.78-1.61 (m, 4H), 1.56-1.46 (m, 4H), 1.45-1.39 (m, 9H).

### Step 7: Preparation of 4-((5-(2-(2-(chloro-14-azaneyl)ethoxy)ethoxy)pantyl)amino)-2-(2,6-dioxopiperidin-3-yl)isoindolin-1,3-dione hydrochloride

To a solution of the compound prepared in the previous step (183 mg, 0.33 mmol) dissolved in DCM (4 mL), 4 M HCl dissolved in dioxane (2.0 mL) was added, and the mixture was stirred at room temperature for 1 h. The solvent was removed in vacuo to prepare the title compound (146 mg, 0.30 mmol, 92%) as a yellow solid.

¹H NMR (500 MHz, CD₃OD) δ 7.57-7.49 (m, 1H), 7.07-7.01 (m, 2H), 5.04-4.99 (m, 1H), 3.73-3.67 (m, 1H), 3.67-3.62 (m, 3H), 3.62-3.59 (m, 4H), 3.59-3.55 (m, 2H), 3.55-3.51 (m, 1H), 3.48 (t, *J* =6.5 Hz, 1H), 3.29-3.24 (m, 2H), 3.10-3.04 (m, 2H) 2.86-2.77 (m, 1H), 2.74-2.61 (m, 2H), 2.10-2.03 (m, 1H), 1.71-1.58 (m, 4H), 1.51-1.41 (m, 2H).

### Step 8: Preparation of N-(2-(2-(5-(2-(2,6-dioxopiperidin-3-yl)-1,3-dioxoisoindolin-4-ylamino)pentyloxy)ethoxy)ethyl)-4-(5-(furan-2-ylmethylamino)-[1,2,4]triazolo[4,3-f]pyrimidin-8-yl)benzamide

A solution of 4-(5-((furan-2-yl)methylamino)-[1,2,4]triazolo[4,3-f]pyrimidin-8-yl)benzoate (20 mg, 0.059 mmol) in DMF To, the compound prepared in the previous step (28 mg, 0.059 mmol), EDCI HCl (12 mg, 0.065 mmol), HOBt HO (10 mg, 0.065 mmol), DIPEA (0.041 mL, 0.236 mmol) was added, and at room temperature Stirred for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 7% as an eluent to prepare the title compound (20 mg, 0.026 mmol, 44%) as a yellow solid.

¹H NMR (500 MHz, CDCl₃) δ 8.31 (s, 1H), 8.28-8.20 (m, 2H), 8.09-8.02 (m, 2H), 7.95-7.87 (m, 2H), 7.45-7.38 (m, 2H), 7.03 (d, *J* = 7.1 Hz, 1H), 6.86-6.79 (m, 1H), 6.77 (d, *J* = 8.5 Hz, 1H), 6.65 (t, *J* = 5.8 Hz, 1H), 6.41-6.34 (m, 2H), 6.13 (t, *J* = 5.6 Hz, 1H), 4.94-4.88 (m, 1H), 4.88-4.85 (m, 2H), 3.74-3.66 (m, 6H), 3.64-3.58 (m, 2H), 3.49 (t, *J* = 6.5 Hz, 2H), 3.18 (q, *J* = 6.7 Hz, 2H), 2.91-2.68 (m, 3H), 2.17-2.09 (m, 1H), 1.67-1.61 (m, 4H), 1.50-1.41 (m, 2H).

### <Example 38> Preparation of N-(5-(2-(2-(2-(2-(2,6-dioxopiperidin-3-yl)-1,3-dioxoisoindolin-4-ylamino)acetamido)ethoxy)ethoxy)pentyl)-4-(5-(furan-2-ylmethylamino)-[1,2,4]triazolo[4,3-f]pyrimidin-8-yl)benzamide

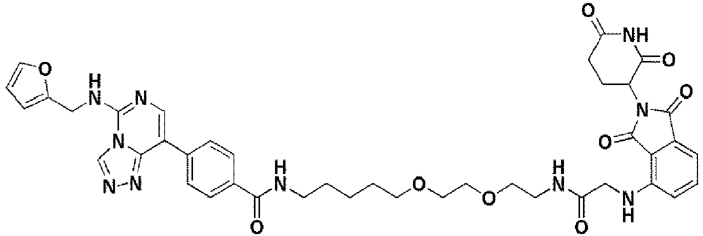

### Step 1: Preparation of tert-butyl (2-(2-((5-(4-(5-((furan-2-ylmethyl)amino)-[1,2,4]triazolo[4,3-c]pyrimidin-8-yl)benzamido)pantyl)oxy)ethoxy)ethyl)carbamate

A solution of 4-(5-((furan-2-yl)methylamino)-[1,2,4]triazolo[4,3-f]pyrimidin-8-yl)benzoate (100 mg, 0.298 mmol) in DMF The compound (86 mg, 0.298 mmol), EDCI HCl (62 mg, 0.327 mmol), HOBt HO (50 mg, 0.327 mmol), DIPEA (0.207 mL, 1.19 mmol) prepared in step 5 of Example 37 was added. was added and stirred at room temperature for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 4% to prepare the title compound (100 mg, 0.130 mmol, 43%) as a yellow solid.

¹H NMR (300 MHz, CDCl₃) δ 8.33 (s, 1H), 8.22 (s, 1H), 8.05-7.98 (m, 2H), 7.94-7.83 (m, 2H), 7.43-7.37 (m, 1H), 6.71 (t, *J* = 5.8 Hz, 1H), 6.53-6.45 (m, 1H), 6.40-6.33 (m, 2H), 5.09 (s, 1H), 4.88 (d, *J* = 5.8 Hz, 2H), 3.66-3.41 (m, 10H), 3.30 (q, *J* = 5.4 Hz, 2H), 1.74-1.58 (m, 4H), 1.53-1.45 (m, 2H), 1.44 (s, 9H).

### Step 2: Preparation of N-(5-(2-(2-aminoethoxy)ethoxy)pantyl)-4-(5-((furan-2-ylmethyl)amino)-[1,2,4]triazolo[4,3-c]pyrimidin-8-yl)benzamide

To a solution of the compound prepared in the previous step (65 mg, 0.107 mmol) dissolved in DCM (2 mL), 4 N HCl dissolved in 1,4-dioxane (1 mL) was added, and the mixture was stirred at room temperature for 1 h. did. The reaction mixture was concentrated in vacuo. The solid was filtered with DCM under reduced pressure to give the title compound (65 mg, quant.) as a yellow oil.

¹H NMR (300 MHz, DMSO-*d*₆) δ 8.98 (t, *J* = 6.1 Hz, 1H), 8.66 (s, 1H), 8.56 (t, *J* = 5.6 Hz, 1H), 8.44 (s, 1H), 8.20 (d, *J* = 8.3 Hz, 2H), 8.15-7.87 (m, 4H), 7.66-7.52 (m, 1H), 6.46-6.30 (m, 2H), 4.77 (d, *J* = 5.9 Hz, 2H), 3.64-3.59 (m, 2H), 3.56-3.48 (m, 4H), 3.40 (t, *J* = 6.5 Hz, 2H), 3.37-3.22 (m, 2H), 3.04-2.89 (m, 2H), 1.66-1.44 (m, 4H), 1.44-1.29 (m, 2H).

### Step 3: Preparation of N-(5-(2-(2-(2-(2-(2,6-dioxopiperidin-3-yl)-1,3-dioxoisoindolin-4-ylamino)acetamido)ethoxy)ethoxy)pentyl)-4-(5-(furan-2-ylmethylamino)-[1,2,4]triazolo[4,3-f]pyrimidin-8-yl)benzamide

To 2-(2-(2,6-dioxopiperidin-3-yl)-1,3-dioxoisoindolin-4-ylamino)acetic acid (18 mg, 0.055 mmol) dissolved in DMF, the compound prepared in the previous step (30 mg, 0.055 mmol), EDCI HCl (12 mg, 0.060 mmol), HOBt HO (9 mg, 0.060 mmol), DIPEA (0.038 mL, 0.220 mmol) were added and stirred at room temperature for 2 h. The reaction mixture was diluted with water and extracted with EtOAc. The collected organic layer was washed with brine, dried over MgSO4, and the solvent was removed under reduced pressure to obtain an oil. The crude mixture was purified through silica gel column chromatography using MeOH/DCM = 5% to prepare the title compound (10 mg, 0.012 mmol, 22%) as a yellow solid.

¹H NMR (300 MHz, CDCl₃) δ 8.88 (s, 1H), 8.33 (s, 1H), 8.22 (s, 1H), 8.06-7.96 (m, 2H), 7.93-7.83 (m, 2H), 7.49 (t, *J* = 7.8 Hz, 1H), 7.44-7.38 (m, 1H), 7.16 (d, *J* = 7.1 Hz, 1H), 7.12-7.02 (m, 1H), 6.78 (d, *J* = 8.5 Hz, 1H), 6.72 (t, *J* = 6.0 Hz, 1H), 6.68-6.55 (m, 2H), 6.43-6.32 (m, 2H), 4.98-4.82 (m, 3H), 3.94 (d, *J* = 5.9 Hz, 2H), 3.66-3.36 (m, 12H), 2.91-2.63 (m, 3H), 2.16-2.06 (m, 1H), 1.71-1.52 (m, 4H), 1.50-1.37 (m, 2H).

### <Example 39> Preparation of N-(5-(2-(2-(2-(2-(2,6-dioxopiperidin-3-yl)-1,3-dioxoisoindolin-4-yloxy)acetamido)ethoxy)ethoxy)pentyl)-4-(5-(furan-2-ylmethylamino)-[1,2,4]triazolo[4,3-f]pyrimidin-8-yl)benzamide

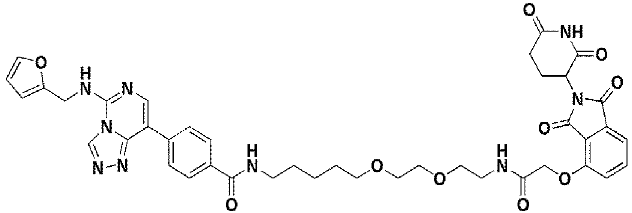

To 2-(2-(2,6-dioxopiperidin-3-yl)-1,3-dioxoisoindolin-4-yloxy)acetic acid (18 mg, 0.055 mmol) in DMF, EDCI HCl (12 mg, 0.060 mmol), HOBt (9 mg, 0.060 mmol), DIPEA (0.038mL, 0.220 mmol), and N-(5-(2-(2-aminoethoxy)ethoxy)pentyl)-4-(5-((furan-2-ylmethyl)amino)-[1,2,4]triazolo[4,3-c]pyrimidin-8-yl)benzamide. (30mg, 0.055 mmol, Prepared in example 38 step 2) And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by silica gel column chromatography (MeOH/DCM = 5% ), target chemical, N-(5-(2-(2-(2-(2-(2,6-dioxopiperidin-3-yl)-1,3-dioxoisoindolin-4-yloxy)acetamido)ethoxy)ethoxy)pentyl)-4-(5-(furan-2-ylmethylamino)-[1,2,4]triazolo[4,3-f]pyrimidin-8-yl)benzamide was obtained (18 mg, 0.022 mmol, 39% yield) as yellow solid.

¹H NMR (500 MHz, CDCl₃) δ 8.81 (s, 1H), 8.33 (s, 1H), 8.22 (s, 1H), 8.04-7.99 (m, 2H), 7.92-7.86 (m, 2H), 7.73-7.67 (m, 1H), 7.60 (t, *J* = 5.6 Hz, 1H), 7.51 (d, *J* = 7.3 Hz, 1H), 7.43-7.40 (m, 1H), 7.15 (d, *J* = 8.4 Hz, 1H), 6.62 (t, *J* = 5.8 Hz, 1H), 6.58 (t, *J* = 5.7 Hz, 1H), 6.41-6.34 (m, 2H), 4.99-4.92 (m, 1H), 4.89 (d, *J* = 5.8 Hz, 2H), 4.63 (s, 2H), 3.68-3.53 (m, 8H), 3.51-3.40 (m, 4H), 2.91-2.69 (m, 3H), 2.18-2.09 (m, 1H), 1.69-1.57 (m, 4H), 1.52-1.41 (m, 2H).

### <Example 40> Preparation of N-(8-(6-(2-(2,6-dioxopiperidin-3-yl)-1,3-dioxoisoindolin-4-ylamino)hexyloxy)octyl)-4-(5-(furan-2-ylmethylamino)-[1,2,4]triazolo[4,3-f]pyrimidin-8-yl)benzamide

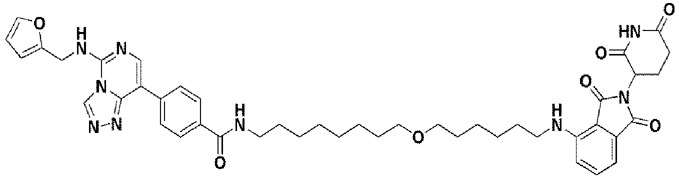

### Step 1: Preparation of tert-butyl (8-hydroxyoctyl)carbamate

To a solution of 8-aminooctane-1-ol (1.00 g, 6.88 mmol) in THF (20 mL) at 0 °C was added BocO (1.90 mL, 8.26 mmol) and TEA (1.44 mL, 10.2 mmol). The resulting mixture was stirred at room temperature overnight. The reaction was quenched and then extracted with EtOAc (2×25 mL). The combined organic layers were dried over Na2SO4 and the solvent was removed in vacuo. The crude mixture was purified by silica gel column chromatography using EtOAc/Hex (1/1) as eluent to give the title compound (1.19 g, 4.85 mmol, 70 %) as a white solid.

¹H NMR (300 MHz, CDCl₃) δ4.50 (s, 1H), 3.64 (t, *J* = 6.3 Hz, 2H), 3.10 (q, *J* = 6.6 Hz, 2H), 1.66-1.52 (m, 2H), 1.44 (s, 9H), 1.38-1.39-1.21 (m, 10H)

### Step 2: Preparation of tert-butyl (8-((6-chlorohexyl)oxy)octyl)carbamate

To a solution of the compound prepared in step 1 (2.00 g, 8.15 mmol) and 1-bromo-6 chlorohexane (3.22 g, 16.3 mmol) in benzene (10 mL), tetrabutylammonium hydrogen sulfate (415 mg, 1.22 mmol) and 50% NaOH (aq) (3.84 mL) was added. The mixture was stirred overnight at 50 °C. The reaction was quenched with water and then extracted with EtOAc (2×25 mL). The combined organic layers were dried over Na2SO4 and the solvent was removed in vacuo. The crude mixture was purified by silica gel column chromatography using EtOAc/Hex (3/7) as eluent to give the title compound (964 mg, 2.65 mmol, 32 %) as a colorless oil.

¹H NMR (300 MHz, CDCl₃) δ4.49 (s, 1H), 3.64-3.38 (m, 2H), 3.46-3.31 (m, 3H), 3..23-2.98 (m, 2H), 1.97-1.79 (m, 3H), 1.70-1.52 (m, 2H), 1.48-1.39 (m, 9H), 1.39-1.18 (m, 8H).

### Step 3: Preparation of 8-((6-iodohexyl)oxy)octyl)carbamate

To a solution of the compound prepared in step 2 (500 mg, 0.989 mmol) in acetone (20 mL) was added sodium iodide (741 mg, 4.94 mmol) and refluxed for 5 hours. The reaction mixture was concentrated to remove the solvent. The crude mixture was purified by silica gel column chromatography using DCM/MeOH (9/1) as eluent to give the title compound (1.06 g, 0.929 mmol, 94 %) as a yellow oil.

¹H NMR (300 MHz, CDCl₃) δ4.51 (s, 1H), 3.53 (t, *J* = 6.6 Hz, 1H), 3.46-3.31 (m, 4H), 3.19 (t, *J* = 6.9 Hz, 1H), 3.15-3.02 (m, 2H), 1.89-1.70 (m, 2H), 1.68-1.53 (m, 4H), 1.50-1.36 (m, 20H), 1.35-1.15 (m, 9H).

### Step 4: Preparation of tert-butyl (8-((6-aminohexyl)oxy)octyl)carbamate

A solution of the compound prepared in step 3 above (1.06 g, 2.32 mmol) in 7N NH3 in MeOH (10.0 mL) was stirred at 50 °C for 16 h. The reaction mixture was treated with 10% aqueous Na2S2O3 and extracted with EtOAc. The extract was washed with brine and then dried over Na2SO4. After evaporation of the solvent, the title compound (367 mg, 1.06 mmol, 49 %) was obtained as a yellow solid.

¹H NMR (300 MHz, CDCl₃) δ7.69 (br s, 2H), 4.59 (br s, 1H), 3.45-3.30 (m, 4H), 3.22-2.96 (m, 4H), 1.99-1.77 (m, 2H), 1.71-1.53 (m, 2H), 1.49-1.39 (m, 15H), 1.36-1.16 (m, 8H).

### Step 5: Preparation of tert-butyl (8-((6-((2-(2,6-dioxopiperidin-3-yl)-1,3-dioxoisoindolin-4-yl)amino)hexyl)oxy)octyl)carbamate

To a solution of the compound (367 mg, 1.06 mmol) prepared in step 4 above in DMSO (4 mL), F-thalidomide (2-(2,6-dioxopiperidin-3-yl)-4-fluoroisoindolin-1,3-dione) (351 mg, 1.27 mmol) and DIPEA (0.37 mL, 2.12 mmol) were added. The mixture was stirred at 90 °C overnight. The reaction was quenched and then extracted with EtOAc (2×25 mL). The combined organic layers were dried over Na2SO4 and the solvent was removed in vacuo. The crude mixture was purified by silica gel column chromatography using EtOAc/Hex (1/1) as eluent to give the title compound (108 mg, 0.18 mmol, 17 %) as a yellow solid.

¹H NMR (300 MHz, CDCl₃) δ 8.10 (br s, 1H), 7.53-7.44 (m, 1H), 7.08 (d, *J* = 6.9 Hz, 1H), 6.88 (d, *J* = 8.4 Hz, 1H), 6.22 (t, *J* = 5.7 Hz, 1H), 4.96-4.85 (m, 1H), 4.05 (br s, 1H), 3.45-3.32 (m, 4H), 3.26 (q, *J* = 6.6 Hz, 2H), 3.18-3.05 (m, 2H), 2.97-2.70 (m, 3H), 2.20-2.07 (m, 1H), 1.73-1.64 (m, 2H), 1.61-1.51 (m, 4H) 1.51-1.37 (m, 18H), 1.36-1.17 (m, 10H).

### Step 6: Preparation of 4-((6-((8-(chloro-14-azaneyl)octyl)oxy)hexyl)amino)-2-(2,6-dioxopiperidin-3-yl)isoindolin-1,3-dione

To a solution of the compound prepared in step 5 above (108 mg, 0.18 mmol) in DCM (4 mL) was added 4 M HCl in dioxane (2.0 mL) and stirred at room temperature for 1 hour. The solvent was removed in vacuo to give the desired compound (88 mg, 0.16 mmol, 92%) as a yellow solid.

¹H NMR (500 MHz, CD₃OD) δ 7.61-7.51 (m, 1H), 7.17-7.05 (m, 2H), 5.11-5.05 (m, 1H), 3.78-3.69 (m, 1H), 3.68-3.61(m, 3H), 3.60-3.53 (m, 1H), 3.45-3.61(m, 4H), 3.35-3.26 (m, 4H), 2.95-2.83 (m, 2H), 2.83-2.59 (m, 3H) 2.17-2.04 (m, 1H), 1.71-1.45 (m, 7H), 1.47-1.38 (m, 4H1.38-1.23 (m, 8H).

### Step 7: Preparation of N-(8-(6-(2-(2,6-dioxopiperidin-3-yl)-1,3-dioxoisoindolin-4-ylamino)hexyloxy)octyl)-4-(5-(furan-2-ylmethylamino)-[1,2,41triazolo[4,3-f]pyrimidin-8-yl)benzamide

In a solution of 4-(5-(furan-2-ylmethylamino)-[1,2,4]triazolo[4,3-f]pyrimidin-8-yl)benzoate (30 mg, 0.087 mmol) in DMF, the compound prepared in step 6 (47 mg, 0.087 mmol), HATU (50 mg, 0.130 mmol), and DIPEA (0.060 mL, 0.348 mmol) were added and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 4 % to give the title compound (24 mg, 0.029 mmol, 33 %) as a yellow solid.

¹H NMR (500 MHz, CDC13) δ 8.34 (s, 1H), 8.24 (s, 1H), 8.05-8.01 (m, 2H), 7.90-7.85 (m, 2H), 7.51-7.45 (m, 1H), 7.43-7.39 (m, 1H), 7.08 (d, *J* = 7.0 Hz, 1H), 6.87 (d, *J* = 8.6 Hz, 1H), 6.62 (t, *J* = 5.8 Hz, 1H), 6.42-6.34 (m, 2H), 6.27-6.16 (m, 2H), 4.95-4.86 (m, 3H), 3.50-3.43 (m, 2H), 3.43-3.37 (m, 4H), 3.29-3.22 (m, 2H), 2.92-2.70 (m, 3H), 2.16-2.07 (m, 1H), 1.74-1.52 (m, 10H), 1.48-1.29 (m, 10H) ; LC/MS (ESI) *m*/*z* 818.1 [M+H]⁺, 816.1 [M-H]⁻

### <Example 41> Preparation of N-(6-(2-(2-(2-(2-(2,6-dioxopiperidin-3-yl)-1,3-dioxoisoindolin-4-ylamino)ethoxy)ethoxy)ethoxy)hexyl)-4-(5-(furan-2-ylmethylamino)-[1,2,4]triazolo[4,3-f]pyrimidin-8-yl)benzamide

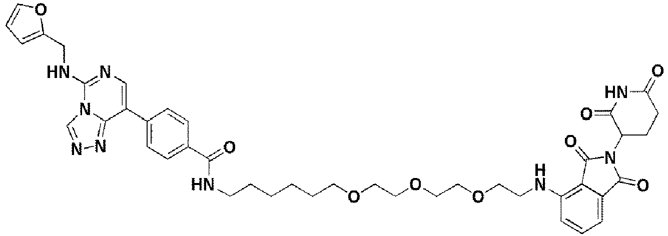

### Step 1: Preparation of tert-butyl (6-hydroxyhexyl)carbamate

To a solution of 6-aminohexane-1-ol (1.00 g, 8.53 mmol) in THF (10 mL) was added Boc O (2.35 mL, 10.2 mmol) and TEA (1.78 mL, 12.8 mmol) at 0 °C. The mixture was stirred at room temperature for 1 h. The reaction was quenched with water and extracted with EtOAc (2 X 25 mL). The collected organic layer was dried over Na2SO4 and the solvent was removed in vacuo. The crude mixture was purified through silica gel column chromatography using EtOAc/Hex (1/1) as an eluent to give the title compound (1.54 g, 7.08 mmol, 83%) as a colorless oil.

¹H NMR (300 MHz, CDCl₃) δ 5.41-5.01 (m, 1H), 3.80-3.63 (m, 2H), 3.64-3.45 (m, 4H), 3.44-3.20 (m, 2H), 1.49-1.37 (m, 9H).

### Step 2: Preparation of 6-((tert-butoxycarbonyl)amino)hexyl 4-methylbenzensulfonate

To a solution of the compound prepared in the previous step (1.54 g, 7.09 mmol) in DCM (10 mL), TsCl (2.70 g, 14.2 mmol), TEA (2.96 mL, 21.3 mmol) and DMAP (86 mg, 0.71 mmol) was added at 0 °C. Then, the solution was stirred at room temperature for 12 h. The reaction was quenched with water and extracted with DCM (2 X 25 mL). The collected organic layer was dried over Na2SO4 and the solvent was removed in vacuo. The crude mixture was purified through silica gel column chromatography using EtOAc/Hex (3/7) as an eluent to give the title compound (2.59 g, 6.97 mmol, 98%) as a white solid.

¹H NMR (300 MHz, CDCl₃) δ 7.78 (d, *J* = 8.1 Hz, 2H), 7.35 (d, *J* = 8.1 Hz, 2H), 4.49 (br s, 1H), 4.01 (t, *J* = 6.3 Hz, 2H), 3.06 (t, *J* = 6.6 Hz, 2H), 2.45 (s, 3H), 1.70-1.57 (m, 2H), 1.43 (s, 9H), 1.42-1.36 (m, 2H), 1.36-1.21 (m, 4H).

### Step 3: Preparation of tert-butyl (6-(2-(2-(2-hydroxyethoxy)ethoxy)ethoxy)hexyl)carbamate

To a solution of the compound prepared in the previous step (1.50 g, 4.03 mmol) dissolved in triethylene glycol (10.0 mL), NaOH (485 mg, 12.1 mmol) was added. The mixture was stirred at 60 ° for 2 h. The reaction was quenched with water and extracted with DCM (2 X 25 mL). The collected organic layer was dried over Na2SO4 and the solvent was removed in vacuo. The crude mixture was purified through silica gel column chromatography using DCM/MeOH (9/1) as an eluent to give the title compound (1.22 g, 3.49 mmol, 87%) as a colorless oil.

¹H NMR (300 MHz, CDCl₃) δ 4.86 (br s, 1H), 3.76-3.68 (m, 2H), 3.68-3.64 (m, 5H), 3.64-3.54 (m, 5H), 3.44 (t, *J* = 6.6 Hz, 2H), 3.24-3.13 (m, 1H), 3.13-3.03 (m, 2H), 1.64-1.52 (m, 2H), 1.51-1.46 (m, 2H), 1.44 (s, 9H), 1.39-1.27 (m, 4H).

### Step 4: Preparation of 2,2-dimethyl-4-oxo-3,12,15,18-tetraoxa-5-azaicosan-20-yl 4-methylbenzensulfonate

To a solution of the compound prepared in the previous step (1.22 g, 3.49 mmol) in DCM (10 mL), TsCl (1.33 g, 6.98 mmol), TEA (1.45 mL, 10.5 mmol) and DMAP (43 mg, 0.35 mmol) was added at 0 °C. Then, the solution was stirred at room temperature for 12 h. The reaction was quenched with water and extracted with DCM (2 X 25 mL). The collected organic layer was dried over Na2SO4 and the solvent was removed in vacuo. The crude mixture was subjected to silica gel column chromatography using EtOAc/Hex (3/7) as an eluent to give the title compound (1.68 g, 3.34 mmol, 96%) in the form of a colorless oil.

¹H NMR (300 MHz, CDCl₃) δ 7.79 (d, *J* = 7.5 Hz, 2H), 7.34 (d, *J* = 7.8 Hz, 2H), 4.54 (br s, 1H), 4.18-4.11 (m, 2H), 3.74-3.65 (m, 2H), 3.64-3.38 (m, 6H), 3.43 (t, *J* = 6.3 Hz, 2H), 3.14-3.06 (m, 2H), 2.44 (s, 3H), 1.61-1.53 (m, 4H), 1.47-1.39 (m, 10H), 1.39-1.29 (m, 2H).

### Step 5: Preparation of tert-butyl (6-(2-(2-(2-iodoethoxy)ethoxy)ethoxy)hexyl)carbamate

To a solution of the compound prepared in the previous step (1.68 g, 3.34 mmol) dissolved in acetone (20 mL), sodium iodide (2.50 mg, 16.7 mmol) was added, and the mixture was stirred at 80° for one day. The reaction mixture was concentrated to remove the solvent. The reaction was quenched with water and extracted with EtOAc (2 X 25 mL). The collected organic layer was dried over Na2SO4, and the solvent was removed in vacuo to prepare the title compound (1.41 g, 3.07 mmol, 92%) as a yellow oil.

¹H NMR (500 MHz, CDCl₃) δ 4.60 (s, 1H), 3.76 (t, *J* = 6.9 Hz, 2H), 3.68-3.66 (m, 4H), 3.66-3.26 (m, 2H), 3.60-3.55 (m, 2H), 3.45 (t, *J* = 6.6 Hz, 2H), 3.26 (t, *J* = 6.9 Hz, 2H), 3.09 (t, *J* = 6.6 Hz, 2H), 1..63-1.53 (m, 2H), 1.51-1.45 (m, 2H), 1.44 (s, 9H), 1.39-1.29 (m, 4H).

### Step 6: Preparation of tert-butyl (6-(2-(2-(2-aminoethoxy)ethoxy)ethoxy)hexyl)carbamate

A solution of the compound prepared in the previous step (1.41 g, 3.51 mmol) dissolved in MeOH (14.0 mL), 7 N NH3 was stirred at 50° for 16 h. The reaction mixture was concentrated to remove the solvent. The crude mixture was purified by silica gel column chromatography using DCM/MeOH (9/1) as an eluent to give the title compound (760 mg, 2.18 mmol, 71%) as a yellow oil.

¹H NMR (300 MHz, CDCl₃) δ 7.64 (br s, 2H), 4.71 (s, 1H), 3.96 (t, *J* = 5.1 Hz, 2H), 3.85-58 (m, 8H), 3.53 (t, *J* = 6.6 Hz, 2H), 3.26 (t, *J* = 6.9 Hz, 2H), 3.33 (t, *J* = 5.1 Hz, 2H), 3.10 (q, *J =* 6.6 Hz, 2H), 1..72-1.55 (m, 2H), 1.55-1.46 (m, 2H), 1.46 (s, 9H), 1.40-1.38 (m, 4H).

### Step 7: Preparation of tert-butyl (6-(2-(2-(2-aminoethoxy)ethoxy)ethoxy)hexyl)carbamate

In a solution of the compound prepared in the previous step (300 mg, 0.86 mmol) dissolved in DMSO (5 mL), 4-fluoro-2-(2,6-dioxopiperidin-3-yl)isoindolin-1,3-dione (285 mg, 1.03 mmol) and DIPEA (0.30 mL, 1.72 mmol) were added. The mixture was stirred at 90 ° for one day. The reaction was quenched with water and extracted with EtOAc (2 X 25 mL). The collected organic layer was dried over Na2SO4 and the solvent was removed in vacuo. The crude mixture was purified by silica gel column chromatography using EtOAc/Hex (1/1) as an eluent to give the title compound (155 mg, 0.26 mmol, 30%) as a yellow solid.

¹H NMR (500 MHz, CDCl₃) δ 8.22 (s, 1H), 7.25-7.45 (m, 1H), 7.10 (d, *J* = 7.0 Hz, 1H), 6.93 (d, *J* = 8.5 Hz, 1H), 6.48 (t, *J* = 11.0 Hz, 1H), 4.94-4.88 (m, 1H), 4.55 (br s, 1H), 3.72 (t, *J* = 5.5 Hz, 2H), 3.69-3.37 (m, 4H), 3.65-3.62 (m, 2H), 3.59-3.54 (m, 2H), 3.47 (q, *J* = 5.5 Hz, 2H), 3.43 (t, *J* = 6.5 Hz, 2H), 3.14-3.04 (m, 2H), 2.93-2.68 (m, 3H), 2.15-2.08 (m, 1H), 1.75-1.66 (m, 1H), 1.61-1.50 (m, 4H) 1.50-1.45 (m, 2H), 1.43 (s, 9H), 1.37-1.27 (m, 4H).

### Step 8: Preparation of 4-((2-(2-(2-((6-aminohexyl)oxy)ethoxy)ethoxy)ethyl)amino)-2-(2,6-dioxopiperidin-3-yl)isoindolin-1,3-dione

To a solution of the compound prepared in the previous step (155 mg, 0.26 mmol) dissolved in DCM (4 mL) was added 4 M HCl dissolved in dioxane (2.0 mL), and stirred at room temperature for 1 h. The solvent was removed under vacuum to prepare the title compound (129 mg, 0.24 mmol, 92%) as a yellow solid.

¹H NMR (500 MHz, DMSO-*d6*) δ 11.0 (s, 1H), 7.76 (br s, 3H), 7.61-7.53. (m, 1H), 7.14(d, *J* = 8.7 Hz, 1H), 7.04 (d, *J* = 7.8 Hz, 1H), 6.60 (t, *J* =6.0 Hz, 1H), 5.10-5.00 (m, 1H), 3.81-3.65 (m, 2H), 3.65-3.58 (m, 2H), 3.57-3.55 (m, 5H), 3.55-3.40 (m, 12H), 2.97-2.80 (m, 1H), 2.79-2.67 (m, 2H) 2.63-2.53 (m, 2H), 2.08-1.95 (m, 1H), 1.58-1.38 (m, 4H), 1.34-1.26 (m, 5H).

### Step 9: Preparation of N-(6-(2-(2-(2-(2-(2,6-dioxopiperidin-3-yl)-1,3-dioxoisoindolin-4-ylamino)ethoxy)ethoxy)ethoxy)hexyl)-4-(5-(furan-2-ylmethylamino)-[1,2,41triazolo[4,3-f)pyrimidin-8-yl)benzamide

A solution of 4-(5-((furan-2-yl)methylamino)-[1,2,4]triazolo[4,3-f]pyrimidin-8-yl)benzoate (20 mg, 0.059 mmol) in DMF To, the compound prepared in the previous step (31 mg, 0.059 mmol), EDCI HCl (12 mg, 0.065 mmol), HOBt HO (10 mg, 0.065 mmol), DIPEA (0.041 mL, 0.236 mmol) was added, and at room temperature Stirred for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 5% to prepare the title compound (19 mg, 0.023 mmol, 39%) as a yellow solid.

¹H NMR (500 MHz, CDCl₃) δ 8.34 (s, 1H), 8.28 (s, 1H), 8.23 (s, 1H), 8.06-8.00 (m, 2H), 7.92-7.85 (m, 2H), 7.48 (dd, *J* = 8.5, 7.1 Hz, 1H), 7.44-7.39 (m, 1H), 7.09 (d, *J* = 7.1 Hz, 1H), 6.92 (d, *J* = 8.5 Hz, 1H), 6.59 (t, *J* = 5.9 Hz, 1H), 6.48 (t, *J* = 5.7 Hz, 1H), 6.42-6.35 (m, 2H), 6.28 (t, *J* = 5.7 Hz, 1H), 4.94-4.86 (m, 3H), 3.72 (t, *J* = 5.4 Hz, 2H), 3.67 (s, 4H), 3.67-3.62 (m, 2H), 3.60-3.55 (m, 2H), 3.52-3.42 (m, 6H), 2.90-2.66 (m, 3H), 2.15-2.08 (m, 1H), 1.68-1.60 (m, 4H), 1.45-1.35 (m, 4H) ; LC/MS (ESI) *m*/*z* 822.1 [M+H]⁺, 820.1 [M-H]⁻

### <Example 42> Preparation of N-(1-(2-(2,6-dioxopiperidin-3-yl)-1,3-dioxoisoindolin-4-ylamino)-3,6,9,12,15,18-hexaoxadocosan-22-yl)-4-(5-(furan-2-ylmethylamino)-[1,2,4]triazolo[4,3-f]pyrimidin-8-yl)benzamide

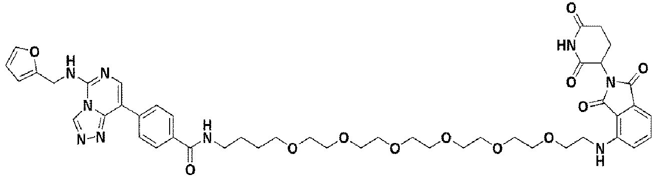

### Step 1: Preparation of tert-butyl (4-hydroxybutyl)carbamate

A solution of 4-aminobutane-1-ol (3.0 g, 33.65 mmol), BocO (14 mL, 47.12 mmol), TEA (5.3 mL, 50.48 mmol) in THF (25.0 mL) was stirred at 25 °C for 1 h. The reaction mixture was diluted with water and extracted with EtOAc. The combined organic phases were dried over Na2SO4, filtered, concentrated and purified by silica chromatography (0-70% gradient of MeOH/DCM) to afford the title compound (4.87 g, 25.73 mmol, 76%).

¹H NMR (300 MHz, CDCl₃) δ 4.64 (s, 1H), 3.72-3.63 (m, 2H), 3.16 (d, *J* = 6.3 Hz, 2H), 1.74 (s, 1H), 1.66-1.54 (m, 4H), 1.44 (s, 9H).

### Step 2: Preparation of 4-((tert-butoxycarbonyl)amino)butyl 4-methylbenzensulfonate

A solution of DMAP (314 mg, 2.57 mmol), TsCl (9.8 g, 51.46 mmol), TEA (11 ml, 77.20 mmol), compound prepared in step 1 above (4.9 g, 25.73 mmol) in DCM (50.0 mL) The mixture was stirred at 25° C. for 16 hours. The reaction mixture was diluted with water and extracted with DCM. The combined organic phases were dried over Na2SO4, filtered, concentrated and purified by silica chromatography (0-30% gradient of EA/HEX) to give the title compound (6.71 g, 19.54 mmol, 76%).

¹H NMR (300 MHz, CDCl₃) δ 7.80 (d, *J* = 8.5 Hz, 2H), 7.37 (d, *J* = 7.9 Hz, 2H), 4.53 (s, 1H), 4.05 (t, *J* = 6.3 Hz, 2H), 3.10 (q, *J* = 6.5 Hz, 2H), 2.47 (s, 3H), 1.73-1.61 (m, 2H), 1.58-1.47 (m, 2H), 1.44 (s, 9H).

### Step 3: Preparation of tert-butyl (1-hydroxy-3,6,9,12-tetraoxahexadecane-16-ylcarbamate

A solution of the compound prepared in step 2 (11.2 g, 32.58 mmol) and NaOH (3.9 g, 97.7 mmol) in tetraethyleneglycol (112 mL) was stirred at 60 °C for 2 hours. The reaction mixture was diluted with brine and extracted with EtOAc. The combined organic phases were dried over Na2SO4, filtered, concentrated and purified by silica chromatography (0-15 % gradient of MeOH/DCM) to give the title compound (3.9 g, 10.7 mmol, 33 %).

### Step 4: Preparation of 2,2-dimethyl-4-oxo-3,10,13,16,19-pentaoxa-5-azahenicosan-21-yl 4-methylbenzensulfonate

A solution of the compound prepared in step 3 above (3.9 g, 10.67 mmol), TsCl (4.06 g, 21.34 mmol), TEA (4.46 mL, 32.01 mmol) and DMAP (130 mg, 1.06 mmol) in DCM (20.0 ml) The mixture was stirred at 25 °C for 16 hours. The reaction mixture was diluted with brine and extracted with EtOAc. The combined organic phases were dried over Na2SO4, filtered, concentrated and purified by silica chromatography (0-15 % MeOH/DCM gradient) to give the title compound (5.12 g, 9.81 mmol, 91 %).

¹H NMR (300 MHz, CDCl₃) δ 7.80 (d, *J* = 8.4 Hz, 2H), 7.42-7.30 (m, 2H), 4.82-4.67 (m, 1H), 4.21-4.11 (m, 2H), 3.74-3.52 (m, 15H), 3.47 (t, *J* = 6.2 Hz, 2H), 3.12 (q, *J* = 6.6 Hz, 2H), 2.45 (s, 3H), 1.69-1.49 (m, 4H), 1.43 (s, 10H).

### Step 5: Preparation of tert-butyl (1-hydroxy-3,6,9,12,15,18-hexaoxadocosan-22-ylcarbamate

A solution of the compound prepared in step 4 (1.7 g, 3.27 mmol) and NaOH (392 mg, 9.81 mmol) in diethyleneglycol (17 mL) was stirred at 60 °C for 2 hours. The reaction mixture was diluted with brine and extracted with EtOAc. The combined organic phases were dried over Na2SO4, filtered, concentrated and purified by silica chromatography (0-15% gradient of MeOH/DCM) to afford the desired compound.

¹H NMR (300 MHz, CDCl-₃) δ 4.85 (s, 1H), 3.78-3.54 (m, 24H), 3.47 (t, *J* = 6.2 Hz, 2H), 3.13 (q, *J* = 6.5 Hz, 2H), 2.92 (s, 1H), 1.66-1.50 (m, 4H), 1.44 (s, 9H).

### Step 6: Preparation of 2,2-dimethyl-4-oxo-3,10,13,16,19,22,25-heptaoxa-5-azaheptacosan-27-yl4-methylbenzensulfonate

A solution of the compound prepared in step 5 (1.24 g, 2.64 mmol), TsCl (1.0 g, 5.28 mmol), TEA (1.1 mL, 7.92 mmol) and DMAP (32 mg, 0.26 mmol) in DCM (20.0 ml) The mixture was stirred at 25 °C for 16 hours. The reaction mixture was diluted with brine and extracted with EtOAc. The combined organic phases were dried over NaSO 4 , filtered, concentrated and silica chromatography (0-15 % gradient of MeOH/DCM) gave the title compound (1.24 mg, 2.04 mmol, 77%).

¹H NMR (300 MHz, CDCl₃) δ 7.80 (d, *J* = 8.4 Hz, 2H), 7.35 (d, *J* = 8.1 Hz, 2H), 4.71 (s, 1H), 4.19-4.12 (m, 2H), 3.71-3.67 (m, 2H), 3.66-3.61 (m, 14H), 3.58 (s, 6H), 3.47 (t, *J* = 6.2 Hz, 2H), 3.13 (q, *J* = 6.3 Hz, 2H), 2.45 (s, 3H), 1.67-1.50 (m, 4H), 1.44 (s, 9H).

### Step 7: Preparation of tert-butyl (1-iodo-3,6,9,12,15,18-hexaoxadocosan-22-yl)carbamate

A solution of the compound prepared in step 6 above (1.24 g, 2.04 mmol) and sodium iodide (18.3 g, 12.24 mmol) in acetone (50 mL) was stirred with N2 for 4 h. The reaction solvent was removed. The reaction mixture was then diluted with water and extracted with DCM. The combined organic phases were combined, washed with brine, dried over Na2SO4 and the solvent removed in vacuo to give the desired compound (1.06 g, 1.88 mmol, 92%).

¹H NMR (300 MHz, CDCl₃) δ 4.75 (s, 1H), 3.76 (t, *J* = 6.9 Hz, 2H), 3.69-3.62 (m, 18H), 3.60-3.56 (m, 2H), 3.47 (t, *J* = 6.2 Hz, 2H), 3.32-3.23 (m, 2H), 3.13 (d, *J* = 6.2 Hz, 2H), 1.66-1.52 (m, 4H), 1.44 (s, 9H).

### Step 8: Preparation of tert-butyl (1-amino-3,6,9,12,15,18-hexaoxadocosan-22-ylcarbamate

A solution of the compound prepared in step 7 above (1.06 g, 1.88 mmol) and 7 N NH3 in MeOH (10 mL) was stirred at 50° C. for 16 h. The reaction solvent was removed. The reaction mixture was then purified by silica chromatography (0-15 % gradient of MeOH/DCM) to give the title compound (543 mg, 1.20 mmol, 63 %).

¹H NMR (300 MHz, CDCl₃) δ 7.51 (s, 2H), 4.89 (s, 1H), 4.01-3.93 (m, 2H), 3.80-3.62 (m, 20H), 3.50 (q, *J* = 6.3 Hz, 2H), 3.30-3.20 (m, 2H), 3.14 (q, *J* = 6.7 Hz, 2H), 1.67-1.50 (m, 4H), 1.44 (s, 9H).

### Step 9: Preparation of tert-butyl (1-((2-(2,6-dioxopiperidin-3-yl)-1,3-dioxoisoindolin-4-yl)amino)-3,6,9,12,15,18-hexaoxadocosan-22-yl)carbamate

To a solution of the compound prepared in step 8 (200 mg, 0.442 mmol) in DMF, F-thalidomide (122 mg, 0.442 mmol) and DIPEA (0.115 mL, 0.663 mmol) were added and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using DCM/MeOH 4% to give the title compound (103mg, 0.145mmol, 33%) as a yellow oil.

¹H NMR (300 MHz, CDCl₃) δ 8.27 (s, 1H), 7.49 (dd, *J* = 8.5, 7.2 Hz, 1H), 7.11 (d, *J* = 7.2 Hz, 1H), 6.92 (d, *J* = 8.5 Hz, 1H), 6.50 (t, *J* = 5.7 Hz, 1H), 4.97-4.85 (m, 1H), 4.71 (s, 1H), 3.72 (t, *J* = 5.3 Hz, 2H), 3.70-3.60 (m, 18H), 3.60-3.53 (m, 2H), 3.53-3.39 (m, 4H), 3.21-3.05 (m, 2H), 2.97-2.67 (m, 3H), 2.18-2.07 (m, 1H), 1.67-1.50 (m, 4H), 1.44 (s, 9H).

### Step 10: Preparation of 4-((22-amino-3,6,9,12,15,18-hexaoxadocosyl)amino)-2-(2,6-dioxopiperidin-3-yl)isoindolin-1,3-dione hydrochloride

To a solution of the compound prepared in step 9 above (103 mg, 0.145 mmol) in DCM (4 mL) was added 4 N HCl in 1,4-dioxane (2 mL) and stirred at room temperature for 3 hours. The reaction mixture was concentrated in vacuo. The solid was filtered with DCM under reduced pressure to give the title compound (75 mg, 0.116 mmol, 80 %) as a yellow oil.

¹H NMR (300 MHz, CD₃OD) δ 7.60 (dd, *J* = 8.6, 7.1 Hz, 1H), 7.12 (t, *J* = 8.0 Hz, 2H), 5.14-5.02 (m, 1H), 3.78 (t, *J* = 5.2 Hz, 2H), 3.76-3.59 (m, 20H), 3.59-3.50 (m, 4H), 3.08-2.98 (m, 2H), 2.93-2.64 (m, 3H), 2.19-2.09 (m, 1H), 1.87-1.70 (m, 4H).

### Step 11: Preparation of N-(1-(2-(2,6-dioxopiperidin-3-yl)-1,3-dioxoisoindolin-4-ylamino)-3,6,9,12,15,18-hexaoxadocosan-22-yl)-4-(5-(furan-2-ylmethylamino)-[1,2,4]triazolo[4,3-f]pyrimidin-8-yl)benzamide

In a solution of 4-(5-(furan-2-ylmethylamino)-[1,2,4]triazolo[4,3-f]pyrimidin-8-yl)benzoate (20 mg, 0.059 mmol) in DMF, the above steps 10 (38 mg, 0.059 mmol), EDCl HCl (12 mg, 0.065 mmol), HOBt HO (10 mg, 0.065 mmol), and DIPEA (0.041 mL, 0.236 mmol) were added, and at room temperature for 2 hours. stirred. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 5 % to give the title compound (29 mg, 0.031 mmol, 53 %) as a yellow solid.

¹H NMR (300 MHz, CDCl₃) δ 8.76 (s, 1H), 8.33 (s, 1H), 8.23 (s, 1H), 8.07-7.98 (m, 2H), 7.96-7.87 (m, 2H), 7.46 (dd, *J* = 8.5, 7.1 Hz, 1H), 7.42-7.37 (m, 1H), 7.07 (d, *J* = 7.1 Hz, 1H), 6.89 (d, *J* = 8.5 Hz, 1H), 6.78 (t, *J* = 5.7 Hz, 1H), 6.69 (t, *J* = 5.8 Hz, 1H), 6.48 (t, *J* = 5.6 Hz, 1H), 6.42-6.33 (m, 2H), 4.96-4.85 (m, 3H), 3.69 (t, *J* = 5.4 Hz, 2H), 3.67-3.58 (m, 20H), 3.58-3.48 (m, 4H), 3.48-3.36 (m, 2H), 2.92-2.68 (m, 3H), 2.16-2.05 (m, 1H), 1.81-1.64 (m, 4H) ; LC/MS (ESI) *m*/*z* 926.1 [M+H]⁺, 924.1 [M-H]⁻

### <Example 43> Preparation of N-(25-(2-(2,6-dioxopiperidin-3-yl)-1,3-dioxoisoindolin-4-ylamino)-24-oxo-3,6,9,12,15,18-hexaoxa-23-azapentacosyl)-4-(5-(furan-2-ylmethylamino)-[1,2,4]triazolo[4,3-f]pyrimidin-8-yl)benzamide

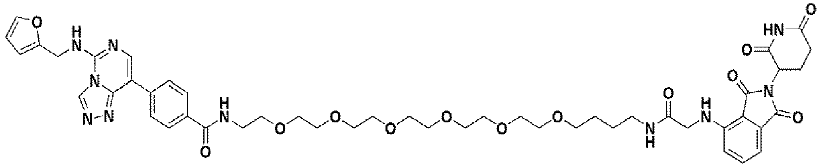

### Step 1: Preparation of tert-butyl (1-(4-(5-((furan-2-ylmethyl)amino)-[1,2,41triazolo[4,3-c]pyrimidin-8-yl)phenyl)-1-oxo-5,8,11,14,17,20-hexaoxa-2-azatetracosan-24-yl)carbamate

In a solution of 4-(5-(furan-2-ylmethylamino)-[1,2,4]triazolo[4,3-f]pyrimidin-8-yl)benzoic acid (100mg, 0.298mmol) in DMF Compound prepared in step 8 of 42 (135mg, 0.298mmol), EDCI HCl (62mg, 0.327mmol), HOBt HO (50mg, 0.327mmol), DIPEA (0.207 mL, 1.19mmol) was added, and at room temperature for 2 hours stirred. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 4 % to give the title compound (100 mg, 0.130 mmol, 43 %) as a yellow solid.

¹H NMR (300 MHz, CDCl₃) δ 8.33 (s, 1H), 8.24 (s, 1H), 8.09-8.00 (m, 2H), 7.98-7.90 (m, 2H), 7.45-7.38 (m, 1H), 7.01 (s, 1H), 6.70-6.58 (m, 1H), 6.42-6.33 (m, 2H), 4.90 (d, *J* = 5.8 Hz, 2H), 4.74 (s, 1H), 3.74-3.58 (m, 22H), 3.58-3.52 (m, 2H), 3.45 (t, *J* = 6.0 Hz, 2H), 3.12 (q, *J* = 6.4 Hz, 2H), 1.65-1.47 (m, 4H), 1.43 (s, 9H).

### Step 2: Preparation of N-(22-amino-3,6,9,12,15,18-hexaoxadocosyl)-4-(5-((furan-2-ylmethyl)amino)-[1,2,4]triazolo[4,3-c]pyrimidin-8-yl)benzamide hydrochloride

To a solution of the compound prepared in step 1 above (100 mg, 0.130 mmol) in DCM (4 mL) was added 4 N HCl in 1,4-dioxane (2 mL) and stirred at room temperature for 1 hour. The reaction mixture was concentrated in vacuo. The solid was filtered under reduced pressure with DCM to give the title compound (101 mg, quant.) as a yellow oil.

¹H NMR (300 MHz, CD₃OD) δ 8.77 (s, 1H), 8.39 (s, 1H), 8.06-7.97 (m, 4H), 7.51-7.46 (m, 1H), 6.49-6.43 (m, 1H), 6.43-6.36 (m, 1H), 4.92 (s, 2H), 3.79-3.66 (m, 20H), 3.62-3.55 (m, 6H), 3.08-2.96 (m, 2H), 1.92-1.68 (m, 4H).

### Step 3: Preparation of N-(25-(2-(2,6-dioxopiperidin-3-yl)-1,3-dioxoisoindolin-4-ylamino)-24-oxo-3,6,9,12,15,18-hexaoxa-23-azapentacosyl)-4-(5-(furan-2-ylmethylamino)-[1,2,4]triazolo[4,3-f]pyrimidin-8-yl)benzamide

In a solution of 2-(2-(2,6-dioxopiperidin-3-yl)-1,3-dioxoisoindolin-4-ylamino)acetic acid (23 mg, 0.070 mmol) in DMF, the compound prepared in step 2 ( 50 mg, 0.070 mmol), EDCI HCl (15 mg, 0.077 mmol), HOBt HO (12 mg, 0.077 mmol), DIPEA (0.048 mL, 0.280 mmol) were added and stirred at room temperature for 2 h. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 7 % to give the title compound (16 mg, 0.016 mmol, 23 %) as a yellow solid.

¹H NMR (500 MHz, CDCl₃) δ 8.79 (s, 1H), 8.33 (s, 1H), 8.23 (s, 1H), 8.05-7.99 (m, 2H), 7.98-7.91 (m, 2H), 7.50 (dd, *J* = 8.4, 7.1 Hz, 1H), 7.43-7.38 (m, 1H), 7.31-7.28 (m, 1H), 7.16 (d, *J* = 7.1 Hz, 1H), 6.97 (t, *J* = 5.9 Hz, 1H), 6.82-6.75 (m, 2H), 6.70 (t, *J* = 5.8 Hz, 1H), 6.42-6.34 (m, 2H), 4.95-4.85 (m, 3H), 3.95 (d, *J* = 5.9 Hz, 2H), 3.73-3.57 (m, 20H), 3.57-3.52 (m, 2H), 3.49-3.43 (m, 2H), 3.38 (t, *J* = 5.7 Hz, 2H), 3.28 (q, *J* = 6.3 Hz, 2H), 2.91-2.68 (m, 3H), 2.15-2.09 (m, 1H), 1.53 (m, 4H).

### <Example 44> Preparation of N-(25-(2-(2,6-dioxopiperidin-3-yl)-1,3-dioxoisoindolin-4-yloxy)-24-oxo-3,6,9,12,15,18-hexaoxa-23-azapentacosyl)-4-(5-(furan-2-ylmethylamino)-[1,2,4]triazolo[4,3-f]pyrimidin-8-yl)benzamide

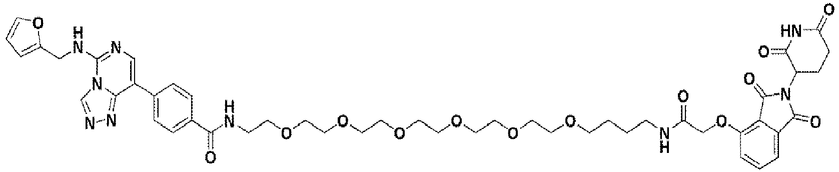

### Step 1: Preparation of tert-butyl (1-(4-(5-((furan-2-ylmethyl)amino)-[1,2,4]triazolo[4,3-c]pyrimidin-8-yl)phenyl)-1-oxo-5,8,11,14,17,20-hexaoxa-2-azatetracosan-24-yl)carbamate

4-(5-((furan-2-yl)methylamino)-[1,2,4]triazolo[4,3-f]pyrimidin-8-yl)benzoic acid (100 mg, 0.298 mmol) dissolved in DMF ) solution, the compound prepared in step 8 of Example 42 (135 mg, 0.298 mmol), EDCI HCl (62 mg, 0.327 mmol), HOBt HO (50 mg, 0.327 mmol), DIPEA (0.207 mL, 1.19 mmol)) was added and stirred at room temperature for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 4% to prepare the title compound (100 mg, 0.130 mmol, 43%) as a yellow solid.

¹H NMR (300 MHz, CDCl₃) δ 8.33 (s, 1H), 8.24 (s, 1H), 8.09-8.00 (m, 2H), 7.98-7.90 (m, 2H), 7.45-7.38 (m, 1H), 7.01 (s, 1H), 6.70-6.58 (m, 1H), 6.42-6.33 (m, 2H), 4.90 (d, *J* = 5.8 Hz, 2H), 4.74 (s, 1H), 3.74-3.58 (m, 22H), 3.58-3.52 (m, 2H), 3.45 (t, *J* = 6.0 Hz, 2H), 3.12 (q, *J* = 6.4 Hz, 2H), 1.65-1.47 (m, 4H), 1.43 (s, 9H).

### Step 2: Preparation of N-(22-amino-3,6,9,12,15,18-hexaoxadocosyl)-4-(5-((furan-2-ylmethyl)amino)-[1,2,4]triazolo[4,3-c]pyrimidin-8-yl)benzamide hydrochloride

To a solution of the compound prepared in step 1 (100 mg, 0.130 mmol) dissolved in DCM (4 mL), 4 N HCl dissolved in 1,4-dioxane (2 mL) was added and stirred at room temperature for 1 h. did. The reaction mixture was concentrated in vacuo. The solid was filtered with DCM under reduced pressure to give the title compound (101 mg, quant.) in the form of a yellow oil.

¹H NMR (300 MHz, CD₃OD) δ 8.77 (s, 1H), 8.39 (s, 1H), 8.06-7.97 (m, 4H), 7.51-7.46 (m, 1H), 6.49-6.43 (m, 1H), 6.43-6.36 (m, 1H), 4.92 (s, 2H), 3.79-3.66 (m, 20H), 3.62-3.55 (m, 6H), 3.08-2.96 (m, 2H), 1.92-1.68 (m, 4H).

### Step 3: Preparation of N-(25-(2-(2,6-dioxopiperidin-3-yl)-1,3-dioxoisoindolin-4-yloxy)-24-oxo-3,6,9,12,15,18-hexaoxa-23-azapentacosyl)-4-(5-(furan-2-ylmethylamino)-[1,2,4]triazolo[4,3-f]pyrimidin-8-yl)benzamide

In a solution of 2-(2-(2,6-dioxopiperidin-3-yl)-1,3-dioxoisoindolin-4-yloxy)acetic acid (23 mg, 0.070 mmol) dissolved in DMF, the compound prepared in the previous step 2 (50 mg, 0.070 mmol), EDCI HCl (15 mg, 0.077 mmol), HOBt HO (12 mg, 0.077 mmol), DIPEA (0.048 mL, 0.280 mmol) were added and stirred at room temperature for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 7% to prepare the title compound (9 mg, 0.009 mmol, 13%) as a white solid.

¹H NMR (500 MHz, CDC13) δ 9.03 (s, 1H), 8.34 (s, 1H), 8.24 (s, 1H), 8.07-8.01 (m, 2H), 7.98-7.92 (m, 2H), 7.72 (dd, *J* = 8.3, 7.3 Hz, 1H), 7.57-7.51 (m, 2H), 7.43-7.39 (m, 1H), 7.18 (d, *J* = 8.3 Hz, 1H), 7.14 (s, 1H), 6.66 (t, *J* = 5.8 Hz, 1H), 6.41-6.34 (m, 2H), 5.00-4.93 (m, 1H), 4.90 (d, *J* = 5.8 Hz, 2H), 4.67-4.59 (m, 2H), 3.73-3.58 (m, 22H), 3.57-3.52 (m, 2H), 3.50-3.45 (m, 2H), 3.45-3.38 (m, 1H), 3.38-3.30 (m, 1H), 2.92-2.71 (m, 3H), 2.19-2.12 (m, 1H), 1.69-1.59 (m, 4H).

### <Example 45> Preparation of N-(1-(2-(2,6-dioxopiperidin-3-yl)-1,3-dioxoisoindolin-4-ylamino)-3,6,9,12,15,18,21-heptaoxapentacosan-25-yl)-4-(5-(furan-2-ylmethylamino)-[1,2,4]triazolo[4,3-f]pyrimidin-8-yl)benzamide

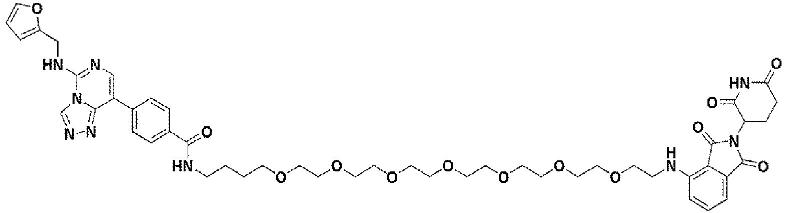

### Step 1: Preparation of tert-butyl(4-hydroxybutyl)carbamate

A solution of 4-aminobutane-1-ol (3.0 g, 33.65 mmol), BocO (14 mL, 47.12 mmol), TEA (5.3 mL, 50.48 mmol) in THF (25.0 mL) was stirred at 25 °C for 1 h. The reaction mixture was diluted with water and extracted with EtOAc. The combined organic phases were dried over Na2SO4, filtered, concentrated and purified by silica chromatography (0-70% gradient of MeOH/DCM) to afford the title compound (4.87 g, 25.73 mmol, 76%).

¹H NMR (300 MHz, CDCl₃) δ 4.64 (s, 1H), 3.72-3.63 (m, 2H), 3.16 (d, *J* = 6.3 Hz, 2H), 1.74 (s, 1H), 1.66-1.54 (m, 4H), 1.44 (s, 9H).

### Step 2: Preparation of 4-((tert-butoxycarbonyl)amino)butyl 4-methylbenzensulfonate

Compound prepared in step 1 above (4.9 g, 25.73 mmol), TsCl (9.8 g, 51.46 mmol), TEA (11 mL, 77.20 mmol), DMAP (314 mg, 2.57 mmol) mL) in DCM (50.0 mL) The mixture was stirred at 25° C. for 16 hours. The reaction mixture was diluted with water and extracted with DCM. The combined organic phases were dried over Na2SO4, filtered, concentrated and purified by silica chromatography (0-30% gradient of EA/HEX) to give the title compound (6.71 g, 19.54 mmol, 76%).

¹H NMR (300 MHz, CDCl₃) δ 7.80 (d, *J* = 8.5 Hz, 2H), 7.37 (d, *J* = 7.9 Hz, 2H), 4.53 (s, 1H), 4.05 (t, *J* = 6.3 Hz, 2H), 3.10 (q, *J* = 6.5 Hz, 2H), 2.47 (s, 3H), 1.73-1.61 (m, 2H), 1.58-1.47 (m, 2H), 1.44 (s, 9H).

### Step 3: Preparation of tert-butyl (1-hydroxy-3,6,9,12-tetraoxahexadecane-16-ylcarbamate

A solution of the compound prepared in step 2 (11.2 g, 32.58 mmol) and NaOH (3.9 g, 97.7 mmol) in tetraethyleneglycol (112 mL) was stirred at 60 °C for 2 hours. The reaction mixture was diluted with brine and extracted with EtOAc. The combined organic phases were dried over Na2SO4, filtered, concentrated and purified by silica chromatography (0-15 % gradient of MeOH/DCM) to give the title compound (3.9 g, 10.7 mmol, 33 %).

### Step 4: Preparation of 2,2-dimethyl-4-oxo-3,10,13,16,19-pentaoxa-5-azahenicosan-21-yl

### 4-methylbenzensulfonate

A mixture of the compound prepared in step 3 (3.9 g, 10.67 mmol), TsCl (4.06 g, 21.34 mmol), TEA (4.46 mL, 32.01 mmol) and DMAP (130 mg, 1.06 mmol) was stirred at 25 °C for 16 hours. stirred. The reaction mixture was diluted with brine and extracted with EtOAc. The combined organic phases were dried over Na2SO4, filtered, concentrated and purified by silica chromatography (0-15 % MeOH/DCM gradient) to give the title compound (5.12 g, 9.81 mmol, 91 %).

¹H NMR (300 MHz, CDCl₃) δ 7.80 (d, *J* = 8.4 Hz, 2H), 7.42-7.30 (m, 2H), 4.82-4.67 (m, 1H), 4.21-4.11 (m, 2H), 3.74-3.52 (m, 15H), 3.47 (t, *J* = 6.2 Hz, 2H), 3.12 (q, *J* = 6.6 Hz, 2H), 2.45 (s, 3H), 1.69-1.49 (m, 4H), 1.43 (s, 10H).

### Step 5: Preparation of tert-butyl (1-hydroxy-3,6,9,12,15,18,21-heptaoxapentacosan-25-ylcarbamate

A solution of the compound prepared in step 4 (1.7 g, 3.27 mmol) and NaOH (392 mg, 9.81 mmol) in triethyleneglycol (17ml) was stirred at 60 °C for 2 hours. The reaction mixture was diluted with brine and extracted with EtOAc. The combined organic phases were dried over Na2SO4, filtered, concentrated and purified by silica chromatography (0-15 % gradient of MeOH/DCM) to give the title compound (1.05 g, 2.11 mmol, 64 %).

¹H NMR (300 MHz, CDCl₃) δ 4.72 (s, 1H), 3.76-3.54 (m, 28H), 3.47 (t, *J* = 6.2 Hz, 2H), 3.13 (q, *J* = 6.6 Hz, 2H), 2.63 (t, *J* = 6.0 Hz, 1H), 1.66-1.50 (m, 4H), 1.44 (s, 9H).

### Step 6: Preparation of 2,2-dimethyl-4-oxo-3,10,13,16,19,22,25,28-octaoxa-5-azatriacontane-30-yl 4-methylbenzensulfonate

To a solution of the compound prepared in step 5 above (1.05 g, 2.11 mmol) in DCM (20.0 mL), TsCl (805 mg, 4.22 mmol), TEA (0.9 mL, 6.33 mmol) and DMAP (26 mg, 0.21 mmol) was stirred at 25 °C for 16 hours. The reaction mixture was diluted with brine and extracted with EtOAc. The combined organic phases were dried over Na2SO4, filtered, concentrated and purified by silica chromatography (0-15 % MeOH/DCM gradient) to give the title compound (1.21 g, 1.85 mmol, 88 %).

¹H NMR (300 MHz, CDCl₃) δ 7.80 (d, *J* = 8.3 Hz, 2H), 7.35 (d, *J* = 8.3 Hz, 2H), 4.74 (s, 1H), 4.23-4.14 (m, 2H), 3.72-3.61 (m, 20H), 3.59 (d, *J* = 2.3 Hz, 6H), 3.47 (t, *J* = 6.1 Hz, 2H), 3.13 (d, *J* = 6.3 Hz, 2H), 2.45 (s, 3H), 1.64-1.51 (m, 4H), 1.44 (s, 9H).

### Step 7: Preparation of tert-butyl (1-iodo-3,6,9,12,15,18,21-heptaoxapentacosan-25-ylcarbamate

A solution of the compound prepared in step 6 above (1.21 g, 1.85 mmol) and NaI (16.6 g, 11.1 mmol) in acetone (50 mL) was stirred with N2 at reflux for 4 hours. The reaction solvent was removed. The reaction mixture was then diluted with water and extracted with DCM. The combined organic phases were washed with brine, dried over Na2SO4 and the solvent removed in vacuo to give the desired compound (1.02 g, 1.68 mmol, 90%).

¹H NMR (300 MHz, CDCl₃) δ 4.81 (s, 1H), 3.76 (t, *J* = 6.9 Hz, 2H), 3.66 (d, *J* = 2.7 Hz, 22H), 3.61-3.55 (m, 2H), 3.47 (t, *J* = 6.1 Hz, 2H), 3.26 (t, *J* = 6.9 Hz, 2H), 3.13 (q, *J* = 6.4 Hz, 2H), 1.66-1.51 (m, 4H), 1.44 (s, 9H).

### Step 8: Preparation of tert-butyl (1-amino-3,6,9,12,15,18,21-heptaoxapentacosan-25-ylcarbamate

A solution of the compound prepared in step 7 (1.02 mg, 1.68 mmol) and 7 N NH3 in MeOH (15 mL) was stirred at 50° C. for 16 hours. The reaction solvent was removed. The reaction mixture was then purified by silica chromatography (0-15 % gradient of MeOH/DCM) to give the desired compound (520 mg, 1.04 mmol, 62 %).

¹H NMR (500 MHz, CDCl₃) δ 7.25 (s, 2H), 4.82 (s, 1H), 3.91-3.87 (m, 2H), 3.73 (dd, *J* = 5.3, 3.5 Hz, 2H), 3.72-3.62 (m, 20H), 3.59-3.55 (m, 2H), 3.46 (t, *J* = 6.5 Hz, 2H), 3.24-3.19 (m, 2H), 3.09 (t, *J* = 6.5 Hz, 2H), 1.60-1.48 (m, 4H), 1.39 (s, 9H).

### Step 9: Preparation of tert-butyl (1-((2-(2,6-dioxopiperidin-3-yl)-1,3-dioxoisoindolin-4-yl)amino)-3,6,9,12,15,18,21-heptaoxapentacosan-25-yl)carbamate

To a solution of the compound (200 mg, 0.4 mmol) prepared in step 8 above in DMSO, F-thalidomide (2-(2,6-dioxopiperidin-3-yl)-4-fluoroisoindolin-1,3-dione) (111 mg , 0.4 mmol) and DIPEA (0.2 mL, 1.2 mmol) were added. The reaction mixture was stirred at room temperature for 30 minutes and then at 90 °C for 12 hours. The resulting mixture was extracted with EtOAc. The organic layers were combined and washed with brine. The organic layer was then dried (Na2SO4) and filtered. The solvent was removed in vacuo and the product was purified by column chromatography using MeOH/DCM 6 % eluent to give the desired compound (152 mg, 0.207 mmol, 52 %) as a brown oil.

¹H NMR (300 MHz, CDCl₃) δ 8.32 (s, 1H), 7.57-7.48 (m, 1H), 7.16-7.09 (m, 1H), 6.99-6.90 (m, 1H), 6.56-6.47 (m, 1H), 4.99-4.88 (m, 1H), 4.81-4.67 (m, 1H), 3.67 (q, *J* = 6.2, 4.7 Hz, 27H), 3.56-3.40 (m, 5H), 3.22-3.05 (m, 2H), 2.98-2.68 (m, 4H), 2.22-2.10 (m, 1H), 1.64-1.52 (m, 2H), 1.46 (s, 9H).

### Step 10: Preparation of 4-((25-amino-3,6,9,12,15,18,21-heptaoxapentacosyl)amino)-2-(2,6-dioxopiperidin-3-yl)isoindolin-1,3-dione hydrochloride

To a solution of the compound prepared in step 9 above (156 mg, 0.21 mmol) in DCM (2 mL) was added 4 M HCl in dioxane. The reaction mixture was stirred at room temperature for 30 minutes. The reaction mixture was concentrated under reduced pressure to give the desired compound (210 mg, crude) as a yellow oil.

### Step 11: Preparation of N-(1-(2-(2,6-dioxopiperidin-3-yl)-1,3-dioxoisoindolin-4-ylamino)-3,6,9,12,15,18,21-heptaoxapentacosan-25-yl)-4-(5-(furan-2-ylmethylamino)-[1,2,4]triazolo[4,3-f]pyrimidin-8-yl)benzamide

In a solution of 4-(5-(furan-2-ylmethylamino)-[1,2,4]triazolo[4,3-f]pyrimidin-8-yl)benzoate (20 mg, 0.059 mmol) in DMF, The compound prepared in 10 (40 mg, 0.059 mmol), EDCI HCl (12 mg, 0.065 mmol), HOBt HO (10 mg, 0.065 mmol), and DIPEA (0.041 mL, 0.236 mmol) were added, and at room temperature for 2 hours. stirred. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 4 % to give the title compound (40 mg, 0.041 mmol, 69 %) as a yellow solid.

¹H NMR (300 MHz, CDCl₃) δ 8.53 (s, 1H), 8.34 (s, 1H), 8.24 (s, 1H), 8.09-8.00 (m, 2H), 7.96-7.88 (m, 2H), 7.47 (dd, *J* = 8.5, 7.2 Hz, 1H), 7.43-7.39 (m, 1H), 7.08 (d, *J* = 7.1 Hz, 1H), 6.90 (d, *J* = 8.6 Hz, 1H), 6.73 (t, *J* = 5.8 Hz, 1H), 6.62 (t, *J* = 5.8 Hz, 1H), 6.49 (t, *J* = 5.6 Hz, 1H), 6.42-6.34 (m, 2H), 4.95-4.85 (m, 3H), 3.70 (t, *J* = 5.4 Hz, 2H), 3.68-3.59 (m, 23H), 3.59-3.49 (m, 4H), 3.49-3.40 (m, 3H), 2.92-2.68 (m, 3H), 2.15-2.06 (m, 1H), 1.81-1.67 (m, 4H) ; LC/MS (ESI) *m*/*z* 970.1 [M+H]⁺, 968.0 [M-H]⁻

### <Example 46> Preparation of N-(28-(2-(2,6-dioxopiperidin-3-yl)-1,3-dioxoisoindolin-4-ylamino)-27-oxo-3,6,9,12,15,18,21-heptaoxa-26-azaoctacosyl)-4-(5-(furan-2-ylmethylamino)-[1,2,4]triazolo[4,3-f]pyrimidin-8-yl)benzamide

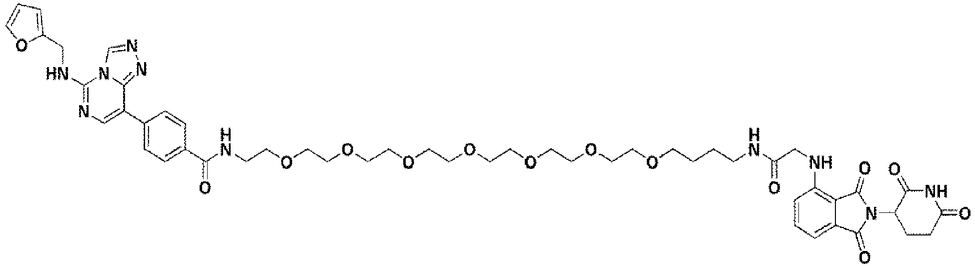

### Step 1: Preparation of tert-butyl (4-hydroxybutyl)carbamate

A solution of 4-aminobutane-1-ol (3.0 g, 33.65 mmol), BocO (14 mL, 47.12 mmol), TEA (5.3 mL, 50.48 mmol) in THF (25.0 mL) was stirred for 1 h at 25 °C.. The reaction mixture was diluted with water and extracted with EtOAc. The collected organic layer was dried over Na2SO4, filtered, concentrated, and purified via silica chromatography(0-70% gradient MeOH/DCM). tert-butyl(4-hydroxybutyl)carbamate (4.87 g, 25.73 mmol, 76) %) was prepared.

¹H NMR (300 MHz, CDCl₃) δ 4.64 (s, 1H), 3.72-3.63 (m, 2H), 3.16 (d, *J* = 6.3 Hz, 2H), 1.74 (s, 1H), 1.66-1.54 (m, 4H), 1.44 (s, 9H).

### Step 2: Preparation of 4-((tert-butoxycarbonyl)amino)butyl 4-methylbenzensulfonate

tert-butyl (4-hydroxybutyl)carbamate (4.9 g, 25.73 mmol), TsCl (9.8 g, 51.46 mmol), TEA (11 mL, 77.20 mmol), DMAP (314 mg, 2.57 mmol) dissolved in DCM (50.0 mL) ) solution was stirred at 25 °C for 16 h. The reaction mixture was diluted with water and extracted with DCM. The collected organic layer was dried over Na2SO4, filtered, concentrated and purified through silica chromatography(0 to 30% gradient EA/HEX). 4-((tert-butoxycarbonyl)amino)butyl 4-methylbenzensulfonate (6.71 g, 19.54 mmol, 76%) was prepared.

¹H NMR (300 MHz, CDCl₃) δ 7.80 (d, *J* = 8.5 Hz, 2H), 7.37 (d, *J* = 7.9 Hz, 2H), 4.53 (s, 1H), 4.05 (t, *J* = 6.3 Hz, 2H), 3.10 (q, *J* = 6.5 Hz, 2H), 2.47 (s, 3H), 1.73-1.61 (m, 2H), 1.58-1.47 (m, 2H), 1.44 (s, 9H).

### Step 3: Preparation of tert-butyl (1-hydroxy-3,6,9,12-tetraoxahexadecane-16-ylcarbamate

A solution of 4-((tert-butoxycarbonyl)amino)butyl 4-methylbenzensulfonate (11.2 g, 32.58 mmol), NaOH (3.9 g, 97.7 mmol) dissolved in tetraethyleneglycol (112 mL) was stirred at 60 °C for 2 h. The reaction mixture was diluted with brine and extracted with EtOAc. The collected organic layer was dried over Na2SO4, filtered, concentrated, and purified through silica chromatography(0-15% gradient MeOH/DCM). tert-butyl (1-hydroxy-3,6,9,12-tetraoxahexadecane-16-yl)carbamate (3.9 g, 10.7 mmol, 33%) was prepared.

### Step 4: Preparation of 2,2-dimethyl-4-oxo-3,10,13,16,19-pentaoxa-5-azahenicosan-21-yl

### 4-methylbenzensulfonate

tert-butyl (1-hydroxy-3,6,9,12-tetraoxahexadecane-16-yl)carbamate (3.9 g, 10.67 mmol), TsCl (4.06 g, 21.34 mmol) dissolved in DCM (20.0 mL), A solution of TEA (4.46 mL, 32.01 mmol), and DMAP (130 mg, 1.06 mmol) was stirred at 25 °C for 16 h. The reaction mixture was diluted with brine and extracted with EtOAc. The collected organic layer was dried over Na2SO4, filtered, concentrated, and purified through silica chromatography (0-15% gradient MeOH/DCM). 2,2-dimethyl-4-oxo-3,10,13,16,19-pentaoxa-5-azahenicosan-21-yl 4-methylbenzensulfonate (5.12 g, 9.81 mmol, 91%) was prepared.

¹H NMR (300 MHz, CDCl₃) δ 7.80 (d, *J* = 8.4 Hz, 2H), 7.42-7.30 (m, 2H), 4.82-4.67 (m, 1H), 4.21-4.11 (m, 2H), 3.74-3.52 (m, 15H), 3.47 (t, *J* = 6.2 Hz, 2H), 3.12 (q, *J* = 6.6 Hz, 2H), 2.45 (s, 3H), 1.69-1.49 (m, 4H), 1.43 (s, 10H).

### Step 5: Preparation of tert-butyl (1-hydroxy-3,6,12,15,18,21-heptaoxapentacosan-25-ylcarbamate

2,2-dimethyl-4-oxo-3,10,13,16,19-pentaoxa-5-azahenicosan-21-yl 4-methylbenzensulfonate (1.7 g, 3.27 mmol) in triethyleneglycol (17 mL), NaOH ( 392 mg, 9.81 mmol) solution was stirred at 60 °C for 2 h. The reaction mixture was diluted with brine and extracted with EtOAc. The collected organic layer was dried over Na2SO4, filtered, concentrated, and purified through silica chromatography(0-15% gradient MeOH/DCM). tert-butyl (1-hydroxy-3,6,9,12-tetraoxa)hexadecane-16-yl)carbamate (1.05 g, 2.11 mmol, 64%) was prepared.

¹H NMR (300 MHz, CDCl₃) δ 4.72 (s, 1H), 3.76-3.54 (m, 28H), 3.47 (t, *J* = 6.2 Hz, 2H), 3.13 (q, *J* = 6.6 Hz, 2H), 2.63 (t, *J* = 6.0 Hz, 1H), 1.66-1.50 (m, 4H), 1.44 (s, 9H).

### Step 6: Preparation of 2,2-dimethyl-4-oxo-3,10,13,16,19,22,25,28-octaoxa-5-azatriacontane-30-yl 4-methylbenzensulfonate

tert-butyl (1-hydroxy-3,6,9,12,15,18,21-heptaoxapentacosan-25-yl)carbamate (1.05 g, 2.11 mmol), TsCl (805 mg, 4.22 mmol), TEA (0.9 mL, 6.33 mmol), and DMAP (26 mg, 0.21 mmol) were stirred for 16 h at 25 °C. The reaction mixture was diluted with brine and extracted with EtOAc. The collected organic layer was dried over Na2SO4, filtered, concentrated, and purified through silica chromatography (0-15% gradient MeOH/DCM). 2,2-dimethyl-4-oxo-3,10,13,16 ,19,22,25,28-octaoxa-5-azatriacontane-30-yl 4-methylbenzensulfonate (1.21 g, 1.85 mmol, 88%) was prepared.

¹H NMR (300 MHz, CDCl₃) δ 7.80 (d, *J* = 8.3 Hz, 2H), 7.35 (d, *J* = 8.3 Hz, 2H), 4.74 (s, 1H), 4.23-4.14 (m, 2H), 3.72-3.61 (m, 20H), 3.59 (d, *J* = 2.3 Hz, 6H), 3.47 (t, *J* = 6.1 Hz, 2H), 3.13 (d, *J* = 6.3 Hz, 2H), 2.45 (s, 3H), 1.64-1.51 (m, 4H), 1.44 (s, 9H).

### Step 7: Preparation of tert-butyl (1-iodo-3,6,9,12,15,18,21-heptaoxapentacosan-25-ylcarbamate

A solution of the compound prepared in the previous step (1.21 g, 1.85 mmol) and NaI (16.6 g, 11.1 mmol) dissolved in acetone (50 mL) was stirred with N2 under reflux for 4 h. The reaction solvent was removed. Then, the reaction mixture was diluted with water and extracted with DCM. The collected organic layer was washed with brine, dried over Na2SO4, and the solvent was removed under vacuum. tert-butyl (1-iodo-3,6,9,12,15,18,21-heptaoxapentacosan-25-yl)carbamate ( 1.02 g, 1.68 mmol, 90%) was prepared.

¹H NMR (300 MHz, CDCl₃) δ 4.81 (s, 1H), 3.76 (t, *J* = 6.9 Hz, 2H), 3.66 (d, *J* = 2.7 Hz, 22H), 3.61-3.55 (m, 2H), 3.47 (t, *J* = 6.1 Hz, 2H), 3.26 (t, *J* = 6.9 Hz, 2H), 3.13 (q, *J* = 6.4 Hz, 2H), 1.66-1.51 (m, 4H), 1.44 (s, 9H).

### Step 8: Preparation of tert-butyl (1-amino-3,6,9,12,15,18,21-heptaoxapentacosan-25-ylcarbamate

tert-butyl (1-iodo-3,6,9,12,15,18,21-heptaoxapentacoic acid-25-yl)carbamate (1.02 mg, 1.68 mmol) and 7 N NH3 dissolved in MeOH (15 mL) The solution was stirred at 50 °C for 16 h. The reaction solvent was removed. The reaction mixture was purified via silica chromatography (0-15% gradient MeOH/DCM) to tert-butyl (1-amino-3,6,9,12,15,18,21-heptaoxapentacoic acid-25-yl) Carbamate (520 mg, 1.04 mmol, 62%) was prepared.

¹H NMR (500 MHz, CDCl₃) δ 7.25 (s, 2H), 4.82 (s, 1H), 3.91-3.87 (m, 2H), 3.73 (dd, *J* = 5.3, 3.5 Hz, 2H), 3.72-3.62 (m, 20H), 3.59-3.55 (m, 2H), 3.46 (t, *J* = 6.5 Hz, 2H), 3.24-3.19 (m, 2H), 3.09 (t, *J* = 6.5 Hz, 2H), 1.60-1.48 (m, 4H), 1.39 (s, 9H).

### Step 9: Preparation of tert-butyl (1-(4-(5-((furan-2-ylmethyl)amino)-[1,2,4]triazolo[4,3-c]pyrimidin-8-yl)phenyl)-1-oxo-5,8,11,14,17,20,23-heptaoxa-2-azaheptacosan-27-yl)carbamate

A solution of 4-(5-((furan-2-yl)methylamino)-[1,2,4]triazolo[4,3-flpyrimidin-8-yl)benzoate (100 mg, 0.298 mmol) in DMF To, the compound prepared in the previous step (147 mg, 0.298 mmol), EDCI HCl (62 mg, 0.327 mmol), HOBt HO (50 mg, 0.327 mmol), DIPEA (0.207 mL, 1.19 mmol) was added, and at room temperature Stirred for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 5% to prepare the title compound (109 mg, 0.134 mmol, 45%) as a yellow solid.

¹H NMR (500 MHz, CDC13) δ 8.34 (s, 1H), 8.25 (s, 1H), 8.09-8.02 (m, 2H), 7.99-7.91 (m, 2H), 7.44-7.39 (m, 1H), 7.03 (s, 1H), 6.64-6.55 (m, 1H), 6.42-6.34 (m, 2H), 4.90 (d, *J* = 5.8 Hz, 2H), 4.73 (s, 1H), 3.73-3.60 (m, 26H), 3.59-3.54 (m, 2H), 3.45 (t, *J* = 6.2 Hz, 2H), 3.16-3.09 (m, 2H), 1.63-1.50 (m, 4H), 1.43 (s, 9H).

### Step 10: Preparation of N-(25-amino-3,6,9,12,15,18,21-heptaoxapentacosyl)-4-(5-((furan-2-ylmethvl)amino)-[1,2,4]triazolo[4,3-c]pyrimidin-8-yl)benzamide

To a solution of the compound prepared in the previous step (109 mg, 0.134 mmol) dissolved in DCM (4 mL), 4 N HCl dissolved in 1,4-dioxane (2 mL) was added, and the mixture was stirred at room temperature for 1 h. did. The reaction mixture was concentrated in vacuo. The solid was filtered with DCM under reduced pressure to prepare the target compound (110 mg, quant.) in the form of a yellow oil.

¹H NMR (300 MHz, CD₃OD) δ 8.65 (s, 1H), 8.36 (s, 1H), 8.10-7.96 (m, 4H), 7.51-7.45 (m, 1H), 6.47-6.36 (m, 2H), 3.77-3.61 (m, 28H), 3.60-3.54 (m, 2H), 3.03 (t, *J* = 5.8 Hz, 2H), 1.88-1.71 (m, 4H).

### Step 11: Preparation of N-(28-(2-(2,6-dioxopiperidin-3-yl)-1,3-dioxoisoindolin-4-ylamino)-27-oxo-3,6,9,12,15,18,21-heptaoxa-26-azaoctacosyl)-4-(5-(furan-2-ylmethylamino)-[1,2,4]triazolo[4,3-f]pyrimidin-8-yl)benzamide

In a solution of 2-(2-(2,6-dioxopiperidin-3-yl)-1,3-dioxoisoindolin-4-ylamino)acetic acid (23 mg, 0.070 mmol) dissolved in DMF, the compound prepared in the previous step ( 52 mg, 0.070 mmol), EDCI HCl (15 mg, 0.077 mmol), HOBt HO (12 mg, 0.077 mmol), DIPEA (0.048 mL, 0.280 mmol) were added and stirred at room temperature for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 5% to prepare the title compound (14 mg, 0.013 mmol, 19%) as a yellow solid.

¹H NMR (500 MHz, CDCl₃) δ 8.75 (s, 1H), 8.34 (s, 1H), 8.24 (s, 1H), 8.06-7.99 (m, 2H), 7.98-7.90 (m, 2H), 7.51 (dd, *J* = 8.4, 7.2 Hz, 1H), 7.44-7.39 (m, 1H), 7.21-7.11 (m, 2H), 6.94 (t, *J* = 5.9 Hz, 1H), 6.83-6.75 (m, 2H), 6.70 (t, *J* = 5.8 Hz, 1H), 6.44-6.34 (m, 2H), 4.98-4.85 (m, 3H), 3.95 (d, *J* = 5.9 Hz, 2H), 3.73-3.54 (m, 26H), 3.50-3.45 (m, 2H), 3.39 (t, *J* = 5.7 Hz, 2H), 3.28 (q, *J* = 6.3 Hz, 2H), 2.91-2.69 (m, 3H), 2.16-2.10 (m, 1H), 1.60-1.48 (m, 4H).

### <Example 47> Preparation of N-(28-(2-(2,6-dioxopiperidin-3-yl)-1,3-dioxoisoindolin-4-yloxy)-27-oxo-3,6,9,12,15,18,21-heptaoxa-26-azaoctacosyl)-4-(5-(furan-2-ylmethylamino)-[1,2,4]triazolo[4,3-f]pyrimidin-8-yl)benzamide

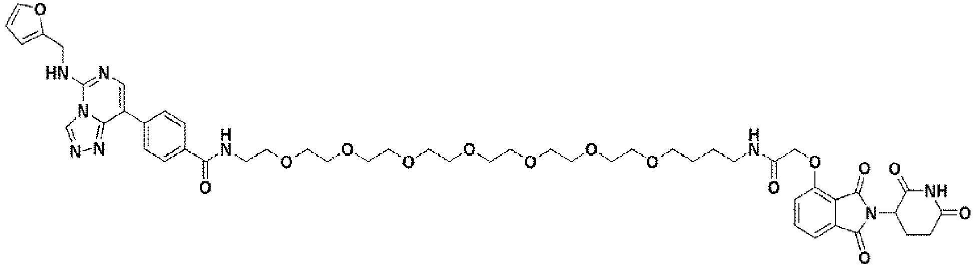

To 2-(2-(2,6-dioxopiperidin-3-yl)-1,3-dioxoisoindolin-4-yloxy)acetic acid (23 mg, 0.070 mmol) in DMF, EDCI HCl (15 mg, 0.077 mmol), HOBt (12 mg, 0.077 mmol), DIPEA (0.048mL, 0.280 mmol), and N-(25-amino-3,6,9,12,15,18,21-heptaoxapentacosyl)-4-(5-((furan-2-ylmethyl)amino)-[1,2,4]triazolo[4,3-c]pyrimidin-8-yl)benzamide.(30mg, 0.055 mmol, Prepared in example 46 step 10) And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by silica gel column chromatography (MeOH/DCM = 5% ), target chemical, N-(28-(2-(2,6-dioxopiperidin-3-yl)-1,3-dioxoisoindolin-4-yloxy)-27-oxo-3,6,9,12,15,18,21-heptaoxa-26-azaoctacosyl)-4-(5-(furan-2-ylmethylamino)-[1,2,4]triazolo[4,3-f]pyrimidin-8-yl)benzamide was obtained (14 mg, 0.013 mmol, 19% yield) as white solid.

¹H NMR (500 MHz, CDCl₃) δ 9.04 (s, 1H), 8.34 (s, 1H), 8.24 (s, 1H), 8.07-8.01 (m, 2H), 7.99-7.91 (m, 2H), 7.73 (t, *J* = 7.5 Hz, 1H), 7.58-7.50 (m, 2H), 7.45-7.38 (m, 1H), 7.18 (d, *J* = 8.3 Hz, 1H), 7.16-7.07 (m, 1H), 6.68 (t, *J* = 5.8 Hz, 1H), 6.42-6.33 (m, 2H), 5.00-4.93 (m, 1H), 4.90 (d, *J* = 5.8 Hz, 2H), 4.67-4.59 (m, 2H), 3.73-3.53 (m, 28H), 3.51-3.45 (m, 2H), 3.45-3.39 (m, 1H), 3.39-3.30 (m, 1H), 2.93-2.70 (m, 3H), 2.19-2.12 (m, 1H), 1.71-1.59 (m, 4H).

### <Example 48> Preparation of N-(1-(2-(2,6-dioxopiperidin-3-yl)-1,3-dioxoisoindolin-4-ylamino)-3,6,9,12,15,18,21,24-octaoxaoctacosan-28-yl)-4-(5-(furan-2-ylmethylamino)-[1,2,4]triazolo[4,3-f]pyrimidin-8-yl)benzamide

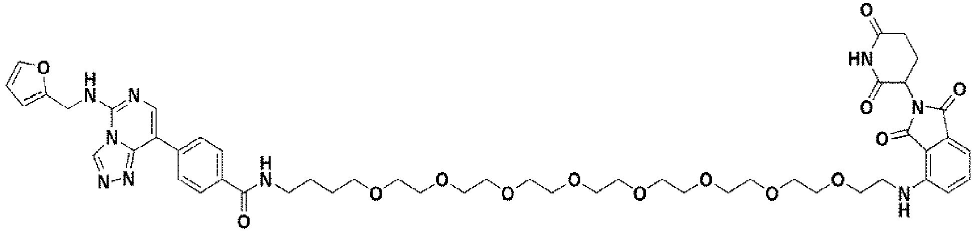

### Step 1: Preparation of 4-((tert-butoxycarbonyl)amino)butyl 4-methylbenzensulfonate

A solution of DMAP (314 mg, 2.57 mmol), TsCl (9.8 g, 51.46 mmol), TEA (11 ml, 77.20 mmol), tert-butyl (4-hydroxybutyl)carbamate (4.9 g, 25.73 mmol) in DCM (50.0 mL) was stirred at 25 °C for 16 hours. The reaction mixture was diluted with water and extracted with DCM. The combined organic phases were dried over Na2SO4, filtered, concentrated and purified by silica chromatography (0-30% gradient of EA/HEX) to give the title compound (6.71 g, 19.54 mmol, 76%).

¹H NMR (300 MHz, CDCl₃) δ 7.80 (d, *J* = 8.5 Hz, 2H), 7.37 (d, *J* = 7.9 Hz, 2H), 4.53 (s, 1H), 4.05 (t, *J* = 6.3 Hz, 2H), 3.10 (q, *J* = 6.5 Hz, 2H), 2.47 (s, 3H), 1.73-1.61 (m, 2H), 1.58-1.47 (m, 2H), 1.44 (s, 9H).

### Step 2: Preparation of tert-butyl (1-hydroxy-3,6,9,12-tetraoxahexadecane-16-ylcarbamate

A solution of the compound prepared in step 2 (11.2 g, 32.58 mmol) and NaOH (3.9 g, 97.7 mmol) in tetraethyleneglycol (112 mL) was stirred at 60 °C for 2 hours. The reaction mixture was diluted with brine and extracted with EtOAc. The combined organic phases were dried over Na2SO4, filtered, concentrated and purified by silica chromatography (0-15 % gradient of MeOH/DCM) to give the title compound (3.9 g, 10.7 mmol, 33 %).

### Step 3: Preparation of 2,2-dimethyl-4-oxo-3,10,13,16,19-pentaoxa-5-azahenicosan-21-yl

### 4-methylbenzensulfonate

A solution of the compound prepared in step 3 above (3.9 g, 10.67 mmol), TsCl (4.06 g, 21.34 mmol), TEA (4.46 mL, 32.01 mmol) and DMAP (130 mg, 1.06 mmol) in DCM (20.0 ml) The mixture was stirred at 25 °C for 16 hours. The reaction mixture was diluted with brine and extracted with EtOAc. The combined organic phases were dried over Na2SO4, filtered, concentrated and purified by silica chromatography (0-15 % MeOH/DCM gradient) to give the title compound (5.12 g, 9.81 mmol, 91 %).

¹H NMR (300 MHz, CDCl₃) δ 7.80 (d, *J* = 8.4 Hz, 2H), 7.42-7.30 (m, 2H), 4.82-4.67 (m, 1H), 4.21-4.11 (m, 2H), 3.74-3.52 (m, 15H), 3.47 (t, *J* = 6.2 Hz, 2H), 3.12 (q, *J* = 6.6 Hz, 2H), 2.45 (s, 3H), 1.69-1.49 (m, 4H), 1.43 (s, 10H).

### Step 4: Preparation of tert-butyl (1-hydroxy-3,6,9,12,15,18,21,24-octaoxaoctacosane-28-yl)carbamate

A solution of the compound prepared in step 4 (1.7 g, 3.27 mmol) and NaOH (392 mg, 9.81 mmol) in tetraethyleneglycol (12 mL) was stirred at 60 °C for 2 hours. The reaction mixture was diluted with brine and extracted with EtOAc. The combined organic phases were dried over Na2SO4, filtered, concentrated and purified by silica chromatography (0-15 % gradient of MeOH/DCM) to give the title compound (1.08 g, 1.99 mmol, 61 %).

¹H NMR (300 MHz, CDCl₃) δ 4.80 (s, 1H), 3.79-3.53 (m, 32H), 3.47 (t, *J* = 6.2 Hz, 2H), 3.13 (d, *J* = 6.4 Hz, 2H), 2.90 (s, 1H), 1.67-1.52 (m, 4H), 1.44 (s, 9H).

### Step 5: Preparation of 2,2-dimethyl-4-oxo-3,10,13,16,19,22,25,28,31-nonaoxa-5-azatritriacontane-33-yl 4-methylbenzensulfonate

A solution of the compound prepared in step 5 above (1.08 g, 1.99 mmol), TsCl (758 mg, 3.98 mmol), TEA (0.83 mL, 5.97 mmol) and DMAP (23 mg, 0.19 mmol) in DCM (20.0 mL) was 25 The mixture was stirred at °C for 16 hours. The reaction mixture was diluted with brine and extracted with EtOAc. The combined organic phases were dried over Na2SO4, filtered, concentrated and purified by silica chromatography (0-15 % MeOH/DCM gradient) to give the title compound (1.24 g, 1.78 mmol, 89 %).

### Step 6: Preparation of tert-butyl (1-iodo-3,6,9,12,15,18,21,24-octaoxaoctacosane-28-yl)carbamate

A solution of the compound prepared in step 6 above (1.24 g, 1.78 mmol) and sodium iodide (16.0 g, 10.7 mmol) in acetone (50 mL) was stirred with N2 for 4 h. The reaction solvent was removed. The reaction mixture was then diluted with water and extracted with DCM. The combined organic phases were combined, washed with brine, dried over Na2SO4 and the solvent removed in vacuo to give the desired compound (1.0 g, 1.53 mmol, 86%).

¹H NMR (300 MHz, CDCl₃) δ 4.81 (s, 1H), 3.76 (t, *J* = 6.9 Hz, 2H), 3.71-3.53 (m, 28H), 3.47 (t, *J* = 6.2 Hz, 2H), 3.26 (t, *J* = 6.9 Hz, 2H), 3.13 (d, *J* = 6.3 Hz, 2H), 1.67-1.50 (m, 4H), 1.44 (s, 9H).

### Step 7: Preparation of tert-butyl (1-amino-3,6,9,12,15,18,21,24-octaoxaoctacosane-28-yl)carbamate

A solution of the compound prepared in step 7 (1.0 g, 1.53 mmol) and 7 N NH3 in MeOH (15 mL) was stirred at 50° C. for 16 hours. The reaction solvent was removed. Then, the reaction mixture was purified by silica chromatography (0-15 % gradient of MeOH/DCM) to give the title compound (445 mg, 0.823 mmol, 53 %).

¹H NMR (300 MHz, CDCl₃) δ 7.32 (s, 1H), 4.89 (s, 1H), 3.94-3.88 (m, 2H), 3.84-3.63 (m, 26H), 3.63-3.57 (m, 2H), 3.49 (t, *J* = 6.2 Hz, 2H), 3.31-3.21 (m, 2H), 3.13 (d, *J* = 6.3 Hz, 2H), 1.69-1.49 (m, 4H), 1.43 (s, 9H).

### Step 8: Preparation of tert-butyl (1-((2-(2,6-dioxopiperidin-3-yl)-1,3-dioxoisoindolin-4-yl)amino)-3,6,9,12,15,18,21,24-octaoxaoctacosane-28-yl)carbamate

F-thalidomide (2-(2,6-dioxopiperidin-3-yl)-4-fluoroisoindolin-1,3-dione) in a solution of the compound prepared in step 8 (500 mg, 0.55 mmol) in DMSO (4 mL) ) (185 mg, 0.67 mmol) and DIPEA (0.19 mL, 1.1 mmol) were added. The mixture was stirred at 90 °C overnight. The reaction was quenched and then extracted with EtOAc (2×25 mL). The combined organic layers were dried over Na2SO4 and the solvent was removed in vacuo. The crude mixture was purified by silica gel column chromatography using EtOAc/Hex (1/1) as eluent to give the title compound (120 mg, 0.15 mmol, 27 %) as a yellow solid.

¹H NMR (300 MHz, CDCl₃) δ 8.14 (br s, 1H), 7.53-7.44 (m, 1H) 7.10 (d, *J* = 7.2 Hz, 1H), 6.92 (d, *J* = 8.7 Hz, 1H), 6.49 (t, *J* = 5.7 Hz, 1H), 4.97-4.84 (m, 1H), 4.72 (br s, 1H), 3.69-3.50 (m, 6H), 3.72 (t, *J* = 5.4 Hz, 1H), 3.69-3.60 (m, 26H), 3.60-3.54 (m, 3H), 3.52-3.43 (m, 4H), 3.18-3.07 (m, 2H), 2.92-2.69 (m, 3H), 2.19-2.03 (m, 1H), 1.84 (s, 2H), 1.66-1.50 (m, 4H), 1.43 (s, 9H).

### Step 9: Preparation of 4-((28-amino-3,6,9,12,15,18,21,24-octaoxaoctacosyl)amino)-2-(2,6-dioxopiperidin-3-yl)isoindolin-1,3-dione hydrochloride

To a solution of the compound prepared in step 9 above (120 mg, 0.14 mmol) in DCM (4 mL) was added 4 M HCl in dioxane (2.0 mL) and stirred at room temperature for 1 hour. The solvent was removed in vacuo to give the desired compound (95 mg, 0.13 mmol, 93%) as a yellow oil.

δ ¹H NMR (300 MHz, CD₃OD) δ 7.61-7.53 (m, 1H), 7.14-7.04 (m, 2H), 5.10-5.01 (m, 1H), 3.83-3.71 (m, 4H), 3.70-3.61 (m, 30H), 3.59-3.46 (m, 7H), 3.36-3.32 (m, 1H), 3.06-3.97 (m, 2H), 2.91-2.63 (m, 3H), 2.17-2.06 (m, 1H) 2.86-1.69 (m, 4H).

### Step 10: Preparation of N-(1-(2-(2,6-dioxopiperidin-3-yl)-1,3-dioxoisoindolin-4-ylamino)-3,6,9,12,15,18,21,24-octaoxaoctacosan-28-yl)-4-(5-(furan-2-ylmethylamino)-[1,2,4]triazolo[4,3-f]pyrimidin-8-yl)benzamide

Step 10 above in a solution of 4-(5-(furan-2-ylmethylamino)-[1,2,4]triazolo[4,3-f]pyrimidin-8-yl)benzoate (20 mg, 0.059 mmol) in DMF (43 mg, 0.059 mmol), EDCI HCl (12 mg, 0.065 mmol), HOBt HO (10 mg, 0.065 mmol), and DIPEA (0.041 mL, 0.236 mmol) were added and stirred at room temperature for 2 hours. did. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 7 % to give the title compound (27 mg, 0.026 mmol, 45 %) as a yellow solid.

¹H NMR (300 MHz, CDCl₃) δ 8.56 (s, 1H), 8.34 (s, 1H), 8.24 (s, 1H), 8.08-7.99 (m, 2H), 7.96-7.87 (m, 2H), 7.47 (dd, *J* = 8.5, 7.2 Hz, 1H), 7.43-7.38 (m, 1H), 7.09 (d, *J* = 7.1 Hz, 1H), 6.90 (d, *J* = 8.5 Hz, 1H), 6.73 (t, *J* = 5.6 Hz, 1H), 6.64 (t, *J* = 5.8 Hz, 1H), 6.49 (t, *J* = 5.6 Hz, 1H), 6.41-6.34 (m, 2H), 4.96-4.85 (m, 3H), 3.71 (t, *J* = 5.3 Hz, 2H), 3.68-3.57 (m, 28H), 3.57-3.49 (m, 4H), 3.49-3.42 (m, 2H), 2.92-2.69 (m, 3H), 2.15-2.07 (m, 1H), 1.77-1.63 (m, 4H) ; LC/MS (ESI) *m*/*z* 1014.1 [M+H]⁺, 1012.0 [M-H]⁻

### <Example 49> Preparation of N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-4-yloxy)acetamide

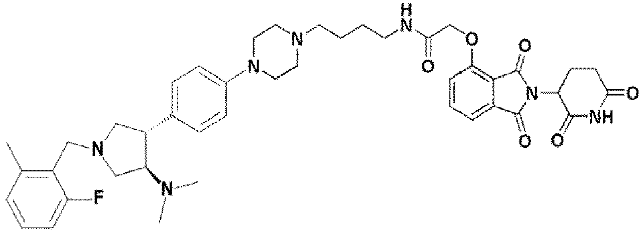

### Step 1: Preparation of tert-butyl (4-hydroxybutyl)carbamate

To a solution of 4-amino butane-1-ol (1 g, 11.2 mmol) in THF (25 mL) was added TEA (2.3 mL, 16.85 mmol) and the reaction was cooled to 5-10 °C. BOC anhydride (3.9 mL, 16.8 mmol) is added and the temperature is raised to room temperature. The reaction is stirred for 1 hour. After completion of the reaction by TLC, the reaction was quenched with water and extracted with EtOAc, the organic layer was dried over sodium sulfate, concentrated in vacuo, and purified by column chromatography to give the title compound (1.6 g, yield 76%) as a colorless oil.

¹H NMR : (300 MHz, DMSO-d6) δ 6.77 (t, *J* = 5.8 Hz, 1H), 4.37 (t, *J* = 5.1 Hz, 1H), 3.40-3.33 (m, 2H), 2.89 (m 2H), 1.37 (m, 13H).

### Step 2: Preparation of 4-((tert-butoxycarbonyl)amino)butyl 4-methylbenzensulfonate

To a solution of the compound prepared in the previous step (1.6 g, 8.45 mmol) in DCM (30 mL) was added TEA (3.5 mL, 25.35 mmol) followed by DMAP (516 mg, 4.22 mmol) and the reaction mass was stirred at 5 °C-10 ° C. TsCl (3.2 g, 16.91 mmol) was charged and the temperature was raised to room temperature. The reaction mass was stirred overnight. After completion of the reaction, the reaction was quenched with water and extracted with EtOAc, the organic layer was dried over sodium sulfate, concentrated in vacuo, and purified by column chromatography to give the title compound (2.0 g, 70 % yield) as a colorless oil.

¹H NMR : (300 MHz, CDCl₃) δ 7.80 (d, *J* = 8.1 Hz, 2H), 7.37 (d, *J* = 8.0 Hz, 2H), 4.53 (s, 1H), 4.05 (t, *J* = 6.2 Hz, 2H), 3.10 (q, *J* = 6.5 Hz, 2H), 2.47 (s, 3H), 1.71 (m,1H), 1.66 (m, 1H), 1.60-1.49 (m, 2H), 1.44 (s, 9H).

### Step 3: Preparation of tert-butyl (4-iodobutyl) carbamate

To a solution of the compound prepared in the previous step (2 g, 5.89 mmol) in acetone (50 ml) was added sodium iodide (3.5 g, 23.29 mmol). The reaction mass was stirred at 60 °C for 2 h. After completion of the reaction, the reaction was distilled, the residue was quenched with water and extracted with EtOAc, the organic layer was dried over sodium sulfate and concentrated in vacuo to give the title compound (1.6 g, 90 % yield) as a red-yellow oil.

¹H NMR : (300 MHz, CDCl₃) δ 4.57 (s, 1H), 3.18 (m, 4H), 1.87 (p, *J* = 7.0 Hz, 2H), 1.61 (p, *J* = 7.1 Hz, 2H), 1.46 (s, 9H).

### Step 4: Preparation of tert-butyl (4-(4-(4-((3S,4R)-4-(dimethylamino)-1-(2-fluoro-6-methylbenzyl)pyrrolidin-3-yl)phenyl)piperazin-1-yl)butyl)carbamate

(3R,4S)-1-(2-fluoro-6-methylbenzyl)-N,N-dimethyl-4-(4-(piperazin-1-yl)phenyl)pyrrolidin-3-amine ( 90 mg, 0.23 mmol) was added to a solution of K2CO3 (100 mg, 0.69 mmol) and the compound prepared in the previous step (90 mg, 0.300 mmol), followed by stirring at 80° C. for 2 hours. After completion of the reaction by TLC, the reaction was quenched with water and extracted with EtOAc, the organic layer was dried over sodium sulfate, concentrated in vacuo and purified by column chromatography to give the title compound as a yellow oil (80 mg, 68 % yield).

¹H NMR : (500 MHz, CDCl₃) δ 7.21 (d, *J* = 8.7 Hz, 2H), 7.13 (m, 1H), 6.97 (d, *J* = 7.5 Hz, 1H), 6.92-6.76 (m, 3H), 5.23 (s, 1H), 3.64 (d, *J* = 2.2 Hz, 2H), 3.20 (t, *J* = 5.0 Hz, 4H), 3.19-3.11 (m, 3H), 2.97 (q, *J* = 7.9, 7.3 Hz, 3H), 2.68 (m, 1H), 2.61 (t, *J* = 5.0 Hz, 4H), 2.56 (dd, *J* = 9.1, 5.5 Hz, 1H), 2.47 (s, 3H), 2.42 (t, *J* = 6.8 Hz, 2H), 2.23 (s, 6H), 1.58 (m, 4H), 1.45 (s, 9H).

### Step 5: Preparation of (3R,4S)-4-(4-(4-(4-aminobutyl)piperizin-1-yl)phenyl)-1-(2-fluoro-6-methylbenzyl)-N,N-dimethylpirrolidin-3-amine hydrochloride

To a suspension of the compound prepared in the previous step (50 mg, 0.0880 mmol) in DCM (1 mL) was added 4.0 N HCl (0.6 mL) and the reaction was stirred at room temperature for 2 h. After completion of the reaction on TLC, the solvent was dried to give the desired compound as a white solid (40 mg, 87 % yield).

¹H NMR : (500 MHz, MeOH-d4) δ 7.56 (d, *J* = 8.2 Hz, 2H), 7.45 (td, *J* = 8.0, 6.0 Hz, 1H), 7.20 (dd, *J* = 14.7, 8.1 Hz, 3H), 7.12 (t, *J* = 9.1 Hz, 1H), 4.75 (q, *J* = 13.9, 13.5 Hz, 2H), 4.63 (d, *J* = 6.8 Hz, 1H), 4.44-4.24 (m, 2H), 4.03 (t, *J* = 9.5 Hz, 1H), 3.94 (d, *J* = 11.3 Hz, 2H), 3.78-3.72 (m, 3H), 3.69 (d, *J* = 8.1 Hz, 3H), 3.67-3.59 (m, 2H), 3.31 (dd, *J* = 10.2, 6.6 Hz, 6H), 3.05 (t, *J* = 7.6 Hz, 2H), 2.63 (s, 3H), 2.02-1.93 (m, 2H), 1.81 (p, *J* = 7.8 Hz, 2H).

### Step 6: Preparation of N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-4-yloxy)acetamide

In a solution of the obtained compound (10 mg, 0.029 mmol) prepared in the previous step, DMF (2 mL), EDCI (6.0 mg, 0.031 mmol), HOBt (4.0 mg, 0.03319 mmol), DIPEA (27 µL, 0.116 mmol) and 2-(1,3-dioxo-2-(2,6-dioxopiperidin-3-yl)isoindolin-4yloxy)acetic acid (10 mg, 0.029 mmol) were stirred at room temperature for 12 hours. After completion of the reaction in TLC, the reaction was quenched with water, extracted with EtOAc and the combined organic layers were washed with water and brine. The organic layer was dried over sodium sulfate, concentrated in vacuo and purified by column chromatography to give the desired compound as a slightly yellow oil (6.0 mg, 25 % yield).

¹H NMR : (500 MHz, CDCl₃) δ 7.81-7.74 (m, 2H), 7.59 (dd, *J* = 7.3, 2.0 Hz, 1H), 7.21 (m,3H), 7.14 (m, 1H), 6.97 (d, *J* = 7.5 Hz, 1H), 6.88 (m, 3H), 4.93-4.83 (m, 1H), 4.74 (d, *J* = 14.1 Hz, 1H), 4.61 (d, *J* = 14.1 Hz, 1H), 3.70-3.55 (m, 3H), 3.27 (m,7H), 3.05-2.93 (m, 2H), 2.86-2.78 (m, 1H), 2.76-2.61 (m, 6H), 2.58-2.48 (m, 3H), 2.46 (s, 3H), 2.33 (s, 4H), 2.21 (m, 1H), 2.06

### <Example 50> Preparation of N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-4-ylamino)acetamide

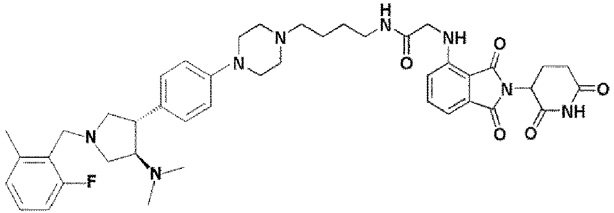

To (3R,4S)-4-(4-(4-(4-aminobutyl)piperazin-1-yl)phenyl)-1-(2-fluoro-6-methylbenzyl)-N,N-dimethyl)pyrrolidin-3-amine hydrochloride_(15 mg, 0.029 mmol) in DMF (2 mL), EDCI (6.2 mg, 0.032 mmol), HOBt (4.3 mg, 0.0319 mmol), DIPEA (27 µL, 0.116 mmol), and 2-(1,3-dioxo-2-(2,6-dioxopiperidin-3-yl)isoindolin-4-yl)amino acetate (10 mg, 0.029 mmol) were added. And it was stirred at a room temperature for 12 hours. After completion of the reaction on TLC, the reaction mixture was quenched with water. Some solid material appeared and the filter was washed with excess water. The filtered solid material was dissolved in chloroform and extracted to remove excess water. The organic layer was dried on anhydrous Na2SO4 and then was concentrated under the vaccum pressure to obtain target chemical, N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-4-ylamino)acetamide (8.0mg, 34 % yield) as yellow oil.

¹H NMR : (500 MHz, CDCl₃) δ 7.56 (m,1H), 7.29 (s, 1H), 7.24 (d, *J* = 7.2 Hz, 1H), 7.21 (d, *J* = 8.4 Hz, 2H), 7.12 (m, 1H), 6.97 (d, *J* = 7.5 Hz, 1H), 6.89-6.81 (m, 4H), 6.74 (q, *J* = 5.5 Hz, 1H), 6.69 (m, 1H), 4.96-4.91 (m, 1H), 3.97 (d, *J* = 5.9 Hz, 2H), 3.63 (d, *J* = 2.3 Hz, 2H), 3.32 (q, *J* = 6.4 Hz, 2H), 3.19-3.10 (m, 5H), 2.98-2.90 (m, 4H), 2.90-2.69 (m, 3H), 2.66-2.61 (m, 1H), 2.59-2.53 (m, 5H), 2.47 (s, 3H), 2.38 (t, *J* = 6.8 Hz, 2H), 2.19 (s, 6H), 2.16-2.12 (m, 1H), 1.53 (m, 4H).

### <Example 51> Preparation of N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-4-yl)azetidin-3-carboxamide

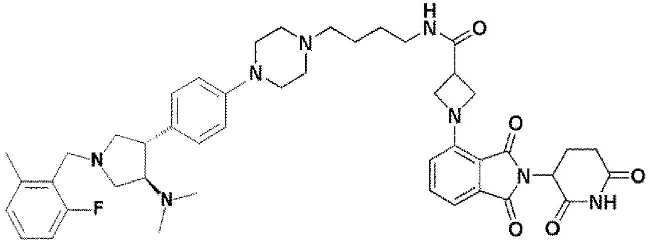

To (3R,4S)-4-(4-(4-(4-aminobutyl)piperazin-1-yl)phenyl)-1-(2-fluoro-6-methylbenzyl)-N,N-dimethyl)pyrrolidin-3-amine hydrochloride (15 mg, 0.029 mmol) in DMF (2 mL), EDCI (6.2 mg, 0.032 mmol), HOBt (4.3 mg, 0.0319 mmol), DIPEA (27 µL, 0.116 mmol), and 1-(2-(2,6-dioxopiperidin-3-yl)-1,3-dioxoisoindolin-4-yl)azetidine-3-carboxylic acid (6.7mg, 0.019 mmol) were added. And it was stirred at a room temperature for 12 hours. After completion of the reaction on TLC, the reaction mixture was quenched with water. Some solid material appeared and the filter was washed with excess water. To remove the residue, the filtered solid material was dissolved in chloroform and extracted. The organic layer was dried on anhydrous Na2SO4 and then was concentrated under the vaccum pressure to obtain target chemical, N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-4-yl)azetidin-3-carboxamide (8.0mg, 50 % yield) as yellow oil

¹H NMR : (500 MHz, CDCl₃) δ 7.48 (m, 1H), 7.39 (d, *J* = 8.1 Hz, 1H), 7.25 (d, *J* = 8.4 Hz, 1H), 7.21 (m,2H), 7.17-7.12 (m, 1H), 6.98 (d, *J* = 7.6 Hz, 1H), 6.94-6.83 (m, 3H), 6.65 (d, *J* = 8.3 Hz, 1H), 6.54 (s, 1H), 4.99-4.88 (m, 1H), 4.50 (d, *J* = 5.7 Hz, 1H), 4.42 (m,2H), 4.25 (d, *J* = 6.4 Hz, 1H), 3.66 (s, 2H), 3.30 (m,9H), 3.08 (m, 1H), 2.98 (s, 2H), 2.90 (s, 2H), 2.71 (m,6H), 2.52 (m, 4H), 2.45 (s, 3H), 2.41 (s, 4H), 2.06 (d, *J* = 11.3 Hz, 3H), 1.66 (m, 3H).

### <Example 52> Preparation of N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-4-yl)piperidin-4-carboxamide

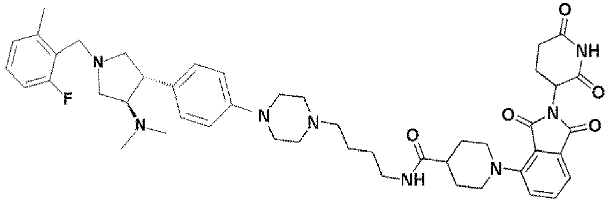

To (3R,4S)-4-(4-(4-(4-aminobutyl)piperazin-1-yl)phenyl)-1-(2-fluoro-6-methylbenzyl)-N,N-dimethyl)pyrrolidin-3-amine hydrochloride (15 mg, 0.019 mmol) in DMF (1 mL), EDCI (4.0 mg, 0.0209 mmol), HOBt (2.8 mg, 0.209 mmol), DIPEA (17 µL, 0.095 mmol), and 1-(1,3-dioxo-2-(2,6-dioxopiperidin-3-yl)isoindolin-4-yl)piperidin-4-carboxylic acid (7.6mg, 0.019 mmol) were added. And it was stirred at a room temperature for 12 hours. After completion of the reaction, the reaction mixture was diluted with water and extracted with ethyl acetate. The organic layer was dried on anhydrous Na2SO4 and then was distilled. Using purification by column chromatography, target chemical, N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-4-yl)piperidin-4-carboxamide was obtained (8.0mg, 50% yield.) as yellow oil.

¹H NMR : (500 MHz, MeOH-d4) δ 7.64 (t, *J* = 7.6 Hz, 1H), 7.35 (d, *J* = 7.1 Hz, 1H), 7.31 (d, *J* = 8.5 Hz, 1H), 7.21 (d, *J* = 8.1 Hz, 2H), 7.15 (q, *J* = 7.2 Hz, 1H), 7.00 (d, *J* = 7.7 Hz, 1H), 6.88 (dd, *J* = 16.5, 8.3 Hz, 2H), 5.09 (dd, *J* = 12.7, 5.5 Hz, 1H), 3.81 (d, *J* = 12.0 Hz, 2H), 3.65 (s, 2H), 3.24 (t, *J* = 6.3 Hz, 2H), 3.14 (m, 5H), 3.01-2.90 (m, 4H), 2.83 (d, *J* = 13.9 Hz, 1H), 2.71 (m, 1H), 2.65 (m, 4H), 2.52-2.48 (m, 1H), 2.45 (m, 4H), 2.18 (s, 6H), 2.10 (m, 1H), 2.05-1.96 (m, 2H), 1.89 (d, *J* = 13.2 Hz, 2H), 1.58 (m, 5H), 1.29 (s, 4H).

### <Example 53> Preparation of N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-5-ylamino)acetamide

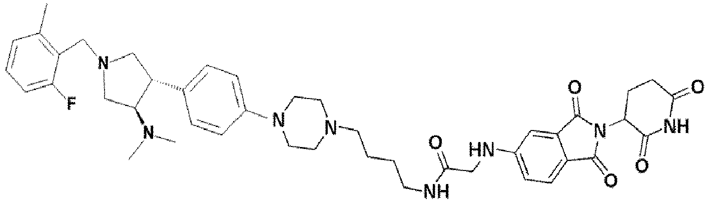

To (3R,4S)-4-(4-(4-(4-aminobutyl)piperazin-1-yl)phenyl)-1-(2-fluoro-6-methylbenzyl)-N,N-dimethyl)pyrrolidin-3-amine hydrochloride (10 mg, 0.019 mmol) in DMF (1 mL), EDCI (4.0 mg, 0.0209 mmol), HOBt (0.0209 mmol), DIPEA (17 µL, 0.095 mmol), and 2-(2-(2,6-dioxopiperidin-3-yl)-1,3-dioxoisoindolin-5-ylamino)acetic acid (7.6mg, 0.019 mmol) were added. And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-5-ylamino)acetamide was obtained (6.0mg, 40% yield) as yellow oil.

¹H NMR : (500 MHz, MeOH-d4) δ 7.64 (d, *J* = 8.3 Hz, 1H), 7.22 (d, *J* = 8.1 Hz, 2H), 7.16 (q, *J* = 7.5 Hz, 1H), 7.01 (d, *J* = 6.1 Hz, 2H), 6.89 (m, 4H), 5.04 (dd, *J* = 12.4, 6.0 Hz, 1H), 3.92 (s, 2H), 3.67 (s, 2H), 3.27 (t, *J* = 6.2 Hz, 2H), 3.15-3.04 (m, 6H), 2.98 (q, *J* = 7.9 Hz, 2H), 2.78-2.70 (m, 1H), 2.69-2.60 (m, 3H), 2.53 (m, 4H), 2.47 (s, 3H), 2.37 (t, *J* = 7.4 Hz, 2H), 2.21 (s, 6H), 1.50 (s, 2H), 1.30 (m, 2H).

### <Example 54> Preparation of N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-5-yl)piperidin-4-carboxamide

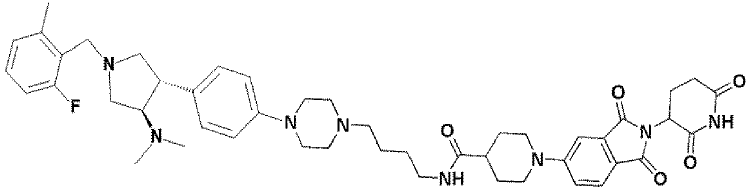

To (3R,4S)-4-(4-(4-(4-aminobutyl)piperazin-1-yl)phenyl)-1-(2-fluoro-6-methylbenzyl)-N,N-dimethyl)pyrrolidin-3-amine hydrochloride (10 mg, 0.019 mmol) in DMF (1 mL), EDCI (4.0 mg, 0.0209 mmol), HOBt (0.0209 mmol), DIPEA (17 µL, 0.095 mmol), and 1-(1,3-dioxo-2-(2,6-dioxopiperidin-3-yl)isoindolin-4-yl)piperidin-4-carboxyl acid (7.6mg, 0.019 mmol) were added. And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-5-yl)piperidin-4-carboxamide was obtained (7.0mg, 43% yield) as yellow oil.

¹H NMR : (300 MHz, MeOH-d4) δ 7.67 (d, *J* = 8.5 Hz, 1H), 7.35 (d, *J* = 2.3 Hz, 1H), 7.22 (m, 3H), 7.17-7.10 (m, 1H), 7.00 (d, *J* = 7.5 Hz, 1H), 6.89 (m, 3H), 5.06 (dd, *J* = 12.2, 5.4 Hz, 1H), 4.08 (d, *J* = 13.1 Hz, 3H), 3.65 (d, *J* = 2.3 Hz, 2H), 3.17 (m, 7H), 3.08-2.92 (m, 5H), 2.88-2.68 (m, 4H), 2.63 (t, *J* = 5.2 Hz, 4H), 2.49 (m, 1H), 2.45 (d, *J* = 7.6 Hz, 4H), 2.17 (s, 6H), 2.02-1.75 (m, 5H), 1.57 (m, 5H), 1.32 (m, 4H).

### <Example 55> Preparation of N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-(2-(2,6-dioxopiperidin-3-yl)-1,3-dioxoisoindolin-4-ylamino)acetamide

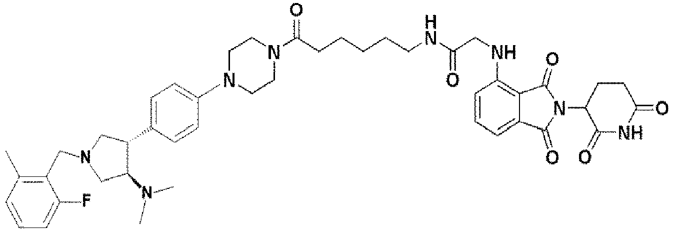

To (3R,4S)-1-(2-fluoro-6-methylbenzyl)-N,N-dimethyl-4-(4-(piperazin-1-yl)phenyl)pyrrolidin-3-amine (10 mg, 0.0252 mmol) in DMF (1 mL), EDCI (5.5 mg, 0.0277 mmol), HOBt (0.0277 mmol), DIPEA (18 µL, 0.100 mmol), and 2-(1,3-dioxo-2-(2,6-dioxopiperidin-3-yl)isoindolin-7-ylamino)-N-(6-carboxyhexyl)acetamide (12.0mg, 0.0252 mmol) were added. And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-(2-(2,6-dioxopiperidin-3-yl)-1,3-dioxoisoindolin-4-ylamino)acetamide was obtained (4.0mg, 20% yield) as yellow oil.

¹H NMR : (500 MHz, MeOH-d4) δ 7.58 (t, *J* = 7.9 Hz, 1H), 7.24 (d, *J* = 8.1 Hz, 2H), 7.15 (m, 2H), 7.01 (d, *J* = 7.6 Hz, 1H), 6.97-6.85 (m, 4H), 5.07 (dd, *J* = 12.6, 5.5 Hz, 1H), 3.99 (s, 2H), 3.69 (m, 4H), 3.65 (m, 2H), 3.22 (m, 4H), 3.11 (m, 4H), 3.01 (q, *J* = 7.6, 6.7 Hz, 2H), 2.89-2.79 (m, 1H), 2.77-2.69 (m, 3H), 2.50 (d, *J* = 7.7 Hz, 1H), 2.47 (s, 3H), 2.38 (t, *J* = 7.5 Hz, 2H), 2.32 (s, 6H), 2.03 (d, *J* = 12.1 Hz, 1H), 1.59 (q, *J* = 7.8 Hz, 2H), 1.52 (q, *J* = 7.2 Hz, 2H), 1.38-1.31 (m, 2H).

### <Example 56> Preparation of N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-(2-(2,6-dioxopiperidin-3-yl)-1,3-dioxoisoindolin-4-yloxy)acetamide

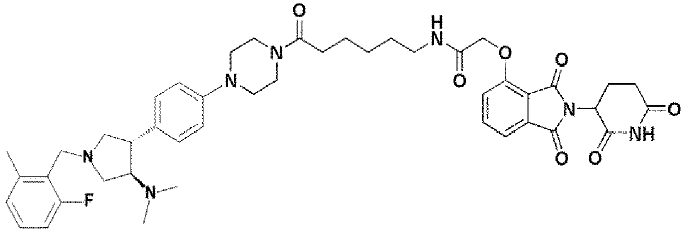

To (3R,4S)-1-(2-fluoro-6-methylbenzyl)-N,N-dimethyl-4-(4-(piperazin-1-yl)phenyl)pyrrolidin-3-amine (10 mg, 0.0252 mmol) in DMF (1 mL), EDCI (5.5 mg, 0.0277 mmol), HOBt (0.0277 mmol), DIPEA (18 µL, 0.100 mmol), and 6-(2-(1,3-dioxo-2-(2,6-dioxopiperidin-3-yl)isoindolin-7-yloxy)acetoamido-hexanoic aicd (12.0mg, 0.0252 mmol) were added. And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-(2-(2,6-dioxopiperidin-3-yl)-1,3-dioxoisoindolin-4-yloxy)acetamide was obtained (7.0mg, 35% yield) as yellow oil.

¹H NMR : (500 MHz, MeOH-d4) δ 7.79 (dd, *J* = 8.4, 7.3 Hz, 1H), 7.52 (d, *J* = 7.3 Hz, 1H), 7.41 (d, *J* = 8.2 Hz, 1H), 7.24-7.19 (m, 2H), 7.15 (m, 1H), 7.00 (d, *J* = 7.6 Hz, 1H), 6.93-6.84 (m, 4H), 5.13 (dd, *J* = 13.6, 5.4 Hz, 1H), 4.74 (s, 2H), 3.69 (t, *J* = 5.2 Hz, 2H), 3.65 (m, 4H), 3.36-3.33 (m, 2H), 3.12 (d, *J* = 5.6 Hz, 3H), 3.06 (t, *J* = 5.3 Hz, 2H), 3.00-2.92 (m, 4H), 2.88-2.81 (m, 1H), 2.76-2.69 (m, 2H), 2.61 (m, 1H), 2.52-2.48 (m, 1H), 2.46 (s, 3H), 2.43 (t, *J* = 7.6 Hz, 2H), 2.16 (s, 6H), 1.63 (m, 4H), 1.43 (m, 2H).

### <Example 57> Preparation of N-(6-(4-(4-((3S,4R)-4-(dimethylamino)-1-(2-fluoro-6-methylbenzyl)pyrrolidin-3-yl)phenyl)piperazin-1-yl)hexyl)-2-(2-(2,6-dioxopiperidin-3-yl)-1,3-dioxoisoindolin-4-ylamino)acetamide

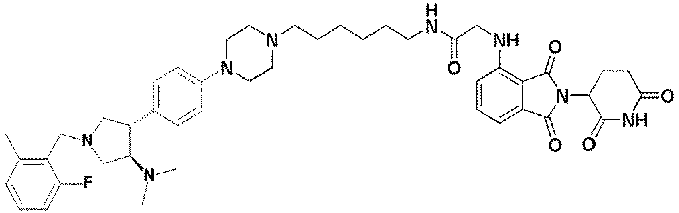

### Step 1: Preparation of tert-butyl (6-(4-(4-((3S,4R)-4-(dimethylamino)-1-(2-fluoro-6-methylbenzyl)pyrrolidin-3-yl)phenyl)piperazin-1-yl)hexyl)carbamate

(3R,4S)-1-(2-fluoro-6-methylbenzyl)-N,N-dimethyl in a suspension of the compound (100 mg, 0.252 mmol) prepared in step 3 of Example 1 in DMF (1 ml) -4-(4-(piperazin-1-yl)phenyl)pyrrolidin-3-amine (107 mg, 0.327 mmol) K2CO3 (104 mg, 0.756 mmol) was added, and for 2 h. After completion of the reaction in TLC, the reaction was quenched with water and extracted with EtOAc, the organic layer was dried over sodium sulfate, concentrated in vacuo and purified by column chromatography to give the title compound as a yellow oil (110 mg, 73 % yield).

¹H NMR : (300 MHz, CDCl₃) δ 7.23-7.10 (m, 3H), 6.98 (d, *J* = 7.6 Hz, 1H), 6.88 (m, 3H), 4.54 (s, 1H), 3.67 (m, 2H), 3.32 (m,2H), 3.24 (m, 5H), 3.12 (t, *J* = 7.8 Hz, 3H), 3.03 (d, *J* = 6.4 Hz, 2H), 2.67 (m, 4H), 2.53 (dd, *J* = 9.1, 6.3 Hz, 1H), 2.44 (m, 11H), 2.08 (m, 8H), 1.63-1.49 (m, 4H), 1.46 (s, 9H), 1.37 (m, 4H).

### Step 2: Preparation of (3R,4S)-4-(4-(4-(6-aminohexyl)piperazin-1-yl)phenyl)-1-(2-fluoro-6-methylbenzyl)-N,N-dimethylpyrrolidin-3-amine hydrochloride

To a suspension of the compound prepared in the previous step (110 mg, 0.184 mmol) in DCM (1 mL) was added 4.0 N HCl (0.8 mL) and the reaction was stirred for 2 h. After completion of the reaction by TLC, the solvent was evaporated to dryness to obtain the target compound as a yellow solid (80 mg, 80% yield).

¹H NMR (500 MHz, MeOD-d4) δ 7.54 (d, *J* = 8.3 Hz, 1H), 7.49-7.39 (m, 1H), 7.22 (d, *J* = 7.7 Hz, 1H), 7.13 (dd, *J* = 20.1, 8.9 Hz, 3H), 4.75 (m, 2H), 4.28 (t, *J* = 11.4 Hz, 1H), 4.07-4.00 (m, 1H), 3.93 (d, *J* = 12.9 Hz, 2H), 3.79-3.69 (m, 4H), 3.68 (s, 4H), 3.65-3.59 (m, 1H), 3.31-3.16 (m, 6H), 2.91 (m,7H), 2.63 (s, 3H), 1.89 (m, 2H), 1.73 (q, *J* = 7.4 Hz, 2H), 1.52 (m, 4H).

### Step 3: Preparation of N-(6-(4-(4-((3S,4R)-4-(dimethylamino)-1-(2-fluoro-6-methylbenzyl)pyrrolidin-3-yl)phenyl)piperazin-1-yl)hexyl)-2-(2-(2,6-dioxopiperidin-3-yl)-1,3-dioxoisoindolin-4-ylamino)acetamide

2-(1,3-dioxo-2-(2,6-dioxopiperidin-3-yl)isoindolin-4-ylamino)acetic acid in a solution of the compound prepared in the previous step (10 mg, 0.019 mmol) in DMF (1.0 mL) acid (6.2 mg, 0.019 mmol), EDCI (6.2 mg, 0.030 mmol), HOBt, 0.032 mmol), DIPEA (27 µL, 0.116 mmol) were added and the reaction was stirred at room temperature for 12 h.. After completion of the reaction by TLC, the reaction was quenched with water and extracted with EtOAc, the organic layer was dried over sodium sulfate, concentrated in vacuo, and purified by column chromatography to give the title compound (8.0 mg, 34 % yield) as a yellow oil.

¹H NMR : (500 MHz, MeOD-d4) δ 7.60 (dd, *J* = 8.5, 7.2 Hz, 1H), 7.28-7.20 (m, 2H), 7.19-7.12 (m, 2H), 7.02 (d, *J* = 7.6 Hz, 1H), 6.93-6.86 (m, 4H), 5.09 (dd, *J* = 12.5, 5.5 Hz, 1H), 4.01 (s, 2H), 3.67 (s, 2H), 3.24 (t, *J* = 6.9 Hz, 2H), 3.19-3.12 (m, 6H), 3.04-2.95 (m, 3H), 2.91-2.82 (m, 1H), 2.74 (m, 1H), 2.63 (m, 5H), 2.52 (dd, *J* = 9.1, 5.6 Hz, 1H), 2.49 (s, 3H), 2.41-2.37 (m, 2H), 2.19 (s, 6H), 2.16-2.10 (m, 1H), 1.52 (m,4H), 1.36-1.32 (m, 4H).

### <Example 58> Preparation of N-(6-(4-(4-((3S,4R)-4-(dimethylamino)-1-(2-fluoro-6-methylbenzyl)pyrrolidin-3-yl)phenyl)piperazin-1-yl)hexyl)-2-(2-(2,6-dioxopiperidin-3-yl)-1,3-dioxoisoindolin-4-yloxy)acetamide

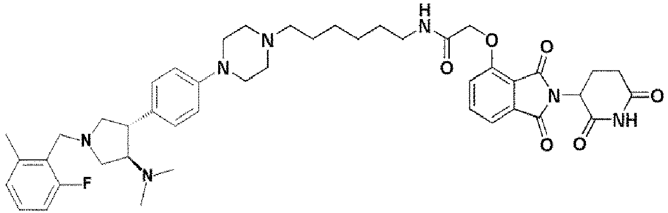

To (3R,4S)-1-(2-fluoro-6-methylbenzyl)-4-(4-(4-(6-aminohexyl)piperazin-1-yl)phenyl)-N,N-dimethylpyrrolidin-3-amine hydrochloride (10 mg, 0.019 mmol) in DMF (1 mL), EDCI (6.2 mg, 0.032 mmol), HOBt (0.0205 mmol), DIPEA (13 µL, 0.0748 mmol), and 2-(1,3-dioxo-2-(2,6-dioxopiperidin-3-yl)isoindolin-4-yloxy)acetic acid (6.2mg, 0.019 mmol) were added. And it was stirred at a room temperature for 12 hours. After completion of the reaction, the reaction mixture was quenched with water and extracted with ethyl acetate.

The organic layer was dried on anhydrous Na2SO4 and then was concentrated to unpurified oil in vacuum pressure. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)hexyl)-2-(2-(2,6-dioxopiperidin-3-yl)-1,3-dioxoisoindolin-4-yloxy)acetamide was obtained (4 mg, 26% yield) as yellow oil.

¹H NMR : (500 MHz, MeOH-d4) δ 7.82 (dd, *J* = 8.5, 7.4 Hz, 1H), 7.55 (d, *J* = 7.3 Hz, 1H), 7.44 (d, *J* = 8.4 Hz, 1H), 7.22 (d, *J* = 8.6 Hz, 2H), 7.16 (m, 1H), 7.01 (d, *J* = 7.6 Hz, 1H), 6.90 (m,3H), 5.13 (dd, *J* = 12.4, 5.6 Hz, 1H), 4.76 (s, 2H), 3.67 (s, 2H), 3.16 (m,6H), 2.99 (t, *J* = 8.1 Hz, 2H), 2.92-2.81 (m, 1H), 2.78-2.63 (m, 7H), 2.50 (dd, *J* = 9.0, 5.8 Hz, 1H), 2.47 (m,5H), 2.26 (s, 6H), 2.18-2.10 (m, 1H), 1.59 (m,4H), 1.41 (m,6H).

### <Example 59> Preparation of N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-4-yl)azetidin-3-carboxamide

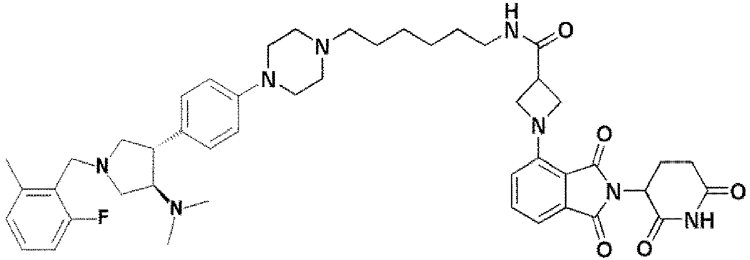

To (3R,4S)-1-(2-fluoro-6-methylbenzyl)-4-(4-(4-(6-aminohexyl)piperazin-1-yl)phenyl)-N,N-dimethylpyrrolidin-3-amine hydrochloride (10 mg, 0.0187 mmol) in DMF (1 mL), EDCI (4.0 mg, 0.020 mmol), HOBt (0.020 mmol), DIPEA (13 µL, 0.076 mmol), and 1-(2-(2,6-dioxopiperidin-3-yl)-1,3-dioxoisoindolin-4-yl)azetidine-3-carboxyl acid (6.6mg, 0.0187 mmol) were added. And it was stirred at a room temperature for 12 hours. After completion of the reaction, the reaction mixture was quenched with water and extracted with ethyl acetate.

The organic layer was dried on anhydrous Na2SO4 and then was concentrated to unpurified oil in vacuum pressure. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-4-yl)azetidin-3-carboxamide was obtained (3 mg, 20% yield) as yellow oil.

¹H NMR : (500 MHz, MeOH-d4) δ 7.55 (m, 1H), 7.23 (m, 2H), 7.19-7.11 (m, 1H), 7.09-7.03 (m, 1H), 7.01 (d, *J* = 7.7 Hz, 1H), 6.96-6.85 (m, 3H), 6.78 (d, *J* = 8.4 Hz, 1H), 5.09-4.99 (m, 1H), 3.68 (s, 2H), 3.35 (s, 2H), 3.26-3.14 (m, 7H), 3.01 (q, *J* = 8.4 Hz, 2H), 2.76-2.66 (m, 6H), 2.48 (m,6H), 2.32 (s, 6H), 1.64-1.48 (m, 6H), 1.44-1.30 (m, 10H).

### <Example 60> Preparation of N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-4-yl)piperidin-4-carboxamide

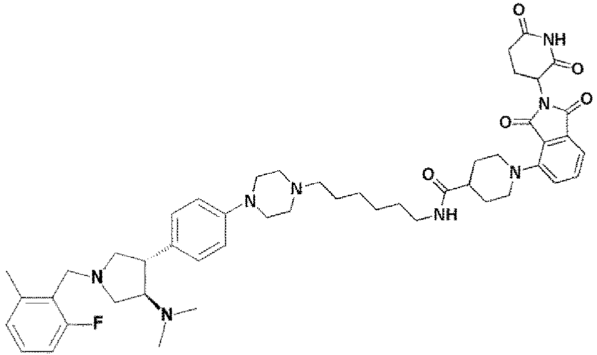

To (3R,4S)-4-(4-(4-(6-aminohexyl)piperazin-1-yl)phenyl)-1-(2-fluoro-6-methylbenzyl)-N,N-dimethylpyrrolidin-3-amine hydrochloride (10 mg, 0.018 mmol, Prepared in example 57 step 2) in DMF (1 mL), EDCI (4.0 mg, 0.0198 mmol), HOBt (0.0198 mmol), DIPEA (16 µL, 0.095 mmol), and 1-(1,3-dioxo-2-(2,6-dioxopiperidin-3-yl)isoindolin-4-yl)piperidine-4-carboxyl acid (7.2mg, 0.018 mmol) were added. And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-4-yl)piperidin-4-carboxamide was obtained (8 mg, 50% yield) as yellow solid.

¹H NMR : (500 MHz, MeOH-d4) δ 7.66 (t, *J* = 7.8 Hz, 1H), 7.35 (dd, *J* = 16.4, 7.6 Hz, 3H), 7.23 (d, *J* = 8.2 Hz, 2H), 7.17 (q, *J* = 7.4 Hz, 1H), 7.01 (d, *J* = 7.6 Hz, 1H), 6.90 (dd, *J* = 16.6, 8.5 Hz, 3H), 5.10 (dd, *J* = 12.6, 5.5 Hz, 1H), 3.83 (d, *J* = 12.0 Hz, 3H), 3.67 (s, 2H), 3.22 (t, *J* = 7.0 Hz, 3H), 3.15 (m, 5H), 3.07-2.96 (m, 4H), 2.92 (m, 1H), 2.87-2.82 (m, 1H), 2.75 (d, *J* = 15.6 Hz, 2H), 2.68-2.60 (m, 5H), 2.51 (m, 1H), 2.46 (m, 4H), 2.19 (s, 6H), 2.14-2.07 (m, 1H), 2.05-1.96 (m, 2H), 1.91 (s, 2H), 1.65-1.51 (m, 5H), 1.43-1.37 (m, 4H).

### <Example 61> Preparation of N-(6-(4-(4-((3S,4R)-4-(dimethylamino)-1-(2-fluoro-6-methylbenzyl)pyrrolidin-3-yl)phenyl)piperazin-1-yl)hexyl)-2-((2-(2,6-dioxopiperidin-3-yl)-1,3-dioxoisoindolin-5-yl)amino)acetamide

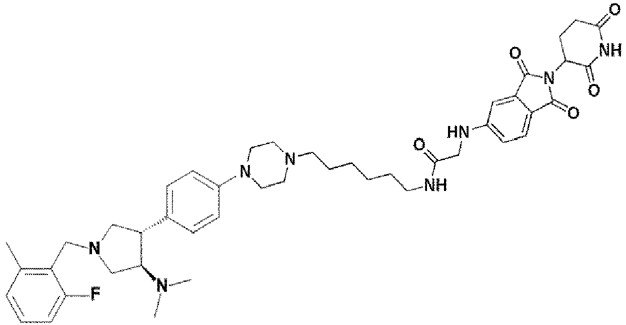

To (3R,4S)-4-(4-(4-(6-aminohexyl)piperazin-1-yl)phenyl)-1-(2-fluoro-6-methylbenzyl)-N,N-dimethylpyrrolidin-3-amine hydrochloride (10 mg, 0.0188 mmol, Prepared in example 57 step 2) in DMF (1 mL), EDCI (4.0 mg, 0.0209 mmol), HOBt (0.0209 mmol), DIPEA (17 µL, 0.095 mmol), and 2-(2-(2,6-dioxopiperidin-3-yl)-1,3-dioxoisoindolin-5-ylamino)acetic acid (6.2mg, 0.0188 mmol) were added. And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)hexyl)-2-((2-(2,6-dioxopiperidin-3-yl)-1,3-dioxoisoindolin-5-yl)amino)acetamide was obtained (6.0 mg, 46% yield) as yellow oil.

¹H NMR : (300 MHz, MeOH-d4) δ 8.00 (s, 1H), 7.64 (d, *J* = 8.3 Hz, 1H), 7.27-7.15 (m, 3H), 7.04-6.98 (m, 2H), 6.90 (m, 4H), 5.05 (m, 1H), 3.92 (s, 2H), 3.68 (d, *J* = 2.3 Hz, 2H), 3.25 (t, *J* = 6.6 Hz, 2H), 3.17 (m,, 6H), 3.01 (m, 5H), 2.88 (s, 3H), 2.71 (m, 8H), 2.48 (m, 5H), 2.27 (s, 6H), 1.52 (m, 4H).

### <Example 62> Preparation of N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-5-yl)piperidin-4-carboxamide

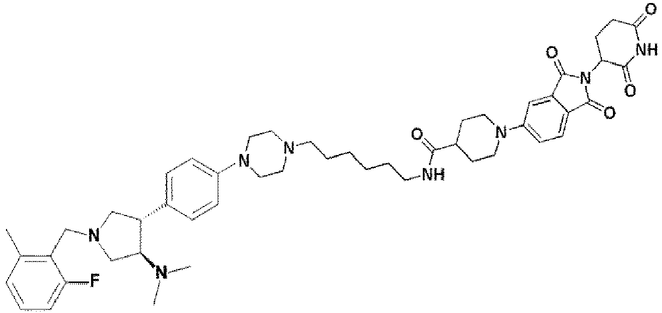

To (3R,4S)-4-(4-(4-(6-aminohexyl)piperazin-1-yl)phenyl)-1-(2-fluoro-6-methylbenzyl)-N,N-dimethylpyrrolidin-3-amine hydrochloride (10 mg, 0.019 mmol, Prepared in example 57 step 2) in DMF (1 mL), EDCI (4.0 mg, 0.0209 mmol), HOBt (0.0209 mmol), DIPEA (17 µL, 0.095 mmol), and 1-(1,3-dioxo-2-(2,6-dioxopiperidin-3-yl)isoindolin-4-yl)piperidine-4-carboxyl acid (7.2mg, 0.018 mmol). And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-5-yl)piperidin-4-carboxamide was obtained (8.0 mg, 45% yield) as yellow oil.

¹H NMR : (300 MHz, MeOH-d4) δ 7.66 (d, *J* = 8.6 Hz, 1H), 7.34 (m, 1H), 7.28-7.19 (m, 3H), 7.19-7.10 (m, 1H), 7.01 (d, *J* = 7.5 Hz, 1H), 6.96-6.82 (m, 3H), 5.06 (dd, *J* = 12.2, 5.4 Hz, 1H), 4.07 (d, *J* = 13.1 Hz, 2H), 3.72-3.65 (m, 2H), 3.19 (m, 7H), 3.02 (m, 5H), 2.90-2.76 (m, 2H), 2.73-2.62 (m, 5H), 2.47 (m, 6H), 2.30 (m, 6H), 2.14-2.07 (m, 1H), 2.02 (m, 1H), 1.86 (m, 2H), 1.78 (m, 2H), 1.55 (m, 4H), 1.39 (m, 4H).

### <Example 63> Preparation of N-(8-(4-(4-((3S,4R)-4-(dimethylamino)-1-(2-fluoro-6-methylbenzyl)pyrrolidin-3-yl)phenyl)piperazin-1-yl)octyl)-1-(2-(2,6-dioxopiperidin-3-yl)-1,3-dioxoisoindolin-5-yl)piperidin-4-carboxamide

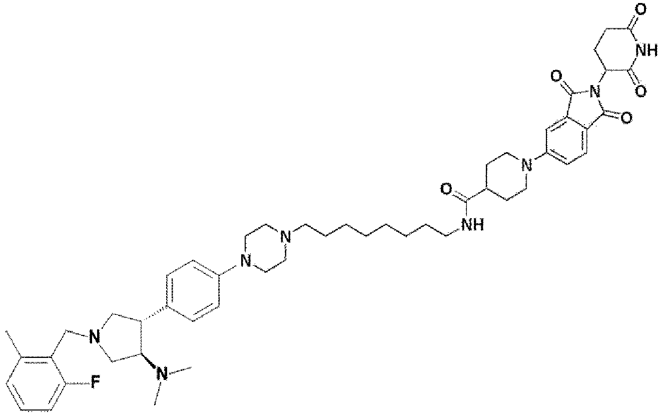

### Step 1: Preparation of tert-butyl (8-hydroxyoctyl) carbamate

To a solution of 8-amino octane-1-ol (1.0 g, 6.88 mmol) in THF (30 mL) was added TEA (1.5 mL, 10.32 mmol) and the reaction was cooled to 5-10 °C. BOC anhydride (1.9 mL, 8.25 mmol) is added and the temperature is raised to room temperature. The reaction is stirred for 1 hour. After completion of the reaction, the reaction was quenched with water and extracted with EtOAc, the organic layer was dried over sodium sulfate, concentrated in vacuo, and purified by column chromatography to give the title compound (1.4 g, 85 % yield) as a white solid.

¹H NMR: (300 MHz, DMSO) δ 6.75 (s, 1H), 4.32 (t, *J* = 5.2 Hz, 1H), 3.37 (m, 2H), 2.89 (q, *J* = 6.6 Hz, 2H), 1.42 (m, 1H), 1.37 (s, 9H), 1.21 (m, 11H).

### Step 2: Preparation of 8-((tert-butoxycarbonyl)amino)octyl 4-methylbenzensulfonate

To a solution of the compound prepared in the previous step (1.4 g, 5.70 mmol) in DCM (30 ml) was added TEA (2.4 mL, 17.1 mmol) followed by DMAP (348 mg, 2.85 mmol) and the reaction mass was stirred at 5 °C-10 cooled to °C. After addition of TsCl (2.2 g, 11.41 mmol), the temperature was raised to room temperature and stirred overnight. After completion of the reaction, the reaction was quenched with water, extracted with EtOAc, the organic layer was dried over sodium sulfate, concentrated in vacuo, and purified by column chromatography to give the title compound (1.8 g, 80 % yield) as a colorless oil.

¹H NMR : (500 MHz, CDCl₃) δ 7.81 (d, *J* = 8.3 Hz, 2H), 7.37 (d, *J* = 8.0 Hz, 2H), 4.52 (s, 1H), 4.03 (t, *J* = 6.5 Hz, 2H), 3.11 (q, *J* = 6.8 Hz, 2H), 2.47 (s, 3H), 1.69-1.66 (m, 1H), 1.65-1.60 (m, 1H), 1.46 (s, 9H), 1.35-1.24 (m, 10H).

### Step 3: Preparation of tert-butyl (8-iodooctyl) carbamate

To a solution of the compound prepared in the previous step (2.20 g, 5.92 mmol) in acetone (50 ml) was added sodium iodide (3.5 g, 23.68 mmol). The reaction mass was stirred at 60 °C for 2 h. After completion of the reaction, the reaction was distilled and the residue was quenched with water and extracted with EtOAc, the organic layer was dried over sodium sulfate and concentrated in vacuo to give the title compound (1.60 g, 90 % yield) as a yellow oil.

¹H NMR: (300 MHz, CDCl₃) δ 4.54 (s, 1H), 3.19 (t, *J* = 7.0 Hz, 2H), 3.11 (q, *J* = 6.7 Hz, 2H), 1.82 (p, *J* = 7.0 Hz, 2H), 1.48 (m, 2H), 1.45 (s, 9H), 1.32 (m, 8H).

### Step 4: Preparation of tert- butyl (8- (4- (4-((3S, 4R) -4- (dimethyl amino) -1- (2- fluoro-6- methyl benzyl) pyrrolidin -3-yl) phenyl) piperazin -1-yl) octyl) carbamate

(3R,4S)-1-(2-fluoro-6-methylbenzyl)-N,N-dimethyl-4-(4-(piperazin-1-yl)phenyl)pyrrolidin-3-amine ( 100 mg, 0.25 mmol) was added with the compound prepared in the previous step (178 mg, 0.504 mmol) K2CO3 (104 mg, 0.756 mmol), and for 1 hour. After completion of the reaction, the reaction mixture was quenched with water and extracted with EtOAc, the organic layer was dried over sodium sulfate, concentrated in vacuo and purified by column chromatography to give the title compound as a yellow oil (100 mg, 64 % yield).

¹H NMR (500 MHz, MeOH-d4) δ 7.23 (d, *J* = 8.6 Hz, 2H), 7.19-7.12 (m, 1H), 7.01 (d, *J* = 7.5 Hz, 1H), 6.91 (dd, *J* = 14.4, 8.9 Hz, 3H), 3.68 (s, 2H), 3.19 (m, 6H), 3.06-2.97 (m, 4H), 2.72 (m, 4H), 2.52-2.45 (m, 6H), 2.31 (s, 6H), 1.59 (s, 2H), 1.47 (d, *J* = 9.9 Hz, 2H), 1.43 (s, 9H), 1.32 (m,10H).

### Step 5: Preparation of (3R,4S)-4-(4-(4-(8-aminooctyl)piperazin-1-yl)phenyl)-1-(2-fluoro-6-methylbenzyl)-N,N-dimethylpyrrolidin-3-amine hydrochloride

To a solution of the compound prepared in the previous step (95 mg, 0.152 mmol) in DCM (1 mL) was added 4.0 N HCl (0.8 mL), and the reaction was stirred at room temperature for 2 h. After completion of the reaction by TLC, the solvent was evaporated to dryness to obtain the desired compound as a yellow solid (80 mg, 94 % yield).

¹H NMR : (500 MHz, MeOH-d4) δ 6.89 (d, *J* = 8.4 Hz, 2H), 6.85-6.76 (m, 1H), 6.57 (d, *J* = 7.7 Hz, 1H), 6.48 (dd, *J* = 18.9, 8.9 Hz, 3H), 4.10 (m, 1H), 3.96 (q, *J* = 8.0 Hz, 1H), 3.64 (m, 1H), 3.43-3.34 (m, 1H), 3.28 (d, *J* = 13.1 Hz, 2H), 3.04 (m, 9H), 2.97 (t, *J* = 11.4 Hz, 1H), 2.65-2.46 (m, 6H), 2.37-2.14 (m, 6H), 1.99 (s, 3H), 1.21 (m, 2H), 1.05 (m, 2H), 0.81 (d, *J* = 5.2 Hz, 8H).

### Step 6: Preparation of N-(8-(4-(4-((3S,4R)-4-(dimethylamino)-1-(2-fluoro-6-methylbenzyl)pyrrolidin-3-yl)phenyl)piperazin-1-yl)octyl)-1-(2-(2,6-dioxopiperidin-3-yl)-1,3-dioxoisoindolin-5-yl)piperidin-4-carboxamide

1-(2-(2,6-dioxopiperidin-3-yl)-1,3-dioxoisoindolin-5-yl)piperidin to a solution of the compound prepared in the previous step (10 mg, 0.0178 mmol) in DMF (1.0 mL) -4-carboxylate (6.0 mg, 0.0178 mmol), EDCI (4.0 mg, 0.0195 mmol), HOBt, 0.0195 mmol), DIPEA (12 µl, 0.0712 mmol) were added and the reaction was stirred at room temperature for 12 h. After completion of the reaction, the reaction material is diluted with water and extracted with EtOAc. The organic layer was dried over anhydrous Na2SO4, distilled and purified by column chromatography to give the desired compound (8.0 mg, 50 % yield) as a yellow oil.

¹H NMR : (500 MHz, MeOH-d4) δ 7.66 (d, *J* = 8.5 Hz, 1H), 7.34 (d, *J* = 2.4 Hz, 1H), 7.24-7.20 (m, 3H), 7.15 (m, 1H), 7.00 (d, *J* = 7.6 Hz, 1H), 6.90 (m, 3H), 5.06 (dd, *J* = 12.6, 5.5 Hz, 1H), 4.07 (d, *J* = 13.2 Hz, 2H), 3.66 (s, 2H), 3.20-3.11 (m, 8H), 3.05-2.96 (m, 4H), 2.87-2.79 (m, 1H), 2.77-2.63 (m, 7H), 2.51-2.44 (m, 6H), 2.25 (s, 6H), 2.12-2.06 (m, 1H), 1.86 (m, 2H), 1.83-1.75 (m, 2H), 1.64-1.46 (m, 6H), 1.36 (m, 6H).

### <Example 64> Preparation of N-(8-(4-(4-((3S,4R)-4-(dimethylamino)-1-(2-fluoro-6-methylbenzyl)pyrrolidin-3-yl)phenyl)piperazin-1-yl)octyl)-2-((2-(2,6-dioxopiperidin-3-yl)-1,3-dioxoisoindolin-5-yl)amino)acetamide

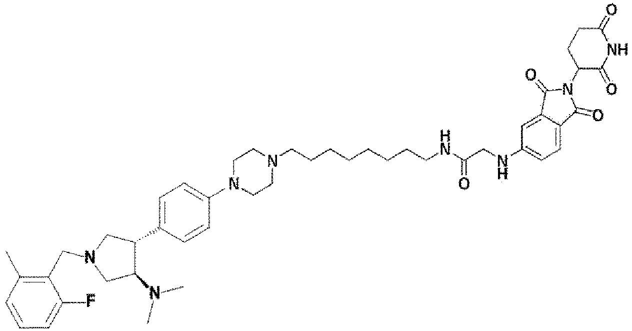

To (3R,4S)-4-(4-(4-(8-aminooctyl)piperazin-1-yl)phenyl)-1-(2-fluoro-6-methylbenzyl)-N,N-dimethylpyrrolidin-3-amine hydrochloride (10 mg, 0.0178 mmol, Prepared in example 63 step 5) in DMF (1.0 mL), EDCI (4.0 mg, 0.0195 mmol), HOBt (0.0195 mmol), DIPEA (12 µL, 0.0712 mmol), and 2-(2-(2,6-dioxopiperidin-3-yl)-1,3-dioxoisoindolin-5-ylamino)acetic acid (6.0mg, 0.0178 mmol). And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, N-(8-(4-(4-((3S,4R)-4-(dimethylamino)-1-(2-fluoro-6-methylbenzyl)pyrrolidin-3-yl)phenyl)piperazin-1-yl)octyl)-2-((2-(2,6-dioxopiperidin-3-yl)-1,3-dioxoisoindolin-5-yl)amino)acetamide was obtained (6.0 mg, 40% yield) as yellow oil.

¹H NMR : (500 MHz, MeOH-d4) δ 7.62 (d, *J* = 8.3 Hz, 1H), 7.22 (d, *J* = 8.1 Hz, 2H), 7.16 (q, *J* = 7.3 Hz, 1H), 7.06-6.94 (m, 2H), 6.89 (dd, *J* = 16.3, 8.6 Hz, 4H), 5.05 (dd, *J* = 12.4, 5.5 Hz, 1H), 3.90 (s, 2H), 3.66 (s, 2H), 3.22 (t, *J* = 6.9 Hz, 2H), 3.14 (m, 5H), 3.05 (m, 1H), 2.97 (q, *J* = 8.8, 8.0 Hz, 2H), 2.88-2.80 (m, 1H), 2.77-2.69 (m, 2H), 2.65 (m, 5H), 2.58 (m, 1H), 2.50 (t, *J* = 7.5 Hz, 1H), 2.47 (s, 3H), 2.44-2.38 (m, 2H), 2.20 (s, 6H), 2.12-2.04 (m, 1H), 1.57-1.44 (m, 6H), 1.36 (m, 6H).

### <Example 65> Preparation of 4-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethoxy)-2-(2,6-dioxopiperidin-3-yl)isoindolin-1,3-dione

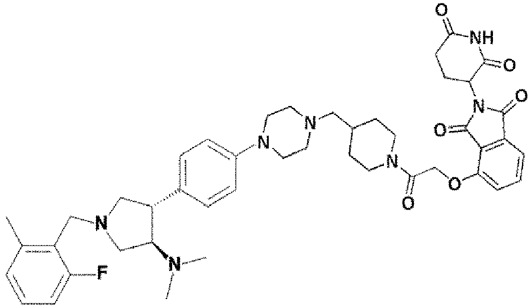

### Step 1: Preparation of tert-butyl 4-((4-(4-((3S,4R)-4-(dimethylamino)-1-(2-fluoro-6-methylbenzyl)pyrrolidin-3-yl)phenyl)piperazin-1-yl)methyl)piperidin-1-carboxylate

(3R,4S)-1-(2-fluoro-6-methylbenzyl)-N,N-dimethyl-4-(4-(piperazin-1-yl)phenyl)pyrrolidin-3-amine ( 100 mg, 0.252 mmol) in tert-butyl 4-formylpiperidin-1-carboxylate (134 mg, 0.630 mmol), AcOH (0.5 mL) was reacted at room temperature for 1 hour. NaBH3CN (95 mg, 1.512 mmol) was charged slowly and the reaction was stirred at room temperature overnight. After completion of the reaction, the reaction mass was distilled and the residue was quenched with 5% K2CO3 solution and extracted with DCM. The organic layer was dried over anhydrous Na2SO4, distilled and purified by column chromatography to give the desired compound (106 mg, 72 % yield) as a white solid.

¹H NMR : (500 MHz, CDCl₃) δ 7.20-7.15 (m, 2H), 7.14-7.08 (m, 1H), 6.96 (d, *J* = 7.6 Hz, 1H), 6.84 (m, 3H), 4.10 (s, 2H), 3.64 (s, 2H), 3.20-3.08 (m, 7H), 3.02 (t, *J* = 8.5 Hz, 1H), 2.95 (t, *J* = 8.5 Hz, 1H), 2.76-2.65 (m, 4H), 2.54 (m, 6H), 2.44 (s, 3H), 2.30 (s, 6H), 2.22 (d, *J* = 7.0 Hz, 2H), 1.46 (s, 9H).

### Step 2: Preparation of (3R,4S)-1-(2-fluoro-6-methylbenzyl)-N,N-dimethyl-4-(4-(4-piperidine-4-ylmethyl)piperazine-1-yl)phenyl)pyrrolidine-3-amine

To a solution of the compound prepared in the previous step (106 mg, 0.213 mmol) in DCM (10 mL) was added 4.0 N HCl in dioxane (1.0 mL) and the reaction was stirred at room temperature for 3 h. After completion of the reaction, it is dried by distillation to obtain a white solid. The crude material was extracted with 5% K2CO3 and EtOAc, the organic layer was dried over sodium sulfate and concentrated in vacuo to give the desired compound (85 mg, 95% yield) as a yellow oil.

¹H NMR (500 MHz, CDCl₃) δ 7.21-7.15 (m, 2H), 7.10 (m, 1H), 6.94 (d, *J* = 7.5 Hz, 1H), 6.86-6.80 (m, 3H), 3.61 (d, *J* = 2.3 Hz, 2H), 3.14 (t, *J* = 5.0 Hz, 4H), 3.09 (m, 3H), 2.96-2.90 (m, 3H), 2.63-2.58 (m, 2H), 2.54 (m, 5H), 2.45 (s, 3H), 2.21 (d, *J* = 7.2 Hz, 2H), 2.16 (s, 6H), 1.79-1.72 (m, 2H), 1.15-1.08 (m, 2H).

### Step 3: Preparation of 4-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethoxy)-2-(2,6-dioxopiperidin-3-yl)isoindolin-1,3-dione

2-(1,3-dioxo-2-(2,6-dioxopiperidin-3-yl)isoindolin-4-yloxy) in a solution of the compound prepared in the previous step (10 mg, 0.0198 mmol) in DMF (1 mL) Acetic acid (6.5 mg, 0.0198 mmol), EDCI (4.0 mg, 0.0217 mmol), HOBt, 0.0217 mmol), DIPEA (20 µL, 0.099 mmol) were added and the reaction was stirred at room temperature for 12 h. After completion of the reaction, the reaction material is diluted with water and extracted with EtOAc. The organic layer was dried over anhydrous Na2SO4, distilled and purified by column chromatography to give the desired compound (4.0 mg, 26 % yield) as a colorless oil.

¹H NMR : (500 MHz, MeOH-d4) δ 7.80 (s, 1H), 7.64 (dd, *J* = 8.6, 7.3 Hz, 1H), 7.38 (d, *J* = 7.3 Hz, 1H), 7.24 (d, *J* = 8.5 Hz, 1H), 7.14-7.09 (m, 2H), 7.05 (m, 1H), 6.90 (d, *J* = 7.6 Hz, 1H), 6.79 (dd, *J* = 18.7, 8.9 Hz, 3H), 5.05 (d, *J* = 14.7 Hz, 1H), 4.99 (m, 2H), 4.35 (d, *J* = 13.2 Hz, 1H), 3.88 (d, *J* = 13.8 Hz, 1H), 3.57 (s, 2H), 3.11-3.00 (m, 7H), 2.88 (m, 2H), 2.81-2.70 (m, 1H), 2.68-2.55 (m, 4H), 2.50 (t, *J* = 4.9 Hz, 4H), 2.42-2.38 (m, 1H), 2.36 (s, 3H), 2.19-2.17 (m, 1H), 2.15 (s, 6H), 2.06-1.99 (m, 1H), 1.78 (d, *J* = 13.8 Hz, 3H), 1.00 (m, 2H), 0.79 (m, 2H).

### <Example 66> Preparation of 4-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-2-(2,6-dioxopiperidin-3-yl)isoindolin-1,3-dione

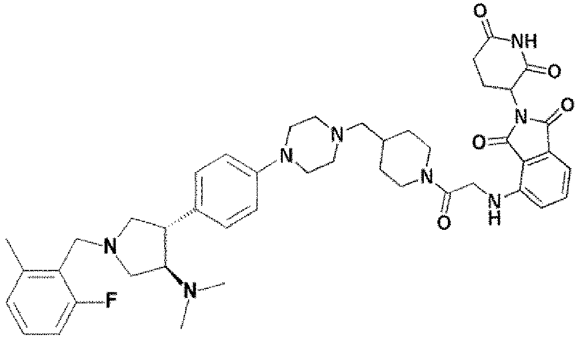

To (3R,4S)-1-(2-fluoro-6-methylbenzyl)-N,N-dimethyl-4-(4-(4-piperidine-4-ylmethyl)piperazine-1-yl)phenyl)pyrrolidine-3-amine (10 mg, 0.0188 mmol, Prepared in example 65 step 2) in DMF (1.0 mL), EDCI (4.0 mg, 0.02076 mmol), HOBt (0.0206 mmol), DIPEA (16 µL, 0.093 mmol), and 2-(1,3-dioxo-2-(2,6-dioxopiperidin-3-yl)isoindolin-4-ylamino)acetic acid (6.5mg, 0.01868 mmol). And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, 4-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-2-(2,6-dioxopiperidin-3-yl)isoindolin-1,3-dione was obtained (5.0 mg, 33% yield) as yellow oil.

¹H NMR : (500 MHz, MeOH-d4) δ 7.80 (s, 1H), 7.45 (dd, *J* = 8.5, 7.2 Hz, 1H), 7.14-7.10 (m, 2H), 7.06 (m, 1H), 6.97 (d, *J* = 7.1 Hz, 1H), 6.90 (dd, *J* = 8.1, 3.8 Hz, 2H), 6.81 (d, *J* = 8.7 Hz, 2H), 6.77 (d, *J* = 8.9 Hz, 1H), 4.96 (dd, *J* = 12.6, 5.5 Hz, 1H), 4.41 (d, *J* = 13.4 Hz, 1H), 4.11 (d, *J* = 16.8 Hz, 1H), 4.06 (d, *J* = 16.7 Hz, 1H), 3.84 (d, *J* = 13.7 Hz, 1H), 3.57 (d, *J* = 2.2 Hz, 2H), 3.06 (m, 6H), 2.93-2.86 (m, 2H), 2.81-2.73 (m, 1H), 2.64 (m, 3H), 2.50 (t, *J* = 5.1 Hz, 4H), 2.39 (dd, *J* = 9.3, 5.3 Hz, 1H), 2.36 (s, 3H), 2.19 (s, 6H), 2.02 (m, 1H), 1.92 (d, *J* = 11.7 Hz, 1H), 1.86-1.72 (m, 3H), 1.47 (m, 1H), 1.13 (m, 1H), 1.03 (m, 2H).

### <Example 67> Preparation of 3-(6-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-4-oxobenzo[d][1,2,3]triazin-3(4H)-yl)piperidin-2,6-dione

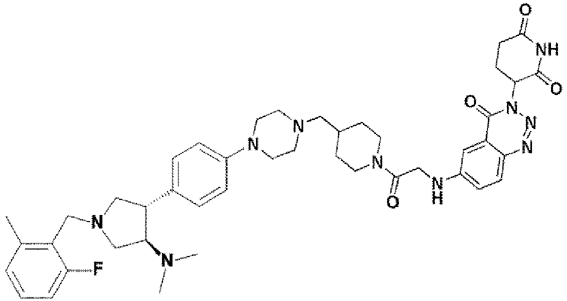

To (3R,4S)-1-(2-fluoro-6-methylbenzyl)-N,N-dimethyl-4-(4-(4-piperidine-4-ylmethyl)piperazine-1-yl)phenyl)pyrrolidine-3-amine (10 mg, 0.0188 mmol, Prepared in example 65 step 2) in DMF (1.0 mL), EDCI (4.0 mg, 0.0207 mmol), HOBt (0.0206 mmol), DIPEA (16 µL, 0.093 mmol), and 2-(2-(2,6-dioxopiperidin-3-yl)-1,3-dioxoisoindolin-5-ylamino)acetic acid (6.5mg, 0.01868 mmol). And it was stirred at a room temperature for 12 hours. After completion of the reaction, the reaction mixture was diluted with water and extracted with ethyl acetate.

The organic layer was dried on anhydrous Na2SO4 and then was distilled. Using purification by column chromatography, target chemical, 3-(6-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-4-oxobenzo[d][1,2,3]triazin-3(4H)-yl)piperidin-2,6-dione was obtained (4.0 mg, 26% yield) as colorless oil.

¹H NMR : (500 MHz, MeOH-d4) δ 7.49 (d, *J* = 8.3 Hz, 1H), 7.12 (d, *J* = 8.7 Hz, 2H), 7.06 (td, *J* = 7.9, 5.7 Hz, 1H), 6.94 (d, *J* = 2.2 Hz, 1H), 6.90 (d, *J* = 7.6 Hz, 1H), 6.82 (m, 3H), 6.77 (d, *J* = 9.0 Hz, 1H), 4.94 (dd, *J* = 12.4, 5.5 Hz, 1H), 4.41 (d, *J* = 13.3 Hz, 1H), 4.06 (d, *J* = 16.7 Hz, 1H), 4.00 (d, *J* = 16.7 Hz, 1H), 3.87 (d, *J* = 13.6 Hz, 1H), 3.58 (d, *J* = 2.2 Hz, 2H), 3.16 (q, *J* = 6.1 Hz, 1H), 3.12-3.09 (m, 1H), 3.07-3.02 (m, 4H), 2.95-2.88 (m, 2H), 2.79-2.72 (m, 1H), 2.63 (m, 3H), 2.51 (t, *J* = 5.1 Hz, 4H), 2.39 (dd, *J* = 9.1, 6.2 Hz, 1H), 2.36 (s, 3H), 2.24 (s, 6H), 2.19 (d, *J* = 6.9 Hz, 2H), 2.03-1.96 (m, 1H), 1.92 (d, *J* = 11.8 Hz, 1H), 1.85-1.73 (m, 3H), 1.13 (m,2H), 1.10-0.96 (m, 2H).

### <Example 68> Preparation of 3-(6-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethoxy)-4-oxobenzo[d][1,2,3]triazin-3(4H)-yl)piperidin-2,6-dione

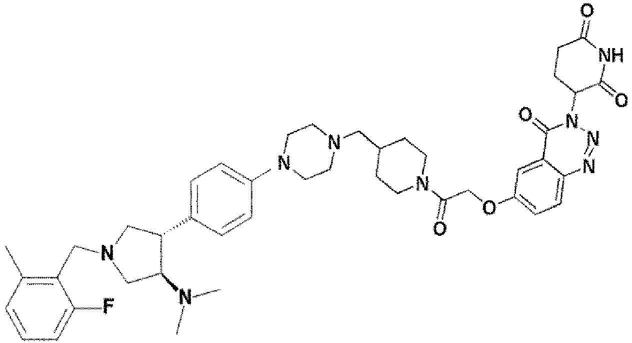

¹H NMR : (300 MHz, CDCl₃) δ 8.18-8.10 (m, 1H), 7.68-7.60 (m, 2H), 7.18 (d, J = 8.4 Hz, 2H), 7.14-7.05 (m, 1H), 6.95 (d, J = 7.5 Hz, 1H), 6.85 (t, J = 8.5 Hz, 3H), 5.84-5.71 (m, 1H), 4.90 (s, 2H), 4.57 (d, J = 13.0 Hz, 1H), 3.82 (d, J = 13.4 Hz, 1H), 3.63 (s, 2H), 3.15 (m, 7H), 3.04-2.89 (m, 4H), 2.87-2.72 (m, 2H), 2.67 (t, J = 12.5 Hz, 1H), 2.53 (m, 5H), 2.43 (s, 3H), 2.30 (s, 6H), 2.24 (d, 2H), 1.94 (m, 2H), 1.84 (m, 2H), 1.15 (m, 2H)

### <Example 69> Preparation of 3-(6-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-4-oxobenzo[d][1,2,3]triazin-3(4H)-yl)piperidin-2,6-dione

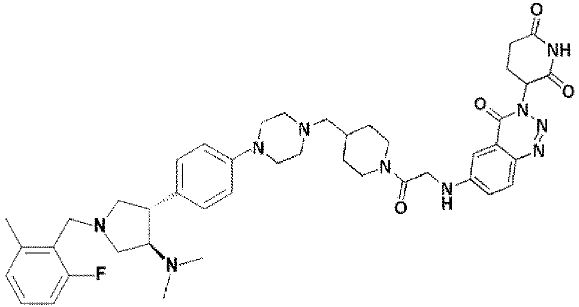

¹H NMR : (300 MHz, CDCl₃) δ 7.96-7.88 (m, 2H), 7.40 (dd, J = 9.0, 2.6 Hz, 1H), 7.28-7.16 (m, 4H), 7.02 (d, J = 7.6 Hz, 1H), 6.93 (m, 3H), 5.89 (m, 1H), 4.54 (d, J = 13.5 Hz, 1H), 4.24 (d, J = 16.8 Hz, 1H), 4.16 (d, J = 16.7 Hz, 1H), 4.02 (d, J = 13.6 Hz, 1H), 3.69 (d, J = 2.5 Hz, 2H), 3.19 (m, 7H), 3.01 (m, 3H), 2.94-2.84 (m, 2H), 2.81 (m, 1H), 2.75-2.69 (m, 1H), 2.63 (t, J = 5.1 Hz, 4H), 2.55-2.50 (m, 1H), 2.48 (s, 3H), 2.40-2.34 (m, 2H), 2.31 (s, 6H), 2.05 (m, 2H), 1.92 (m, 2H), 1.62 (m, 2H), 1.38 (m, 2H).

### <Example 70> Preparation of 3-(5-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-4-oxobenzo[d][1,2,3]triazin-3(4H)-yl)piperidin-2,6-dione

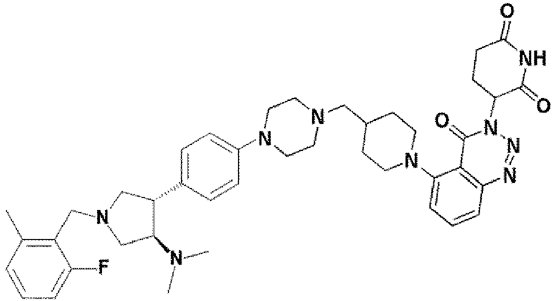

¹H NMR : (300 MHz, CDCl₃) δ 7.79 (t, J = 8.0 Hz, 1H), 7.66 (dd, J = 7.9, 1.0 Hz, 1H), 7.24 (m, 1H), 7.18 (d, J = 8.5 Hz, 2H), 7.10 (m, 1H), 6.95 (d, J = 7.5 Hz, 1H), 6.85 (m, 3H), 5.80 (dd, J = 11.1, 5.1 Hz, 1H), 4.88 (s, 1H), 3.63 (d, J = 2.3 Hz, 2H), 3.49 (s, 2H), 3.16 (m, 5H), 3.04-2.86 (m, 4H), 2.85-2.68 (m, 4H), 2.62-2.50 (m, 4H), 2.44 (s, 3H), 2.32 (d, J = 6.6 Hz, 2H), 2.27 (s, 6H), 2.03 (d, J = 5.9 Hz, 3H), 1.88 (m, 3H), 1.60 (t, J = 11.8 Hz, 2H).

### <Example 71> Preparation of 3-(6-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-4-oxobenzo[d][1,2,3]triazin-3(4H)-yl)piperidin-2,6-dione

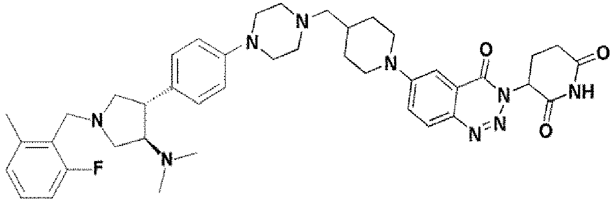

¹H NMR : (300 MHz, CDCl₃) δ7.98 (d, J = 9.1 Hz, 1H), 7.52 (d, J = 2.9 Hz, 1H), 7.44 (dd, J = 9.2, 2.9 Hz, 1H), 7.19 (d, J = 8.6 Hz, 2H), 7.12 (m, 1H), 6.95 (d, J = 7.5 Hz, 1H), 6.89-6.78 (m, 3H), 5.76 (m, 1H), 4.05 (d, J = 13.2 Hz, 2H), 3.63 (d, J = 2.3 Hz, 2H), 3.16 (m, 5H), 3.12-3.04 (m, 2H), 3.04-2.91 (m, 6H), 2.90-2.71 (m, 3H), 2.55 (m, 5H), 2.44 (s, 3H), 2.41-2.32 (m, 2H), 2.27 (s, 6H), 1.94 (m, 2H), 1.34 (m, 2H).

### <Example 72> Preparation of 3-(6-(2-(4-((4-(4-(5-(furan-2-ylmethylamino)-[1,2,4]triazolo[4,3-f]pyrimidin-8-yl)phenyl)piperazin-1-yl)methyl)piperidin-1-yl)-2-oxoethylamino)-4-oxobenzo[d][1,2,3]triazin-3(4H)-yl)piperidin-2,6-dione

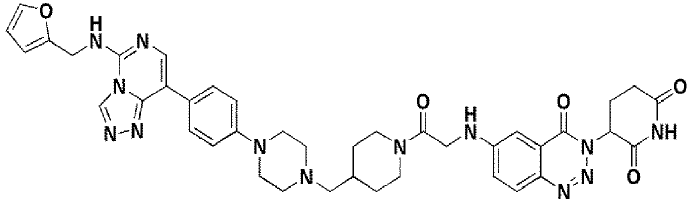

### Step 1: tert-butyl 4-(4-(4-(5-((furan-2-ylmethyl)amino)-[1.2.4]triazolo[4.3-c]pyrimidin-8-yl)phenyl)piperazine-1-carbonyl)piperidin-1-carboxylate

N-(furan-2-ylmethyl)-8-(4-(piperazin-1-yl)phenyl)-[1,2,4]triazolo[4,3-f]pyrimidin-5- in MeOH (3 mL) A solution of amine hydrochloride (30 mg, 0.080 mmol), tert-butyl-4-formylpiperidin-1-carboxylate (43 mg, 0.199 mmol) and AcOH (0.3 ml) was added and stirred at room temperature for 1 hour. NaBH3CN (20 mg, 0.319 mmol) was charged slowly and the reaction was stirred at room temperature for 1 h. After completion of the reaction, the reaction mass was distilled and the residue was quenched with 5% K2CO3 solution and extracted with DCM, dried over MgSO4 and the solvent removed in vacuo to give an oil. The crude mixture was purified by silica gel column chromatography using DCM / MeOH = 5 % to give the title compound (18 mg, 0.030 mmol, 38 %) as a yellow oil.

¹H NMR (500 MHz, CDCl₃) δ 8.31 (d, *J* = 1.5 Hz, 1H), 8.12 (d, *J* = 1.5 Hz, 1H), 7.88-7.81 (m, 2H), 7.41 (s, 1H), 7.08-6.99 (m, 2H), 6.44 (t, *J* = 5.7 Hz, 1H), 6.41-6.34 (m, 2H), 4.87 (d, *J* = 5.7 Hz, 2H), 4.19-4.02 (m, 2H), 3.33-3.19 (m, 4H), 2.71 (t, *J* = 12.4 Hz, 2H), 2.65-2.52 (m, 4H), 2.25 (d, *J* = 7.0 Hz, 2H), 1.82-1.73 (m, 3H), 1.46 (s, 9H), 1.17-1.05 (m, 2H).

### Step 2: Preparation of 8-(4-(4-((1-chloro-115-piperidin-4-yl)methyl)piperazin-1-yl)phenyl)-N-(furan-2-ylmethyl)-[1,2,4]triazolo[4,3-c]pyrimidin-5-amine

To a solution of the compound prepared in the previous step (82 mg, 0.143 mmol) in DCM (2 mL) was added 4 M HCl in dioxane (1 ml). The reaction mixture was stirred at room temperature for 1 hour. The reaction mixture was concentrated in vacuo to afford the desired compound (90 mg, crude) as a light brown solid.

### Step 3: Preparation of 3-(6-(2-(4-((4-(4-(5-(furan-2-ylmethylamino)-[1,2,4]triazolo[4,3-f]pyrimidin-8-yl)phenyl)piperazin-1-yl)methyl)piperidin-1-yl)-2-oxoethylamino)-4-oxobenzo[d][1,2,3]triazin-3(4H)-yl)piperidin-2,6-dione

2-(3-(2,6-dioxopiperidin-3-yl)-4-oxo-3,4-dihydrobenzo[d][1,2,3]triazin-6-ylamino)acetic acid (16 mg, 0.049) in DMF mmol), the compound prepared in the previous step (25 mg, 0.049 mmol), EDCI HCl (10 mg, 0.054 mmol), HOBt HO (8 mg, 0.054 mmol), and DIPEA (0.034 mL, 0.196 mmol) were added, Stirred at room temperature for 2 hours. The reaction mixture was diluted with water and extracted with EtOAc. The combined organic layers were washed with brine, dried over MgSO4 and the solvent removed in vacuo to give an oil. The crude mixture was purified on PREP TLC using MeOH / DCM = 5% to give the title compound (5 mg, 0.006 mmol, 13%) as a white solid.

¹H NMR (500 MHz, CDCl₃) δ 8.31 (s, 1H), 8.18-8.10 (m, 2H), 7.95 (d, *J* = 8.9 Hz, 1H), 7.84 (d, *J* = 8.3 Hz, 2H), 7.41 (s, 1H), 7.26-7.22 (m, 1H), 7.14 (s, 1H), 7.04 (d, *J* = 8.3 Hz, 2H), 6.45 (t, *J* = 5.8 Hz, 1H), 6.41-6.34 (m, 2H), 6.06-6.00 (m, 1H), 5.83-5.74 (m, 1H), 4.87 (d, *J* = 5.8 Hz, 2H), 4.70-4.62 (m, 1H), 4.05-3.96 (m, 2H), 3.85-3.76 (m, 1H), 3.33-3.23 (m, 4H), 3.11 (t, *J* = 12.7 Hz, 1H), 3.01-2.92 (m, 2H), 2.92-2.71 (m, 2H), 2.67-2.54 (m, 4H), 2.44-2.34 (m, 1H), 2.32-2.25 (m, 2H), 2.07-1.87 (m, 3H), 1.23-1.12 (m, 2H). LC/MS (ESI) *m*/*z* 786.1 [M+H]⁺, 784.1 [M-H]⁻

### <Example 73> Preparation of 2-(3-(2,6-dioxopiperidin-3-yl)-4-oxo-3,4-dihydrobenzo[d][1,2,3]triazin-6-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide

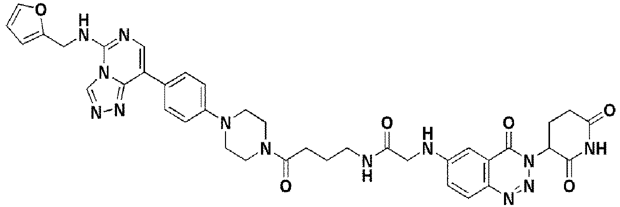

To 2-(3-(2,6-dioxopiperidin-3-yl)-4-oxo-3,4-dihydrobenzo[d][1,2,3]triazin-6-ylamino)acetic acid (17 mg, 0.051 mmol) in DMF, EDCI HCl (11 mg, 0.057mmol), HOBt (9 mg, 0.057 mmol), DIPEA (0.035mL, 0.204 mmol), and 4-amino-1-(4-(4-(5-((puran-2-ylmethyl)amino)-[1,2,4]triazolo[4,3-c]pyrimidin-8-yl)phenyl)piperazine-1-yl)butane-1-on (25mg, 0.051 mmol, Prepared in example 12 step 3). And it was stirred at a room temperature for 2 hours. After completion of the reaction, the reaction mixture was diluted with water and extracted with ethyl acetate.

The organic layer was washed with brine and dried on anhydrous Na2SO4. The solvent was then removed in vacuo to give an oil. Using purification by PREP TLC (MeOH/DCM = 5%), target chemical, 2-(3-(2,6-dioxopiperidin-3-yl)-4-oxo-3,4-dihydrobenzo[d][1,2,3]triazin-6-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide was obtained (17 mg, 0.022 mmol, 43% yield) as white solid.

¹H NMR (500 MHz, CDCl₃) δ 9.35 (s, 1H), 8.31 (s, 1H), 8.10 (s, 1H), 7.82 (d, *J* = 8.3 Hz, 3H), 7.52-7.42 (m, 1H), 7.15-7.06 (m, 2H), 6.97 (d, *J* = 8.3 Hz, 2H), 6.54 (t, *J* = 5.9 Hz, 1H), 6.40-6.32 (m, 2H), 5.88-5.73 (m, 2H), 4.86 (d, *J* = 5.7 Hz, 2H), 3.90-3.82 (m, 2H), 3.76-3.67 (m, 2H), 3.61-3.53 (m, 2H), 3.41-3.31 (m, 2H), 3.22-3.16 (m, 2H), 3.16-3.08 (m, 2H), 2.97-2.77 (m, 3H), 2.47-2.39 (m, 2H), 2.36-2.27 (m, 1H), 1.97-1.83 (m, 2H). LC/MS (ESI) *m*/*z* 775.1 [M+H]⁺, 773.0 [M-H]⁻

### <Example 74> Preparation of 1-(3-(2,6-dioxopiperidin-3-yl)-4-oxo-3,4-dihydrobenzo[d][1,2,3]triazin-6-yl)-N-(4-(4-(4-(5-(furan-2-ylmethylamino)-[1,2,4]triazolo[4,3-f]pyrimidin-8-yl)phenyl)piperazin-1-yl)-4-oxobutyl)piperidin-4-carboxamide

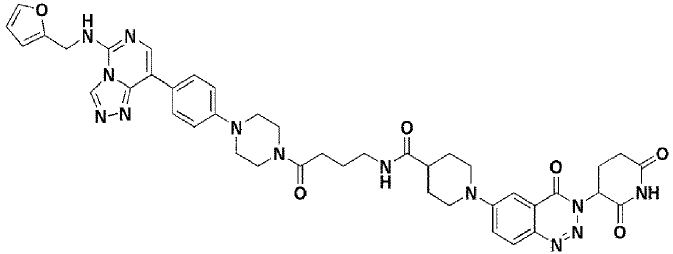

To 3-(6-(4-carboxypiperidin-1-yl)-4-oxobenzo[d][1,2,3]triazin-3(4H)-yl)piperidin-2,6-dione (19 mg, 0.051 mmol) in DMF, EDCI HCl (11 mg, 0.057mmol), HOBt (9 mg, 0.057 mmol), DIPEA (0.035mL, 0.204 mmol), and 4-amino-1-(4-(4-(5-((puran-2-ylmethyl)amino)-[1,2,4]triazolo[4,3-c]pyrimidin-8-yl)phenyl)piperazine-1-yl)butane-1-on (25mg, 0.051 mmol, Prepared in example 12 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5%), target chemical, 1-(3-(2,6-dioxopiperidin-3-yl)-4-oxo-3,4-dihydrobenzo[d][1,2,3]triazin-6-yl)-N-(4-(4-(4-(5-(furan-2-ylmethylamino)-[1,2,4]triazolo[4,3-f]pyrimidin-8-yl)phenyl)piperazin-1-yl)-4-oxobutyl)piperidin-4-carboxamide was obtained (15 mg, 0.018 mmol, 35% yield) as white solid.

¹H NMR (500 MHz, CDCl₃) δ 8.67 (s, 1H), 8.32 (s, 1H), 8.13 (s, 1H), 7.97 (d, *J* = 9.1 Hz, 1H), 7.90-7.84 (m, 2H), 7.49 (d, *J* = 2.9 Hz, 1H), 7.44-7.38 (m, 2H), 7.06-6.99 (m, 2H), 6.72 (t, *J* = 5.2 Hz, 1H), 6.51 (t, *J* = 5.8 Hz, 1H), 6.40-6.33 (m, 2H), 5.82-5.75 (m, 1H), 4.88 (d, *J* = 5.7 Hz, 2H), 4.06-3.96 (m, 2H), 3.83-3.77 (m, 2H), 3.69-3.61 (m, 2H), 3.33 (q, *J* = 5.9 Hz, 2H), 3.28-3.23 (m, 2H), 3.23-3.17 (m, 2H), 3.03-2.78 (m, 5H), 2.48 (t, *J* = 6.4 Hz, 2H), 2.40-2.32 (m, 2H), 2.00-1.89 (m, 3H), 1.86-1.76 (m, 2H). LC/MS (ESI) *m*/*z* 828.1 [M+H]⁺, 826.0 [M-H]⁻

### <Example 75> Preparation of 3-(5-(6-(4-(4-(5-(furan-2-ylmethylamino)-[1,2,4]triazolo[4,3-f]pyrimidin-8-yl)phenyl)piperazin-1-yl)-6-oxohexylamino)-4-oxobenzo[d][1,2,3]triazin-3(4H)-yl)piperidin-2,6-dione

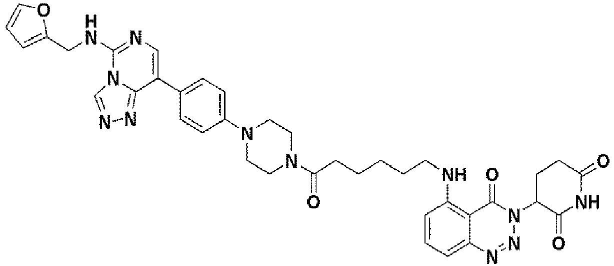

### Step 1: Preparation of tert-butyl 6-aminohexanoate

A solution of 6-aminohexanoic acid (1 g, 7.62 mmol) in thionyl chloride (76.2 ml, 76.2 mmol) was stirred at room temperature for 1.5 h, after which the excess thionyl chloride was removed in vacuo. A solution of NaHCO3 (1.40 g, 16.7 mmol) in t-BuOH (10 ml) was added to the residue under stirring and the reaction mixture was stirred overnight at room temperature. The volatiles were removed in vacuo and the residue was dissolved in EtOAc (30 ml) and NaOH (1M, aq) (20 ml). The combined organic layers were washed with water (30 ml) and brine (20 ml × 3), dried over MgSO 4 and the solvent removed in vacuo to give the title compound (521 mg, 6.94 mmol, 91 %) as a yellow clear oil.

¹H NMR (300 MHz, CDCl₃) δ2.78 (t, *J* = 7.3 Hz, 2H), 2.21 (t, *J* = 7.4 Hz, 2H), 1.70-1.49 (m, 4H), 1.44 (s, 9H), 1.42-1.30 (m, 2H).

### Step 2: Preparation of tert-butyl 6-((3-(2,6-dioxopiperidin-3-yl)-4-oxo-3,4-dihydrobenzo[d][1,2,3]triazin-5-yl)amino)hexanoate

3-(5-fluoro-4-oxobenzo[d][1,2,3]triazin-3(4H)-yl)piperidin- to a solution of the compound prepared in step 1 (100 mg, 0.534 mmol) in DMF 2,6-dione (98 mg, 0.356 mmol) and DIPEA (0.093 mL, 0.534 mmol) were added, and the mixture was stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc/Hx = 26 % to give the title compound (89 mg, 0.200 mmol, 56 %) as a yellow solid.

¹H NMR (300 MHz, CDCl₃) δ 8.15 (t, *J* = 4.8 Hz, 1H), 8.01 (s, 1H), 7.69 (t, *J* = 8.1 Hz, 1H), 7.26-7.23 (m, 1H), 6.78 (d, *J* = 8.5 Hz, 1H), 5.73-5.62 (m, 1H), 3.21 (q, *J* = 6.7 Hz, 2H), 3.02-2.74 (m, 3H), 2.42-2.32 (m, 1H), 2.24 (t, *J* = 7.4 Hz, 2H), 1.80-1.59 (m, 4H), 1.59-1.45 (m, 2H), 1.44 (s, 9H).

### Step 3: Preparation of 6-((3-(2,6-dioxopiperidin-3-yl)-4-oxo-3,4-dihydrobenzo[d][1,2,3]triazin-5-yl)amino)hexanoate.

To a solution of the compound prepared in step 1 above (85 mg, 0.191 mmol) in DCM (2 ml) was added TFA (2 ml), and the resulting mixture was stirred at room temperature for 1 hour. The reaction mixture was concentrated in vacuo to give the title compound (92 mg, crude) as a brown oil.

### Step 4: Preparation of 3-(5-(6-(4-(4-(5-(furan-2-ylmethylamino)-[1,2,4]triazolo[4,3-f]pyrimidin-8-yl)phenyl)piperazin-1-yl)-6-oxohexylamino)-4-oxobenzo[d][1,2,3]triazin-3(4H)-yl)piperidin-2,6-dione

N-(furan-2-ylmethyl)-8-(4-(piperazin-1-yl)phenyl)-[1,2,4]triazolo[4,3-f]pyrimidin-5-amine (26 mg) in DMF , 0.063 mmol), the compound prepared in step 3 above (24 mg, 0.063 mmol), EDCI HCl (13 mg, 0.069 mmol), HOBt HO (11 mg, 0.069 mmol), DIPEA (0.044 mL, 0.252 mmol) ) and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 3 % to give the title compound (5 mg, 0.006 mmol, 10 %) as a yellow solid.

¹H NMR (500 MHz, CDCl₃) δ 8.43 (s, 1H), 8.31 (s, 1H), 8.16 (t, *J* = 5.2 Hz, 1H), 8.12 (s, 1H), 7.89-7.83 (m, 2H), 7.69 (t, *J* = 8.1 Hz, 1H), 7.43-7.38 (m, 1H), 7.26-7.23 (m, 1H), 7.09-7.01 (m, 2H), 6.78 (d, *J* = 8.5 Hz, 1H), 6.47 (t, *J* = 5.8 Hz, 1H), 6.40-6.33 (m, 2H), 5.68-5.59 (m, 1H), 4.88 (d, *J* = 5.5 Hz, 2H), 3.92-3.84 (m, 1H), 3.80-3.72 (m, 1H), 3.72-3.60 (m, 2H), 3.32-3.16 (m, 6H), 3.01-2.74 (m, 3H), 2.47-2.39 (m, 2H), 2.39-2.30 (m, 1H), 1.81-1.72 (m, 4H), 1.58-1.48 (m, 2H).

### <Example 76> Preparation of 3-(6-(6-(4-(4-(5-(furan-2-ylmethylamino)-[1,2,4]triazolo[4,3-f]pyrimidin-8-yl)phenyl)piperazin-1-yl)-6-oxohexylamino)-4-oxobenzo[d][1,2,3]triazin-3(4H)-yl)piperidin-2,6-dione

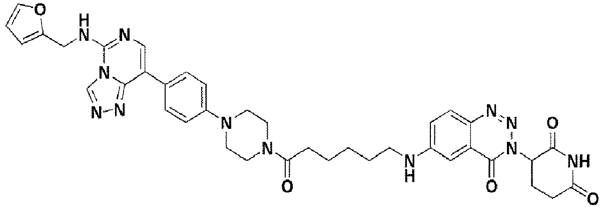

### Step 1: Preparation of tert-butyl 6-((3-(2,6-dioxopiperidin-3-yl)-4-oxo-3,4-dihydrobenzo[d][1,2,3]triazin-6-yl)amino)hexanoate

3-(5-fluoro-4-oxobenzo[d][1,2,3]triazin-3(4H)- to a solution of the compound (100 mg, 0.534 mmol) prepared in step 1 of Example 75 in DMSO yl)piperidin-2,6-dione (122 mg, 0.445 mmol) was added, DIPEA (0.116 mL, 0.667 mmol) was added, and the mixture was stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc / Hx = 55 % to give the title compound (106 mg, 0.239 mmol, 53 %) as a white solid.

¹H NMR (300 MHz, CDCl₃) δ 8.54 (s, 1H), 7.84 (d, *J* = 8.8 Hz, 1H), 7.16-7.08 (m, 1H), 7.08-6.98 (m, 1H), 5.86-5.72 (m, 1H), 4.88 (t, *J* = 5.3 Hz, 1H), 3.21 (q, *J* = 6.7 Hz, 2H), 3.04-2.75 (m, 3H), 2.44-2.32 (m, 1H), 2.25 (t, *J* = 7.2 Hz, 2H), 1.74-1.57 (m, 4H), 1.51-1.37 (m, 11H).

### Step 2: Preparation of 6-((3-(2,6-dioxopiperidin-3-yl)-4-oxo-3,4-dihydrobenzo[d][1,2,3]triazin-6-yl)amino)hexanoate.

To a solution of the compound prepared in the previous step (100 mg, 0.225 mmol) in DCM (2 ml) was added TFA (2 ml), and the resulting mixture was stirred at room temperature for 3 hours. The reaction mixture was concentrated in vacuo to give the desired compound (102 mg, crude) as a yellow oil.

### Step 3: Preparation of 3-(6-(6-(4-(4-(5-(furan-2-ylmethylamino)-[1,2,4]triazolo[4,3-f]pyrimidin-8-yl)phenyl)piperazin-1-yl)-6-oxohexylamino)-4-oxobenzo[d][1,2,3]triazin-3(4H)-yl)piperidin-2,6-dione

N-(furan-2-ylmethyl)-8-(4-(piperazin-1-yl)phenyl)-[1,2,4]triazolo[4,3-f]pyrimidin-5-amine hydrochloride in DMF (20 mg, 0.049 mmol), the compound prepared in the previous step (18 mg, 0.049 mmol), EDCl HCl (10 mg, 0.054 mmol), HOBt HO (8 mg, 0.054 mmol), DIPEA (0.034 mL, 0.196 mmol) were added. and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH / DCM = 5 % to give the title compound (6 mg, 0.008 mmol, 16 %) as a white solid.

¹H NMR (500 MHz, CDCl₃) δ8.40 (s, 1H), 8.32 (d, *J* = 1.6 Hz, 1H), 8.12 (d, *J* = 1.6 Hz, 1H), 7.90-7.83 (m, 3H), 7.41 (s, 1H), 7.20-7.15 (m, 1H), 7.12-7.06 (m, 1H), 7.06-7.01 (m, 2H), 6.49 (t, *J* = 5.7 Hz, 1H), 6.41-6.34 (m, 2H), 5.80-5.71 (m, 1H), 5.01 (t, *J* = 5.3 Hz, 1H), 4.88 (d, *J* = 5.7 Hz, 2H), 3.87-3.78 (m, 2H), 3.70-3.63 (m, 2H), 3.29 (q, *J* = 6.5 Hz, 2H), 3.27-3.19 (m, 4H), 3.01-2.77 (m, 3H), 2.43 (t, *J* = 7.2 Hz, 2H), 2.40-2.33 (m, 1H), 1.78-1.69 (m, 4H), 1.54-1.45 (m, 2H). LC/MS (ESI) *m*/*z* 745.1 [M+H]⁺, [M-H]

### <Example 77> Preparation of 3-(5-(8-(4-(4-(5-(furan-2-ylmethylamino)-[1,2,4]triazolo[4,3-f]pyrimidin-8-yl)phenyl)piperazin-1-yl)-8-oxooctylamino)-4-oxobenzo[d][1,2,3]triazin-3(4H)-yl)piperidin-2,6-dione

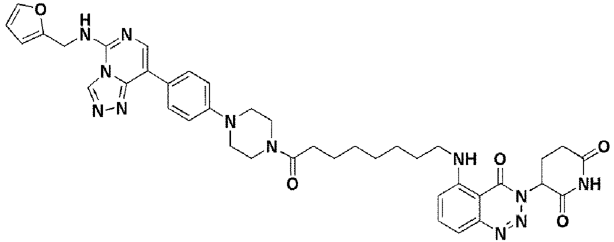

### Step 1: Preparation of tert-butyl 8-((3-(2,6-dioxopiperidin-3-yl)-4-oxo-3,4-dihydrobenzo[d][1,2,3]triazin-5-yl)amino)octanoate

3-(5-fluoro-4-oxobenzo[d][1,2,3]triazin-3(4H)-yl)piperidin- in a solution of tert-butyl 8-aminooctanoate (117 mg, 0.543 mmol) in DMSO 2,6-dione (100 mg, 0.362 mmol) and DIPEA (0.094 mL, 0.543 mmol) were added and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc / Hx = 29 % to give the title compound (150 mg, 0.318 mmol, 87 %) as a yellow solid.

¹H NMR (300 MHz, CDCl₃) δ 8.62 (s, 1H), 8.16 (t, *J* = 5.2 Hz, 1H), 7.67 (t, *J* = 8.1 Hz, 1H), 7.27-7.19 (m, 1H), 6.77 (d, *J* = 8.5 Hz, 1H), 5.78-5.64 (m, 1H), 3.26-3.12 (m, 2H), 3.01-2.77 (m, 3H), 2.41-2.29 (m, 1H), 2.21 (t, *J* = 7.5 Hz, 2H), 1.76-1.64 (m, 2H), 1.64-1.52 (m, 2H), 1.44 (s, 9H), 1.43-1.30 (m, 6H).

### Step 2: Preparation 8-((3-(2,6-dioxopiperidin-3-yl)-4-oxo-3,4-dihydrobenzo[d][1,2,3]triazin-5-yl)amino)octanoate

To a solution of the compound prepared in the previous step (150 mg, 0.318 mmol) in DCM (2 ml) was added TFA (2 ml), and the resulting mixture was stirred at room temperature for 1 hour. The reaction mixture was concentrated in vacuo to give an oil. The crude mixture was purified by silica gel column chromatography using EtOAc / Hex = 50 % to give the title compound (55 mg, 0.132 mmol, 41 %) as a yellow solid.

¹H NMR (300 MHz, CDCl₃) δ 8.99 (s, 1H), 8.15 (s, 1H), 7.67 (t, *J* = 8.2 Hz, 1H), 7.36-7.18 (m, 2H), 6.77 (d, *J* = 8.5 Hz, 1H), 5.85-5.57 (m, 1H), 3.31-3.13 (m, 2H), 3.02-2.77 (m, 3H), 2.44-2.25 (m, 3H), 1.78-1.52 (m, 4H), 1.52-1.32 (m, 6H).

### Step 3: Preparation of 3-(5-(8-(4-(4-(5-(furan-2-ylmethylamino)-[1,2,4]triazolo[4,3-f]primidin-8-yl)phenyl)piperazin-1-yl)-8-oxooctylamino)-4-oxobenzo[d][1,2,3]triazin-3(4H)-yl)piperidin-2,6-dione

N-(furan-2-ylmethyl)-8-(4-(piperazin-1-yl)phenyl)-[1,2,4]triazolo[4,3-f]pyrimidin-5-amine hydrochloride in DMF (25 mg, 0.060 mmol) in a solution of the compound prepared in the previous step (25 mg, 0.060 mmol), EDCI HCl (12 mg, 0.066 mmol), HOBt HO (10 mg, 0.066 mmol), DIPEA (0.042 mL, 0.240 mmol) was added and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified on PREP TLC using MeOH / DCM = 5 % to give the title compound (14 mg, 0.018 mmol, 30 %) as a yellow solid.

¹H NMR (500 MHz, CDCl₃) δ 8.56 (s, 1H), 8.31 (d, *J* = 1.5 Hz, 1H), 8.21-8.15 (m, 1H), 8.12 (d, *J* = 1.5 Hz, 1H), 7.89-7.82 (m, 2H), 7.68 (t, *J* = 8.1 Hz, 1H), 7.41 (s, 1H), 7.24 (d, *J* = 7.7 Hz, 1H), 7.07-7.02 (m, 2H), 6.78 (d, *J* = 8.5 Hz, 1H), 6.50 (t, *J* = 5.9 Hz, 1H), 6.40-6.34 (m, 2H), 5.69-5.60 (m, 1H), 4.87 (d, *J* = 5.8 Hz, 2H), 3.88-3.73 (m, 2H), 3.70-3.60 (m, 2H), 3.31-3.15 (m, 6H), 3.01-2.73 (m, 3H), 2.44-2.31 (m, 3H), 1.81-1.61 (m, 4H), 1.51-1.34 (m, 6H).

### <Example 78> Preparation of 3-(6-(8-(4-(4-(5-(furan-2-ylmethylamino)-[1,2,4]triazolo[4,3-f]pyrimidin-8-yl)phenyl)piperazin-1-yl)-8-oxooctylamino)-4-oxobenzo[d][1,2,3]triazin-3(4H)-yl)piperidin-2,6-dione

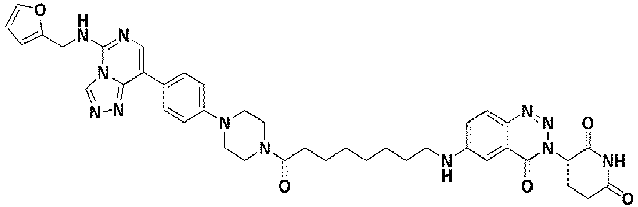

### Step 1: Preparation of tert-butyl 8-((3-(2,6-dioxopiperidin-3-yl)-4-oxo-3,4-dihydrobenzo[d][1,2,3]triazin-6-yl)amino)octanoate.

3-(5-fluoro-4-oxobenzo[d][1,2,3]triazin-3(4H)-yl)piperidin in a solution of all tert-butyl 8-aminooctanoate (117 mg, 0.543 mmol) in DMSO -2,6-dione (100 mg, 0.362 mmol) and DIPEA (0.094 mL, 0.543 mmol) were added and stirred at 90 °C overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc / Hx = 35 % to give the title compound (123 mg, 0.260 mmol, 72 %) as a white solid.

¹H NMR (300 MHz, CDCl₃) δ 8.09 (s, 1H), 7.91 (d, *J* = 8.7 Hz, 1H), 7.20 (d, *J* = 2.6 Hz, 1H), 7.08 (dd, *J* = 8.7, 2.6 Hz, 1H), 5.82-5.71 (m, 1H), 4.62 (t, *J* = 5.4 Hz, 1H), 3.25 (q, *J* = 6.7 Hz, 2H), 3.03-2.74 (m, 3H), 2.45-2.32 (m, 1H), 2.21 (t, *J* = 7.4 Hz, 2H), 1.74-1.63 (m, 2H), 1.63-1.53 (m, 2H), 1.44 (s, 9H), 1.43-1.29 (m, 6H).

### Step 2: Preparation of 8-((3-(2,6-dioxopiperidin-3-yl)-4-oxo-3,4-dihydrobenzo[d][1,2,3]triazin-5-yl)amino)octanoic acid

To a solution of the compound prepared in the previous step (123 mg, 0.260 mmol) in DCM (2 ml) was added TFA (2 ml), and the resulting mixture was stirred at room temperature for 1 hour. The reaction mixture was concentrated in vacuo to give the title compound (123 mg, crude) as a yellow oil.

### Step 3: Preparation of 3-(6-(8-(4-(4-(5-(furan-2-ylmethylamino)-[1,2,4]triazolo[4,3-f)pyrimidin-8-yl)phenyl)piperazin-1-yl)-8-oxooctylamino)-4-oxobenzo[d1][1,2,3]triazin-3(4H)-yl)piperidin-2,6-dione

N-(furan-2-ylmethyl)-8-(4-(piperazin-1-yl)phenyl)-[1,2,4]triazolo[4,3-f]pyrimidin-5-amine hydrochloride in DMF (25 mg, 0.060 mmol), the compound prepared in the previous step (25 mg, 0.060 mmol), EDCI HCl (12 mg, 0.066 mmol), HOBt HO (10 mg, 0.066 mmol), DIPEA (0.042 mL, 0.240 mmol) were added. and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified on PREP TLC using MeOH / DCM = 5% to give the title compound (3 mg, 0.004 mmol, 6%) as a yellow solid.

¹H NMR (500 MHz, CDCl₃) δ 8.31 (d, *J* = 1.6 Hz, 1H), 8.19 (s, 1H), 8.12 (d, *J* = 1.6 Hz, 1H), 7.91 (d, *J* = 8.9 Hz, 1H), 7.88-7.83 (m, 2H), 7.41 (s, 1H), 7.20 (s, 1H), 7.12-7.07 (m, 1H), 7.07-7.00 (m, 2H), 6.47 (t, *J* = 5.9 Hz, 1H), 6.41-6.34 (m, 2H), 5.79-5.71 (m, 1H), 4.88 (d, *J* = 5.8 Hz, 2H), 4.70 (t, *J* = 5.9 Hz, 1H), 3.85-3.77 (m, 2H), 3.70-3.61 (m, 2H), 3.30-3.19 (m, 6H), 3.02-2.76 (m, 3H), 2.42-2.33 (m, 3H), 1.74-1.65 (m, 4H), 1.50-1.35 (m, 6H) ; LC/MS (ESI) *m*/*z* 773.1 [M+H]+, 771.1 [M-H]⁻

### <Example 79> Preparation of 2-(3-(2,6-dioxopiperidin-3-yl)-4-oxo-3,4-dihydrobenzo[d][1,2,3]triazin-6-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide

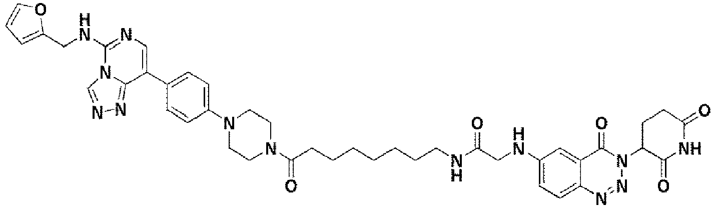

To 2-(3-(2,6-dioxopiperidin-3-yl)-4-oxo-3,4-dihydrobenzo[d][1,2,3]triazin-6-ylamino)acetic acid (17 mg, 0.051 mmol) in DMF, EDCI HCl (11 mg, 0.057mmol), HOBt (9 mg, 0.057 mmol), DIPEA (0.035mL, 0.204 mmol), and 8-amino-1-(4-(4-(5-((puran-2-ylmethyl)amino-[1,2,4]triazolo[4,3-c]pyrimidin-8-yl)phenyl)piperazine-1-yl)octane-1-on (28mg, 0.051 mmol, Prepared in example 16 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5%), target chemical, 2-(3-(2,6-dioxopiperidin-3-yl)-4-oxo-3,4-dihydrobenzo[d][1,2,3]triazin-6-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide was obtained (17 mg, 0.022 mmol, 43% yield) as white solid.

¹H NMR (500 MHz, CDCl₃) δ 9.03 (s, 1H), 8.32 (d, *J* = 1.5 Hz, 1H), 8.11 (d, *J* = 1.5 Hz, 1H), 7.89 (d, *J* = 8.7 Hz, 1H), 7.87-7.81 (m, 2H), 7.40 (s, 1H), 7.20-7.12 (m, 2H), 7.06-7.00 (m, 2H), 6.63 (t, *J* = 5.9 Hz, 1H), 6.52 (t, *J* = 6.0 Hz, 1H), 6.41-6.34 (m, 2H), 5.82 (t, *J* = 5.5 Hz, 1H), 5.81-5.72 (m, 1H), 4.87 (d, *J* = 5.8 Hz, 2H), 3.94-3.86 (m, 2H), 3.84-3.74 (m, 2H), 3.69-3.59 (m, 2H), 3.34-3.15 (m, 6H), 2.99-2.78 (m, 3H), 2.43-2.29 (m, 3H), 1.67-1.55 (m, 2H), 1.55-1.44 (m, 2H), 1.38-1.27 (m, 6H). LC/MS (ESI) *m*/*z* 830.1 [M+H]⁺, 828.1 [M-H]⁻

### <Example 80> Preparation of 1-(3-(2,6-dioxopiperidin-3-yl)-4-oxo-3,4-dihydrobenzo[d][1,2,3]triazin-6-yl)-N-(8-(4-(4-(5-(furan-2-ylmethylamino)-[1,2,4]triazolo[4,3-f]pyrimidin-8-yl)phenyl)piperazin-1-yl)-8-oxooctyl)piperidin-4-carboxamide

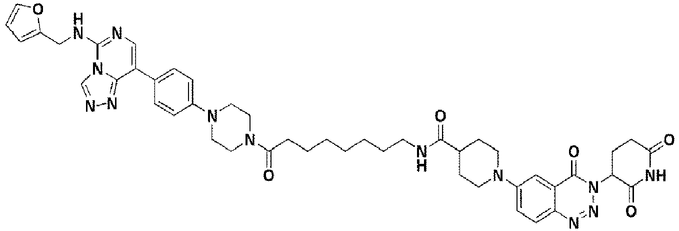

To 3-(6-(4-carboxypiperidin-1-yl)-4-oxobenzo[d][1,2,3]triazin-3(4h)-yl)piperidin-2,6-dione (19mg, 0.051 mmol) in DMF, EDCI HCl (11 mg, 0.057mmol), HOBt (9 mg, 0.057 mmol), DIPEA (0.035mL, 0.204 mmol), and 8-amino-1-(4-(4-(5-((puran-2-ylmethyl)amino-[1,2,4]triazolo[4,3-c]pyrimidin-8-yl)phenyl)piperazine-1-yl)octane-1-on (28mg, 0.051 mmol, Prepared in example 16 step 3). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5%), target chemical, 1-(3-(2,6-dioxopiperidin-3-yl)-4-oxo-3,4-dihydrobenzo[d][1,2,3]triazin-6-yl)-N-(8-(4-(4-(5-(furan-2-ylmethylamino)-[1,2,4]triazolo[4,3-f]pyrimidin-8-yl)phenyl)piperazin-1-yl)-8-oxooctyl)piperidin-4-carboxamide was obtained (23 mg, 0.026 mmol, 51% yield) as ivory color solid.

¹H NMR (500 MHz, CDCl₃) δ 8.39 (s, 1H), 8.31 (d, *J* = 1.6 Hz, 1H), 8.12 (d, *J* = 1.6 Hz, 1H), 7.98 (dd, *J* = 9.1, 1.6 Hz, 1H), 7.90-7.83 (m, 2H), 7.55-7.49 (m, 1H), 7.46-7.41 (m, 1H), 7.40 (s, 1H), 7.08-7.00 (m, 2H), 6.52 (t, *J* = 6.0 Hz, 1H), 6.40-6.34 (m, 2H), 5.82-5.74 (m, 1H), 5.65 (t, *J* = 5.5 Hz, 1H), 4.87 (d, *J* = 5.7 Hz, 2H), 4.12-4.00 (m, 2H), 3.84-3.75 (m, 2H), 3.70-3.61 (m, 2H), 3.30-3.19 (m, 6H), 3.10-3.01 (m, 2H), 3.01-2.77 (m, 3H), 2.43-2.31 (m, 4H), 2.01-1.93 (m, 2H), 1.91-1.80 (m, 2H), 1.69-1.61 (m, 2H), 1.55-1.47 (m, 2H), 1.42-1.30 (m, 6H). LC/MS (ESI) *m*/*z* 884.2 [M+H]⁺, 882.0 [M-H]⁻

### <Example 81> Preparation of 3-(5-(10-(4-(4-(5-(furan-2-ylmethylamino)-[1,2,4]triazolo[4,3-f]pyrimidin-8-yl)phenyl)piperazin-1-yl)-10-oxodecylamino)-4-oxobenzo[d][1,2,3]triazin-3(4H)-yl)piperidin-2,6-dione

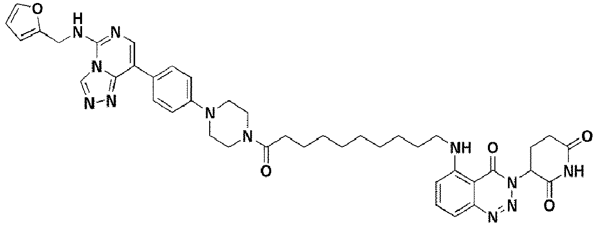

### Step 1: Preparation of tert-butyl 10-((3-(2,6-dioxopiperidin-3-yl)-4-oxo-3,4-dihydrobenzo[d][1,2,3]triazin-5-yl)amino)decanoate.

In a solution of tert-butyl 10-aminodecanoate (100 mg, 0.410 mmol) dissolved in DMF, 3-(5-fluoro-4-oxobenzo[d][1,2,3]triazin-3(4H) -yl)piperidin-2,6-dione (76 mg, 0.273 mmol) and DIPEA (0.071 mL, 0.388 mmol) were added, and the mixture was stirred at 90 °C for one day. The reaction mixture was diluted with water and extracted with EtOAc. The collected organic layer was washed with brine, dried over MgSO4, and the solvent was removed under vacuum to obtain an oil. The crude mixture was purified through silica gel column chromatography using EtOAc/Hx = 30% to prepare the title compound (77 mg, 0.154 mmol, 56%).

¹H NMR (300 MHz, CDCl₃) δ 8.15 (t, *J* = 4.9 Hz, 1H), 8.06 (s, 1H), 7.69 (t, *J* = 8.1 Hz, 1H), 7.26-7.22 (m, 1H), 6.78 (d, *J* = 8.5 Hz, 1H), 5.74-5.63 (m, 1H), 3.19 (q, *J* = 6.5 Hz, 2H), 3.03-2.75 (m, 3H), 2.42-2.31 (m, 1H), 2.20 (t, *J* = 7.5 Hz, 2H), 1.77-1.64 (m, 2H), 1.63-1.49 (m, 4H), 1.44 (s, 9H), 1.41-1.27 (m, 8H).

### Step 2: Preparation of 10-((3-(2,6-dioxopiperidin-3-yl)-4-oxo-3,4-dihydrobenzo[d][1,2,3]triazin-5-yl)amino)decanoate.

To a solution of the compound prepared in the previous step (77 mg, 0.191 mmol) dissolved in DCM (2 ml), TFA (2 ml) was added, and the mixture was stirred at room temperature for 1 h. The reaction mixture was concentrated in vacuo to give the title compound (80 mg, crude) in the form of a brown oil.

### Step 3: Preparation of 3-(5-(10-(4-(4-(5-(furan-2-ylmethylamino)-[1,2,4]triazolo[4,3-f]pyrimidin-8-yl)phenyl)piperazin-1-yl)-10-oxodecylamino)-4-oxobenzo[d][1,2,3]triazin-3(4H)-yl)piperidin-2,6-dione

N-(furan-2-ylmethyl)-8-(4-(piperazin-1-yl)phenyl)-[1,2,4]triazolo[4,3-f]pyrimidin-5-amine hydrochloride dissolved in DMF In a solution of (26 mg, 0.063 mmol), the compound prepared in the previous step (28 mg, 0.063 mmol), EDCI HCl (13 mg, 0.069 mmol), HOBt HO (11 mg, 0.069 mmol), DIPEA (0.044 mL, 0.252) mmol) and stirred at room temperature for 2 h. The reaction mixture was diluted with water and extracted with EtOAc. The collected organic layer was washed with brine, dried over MgSO4, and the solvent was removed under vacuum to obtain an oil. The crude mixture was purified through silica gel column chromatography using MeOH/DCM = 3% to prepare the title compound (3 mg, 0.003 mmol, 6%) as a yellow solid.

¹H NMR (500 MHz, CDCl₃) δ 8.40 (s, 1H), 8.31 (s, 1H), 8.16 (t, *J* = 5.1 Hz, 1H), 8.12 (s, 1H), 7.89-7.82 (m, 2H), 7.68 (t, *J* = 8.1 Hz, 1H), 7.43-7.39 (m, 1H), 7.24 (d, *J* = 7.7 Hz, 1H), 7.08-7.01 (m, 2H), 6.77 (d, *J* = 8.5 Hz, 1H), 6.46 (t, *J* = 5.8 Hz, 1H), 6.41-6.34 (m, 2H), 5.69-5.63 (m, 1H), 4.88 (d, *J* = 5.5 Hz, 2H), 3.81 (t, *J* = 5.3 Hz, 2H), 3.65 (t, *J* = 5.2 Hz, 2H), 3.28-3.16 (m, 6H), 2.99-2.76 (m, 3H), 2.42-2.36 (m, 2H), 2.36-2.31 (m, 1H), 1.73-1.63 (m, 4H), 1.48-1.40 (m, 2H), 1.40-1.31 (m, 8H).

### <Example 82> Preparation of 3-(6-(10-(4-(4-(5-(furan-2-ylmethylamino)-[1,2,4]triazolo[4,3-f]pyrimidin-8-yl)phenyl)piperazin-1-yl)-10-oxodecylamino)-4-oxobenzo[d] [1,2,3]triazin-3(4H)-yl)piperidin-2,6-dione

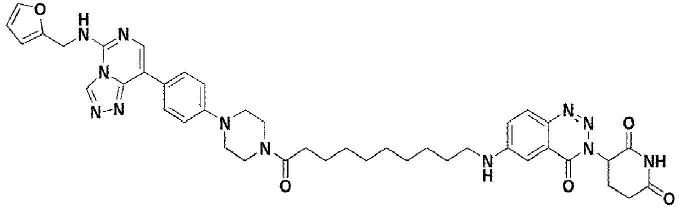

To N-(furan-2-ylmethyl)-8-(4-(piperazin-1-yl)phenyl)-[1,2,4]triazolo[4,3-f]pirimidin-5-amine hydrochloride (20mg, 0.048 mmol) in DMF, HATU (27 mg, 0.073 mmol), DIPEA (0.033mL, 0.192 mmol), and 10-amino-1-(4-(4-(5-((furan-2-ylmethyl)amino-[1,2,4]triazolo[4,3-c]pirimidin-8-yl)phenyl)piperazin-1-yl)decane-1-on (21mg, 0.048 mmol, Prepared in example 18 step 4). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5%), target chemical, 3-(6-(10-(4-(4-(5-(furan-2-ylmethylamino)-[1,2,4]triazolo[4,3-f]pyrimidin-8-yl)phenyl)piperazin-1-yl)-10-oxodecylamino)-4-oxobenzo[d][1,2,3]triazin-3(4H)-yl)piperidin-2,6-dione was obtained (10 mg, 0.012 mmol, 26% yield) as yellow solid.

¹H NMR (500 MHz, CDCl₃) δ 8.32 (s, 1H), 8.31 (s, 1H), 8.12 (s, 1H), 7.89 (d, *J* = 8.9 Hz, 1H), 7.88-7.83 (m, 2H), 7.43-7.39 (m, 1H), 7.19 (d, *J* = 2.6 Hz, 1H), 7.08 (dd, *J* = 8.9, 2.7 Hz, 1H), 7.06-7.01 (m, 2H), 6.50 (t, *J* = 5.8 Hz, 1H), 6.40-6.34 (m, 2H), 5.79-5.73 (m, 1H), 4.87 (d, *J* = 5.7 Hz, 2H), 4.72 (t, *J* = 5.4 Hz, 1H), 3.85-3.76 (m, 2H), 3.70-3.61 (m, 2H), 3.30-3.18 (m, 6H), 3.01-2.77 (m, 3H), 2.43-2.33 (m, 3H), 1.66 (p, *J* = 7.3 Hz, 6H), 1.45-1.30 (m, 10H). LC/MS (ESI) *m*/*z* 801.2 [M+H]⁺, 799.0 [M-H]⁻

### <Example 83> Preparation of 2-(3-(2,6-dioxopiperidin-3-yl)-4-oxo-3,4-dihydrobenzo[d][1,2,3]triazin-6-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide

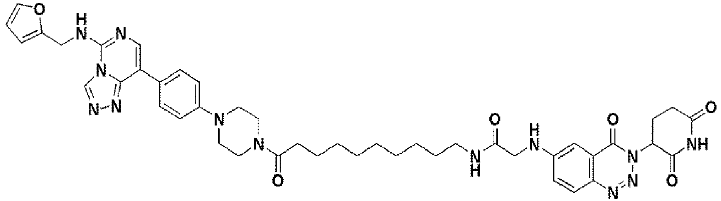

To 2-(3-(2,6-dioxopiperidin-3-yl)-4-oxo-3,4-dihydrobenzo[d][1,2,3]triazin-6-ylamino)acetic acid (11mg, 0.034 mmol) in DMF, EDCl HCl(7mg, 0.038 mmol), HOBt (6 mg, 0.038 mmol), DIPEA (0.024mL, 0.137 mmol), and 10-amino-1-(4-(4-(5-((furan-2-ylmethyl)amino-[1,2,4]triazolo[4,3-c]pirimidin-8-yl)phenyl)piperazin-1-yl)decane-1-on (20mg, 0.034 mmol, Prepared in example 18 step 4). And it was stirred at a room temperature for 2 hours. After completion of the reaction, the reaction mixture was diluted with water and extracted with ethyl acetate.

The collected organic layer was washed with brine and dried on anhydrous Na2SO4. The solvent was then removed in vacuo to give an oil. Using purification by PREP TLC (MeOH/DCM = 5%), target chemical, 2-(3-(2,6-dioxopiperidin-3-yl)-4-oxo-3,4-dihydrobenzo[d][1,2,3]triazin-6-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide was obtained (8 mg, 0.009 mmol, 27% yield) as white solid.

¹H NMR (500 MHz, CDCl₃) δ 8.66 (s, 1H), 8.31 (s, 1H), 8.12 (s, 1H), 7.95-7.90 (m, 1H), 7.88-7.83 (m, 2H), 7.44-7.38 (m, 1H), 7.21-7.15 (m, 2H), 7.07-6.99 (m, 2H), 6.50 (t, *J* = 5.8 Hz, 1H), 6.42-6.33 (m, 3H), 5.82-5.74 (m, 1H), 5.70 (t, *J* = 5.0 Hz, 1H), 4.87 (d, *J* = 5.6 Hz, 2H), 3.90 (d, *J* = 4.9 Hz, 2H), 3.84-3.77 (m, 2H), 3.69-3.61 (m, 2H), 3.30 (q, *J* = 6.7 Hz, 2H), 3.27-3.17 (m, 4H), 2.99-2.78 (m, 3H), 2.41-2.32 (m, 3H), 1.75-1.55 (m, 2H), 1.54-1.44 (m, 2H), 1.38-1.26 (m, 10H)., LC/MS (ESI) *m*/*z* 858.1 [M+H]⁺, 856.2 [M-H]⁻

### <Example 84> Preparation of N-(4-(4-(4-((3S,4R)-4-(dimethylamino)-1-(2-fluoro-6-methylbenzyl)pyrrolidin-3-yl)phenyl)piperazin-1-yl)butyl)-2-(3-(2,6-dioxopiperidin-3-yl)-4-oxo-3,4-dihydrobenzo[d][1,2,3]triazin-6-ylamino)acetamide

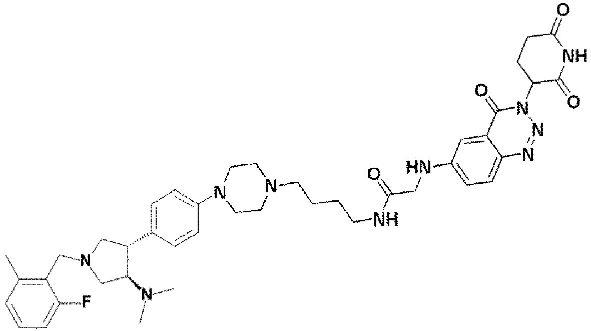

To (3R,4S)-4-(4-(4-(4-aminobutyl)piperizin-1-yl)phenyl)-1-(2-fluoro-6-methylbenzyl)-N,N-dimethylpirrolidin-3-amine hydrochloride (15mg, 0.029 mmol, prepared in example 49 step 5) in DMF (2mL), EDCl (8.5mg, 0.043 mmol), HOBt (0.043 mmol), DIPEA (8 µ L, 0.043 mmol), and 3-(6-(2-carboxyethylamino)-4-oxobenzo[d][1,2,3]triazin-3(4H)-yl)piperidin-2,6-dione (10mg, 0.029 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(4-(4-(4-((3S,4R)-4-(dimethylamino)-1-(2-fluoro-6-methylbenzyl)pyrrolidin-3-yl)phenyl)piperazin-1-yl)butyl)-2-(3-(2,6-dioxopiperidin-3-yl)-4-oxo-3,4-dihydrobenzo[d][1,2,3]triazin-6-ylamino)acetamide was obtained (8 mg, 0.009 mmol, 27% yield) as yellow oil.

¹H NMR : (300 MHz, CDCl₃) δ 7.89 (d, *J* = 8.8 Hz, 1H), 7.35 (m, 1H), 7.25-7.08 (m, 5H), 6.96 (d, *J* = 7.5 Hz, 1H), 6.92-6.79 (m, 3H), 5.82 (s, 1H), 5.78-5.66 (m, 1H), 4.90 (s, 1H), 3.91 (d, *J* = 3.9 Hz, 2H), 3.66 (dd, *J* = 5.0, 2.3 Hz, 2H), 3.36 (s, 2H), 3.29-3.09 (m, 6H), 3.07-2.83 (m, 5H), 2.75 (m,5H), 2.61-2.42 (m, 6H), 2.30 (m, 5H), 2.05 (d, *J* = 5.8 Hz, 2H), 1.63 (s, 4H).

### <Example 85> Preparation of N-(4-(4-(4-((3S,4R)-4-(dimethylamino)-1-(2-fluoro-6-methylbenzyl)pyrrolidin-3-yl)phenyl)piperazin-1-yl)butyl)-1-(3-(2,6-dioxopiperidin-3-yl)-4-oxo-3,4-dihydrobenzo[d][1,2,3]triazin-6-yl)azetidin-3-carboxamide

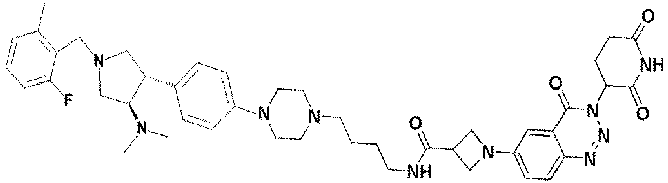

To (3R,4S)-4-(4-(4-(4-aminobutyl)piperizin-1-yl)phenyl)-1-(2-fluoro-6-methylbenzyl)-N,N-dimethylpirrolidin-3-amine hydrochloride (10mg, 0.019 mmol, prepared in example 49 step 5) in DMF (1mL), EDCl (4.0mg, 0.0209 mmol), HOBt (0.0209 mmol), DIPEA (17 µ L, 0.095 mmol), and 3-(6-(3-carboxyazetidin-1-yl)-4-oxobenzo[d][1,2,3]triazin-3(4H)-yl)piperidin-2,6-dione (7.0 mg, 0.019 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(4-(4-(4-((3S,4R)-4-(dimethylamino)-1-(2-fluoro-6-methylbenzyl)pyrrolidin-3-yl)phenyl)piperazin-1-yl)butyl)-1-(3-(2,6-dioxopiperidin-3-yl)-4-oxo-3,4-dihydrobenzo[d][1,2,3]triazin-6-yl)azetidin-3-carboxamide was obtained (6.0 mg, 37% yield) as colorless oil.

¹H NMR : (500 MHz, MeOH-d4) δ 7.97 (d, *J* = 9.2 Hz, 1H), 7.22 (d, *J* = 8.2 Hz, 2H), 7.19-7.14 (m, 1H), 7.11 (d, *J* = 9.0 Hz, 1H), 7.01 (d, *J* = 8.3 Hz, 2H), 6.89 (d, *J* = 8.6 Hz, 3H), 5.86 (m, 1H), 4.29 (t, *J* = 8.3 Hz, 2H), 4.20 (t, *J* = 7.1 Hz, 2H), 3.66 (s, 2H), 3.64-3.59 (m, 1H), 3.29 (m, 2H), 3.15 (m, 4H), 3.04 (m, 2H), 2.99-2.90 (m, 3H), 2.84 (m, 3H), 2.64 (t, *J* = 4.9 Hz, 4H), 2.50 (t, *J* = 7.1 Hz, 1H), 2.47 (s, 3H), 2.44 (d, *J* = 7.2 Hz, 1H), 2.33 (m, 1H), 2.19 (s, 6H), 2.03 (d, *J* = 11.7 Hz, 1H), 1.59 (m,2H), 1.33 (m, 2H).

### <Example 86> Preparation of N-(4-(4-(4-((3S,4R)-4-(dimethylamino)-1-(2-fluoro-6-methylbenzyl)pyrrolidin-3-yl)phenyl)piperazin-1-yl)butyl)-1-(3-(2,6-dioxopiperidin-3-yl)-4-oxo-3,4-dihydrobenzo[d][1,2,3]triazin-6-yl)piperidin-4-carboxamide

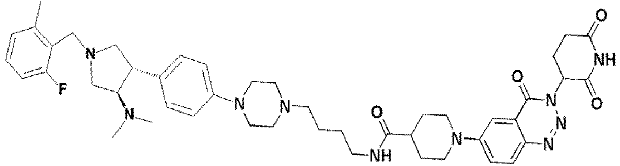

To (3R,4S)-4-(4-(4-(4-aminobutyl)piperizin-1-yl)phenyl)-1-(2-fluoro-6-methylbenzyl)-N,N-dimethylpirrolidin-3-amine hydrochloride (10mg, 0.019 mmol, prepared in example 49 step 5) in DMF (1mL), EDCl (4.0mg, 0.0209 mmol), HOBt (0.0209 mmol), DIPEA (17 µ L, 0.095 mmol), and 3-(6-(4-carboxypiperidin-1-yl)-4-oxobenzo[d][1,2,3]triazin-3(4H)-yl)piperidin-2,6-dione (7.5 mg, 0.019 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(4-(4-(4-((3S,4R)-4-(dimethylamino)-1-(2-fluoro-6-methylbenzyl)pyrrolidin-3-yl)phenyl)piperazin-1-yl)butyl)-1-(3-(2,6-dioxopiperidin-3-yl)-4-oxo-3,4-dihydrobenzo[d][1,2,3]triazin-6-yl)piperidin-4-carboxamide was obtained (6.0 mg, 40% yield) as colorless oil

¹H NMR : (500 MHz, MeOH-d4) δ 7.98 (d, *J* = 8.6 Hz, 1H), 7.67 (d, *J* = 9.1 Hz, 1H), 7.52 (s, 1H), 7.22 (d, *J* = 8.2 Hz, 1H), 7.16 (q, *J* = 7.2 Hz, 1H), 7.00 (d, *J* = 7.7 Hz, 1H), 6.89 (dd, *J* = 16.4, 8.5 Hz, 3H), 5.88 (m, 1H), 4.16 (d, *J* = 13.5 Hz, 2H), 3.66 (s, 1H), 3.24 (m 2H), 3.16 (m, 5H), 3.11-3.03 (m, 4H), 3.00 (s, 1H), 2.95 (d, *J* = 7.2 Hz, 1H), 2.87 (s, 1H), 2.80 (m, 1H), 2.64 (m, 4H), 2.51 (d, *J* = 7.3 Hz, 1H), 2.47 (s, 3H), 2.44 (d, *J* = 7.7 Hz, 1H), 2.19 (s, 6H), 2.04 (m, 1H), 1.92 (d, *J* = 13.7 Hz, 2H), 1.87-1.81 (m, 2H), 1.57 (m, 5H), 1.33 (m, 4H).

### <Example 87> Preparation of N-(6-(4-(4-((3S,4R)-4-(dimethylamino)-1-(2-fluoro-6-methylbenzyl)pyrrolidin-3-yl)phenyl)piperazin-1-yl)hexyl)-2-(3-(2,6-dioxopiperidin-3-yl)-4-oxo-3,4-dihydrobenzo[d][1,2,3]triazin-6-ylamino)acetamide

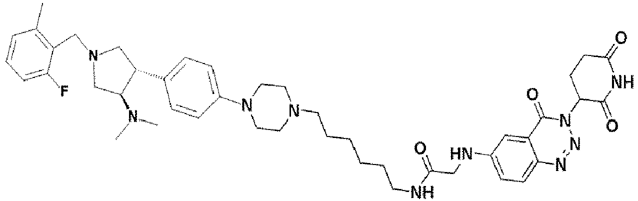

To (3R,4S)-4-(4-(4-(6-aminohexyl)piperazin-1-yl)phenyl)-1-(2-fluoro-6-methylbenzyl)-N,N-dimethylpyrrolidin-3-amine hydrochloride (10mg, 0.018 mmol, prepared in example 57 step 2) in DMF (1mL), EDCl (4.0mg, 0.0198 mmol), HOBt (0.0198 mmol), DIPEA (5 µ L, 0.027 mmol), and 3-(6-(2-carboxyethylamino)-4-oxobenzo[d][1,2,3]triazin-3(4H)-yl)piperidin-2,6-dione (6.2 mg, 0.018 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)hexyl)-2-(3-(2,6-dioxopiperidin-3-yl)-4-oxo-3,4-dihydrobenzo[d][1,2,3]triazin-6-ylamino)acetamide was obtained (5.0 mg, 33% yield) as yellow oil

¹H NMR : (500 MHz, MeOH-d4) δ 7.96 (d, *J* = 9.0 Hz, 1H), 7.36 (dd, *J* = 9.0, 2.7 Hz, 1H), 7.25 (d, *J* = 8.6 Hz, 2H), 7.22-7.13 (m, 2H), 7.03 (d, *J* = 7.6 Hz, 1H), 6.97-6.88 (m, 3H), 5.87 (m, 1H), 3.97 (s, 2H), 3.70 (d, *J* = 2.2 Hz, 2H), 3.30-3.17 (m, 8H), 3.09-3.00 (m, 2H), 2.97-2.84 (m, 2H), 2.83-2.70 (m, 6H), 2.54-2.50 (m, 2H), 2.49 (s, 4H), 2.36 (s, 6H), 2.05 (m,3H), 1.32 (m, 8H).

### <Example 88> Preparation of N-(6-(4-(4-((3S,4R)-4-(dimethylamino)-1-(2-fluoro-6-methylbenzyl)pyrrolidin-3-yl)phenyl)piperazin-1-yl)hexyl)-1-(3-(2,6-dioxopiperidin-3-yl)-4-oxo-3,4-dihydrobenzo[d][1,2,3]triazin-6-yl)piperidin-4-carboxamide

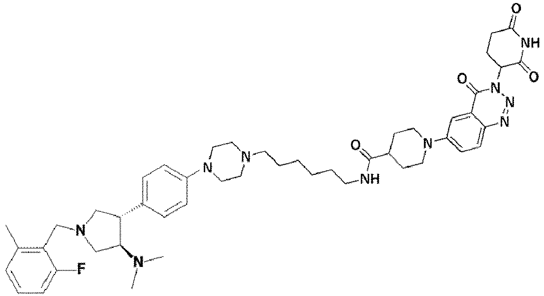

To (3R,4S)-4-(4-(4-(6-aminohexyl)piperazin-1-yl)phenyl)-1-(2-fluoro-6-methylbenzyl)-N,N-dimethylpyrrolidin-3-amine hydrochloride (10mg, 0.018 mmol, prepared in example 57 step 2) in DMF (1mL), EDCl (4.0mg, 0.0198 mmol), HOBt (0.0198 mmol), DIPEA (16 µ L, 0.095 mmol), and 3-(6-(4-carboxypiperidin-1-yl)-4-oxobenzo[d][1,2,3]triazin-3(4H)-yl)piperidin-2,6-dione (7.2 mg, 0.018 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)hexyl)-1-(3-(2,6-dioxopiperidin-3-yl)-4-oxo-3,4-dihydrobenzo[d][1,2,3]triazin-6-yl)piperidin-4-carboxamide was obtained (7.0 mg, 40% yield) as colorless oil

¹H NMR : (500 MHz, MeOD-d4) δ 8.01 (d, *J* = 9.2 Hz, 1H), 7.78-7.65 (m, 1H), 7.55 (d, *J* = 2.9 Hz, 1H), 7.24 (d, *J* = 8.4 Hz, 2H), 7.18 (q, *J* = 7.6 Hz, 1H), 7.03 (d, *J* = 7.6 Hz, 1H), 6.91 (dd, *J* = 18.7, 8.8 Hz, 3H), 5.90 (dd, *J* = 11.6, 5.5 Hz, 1H), 4.18 (d, *J* = 13.1 Hz, 2H), 3.69 (s, 2H), 3.19 (m, 7H), 3.13-3.07 (m, 2H), 3.01 (m, 2H), 2.98-2.80 (m, 4H), 2.70 (m, 4H), 2.52 (dd, *J* = 8.7, 5.4 Hz, 2H), 2.48 (m, 4H), 2.36 (m, 1H), 2.27 (s, 6H), 2.06 (s, 1H), 1.96-1.89 (m, 3H), 1.88-1.80 (m, 2H), 1.46-1.36 (m, 4H).

### <Example 89> Preparation of N-(6-(4-(4-((3S,4R)-4-(dimethylamino)-1-(2-fluoro-6-methylbenzyl)pyrrolidin-3-yl)phenyl)piperazin-1-yl)hexyl)-1-(3-(2,6-dioxopiperidin-3-yl)-4-oxo-3,4-dihydrobenzo[d][1,2,3]triazin-6-yl)azetidin-3-carboxamide

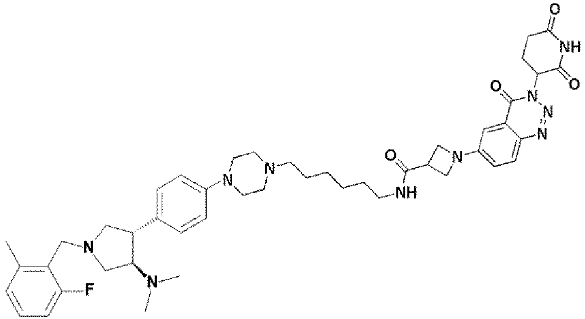

To (3R,4S)-4-(4-(4-(6-aminohexyl)piperazin-1-yl)phenyl)-1-(2-fluoro-6-methylbenzyl)-N,N-dimethylpyrrolidin-3-amine hydrochloride (10mg, 0.018 mmol, prepared in example 57 step 2) in DMF (1mL), EDCl (4.0mg, 0.0198 mmol), HOBt (0.0198 mmol), DIPEA (17 µ L, 0.095 mmol), and 3-(6-(3-carboxyazetidin-1-yl)-4-oxobenzo[d][1,2,3]triazin-3(4H)-yl)piperidin-2,6-dione (7.2 mg, 0.018 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)hexyl)-1-(3-(2,6-dioxopiperidin-3-yl)-4-oxo-3,4-dihydrobenzo[d][1,2,3]triazin-6-yl)azetidin-3-carboxamide was obtained (8.0 mg, 50% yield) as colorless oil

¹H NMR : (300 MHz, MeOH-d4) δ 7.98 (d, *J* = 8.8 Hz, 1H), 7.23 (d, *J* = 8.3 Hz, 2H), 7.14 (m, 2H), 7.06-6.98 (m, 2H), 6.91 (d, *J* = 8.6 Hz, 3H), 5.89 (m, 1H), 4.30 (t, *J* = 8.3 Hz, 2H), 4.20 (t, *J* = 7.0 Hz, 2H), 3.65 (m, 2H), 3.26 (t, *J* = 6.8 Hz, 1H), 3.17 (s, 4H), 3.10-2.94 (m, 3H), 2.92-2.77 (m, 3H), 2.66 (s, 5H), 2.46 (m, 5H), 2.23 (m, 6H), 1.58 (m, 4H), 1.41 (m, 4H),1.31 (s,5H).

### <Example 90> Preparation of N-(8-(4-(4-((3S,4R)-4-(dimethylamino)-1-(2-fluoro-6-methylbenzyl)pyrrolidin-3-yl)phenyl)piperazin-1-yl)octyl)-2-(3-(2,6-dioxopiperidin-3-yl)-4-oxo-3,4-dihydrobenzo[d][1,2,3]triazin-6-ylamino)acetamide

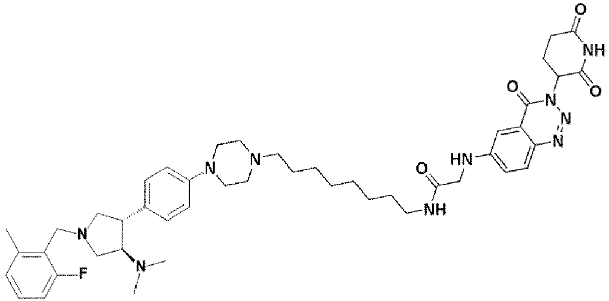

To (3R,4S)-4-(4-(4-(8-aminooctyl)piperazin-1-yl)phenyl)-1-(2-fluoro-6-methylbenzyl)-N,N-dimethylpyrrolidin-3-amine hydrochloride (10mg, 0.017 mmol, prepared in example 63 step 5) in DMF (1.0 mL), EDCl (4.0mg, 0.0187 mmol), HOBt (0.0187 mmol), DIPEA (12.0 µ L, 0.068 mmol), and 3-(6-(2-carboxyethylamino)-4-oxobenzo[d][1,2,3]triazin-3(4H)-yl)piperidin-2,6-dione (6.0 mg, 0.017 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(8-(4-(4-((3S,4R)-4-(dimethylamino)-1-(2-fluoro-6-methylbenzyl)pyrrolidin-3-yl)phenyl)piperazin-1-yl)octyl)-2-(3-(2,6-dioxopiperidin-3-yl)-4-oxo-3,4-dihydrobenzo[d][1,2,3]triazin-6-ylamino)acetamide was obtained (5.0 mg, 33% yield).

¹H NMR : (500 MHz, MeOH-d4) δ 7.91 (d, *J* = 8.9 Hz, 1H), 7.32 (dd, *J* = 8.9, 2.7 Hz, 1H), 7.23-7.18 (m, 2H), 7.17-7.10 (m, 2H), 6.99 (d, *J* = 7.6 Hz, 1H), 6.92-6.83 (m, 3H), 5.86 (dd, *J* = 11.7, 5.4 Hz, 1H), 3.94 (s, 2H), 3.65 (s,2H), 3.22 (t, *J* = 6.9 Hz, 2H), 3.17-3.09 (m, 5H), 3.04-2.76 (m, 7H), 2.62 (m, 5H), 2.50 (dd, *J* = 9.1, 5.7 Hz, 1H), 2.46 (s, 3H), 2.41-2.34 (m, 2H), 2.34-2.28 (m, 1H), 2.17 (s, 6H), 1.48 (m,5H), 1.32-1.28 (m, 7H).

### <Example 91> Preparation of N-(8-(4-(4-((3S,4R)-4-(dimethylamino)-1-(2-fluoro-6-methylbenzyl)pyrrolidin-3-yl)phenyl)piperazin-1-yl)octyl)-1-(3-(2,6-dioxopiperidin-3-yl)-4-oxo-3,4-dihydrobenzo[d][1,2,3]triazin-6-yl)azetidin-3-carboxamide

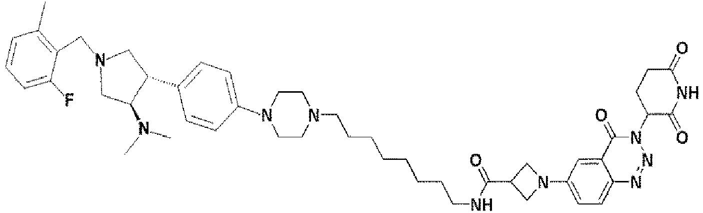

To (3R,4S)-4-(4-(4-(8-aminooctyl)piperazin-1-yl)phenyl)-1-(2-fluoro-6-methylbenzyl)-N,N-dimethylpyrrolidin-3-amine hydrochloride (10mg, 0.017 mmol, prepared in example 63 step 5) in DMF (1.0 mL), EDCl (4.0mg, 0.0187 mmol), HOBt (0.0187 mmol), DIPEA (12.0 µ L, 0.068 mmol), and 3-(6-(3-carboxyazetidin-1-yl)-4-oxobenzo[d][1,2,3]triazin-3(4H)-yl)piperidin-2,6-dione (6.0 mg, 0.017 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(8-(4-(4-((3S,4R)-4-(dimethylamino)-1-(2-fluoro-6-methylbenzyl)pyrrolidin-3-yl)phenyl)piperazin-1-yl)octyl)-1-(3-(2,6-dioxopiperidin-3-yl)-4-oxo-3,4-dihydrobenzo[d][1,2,3]triazin-6-yl)azetidin-3-carboxamide was obtained (6.0 mg, 37% yield.) as colorless oil.

¹H NMR : (500 MHz, MeOH-d4) δ 7.96 (d, *J* = 8.8 Hz, 1H), 7.21 (d, *J* = 8.2 Hz, 2H), 7.15 (q, *J* = 7.4 Hz, 1H), 7.10 (dd, *J* = 8.7, 2.2 Hz, 1H), 7.02-6.98 (m, 2H), 6.88 (m, 3H), 5.86 (dd, *J* = 11.0, 5.7 Hz, 1H), 4.27 (t, *J* = 8.2 Hz, 2H), 4.18 (t, *J* = 7.0 Hz, 2H), 3.65 (s, 2H), 3.63-3.53 (m, 1H), 3.23 (t, *J* = 7.1 Hz, 2H), 3.14 (m,5H), 3.06-3.00 (m, 1H), 2.99-2.75 (m, 5H), 2.64 (m, 5H), 2.50 (t, *J* = 7.5 Hz, 1H), 2.46 (s, 3H), 2.41 (t, *J* = 8.0 Hz, 2H), 2.36-2.28 (m, 1H), 2.19 (s, 6H), 1.62-1.44 (m, 6H), 1.36 (m, 6H).

### <Example 92> Preparation of N-(8-(4-(4-((3S,4R)-4-(dimethylamino)-1-(2-fluoro-6-methylbenzyl)pyrrolidin-3-yl)phenyl)piperazin-1-yl)octyl)-1-(3-(2,6-dioxopiperidin-3-yl)-4-oxo-3,4-dihydrobenzo[d][1,2,3]triazin-6-yl)piperidin-4-carboxamide

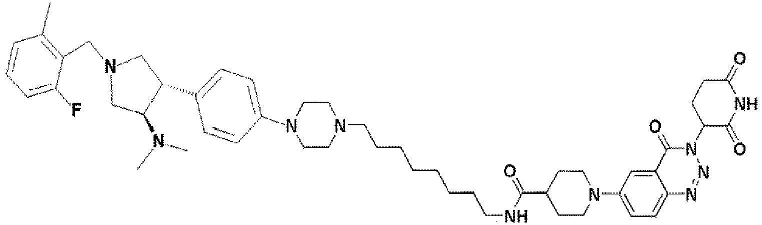

To (3R,4S)-4-(4-(4-(8-aminooctyl)piperazin-1-yl)phenyl)-1-(2-fluoro-6-methylbenzyl)-N,N-dimethylpyrrolidin-3-amine hydrochloride (10mg, 0.017 mmol, prepared in example 63 step 5) in DMF (1.0 mL), EDCl (4.0mg, 0.0187 mmol), HOBt (0.0187 mmol), DIPEA (12.0 µ L, 0.068 mmol), and 3-(6-(2-carboxyethylamino)-4-oxobenzo[d][1,2,3]triazin-3(4H)-yl)piperidin-2,6-dione (6.0 mg, 0.017 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(8-(4-(4-((3S,4R)-4-(dimethylamino)-1-(2-fluoro-6-methylbenzyl)pyrrolidin-3-yl)phenyl)piperazin-1-yl)octyl)-1-(3-(2,6-dioxopiperidin-3-yl)-4-oxo-3,4-dihydrobenzo[d][1,2,3]triazin-6-yl)piperidin-4-carboxamide was obtained (6.0 mg, 37% yield.) as colorless oil

¹H NMR : (500 MHz, MeOH-d4) δ 7.97 (d, *J* = 9.2 Hz, 1H), 7.66 (dd, *J* = 9.2, 3.0 Hz, 1H), 7.51 (d, *J* = 2.9 Hz, 1H), 7.21 (d, *J* = 8.6 Hz, 2H), 7.15 (m 1H), 7.00 (d, *J* = 7.6 Hz, 1H), 6.92-6.84 (m, 3H), 5.88 (dd, *J* = 11.6, 5.5 Hz, 1H), 4.15 (d, *J* = 13.1 Hz, 2H), 3.65 (s, 2H), 3.15 (m, 7H), 3.09-2.99 (m, 3H), 2.98-2.90 (m, 3H), 2.85 (m, 2H), 2.63 (m, 5H), 2.49 (dd, *J* = 9.3, 5.4 Hz, 2H), 2.46 (s, 3H), 2.44-2.39 (m, 2H), 2.33 (m, 1H), 2.18 (s, 6H), 1.84 (m, 4H), 1.53 (m, 4H), 1.36 (m, 8H).

### <Example 93> Preparation of N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-4-yl)oxy)acetamide

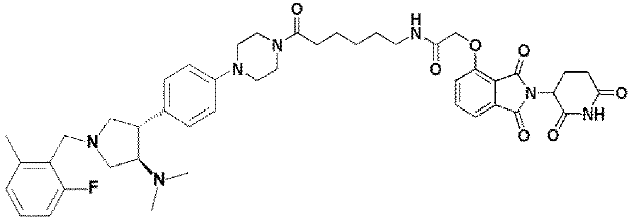

To (3R,4S)-1-(2-fluoro-6-methylbenzyl)-N,N-dimethyl-4-(4-(piperazine-1-yl)phenyl)pyrrolidin-3-amine (10mg, 0.0252 mmol) in DMF (1.0 mL), EDCl (5.5mg, 0.0277 mmol), HOBt (0.0277 mmol), DIPEA (18.0 µ L, 0.100 mmol), and 6-(2-(1,3-dioxo-2-(2,6-dioxopiperidin-3-yl)isoindolin-7-yloxy)acetamido)hexanoic acid (12.0 mg, 0.0252 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-4-yl)oxy)acetamide was obtained (7.0 mg, 35% yield.) as yellow oil

¹H NMR : (500 MHz, MeOH-d4) δ 7.79 (dd, J = 8.4, 7.3 Hz, 1H), 7.52 (d, J = 7.3 Hz, 1H), 7.41 (d, J = 8.2 Hz, 1H), 7.24-7.19 (m, 2H), 7.15 (m, 1H), 7.00 (d, J = 7.6 Hz, 1H), 6.93-6.84 (m, 4H), 5.13 (dd, J = 13.6, 5.4 Hz, 1H), 4.74 (s, 2H), 3.69 (t, J = 5.2 Hz, 2H), 3.65 (m, 4H), 3.36-3.33 (m, 2H), 3.12 (d, J = 5.6 Hz, 3H), 3.06 (t, J = 5.3 Hz, 2H), 3.00-2.92 (m, 4H), 2.88-2.81 (m, 1H), 2.76-2.69 (m, 2H), 2.61 (m, 1H), 2.52-2.48 (m, 1H), 2.46 (s, 3H), 2.43 (t, J = 7.6 Hz, 2H), 2.16 (s, 6H), 1.63 (m, 4H), 1.43 (m, 2H).

### <Example 94> Preparation of N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-4-yl)amino)acetamide

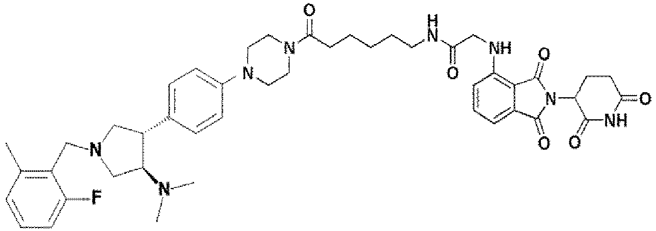

To (3R,4S)-1-(2-fluoro-6-methylbenzyl)-N,N-dimethyl-4-(4-(piperazine-1-yl)phenyl)pyrrolidin-3-amine (10mg, 0.0252 mmol) in DMF (1.0 mL), EDCl (5.5mg, 0.0277 mmol), HOBt (0.0277 mmol), DIPEA (18.0 µ L, 0.100 mmol), and 2-(1,3-dioxo-2-(2,6-dioxopiperidin-3-yl)isoindolin-7-ylamino)-N-(6-carboxyhexyl)acetamide (12.0 mg, 0.0252 mmol,). And it was stirred at a room temperature for 12 hours. After completion of the reaction, the reaction mixture was diluted with water and extracted with ethyl acetate.

The organic layer was washed with brine and dried on anhydrous Na2SO4, and then was distilled. Using purification by column chromatography, target chemical, N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-4-yl)amino)acetamide was obtained (4.0 mg, 20% yield.) as yellow oil

¹H NMR : (500 MHz, MeOH-d4) δ 7.58 (t, J = 7.9 Hz, 1H), 7.24 (d, J = 8.1 Hz, 2H), 7.15 (m, 2H), 7.01 (d, J = 7.6 Hz, 1H), 6.97-6.85 (m, 4H), 5.07 (dd, J = 12.6, 5.5 Hz, 1H), 3.99 (s, 2H), 3.69 (m, 4H), 3.65 (m, 2H), 3.22 (m, 4H), 3.11 (m, 4H), 3.01 (q, J = 7.6, 6.7 Hz, 2H), 2.89-2.79 (m, 1H), 2.77-2.69 (m, 3H), 2.50 (d, J = 7.7 Hz, 1H), 2.47 (s, 3H), 2.38 (t, J = 7.5 Hz, 2H), 2.32 (s, 6H), 2.03 (d, J = 12.1 Hz, 1H), 1.59 (q, J = 7.8 Hz, 2H), 1.52 (q, J = 7.2 Hz, 2H), 1.38-1.31 (m, 2H).

### <Example 95> Preparation of (2S,4R)-1-((S)-2-(2-(3-(4-(4-(5-(furan-2-ylmethylamino)-[1,2,4]triazolo[4,3-f]pyrimidin-8-yl)phenyl)piperazin-1-yl)propoxy)acetamido)-3,3-dimethylbutanoyl)-4-hydroxy-N-(4-(4-methylthiazol-5-yl)benzyl)pyrrolidin-2-carboxamide

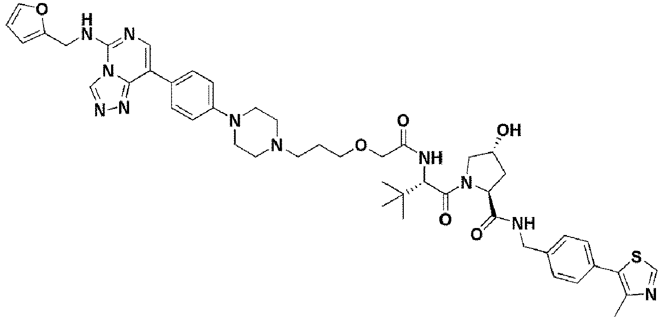

### Step 1: Preparation of tert-butyl 2-(3-chloropropoxy)acetate.

To a solution of 3-chloropropane-1-ol (3 g, 31.73 mmol) in THF was added tert-butyl Bromoacetate (5.63 ml, 38.07 mmol) and potassium tert-butoxide (5.34 g, 47.60 mmol) at 0 °C. was added. The reaction mixture was stirred at room temperature overnight. The reaction mixture was diluted with water (20 ml) and extracted with EtOAc (50 ml×2). The combined organic layers were washed with brine, dried over MgSO4 and the solvent removed in vacuo to give an oil. The crude mixture was purified by silica gel column chromatography using EtOAc/Hx = 23 % to give the title compound (1.60 g, 7.66 mmol, 24 %) as a clear oil.

¹H NMR (300 MHz, CDCl₃) δ 3.95 (s, 2H), 3.66 (q, J = 6.2 Hz, 4H), 2.11-1.99 (m, 2H), 1.47 (s, 9H).

### Step 2: Preparation of tert-butyl 2-(3-iodopropoxy)acetate.

To a solution of the compound prepared in step 1 above (1.60 g, 7.66 mmol) in acetone (20 ml) was added sodium iodide (4.60 g, 30.6 mmol) at room temperature. The reaction mixture was stirred at reflux overnight. The reaction mixture was diluted with water and extracted with EtOAc (50 mL×2). The combined organic layers were dried over MgSO4 and the solvent was removed in vacuo to give the desired compound (2.30 g, quant.) as a brown transparent oil.

¹H NMR (300MHz, DMSO-d₆) δ 3.97 (s, 2H), 3.48 (t, J = 6.0 Hz, 2H), 3.33-3.23 (m, 2H), 2.10-1.90 (m, 2H), 1.43 (s, 9H).

### Step 3: Preparation of tert-butyl 2-(3-(4-(4-(5-((furan-2-ylmethyl)amino)-[1,2,4]triazolor[4,3-c]pyrimidin-8-yl)phenyl)piperazin-1-yl)propoxy)acetate.

N-(furan-2-ylmethyl)-8-(4-(piperazin-1-yl)phenyl)-[1,2,4]triazolo[4,3-f]pyrimidin-5-amine hydrochloride (50mg) in DMF ^{∗}2, 0.243 mmol), the compound prepared in step 2 (109 mg, 0.364 mmol) and DIPEA (0.127 mL, 0.729 mmol) were added and stirred at 60 °C overnight. The reaction mixture was diluted with water and extracted with EtOAc (50 mL×2). The combined organic layers were washed with brine, dried over MgSO4 and the solvent removed in vacuo to give an oil. The crude mixture was purified by silica gel column chromatography using MeOH/DCM = 4 % to give the title compound (57 mg, 0.104 mmol, 42 %) as a yellow solid.

¹H NMR (300 MHz, CDCl₃) δ 8.31 (s, 1H), 8.12 (s, 1H), 7.88-7.81 (m, 2H), 7.43-7.38 (m, 1H), 7.07-6.99 (m, 2H), 6.43 (t, J = 5.8 Hz, 1H), 6.40-6.33 (m, 2H), 4.87 (d, J = 5.8 Hz, 2H), 3.97 (s, 2H), 3.61 (t, J = 6.3 Hz, 2H), 3.30 (s, 4H), 2.67 (s, 4H), 2.61-2.48 (m, 2H), 1.97-1.82 (m, 2H), 1.49 (s, 9H).

### Step 4: Preparation of 2-(3-(4-(4-(5-((furan-2-ylmethyl)amino)-[1,2,4]triazolor[4,3-c]pyrimidin-8-yl)phenyl)piperazin-1-yl)propoxy)acetate.

To a solution of the compound prepared in step 3 above (57 mg, 0.104 mmol) in DCM (1 ml) was added TFA (1 ml), and the resulting mixture was stirred at room temperature for 1 hour. The reaction mixture was concentrated in vacuo to give the desired compound (50 mg, crude) as an oil.

¹H NMR (300 MHz, DMSO-d₆) δ 9.47 (s, 1H), 8.77 (t, J = 6.1 Hz, 1H), 8.61 (s, 1H), 8.26 (s, 1H), 8.06-7.97 (m, 2H), 7.61-7.54 (m, 1H), 7.21-7.06 (m, 2H), 6.44-6.29 (m, 2H), 4.75 (d, J = 6.0 Hz, 2H), 4.06 (s, 2H), 4.03-3.86 (m, 2H), 3.75-3.63 (m, 2H), 3.59 (t, J = 5.6 Hz, 2H), 3.38-3.14 (m, 4H), 3.12-2.95 (m, 2H), 2.06-1.93 (m, 2H),

### Step 5: Preparation of (2S,4R)-1-((S)-2-(2-(3-(4-(4-(5-(furan-2-ylmethylamino)-[1,2,4]triazolor[4,3-flpyrimidin-8-yl)phenyl)piperazin-1-yl)propoxy)acetamido)-3,3-dimethylbutanoyl)-4-hydroxy-N-(4-(4-methylthiazol-5-yl)benzyl)pyrrolidin-2-carboxamide

(2R,4R)-1-((R)-2-amino-3,3-dimethylbutanoyl)-4-hydroxy-N-(4-(4-methylthiazole-5-yl)benzyl)pyrrolidin-2- in DMF To a solution of carboxamide hydrochloride (48 mg, 0.104 mmol), the compound prepared in the previous step (50 mg, 0.104 mmol), HATU (60 mg, 0.156 mmol), and DIPEA (0.072 mL, 0.416 mmol) were added and stirred at room temperature for 2 hours. stirred. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 5 % to give the title compound (1 mg, 0.001 mmol, 1 %) as a pale yellow solid.

¹H NMR (500 MHz, CD₃OD) δ 8.72 (s, 1H), 8.29 (s, 1H), 7.97 (s, 1H), 7.71-7.63 (m, 2H), 7.39-7.28 (m, 5H), 6.96-6.90 (m, 2H), 6.26 (d, J = 1.4 Hz, 2H), 4.72 (s, 2H), 4.61 (s, 1H), 4.51-4.48 (m, 1H), 4.48-4.43 (m, 1H), 4.43-4.38 (m, 1H), 3.97-3.92 (m, 1H), 3.90-3.84 (m, 1H), 3.79-3.75 (m, 1H), 3.73-3.68 (m, 1H), 3.59-3.49 (m, 4H), 3.16-3.11 (m, 4H), 2.59 (m, 4H), 2.54-2.47 (m, 2H), 2.35 (s, 3H), 2.18-2.11 (m, 1H), 1.85-1.80 (m, 2H), 0.95 (s, 8H) ; LC/MS (ESI) m/z 904.1 [M+H]+, 902.1 [M-H]⁻

### <Example 96> Preparation of (2S,4R)-1-((S)-2-(2-(4-(4-(4-(5-(furan-2-ylmethylamino)-[1,2,4]triazolo[4,3-f]pyrimidin-8-yl)phenyl)piperazin-1-yl)butoxy)acetamido)-3,3-dimethylbutanoyl)-4-hydroxy-N-(4-(4-methylthiazol-5-yl)benzyl)pyrrolidin-2-carboxamide

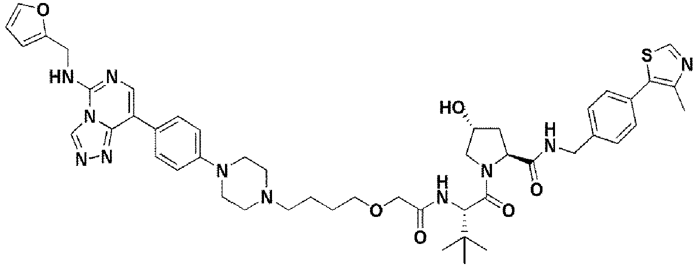

### Step 1: Preparation of tert-butyl 2-(4-chlorobutoxy)acetate.

To a solution of 4-chlorobutane-1-ol (3 g, 27.6 mmol), tert-butyl bromoacetate (4.08 ml, 27.6 mmol) in THF at 0 °C, potassium tert-butoxide (4.65 g, 41.4 mmol) was added at room temperature. Stir overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EA/Hx 30 % to obtain the title compound (1.05 g, 4.71 mmol, 17 %) as a colorless oil.

¹H NMR (300MHz, CDCl₃) δ 3.95 (s, 2H), 3.60 (t, J = 6.5 Hz, 2H), 3.55 (t, J = 6.1 Hz, 2H), 1.97-1.86 (m, 2H), 1.83-1.72 (m, 2H), 1.48 (s, 9H).

### Step 2: Preparation of tert-butyl 2-(4-iodobutoxy)acetate

Sodium iodide (673 mg, mmol) was added to an acetone solution of the compound (100 mg, 0.449 mmol) prepared in step 1, and the mixture was stirred at 80° C. for 4 hours. The solvent was removed under vacuum. The reaction mixture was diluted with 10% Na2S2O3 (aq.) and extracted with EtOAc. The combined organic layers were washed with brine, dried over MgSO4, and the solvent was removed in vacuo to give the title compound (70 mg, 0.245 mmol, 50 %) as a colorless oil.

¹H NMR (300MHz, CDCl₃) δ 3.94 (s, 2H), 3.54 (t, J = 6.1 Hz, 2H), 3.25 (t, J = 6.9 Hz, 2H), 2.00-1.91 (m, 2H), 1.80-1.68 (m, 2H), 1.48 (s, 9H)

### Step 3: Preparation of tert-butyl 2-(4-(4-(4-(5-((furan-2-ylmethyl)amino)-[1,2,4]triazolor[4,3-c]pyrimidin-8-yl)phenyl)piperazin-1-yl)butoxy)acetate.

N-(furan-2-ylmethyl)-8-(4-(piperazin-1-yl)phenyl)-[1,2,4]triazolo[4,3-f]pyrimidin-5-amine hydrochloride in DMF (40 mg, 0.097 mmol), the compound prepared in step 2 (46 mg, 0.145 mmol) and DIPEA (0.051 mL, 0.291 mmol) were added, and the mixture was stirred at 60° C. overnight. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 4 % to give the title compound (28 mg, 0.050 mmol, 51 %) as a yellow solid.

¹H NMR (300 MHz, CDCl₃) δ 8.31 (s, 1H), 8.12 (s, 1H), 7.84 (d, J = 8.4 Hz, 2H), 7.40 (s, 1H), 7.03 (d, J = 8.4 Hz, 2H), 6.46 (t, J = 5.8 Hz, 1H), 6.41-6.31 (m, 2H), 4.87 (d, J = 5.8 Hz, 2H), 3.96 (s, 2H), 3.61-3.50 (m, 2H), 3.28 (t, J = 5.0 Hz, 4H), 2.65 (t, J = 5.0 Hz, 4H), 2.53-2.38 (m, 2H), 1.75-1.59 (m, 4H), 1.49 (s, 9H).

### Step 4: Preparation of 2-(4-(4-(4-(5-((furan-2-ylmethyl)amino)-[1,2,4]triazolo[4,3-c]pyrimidin-8-yl)phenyl)piperazin-1-yl)butoxy)acetate

To a solution of the compound prepared in step 3 above (28 mg, 0.050 mmol) in DCM (1 ml) was added TFA (1 ml), and the resulting mixture was stirred at room temperature for 1 hour. The reaction mixture was concentrated in vacuo to give the title compound (30 mg, quant) as an oil.

### Step 5: Preparation of (2S,4R)-1-((S)-2-(2-(4-(4-(4-(5-(furan-2-ylmethylamino)-[1,2,4]triazolor[4,3-flyrimidin-8-yl)phenyl)piperazin-1-yl)butoxy)acetamido)-3,3-dimethylbutanoyl)-4-hydroxy-N-(4-(4-methylthiazol-5-yl)benzyl)pyrrolidin-2-carboxamide

(2S,4R)-1-((S)-2-amino-3,3-dimethylbutanoyl)-4-hydroxy-N-(4-(4-methylthiazole-5-yl)benzyl)pyrrolidin-2- in DMF To a solution of carboxamide hydrochloride (23 mg, 0.050 mmol), the compound prepared in step 4 (25 mg, 0.050 mmol), HATU (28 mg, 0.075 mmol), and DIPEA (0.035 mL, 0.200 mmol) were added and stirred at room temperature for 2 stirred for hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 14 % to give the final title compound (18 mg, 0.019 mmol, 39 %) as a pale yellow solid.

¹H NMR (300 MHz, CDCl₃) δ 8.66 (s, 1H), 8.30 (s, 1H), 8.11 (s, 1H), 7.83 (d, J = 8.5 Hz, 2H), 7.61 (s, 1H), 7.44-7.37 (m, 1H), 7.37-7.28 (m, 4H), 7.21 (s, 1H), 6.96 (d, J = 8.5 Hz, 2H), 6.47 (t, J = 5.8 Hz, 1H), 6.42-6.33 (m, 2H), 4.87 (d, J = 5.7 Hz, 2H), 4.75 (t, J = 7.9 Hz, 1H), 4.61-4.51 (m, 2H), 4.51-4.44 (m, 1H), 4.38-4.28 (m, 1H), 4.12-3.98 (m, 2H), 3.92-3.81 (m, 1H), 3.70-3.61 (m, 1H), 3.60-3.46 (m, 2H), 3.35 (s, 4H), 2.89 (s, 6H), 2.50 (s, 3H), 2.47-2.38 (m, 1H), 2.22-2.11 (m, 1H), 1.78 (s, 2H), 1.73-1.60 (m, 2H), 0.98 (s, 9H) ; LC/MS (ESI) m/z 918.2 [M+H]⁺, 916.0 [M-H]⁻

### <Example 97> Preparation of (2S,4R)-1-((S)-2-(2-(2-(2-(4-(4-(5-(furan-2-ylmethylamino)-[1,2,4]triazolo[4,3-f]pyrimidin-8-yl)phenyl)piperazin-1-yl)ethoxy)ethoxy)acetamido)-3,3-dimethylbutanoyl)-4-hydroxy-N-(4-(4-methylthiazol-5-yl)benzyl)pyrrolidin-2-carboxamide

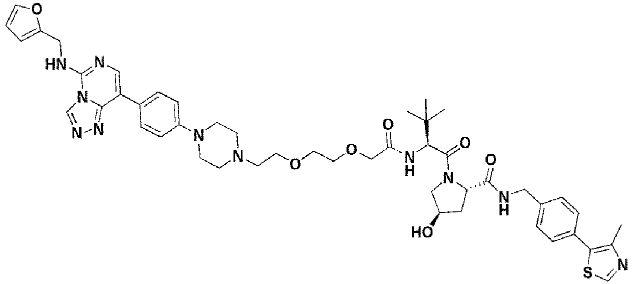

### Step 1: Preparation of tert-butyl 2-(2-(2-(benzyloxy)ethoxy)ethoxy)acetate.

In a solution of di(ethylene glycol)benzyl ether (9 mL, 54 mmol, 1 eq.) in DCM (64 mL), 37 % aq. NaOH (64 mL), t-butylbromoacetate (30 mL, 204 mmol, 4 eq.), TBAB (17 g, 52 mmol, 1.02 eq.) were added at room temperature. The reaction mixture was stirred at room temperature overnight. The reaction mixture was diluted with water and extracted with EtOAc (50 mL×2). The combined organic layers were dried over MgSO4 and the solvent was removed in vacuo. The crude mixture was purified by silica gel column chromatography using (EtOAc 100 %) as eluent to give the title compound (10.4 g, 35.3 mmol, 70 %) as a clear oil.

¹H NMR (300 MHz, CDCl₃) δ 7.38-7.33 (m, 3H), 7.33-7.26 (m, 1H), 4.58 (s, 2H), 4.05 (s, 2H), 3.80-3.61 (m, 8H), 1.49 (s, 9H).

### Step 2: Preparation of tert-butyl 2-(2-(2-hydroxyethoxy)ethoxy)acetate.

A solution of the compound prepared in step 1 above (10.4 g, 35 mmol, 1 eq.) and palladium on carbon [10 % (wt/ vol), 416 mg] in MeOH (140 mL) at 40 °C overnight in a hydrogen atmosphere ( hydrogen balloon). The palladium on carbon was removed by celite filtration and washed twice with MeOH. The combined filtrates were concentrated under reduced pressure to give the title compound (6.81 g, 33.3 mmol, 94 %) as a colorless oil, which was used in the next step without further purification.

¹H NMR (300 MHz, CDCl₃) δ 4.03 (s, 2H), 3.80-3.68 (m, 6H), 3.69-3.58 (m, 2H), 2.59 (s, 1H), 1.49 (s, 9H).

### Step 3: Preparation of tert-butyl 2-(2-(2-(tosyloxy)ethoxy)ethoxy)acetate.

A solution of the compound prepared in step 2 above (6.8 g, 33 mmol, 1 eq.) and TsCl (7.6 g, 40 mmol, 1.2 eq.) in pyridine (18 mL) was stirred at room temperature for 1 h. The reaction mixture was partitioned between EtOAc and water. The organic layer was collected, washed with cold 1 N HCl (80 mL) followed by brine, dried over Na 2 SO 4 and concentrated under reduced pressure to give a crude residue, which was purified by silica gel flash column chromatography to obtain the desired compound. (10.9 g, 30.4 mmol, 92%) was obtained as a yellow oil.

¹H NMR (300 MHz, CDCl₃) δ 7.80 (d, J = 8.3 Hz, 2H), 7.34 (d, J = 8.1 Hz, 2H), 4.20-4.13 (m, 2H), 3.98 (s, 2H), 3.73-3.68 (m, 2H), 3.68-3.59 (m, 4H), 2.44 (s, 3H), 1.47 (s, 9H).

### Step 4: Preparation of tert-butyl 2-(2-(2-iodoethoxy)ethoxy)acetate.

To a solution of the compound prepared in step 3 above (1.00 g, 2.67 mmol) in acetone (20 ml) was added sodium iodide (1.60 g, 10.68 mmol) at room temperature. The reaction mixture was stirred at reflux overnight. The reaction mixture was diluted with water and extracted with EtOAc (50 mL×2). The combined organic layers were dried over MgSO4 and the solvent was removed in vacuo to give the title compound (837 mg, 2.53 mmol, 94%) as a brown oil.

¹H NMR (300MHz, CDCl₃) δ 4.05 (s, 2H), 3.78 (t, J = 6.9 Hz, 2H), 3.75-3.68 (m, 4H), 3.28 (t, J = 6.9 Hz, 2H), 1.48 (s, 9H).

### Step 5: Preparation of tert-butyl 2-(2-(2-(4-(4-(5-((furan-2-ylmethyl)amino)-[1,2,4]triazolor[4,3-c]pyrimidin-8-yl)phenyl)piperazin-1-yl)ethoxy)ethoxy)acetate.

N-(furan-2-ylmethyl)-8-(4-(piperazin-1-yl)phenyl)-[1,2,4]triazolo[4,3-f]pyrimidin-5-amine hydrochloride in DMF (50 mg, 0.121 mmol), the compound prepared in step 4 (60 mg, 0.182 mmol) and DIPEA (0.063 mL, 0.363 mmol) were added, and the mixture was stirred at 60° C. overnight. The reaction mixture was diluted with water (20 ml) and extracted with EtOAc (50 ml×2). The combined organic layers were washed with brine, dried over MgSO4 and the solvent removed in vacuo to give an oil. The crude mixture was purified by silica gel column chromatography using MeOH/DCM = 5 % to give the title compound (23 mg, 0.039 mmol, 33 %) as a yellow solid.

¹H NMR (300 MHz, CDCl₃) δ 8.31 (s, 1H), 8.12 (s, 1H), 7.90-7.79 (m, 2H), 7.44-7.37 (m, 1H), 7.08-6.99 (m, 2H), 6.46 (t, J = 5.8 Hz, 1H), 6.41-6.32 (m, 2H), 4.87 (d, J = 5.8 Hz, 2H), 4.03 (s, 2H), 3.84 (s, 2H), 3.76-3.66 (m, 4H), 3.42 (s, 4H), 3.05-2.77 (m, 4H), 1.48 (s, 9H).

### Step 6: Preparation of 2-(2-(2-(4-(4-(5-((furan-2-ylmethyl)amino)-[1,2,4]triazolor[4,3-c]pyrimidin-8-yl)phenyl)piperazin-1-yl)ethoxy)ethoxy)acetate.

To a solution of the compound prepared in step 5 above (23 mg, 0.040 mmol) in DCM (1 ml) was added TFA (1 ml), and the resulting mixture was stirred at room temperature for 1 hour. The reaction mixture was concentrated in vacuo to give the desired compound (25 mg, crude) as an oil.

### Step 7: Preparation of Preparation of (2S,4R)-1-((S)-2-(2-(2-(2-(4-(4-(5-(furan-2-ylmethylamino)-[1,2,4]triazolo[4,3-f]pyrimidin-8-yl)phenyl)piperazin-1-yl)ethoxy)ethoxy)acetamido)-3,3-dimethylbutanoyl)-4-hydroxy-N-(4-(4-methylthiazol-5-yl)benzyl)pyrrolidin-2-carboxamide

(2S,4R)-1-((S)-2-amino-3,3-dimethylbutanoyl)-4-hydroxy-N-(4-(4-methylthiazole-5-yl)benzyl)pyrrolidin-2- in DMF The compound prepared in step 6 (25mg, 0.048mmol), HATU (27mg, 0.072mmol), and DIPEA (0.034mL, 0.192mmol) were added to a solution of carboxamide hydrochloride (22mg, 0.048mmol) and stirred at room temperature for 2 hours.. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 20 % to give the title compound (10 mg, 0.010 mmol, 22 %) as a pale yellow solid.

¹H NMR (500 MHz, CD₃OD) δ 10.06 (s, 1H), 8.97 (s, 1H), 8.28 (s, 1H), 7.84-7.69 (m, 2H), 7.63-7.48 (m, 5H), 7.24 (s, 2H), 6.55-6.47 (m, 1H), 6.45-6.37 (m, 1H), 4.76 (s, 1H), 4.66-4.58 (m, 1H), 4.52 (s, 1H), 4.49-4.37 (m, 2H), 4.21-4.04 (m, 3H), 3.99 (s, 4H), 3.94-3.87 (m, 1H), 3.87-3.73 (m, 7H), 3.67-3.48 (m, 3H), 2.67-2.58 (m, 3H), 2.36-2.27 (m, 1H), 2.13-2.00 (m, 2H), 1.08 (s, 9H) ; LC/MS (ESI) m/z 935.33 [M+H]⁺

### <Example 98> Preparation of (2S,4R)-1-((S)-2-(6-(4-(4-(5-(furan-2-ylmethylamino)-[1,2,4]triazolo[4,3-f]pyrimidin-8-yl)phenyl)piperazin-1-yl)-6-oxohexanamido)-3,3-dimethylbutanoyl)-4-hydroxy-N-(4-(4-methylthiazol-5-yl)benzyl)pyrrolidin-2-carboxamide

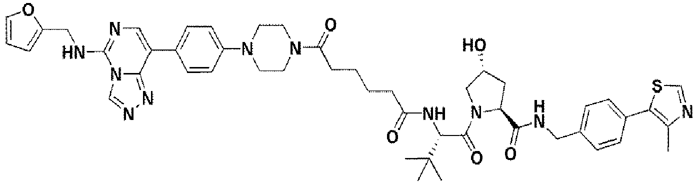

### Step 1: Preparation of methyl 6-(4-(4-(5-((furan-2-ylmethyl)amino)-[1,2,4]triazolo[4,3-c]pyrimidin-8-yl)phenyl)piperazin-1-yl)-6-oxohexanoic acid.

To a solution of monomethyl adipate (58 mg, 0.364 mmol) in DMF was added N-(furan-2-ylmethyl)-8-(4-(piperazin-1-yl)phenyl)-[1,2,4]triazolo[ 4,3-f]pyrimidin-5-amine hydrochloride (150 mg, 0.364 mmol), HATU (207 mg, 0.546 mmol), DIPEA (0.253 mL, 1.45 mmol) was stirred at room temperature overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc:Hex = 75 % to give the title compound (90 mg, 0.173 mmol, 47 %) as an ivory solid.

¹H NMR (300 MHz, CDCl₃) δ 8.31 (s, 1H), 8.12 (s, 1H), 7.90-7.82 (m, 2H), 7.44-7.38 (m, 1H), 7.08-7.00 (m, 2H), 6.45 (t, *J* = 5.8 Hz, 1H), 6.41-6.34 (m, 2H), 4.88 (d, *J* = 5.7 Hz, 2H), 3.86-3.76 (m, 2H), 3.67 (s, 3H), 3.67-3.60 (m, 2H), 3.30-3.18 (m, 4H), 2.48-2.31 (m, 4H), 1.80-1.66 (m, 4H).

### Step 2: Preparation of 6-(4-(4-(5-((furan-2-ylmethyl)amino)-[1,2,4]triazolo[4,3-c]pyrimidin-8-yl)phenyl)piperazin-1-yl)-6-oxohexanoic acid

To a solution of the compound prepared in step 1 above (90 mg, 0.173 mmol) in MeOH (1 ml) and THF (3 ml) was added LiOH HO (18 mg, 0.432 mmol) in water (1 ml). The reaction mixture was stirred at room temperature for 3 hours. The reaction mixture was acidified to pH 4 with 1 N HCl, diluted water was added and extracted with EtOAc (50 mL×2). The combined organic layers were dried over MgSO4 and the solvent was removed in vacuo to give the title compound (70 mg, 0.139 mmol, 80 %) as an ivory solid.

¹H NMR (300 MHz, DMSO-*d*₆) δ 8.74 (t, *J* = 5.9 Hz, 1H), 8.61 (s, 1H), 8.24 (s, 1H), 8.04-7.93 (m, 2H), 7.61-7.55 (m, 1H), 7.17-7.06 (m, 2H), 6.43-6.37 (m, 1H), 6.36-6.30 (m, 1H), 4.74 (d, *J* = 6.0 Hz, 2H), 3.70-3.57 (m, 4H), 3.31-3.14 (m, 4H), 2.42-2.32 (m, 2H), 2.30-2.19 (m, 2H), 1.60-1.44 (m, 4H).

### Step 3: Preparation of 2S,4R)-1-((S)-2-(6-(4-(4-(5-(furan-2-ylmethylamino)-[1,2,4]triazolor[4,3-flpyrimidin-8-yl)phenyl)piperazin-1-yl)-6-oxohexanamido)-3,3-dimethylbutanoyl)-4-hydroxy-N-(4-(4-methylthiazol-5-yl)benzyl)pyrrolidin-2-carboxamide

(2S,4R)-1-((S)-2-amino-3,3-dimethylbutanoyl)-4-hydroxy-N-(4-(4-methylthiazole-5-yl)benzyl)pyrrolidin-2- in DMF To a solution of carboxamide hydrochloride (18 mg, 0.039 mmol), the compound prepared in step 2 (20 mg, 0.039 mmol), HATU (22 mg, 0.058 mmol), and DIPEA (0.027 mL, 0.156 mmol) were added at room temperature 2 stirred for hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 10 % to give the title compound (2 mg, 0.002 mmol, 5 %) as a pale yellow solid.

### <Example 99> Preparation of (2S,4R)-1-((S)-2-(8-(4-(4-(5-(furan-2-ylmethylamino)-[1,2,4]triazolo[4,3-f]pyrimidin-8-yl)phenyl)piperazin-1-yl)-8-oxooctanamido)-3,3-dimethylbutanoyl)-4-hydroxy-N-(4-(4-methylthiazol-5-yl)benzyl)pyrrolidin-2-carboxamide

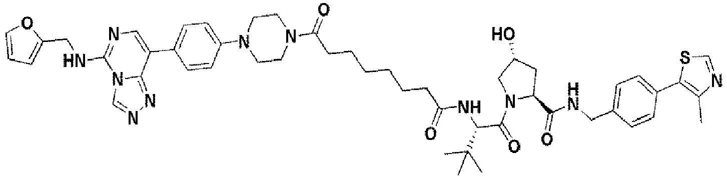

### Step 1: Preparation of dimethyl octanedioate.

Octanedioate (1.00 g, 5.74 mmol) was suspended in anhydrous DCM and cooled to 0 °C. After oxalyl chloride (1.48 mL, 17.2 mmol) was added slowly, the solution was stirred at room temperature for 2 h. The resulting solution was concentrated under reduced pressure and the residue was dissolved in anhydrous MeOH (5 ml per 1 mmol) and stirred at room temperature for 30 min. The resulting solution was concentrated under reduced pressure and dissolved in aqueous saturated NaHCO3 solution. The solution was extracted with EtOAc (2 × 25 mL). The combined organic phases were washed with saturated aqueous NaCl solution, dried over Na2SO4, filtered, and the solution was concentrated under reduced pressure to give the desired compound (1.00 g, 4.94 mmol, 86%) as a white oil.

¹H NMR (300 MHz, CDCl₃) δ 3.53 (s, 6H), 2.17 (t, J =7.5 Hz, 4H), 1.58-1.42 (m, 4H), 1.29-1.15 (m, 4H).

### Step 2: Preparation of 8-methoxy-8-oxooctanoate

To a solution of the compound prepared in step 1 above (1.00 g, 4.94 mmol) and MeOH (10 mL) was added dropwise to a mixture of KOH (277 mg, 4.94 mmol) in MeOH (20 mL) over 10 min, at room temperature 21 stirred for hours. The reaction mixture was then concentrated to remove methanol. The product-containing aqueous layer was adjusted to pH 4 with 1M HCl. The reaction was quenched with water and extracted with EtOAc (2×25 mL). The combined organic layers were dried over Na2SO4 and the solvent was removed under reduced pressure to give the title compound (512 mg, 2.72 mmol, 55%) as a white solid.

¹H NMR (300 MHz, CDCl₃) δ 3.64 (s, 3H), 2.36-2.20 (m, 4H), 1.71-1.50 (m, 4H), 01.41-1.25 (m, 4H).

### Step 3: Preparation of methyl 8-(4-(4-(5-((furan-2-ylmethyl)amino)-[1,2,4]triazolo[4,3-c]pyrimidin-8-yl)phenyl)piperazin-1-yl)-8-oxooctanoate

To a solution of the compound (210 mg, 1.12 mmol) prepared in step 2 above in DMF, N-(furan-2-ylmethyl)-8-(4-(piperazin-1-yl)phenyl)-[1,2, 4]triazolo[4,3-f]pyrimidin-5-amine hydrochloride (460 mg, 1.12 mmol), HATU (639 mg, 1.68 mmol), DIPEA (0.780 mL, 4.48 mmol) were added and stirred at room temperature overnight. The reaction mixture was diluted with water and extracted with EtOAc. The combined organic layers were washed with brine, dried over MgSO4 and the solvent removed in vacuo to give an oil. The crude mixture was purified by silica gel column chromatography using EtOAc:Hex = 70% to give the title compound (280 mg, 0.513 mmol, 45%) as a pale yellow solid.

¹H NMR (300 MHz, CDCl₃) δ 8.30 (s, 1H), 8.12 (s, 1H), 7.92-7.81 (m, 2H), 7.44-7.37 (m, 1H), 7.10-6.99 (m, 2H), 6.52 (t, J = 5.8 Hz, 1H), 6.42-6.32 (m, 2H), 4.87 (d, J = 5.8 Hz, 2H), 3.80 (t, J = 5.2 Hz, 2H), 3.67 (s, 3H), 3.81-3.60 (m, 2H), 3.22 (q, J = 6.0 Hz, 4H), 2.43-2.27 (m, 4H), 1.74-1.57 (m, 4H), 1.46-1.30 (m, 4H).

### Step 4: Preparation of 8-(4-(4-(5-((furan-2-ylmethyl)amino)-[1,2,4]triazolo[4,3-c]pyrimidin-8-yl)phenyl)piperazin-1-yl)-8-oxooctanoate

To a solution of the compound prepared in step 3 above (280 mg, 0.513 mmol) in MeOH (1 ml) and THF (3 ml) was added LiOH HO (53 mg, 1.28 mmol) in water (1 ml). The reaction mixture was stirred at room temperature for 3 hours. The reaction mixture was acidified to pH 4 with 1 N HCl, diluted water and extracted with EtOAc (50 mL×2). The combined organic layers were dried over MgSO4 and the solvent was removed in vacuo to give the title compound (220 mg, 0.413 mmol, 80 %) as an ivory solid.

¹H NMR (300 MHz, DMSO-*d*₆) δ 8.75 (t, *J* = 6.1 Hz, 1H), 8.61 (s, 1H), 8.25 (s, 1H), 8.04-7.94 (m, 2H), 7.61-7.54 (m, 1H), 7.20-7.08 (m, 2H), 6.43-6.36 (m, 1H), 6.36-6.29 (m, 1H), 4.74 (d, *J* = 6.0 Hz, 2H), 3.69-3.56 (m, 4H), 3.32-3.15 (m, 4H), 2.36 (t, *J* = 7.4 Hz, 2H), 2.20 (t, *J* = 7.3 Hz, 2H), 1.58-1.41 (m, 4H), 1.37-1.22 (m, 4H).

### Step 5: Preparation of (2S,4R)-1-((S)-2-(8-(4-(4-(5-(furan-2-ylmethylamino)-[1,2,4]triazolor[4,3-f]pyrimidin-8-yl)phenyl)piperazin-1-yl)-8-oxooctanamido)-3,3-dimethylbutanoyl)-4-hydroxy-N-(4-(4-methylthiazol-5-yl)benzyl)pyrrolidin-2-carboxamide

(2S,4R)-1-((S)-2-amino-3,3-dimethylbutanoyl)-4-hydroxy-N-(4-(4-methylthiazole-5-yl)benzyl)pyrrolidin-2- in DMF The compound prepared in step 4 (20mg, 0.037mmol), HATU (21mg, 0.055mmol), and DIPEA (0.026mL, 0.148mmol) were added to a solution of carboxamide (18mg, 0.037mmol) and stirred at room temperature for 2 hours.. The reaction mixture was diluted with water and extracted with EtOAc. The combined organic layers were washed with brine, dried over MgSO4 and the solvent removed in vacuo to give an oil. The crude mixture was purified by silica gel column chromatography in MeOH/DCM = 10 % to give the title compound (15 mg, 0.015 mmol, impurity) as an ivory solid.

¹H NMR (500 MHz, chloroform-*d*) δ 8.72 (s, 1H), 8.33 (s, 1H), 8.12 (s, 1H), 7.83 (d, *J* = 8.4 Hz, 2H), 7.56-7.43 (m, 2H), 7.43-7.38 (m, 1H), 7.38-7.31 (m, 4H), 7.08-6.97 (m, 2H), 6.51 (t, *J* = 5.8 Hz, 1H), 6.41-6.33 (m, 2H), 4.87 (d, *J* = 5.7 Hz, 2H), 4.75 (t, *J* = 8.2 Hz, 1H), 4.72-4.65 (m, 1H), 4.64-4.59 (m, 1H), 4.58-4.49 (m, 2H), 4.41-4.30 (m, 1H), 4.23-4.13 (m, 1H), 3.83-3.71 (m, 2H), 3.69-3.58 (m, 3H), 3.29-3.11 (m, 4H), 2.50 (s, 3H), 2.37 (t, *J* = 7.7 Hz, 1H), 2.24-2.21 (m, 1H), 1.68-1.53 (m, 4H), 1.36-1.29 (m, 8H), 0.98 (s, 9H) ; LC/MS (ESI) *m*/*z* 944.2 [M+H]⁺, 942.1 [M-H]⁻

### <Example 100> Preparation of (2S,4R)-1-((S)-2-tert-butyl-23-(4-(4-(5-(furan-2-ylmethylamino)-[1,2,4]triazolo[4,3-f]pyrimidin-8-yl)phenyl)piperazin-1-yl)-4,23-dioxo-6,9,12,15,18,21-hexaoxa-3-azatricosan-1-oyl)-4-hydroxy-N-(4-(4-methylthiazol-5-yl)benzyl)pyrrolidin-2-carboxamide

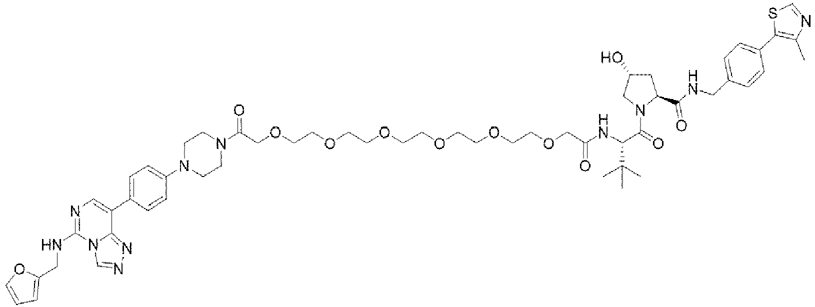

### Step 1: Preparation of 1-phenyl-2,5,8,11-tetraoxatridecane-13-yl 4-methylbenzensulfonate

To a solution of tetraethylene glycol monobenzyl ether (1 g, 3.52 mmol) in DCM (20 ml) at room temperature, TEA (1.47 ml, 10.56 mmol) and DMAP (43 mg, 0.352 mmol) were added, followed by TsCl (1.34 g, 7.03). mmol) was added at 0 °C. The reaction mixture was stirred at room temperature overnight. The reaction mixture was diluted with water and then extracted with DCM (50 mL×2). The combined organic layers were dried over MgSO4 and the solvent was removed in vacuo. The crude mixture was purified by silica gel column chromatography using (EtOAc/Hx = 45 %) as eluent to give the title compound (1.54 g, 3.52 mmol, quant.) as a clear oil.

¹H NMR (300 MHz, CDCl₃) δ 7.84-7.75 (m, 2H), 7.39-7.27 (m, 7H), 4.56 (s, 2H), 4.19-4.13 (m, 2H), 3.71-3.61 (m, 10H), 3.58 (s, 4H), 2.44 (s, 3H).

### Step 2: Preparation of 1-phenyl-2,5,8,11,14,17-hexaoxanonadecane-19-ol

To a solution of the compound (1 g, 2.28 mmol) prepared in example 100 step 1 in diethyleneglycol (10 ml), NaOH (274 mg, 6.84 mmol) was added at room temperature. The reaction mixture was stirred at 60 °C for 1 h. The reaction mixture was diluted with water and extracted with EtOAc (50 mL×8). The combined organic layers were dried over MgSO4 and the solvent was removed in vacuo. The crude mixture was purified by silica gel column chromatography to give the title compound (494 mg, 1.326 mmol, 58 %) as a clear oil.

¹H NMR (300MHz, CDCl₃) δ 7.36-7.29 (m, 4H), 7.29-7.19 (m, 1H), 4.53 (s, 2H), 3.72-3.59 (m, 22H), 3.55 (q, J = 5.4, 4.5 Hz, 3H), 3.41 (s, 1H).

### Step 3: Preparation of tert-butyl 1-phenyl-2,5,8,11,14,17,20-heptaoxadocosan-22-oate

To a solution of the compound prepared in example 100 step 2 (200 mg, 0.537 mmol) in DCM (1.28 ml), 37% aq. NaOH (1.28 ml), t-butyl bromoacetate (0.315 ml, 2.148 mmol), TBAB (176 mg, 0.547 mmol) were added at room temperature. The reaction mixture was stirred at room temperature overnight. The reaction mixture was diluted with water and extracted with EtOAc (50 mL×2). The combined organic layers were dried over MgSO4 and the solvent was removed in vacuo. The crude mixture was purified by silica gel column chromatography using (EtOAc 100 %) as eluent to give the target chemical (150 mg, 0.308 mmol, 57 %) as a clear oil.

¹H NMR (300 MHz, CDCl₃) δ 7.34 (d, J = 4.3 Hz, 4H), 7.32-7.28 (m, 1H), 4.57 (s, 2H), 4.02 (s, 2H), 3.74-3.68 (m, 5H), 3.68-3.60 (m, 20H), 1.47 (s, 9H).

### Step 4: Preparation of tert-butyl 20-hydroxy-3,6,9,12,15,18-hexaoxicosanoate

To a solution of the chemical prepared in example 100 step 3 (140 mg, 0.287 mmol) in EtOH (7 ml) was added Pd/C (10 wt %) (12 mg), and the resulting mixture was placed under hydrogen. The reaction mixture was stirred at room temperature overnight. The reaction mixture was filtered through Celite, and the Celite pad was washed several times with ethanol. The filtrate was concentrated in vacuo to give the target chemical (150 mg, 0.279 mmol, 97 %) as an oil.

¹H NMR (300 MHz, CDC13) δ 4.02 (s, 2H), 3.76-3.57 (m, 24H), 3.23 (s, 1H), 1.48 (s, 9H).

### Step 5: Preparation of 22,22-dimethyl-20-oxo-3,6,9,12,15,18,21-heptaoxaicosanoate

To a solution of the compound prepared in example 100 step 4 (111 mg, 0.297 mmol) in ACN/H2O (2 ml) was added BAIB (210 mg, 0.653 mmol), TEMPO (10 mg, 0.065 mmol), and the resulting mixture was cooled to room temperature was stirred for 5 hours. The reaction mixture was concentrated in vacuo. The crude mixture was purified by silica gel column chromatography using (DCM/MeOH = 5 %) as eluent to give the target chemical (30 mg, 0.073 mmol, 26 %) as a yellow oil.

¹H NMR (300 MHz, CDCl₃) δ 4.14 (s, 2H), 4.02 (s, 2H), 3.76-3.64 (m, 25H), 1.47 (s, 9H).

### Step 6: Preparation of tert-butyl (S)-22-((2R,4S)-4-hydroxy-2-((4-(4-methylthiazole-5-yl)benzyl)carbamoyl)pyrrolidin-1-carbonyl)-23,23-dimethyl-20-oxo-3,6,9,12,15,18-hexaoxa-21-azatetracosanoate

To a solution of the compound prepared in example 100 step 5 (30 mg, 0.073 mmol) in DMF was added VHL ligand (34 mg, 0.073 mmol), HATU (42 mg, 0.110 mmol), DIPEA (0.051 mL, 0.292 mmol), The mixture was stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 5-11 % to give the target chemical (34 mg, 0.041 mmol, 56 %) as a white solid.

¹H NMR (300 MHz, CDCl₃) δ 8.68 (s, 1H), 7.40-7.29 (m, 5H), 7.17 (d, J = 8.6 Hz, 1H), 4.72 (t, J = 8.0 Hz, 1H), 4.60-4.48 (m, 3H), 4.37 (dd, J = 15.0, 5.5 Hz, 1H), 4.11-3.94 (m, 5H), 3.70-3.62 (m, 21H), 3.14 (s, 1H), 2.52 (s, 3H), 2.50-2.43 (m, 1H), 2.23-2.11 (m, 1H), 1.47 (s, 9H), 0.96 (s, 9H).

### Step 7: Preparation of (S)-22-((2R,4S)-4-hydroxy-2-((4-(4-methylthiazole-5-yl)benzyl)carbamoyl)pyrrolidin-1-carbonyl)-23,23-dimethyl-20-oxo-3,6,9,12,15,18-hexaoxa-21-azatetracosanoate

To a solution of the compound prepared in example 100 step 6 (30 mg, 0.036 mmol) in DCM (1 ml), TFA (1 ml) was added, and the resulting mixture was stirred at room temperature for 1 hour. The reaction mixture was concentrated in vacuo to give the target chemical (30 mg, quant) as an oil.

¹H NMR (300 MHz, CDCl₃) δ 9.79 (s, 1H), 7.66 (d, J = 8.4 Hz, 1H), 7.55 (t, J = 6.3 Hz, 1H), 7.47 (d, J = 8.0 Hz, 2H), 7.39 (d, J = 8.0 Hz, 2H), 4.83-4.68 (m, 3H), 4.58 (d, J = 8.5 Hz, 1H), 4.36 (dd, J = 15.6, 5.1 Hz, 1H), 4.25 (d, J = 11.4 Hz, 1H), 4.19 (s, 2H), 4.13 (d, J = 6.5 Hz, 2H), 3.84 (dd, J = 11.4, 3.1 Hz, 1H), 3.78-3.63 (m, 20H), 2.64 (s, 3H), 2.47-2.28 (m, 2H), 1.02 (s, 9H).

### Step 8: Preparation of (2S,4R)-1-((S)-2-tert-butyl-23-(4-(4-(5-(furan-2-ylmethylamino)-[1,2,4]triazolo[4,3-f]pyrimidin-8-yl)phenyl)piperazin-1-yl)-4,23-dioxo-6,9,12,15,18,21-hexaoxa-3-azatricosan-1-oyl)-4-hydroxy-N-(4-(4-methylthiazol-5-yl)benzyl)pyrrolidin-2-carboxamide

N-(furan-2-ylmethyl)-8-(4-(piperazin-1-yl)phenyl)-[1,2,4]triazolo[4,3-f]pyrimidin-5-amine hydrochloride in DMF (15 mg, 0.036 mmol), the compound prepared in example 100 step 7 (28 mg, 0.036 mmol), HATU (21 mg, 0.054 mmol), and DIPEA (0.025 mL, 0.144 mmol) were added and stirred at room temperature for 2 hours.. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 6 % to give the final target chemical (6 mg, 0.005 mmol, 14 %) as a pale yellow solid.

¹H NMR (300 MHz, CD3OD) δ 8.87 (s, 1H), 8.40 (s, 1H), 8.09 (s, 1H), 7.81 (d, J = 8.6 Hz, 2H), 7.53-7.38 (m, 6H), 7.11 (d, J = 8.6 Hz, 2H), 6.43-6.33 (m, 2H), 4.84 (s, 2H), 4.72 (s, 1H), 4.63-4.55 (m, 2H), 4.55-4.49 (m, 2H), 4.43-4.33 (m, 3H), 4.09-4.04 (m, 2H), 3.93-3.76 (m, 5H), 3.76-3.62 (m, 26H), 3.61-3.55 (m, 1H), 3.31-3.23 (m, 4H), 2.47 (s, 3H), 2.28-2.02 (m, 4H), 1.06 (s, 9H) ; LC/MS (ESI) m/z 1124.0 [M+H]⁺, 1122.0 [M-H]⁻

### <Example 101> Preparation of (2S,4R)-1-((S)-2-(8-(3-(2-(4-(4-(5-(furan-2-ylmethylamino)-[1,2,4]triazolo[4,3-f]pyrimidin-8-yl)phenyl)piperazin-1-yl)-2-oxoethyl)phenoxy)octanamido)-3,3-dimethylbutanoyl)-4-hydroxy-N-(4-(4-methylthiazol-5-yl)benzyl)pyrrolidin-2-carboxamide

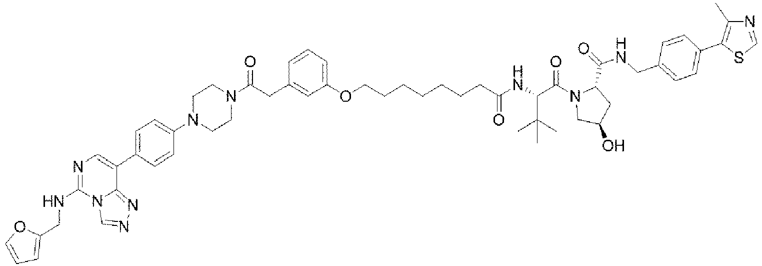

### Step 1: Preparation of tert-butyl 8-bromooctanoate

A solution of 8-bromooctanoate acid (5 g, 22 mmol, 1 eq.) in anhydrous THF (100 mL) was cooled to -40 °C and trifluoroacetic anhydride (6 mL, 44 mmol, 2 eq.) was slowly added. After stirring at -40 °C for 30 min, tert-butanol (29 mL, 300 mmol, 13.4 eq.) was added and the solution was warmed to room temperature and stirred for 16 h. The reaction mixture was poured slowly into a mixture of crushed ice and saturated NaHCO3 (aq). The aqueous layer was extracted with ethyl acetate and the collected organics were washed with brine, dried over MgSO4 and concentrated under reduced pressure. The crude mixture was purified by silica gel column chromatography to give the target chemical (2.7 g, 9.67 mmol, 44 %) as a clear oil.

¹H NMR (300 MHz, CDCl₃) δ 3.39 (t, J = 6.8 Hz, 2H), 2.19 (t, J = 7.4 Hz, 2H), 1.91-1.76 (m, 2H), 1.66-1.50 (m, 2H), 1.48-1.39 (m, 11H), 1.38-1.27 (m, 4H).

### Step 2: Preparation of tert-butyl 8-(3-(2-methoxy-2-oxoethyl)phenoxy)octanoate

A solution of methyl 2-(3-hydroxyphenyl)acetate (2 g, 12 mmol, 1 eq.) and K2CO3 (3.3 g, 24 mmol, 2 eq.) in DMF was stirred at room temperature for 30 min. The chemical prepared in example 101 step 1 (4.85 g, 12 mmol, 1 eq.) in acetone was added dropwise to the reaction mixture, followed by stirring at 85° C. overnight. The reaction solvent was removed. It was then diluted with water and ethyl acetate. The organic layer was removed and the aqueous layer was extracted with EtOAc. The organics were collected, washed with brine, dried over Na2SO4, the solvent was removed in vacuo, and the target chemical (1.7g, 4.66mmol, 39%) was purified by column chromatography.

¹H NMR (300 MHz, CDCl₃) δ 7.21 (t, J = 7.8 Hz, 1H), 6.87-6.76 (m, 3H), 3.93 (t, J = 6.5 Hz, 2H), 3.68 (s, 3H), 3.59 (s, 2H), 2.21 (t, J = 7.5 Hz, 2H), 1.75 (q, J = 6.6 Hz, 2H), 1.67-1.54 (m, 2H), 1.51-1.41 (m, 11H), 1.41-1.30 (m, 4H).

### Step 3: Preparation of 8-(3-(2-methoxy-2-oxoethyl)phenoxy)octanoate

TFA was added to the DCM solution of the chemical (1.7 g, 4.6 mmol) prepared in example 101 step 2. The reaction mixture was stirred at room temperature for 2 hours. After completion of the reaction, the mixture was extracted with EA, dried over Na2SO4, and purified by column chromatography using MeOH/DCM 5% eluent to give the target chemical (1.6 g, crude) as a clear oil.

¹H NMR (300 MHz, CDCl₃) δ 9.20 (s, 1H), 7.25 (t, J = 7.8 Hz, 1H), 6.91-6.78 (m, 3H), 3.97 (t, J = 6.5 Hz, 2H), 3.72 (s, 3H), 3.63 (s, 2H), 2.41 (t, J = 7.4 Hz, 2H), 1.80 (p, J = 6.7 Hz, 2H), 1.74-1.61 (m, 2H), 1.55-1.35 (m, 6H).

### Step 4: Preparation of methyl 2-(3-((8-(((S)-1-((2S,4R)-4-hydroxy-2-((4-(4-methylthiazole-5-yl)benzyl)carbamoyl)pyrrolidin-1-yl)-3,3-dimethyl-1-oxobutane-2-yl)amino)-8-oxooctyl)oxy)phenyl)acetate

Chemical prepared in example 101 step 3 (1.6 g, 2.9 mmol, 1 eq.), (2S,4R)-1-((S)-2-amino-3,3-dimethylbutanoyl)- in anhydrous DMF (11 mL) 4-hydroxy-N-(4-(4-methylthiazole-5-yl)benzyl)pyrrolidin-2-carboxamide hydrochloride (1.3 g, 2.9 mmol, 1 eq.) and DIPEA (2 mL, 11 mmol, 4 eq.) was added to HATU (1.6 g, 4.3 mmol, 1.5 eq) at 0 °C, and the resulting mixture was stirred at room temperature overnight. LC-MS showed the reaction was complete. The mixture was partitioned between EtOAc and water. The aqueous layer was extracted with EtOAc. The combined organic layers were collected, washed with brine, dried over Na2SO4 and concentrated under reduced pressure to give a crude residue, which was purified to give the target chemical (1.09 g, 1.52 mmol, 53%) as a white solid.

### Step 5: Preparation of methyl 2-(3-((8-(((S)-1-((2S,4R)-4-hydroxy-2-((4-(4-methylthiazole-5-yl)benzyl)carbamoyl)pyrrolidin-1-yl)-3,3-dimethyl-1-oxobutane-2-yl)amino)-8-oxooctyl)oxy)phenyl)acetate

The chemical prepared in example 101 step 4 (1.09 g, 1.5 mmol, 1 eq.) and lithium hydroxide hydrate (320 mg, 7.6 mmol, 5 eq. mL) was added. The resulting mixture was stirred at ambient temperature for 18 hours. The THF was removed by concentration. The residue was diluted with ice water and the pH was slowly adjusted to 2-3 with 3N HCl. The resulting suspension was filtered and washed with water. The solid was collected by filtration and dried in an oven to give the target chemical (1.09 g).

### Step 6: Preparation of Preparation of (2S,4R)-1-((S)-2-(8-(3-(2-(4-(4-(5-(furan-2-ylmethylamino)-[1,2,4]triazolor[4,3-f]pyrimidin-8-yl)phenyl)piperazin-1-yl)-2-oxoethyl)phenoxy)octanamido)-3,3-dimethylbutanoyl)-4-hydroxy-N-(4-(4-methylthiazol-5-yl)benzyl)pyrrolidin-2-carboxamide

N-(furan-2-ylmethyl)-8-(4-(piperazin-1-yl)phenyl)-[1,2,4]triazolo[4,3-f]pyrimidin-5-amine hydrochloride (20mg) in DMF , 0.039 mmol), the chemical prepared in example 101 step 5 (34 mg, 0.048 mmol), HATU (27 mg, 0.072 mmol), and DIPEA (0.033 mL, 0.192 mmol) were added and stirred at room temperature for 2 hours. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 4 % to give the target chemical (22 mg, 0.020 mmol, 43 %) as a pale yellow solid.

¹H NMR (300 MHz, CDCl₃) δ 8.67 (s, 1H), 8.30 (s, 1H), 8.10 (s, 1H), 7.85-7.79 (m, 2H), 7.42-7.39 (m, 1H), 7.38-7.30 (m, 5H), 7.24-7.18 (m, 1H), 7.00-6.94 (m, 2H), 6.84-6.79 (m, 2H), 6.79-6.73 (m, 1H), 6.52 (t, J = 5.8 Hz, 1H), 6.39-6.32 (m, 2H), 6.19 (d, J = 8.6 Hz, 1H), 4.86 (d, J = 5.7 Hz, 2H), 4.69 (t, J = 7.9 Hz, 1H), 4.58-4.47 (m, 3H), 4.36-4.28 (m, 1H), 4.09-4.03 (m, 1H), 3.91 (t, J = 6.5 Hz, 2H), 3.85-3.78 (m, 2H), 3.75 (s, 2H), 3.64-3.57 (m, 3H), 3.23-3.16 (m, 2H), 3.08-3.01 (m, 2H), 2.51 (s, 3H), 2.50-2.44 (m, 1H), 2.20-2.14 (m, 2H), 2.13-2.04 (m, 1H), 1.77-1.70 (m, 2H), 1.63-1.54 (m, 2H), 1.46-1.37 (m, 2H), 1.37-1.24 (m, 6H), 0.94 (s, 9H); LC/MS (ESI) m/z 1064.1 [M+H]⁺, 1062.0 [M-H]⁻

### <Example 102> Preparation of (2S,4R)-1-((S)-2-(2-(2-(2-(3-(2-(4-(4-(5-(furan-2-ylmethylamino)-[1,2,4]triazolo[4,3-f]pyrimidin-8-yl)phenyl)piperazin-1-yl)-2-oxoethyl)phenoxy)ethoxy)ethoxy)acetamido)-3,3-dimethylbutanoyl)-4-hydroxy-N-(4-(4-methylthiazol-5-yl)benzyl)pyrrolidin-2-carboxamide

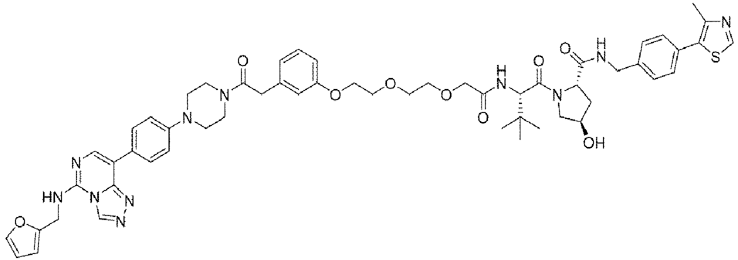

### Step 1: Preparation of 1-(2-(2-(benzyloxy)ethoxy)ethoxy)-3,3-dimethylbutane-2-on

In a solution of di(ethylene glycol)benzyl ether (9 mL, 54 mmol, 1 eq.) in DCM (64 mL), 37 % aq. NaOH (64 mL), tert-butylbromoacetate (30 mL, 204 mmol, 4 eq.), TBAB (17 g, 52 mmol, 1.02 eq.) were added at room temperature. The reaction mixture was stirred at room temperature overnight. The reaction mixture was diluted with water and extracted with EtOAc (50 mL×2). The combined organic layers were dried over MgSO4 and the solvent was removed in vacuo. The crude mixture was purified by silica gel column chromatography using (EtOAc 100 %) as eluent to give the target chemical (10.4 g, 35.3 mmol, 70 %) as a clear oil.

¹H NMR (300 MHz, CDCl₃) δ 7.38-7.33 (m, 3H), 7.33-7.26 (m, 1H), 4.58 (s, 2H), 4.05 (s, 2H), 3.80-3.61 (m, 8H), 1.49 (s, 9H).

### Step 2: Preparation of 1-(2-(2-hydroxyethoxy)ethoxy)-3,3-dimethylbutane-2-on

A mixture of the compound prepared in example 102 step 1 (10.4 g, 35 mmol, 1 eq.) and palladium on carbon [10 % (wt/vol), 416 mg] in MeOH (140 mL) under a hydrogen atmosphere (hydrogen balloon) Stir overnight at 40 °C. Palladium on carbon was removed by celite filtration and washed twice with MeOH. The combined filtrates were concentrated under reduced pressure to give the target chemical (6.81 g, 33.3 mmol, 94 %) as a colorless oil, which was used in the next step without further purification.

### Step 3: Preparation of 2-(2-(3,3-dimethyl-2-oxobutoxy)ethoxy)ethyl 4-methylbenzensulfonate

The chemical prepared in example 102 step 2 (6.8 g, 33 mmol, 1 eq.) and TsCl (7.6 g, 40 mmol, 1.2 eq.) in pyridine (18 mL) were stirred at room temperature for 1 h. The reaction mixture was partitioned between EtOAc and water. The organic layer was collected, washed with cold 1N HCl (aq.) (80 mL) followed by brine, dried over Na2SO4 and concentrated under reduced pressure to give a crude residue, which was purified by silica gel column chromatography. to give the target chemical (10.9 g, 30.4 mmol, 92 %) as a yellow oil.

¹H NMR (300 MHz, CDCl₃) δ 7.80 (d, J = 8.3 Hz, 2H), 7.34 (d, J = 8.1 Hz, 2H), 4.20-4.13 (m, 2H), 3.98 (s, 2H), 3.73-3.68 (m, 2H), 3.68-3.59 (m, 4H), 2.44 (s, 3H), 1.47 (s, 9H).

### Step 4: Preparation of tert-butyl 2-(2-(2-(3-(2-methoxy-2-oxoethyl)phenoxy)ethoxy)ethoxy)acetate

A solution of methyl 2-(3-hydroxyphenyl)acetate (1 g, 6 mmol, 1 eq.) and K2CO3 (1.7 g, 12 mmol, 2 eq.) in DMF was stirred at room temperature for 30 min. The chemical prepared in example 102 step 3 (2.4 g, 6 mmol, 1 eq.) in acetone was added dropwise to the reaction mixture, followed by stirring at 85° C. overnight. After removing the reaction solvent, it was diluted with water and EtOAc. The organic layers were combined, washed with brine, dried over Na2SO4, the solvent was removed in vacuo, and purified by column chromatography to give the target chemical (1.8g, 4.89mmol, 83%).

¹H NMR (300 MHz, CDCl₃) δ 7.28-7.20 (m, 1H), 6.91-6.82 (m, 3H), 4.16 (t, J = 4.9 Hz, 2H), 4.06 (s, 2H), 3.92-3.86 (m, 2H), 3.82-3.73 (m, 4H), 3.71 (s, 3H), 3.61 (s, 2H), 1.50 (s, 9H).

### Step 5: Preparation of 2methyl 2-(3-(2-(2-(2-tert-butoxy-2-oxoethoxy)ethoxy)ethoxy)phenyl)acetate

TFA was added to the DCM solution of the chemical (1.7 g, 4.6 mmol) prepared in example 102 step 4. The reaction mixture was stirred at room temperature for 2 hours. After the reaction was complete, the mixture was extracted with EtOAc, dried over Na2SO4 and purified by column chromatography using MeOH/DCM 5% eluent to give the title compound (2.5 g, crude) as a yellow oil.

### Step 6: Preparation of 2-(2-(2-(3-(2-methoxy-2-oxoethyl)phenoxy)ethoxy)ethoxy)acetate

(2S,4R)-1-((S)-2-amino-3,3-dimethylbutanoyl)-4-hydroxy-N-(4-(4-methylthiazole-5-yl)benzyl)pyrrolidin-2-carboxamide hydrochloride(150 mg, 0.3 mmol, 1eq.), in Anhydrous DMF (2 mL), HATU (183 mg, 0.5 mmol, 1.5eq), and DIPEA (0.2ml, 1.3 mmol, 4eq) and the chemical prepared in example 102 step 5 (100 mg, 0.3 mmol, leq.) were added and the resulting mixture was stirred at room temperature overnight. LC-MS showed the reaction was complete. The mixture was partitioned between EtOAc and water. The aqueous layer was extracted with EtOAc. The combined organic layers were collected, washed with brine, dried over Na2SO4 and concentrated under reduced pressure to give a crude residue, which was purified to give the target chemical (124mg, 0.171mmol, 53%) as a white solid.

¹H NMR (300 MHz, CDCl₃) δ 8.72 (d, J = 2.9 Hz, 1H), 7.42 (t, J = 6.0 Hz, 1H), 7.39-7.29 (m, 4H), 7.20 (t, J = 7.9 Hz, 1H), 6.88-6.75 (m, 3H), 4.70 (q, J = 6.7, 5.6 Hz, 1H), 4.65-4.46 (m, 3H), 4.39-4.27 (m, 2H), 4.20-4.08 (m, 2H), 4.06 (s, 1H), 4.04-3.96 (m, 3H), 3.85 (t, J = 4.9 Hz, 2H), 3.78-3.59 (m, 10H), 3.57 (s, 2H), 3.46 (s, 2H), 2.51 (s, 3H), 2.48-2.41 (m, 1H), 2.18-2.06 (m, 1H), 1.54-1.45 (m, 1H), 1.43 (d, J = 6.5 Hz, 1H), 1.29-1.19 (m, 1H), 0.95 (s, 9H)

### Step 7: Preparation of Preparation of (2S,4R)-1-((S)-2-(2-(2-(2-(3-(2-(4-(4-(5-(furan-2-ylmethylamino)-[1,2,4]triazolo[4,3-f]pyrimidin-8-yl)phenyl)piperazin-1-yl)-2-oxoethyl)phenoxy)ethoxy)ethoxy)acetamido)-3,3-dimethylbutanoyl)-4-hydroxy-N-(4-(4-methylthiazol-5-yl)benzyl)pyrrolidin-2-carboxamide

N-(furan-2-ylmethyl)-8-(4-(piperazin-1-yl)phenyl)-[1,2,4]triazolo[4,3-f]pyrimidin-5-amine hydrochloride in DMF (20 mg, 0.039 mmol), HATU (27 mg, 0.072 mmol), DIPEA (0.033 mL, 0.192 mmol), and the chemical prepared in example 102 step 6 (34 mg, 0.048 mmol) were added at room temperature for 2 hours. stirred. The reaction mixture was diluted with water and extracted with EtOAc. The combined organic layers were washed with brine, dried over MgSO4 and the solvent removed in vacuo to give an oil. The crude mixture was purified by silica gel column chromatography using MeOH/DCM = 7 % to give the title compound (6 mg, 0.005 mmol, 12 %) as an ivory solid.

¹H NMR (500 MHz, CDCl₃) δ 8.67 (s, 1H), 8.30 (s, 1H), 8.10 (s, 1H), 7.85-7.81 (m, 2H), 7.42-7.40 (m, 1H), 7.39-7.30 (m, 6H), 7.20 (t, J = 7.9 Hz, 1H), 7.01-6.95 (m, 2H), 6.86-6.81 (m, 2H), 6.80-6.75 (m, 1H), 6.46 (t, J = 5.8 Hz, 1H), 6.39-6.35 (m, 2H), 4.87 (d, J = 5.6 Hz, 2H), 4.71 (t, J = 7.9 Hz, 1H), 4.58-4.46 (m, 3H), 4.36-4.29 (m, 1H), 4.14-4.09 (m, 2H), 4.09-4.05 (m, 1H), 4.04-3.96 (m, 2H), 3.84 (t, J = 4.8 Hz, 2H), 3.83-3.77 (m, 2H), 3.75-3.72 (m, 4H), 3.72-3.68 (m, 2H), 3.63-3.58 (m, 3H), 3.50-3.46 (m, 1H), 3.21-3.17 (m, 2H), 3.08-3.03 (m, 2H), 2.56-2.51 (m, 1H), 2.51 (s, 3H), 2.13-2.06 (m, 1H), 0.94 (s, 9H) ; LC/MS (ESI) m/z 1068.0 [M+H]⁺, 1065.8 [M-H]⁻

The compounds of Table 1 below were additionally prepared in the same or similar manner to Examples 1 to 102, and their analysis data are shown in Table 2.

**[Table 1]**

| **Example** | **Structure** |
|---|---|
| **103** | 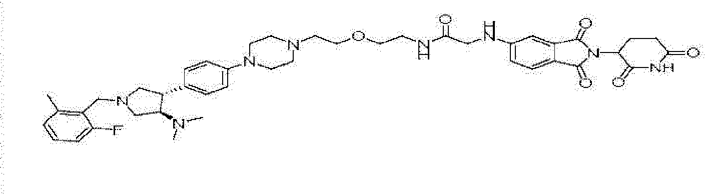 |
| **104** | 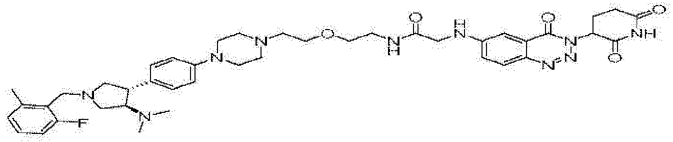 |
| **105** | 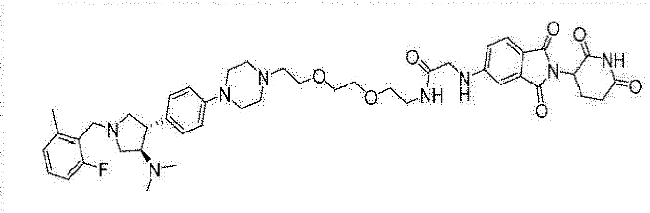 |
| **106** | 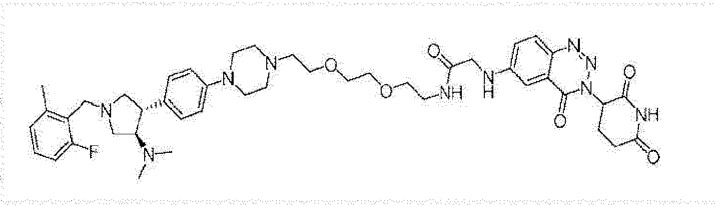 |
| **107** | 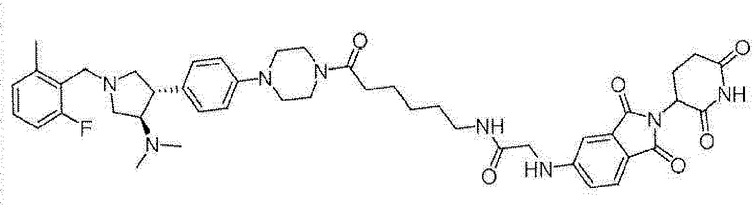 |
| **108** | 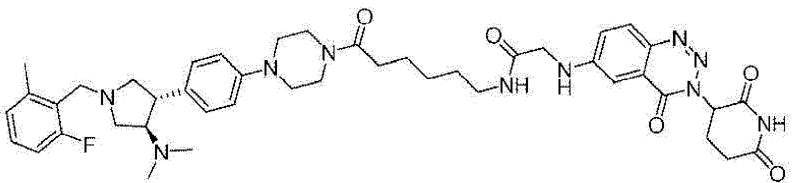 |
| **109** | 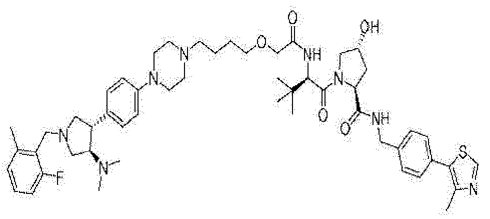 |
| **110** | 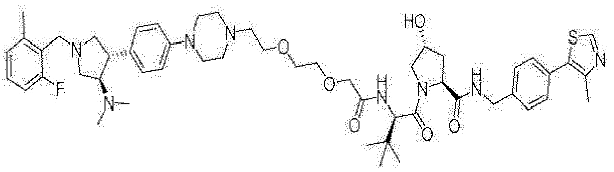 |
| **111** | 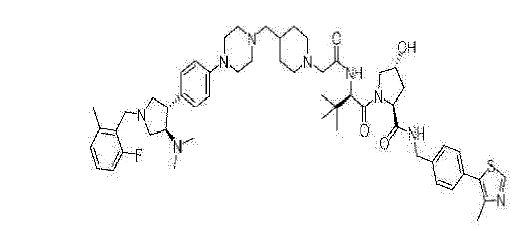 |
| **112** | 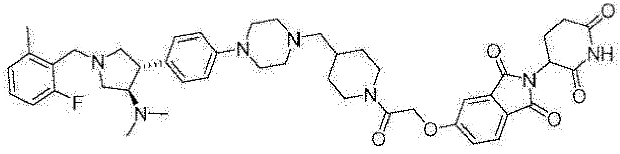 |
| **113** | 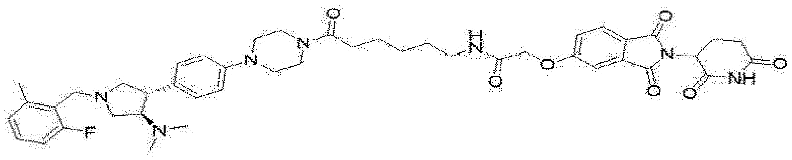 |
| **114** | 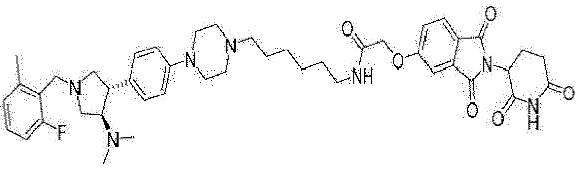 |
| **115** | 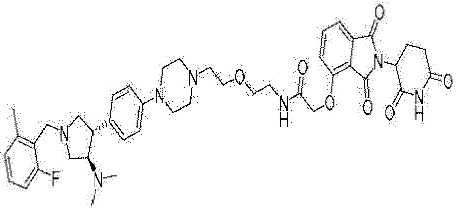 |
| **116** | 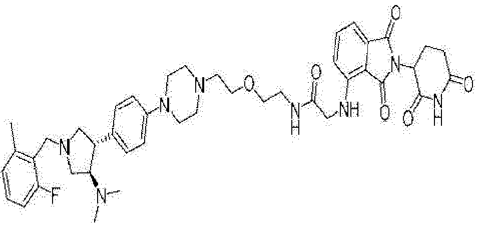 |
| **117** | 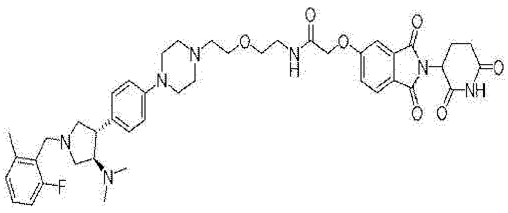 |
| **118** | 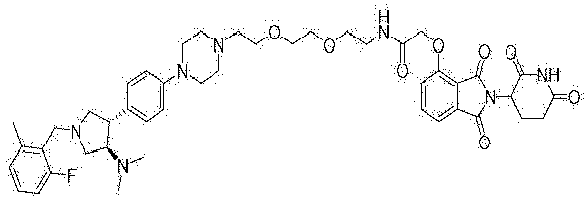 |
| **119** | 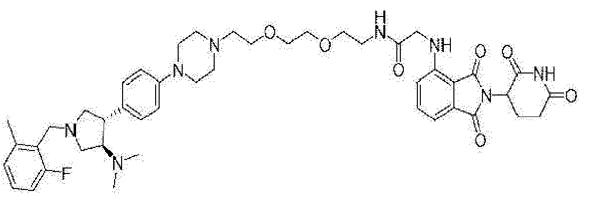 |
| **120** | 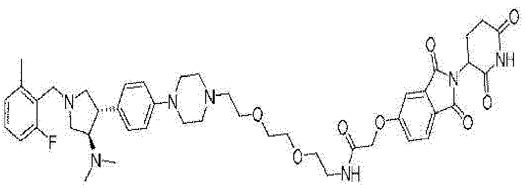 |
| **121** | 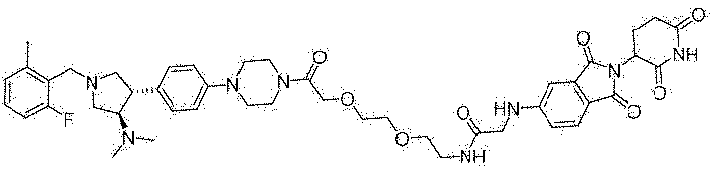 |
| **122** | 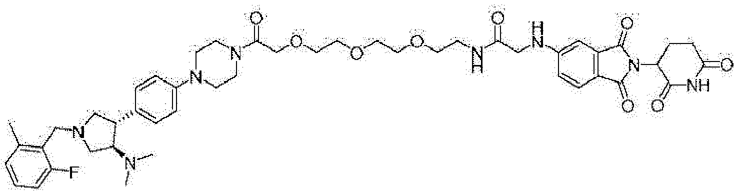 |
| **123** | 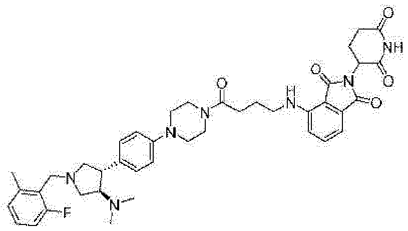 |
| **124** | 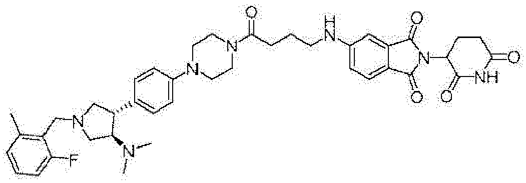 |
| **125** | 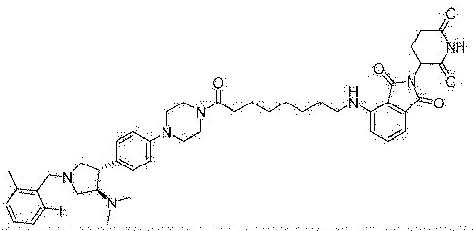 |
| **126** | 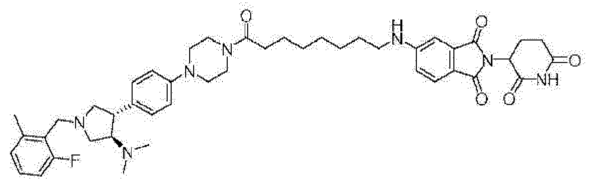 |
| **127** | 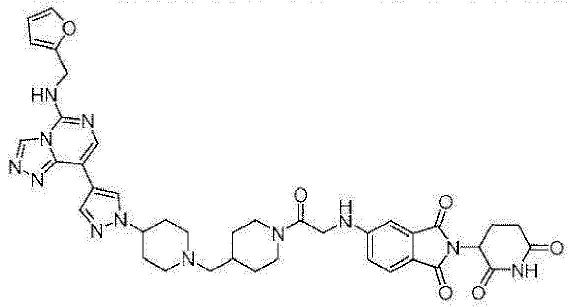 |
| **128** | 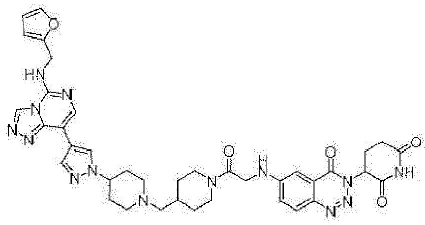 |
| **129** | 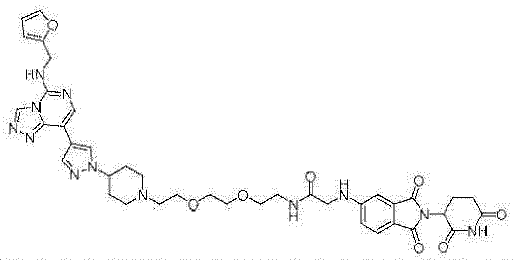 |
| **130** | 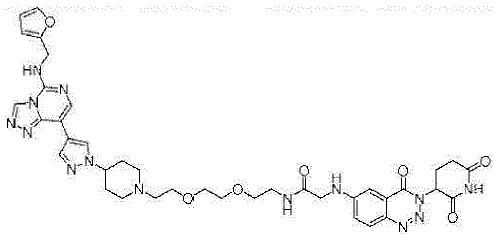 |
| **131** | 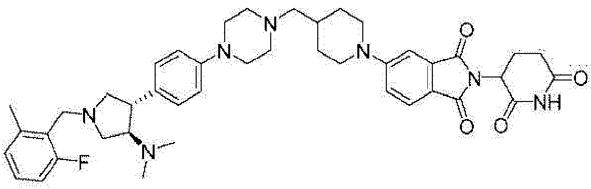 |
| **132** | 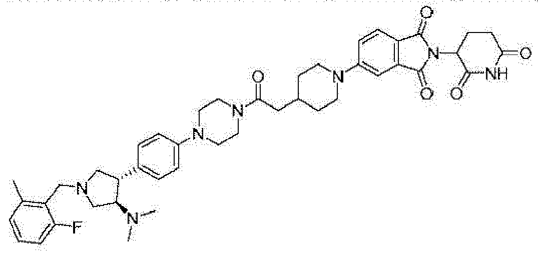 |
| **133** | 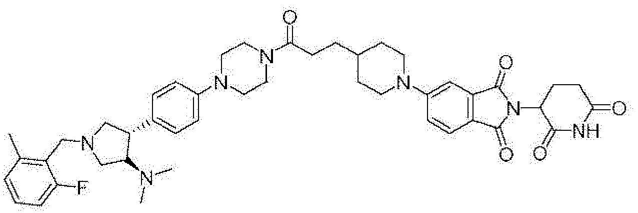 |
| **134** | 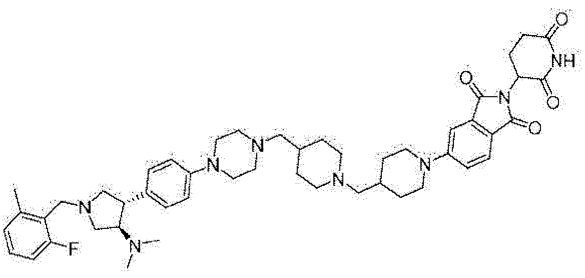 |
| **135** | 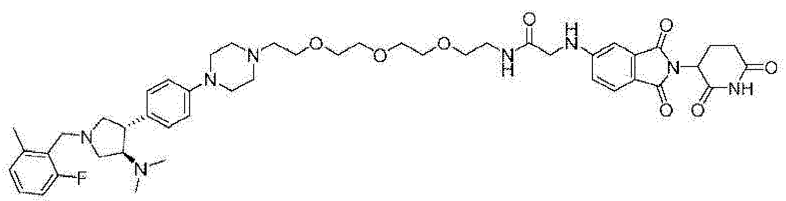 |
| **136** | 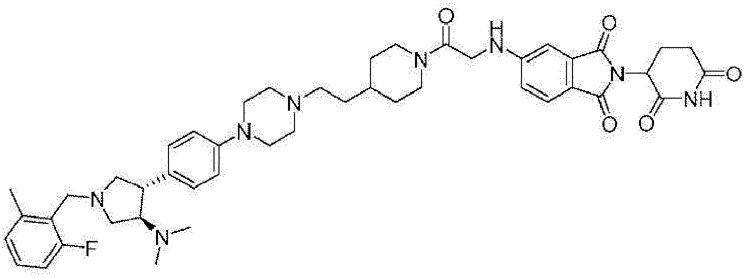 |
| **137** | 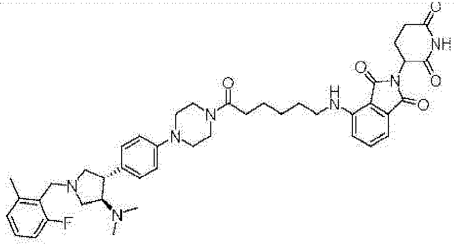 |
| **138** | 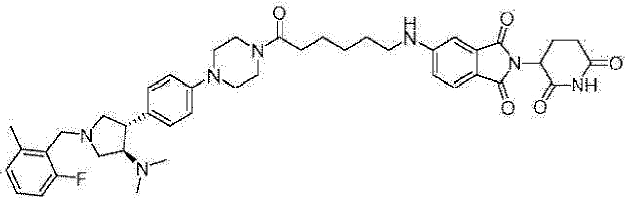 |
| **139** | 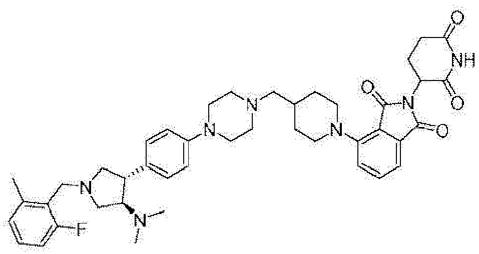 |
| **140** | 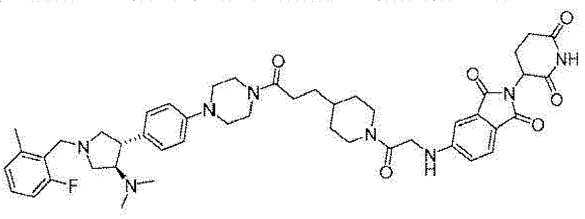 |
| **141** | 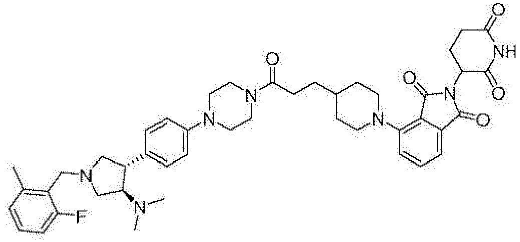 |
| **142** | 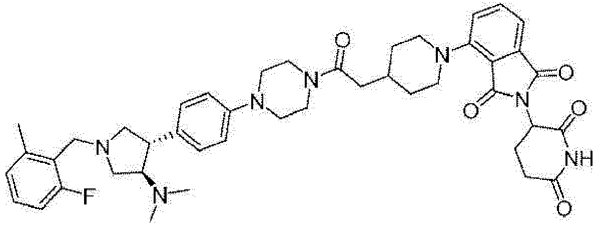 |
| **143** | 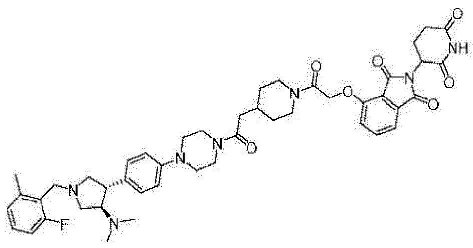 |
| **144** | 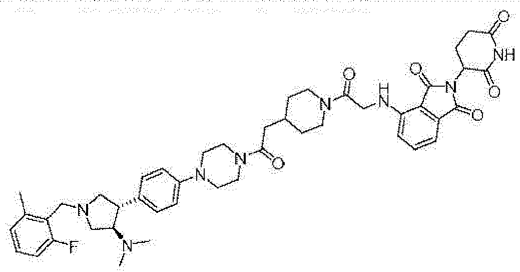 |
| **145** | 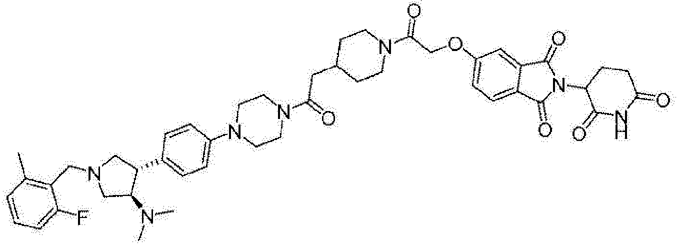 |
| **146** | 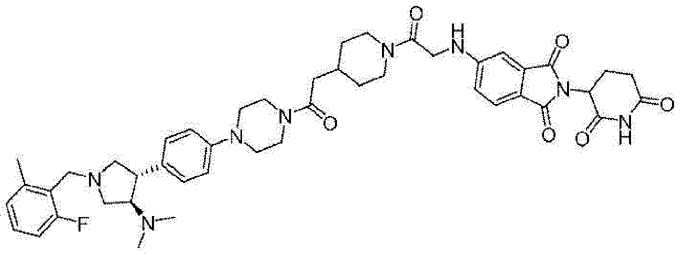 |
| **147** | 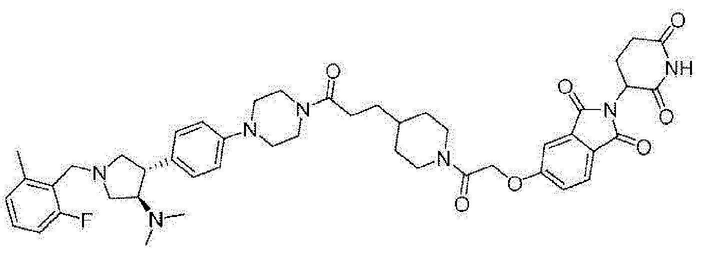 |
| **148** | 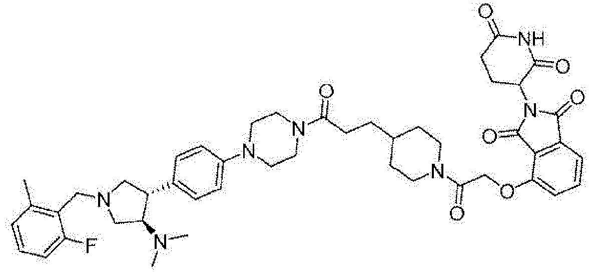 |
| **149** | 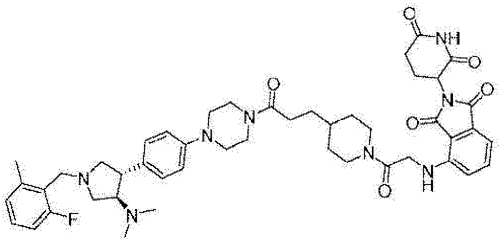 |
| **150** | 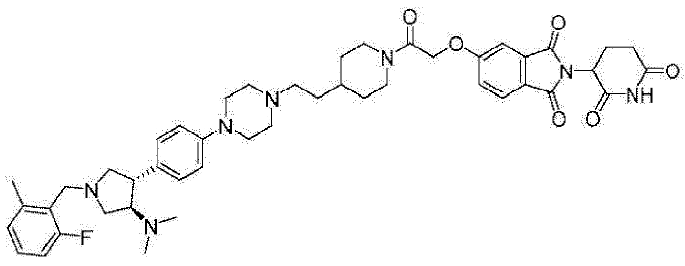 |
| **151** | 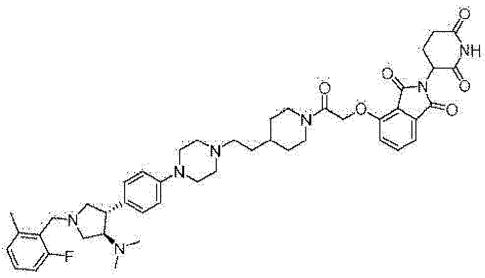 |
| **152** | 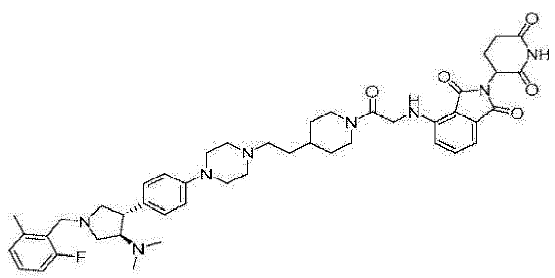 |
| **153** | 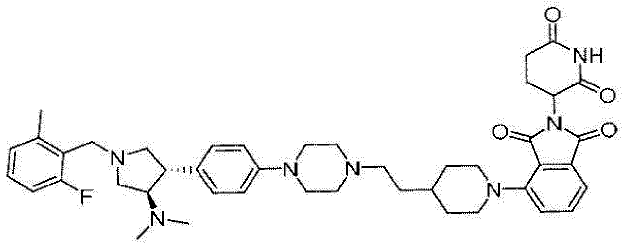 |
| **154** | 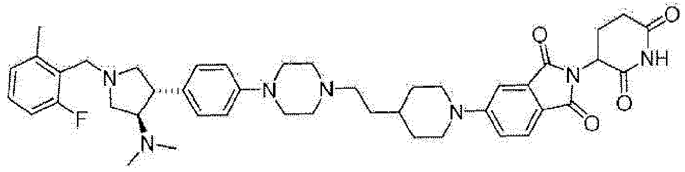 |
| **155** | 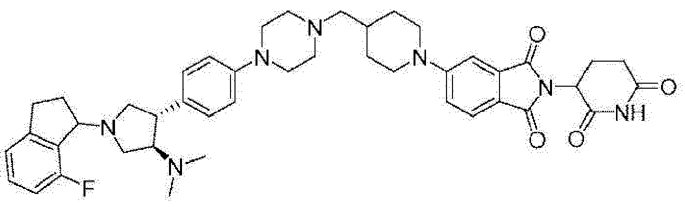 |
| **156** | 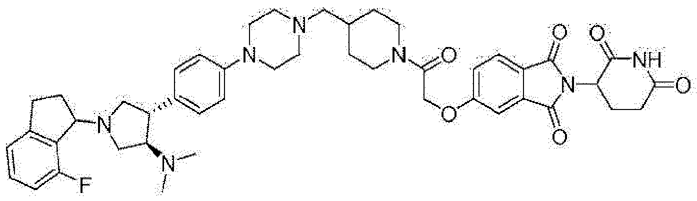 |
| **157** | 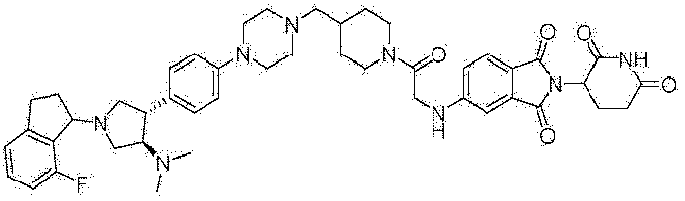 |
| **158** | 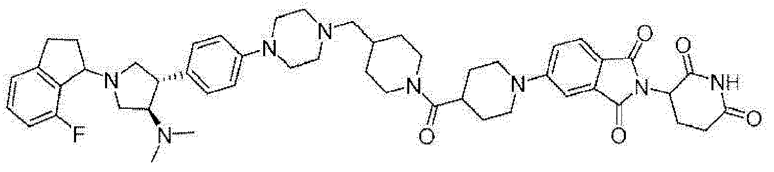 |
| **159** | 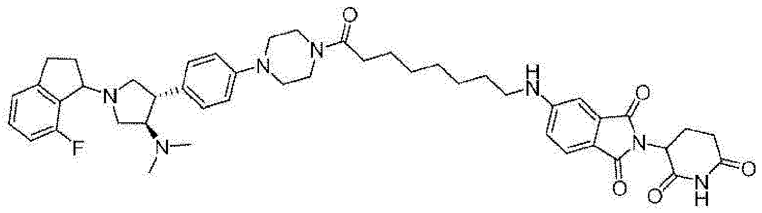 |
| **160** | 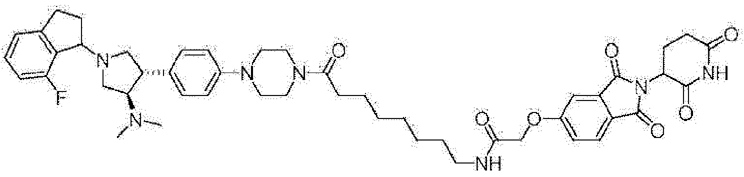 |
| **161** | 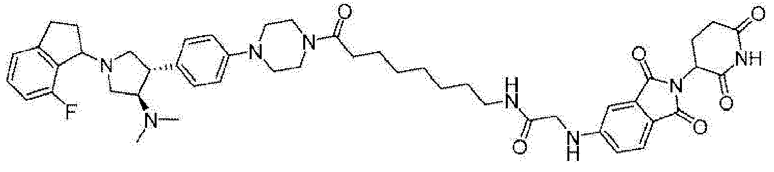 |
| **162** | 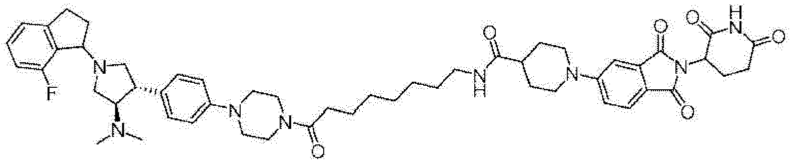 |
| **163** | 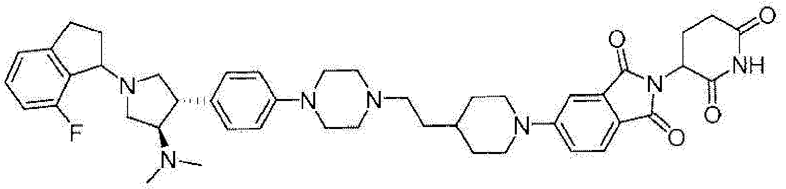 |
| **164** | 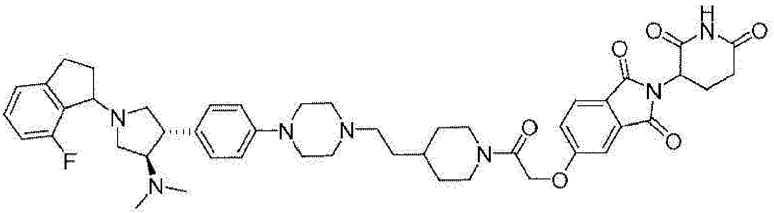 |
| **165** | 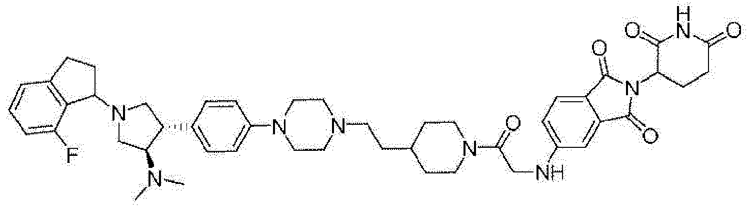 |
| **166** | 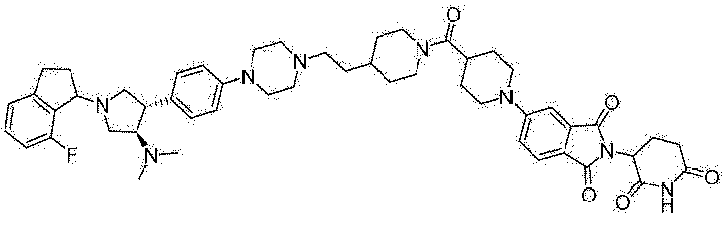 |
| **167** | 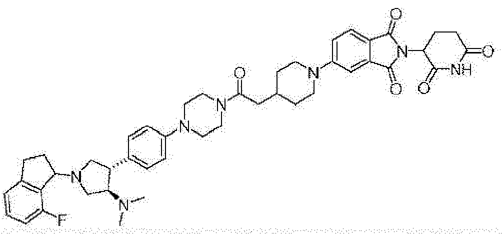 |
| **168** | 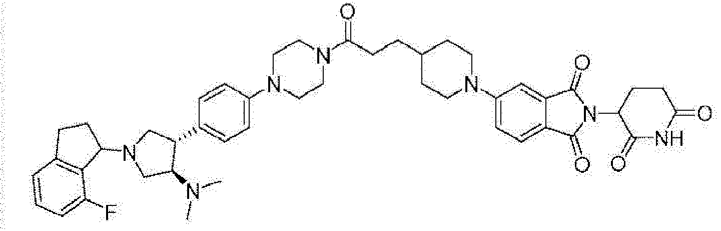 |
| **169** | 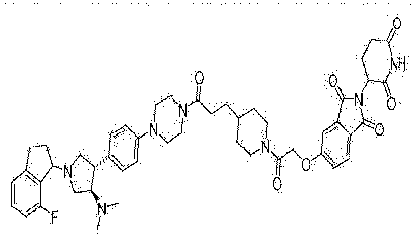 |
| **170** | 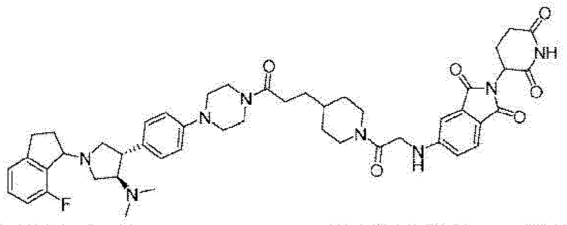 |
| **171** | 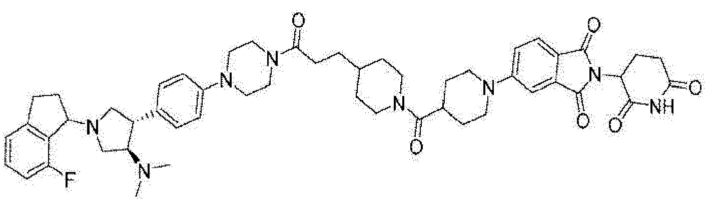 |
| **172** | 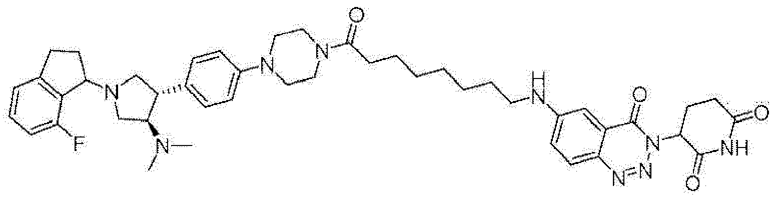 |
| **173** | 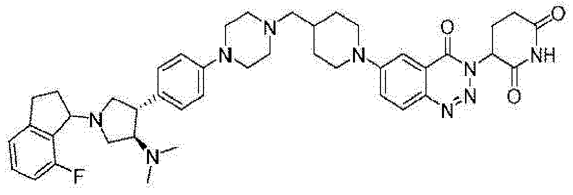 |
| 1**74** | 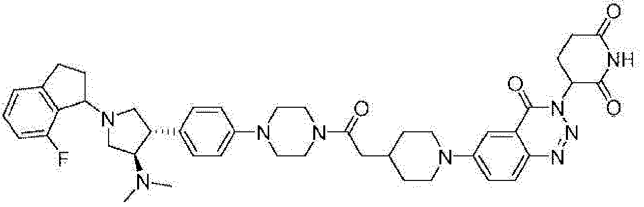 |
| **175** | 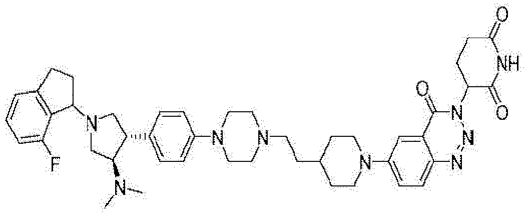 |
| **176** | 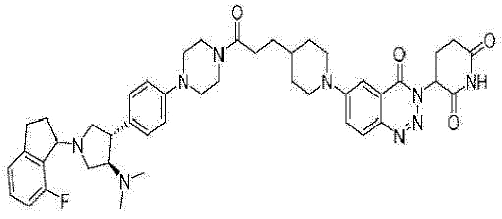 |
| **177** | 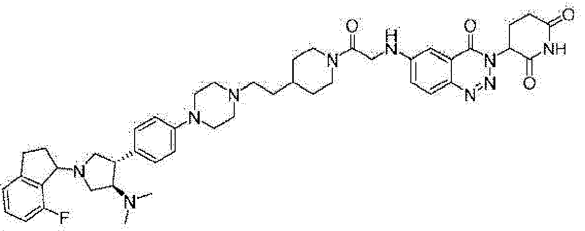 |
| **178** | 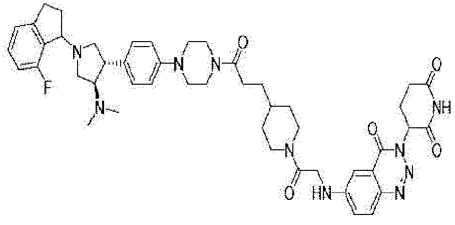 |
| **179** | 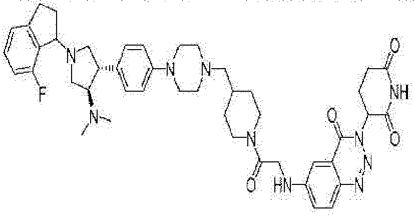 |
| **180** | 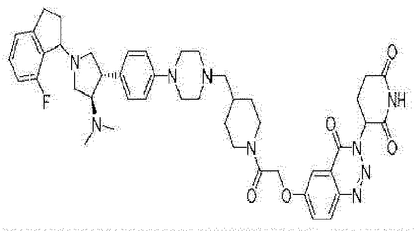 |

**[Table 2]**

| Exa mple | Analytical Data |
|---|---|
| 103 | 1H NMR (400 MHz, MeOD) δ 7.63 (d. J = 8.3 Hz, 1H), 7.23 (d, J = 8.3 Hz, 2H), 7.17 (q, J = 7.4 Hz, 1H), 7.06 - 7.00 (m, 2H), 6.89 (m. 4H), 5.05 (m, 1H), 3.94 (s. 2H), 3.68 (s. 2H), 3.58 (t, J = 5.5 Hz, 2H), 3,52 (t, J = 5,2 Hz, 2H), 3.44 (t, J = 5.2 Hz, 2H). 3.13 (m. 5H), 3.00 (m, 4H), 2:33 - 2.66 (m, 4H). 2.64 (t. J = 5.1 Hz, 4H), 2.59 - 2.45 (m, 6H), 2.19 (s, 6H). |
| 104 | 1H NMR (400 MHz. MeOD) δ 7.92 (d, J = 8.9 Hz, 1H), 7.34 (dd, J = 8.9, 2.6 Hz, 1H), 7.23 - 7.11 (m, 4H), 7.00 (d, J = 7.6 Hz, 1H), 6.91 - 6.82 (m. 3H), 5,93 - 5.79 (m. 1H), 3.97 (s. 2H). 3,66 (s. 2H). 3.56 (t, J = 5.5 Hz. 2H), 3,51 (t, J = 5.2 Hz, 2H), 3.44 (t, J = 5.2 Hz, 2H), 3.11 (m, 5H), 3,06 - 2.74 (m, 9H), 2.61 (m, 5H), 2.52 (t, J = 5.5 Hz, 3H). 2.47 (s, 3H). 2.31 (m, 1H), 2.19 (s, 6H). |
| 105 | 1H NMR (400 MHz, MeOH-d4) δ 7.63 (d, J = 8.3 Hz, 1H), 7.26 - 7.12 (m, 3H). 7.05 - 6.98 (m, 2H), 6.89 (m, 4H), 5.06 (dd, J = 12.4, 5.4 Hz, 1H), 3.94 (s, 2H), 3.72 - 3.61 (m, 4H), 3.60 - 3.52 (m, 5H), 3.43 (t, J = 5.3 Hz, 2H), 3.13 (m, 7H), 3.03 - 2.95 (m. 2H), 2.90 - 2.74 (m, 3H). 2.68 (m, 8H), 2.54 - 2.50 (m. 1H), 2.48 (s, 3H), 2.24 (s, 6H). |
| 106 | 1H NMR (400 MHz, MeOD) δ 7.94 (s, 1H), 7.34 (dd, J = 9.0, 2.7 Hz, 1H), 7.25 - 7.18 (m. 2H), 7.17 (m, 2H), 7.02 (d, J = 7.6 Hz. 1H), 6.90 (m, 3H), 5.91 - 5.83 (m, 1H), 3.98 (s, 2H), 3.70 - 3.66 (m, 2H), 3.62 (t, J = 5.7 Hz, 2H), 3.53 (m, 5H), 3.43 (t, J = 5.3 Hz, 2H), 3.37 (s, 3H), 3.15 (m, 4H), 3.00 (m, 3H), 2.92 - 2,77 (m, 3H), 2.69 (t, J = 5.0 Hz, 4H), 2.62 (t, J = 5.7 Hz, 2H), 2.54 - 2.50 (m, 1H), 2.48 (s, 3H), 2.36 - 2.29 (m. 1H), 2.20 (s. 6H). |
| 107 | 1H NMR (500 MHz, MeOD) δ 7.60 (d. J = 8.3 Hz. 1H), 7.25 - 7.20 (m, 2H), 7.18 - 7.10 (m, 1H), 7.00 (d, J = 7.6 Hz, 1H), 6.96 (m. 1H), 6.94 - 6.89 (m, 2H), 6.86 (d, J = 8.7 Hz, 2H), 5.02 (dd, J = 12.6. 5.5 Hz. 1H), 3.89 (s, 2H), 3.67 (m, 4H), 3.62 (t, J = 5.1 Hz. 2H), 3.30 (m. 2H), 3.21 (m. 4H), 3.10 (t, J = 5.2 Hz, 2H), 3.06 (t, J = 5.5 Hz, 2H), 3.00 (q, J = 7.5, 6.5 Hz, 2H), 2.72 (m, 3H), 2.48 (t, J = 7.6 Hz, 1H), 2.45 (s, 3H), 2.35 (t, J = 7.6 Hz, 2H), 2.31 (s, 6H). 2.06 (m, 1H), 1.57 (m, 2H), 1.50 (m, 2H), 1.29 (m,2H). |
| 108 | 1H NMR (400 MHz, MeOD) δ 7.95 (d, J = 8,9 Hz, 1H), 7.40 - 7.32 (m, 1H), 7.24 (d, J = 8.2 Hz, 2H), 7.17 (m, 2H), 7.02 (d, J = 7.6 Hz, 1H), 6.90 (dd, J = 15.8, 8.5 Hz, 3H), 5.87 (m, 1H), 3.96 (s. 2H), 3.69 (m. 4H). 3.62 (t, J = 5.3 Hz, 2H), 3.26 (t, J = 6.7 Hz, 2H), 3.16 (m, 2H), 3.10 (m, 4H), 3.00 (m, 2H), 2.91 (m, 2H), 2.82 (t, J = 13.9 Hz, 2H), 2.66 (dd, J = 9.6, 5.5 Hz, 1H) 2.55 - 2.50 (m, 1H), 2.49 (s, 3H), 2.35 (t, J = 7.5 Hz, 2H), 2.22 (s, 6H). |
| 109 | 1H NMR (300 MHz, CDC13) δ 8.66 (s, 1H), 7.41 - 7.28 (m. 4H), 7.18 (d, J = 8.5 Hz, 3H), 7.10 (dd, J = 8.0, 5.7 Hz, 1H), 6.95 (d, J = 7.5 Hz, 1H), 6.82 (m. 38), 4.74 (t, J = 7.9 Hz, 1H), 4,53 (m, 3H), 4.33 (dd, J = 15.0, 5.2 Hz, 1H), 4.10 (d, J. = 11.5 Hz, 1H), 3.97 (d, J = 15.4 Hz, 1H), 3.89 (d, J = 15.4 Hz. 1H), 3.61 (m, 3H), 3.52 (dd, J = 12.7, 6.6 Hz, 2H), 3.16 (m, 5H), 2.97 (m, 2H), 2.76 (m, 1H), 2.60 (m. 4H), 2.55 (d, J = 7.5 Hz. 1H), 2.51 (m, 4H), 2.43 (m, 5H), 2.28 (s, 6H), 2.19 - 2.05 (m, 2H), 2.08 - 1.95 (m, 2H). 1.25 (m, 4H), 0.88 (s, 9H). |
| 110 | 1H NMR (300 MHz, CDCl3) δ 8.67 (s, 1H), 7.33 (m, 5H), 7.22 ― 7.14 (m, 2H), 7.10 (dd, J = 7.9, 5.7 Hz, 1H), 6.95 (d, J = 7.5 Hz, 1H), 6.89 ― 6.77 (m, 3H), 4.77 ― 4.65 (m, 1H), 4.61 ― 4.45 (m, 3H), 4.32 (dd, J = 14.8, 5.4 Hz, 1H), 4.06 (m, 3H), 3.73 ― 3.55 (m, 9H), 3.15 (t, J = 5.1 Hz, 5H), 3.05 (m, 1H), 3.01 ― 2.91 (m, 2H), 2.73 (m, 1H), 2.65 (t, J = 5.3 Hz, 6H), 2.53 (d, J = 5.8 Hz, 1H), 2.51 (s, 3H), 2.47 (m, 1H), 2.44 (s, 3H), 2.24 (s, 6H), 2.15 ― 2.07 (m, 1H), 2.03 (d, J = 6.3 Hz, 1H), 1.25 (m, 2H), 0.95 (s, 9H). |
| 111 | 1H NMR (400 MHz, CDCl3) *δ* 8.68 (s, 1H), 7.91 (d, J = 8.2 Hz, 1H), 7.36 (m, 6H), 7.17 (d, J = 8.3 Hz, 2H), 7.12 (m, 1H), 6.95 (d, J = 7.6 Hz, 1H), 6.90 - 6.78 (m, 3H), 4.76 (t, J = 7.9 Hz, 1H), 4.61 ― 4.49 (m, 2H), 4.42 ― 4.30 (m, 2H), 4.18 (d, J = 11.5 Hz, 1H), 3.66 ― 3.54 (m, 3H), 30.26 ― 3,10 (m, 5H), 2.99 (m, 4H), 2.84 (d, J = 11.1 Hz, 2H), 2.53 (m, 9H), 2.43 (s, 3H), 2.32 (s, 6H), 2.23 (d, J = 7.2 Hz, 2H), 2.13 (m, 3H), 2.06 ― 1.97 (m, 2H), 1.79 (m, 3H), 1.20 (m, 1H), 1.13 ― 1.04 (m, 1H), 0.95 (s, 9H). |
| 112 | 1H NMR (400 MHz, CDCl3) δ 8.17 (s, 1H), 7.80 (d, J = 8.3 Hz, 1H), 7.34 (d, J = 23 Hz, 1H), 7.30 (dd, J = 8.3, 2.4 Hz, 1H), 7.17 (d, J = 8.3 Hz, 2H), 7.16 ― 7.07 (m, 1H), 6.95. (d, J = 7.6 Hz, 1H), 6.85 (t, J = 9.2 Hz, 3H), 4.95 (dd, J = 12.1, 5.2 Hz, 1H), 4.84 (d, J = 3.5 Hz, 2H), 4.55 (d, J = 13.4 Hz, 1H), 3.85 (d, J = 13.5 Hz, 1H), 3.63 (s, 2H), 3.22 (m, 2H), 3.15 (t, J = 4.9 Hz, 4H), 3.11 ― 3.01 (m, 2H), 3.00 ― 2.90 (m, 2H), 2.89 ― 2.80 (m, 2H), 2.79 ― 2.69 (m, 2H), 2.65 (m, 1H), 2.54 (m, 6H), 2.43 (s, 3H), 2.35 (s, 6H), 2.24 (d, J = 6.9 Hz, 2H), 2.17 ― 2.11 (m, 1H), 2.03 (d, J = 7.9 Hz, 1H), 1.93 (d, J = 13.2 Hz, 1H), 1.84 (m, 2H), 1.14 (m, 2H). |
| 113 | 1H NMR (300 MHz, CDCl3) δ 7.84 (d, J = 8.3 Hz, 1H), 7.42 (d, J = 2.3 Hz, 1H), 7.28 (dd J = 2.3, 0.9 Hz, 1H), 7.25 (dd, J = 2.3, 0.9 Hz, 1H), 7,20 (d, J = 8.5 Hz, 2H), 7.12 (m, 1H), 6.95 (d, J = 7.5 Hz, 1H), 6.89 ― 6.81 (m, 3H), 6.75 (t, J = 6.1 Hz, 1H), 4.95 (m, 1H), 4.60 (s, 2H), 3.76 (t, J = 5.1 Hz, 2H), 3.62 (m, 4H), 3.40 (q, J = 6.7 Hz, 2H), 3.24 (m, 2H), 3.18 ― 3.04 (m, 5H), 3.02 ― 2.81 (m, 4H), 2.78 ― 2.68 (m, 1H), 2.52 (m, 1H), 2.43 (s, 3H), 2.37 (m, 8H), 2.18 ― 2.10 (m, 1H), 1.75 ― 1.65 (m, 2H), 1.60 (q, J = 7.3 Hz, 2H), 1.42 (q, J = 8.2 Hz, 2H). |
| 114 | 1H NMR (400 MHz, CDCl3) δ 7.84 (d, J = 8.3 Hz, 1H), 7.40 (d, J = 2.3 Hz, 1H), 7.27 ms, 1H), 7.25 (m, 1H), 7.17 (d, J = 8.3 Hz, 2H), 7.15 ― 7.08 (m, 1H), 6.95 (d, J = 7.6 Hz, 1H), 6.85 (t, J = 9.1 Hz, 3H), 6.50 (m, 1H), 4.96 (dd, J = 12.1, 5.3 Hz, 1H), 4.61 (s, 2H), 3.64 (d, J = 3.4 Hz, 2H), 3.37 (q, J = 6.9 Hz, 2H), 3.30 ― 3.13 (m, 6H), 3.10 ― 2.91 (m, 3H), 2.90 ― 2.72 (m, 4H), 2.65 (m, 4H), 2.51 (t, J = 7.6 Hz, 1H), 2.42 (m, 4H), 2.37 (m, 6H), 2.19 ― 2.11 (m, 1H), 2.03 (d, J = 7.9 Hz, 1H), 1.25 (s, 6H), |
| 115 | 1H NMR (400 MHz, MeOD) δ 7.77 (t, J = 7.8 Hz, 1H), 7.48 (d, J = 7.3 Hz, 1H), 7.43 (d, J = 8.5 Hz, 1H), 7.19 (dd, J = 8.1, 5.8 Hz, 3H), 7.03 (d, J = 7.6 Hz, 1H), 6.91 (t, J = 9.1 Hz, 1H), 6.81 (d, J = 8.3 Hz, 2H), 5.11 (m, 1H), 4.79 .(s, 2H), 3.70 (m, 3H), 3.66 ― 3.60 (m, 2H), 3.55 (t, J = 5.1 Hz, 2H), 3.37 (s, 2H), 3.23 (m, 1H), 3.10 (m, 4H), 3.03 (m, 2H), 2.90 ― 2.63 (m, 10H), 2.55 ― 2.50 (m, 1H) 2.48 (s, 3H), 2.40 (s, 6H), 2.13 ― 2.01 (m, 2H). |
| 116 | 1H NMR (400 MHz, MeOD-d4) δ 7.58 (dd, J = 8.5, 7.2 Hz, 1H), 7.27 ― 7.20 (m, 2H), 7.17 (dd, J = 7.9, 5.8 Hz, 1H), 7.12 (d, J = 7.1 Hz, 1H), 7.02 (d, J = 7.4 Hz, 1H), 6.90 (t, J = 8.6 Hz, 4H), 5.04 (m, 1H), 4.03 (d, J = 4.5 Hz, 2H), 3.74 ― 3.67 (m, 2H), 3.66 ― 3.59 (m, 3H), 3.54 (t, J = 5.2 Hz, 2H), 3.45 (t, J = 5.4 Hz, 2H), 3.31 (m, 1H), 3.23 (q, J = 6.4 Hz, 1H), 3.15 (t, J = 5.0 Hz, 4H), 3.10 ― 2.98 (m, 2H), 2.82 ― 2.73 (m, 2H), 2.70 (m, 5H), 2.63 (t, J = 5.3 Hz, 2H), 2.55 ― 2.50 (m, 1H), 2.48 (s, 3H), 2.39 (s, 6H), 2.04 (m, 1H). |
| 117 | 1H NMR (400 MHz, MeOD) δ 7.77 (d, J = 8.3 Hz, 1H), 7.50 (m, 1H), 7.41 (dd, J = 8.3, 2.3 Hz, 1H), 7.19 (m, 3H), 7.02 (d, J = 7.6 Hz, 1H), 6.90 (t, J = 9.1 Hz, 1H), 6.80 (dd, J = 8.7, 3.2 Hz, 2H), 5.11 (dd, J = 12.5, 5.4 Hz, 1H), 4.73 (s, 2H), 3.70 (m, 2H), 3.68 ― 3.64 (m, 2H), 3.59 (t, J = 5.3 Hz, 2H), 3.52 (t, J = 5.4 Hz, 2H), 3.24 (m, 2H), 3.13 (t, J = 4.8 Hz, 4H), 3.02 (m, 2H), 2.84 (m, 1H), 2.74 (m, 6H), 2.68 (t, J = 5.2 Hz, 2H), 2.51 (d, J = 7.9 Hz, 1H), 2.48 (s, 3H), 2.35 (s, 6H), 2.16 ― 2.08 (m, 1H), 2.04 (m, 1H). |
| 118 | 1H NMR (400 MHz, MeOR-d4) δ 7.80 (t, J = 7.9 Hz, 1H), 7.53 (d, J = 7.3 Hz, 1H), 7.43 (d, J = 8.4 Hz, 1H), 7.25 (d, J = 8.4 Hz, 2H), 7.22 ― 7.15 (m, 1H), 7.04 (d, J = 7.6 Hz, 1H), 6.92 (m, 3H), 5.14 (dd, J = 12.6, 5.5 Hz, 1H), 4.78 (s, 2H), 3.75 (m, 4H), 3.71 ― 3.61 (m, 7H), 3.53 (t, J = 5.3 Hz, 2H), 3.41 ― 3.35 (m, 1H), 3.24 (t, J = 5.0 Hz, 4H), 3.16 (t, J = 8.6 Hz, 1H), 3.07 (dd, J = 10.7, 7.4 Hz, 1H), 2.96 (t, J = 5.2 Hz, 4H), 2.93 ― 2.82 (m, 3H), 2.80 ― 2.70 (m, 2H), 2.64 (s, 6H), 2.49 (d, J = 5.6 Hz, 4H), 2.14 (m, 1H), 2.05 (m, 1H). |
| 119 | 1H NMR (400 MHz, MeOD) δ 7.57 (m, 1H), 7.23 (d, J = 8.3 Hz, 2H), 7.20 ― 7.15 (m, 1H), 7.13 (d, J = 7.1 Hz, 1H), 7.02 (d, J = 7.5 Hz, 1H), 6.91 (m, 4H), 5.08 (dd, J = 12,2, 5.4 Hz, 1H), 4.02 (s, 2H), 3.69 (m, 2H), 3.66 (t, J = 5.6 Hz, 2H), 3.63 ― 3.53 (m, 7H), 3.43 (t, J = 5.4 Hz, 2H), 3.24 (m, 2H), 3.17 (t, J = 5.1 Hz, 4H), 3.02 (q, J = 7.7, 7.3 Hz, 2H), 2.86 ― 2.67 (m, 10H), 2.53 ― 2.49 (m, 1H), 2.48 (s, 3H), 2.34 (s, 6H), 2.11 (m, 1H). |
| 120 | 1H NMR (300 MHz. MeOD) δ 7.82 (d, J = 8.3 Hz, 1H), 7.48 (d, J = 2.3 Hz, 1H), 7.39 (dd, J = 8.3, 2.4 Hz, 1H), 7.26 ― 7.19 (m, 2H), 7.19 ― 7.10 (m, 1H), 7.02 (d, J = 7.6 Hz, 1H), 6.90 (m, 4H), 5.12 (dd, J = 12.2, 5.4 Hz, 1H), 4.73 (s, 2H), 3.72 ― 3.66 (m, 4H), 3.66 ― 3.56 (m, 6H), 3.50 (t. J = 5.1 Hz, 2H), 3.37 (m, 1H), 3.21 ― 3.10 (m, 6H), 3.00 (t, J = 8.0 Hz, 2H), 2.84 ― 2.74 (m, 2H), 2.73 ― 2.59 (m, 8H), 2.55 ― 2.49 (m, 1H), 2.48 (s, 3H), 2.28 (s, 6H), 2.13 (m, 1H). |
| 121 | 1H NMR (400 MHz, MeOD) δ 7.61 (d, J = 8.3 Hz, 1H), 7.24 (d, J = 8.3 Hz, 2H), 7.20 ― 7.12 (m, 1H), 7.04 ― 6.98 (m, 2H), 6.97 ― 6.84 (m, 4H), 5.04 (dd, J = 12.5, 5.4 Hz, 1H), 4.25 (s, 2H), 3.94 (s, 2H), 3.76 ― 3.66 4H), 3.60 (m, 5H), 3.55 (t, J = 5.2 Hz, 2H), 3.42 (t, J = 5.2 Hz, 2H), 3.37 (s, 2H), 3.19 (m, 2H), 3.12 (m, 4H), 3.01 (dd, J = 9.4, 6.4 Hz, 2H), 2.88 ― 2.78 (m, 1H), 2.71 (m, 3H), 2.48 (m, 4H), 2.30 (s, 6H), |
| 122 | 1H NMR (400 MHz, MeOD) δ 7.62 (d, J = 8.3 Hz, 1H), 7.26 (d, J = 8.3 Hz, 2H), 7.19 (q, J = 7.5 Hz, 1H), 7.05 ― 6.99 (m, 2H), 6.94 (d, J = 8.7 Hz, 2H), 6.90 (m, 2H), 5.05 (dd, J = 12.4. 5.5 Hz, 1H), 4.30 (s, 2H), 3.94 (s, 2H), 3.72 (m, 4H), 3.65 (m, 6H), 3.58 ― 3.49 (m, 5H), 3.41 (t, J = 5.2 Hz, 2H), 3,37 (s, 2H), 3.32 ― 3.20 (m, 2H), 3.19 ― 3.10 (m, 4H), 3.07 (d, J = 9.0 Hz, 1H), 3.03 (d, J = 8.4 Hz, 1H), 2.89 ― 2.78 (m, 2H), 2.76 ― 2.68 (m, 2H), 2.47 (s, 9H) 2.05 (d, J = 10.2 Hz, 1H). |
| 123 | 1H NMR (400 MHz, CDC13) δ 8.34 (s, 1H), 7.49 (t, J = 7.8 Hz, 1H), 7.21 (d, J = 8.2 Hz, 2H), 7.15 ― 7.06 (m, 2H), 6.97 (dd, J = 16.4, 8.0 Hz, 2H), 6.84 (m, 3H), 6.33 (t, J = 6.0 Hz, 1H), 4.96 ― 4.88 (m, 1H), 3.82 ― 3.74 (m, 2H), 3.67 ― 3.50 (m, 4H), 3.40 (q, J = 6.6 Hz, 2H), 3.14 (m, 6H), 2.98 (m, 2H), 2.87 ― 2.66 (m, 4H), 2.56 ― 2.51 (m, 1H), 2.48 (t, J = 6.9 Hz, 2H), 2.44 (s, 3H), 2.28 (s, 6H), 2.12 (in, 1H), 2.04 (m, 2H), |
| 124 | 1H NMR (400 MHz, CDCl3) *δ* 8.39 (s, 1H), 7.60 (d, J = 8.3 Hz, 1H), 7.23 (d, J = 8.2 Hz, 2H), 7.14 (m, 1H), 6.97 (d, J = 7.3 Hz, 2H), 6.91 - 6.81 (m, 3H), 6.76 (dd, J = 8.4, 2.1 Hz, 1H), 5.51 (t, J = 5.2 Hz, 1H), 4.94 (dd, J = 12.1, 5.3 Hz, 1H), 3.81 (d, J = 4.4 Hz, 2H), 3.78 - 3.70 (m, 1H), 3.64 (m, 4H), 3.31 (q, J = 6.1 Hz, 2H), 3.24 - 3.11 (m, 6H), 3.03 (t, J = 8.7 Hz, 1H), 2.98 (t, J = 8.5 Hz, 1H), 2.94 - 2.86 (m, 1H), 2.86 - 2.71 (m, 3H), 2.55 (m, 3H), 2.46 (s, 3H), 2.31 (s, 6H), 2.16 - 2.06 (m, 3H). |
| 125 | 1H NMR (400 MHz, CDCl3) *δ* 8.42 (m, 1H), 7.49 (t, J = 7.8 Hz, 1H), 7.20 (d, J = 8.2 Hz, 2H), 7.16 - 7.11 (m, 1H), 7.08 (d, J = 7.1 Hz, 1H), 6.95 (d, J = 7.6 Hz, 1H), 6.86 (m, 4H), 6.27 - 6.19 (m, 1H), 4.87 (m, 1H), 3.84 - 3.71 (m, 2H), 3.62 (m, 4H), 3.26 (q, J = 6.5 Hz, 2H), 3.23 - 3.08 (m, 6H), 3.05 - 2.64 (m, 7H), 2.51 (m, 1H), 2.43 (s, 3H), 2.39 - 2.26 (m, 8H), 2.18 - 2.06 (m, 1H), 2.03 (d, J = 8.2 Hz, 1H), 1.67 (m, 4H), 1.40 (m, 4H). |
| 126 | 1H NMR (500 MHz, CDCl3) *δ* 8.22 (s, 1H), 7.60 (d, J = 8.3 Hz, 1H), 7.22 - 7.18 (m, 2H), 7.12 (m, 1H), 6.98 - 6.93 (m, 2H), 6.85 (m, 3H), 6.73 (dd, J = 8.3, 2.2 Hz, 1H), 4.97 - 4.87 (m, 1H), 4.62 (t, J = 5.4 Hz, 1H), 3.76 (t, J = 5.2 Hz, 2H), 3.66 - 3.58 (m, 5H), 3.21 (q, J = 6.8 Hz, 3H), 3.13 (m, 6H), 3.06 - 2.92 (m, 3H), 2.91 - 2.68 (m, 4H), 2.52 (dd, J = 9.1, 5.3 Hz, 1H), 2.43 (s, 3H), 2.36 (m, 8H), 1.65 (m, 4H), 1.45 - 1.34 (m, 4H). |
| 127 | 1H NMR (400 MHz, CDCl3) *δ* 8.31 (s, 2H), 8.17 (s, 1H), 8.12 (s, 1H), 8.00 (s, 1H), 7.64 (d, J = 8.3 Hz, 1H), 7.40 (s, 1H), 6.96 (d, J = 2.2 Hz, 1H), 6.84 (dd, J = 83, 2.2 Hz, 1H), 6.42 (t, J = 5.8 Hz, 1H), 6.39 - 6.33 (m, 2H), 5.97 - 5.90 (m, 1H), 4.98 - 4.90 (m, 1H), 4.86 (d, J = 5.7 Hz, 2H), 4.69 - 4.60 (m, 1H), 4.26 -4.15 (m, 1H), 3.96 (d, j = 3.8 Hz, 2H), 3.81 - 3.73 (m, 1H), 3.16-3.06 (m, 1H), 3.06-2.94 (m, 2H), 2.94-2.68 (m, 4H), 2.26 (d, J = 6.9 Hz, 2H), 2.21-2.10 (m, 6H), 2.07-1.93 (m, 2H), 1.93-1.78 (m, 2H), 1.22-1.09 (m, 2H). |
| 128 | 1H NMR (400 MHz, CDCl3) *δ* 8.34-8.26 (m, 2H), 8.20 (s, 1H), 8.19-8.14 (m, 1H), 8.02-7.98 (m, 1H), 7.95 (d, J = 8.8 Hz, 1H), 7.40 (s, 1H), 7.26-7.22 (m, 1H), 7.17-7.12 (m, 1H), 6.42 (t, J = 5.7 Hz, 1H), 6.39-6.33 (m, 2H), 6.07-6.00 (m, 1H), 5.83-5.75 (m, 1H), 4.85 (d, J = 5.7 Hz, 2H), 4.69-4.61 (m, 1H), 4.26-4.14 (m, 1H), 4.00 (d, J = 3.7 Hz, 2H), 3.86-3.77 (m, 1H), 3.16-3.05 (m, 1H), 3.05-2.92 (m, 4H), 2.92-2.69 (m, 2H), 2.26 (d, J = 7.2 Hz, 2H), 2.23-2.11 (m, 6H), 2.05-1.90 (m, 2H), 1.90-1.75 (m, 2H), 1.21-1.06 (m, 2H). |
| 129 | 1H NMR (400 MHz, CDCl3) *δ* 8.86 (s, 1H), 8.32 (d, J = 7.1 Hz, 2H), 8.15 (s, 1H), 7.97 (s, 1H), 7.57 (d, J = 8.3 Hz, 1H), 7.40 (s, 1H), 7.04-6.93 (m, 1H), 6.76 (dd, J = 8.3, 2.1 Hz, 1H), 6.45 (t, J = 5.9 Hz, 1H), 6.40-6.33 (m, 2H), 6.16-5.93 (m, 1H), 4.96-4.88 (m, 1H), 4.85 (d, J = 5.7 Hz, 2H), 4.36-4.21 (m, 1H), 3.94 (d, J = 5.3 Hz, 2H), 3.70-3.62 (m, 2H), 3.62-3.55 (m, 6H), 3.55-3.46 (m, 2H), 3.28-3.12 (m, 2H), 2.91-2.64 (m, 5H), 2.17-1.98 (m, 5H). |
| 130 | 1H NMR (400 MHz, CDCl3) *δ* 9.08 (s, 1H), 8.31 (d, J = 5.7 Hz, 2H), 8.13 (s, 1H), 7.97 (s, 1H), 7.87 (d, J = 8.8 Hz, 1H), 7.40 (s, 1H), 7.22-7.12 (m, 2H), 6.44 (t, J = 5.8 Hz, 1H), 6.40-6.33 (m, 2H), 6.10 (s, 1H), 5.77-5.66 (m, 1H), 4.85 (d, J = 5.7 Hz, 2H), 4.38-4.20 (m, 1H), 3.99 (d, J = 5.2 Hz, 2H), 3.71-3.62 (m, 2H), 3.62-3.55 (m, 6H), 3.55-3.48 (m, 2H), 3.32-3.10 (m, 2H), 3.01-2.65 (m, 5H), 2.39-2.33-(m, 1H), 2.31-2.24 (m, 2H), 2.07-1.95 (m, 2H). |
| 131 | 1H NMR (400 MHz, CDCl3) *δ* 8.13 (s, 1H), 7.67 (d, J = 8.5 Hz, 1H), 7.28 (m,, 1H), 7.18 (d, J = 8.3 Hz, 2H), 7.12 (q, J = 7.4 Hz, 1H), 7.05 (dd, J = 8.7, 2.2 Hz, 1H), 6.95 (d, J = 7.6 Hz, 1H), 6.84 (m, 3H), 4.93 (dd. J = 12.2, 5.3 Hz, 1H), 3.96 (d, J = 13.0 Hz, 2H), 3.64 (s, 2H), 3.22 (m, 1H), 3.17 (t, J = 5.0 Hz, 4H), 3.05 (m, 1H), 2.98 (t, J = 12.2 Hz, 3H), 2.92 - 2.69 (m, 3H), 2.57 (t, J = 4.9 Hz, 4H), 2.51 (t, J = 7.7 Hz, 1R), 2.43 (s, 3H), 2.34 (s, 6H), 2.27 (d, J = 7.1 Hz, 2H), 2.16 - 2.09 (m, 1H), 2.03 (d, J = 7.7 Hz, 1H), 1.92 (d, J = 13.3 Hz, 2H), 1.33 - 1.27 (m, 2H), |
| 132 | 1H NMR (400 MHz, CDCl3) *δ* 8.12 (s, 1H), 7.69 (d, J = 8.6 Hz, 1H), 7.30 (m, 1H), 7.23 (d, J = 8.3 Hz, 2H), 7.14 (q, J = 7.3 Hz, 1H), 7.06 (dd, J = 8.7, 2.3 Hz, 1H), 6.98 (d, J = 7.6 Hz, 1H), 6.88 (t, J = 8.2 Hz, 3H), 4.96 (dd, J = 12.2, 5.3 Hz, 1H), 3.98 (d, J = 13.0 Hz, 2H), 3.80 (t, J = 5.3 Hz, 2H), 3.66 (m, 4H), 3.23 (m, 1H), 3.16 (m, 4H), 3.05 (m, 4H), 2.98 - 2.65 (m, 6H) 2.54 (t, J = 7.6 Hz, 1H), 2.46 (s, 3H), 2.34 (d, J = 6.6 Hz, 6H), 2.25 - 2.09 (m, 3H), 2.05 (d, J = 8.0 Hz, 1H), 1.94 (d, J = 12.3 Hz, 2H), 1.36 (m, 2H). |
| 133 | 1H NMR (400 MHz, CDCl3) δ 8.07 (s, 1H), 7.67 (d, J = 8.5 Hz, 1H), 7.26 (m, 1H), 7.21 (d, J = 8.2 Hz, 2H), 7.12 (q, J = 7.4 Hz, 1H), 7.04 (m, 1H), 6.95 (d, J = 7.6 Hz, 1H), 6.85 (t, J = 8.0 Hz, 3H), 4.93 (dd, J = 12.2, 5.3 Hz, 1H), 3.95 (d, J = 13.0 Hz, 2H), 3.77 (t, J = 5.1 Hz, 2H), 3.62 (m, 4H), 3.21 (m, 1H), 3.13 (m, 5H), 2.98 (m, 3H), 2.93 - 2.66 (m, 4H), 2.51 (m, 1H), 2.43 (s, 3H), 2.41 (d, J = 7.0 Hz, 2H), 2.33 (s, 6H), 2.17 - 2.08 (m, 1H), 2,03 (d, J = 8.8 Hz, 1H), 1.85 (d, J = 13.0 Hz, 2H), 1.65 (m, 3H), 1.38 - 1.28 (m, 2H). |
| 134 | 1H NMR (500 MHz, Chloroform-d) δ 8.13 - 7.93 (m, 1H), 7.67 (d, J = 8.5 Hz, 1H), 7.27 (m, 1H), 7.16 (d, J = 8.3 Hz, 2H), 7.12 (t, J = 7.1 Hz, 1H), 7.04 (d, J = 8.9 Hz, 1H), 6.95 (d, J = 7.6 Hz, 1H), 6.84 (m, 3H), 4.94 (dd, J = 12.2, 5.4 Hz, 1H), 3.95 (d, J = 13.1 Hz, 2H), 3.66 (d, J = 15.0 Hz, 2H), 3.62 (d, J = 12.4 Hz, 1H), 3.26 (s, 2H), 3.16 (m, 4H), 3.09 (m, 2H), 2.98 (t, J = 12.5 Hz, 4H), 2.89 (d, J = 17.4 Hz, 1H), 2.83 - 2.66 (m, 3H), 2,55 (m, 4H). 2.48 (m, 4H), 2.42 (s, 3H), 2.26 (m, 3H), 2.17 - 2.10 (m, 1H), 2.03 (d, J = 10.0 Hz, 2H), 1.88 (d, J = 71.0 Hz, 6H), 1.64 - 1.47 (m, 4H), 1.25 (m, 3H), |
| 135 | 1H NMR (400 MHz, MeOD) δ 7.92 (s, 1H), 7.63 (d, J = 8.3 Hz, 1H), 7.26 (d, J = 8.3 Hz, 2H), 7.19 (q, J = 7.3 Hz, 1H), 7.08 - 6.99 (m, 2H), 6.95 (d, J = 8.2 Hz, 2H), 6.90 (d, J = 3.8 Hz, 2H), 5.06 (dd, J = 12.5, 5.4 Hz, 1H), 3.94 (s, 2H), 3.73 (m, 4H), 3.63 (m, 4H), 3.57 (s, 5H), 3.54 (t, J. = 5.3 Hz, 2H), 3.42 (t, J = 5.4 Hz, 2H), 3.37 (s, 2H), 3.23 (m, 4H), 3.09 (m, 3H), 2.90 (m, 4H), 2.86 - 2.80 (m, 3H), 2.76 - 2.67 (m, 2H), 2.56 (s, 6H), 2.50 (m, 1H), 2.48 (s, 3H), 2.05 (d, J = 9.8 Hz, 2H). |
| 136 | 1H NMR (500 MHz. Chloroform-d) *δ* 8.63 (s, 1H), 7.62 (d, J = 8.3 Hz, 1H), 7.20 - 7.17 (m, 2H), 7.12 (m, 2H), 6.95 (q, J = 3.8, 2.8 Hz, 2H), 6.88 - 6.81 (m, 4H), 5.97 (t, J = 4.0 Hz, 1H), 4.93 (dd, J = 12.3, 5.4 Hz, 1H), 4.62. (d, J = 13.1 Hz, 1H), 3.95 (d, J = 3.7 Hz, 2H), 3.75 (d, J = 13.4 Hz, 1H), 3.67 - 3.58 (m, 2H), 3.19 (m, 6H), 3.09 (t, J = 12.8 Hz, 1H), 3.05 - 3.01 (m, 1H), 2.97 (t, J = 8.3 Hz, 1H). 2.90 - 2.82 (m, 2H), 2.81 - 2.67 (m, 3H), 2.61 (m, 4H), 2.51 (dd, J = 9.3, 5.5 Hz, 1H), 2.46 (m, 2H), 2.43 (s, 3H), 2.34 (s, 6H), 2.16 - 2.09 (m, 1H), 1.87 (d, J = 13.0 Hz, 1H), 1.81 (d, J = 13.2 Hz, 1H), 1.62 (m, 1H), 1.53 (q, J = 7.2 Hz, 2H) 1.20 (m, 1H). |
| 137 | 1H NMR (500 MHz, Chloroform-d) *δ* 8.49 (m, 1H), 7.53 - 7.45 (m, 1H), 7.20 (dd, J = 8.3, 5.0 Hz, 2H), 7.15 - 7.05 (m, 2H), 6.95 (d, J = 7.5 Hz, 1H), 6.91 - 6.82 (m, 4H), 6.23 (t, J = 5.7 Hz, 1H), 4.86 (ddd, J = 31.9, 12.3, 5.4 Hz, 1H), 3.86 - 3.68 (m, 2H), 3.67 - 3.57 (m, 4H), 3.29 (q, J = 6.5 Hz, 2H), 3.23 - 3.08 (m, 6H), 3.03 (m, 1H), 2.98 - 2.91 (m, 1H), 2.90 - 2.68 (m, 4H), 2.53 - 2.47 (m, 1H), 2.43 (s, 3H), 2.39 (m, 2H), 2.32 (s, 6H), 2.10 (m, 1H), 1.71 (p, J = 7.0 Hz, 4H), 1.49 (m, 2H), |
| 138 | 1H NMR (400 MHz, Chloroform-d) δ 8.46 (s, 1H), 7.56 (d, J = 8,3 Hz, 1H), 7,20 (d, J = 8,2 Hz. 2H), 7.12 (q. J = 7,2 Hz, 1H), 6.99 ― 6.91 (m, 2H), 6.85 (t, J = 9.1 Hz, 3H), 6.72 (dd, J = 8.3. 2.1 Hz, 1H), 4,92 (m, 2H), 3.78 (t J = 5,4 Hz, 2H), 3.62 (m, 4H), 3.23 (m, 3H), 3.12 (q, J = 5.5 Hz, 4H), 3.00 (m,3H), 2.80 (m, 4H), 2.51 (t, J = 7.1 Hz. 1H), 2.41 (m 5H), 2.34 (s, 6H), 2.11 (m, 1H), 2,04 (m, 1H), 1.71 (h, J = 7.5 Hz, 4H), 1.47 (p, J = 7,9 Hz, 2H). |
| 139 | 1H NMR (500 MHz, Chloroform-d) δ 8.02 (m, 1H), 7.57 (t, J = 7.8 Hz, 1H), 7.37 (d, J = 7.0 Hz, 1H), 7.17 (t, J = 8.3 Hz, 4H), 7.14 ― 7.09 (m, 1H), 6.96 (d, J = 7.6 Hz, 1H), 6.86 (m, 4H), 4.96 (dd, J = 12.3, 5.4 Hz, 1H), 3.76 (t, J = 12.7 Hz, 3H), 3.65 (q, J = 12.4 Hz, 2H), 3:26 (m, 1H), 3.17 (m, 5H), 3.01 (m, 1H), 2.94 ― 2.85 (m, 3H), 2.78 (m, 2H), 2.58 (m, 4H), 2.47 (m, 3H), 2.42 (s, 3H), 2,31 (d, J = 7.1 Hz, 2H), 2.17 ― 2.08 (m, 1H), 2.03 (d, J = 9.5. Hz, 1H), 1.94 (d, J = 12.9 Hz, 2H), 1.75 (m,1H), 1.49 (m,3H), |
| 140 | 1H NMR (400 MHz. Chloroform-d) δ 8.10 (m, 1H), 7.64 (d, J = 8.3 Hz, 1H), 7.20 (d, J = 8.2 Hz, 2H), 7.13 (q, J = 7,2 Hz, 1H), 6.99 ― 6.94 (m, 2H), 6.91 ― 6.78 (m, 4H), 5.91 (m, 1H), 4.93 (dd, J = 12.2, 5.3 Hz, 1H), 4.63 (d. J = 13.4 Hz, 1H), 3.95 (d, J = 3.7 Hz, 2H), 3.77 (m, 3H), 3.70 ― 3.56 (m, 5H), 3.30 (m,2H), 3.13 (m, 6H), 2.98 (m, 2H), 2.93 ― 2.63 (m, 5H), 2.46 (s, 5H), 2.42 (m, 5H), 2.21 ― 1,98 (m, 2H), 1.86 (dd, J = 22.5, 13.3 Hz, 2H), 1.66 (m, 2H), 1.54 (t, J = 7,4 Hz, 1H), 1,19 (s, 1H), |
| 141 | 1H NMR (500 MHz, Chloroform-d) *δ* 8.10 (s, 1H), 7.57 (t, J = 7.8 Hz, 1H). 7.37 (d, J = 7.1 Hz, 1H), 7.21 (d, J = 8.1 Hz, 2H), 7.17 (d, J = 8.4 Hz, 1H), 7.12 (q, J = 7.5 Hz, 1H), 6.95 (d, J = 7.5 Hz, 1H), 6.85 (m. 3H), 4.95 (m. 1H), 4.27 (q, J = 5.6 Hz, 1H), 3.81 ― 3.68 (m, 4H), 3.63 (m, 4H), 3.21 (m, 1H), 3.14 (m, 4H), 3.00 (d, J = 28.6 Hz, 2H), 2.93 ― 2.79 (m. 4H), 2.80 ― .2.66 (m, 1H), 2.52 (m, 1H), 2.43 (m, 5H), 2.34 (s, 6H), 2.11 (m, 1H), 2.03 (m,1H), 1.86 (d, J = 10.1 Hz, 2H), 1.70 (m, 2H), 1.46 (m, 1H), 1.33 (m, 1H), 0.95 (t, J = 7,5 Hz, 1H). |
| 142 | 1H NMR (400 MHz, Chloroform-d) *δ* 8.28 (s, 1H), 7.57 (t, J = 7.8 Hz, 1H), 7.37 (d, J = 7.1 Hz, 1H), 7.19 (dd, J = 16.3, 8.3 Hz, 3H), 7.12 (q, J = 7,2 Hz, 1H), 6.95 (d. J = 7.6 Hz, 1H), 6.85 (q, J = 5.1, 4,5 Hz, 3H), 4.95 (dd, J = 12,2, 5.4 Hz, 1H), 3.79 (t, J = 4,9 Hz, 2H), 3.72 (t, J = 10.0 Hz, 2H), 3.64 (m, 3H), 3.25 ― 3.09 (m, 6H), 3.08 ― 2.64 (m, 9H), 2.52 (dd. J = 9.1, 5.6 Hz, 1H), 2.44 (s, 3H), 2.40 ― 2.19 (m, 8H), 2.10 (m, 2H), 1.93 (d. J = 12.8 Hz, 2H), 1.55 (q, J = 12.8 Hz, 2H), 1.33 (m, 1H). |
| 143 | 1H NMR (500 MHz, Chlorofonn-d) *δ* 8.91 (m, 1H), 8.50 (m, 1H), 7.68 (t, J = 7.9 Hz, 1H), 7.51 (m, 1H), 7.36 ― 7.28 (m, 1H), 7.24 ― 7.18 (m, 2H), 7.12 (q, J = 7.4 Hz, 1H), 6.96 (d, J = 7.5. Hz, 1H), 6.85 (t, J = 8.3 Hz, 3H), 5.11 ― 4.79 (m, 3H), 4.53 (d, J = 13.2 Hz, 1H), 4.04 (m,1H), 3.74 (m, 2H), 3.62 (d, J = 14.6 Hz, 4H), 3.32 ― 3.02 (m, 8H), 2.97 (m, 1H), 2.91 ― 2.58 (m, 6H), 2.51 (m, 1H), 2.43 (s, 3H), 2.39 ― 2.24 (m, 7H), 2.18 ― 2.07 (m, 2H), 1.84 (m, 2H), 1.27 (m, 1H), 1.23 ― 1.10 (m, 2H). |
| 144 | 1H NMR (500 MHz. Chloroform-d) *δ* 8.24 (m, 1H). 7.50 (t, J = 7.8 Hz, 1H), 7.23 ― 7.17 (m, 3H), 7.12 (m, 2H), 6.95.(d. J = 7.5 Hz, 1H), 6.85 (t, J = 8.7 Hz, 3H), 6.79 (d. J = 8.5 Hz, 1H), 4.92 (dd, J = 12.4, 5,4 Hz, 1H), 4.66 (d, J = 13.3 Hz, 1H), 4.02 (d, J = 4.4 Hz, 2H), 3.83 ― 3.68 (m, 3H), 3.66 ― 3.55 (in, 4H), 3.19 (d, J = 27.1 Hz, 2H), 3.13 (m. 5H), 3.04 (m, 1H), 2,97 (t, J = 8.6 Hz, 1H), 2.92― 2.77 (m, 3H), 2.73 (m, 2H), 2.57 ― 2.48. (m, 1H), 2.43 (s, 3H), 2,32 (m, 7H), 2.18 (m, 1H), 2.12 (m. 1H), 1.97 (m, 2H), 1.83 (d. J = 13.3 Hz, 1H), 1.21 (m, 2H). |
| 145 | 1H NMR (500 MHz, Chloroform-d) *δ* 8.40 (s, 1H), 7.80 (d, J = 8.3 Hz, 1H), 7.34 (s, 1H), 7.30 (d, J = 8.4 Hz, 1H), 7.21 (d, J = 8.3 Hz, 2H), 7.16 ― 7.08 (m, 1H), 6.95 (d, J = 7.5 Hz, 1H), 6.85 (t, J = 8.7 Hz, 3H), 4.95 (m, 1H), 4.87 (d, J = 14,0 Hz, 1H), 4.82 (d, J = 14.0 Hz, 1H), 4.57 (d, J = 13.2 Hz, 1H), 3.84 (d, J = 13.5 Hz, 1H), 3.77 (t, J = 5.1 Hz, 2H), 3.68 ― 3.57 (m, 4H), 3.20 (d, J = 16.8 Hz, 2H), 3.13 (m, 5H), 3.08 ― 2.94 (m, 2H), 2.93 ― 2.63 (m, 5H), 2.52 (m, 1H), 2,43 (s, 3H), 2.40 ― 2.28 (m. 7H), 2.27 ― 2.07 (m, 3H), 1.18 (s, 2H), 1.96 (d, J = 13.0 Hz, 1H), 1.83 (d, J = 13.3 Hz, 1H), 1.18 (m, 2H). |
| 146 | 1H NMR (500 MHz, Chloroform-d) *δ* 8.27 (s, 1H), 7.66 (d, J = 8.3 Hz, 1H), 7.23 (d. J = 8.5 Hz, 2H), 7.14 (q, J = 7.2 Hz, 1H), 6.98 (d, J = 6.7 Hz, 2H), 6.88 (t, J = 8.5 Hz, 4H), 5.94 (m. 1H), 4.95 (dd, J = 12.4, 5.4 Hz, 1H), 4.68 (d, J = 13.3 Hz, 1H), 3.97 (d, J = 3.8 Hz, 2H), 3.85 - 3.73 (m, 3H), 3.65 (m, 4H), 3.22 (d, J = 19.7 Hz, 2H), 3.16 (m. 5H), 3.08 ― 2,97 (m, 2H), 2.94 ― 2,68 (m, 5H), 2.54 (t, J = 7.7 Hz, 1H), 2.45 (s, 3H), 2.35 (m, 7H), 2.27 (m, 1H), 2.18 ― 2,11 (m, 1H), 2.01 (d, J = 13.4 Hz, 2H), 1.90 (d, J = 13.2 Hz, 1H), 1.23 (m, 2H), |
| 147 | 1H NMR (400 MHz. Chlorofonn-d) *δ* 8.29 (s, 1H), 7.80 (d, J = 8.2 Hz, 1H), 7.37 ― 7.28 (m, 2H), 7.20 (d, J = 8.2 Hz, 2H), 7.13 (q, J = 7.3 Hz, 1H), 6.96 (d, J = 7.6 Hz, 1H), 6.85 (q, J = 5.2 Hz, 3H), 4.94 (dd, J = 12.1, 5.5 Hz, 1H), 4.84 (m, 2H), 4.55 (d, J = 13.4 Hz, 1H), 3.89 ― 3.73 (m, 3H), 3.69 ― 3.57 (m, 4H), 3.28 (m, 2H), 3.12 (m, 6H), 3.02 ― 2.85 (m, 3H), 2.84 ― 2.60 (m, 3H), 2.50 (t, J = 7.4 Hz, 1H), 2.41 (m, 9H), 2.20 ― 2.06 (m, 1H), 1.82 (dd, J = 23.0, 13.3 Hz, 2H), 1,69 ― 1.59 (m, 2H), 1.50 (m, 2H), 1,32 (m, 1H), 1.17 (m, 2H). |
| 148 | 1H NMR (500 MHz, Chloroform-d) *δ* 8.88 (s. 1H), 7.68 (t, J = 7.9 Hz, 1H), 7.51 (d, J = 7.3 Hz, 1H), 7.36 (d, J = 8.4 Hz, 0.5H), 7.29 (d, J = 8.4 Hz, 0.5H), 7.20 (d, J = 8,2 Hz, 2H), 7.12 (q, J = 7.3 Hz, 1H), 6.96 (d, J = 7.5 Hz, 1H), 6,85 (m, 3H), 5,09 (d, J = 14.1 Hz, 1H), 5.00 ― 4.84 (m, 3H), 4,51 (dd, J = 24.0, 13.2 Hz, 1H), 4.06 (d, J = 13.4 Hz, 1H), 3.92 (d, J = 13.7 Hz, 1H), 3.77 (m. 2H), 3.62 (m. 4H), 3.23 (s, 1H), 3.13 (m. 4H), 3.06 (t, J = 12.6 Hz, 2H), 2.97 (m, 1H), 2.93 ― 2.66 (m, 4H), 2.61 (q, J = 12.1 Hz, 1H), 2.51 (m, 1H), 2.43 (s, 3H), 2.38 (m. 7H), 2,14 (m, 1H). 1.76 (m, 2H), 1.67 ― 1.52 (m. 3H), 1.29 (m, 1H), 1.16 (m, 2H). |
| 149 | 1H NMR (500 MHz, Chlorofonn-d) *δ* 8.25 (s, 1H), 7.50 (t, J = 7.8 Hz, 1H). 7.21 (d, J = 7.7 Hz, 3H), 7.12 (dd, J = 10,4, 6.8 Hz, 2H), 6.96 (d, J = 7,5 Hz, 1H), 6.86 (t, J = 7.8 Hz, 3H), 6.82 ― 6.76 (m, 1H), 4,92 (dd, J = 12.4, 5.3 Hz, 1H), 4.64 (d, J = 13.3 Hz, 1H), 4.02 (d, J = 4.4 Hz, 2H), 3.76 (m, 3H), 3.62 (dd, J = 12.3, 7.4 Hz, 4H), 3.25 (s, 2H), 3.13 (m. 5H), 3.07 (d, J = 11.9 Hz, 2H), 2.98 (m, 1H), 2.91 ― 2.63 (m, 5H), 2.52 (m, 1H), 2.43 (s, 3H), 2.40 (m, 7H), 2,13 (m, 1H), 1.83 (m, 2H), 1.74 ― 1,56 (m, 4H), 1.23 ― 1.11 (m, 2H). |
| 150 | 1H NMR (500 MHz, Chlorofonn-d) *δ* 8.44 (s, 1H), 7.80 (d, J = 8.3 Hz, 1H), 7.34 (d. J = 2.3 Hz, 1H), 7.30 (m, 1H), 7.18 (d J = 8.3 Hz, 2H), 7.12 (q, J = 7.3 Hz, 1H), 6.95 (d, J = 7.5 Hz, 1H), 6.85 (t, J = 8.7 Hz, 3H), 4.95 (dd, J = 12.3, 5.3 Hz, 1H), 4.86 (d, J =14.0 Hz, 1H), 4.82 (d, J = 13.9 Hz, 1H), 4.54 (d, J = 13.3 Hz, 1H), 3.83 (d, J = 13.5 Hz, 1H), 3.63 (m, 2H), 3.18 (m, 6H), 3.13 ― 3.01 (m, 2H), 3.00 ― 2.88 (m, 2H), 2.86 ― 2.63 (m, 4H), 2.60 (m, 4H), 2.51 (t, J = 7.6 Hz, 1H), 2.43 (m, 5H), 2.36 (s, 6H), 2.17 ― 2.09 (m, 1H), 1,84 (d, J = 13.1 Hz, 1H), 1.78 (d, J = 13.4 Hz, 1H), 1.61 (m, 1H). 1.51 (q, J = 7.1 Hz, 2H), 1.23 ― 1.13 (m, 2H). |
| 151 | 1H NMR (500 MHz, Chloroform-d) *δ* 7.69 (q, J = 7.4 Hz, 1H), 7.51 (dd, J = 7.4, 4.1 Hz, 1H), 7.35 (d, 0.5H), 7.25 (m, 0.5H), 7.17 (d, J = 8.2 Hz, 2H), 7.12 (q, J = 7.3 Hz, 1H), 6.95 (d, J = 7.6 Hz, 1H), 6.85 (m, 3H), 5.04 (m, 0.5H), 5.00 ― 4.87 (m, 2H), 4.83 (m, 0.5H), 4,51 (dd, J = 28.9, 13.1 Hz, 1H), 4.04 (m, 0.5H), 3.79 (m, 0.5H), 3,63 (s, 2H), 3.49 (m, 1H), 3,21 (m, 6H), 3.04 (m, 3H), 2.92 - 2.74 (m, 3H), 2.74 - 2.59 (m, 6H), 2.51 (m, 1H), 2.43 (s, 3H), 2.35 (s, 6H), 2.23 ― 2.10 (m, 2H), 1.81 (d, J = 13.6 Hz, 1H), 1.72 (d, J = 13.2 Hz, 2H), 1.56 (m, 2H), 1.39 (m, 2H). |
| 152 | 1H NMR (500 MHz, Chloroform-d) *δ* 8.66 (m, 1H), 7.50 (t, J = 7.8 Hz, 1H), 7.24 - 7.16 (m, 3H), 7.12 (dd, J = 14.2, 7.2 Hz, 2H), 6.95 (d, J = 7.5.Hz, 1H), 6.85 (t, J = 8.5 Hz, 3H), 6.79 (d, J = 8.5 Hz, 1H), 4.92 (dd, J = 13.3, 5.0 Hz, 1H), 4.63 (d, J = 13.1 Hz, 1H), 4.03 (d, J = 4.3 Hz, 2H), 3.74 (m, 1H), 3.63 (s, 2H), 3.18 (m, 6H), 3.13 - 2.93 (m, 4H), 2.91 - 2.77 m, 3H), 2.71 (m, 2H), 2.60 (m, 4H), 2.52 (m, 1H), 2.43 (m, 5H), 2.33 (s, 6H), 2.12 (m, 1H), 1.89 ― 1.75 (m, 2H), 1.61 (m, 1H), 1.52 (m, 1H), 1.21 (m, 2H). |
| 153 | 1H NMR (500 MHz, Chloroform-d) *δ* 8.17 (s, 1H), 7.57 (t, J = 7.8 Hz, 1H), 7.37 (d, J = 7.1 Hz, 1H), 7.18 (dd, J = 8.7, 2.6 Hz, 3H), 7.12 (q, J = 7.3 Hz, 1H), 6.95 (d, J = 7.5 Hz, 1H), 6,86 (m,3H), 4.96 (dd, J = 12.4, 5.4 Hz, 1H), 3.73 (t, J = 12.6 Hz, 2H), 3,65 (m, 2H), 3.20 (m, 5H), 3.06 (m, 1H), 2.98 (t, J = 8.6 Hz, 1H), 2.95 ― 2.68 (m, 7H), 2.63 (m, 4H), 2.51 (m, 3H), 2.40 (d, J = 23.1 Hz, 9H), 2.18 (m, 1H), 2.13 - 2,08 (m, 1H), 2.05 (m, 1H), 1.85 (d, J = 9.1 Hz, 2H), 1.57 (m, 2H), 1.27 - 1.22 (m, 1H). |
| 154 | 1H NMR (500 MHz, Chloroform-d) *δ* 8.04 (s, 1H), 7.67 (d, J = 8.5 Hz, 1H), 7.17 (d, J = 8.2 Hz, 2H), 7.13 (q, J = 7.1 Hz, 1H), 7.04 (d, J = 8.5 Hz, 1H), 6.95 (d, J = 7.6 Hz, 1H), 6.85 (m, 3H), 4.94 (dd, J = 12.2, 5.4 Hz, 1H), 3,94 (d, J = 13.0 Hz, 2H), 3.64 (m, 2H), 3.22 (m, 5H), 3.08 (m, 2H), 3.00 - 2.67 (m, 7H), 2,61 (m,4H), 2.53 - 2.44 (m, 4H), 2.42 (s, 6H), 2.17 ― 2,07 (m, 1H), 1,84 (d, J = 13.1 Hz, 2H), 1.63 (m, 3H), 1.54 (m, 2H), 1.33 (m, 3H). |
| 155 | [M+H]⁺ : 762 |
| 156 | [M+H]⁺ : 820 |
| 157 | [M+H]⁺ : 819 |
| 158 | [M+H]⁺ : 873 |
| 159 | [M+H]⁺ : 806 |
| 160 | [M+H]⁺ : 864 |
| 161 | [M+H]⁺ : 863 |
| 162 | [M+H]⁺ : 917 |
| 163 | [M+H]⁺ : 776 |
| 164 | [M+H]⁺ : 834 |
| 165 | [M+H]⁺ : 833 |
| 166 | [M+H]⁺ : 887 |
| 167 | [M+H]⁺ : 790 |
| 168 | [M+H]⁺ : 804 |
| 169 | [M+H]⁺ : 862 |
| 170 | [M+H]⁺ : 861 |
| 171 | [M+H]⁺ : 915 |
| 172 | [M+H]⁺ : 806 |
| 173 | [M+H]⁺ : 762 |
| | |
| 174 | [M+H]⁺ : 790 |
| 175 | [M+H]⁺ : 776 |
| 176 | [M+H]⁺ : 804 |
| 177 | [M+H]⁺ : 833 |
| 178 | [M+H]⁺ : 861 |
| 179 | [M+H]⁺ : 819 |
| 180 | [M+H]⁺ : 820 |

### [Experiment 1] EED proteolytic activity Evaluation 1

In order to evaluate the EED proteolytic activity of the compound according to the present invention, an experiment was conducted as follows.

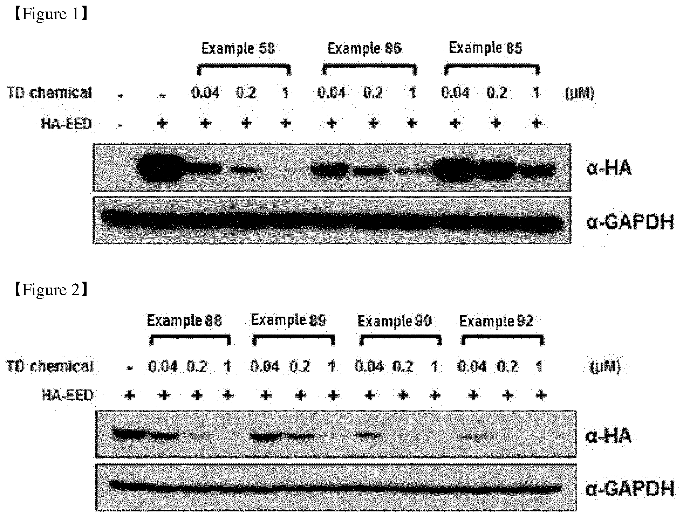

Specifically, 5 × 10⁶ HEK293T cells were injected into each plate, and the next day, a plasmid (1 µg) expressing HA-EED was transfected using Fugene. The next day, cells were removed using trypsin and 1 × 10⁶ cells were injected into 6 wells. The next day, each well was treated so that the final concentrations of the Example compounds were 40 nM, 200 nM, and 1000 nM. One well was treated with DMSO at the same percentage. After 24 hours of treatment, the cells were collected and cell lysate using RIPA buffer (50mM Tris, pH7.5, 150mM NaCl, 1% Triton X-100, 0.1% SDS, 2mM EDTA, 0.5% deoxycholate, and protease inhibitor cocktail) was prepared and western blot was performed, and the results are shown in FIG. 1 and FIG. 2.

Referring to Fig. 1 and FIG. 2, it is confirmed that the Example compounds according to the present invention has EED proteolytic activity.

### [Experiment 2] EED proteolytic activity Evaluation 2

In order to evaluate the EED proteolytic activity of the compound according to the present invention, an experiment was conducted as follows.

Specifically, 2 × 10⁵ Hela cells were injected into a 12-well plate, and after 8 hours of stabilization, each well was treated with the example compounds to have final concentrations of 1 nM, 10 nM, 100 nM, and 1000 nM. One well was treated with the volume of DMSO contained in the sample corresponding to the highest concentration in the experimental group. After 20 hours of treatment, the cells were collected and lysed using 80µl of SDS Lysis buffer (50mM Tris pH8.0, 1% SDS, 1mM EDTA, 0.5mM PMSF, and protease inhibitor cocktail) and then sonication (30 sec on /59 sec off), 5 cycles) to prepare a cell lysate, followed by protein quantification through BCA, followed by Western blot, and the results are shown in Table 3. (Maximum EED degradation rate: A: >50%, B: 25~50%, C: <25%)

Referring to Table 3, it is confirmed that the example compounds according to the present invention have EED proteolytic activity.

**[Table 3]**

| Example | EED degradation rate |
|---|---|
| 121 | A |
| 122 | A |
| 123 | B |
| 124 | B |
| 125 | A |
| 126 | B |
| 127 | C |
| 128 | C |
| 129 | C |
| 130 | C |
| 131 | A |
| 132 | A |
| 133 | A |
| 134 | B |
| 135 | B |
| 136 | A |
| 137 | A |
| 138 | B |
| 139 | C |
| 140 | A |
| 141 | B |
| 142 | C |
| 143 | A |
| 144 | A |
| 145 | B |
| 146 | A |
| 147 | B |
| 148 | A |
| 149 | B |
| 150 | A |
| 151 | A |
| 152 | A |
| 153 | A |
| 154 | A |

Therefore, the novel compound represented by Formula 1 according to the present invention is a useful compound, that is, a degrader compound that induces degradation of embryonic ectoderm development (EED), and a critical compund through the Ubiquitin Proteasome System (UPS). There is an aspect that remarkably achieves degradation-inducing activity. Therefore, it can be seen that it can be provided as a pharmaceutical composition for the prevention or treatment of EED, EZH2, or PRC2 related diseases or conditions, and a health functional food composition for prevention or improvement containing it as an active ingredient.

## Claims

1. A compound represented by the following chemical formula 1, a stereoisomer thereof, or a pharmaceutically acceptable salt thereof:
[Chemical formula 1] **ULB―L―PTM**
In chemical formula 1,
PTM is an EED (embryonic ectoderm development) targeting molecule,
L (Linker) is a single bond or a chemical linker, and
ULB is cereblon, MDM2 (Mouse double minute 2 homolog), cIAP (Cellular Inhibitor of Apoptosis Protein 1) or VHL (von Hippel-Lindau tumor suppressor) E3 ubiquitin ligase binding binder.

2. The compound of claim 1, a stereoisomer thereof, or a pharmaceutically acceptable salt thereof, wherein the cereblon E3 ubiquitin ligase binding binder is any one represented by one of the chemical formulas represented by the following (a) to (g). 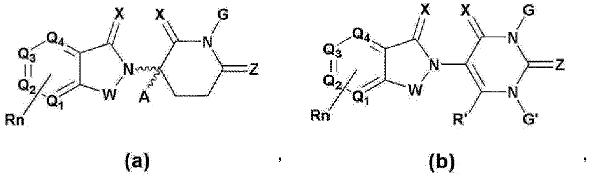 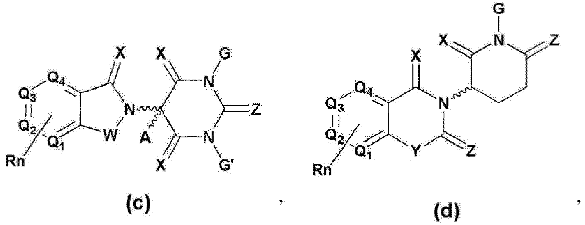 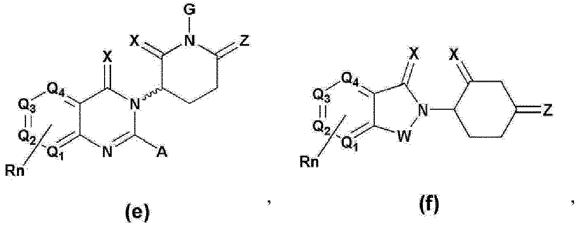 or 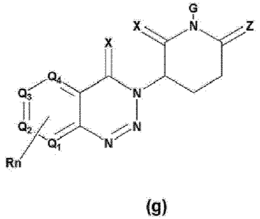 In the chemical formulas (a), (b), (c), (d), (e), (f), and (g),
W is CH₂, CHR, C=O, SO₂, NH, or N-C₁₋₁₀ straight or branched alkyl,
each X is independently O, S, or H₂,
Y is NH, N-C₁₋₁₀ straight or branched alkyl, N-C₆₋₁₀ aryl, N-heteroaryl, said heteroaryl being a 5-10-membered ring heteroaryl comprising one or more heteroatoms selected from the group consisting of N, O, and S, N-C₃₋₁₀ cycloalkyl, N-heterocycloalkyl, said heterocycloalkyl being a 3-10 membered ring heterocycloalkyl comprising one or more heteroatoms selected from the group consisting of N, O, and S, O, or S,
Z is O, S, or H₂,
G and G' are each independently H, C₁₋₁₀ straight or branched alkyl, OH, R'-substituted or unsubstituted CH2-heterocyclyl, said heterocyclyl being a 3-10 membered ring heterocyclyl comprising one or more heteroatoms selected from the group consisting of N, O, and S, or R'-substituted or unsubstituted benzyl,
Q₁, Q₂, Q₃, and Q₄ are each independently carbon substituted with R', or N,
A is C₁₋₁₀ straight or branched alkyl, C₃₋₁₀ cycloalkyl, or halogen,
R is -CONR'R", -OR', -NR'R", -SR', -SO₂R', -SO₂NR'R", -CR'R"-, -CR'NR'R"-, -C₆₋₁₀ aryl, -heteroaryl, said heteroaryl being a 5-10 membered heteroaryl comprising one or more heteroatoms selected from the group consisting of N, O, and S, -C₁₋₁₀ straight or branched alkyl, - C₃₋₁₀ cycloalkyl, -heterocyclyl said heterocyclyl being a 3-10 membered ring heterocyclyl comprising one or more heteroatoms selected from the group consisting of N, O, and S, - P(O)(OR')R", -P(O)R'R", -OP(O)(OR')R", -OP(O)R'R", -Cl, -F, -Br, -I, -CF₃, -CN, - NR'SO₂NR'R", -NR'CONR'R", -CONR'COR", -NR'C(=N-CN)NR'R", -C(=N-CN)NR'R", - NR'C(=N-CN)R", -NR'C(=CNO₂)NR'R", -SO₂NR'COR", -NO₂, -CO₂R', -C(C=N-OR')R", - CR'=CR'R", -CCR', -S(C=O)(C=N-R')R", -SF₅, or -OCF₃,
R' and R" are each independently selected from the group consisting of H, C₁₋₁₀ straight or branched alkyl, C₃₋₁₀ cycloalkyl, C₆₋₁₀ aryl, heteroaryl said heteroaryl being a 5-10 membered ring heteroaryl comprising one or more heteroatoms selected from the group consisting of N, O, and S, and heterocyclyl, said heterocyclyl being a 3-10 membered ring heterocyclyl comprising one or more heteroatoms selected from the group consisting of N, O, and S,
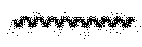 represents stereospecific (R or S) or non-stereospecific binding, and
Rₙ is -(CH₂)ₘ-NA-, -(CH₂)ₘ-O-, -O-(CH₂)ₘ-(C=O)-NA- or -NA-(CH₂)ₘ-(C=O)-NA-, again, wherein m is an integer from 0 to 5,
the A is each H, or an unsubstituted or substituted C₁₋₁₀ straight or branched alkyl,
here again, the substituted alkyl is substituted with halogen, nitro, or cyano, and Rₙ is covalently bonded to the L (Linker).

3. The compound of claim 1, a stereoisomer thereof, or a pharmaceutically acceptable salt thereof, wherein the VHL (von Hippel-Lindau tumor suppressor) E3 ubiquitin ligase binding binder is represented by the following chemical formula h: 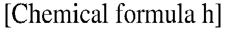 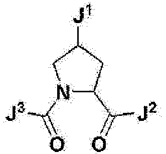 In the chemical formula h,
J¹ is OJ⁴,
J² is optionally unsubstituted or substituted -NJ⁵-(CH₂)o-C₆₋₁₀aryl-5-10 membered heteroaryl comprising one or more heteroatoms selected from the group consisting of N, O, and S,
the substituted-NJ⁵-(CH₂)o-C₆₋₁₀aryl-5-10 membered heteroaryl is substituted with C₁₋₁₀ straight or branched alkyl,
here, the J⁵ is H or C₁₋₅ straight or branched alkyl, and o is 0-5, and
J³ is -CJ⁶-NJ⁷-,
here, J⁶ is C₁₋₁₀ straight or branched alkyl, J⁷ is H or C₁₋₅ straight or branched alkyl, and J³ is covalently bonded to the L (Linker).

4. The compound of claim 1, a stereoisomer thereof, or a pharmaceutically acceptable salt thereof, wherein
the L (linker) is at least one selected from the group consisting of single bond, -(CH₂)ᵢ-, phenylene, -(CH₂)ᵢ-O-, -(CH₂-CH₂-O)ᵢ-, -(CH₂)ᵢ-S-, -(CH₂)ᵢ-NR-, -(CH₂)ᵢ-W₁U₁-, 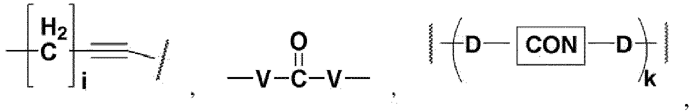 and combinations thereof, here,
D is each independently a single bond, 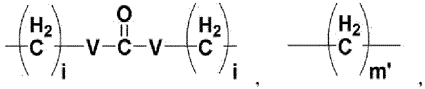 or 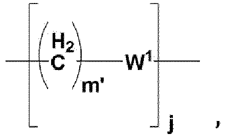
each i, m' or j is each independently 0 to 100,
V is independently a single bond, O, S, or N-R,
W₁U₁ forms an amide group, a urethane group, an ester or thioester group,
W¹ is O, S, or N-R,
R is H, C₁₋₃ alkyl, an alkanol group, or 3 to 10 membered heterocycloalkyl comprising one or more heteroatoms selected from the group consisting of N, O, and S,
CON is a single bond, piperazinyl, substituted or unsubstituted alkylene, 3 to 10 membered heterocycloalkylene comprising one or more heteroatoms selected from the group consisting of N, O, and S, 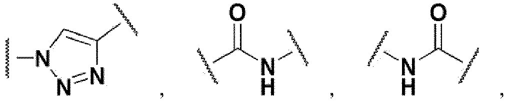 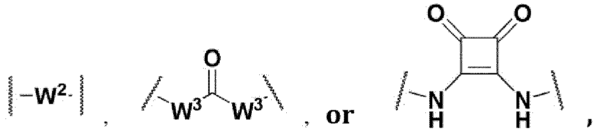
here again, W² is O, S, NR', S(O), S(O)₂, -S(O)₂O, or -OS(O)₂O,
W³ is O, S, CHR⁶, or NR⁶, and
R' is H, or C₁₋₃ alkyl unsubstituted or substituted with one or two hydroxy.

5. The compound of claim 1, a stereoisomer thereof, or a pharmaceutically acceptable salt thereof, wherein the L (Linker) is any one selected from the group consisting of 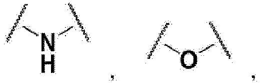 C₁₋₂₀ straight or branched alkylene, C₁₋₂₀ straight or branched alkenylene containing one or more double bonds, C₁₋₂₀ straight or branched alkynylene containing one or more triple bonds, phenylene, 5 to 6 membered heteroarylene comprising one or more heteroatoms selected from the group consisting of N, O, and S, C₃₋₁₀ cycloalkylene, and 3 to 10 membered heterocycloalkylene comprising one or more heteroatoms selected from the group consisting of N, O and S,
or their combination of 2 to 20.

6. The compound of claim 1, a stereoisomer thereof, or a pharmaceutically acceptable salt thereof, wherein the EED (embryonic ectoderm development) targeting molecule is represented by one of the chemical formulas represented by the following (i) to (j) and (i') to (j'): 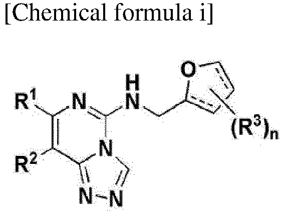 in the chemical formula i,
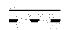 is a single bond or a double bond;
R¹ is independently selected from H, halogen and C1-C4 alkyl;
R₂ is independently selected from halogen, phenyl, and 5- to 6-membered heteroaryl comprising carbon atoms and 1-4 heteroatoms selected from N, NR^{a}, O, and S(O)ₚ; wherein said phenyl and heteroaryl are substituted with 0-3 R^{2A};
each R^{2A} is independently halogen, CN, -(O)ₘ-(C1-C6 alkyl substituted with 0-1 R^{2B}), C1-C6 haloalkyl, C1-C6 haloalkoxy, R^{2C}, -OR^{2C}, -C(=O)R^{2D}, NR^{2E}R^{2F}, -C(=O)NR^{2E}R^{2F}, - NHC(=O)R^{2D}, -S(=O)₂R^{2D}, -S(=O)₂NR^{2E}R^{2F}, -NHS(=O)₂(C1-C4alkyl), and -CR^{2C}R^{2E}R^{2G};
R^{2B} is independently OH, NR^{e}R^{f}, C1-C4 alkoxy, -C(=O)NR^{e}R^{f}, -S(=O)₂(C1-C4 alkyl), - NHC(=O)(C1-C4 alkyl), and 5- to 6-membered heterocycloalkyl comprising carbon atoms and 1-2 heteroatoms selected from N, NR^{a}, O, and S(O)ₚ; wherein said heterocycloalkyl is substituted with 0-2 R^{c};
each R^{2C} is independently selected from C3-C6 cycloalkyl, phenyl, and 4- to 7-membered heterocycle comprising carbon atoms and 1-4 heteroatoms selected from N, NR^{a}, O, and S(O)ₚ; wherein each moiety is substituted with 0-2 R^{c};
each R^{2D} is independently selected from C1-C4 alkyl and R^{2C};
R^{2E} and R^{2G} at each occurrence are independently selected from H and C1-C4 alkyl;
each R^{2F} is independently selected from H and C1-C4 alkyl substituted with 0-1 R^{d};
R³ is independently selected from OH and C1-C4 alkyl;
each R^{a} is independently H, O, C1-C4 alkyl substituted with 0-1 R^{b}, -C(=O)H, -C(=O)(C1-C4 alkyl), -CO2(C1-C4) alkyl), C3-C6 cycloalkyl, and benzyl;
R^{b} is independently selected from halogen, OH and C1-C4 alkoxy;
each R^{c} is independently selected from =O, halogen, OH, C1-C4 alkyl, C1-C4 haloalkyl, C1-C4 alkoxy, and C1-C4 haloalkoxy;
R^{d} is independently selected from OH and NR^{e}R^{f};
R^{e} and R^{f} at each occurrence are independently selected from H and C1-C4 alkyl;
each p is independently selected from 0, 1 and 2;
m and n at each occurrence are independently selected from 0 and 1); 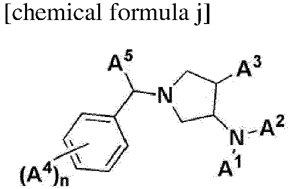
in the chemical formula j,
A¹ and A² are independently C1-C2 alkyl;
A³ is a substituted or unsubstituted C₆₋₁₀ aryl, a 5- to 10-membered substituted or unsubstituted heteroaryl comprising at least one hetero atom selected from the group consisting of N, O, and S, or 3- to 10-membered substituted or unsubstituted heterocycloalkyl comprising one or more heteroatoms selected from the group consisting of N, O, and S,
wherein said substituted aryl, substituted heteroaryl, and substituted heterocycloalkyl are substituted with one or more substituents selected from the group consisting of R¹, OR¹, SR¹, S(O)R¹, SO₂R¹, C(O)R¹, CO(O)R¹, OC(O)R¹, OC(O)OR¹, NH₂, NHR¹, N(R¹)₂, NHC(O)R¹, NR¹C(O)R¹, NHS(O)₂R¹, NR¹S(O)₂R¹, NHC(O)OR¹ NR¹C(O)OR¹, NHC(O)NH₂, NHC(O)NHR¹, NHC(O)N(R¹)₂, NR1C(O)NHR¹, NR¹C(O)N(R¹)₂, C(O)NH₂, C(O)NHR¹, C(O)N(R¹)₂, C(O)NHOH, C(O)NHOR¹, C(O)NHSO₂R¹ C(O)NR¹SO₂R¹, SO₂NH₂, SO₂NHR¹, SO₂N(R¹)₂, C(O)H, C(O)OH, C(N)NH₂, C(N)NHR¹, C(N)N(R¹)₂, CNOH, CNOCH₃, OH, (O), CN, NO₂, CF₃, CF₂CF₃, OCF₃, OCF₂CF₃, F, Cl, Br and I;
A⁴ is each independently a substituent selected from the group consisting of straight or branched C1-C6 alkyl, OH and halogen, and n is 0 to 5;
A⁵ is a substituent selected from the group consisting of straight or branched C1-C6 alkyl, OH and halogen;
R¹ is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R¹ is straight or branched C1-C6 alkyl, straight or branched C2-C6 alkenyl, and straight or branched C2-C6 alkynyl, it is optionally substituted with one or more substituents selected from the group consisting of R², OR², SR², S(O)R², SO₂R², C(O)R², CO(O)R², OC(O)R², OC(O)OR², NH₂, NHR², N(R²)², NHC(O)R², NR²C(O)R², NHS(O)₂R², NR²S(O)₂R², NHC(O)OR², NR²C(O)OR², NHC(O)NH₂, NHC(O)NHR², NHC(O)N(R²)₂, NR²C(O)NHR², NR²C(O)N(R²)₂, C(O)NH₂, C(O)NHR², C(O)N(R²)₂, C(O)NHOH, C(O)NHOR₂, C(O)NHSO₂R², C(O)NR²SO₂R², SO₂NH₂, SO₂NHR², SO₂N(R²)₂, C(O)H, C(O)OH, OH, (O), CN, NO₂, CF₃, CF₂CF₃, OCF₃, OCF₂CF₃, and halogen,
on the other hand, when R¹ is aryl, heteroaryl, heterocycloalkyl, cycloalkyl, and cycloalkenyl, it is optionally substituted with one or more substituents selected from the group consisting of R³, OR³, SR³, S(O)R³, SO₂R³, C(O)R³, CO(O)R³, OC(O)R³, OC(O)OR³, NH₂, NHR³, N(R³)₂, NHC(O)R³, NR³C(O)R³, NHS(O)₂R³, NR³S(O)₂R³, NHC(O)OR³, NR³C(O)OR³, NHC(O)NH₂, NHC(O)NHR³, NHC(O)N(R³)₂, NR³C(O)NHR³, NR³C(O)N(R³)₂, C(O)NH₂, C(O)NHR³, C(O)N(R³)₂, C(O)NHOH, C(O)NHOR³, C(O)NHSO₂R³, C(O)NR³SO₂R³, SO₂NH₂, SO₂NHR³, SO₂N(R³)₂, C(O)H, C(O)OH, C(O)C(O)NH₂, C(O)C(O)NHR³, C(O)C(O)N(R³)₂, C(N)NH₂, C(N)NHR³, C(N)N(R³)₂, CNOH, CNOCH₃, OH, (O), CN, NO₂, CF₃, CF₂CF₃, OCF₃, OCF₂CF₃, and halogen;
R₂ is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R₂ is straight or branched C1-C6 alkyl, straight or branched C2-C6 alkenyl, and straight or branched C2-C6 alkynyl, it is optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen,
on the other hand, when R₂ is aryl, heteroaryl, heterocycloalkyl, cycloalkyl, and cycloalkenyl, it is optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen;
R³ is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R³ is straight or branched C1-C6 alkyl, straight or branched C2-C6 alkenyl, and straight or branched C2-C6 alkynyl, it is optionally substituted with one or more substituents selected from the group consisting of R⁴, OR⁴, SR⁴, S(O)R⁴, SO₂R⁴, C(O)R⁴, CO(O)R⁴, OC(O)R⁴, OC(O)OR⁴, NH₂, NHR⁴, N(R⁴)₂, NHC(O)R⁴, NR⁴C(O)R⁴, NHS(O)₂R⁴, NR⁴S(O)₂R⁴, NHC(O)OR₄, NR⁴C(O)OR⁴, NHC(O)NH₂, NHC(O)NHR⁴, NHC(O)N(R⁴)₂, NR⁴C(O)NHR⁴, NR⁴C(O)N(R⁴)₂, C(O)NH₂, C(O)NHR⁴, C(O)N(R⁴)₂, C(O)NHOH, C(O)NHOR⁴, C(O)NHSO₂R⁴, C(O)NR⁴SO₂R⁴, SO₂NH₂, SO₂NHR⁴, SO₂N(R⁴)₂, C(O)H, C(O)OH, OH, (O), CN, NO₂, CF₃, CF₂CF₃, OCF₃, OCF₂CF₃, and halogen,
meanwhile, when R³ is aryl, heteroaryl, heterocycloalkyl, cycloalkyl, and cycloalkenyl, it is optionally substituted with one or more substituents selected from the group consisting of R⁵, OR⁵, SR⁵, S(O)R⁵, SO₂R⁵, C(O)R⁵, CO(O)R⁵, OC(O)R⁵, OC(O)OR⁵, NH₂, NHR⁵, N(R⁵)₂, NHC(O)R⁵, NR⁵C(O)R⁵, NHS(O)₂R⁵, NR⁵S(O)₂R⁵, NHC(O)OR⁵, NR⁵C(O)OR⁵, NHC(O)NH₂, NHC(O)NHR⁵, NHC(O)N(R⁵)₂, NR⁵C(O)NHR⁵, NR⁵C(O)N(R⁵)₂, C(O)NH₂, C(O)NHR⁵, C(O)N(R⁵)₂, C(O)NHOH, C(O)NHOR⁵, C(O)NHSO₂R⁵, C(O)NR⁵SO₂R⁵, SO₂NH₂, SO₂NHR⁵, SO₂N(R⁵)₂, C(O)H, C(O)OH, C(N)NH₂, C(N)NHR⁵, C(N)N(R⁵)₂, CNOH, CNOCH₃, OH, (O), CN, NO₂, CF₃, CF₂CF₃, OCF₃, OCF₂CF₃, and halogen;
R⁴ is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R⁴ is straight or branched C1-C6 alkyl, straight or branched C2-C6 alkenyl, and straight or branched C2-C6 alkynyl, it is optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen,
meanwhile, when R⁴ is aryl, heteroaryl, heterocycloalkyl, cycloalkyl, and cycloalkenyl, it is optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen;
R⁵ is each independently selected from the group consisting of straight or branched C1-C6 alkyl, straight or branched C2-C6 alkenyl, straight or branched C2-C6 alkynyl, C6-10 aryl, 5-to 10-membered heteroaryl comprising one or more heteroatoms selected from the group consisting of N, O, and S, 3- to 10-membered heterocycloalkyl comprising one or more heteroatoms selected from the group consisting of N, O, and S, C3-C10 cycloalkyl, and C3-C10 cycloalkenyl,
wherein, when R⁵ is straight or branched C1-C6 alkyl, straight or branched C2-C6 alkenyl, and straight or branched C2-C6 alkynyl, it is optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen,
meanwhile, when R⁵ is aryl, heteroaryl, heterocycloalkyl, cycloalkyl, and cycloalkenyl, it is optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, and halogen); 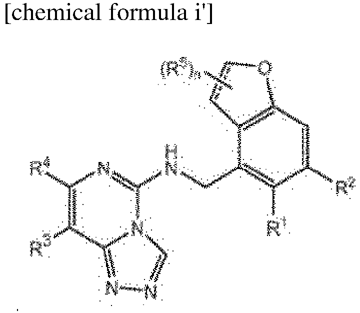
in the chemical formula i',
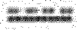 is a single bond or a double bond;
R¹ and R² are independently H or halogen;
R³ is independently selected from halogen, phenyl, and 5- to 6-membered heteroaryl comprising carbon atoms and 1-4 heteroatoms selected from N, NR^{a}, O, and S(O)ₚ; wherein said phenyl and heteroaryl are substituted with 0-3 R^{3A};
each R^{3A} is independently selected from halogen, CN, -(O)ₘ-(C₁-C₆ alkyl substituted with 0-1 R^{3B}), C₁-C₆ haloalkyl, C₁-C₆ haloalkoxy, R^{3C}, -OR^{3C}, -C(=O)R^{3D}, NR^{3E}R^{3F}, -C(=O)NR^{3E}R^{3F}, -NHC(=O)R^{3D}, -S(=O)₂R^{3D}, -S(=O)₂NR^{3E}R^{3F}, -NHS(=O)₂(C₁-C₄ alkyl), and -CR^{3C}R^{3E}R^{3G};
R^{3B} is independently selected from OH, NR^{e}R^{f}, C1-C4 alkoxy, -C(=O)NR^{e}R^{f}, -S(=O)₂(C₁-C₄ alkyl), -NHC(=O)(C₁-C₄ alkyl), and 5- to 6-membered heterocycloalkyl comprising carbon atoms and 1-2 heteroatoms selected from N, NR^{a}, O, and S(O)ₚ; wherein said heterocycloalkyl is substituted with 0-2 R^{c};
each R^{3C} is independently selected from C₃-C₆ cycloalkyl, phenyl, and 4- to 7-membered heterocycle comprising carbon atoms and 1-4 heteroatoms selected from N, NR^{a}, O, and S(O)ₚ; wherein each moiety is substituted with 0-2 R^{c};
each R^{3D} is independently selected from C₁-C₄ alkyl and R^{3C};
R^{3E} and R^{3G} at each occurrence are independently selected from H and C₁-C₄ alkyl;
each R^{3F} is independently selected from H and C₁-C₄ alkyl substituted with 0-1 R^{d};
R⁴ is independently selected from H, halogen and C₁-C₄ alkyl;
R⁵ is independently selected from OH and C₁-C₄ alkyl;
each R^{a} is independently H, →O, C₁-C₄ alkyl substituted with 0-1 R^{b}, -C(=O)H, - C(=O)(C₁-C₄ alkyl), -CO₂(C₁-C₄ alkyl), C3-C6 cycloalkyl, and benzyl;
R^{b} is independently selected from halogen, OH and C₁-C₄ alkoxy;
each R^{c} is independently selected from =O, halogen, OH, C₁-C₄ alkyl, C₁-C₄ haloalkyl, C₁-C₄ alkoxy, and C₁-C₄ haloalkoxy;
R^{d} is independently selected from OH and NR^{e}R^{f};
R^{e} and R^{f} at each occurrence are independently selected from H and C₁-C₄ alkyl;
each p is independently selected from 0, 1 and 2;
m and n at each occurrence are independently selected from 0 and 1; and 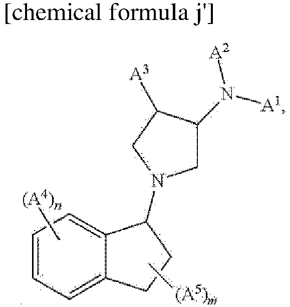
in the chemical formula j',
A¹ and A² are each independently C₁-C₂ alkyl;
A³ is selected from the group consisting of aryl, heterocyclyl and heteroaryl, wherein the aryl, heterocyclyl and heteroaryl of A³ are optionally substituted with one or more substituents selected from the group consisting of R¹, OR¹, SR¹, S(O)R¹, SO₂R¹, C(O)R¹, CO(O)R¹, OC(O)R¹, OC(O)OR¹, NH₂, NHR¹, N(R¹)₂, NHC(O)R¹, NR¹C(O)R¹, NHS(O)₂R¹, NR¹S(O)₂R¹, NHC(O)OR¹ NR¹C(O)OR¹, NHC(O)NH₂, NHC(O)NHR¹, NHC(O)N(R¹)₂, NR¹C(O)NHR¹, NR¹C(O)N(R¹)₂, C(O)NH₂, C(O)NHR¹, C(O)N(R¹)₂, C(O)NHOH, C(O)NHOR¹, C(O)NHSO₂R¹, C(O)NR¹SO₂R¹, SO₂NH₂, SO₂NHR¹, SO₂N(R¹)2, C(O)H, C(O)OH, C(N)NH₂, C(N)NHR¹, C(N)N(R¹)₂, CNOH, CNOCH₃, OH, (O), CN, NO₂, CF₃, CF₂CF₃, OCF₃, OCF₂CF₃, F, Cl, Br and I;
each A⁴ is a substituent independently selected from the group consisting of C₁-C₆ alkyl, OH, F, Cl, Br and I, and is a substituent on the substitutable position of the benzene ring of indane;
each A⁵ is a substituent independently selected from the group consisting of C₁-C₆ alkyl, OH, F, Cl, Br and I, and is a substituent on a substitutable position of the cyclopentane ring of indane;
each R¹ is independently selected from the group consisting of C₁-C₆ alkyl, C₂-C₆ alkenyl, C₂-C₆ alkynyl, aryl, heteroaryl, heterocyclyl, cycloalkyl and cycloalkenyl; wherein C₁-C₆ alkyl, C2-C6 alkenyl, and C₂-C₆ alkynyl of each R¹ are optionally substituted with one or more substituents selected from the group consisting of R², OR², SR², S(O)R², SO₂R², C(O)R², CO(O)R², OC(O)R², OC(O)OR², NH₂, NHR², N(R²)₂, NHC(O)R², NR²C(O)R², NHS(O)₂R², NR²S(O)₂R², NHC(O)OR², NR²C(O)OR², NHC(O)NH₂, NHC(O)NHR², NHC(O)N(R²)₂, NR²C(O)NHR², NR²C(O)N(R²)₂, C(O)NH₂, C(O)NHR², C(O)N(R²)₂, C(O)NHOH, C(O)NHOR², C(O)NHSO₂R², C(O)NR²SO₂R², SO₂NH₂, SO₂NHR², SO₂N(R²)₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R¹ are optionally substituted with one or more substituents selected from the group consisting of R³, OR³, SR³, S(O)R³, SO₂R³, C(O)R³, CO(O)R³, OC(O)R³, OC(O)OR³, NH₂, NHR³, N(R³)₂, NHC(O)R³, NR³C(O)R³, NHS(O)₂R³, NR³S(O)₂R³, NHC(O)OR³, NR³C(O)OR³, NHC(O)NH₂, NHC(O)NHR³, NHC(O)N(R³)₂, NR³C(O)NHR³, NR³C(O)N(R³)₂, C(O)NH₂, C(O)NHR³, C(O)N(R³)₂, C(O)NHOH, C(O)NHOR³, C(O)NHSO₂R³, C(O)NR³SO₂R³, SO₂NH₂, SO₂NHR³, SO₂N(R³)₂, C(O)H, C(O)OH, C(O)C(O)NH₂, C(O)C(O)NHR³, C(O)C(O)N(R³)₂, C(N)NH₂, C(N)NHR³, C(N)N(R³)₂, CNOH, CNOCH₃, OH, (O), CN, NO₂, CF₃, CF₂CF₃, OCF₃, OCF₂CF₃, F, Cl, Br and I;
each R² is independently selected from the group consisting of C₁-C₆ alkyl, C₂-C₆ alkenyl, C₂-C₆ alkynyl, aryl, heteroaryl, heterocyclyl, cycloalkyl and cycloalkenyl; wherein C₁-C₆ alkyl, C2-C6 alkenyl, and C₂-C₆ alkynyl of each R² are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R² are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I;
each R3 is independently selected from the group consisting of C₁-C₆ alkyl, C₂-C₆ alkenyl, C₂-C₆ alkynyl, aryl, heteroaryl, heterocyclyl, cycloalkyl and cycloalkenyl; wherein C₁-C₆ alkyl, C2-C6 alkenyl, and C₂-C₆ alkynyl of each R³ are optionally substituted with one or more substituents selected from the group consisting of R⁴, OR⁴, SR⁴, S(O)R⁴, SO₂R⁴, C(O)R⁴, CO(O)R⁴, OC(O)R⁴, OC(O)OR⁴, NH₂, NHR⁴, N(R⁴)₂, NHC(O)R⁴, NR⁴C(O)R⁴, NHS(O)₂R⁴, NR⁴S(O)₂R⁴, NHC(O)OR⁴, NR⁴C(O)OR⁴, NHC(O)NH₂, NHC(O)NHR⁴, NHC(O)N(R⁴)₂, NR⁴C(O)NHR⁴, NR⁴C(O)N(R⁴)₂, C(O)NH₂, C(O)NHR⁴, C(O)N(R⁴)₂, C(O)NHOH, C(O)NHOR,⁴ C(O)NHSO₂R⁴, C(O)NR⁴SO₂R⁴, SO₂NH₂, SO₂NHR⁴, SO₂N(R⁴)₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R³ are optionally substituted with one or more substituents selected from the group consisting of R⁵, OR⁵, SR⁵, S(O)R⁵, SO₂R⁵, C(O)R⁵, CO(O)R⁵, OC(O)R⁵, OC(O)OR⁵, NH₂, NHR⁵, N(R⁵)₂, NHC(O)R⁵, NR⁵C(O)R⁵, NHS(O)₂R⁵, NR⁵S(O)₂R⁵, NHC(O)OR⁵, NR⁵C(O)OR⁵, NHC(O)NH₂, NHC(O)NHR⁵, NHC(O)N(R⁵)₂, NR⁵C(O)NHR⁵, NR⁵C(O)N(R⁵)₂, C(O)NH₂, C(O)NHR⁵, C(O)N(R⁵)₂, C(O)NHOH, C(O)NHOR⁵, C(O)NHSO₂R⁵, C(O)NR⁵SO₂R⁵, SO₂NH₂, SO₂NHR⁵, SO₂N(R⁵)₂, C(O)H, C(O)OH, C(N)NH₂, C(N)NHR⁵, C(N)N(R⁵)₂, CNOH, CNOCH₃, OH, (O), CN, NO₂, CF₃, CF₂CF₃, OCF₃, OCF₂CF₃, F, Cl, Br and I;
each R⁴ is independently selected from the group consisting of C₁-C₆ alkyl, C₂-C₆ alkenyl, C₂-C₆ alkynyl, aryl, heteroaryl, heterocyclyl, cycloalkyl and cycloalkenyl; wherein C₁-C₆ alkyl, C2-C6 alkenyl, and C₂-C₆ alkynyl of each R⁴ are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R⁴ are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I; and
each R⁵ is independently selected from the group consisting of C₁-C₆ alkyl, C₂-C₆ alkenyl, C₂-C₆ alkynyl, aryl, heteroaryl, heterocyclyl, cycloalkyl and cycloalkenyl; wherein C₁-C₆ alkyl, C2-C6 alkenyl, and C₂-C₆ alkynyl of each R⁵ are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I; wherein aryl, heteroaryl, heterocyclyl, cycloalkyl, and cycloalkenyl of each R⁵ are optionally substituted with one or more substituents selected from the group consisting of NH₂, C(O)NH₂, SO₂NH₂, C(O)H, C(O)OH, OH, (O), CN, NO₂, CF₃, CF₂CF₃, OCF₃, OCF₂CF₃, F, Cl, Br and I;
n is 0, 1 or 2; and
m is 0 or 1.

7. The compound of claim 1, a stereoisomer thereof, or a pharmaceutically acceptable salt thereof, wherein
the PTM is 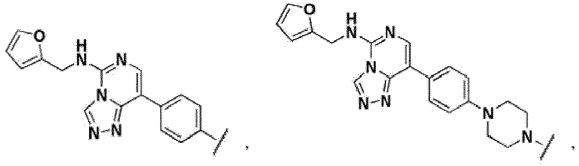 or 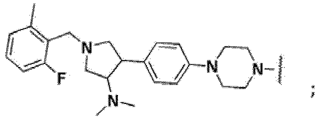
the L (Linker) is 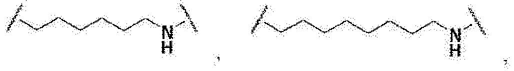 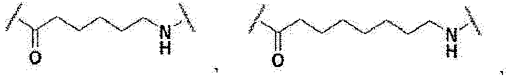 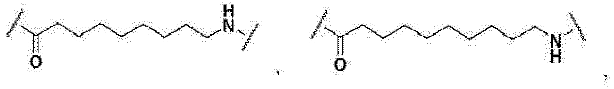 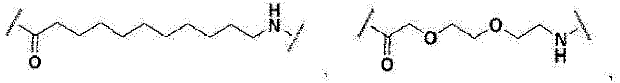 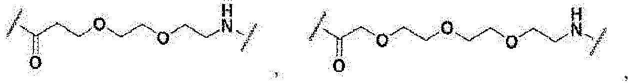 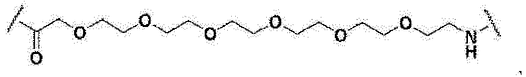 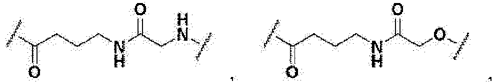 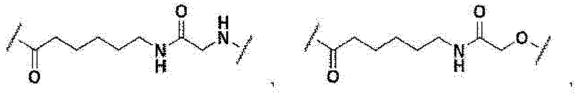 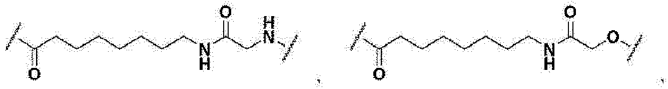 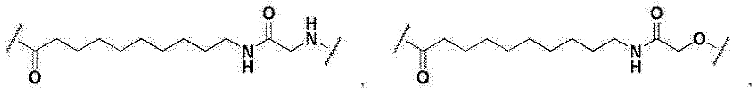 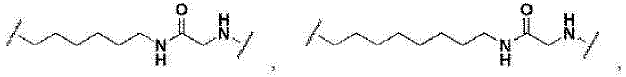 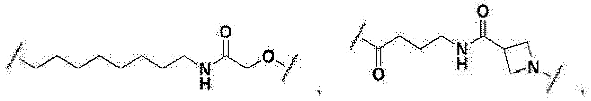 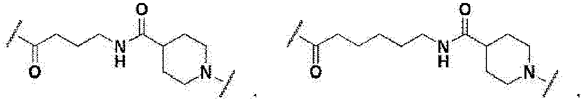 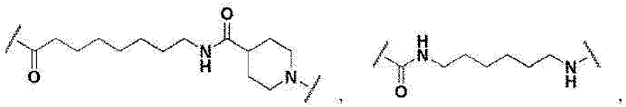 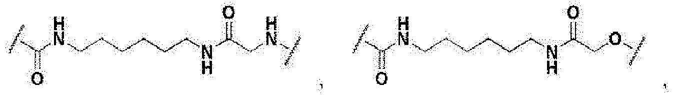 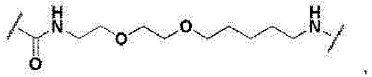 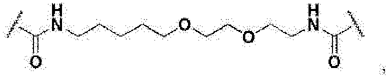 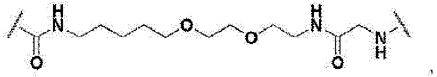 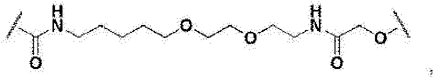 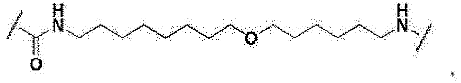 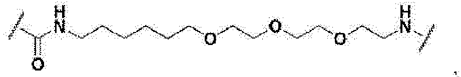 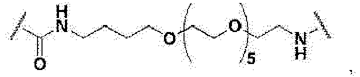 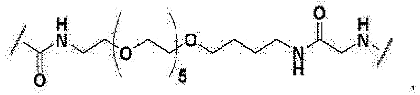 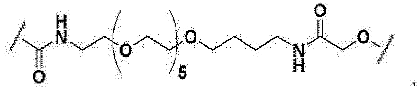 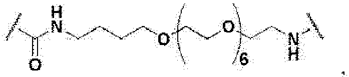 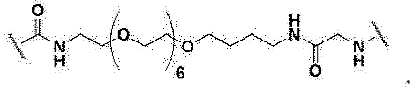 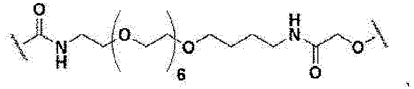 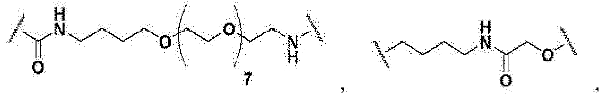 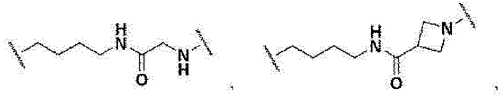 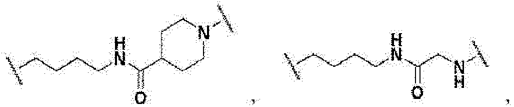 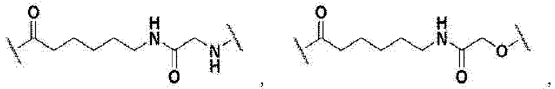 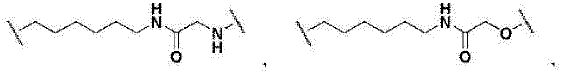 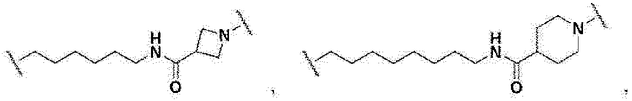 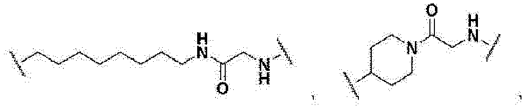 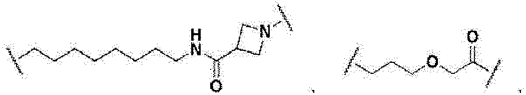 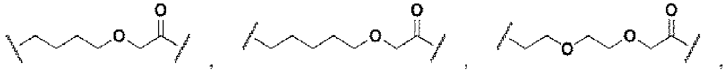 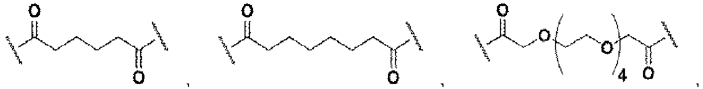 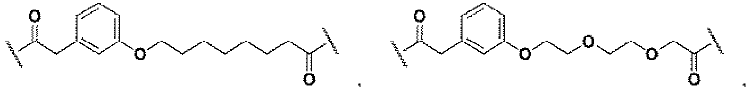 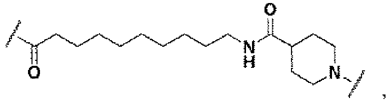 **or** 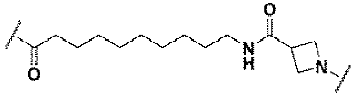 **; and**
the ULB is 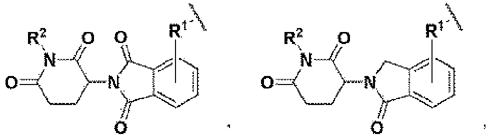 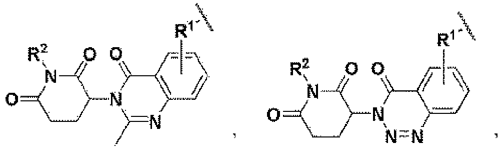 or 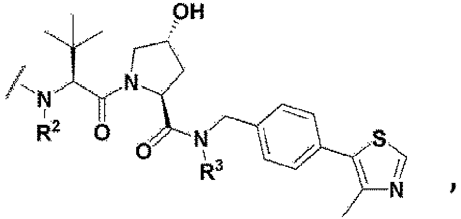
wherein R¹ is -(CH₂)ₙ-NR³-, -(CH₂)ₙ-O-, -O-(CH₂)ₙ-(C=O)-NR³- or -NR³-(CH₂)ₙ-(C=O)-NR³-,
here again, where n is an integer from 0 to 5,
the R² and R³ are each independently H, or unsubstituted or substituted C₁₋₁₀ straight or branched alkyl, and
here again, the substituted alkyl is substituted with a halogen, nitro, or cyano.

8. The compound of claim 1, a stereoisomer thereof, or a pharmaceutically acceptable salt thereof, wherein the compound represented by chemical formula 1 is a compound selected from the group consisting of:
(1) 2-(2,6-dioxopiperidin-3-yl)-4-(6-(4-(4-(5-(furan-2-ylmethylamino)-[1,2,4]triazolo[4,3-f]pyrimidin-8-yl)phenyl)piperazin-1-yl)hexylamino)isoindolin-1,3-dione;
(2) 2-(2,6-dioxopiperidin-3-yl)-4-(8-(4-(4-(5-(furan-2-ylmethylamino)-[1,2,4]triazolo[4,3-f]pyrimidin-8-yl)phenyl)piperazin-1-yl)octylamino)isoindolin-1,3-dione;
(3) 2-(2,6-dioxopiperidin-3-yl)-4-(6-(4-(4-(5-(furan-2-ylmethylamino)-[1,2,4]triazolo[4,3-f]pyrimidin-8-yl)phenyl)piperazin-1-yl)-6-oxohexylamino)isoindolin-1,3-dione;
(4) 2-(2,6-dioxopiperidin-3-yl)-4-(8-(4-(4-(5-(furan-2-ylmethylamino)-[1,2,4]triazolo[4,3-f]pyrimidin-8-yl)phenyl)piperazin-1-yl)-8-oxooctylamino)isoindolin-1,3-dione;
(5) 2-(2,6-dioxopiperidin-3-yl)-4-(9-(4-(4-(5-(furan-2-ylmethylamino)-[1,2,4]triazolo[4,3-f]pyrimidin-8-yl)phenyl)piperazin-1-yl)-9-oxononylamino)isoindolin-1,3-dione;
(6) 2-(2,6-dioxopiperidin-3-yl)-4-(10-(4-(4-(5-(furan-2-ylmethylamino)-1,2,4]triazolo[4,3-f]pyrimidin-8-yl)phenyl)piperazin-1-yl)-10-oxodecylamino)isoindolin-1,3-dione;
(7) 2-(2,6-dioxopiperidin-3-yl)-4-(11-(4-(4-(5-(furan-2-ylmethylamino)-[1,2,4]triazolo[4,3-f]pyrimidin-8-yl)phenyl)piperazin-1-yl)-11-oxoundecylamino)isoindolin-1,3-dione;
(8) 2-(2,6-dioxopiperidin-3-yl)-4-(2-(2-(2-(4-(4-(5-(furan-2-ylmethylamino)-[1,2,4]triazolo[4,3-f]pyrimidin-8-yl)phenyl)piperazin-1-yl)-2-oxoethoxy)ethoxy)ethylamino)isoindolin-1,3-dione;
(9) 2-(2,6-dioxopiperidin-3-yl)-4-(2-(2-(3-(4-(4-(5-(furan-2-ylmethylamino)-[1,2,4]triazolo[4,3-f]pyrimidin-8-yl)phenyl)piperazin-1-yl)-3-oxopropoxy)ethoxy)ethylamino)isoindolin-1,3-dione;
(10) 2-(2,6-dioxopiperidin-3-yl)-4-(2-(2-(2-(2-(4-(4-(5-(furan-2-ylmethylamino)-1,2,4]triazolo[4,3-f]pyrimidin-8-yl)phenyl)piperazin-1-yl)-2-oxoethoxy)ethoxy)ethoxy)ethylamino)isoindolin-1,3-dione;
(11) 2-(2,6-dioxopiperidin-3-yl)-4-(20-(4-(4-(5-(furan-2-ylmethylamino)-[1,2,4]triazolo[4,3-f]pyrimidin-8-yl)phenyl)piperazin-1-yl)-20-oxo-3,6,9,12,15,18-hexaoxicosylamino)isoindolin-1,3-dione;
(12) 2-(2-(2,6-dioxopiperidin-3-yl)-1,3-dioxoisoindolin-4-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide;
(13) 2-(2-(2,6-dioxopiperidin-3-yl)-1,3-dioxoisoindolin-4-yloxy)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide;
(14) 2-(2-(2,6-dioxopiperidin-3-yl)-1,3-dioxoisoindolin-4-ylamino)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide;
(15) 2-(2-(2,6-dioxopiperidin-3-yl)-1,3-dioxoisoindolin-4-yloxy)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide;
(16) 2-(2-(2,6-dioxopiperidin-3-yl)-1,3-dioxoisoindolin-4-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide:
(17) 2-(2-(2,6-dioxopiperidin-3-yl)-1,3-dioxoisoindolin-4-yloxy)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide;
(18) 2-(2-(2,6-dioxopiperidin-3-yl)-1,3-dioxoisoindolin-4-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide;
(19) 2-(2-(2,6-dioxopiperidin-3-yl)-1,3-dioxoisoindolin-4-yloxy)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide;
(20) 2-(2-(2,6-dioxopiperidin-3-yl)-1,3-dioxoisoindolin-4-ylamino)-N-(6-(4-(4-(5-(furan-2-ylmethylamino)-[1,2,4]triazolo[4,3-f]pyrimidin-8-yl)phenyl)piperazin-1-yl)hexyl)acetamide;
(21) 2-(2-(2,6-dioxopiperidin-3-yl)-1,3-dioxoisoindolin-4-ylamino)-N-(8-(4-(4-(5-(furan-2-ylmethylamino)-[1,2,4]triazolo[4,3-f]pyrimidin-8-yl)phenyl)piperazin-1-yl)octyl)acetamide;
(22) 2-(2,6-dioxopiperidin-3-yl)-5-(8-(4-(4-(5-(furan-2-ylmethylamino)-[1,2,4]triazolo[4,3-f]pyrimidin-8-yl)phenyl)piperazin-1-yl)-8-oxooctylamino)isoindolin-1,3-dione;
(23) 2-(2,6-dioxopiperidin-3-yl)-5-(10-(4-(4-(5-(furan-2-ylmethylamino)-[1,2,4]triazolo[4,3-f]pyrimidin-8-yl)phenyl)piperazin-1-yl)-10-oxodecylamino)isoindolin-1,3-dione;
(24) 2-(2-(2,6-dioxopiperidin-3-yl)-1,3-dioxoisoindolin-5-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide;
(25) 2-(2-(2,6-dioxopiperidin-3-yl)-1,3-dioxoisoindolin-5-ylamino)-N-(6-(4-(4-(5-(furan-2-ylmethylamino)-[1,2,4]triazolo[4,3-f]pyrimidin-8-yl)phenyl)piperazin-1-yl)-6-oxohexyl)acetamide;
(26) 2-(2-(2,6-dioxopiperidin-3-yl)-1,3-dioxoisoindolin-5-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide;
(27) 2-(2-(2,6-dioxopiperidin-3-yl)-1,3-dioxoisoindolin-5-ylamino)-N-(10-(4-(4-(5-(furan-2-ylmethylamino)-[1,2,4]triazolo[4,3-f]pyrimidin-8-yl)phenyl)piperazin-1-yl)-10-oxodecyl)acetamide;
(28) 1-(2-(2,6-dioxopiperidin-3-yl)-1,3-dioxoisoindolin-4-yl)-N-(4-(4-(4-(5-(furan-2-ylmethylamino)-[1,2,4]triazolo[4,3-f]pyrimidin-8-yl)phenyl)piperazin-1-yl)-4-oxobutyl)azetidin-3-carboxamide;
(29) 1-(2-(2,6-dioxopiperidin-3-yl)-1,3-dioxoisoindolin-5-yl)-N-(4-(4-(4-(5-(furan-2-ylmethylamino)-[1,2,4]triazolo[4,3-f]pyrimidin-8-yl)phenyl)piperazin-1-yl)-4-oxobutyl)piperidin-4-carboxamide;
(30) 1-(2-(2,6-dioxopiperidin-3-yl)-1,3-dioxoisoindolin-5-yl)-N-(6-(4-(4-(5-(furan-2-ylmethylamino)-[1,2,4]triazolo[4,3-f]pyrimidin-8-yl)phenyl)piperazin-1-yl)-6-oxohexyl)piperidin-4-carboxamide;
(31) 1-(2-(2,6-dioxopiperidin-3-yl)-1,3-dioxoisoindolin-5-yl)-N-(8-(4-(4-(5-(furan-2-ylmethylamino)-[1,2,4]triazolo[4,3-f]pyrimidin-8-yl)phenyl)piperazin-1-yl)-8-oxooctyl)piperidin-4-carboxamide;
(32) N-(6-(2-(2,6-dioxopiperidin-3-yl)-1,3-dioxoisoindolin-4-ylamino)hexy1)-4-(5-(furan-2-ylmethylamino)-[1,2,4]triazolo[4,3-f]pyrimidin-8-yl)benzamide;
(33) N-(6-(2-(2-(2,6-dioxopiperidin-3-yl)-1,3-dioxoisoindolin-4-ylamino)acetamido)hexyl)-4-(5-(furan-2-ylmethylamino)-[1,2,4]triazolo[4,3-f]pyrimidin-8-yl)benzamide;
(34) N-(6-(2-(2-(2,6-dioxopiperidin-3-yl)-1,3-dioxoisoindolin-4-yloxy)acetamido)hexyl)-4-(5-(furan-2-ylmethylamino)-[1,2,4]triazolo[4,3-f]pyrimidin-8-yl)benzamide;
(35) 1-(2-(2,6-dioxopiperidin-3-yl)-1,3-dioxoisoindolin-5-yl)-N-(10-(4-(4-(5-(furan-2-ylmethylamino)-[1,2,4]triazolo[4,3-flpyrimidin-8-yl)phenyl)piperazin-1-yl)-10-oxodecyl)piperidin-4-carboxamide;
(36) 1-(2-(2,6-dioxopiperidin-3-yl)-1,3-dioxoisoindolin-4-yl)-N-(10-(4-(4-(5-(furan-2-ylmethylamino)-[1,2,4]triazolo[4,3-f]pyrimidin-8-yl)phenyl)piperazin-1-yl)-10-oxodecyl)azetidin- 3-carboxamide;
(37) N-(2-(2-(5-(2-(2,6-dioxopiperidin-3-yl)-1,3-dioxoisoindolin-4-ylamino)pentyloxy)ethoxy)ethyl)-4-(5-(furan-2-ylmethylamino)-[1,2,4]triazolo[4,3-f]pyrimidin-8-yl)benzamide;
(38) N-(5-(2-(2-(2-(2-(2,6-dioxopiperidin-3-yl)-1,3-dioxoisoindolin-4-ylamino)acetamido)ethoxy)ethoxy)pentyl)-4-(5-(furan-2-ylmethylamino)-[1,2,4]triazolo[4,3-f]pyrimidin-8-yl)benzamide;
(39) N-(5-(2-(2-(2-(2-(2,6-dioxopiperidin-3-yl)-1,3-dioxoisoindolin-4-yloxy)acetamido)ethoxy)ethoxy)pentyl)-4-(5-(furan-2-ylmethylamino)-[1,2,4]triazolo[4,3-f]pyrimidin-8-yl)benzamide;
(40) N-(8-(6-(2-(2,6-dioxopiperidin-3-yl)-1,3-dioxoisoindolin-4-ylamino)hexyloxy)octyl)-4-(5-(furan-2-ylmethylamino)-[1,2,4]triazolo[4,3-f]pyrimidin-8-yl)benzamide;
(41) N-(6-(2-(2-(2-(2-(2,6-dioxopiperidin-3-yl)-1,3-dioxoisoindolin-4-ylamino)ethoxy)ethoxy)ethoxy)hexyl)-4-(5-(furan-2-ylmethylamino)-[1,2,4]triazolo[4,3-f]pyrimidin-8-yl)benzamide;
(42) N-(1-(2-(2,6-dioxopiperidin-3-yl)-1,3-dioxoisoindolin-4-ylamino)-3,6,9,12,15,18-hexaoxadocosan-22-yl)-4-(5-(furan-2-ylmethylamino)-[1,2,4]triazolo[4,3-f]pyrimidin-8-yl)benzamide;
(43) N-(25-(2-(2,6-dioxopiperidin-3-yl)-1,3-dioxoisoindolin-4-ylamino)-24-oxo-3,6,9,12,15,18-hexaoxa-23-azapentacosyl)-4-(5-(furan-2-ylmethylamino)-[1,2,4]triazolo[4,3-f]pyrimidin-8-yl)benzamide;
(44) N-(25-(2-(2,6-dioxopiperidin-3-yl)-1,3-dioxoisoindolin-4-yloxy)-24-oxo-3,6,9,12,15,18-hexaoxa-23-azapentacosyl)-4-(5-(furan-2-ylmethylamino)-[1,2,4]triazolo[4,3-f]pyrimidin-8-yl)benzamide;
(45) N-(1-(2-(2,6-dioxopiperidin-3-yl)-1,3-dioxoisoindolin-4-ylamino)-3,6,9,12,15,18,21-heptaoxapentacosan-25-yl)-4-(5-(furan-2-ylmethylamino)-[1,2,4]triazolo[4,3-f]pyrimidin-8-yl)benzamide;
(46) N-(28-(2-(2,6-dioxopiperidin-3-yl)-1,3-dioxoisoindolin-4-ylamino)-27-oxo-3,6,9,12,15,18,21-heptaoxa-26-azaoctacosyl)-4-(5-(furan-2-ylmethylamino)-[1,2,4]triazolo[4,3-f]pyrimidin-8-yl)benzamide;
(47) N-(28-(2-(2,6-dioxopiperidin-3-yl)-1,3-dioxoisoindolin-4-yloxy)-27-oxo-3,6,9,12,15,18,21-heptaoxa-26-azaoctacosyl)-4-(5-(furan-2-ylmethylamino)-[1,2,4]triazolo[4,3-f]pyrimidin-8-yl)benzamide;
(48) N-(1-(2-(2,6-dioxopiperidin-3-yl)-1,3-dioxoisoindolin-4-ylamino)-3,6,9,12,15,18,21,24-octaoxaoctacosan-28-yl)-4-(5-(furan-2-ylmethylamino)-[1,2,4]triazolo[4,3-f]pyrimidin-8-yl)benzamide;
(49) N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-4-yloxy)acetamide;
(50) N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-4-ylamino)acetamide;
(51) N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-4-yl)azetidin-3-carboxamide;
(52) N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-4-yl)piperidin-4-carboxamide;
(53) N-(4-(4-(4-((3S,4R)-4-(dimethylamino)-1-(2-fluoro-6-methylbenzyl)pyrrolidin-3-yl)phenyl)piperazin-1-yl)butyl)-2-(2-(2,6-dioxopiperidin-3-yl)-1,3-dioxoisoindolin-5-ylamino)acetamide;
(54) N-(4-(4-(4-((3S,4R)-4-(dimethylamino)-1-(2-fluoro-6-methylbenzyl)pyrrolidin-3-yl)phenyl)piperazin-1-yl)butyl)-1-(2-(2,6-dioxopiperidin-3-yl)-1,3-dioxoisoindolin-5-yl)piperidin-4-carboxamide;
(55) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-(2-(2,6-dioxopiperidin-3-yl)-1,3-dioxoisoindolin-4-ylamino)acetamide;
(56) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-(2-(2,6-dioxopiperidin-3-yl)-1,3-dioxoisoindolin-4-yloxy)acetamide;
(57) N-(6-(4-(4-((3S,4R)-4-(dimethylamino)-1-(2-fluoro-6-methylbenzyl)pyrrolidin-3-yl)phenyl)piperazin-1-yl)hexyl)-2-(2-(2,6-dioxopiperidin-3-yl)-1,3-dioxoisoindolin-4-ylamino)acetamide;
(58) N-(6-(4-(4-((3S,4R)-4-(dimethylamino)-1-(2-fluoro-6-methylbenzyl)pyrrolidin-3-yl)phenyl)piperazin-1-yl)hexyl)-2-(2-(2,6-dioxopiperidin-3-yl)-1,3-dioxoisoindolin-4-yloxy)acetamide;
(59) N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-4-yl)azetidin-3-carboxamide;
(60) N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-4-yl)piperidin-4-carboxamide;
(61) N-(6-(4-(4-((3S,4R)-4-(dimethylamino)-1-(2-fluoro-6-methylbenzyl)pyrrolidin-3-yl)phenyl)piperazin-1-yl)hexyl)-2-((2-(2,6-dioxopiperidin-3-yl)-1,3-dioxoisoindolin-5-yl)amino)acetamide;
(62) N-(6-(4-(4-((3S,4R)-4-(dimethylamino)-1-(2-fluoro-6-methylbenzyl)pyrrolidin-3-yl)phenyl)piperazin-1-yl)hexyl)-1-(2-(2,6-dioxopiperidin-3-yl)-1,3-dioxoisoindolin-5-yl)piperidin-4-carboxamide;
(63) N-(8-(4-(4-((3S,4R)-4-(dimethylamino)-1-(2-fluoro-6-methylbenzyl)pyrrolidin-3-yl)phenyl)piperazin-1-yl)octyl)-1-(2-(2,6-dioxopiperidin-3-yl)-1,3-dioxoisoindolin-5-yl)piperidin-4-carboxamide;
(64) N-(8-(4-(4-((3S,4R)-4-(dimethylamino)-1-(2-fluoro-6-methylbenzyl)pyrrolidin-3-yl)phenyl)piperazin-1-yl)octyl)-2-((2-(2,6-dioxopiperidin-3-yl)-1,3-dioxoisoindolin-5-yl)amino)acetamide;
(65) 4-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethoxy)-2-(2,6-dioxopiperidin-3-yl)isoindolin-1,3-dione;
(66) 4-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-2-(2,6-dioxopiperidin-3-yl)isoindolin-1,3-dione;
(67) 3-(6-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-4-oxobenzo[d][1,2,3]triazin-3(4H)-yl)piperidin-2,6-dione;
(68) 3-(6-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethoxy)-4-oxobenzo[d][1,2,3]triazin-3(4H)-yl)piperidin-2,6-dione;
(69) 3-(6-((2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethyl)amino)-4-oxobenzo[d][1,2,3]triazin-3(4H)-yl)piperidin-2,6-dione;
(70) 3-(5-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-4-oxobenzo[d][1,2,3]triazin-3(4H)-yl)piperidin-2,6-dione;
(71) 3-(6-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-4-oxobenzo[d][1,2,3]triazin-3(4H)-yl)piperidin-2,6-dione;
(72) 3-(6-(2-(4-((4-(4-(5-(furan-2-ylmethylamino)-[1,2,4]triazolo[4,3-f]pyrimidin-8-yl)phenyl)piperazin-1-yl)methyl)piperidin-1-yl)-2-oxoethylamino)-4-oxobenzo[d][1,2,3]triazin-3(4H)-yl)piperidin-2,6-dione;
(73) 2-(3-(2,6-dioxopiperidin-3-yl)-4-oxo-3,4-dihydrobenzo[d][1,2,3]triazin-6-ylamino)-N-(4-(4-(4-(5-(furan-2-ylmethylamino)-[1,2,4]triazolo[4,3-f]pyrimidin-8-yl)phenyl)piperazin-1-yl)-4-oxobutyl)acetamide;
(74) 1-(3-(2,6-dioxopiperidin-3-yl)-4-oxo-3,4-dihydrobenzo[d][1,2,3]triazin-6-yl)-N-(4-(4-(4-(5-(furan-2-ylmethylamino)-[1,2,4]triazolo[4,3-f]pyrimidin-8-yl)phenyl)piperazin-1-yl)-4-oxobutyl)piperidin-4-carboxamide;
(75) 3-(5-(6-(4-(4-(5-(furan-2-ylmethylamino)-[1,2,4]triazolo[4,3-f]pyrimidin-8-yl)phenyl)piperazin-1-yl)-6-oxohexylamino)-4-oxobenzo[d][1,2,3]triazin-3(4H)-yl)piperidin-2,6-dione;
(76) 3-(6-(6-(4-(4-(5-(furan-2-ylmethylamino)-[1,2,4]triazolo[4,3f]pyrimidin-8-yl)phenyl)piperazin-1-yl)-6-oxohexylamino)-4-oxobenzo[d][1,2,3]triazin-3(4H)-yl)piperidin-2,6-dione;
(77) 3-(5-(8-(4-(4-(5-(furan-2-ylmethylamino)-[1,2,4]triazolo[4,3-f]pyrimidin-8-yl)phenyl)piperazin-1-yl)-8-oxooctylamino)-4-oxobenzo[d][1,2,3]triazin-3(4H)-yl)piperidin-2,6-dione;
(78) 3-(6-(8-(4-(4-(5-(furan-2-ylmethylamino)-[1,2,4]triazolo[4,3-f]pyrimidin-8-yl)phenyl)piperazin-1-yl)-8-oxooctylamino)-4-oxobenzo[d][1,2,3]triazin-3(4H)-yl)piperidin-2,6-dione;
(79) 2-(3-(2,6-dioxopiperidin-3-yl)-4-oxo-3,4-dihydrobenzo[d][1,2,3]triazin-6-ylamino)-N-(8-(4-(4-(5-(furan-2-ylmethylamino)-[1,2,4]triazolo[4,3-f]pyrimidin-8-yl)phenyl)piperazin-1-yl)-8-oxooctyl)acetamide;
(80) 1-(3-(2,6-dioxopiperidin-3-yl)-4-oxo-3,4-dihydrobenzo[d][1,2,3]triazin-6-yl)-N-(8-(4-(4-(5-(furan-2-ylmethylamino)-[1,2,4]triazolo[4,3-f]pyrimidin-8-yl)phenyl)piperazin-1-yl)-8-oxooctyl)piperidin-4-carboxamide;
(81) 3-(5-(10-(4-(4-(5-(furan-2-ylmethylamino)-[1,2,4]triazolo[4,3-f]pyrimidin-8-yl)phenyl)piperazin-1-yl)-10-oxodecylamino)-4-oxobenzo[d][1,2,3]triazin-3(4H)-yl)piperidin-2,6-dione;
(82) 3-(6-(10-(4-(4-(5-(furan-2-ylmethylamino)-[1,2,4]triazolo[4,3-f]pyrimidin-8-yl)phenyl)piperazin-1-yl)-10-oxodecylamino)-4-oxobenzo[d][1,2,3]triazin-3(4H)-yl)piperidin-2,6-dione;
(83) 2-(3-(2,6-dioxopiperidin-3-yl)-4-oxo-3,4-dihydrobenzo[d][1,2,3]triazin-6-ylamino)-N-(10-(4-(4-(5-(furan-2-ylmethylamino)-[1,2,4]triazolo[4,3-flpyrimidin-8-yl)phenyl)piperazin-1-yl)-10-oxodecyl)acetamide;
(84) N-(4-(4-(4-((3S,4R)-4-(dimethylamino)-1-(2-fluoro-6-methylbenzyl)pyrrolidin-3-yl)phenyl)piperazin-1-yl)butyl)-2-(3-(2,6-dioxopiperidin-3-yl)-4-oxo-3,4-dihydrobenzo[d] [1,2,3]triazin-6-ylamino)acetamide;
(85) N-(4-(4-(4-((3S,4R)-4-(dimethylamino)-1-(2-fluoro-6-methylbenzyl)pyrrolidin-3-yl)phenyl)piperazin-1-yl)butyl)-1-(3-(2,6-dioxopiperidin-3-yl)-4-oxo-3,4-dihydrobenzo[d][1,2,3]triazin-6-yl)azetidin-3-carboxamide;
(86) N-(4-(4-(4-((3S,4R)-4-(dimethylamino)-1-(2-fluoro-6-methylbenzyl)pyrrolidin-3-yl)phenyl)piperazin-1-yl)butyl)-1-(3-(2,6-dioxopiperidin-3-yl)-4-oxo-3,4-dihydrobenzo[d] [1,2,3]triazin-6-yl)piperidin-4-carboxamide;
(87) N-(6-(4-(4-((3S,4R)-4-(dimethylamino)-1-(2-fluoro-6-methylbenzyl)pyrrolidin-3-yl)phenyl)piperazin-1-yl)hexyl)-2-(3-(2,6-dioxopiperidin-3-yl)-4-oxo-3,4-dihydrobenzo[d] [1,2,3]triazin-6-ylamino)acetamide;
(88) N-(6-(4-(4-((3S,4R)-4-(dimethylamino)-1-(2-fluoro-6-methylbenzyl)pyrrolidin-3-yl)phenyl)piperazin-1-yl)hexyl)-1-(3-(2,6-dioxopiperidin-3-yl)-4-oxo-3,4-dihydrobenzo[d] [1,2,3]triazin-6-yl)piperidin-4-carboxamide;
(89) N-(6-(4-(4-((3S,4R)-4-(dimethylamino)-1-(2-fluoro-6-methylbenzyl)pyrrolidin-3-yl)phenyl)piperazin-1-yl)hexyl)-1-(3-(2,6-dioxopiperidin-3-yl)-4-oxo-3,4-dihydrobenzo[d][1,2,3]triazin-6-yl)azetidin-3-carboxamide;
(90) N-(8-(4-(4-((3S,4R)-4-(dimethylamino)-1-(2-fluoro-6-methylbenzyl)pyrrolidin-3-yl)phenyl)piperazin-1-yl)octyl)-2-(3-(2,6-dioxopiperidin-3-yl)-4-oxo-3,4-dihydrobenzo[d] [1,2,3]triazin-6-ylamino)acetamide;
(91) N-(8-(4-(4-((3S,4R)-4-(dimethylamino)-1-(2-fluoro-6-methylbenzyl)pyrrolidin-3-yl)phenyl)piperazin-1-yl)octyl)-1-(3-(2,6-dioxopiperidin-3-yl)-4-oxo-3,4-dihydrobenzo[d][1,2,3]triazin-6-yl)azetidin-3-carboxamide;
(92) N-(8-(4-(4-((3S,4R)-4-(dimethylamino)-1-(2-fluoro-6-methylbenzyl)pyrrolidin-3-yl)phenyl)piperazin-1-yl)octyl)-1-(3-(2,6-dioxopiperidin-3-yl)-4-oxo-3,4-dihydrobenzo[d] [1,2,3]triazin-6-yl)piperidin-4-carboxamide;
(93) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-4-yl)oxy)acetamide;
(94) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-4-yl)amino)acetamide;
(95) (2S,4R)-1-((S)-2-(2-(3-(4-(4-(5-(furan-2-ylmethylamino)-[1,2,4]triazolo[4,3-f]pyrimidin-8-yl)phenyl)piperazin-1-yl)propoxy)acetamido)-3,3-dimethylbutanoyl)-4-hydroxy-N-(4-(4-methylthiazol-5-yl)benzyl)pyrrolidin-2-carboxamide;
(96) (2S,4R)-1-((S)-2-(2-(4-(4-(4-(5-(furan-2-ylmethylamino)-[1,2,4]triazolo[4,3-f]pyrimidin-8-yl)phenyl)piperazin-1-yl)butoxy)acetamido)-3,3-dimethylbutanoyl)-4-hydroxy-N-(4-(4-methylthiazol-5-yl)benzyl)pyrrolidin-2-carboxamide;
(97) (2S,4R)-1-((S)-2-(2-(2-(2-(4-(4-(5-(furan-2-ylmethylamino)-[1,2,4]triazolo[4,3-f]pyrimidin-8-yl)phenyl)piperazin-1-yl)ethoxy)ethoxy)acetamido)-3,3-dimethylbutanoyl)-4-hydroxy-N-(4-(4-methylthiazol-5-yl)benzyl)pyrrolidin-2-carboxamide;
(98) (2S,4R)-1-((S)-2-(6-(4-(4-(5-(furan-2-ylmethylamino)-[1,2,4]triazolo[4,3-f]pyrimidin-8-yl)phenyl)piperazin-1-yl)-6-oxohexanamido)-3,3-dimethylbutanoyl)-4-hydroxy-N-(4-(4-methylthiazol-5-yl)benzyl)pyrrolidin-2-carboxamide;
(99) (2S,4R)-1-((S)-2-(8-(4-(4-(5-(furan-2-ylmethylamino)-[1,2,4]triazolo[4,3-f]pyrimidin-8-yl)phenyl)piperazin-1-yl)-8-oxooctanamido)-3,3-dimethylbutanoyl)-4-hydroxy-N-(4-(4-methylthiazol-5-yl)benzyl)pyrrolidin-2-carboxamide;
(100) (2S,4R)-1-((S)-2-tert-butyl-23-(4-(4-(5-(furan-2-ylmethylamino)-[1,2,4]triazolo[4,3-f]pyrimidin-8-yl)phenyl)piperazin-1-yl)-4,23-dioxo-6,9,12,15, 18,21-hexaoxa-3-azatricosan-1-oyl)-4-hydroxy-N-(4-(4-methylthiazol-5-yl)benzyl)pyrrolidin-2-carboxamide;
(101) (2S,4R)-1-((S)-2-(8-(3-(2-(4-(4-(5-(furan-2-ylmethylamino)-[1,2,4]triazolo[4,3-f]pyrimidin-8-yl)phenyl)piperazin-1-yl)-2-oxoethyl)phenoxy)octanamido)-3,3-dimethylbutanoyl)-4-hydroxy-N-(4-(4-methylthiazol-5-yl)benzyl)pyrrolidin-2-carboxamide;
(102) (2S,4R)-1-((S)-2-(2-(2-(2-(3-(2-(4-(4-(5-(furan-2-ylmethylamino)-[1,2,4]triazolo[4,3-f]pyrimidin-8-yl)phenyl)piperazin-1-yl)-2-oxoethyl)phenoxy)ethoxy)ethoxy)acetamido)-3,3-dimethylbutanoyl)-4-hydroxy-N-(4-(4-methylthiazol-5-yl)benzyl)pyrrolidin-2-carboxamide;
(103) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-5-yl)amino)acetamide;
(104) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((3-(2,6-dioxopiperidin-3-yl)-4-oxo-3,4-dihydrobenzo[d] [1,2,3]triazin-6-yl)amino)acetamide;
(105) N-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)ethyl)-2-((2-(2,6-dioxopiperidin-3-yl)-1,3-dioxoisoindolin-5-yl)amino)acetamide;
(106) N-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)ethyl)-2-((3-(2,6-dioxopiperidin-3-yl)-4-oxo-3,4-dihydrobenzo[d][1,2,3]triazin-6-yl)amino)acetamide;
(107) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-5-yl)amino)acetamide;
(108) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((3-(2,6-dioxopiperidin-3-yl)-4-oxo-3,4-dihydrobenzo[d] [1,2,3]triazin-6-yl)amino)acetamide;
(109) (2S,4R)-1-((R)-2-(2-(4-(4-(4-((3S,4R)-4-(dimethylamino)-1-(2-fluoro-6-methylbenzyl)pyrrolidin-3-yl)phenyl)piperazin-1-yl)butoxy)acetamido)-3,3-dimethylbutanoyl)-4-hydroxy-N-(4-(4-methylthiazol-5-yl)benzyl)pyrrolidin-2-carboxamide(carboxamide);
(110) (2S,4R)-1-((R)-2-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)acetamido)-3,3-dimethylbutanoyl)-4-hydroxy-N-(4-(4-methylthiazol-5-yl)benzyl)pyrrolidin-2-carboxamide;
(111) (2S,4R)-1-((R)-2-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)acetamido )-3,3-dimethylbutanoyl)-4-hydroxy-N-(4-(4-methylthiazol-5-yl)benzyl)pyrrolidin-2-carboxamide;
(112) 5-(2-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-oxoethoxy)-2-(2,6-dioxopiperidin-3-yl)isoindolin-1,3-dione;
(113) N-(6-(4-(4-((3S,4R)-4-(dimethylamino)-1-(2-fluoro-6-methylbenzyl)pyrrolidin-3-yl)phenyl)piperazin-1-yl)-6-oxohexyl)-2-((2-(2,6-dioxopiperidin-3-yl)-1,3-dioxoisoindolin-5-yl)oxy)acetamide;
(114) N-(6-(4-(4-((3S,4R)-4-(dimethylamino)-1-(2-fluoro-6-methylbenzyl)pyrrolidin-3-yl)phenyl)piperazin-1-yl)hexyl)-2-((2-(2,6-dioxopiperidin-3-yl)-1,3-dioxoisoindolin-5-yl)oxy)acetamide;
(115) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-4-yl)oxy)acetamide;
(116) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-4-yl)amino)acetamide;
(117) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-5-yl)oxy)acetamide;
(118) N-(2-(2-(4-(4-((3S,4R)-4-(dimethylamino)-1-(2-fluoro-6-methylbenzyl)pyrrolidin-3-yl)phenyl)piperazin-1-yl)ethoxy)ethyl)-2-((2-(2,6-dioxopiperidin-3-yl)-1,3-dioxoisoindolin-5-yl)oxy)acetamide;
(119) N-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)ethyl)-2-((2-(2,6-dioxopiperidin-3-yl)-1,3-dioxoisoindolin-4-yl)amino)acetamide;
(120) N-(2-(2-(2-(4-(4-((3S,4R)-4-(dimethylamino)-1-(2-fluoro-6-methylbenzyl)pyrrolidin-3-yl)phenyl)piperazin-1-yl)ethoxy)ethoxy)ethyl)-2-((2-(2,6-dioxopiperidin-3-yl)-1,3-dioxoisoindolin-5-yl)oxy)acetamide;
(121) N-(2-(2-(2-(4-(4-((3S,4R)-4-(dimethylamino)-1-(2-fluoro-6-methylbenzyl)pyrrolidin-3-yl)phenyl)piperazin-1-yl)-2-oxoethoxy)ethoxy)ethyl)-2-((2-(2,6-dioxopiperidin-3-yl)-1,3-dioxoisoindolin-5-yl)amino)acetamide;
(122) N-(2-(2-(2-(2-(4-(4-((3S,4R)-4-(dimethylamino)-1-(2-fluoro-6-methylbenzyl)pyrrolidin-3-yl)phenyl)piperazin-1-yl)-2-oxoethoxy)ethoxy)ethoxy)ethyl)-2-((2-(2,6-dioxopiperidin-3-yl)-1,3-dioxoisoindolin-5-yl)amino)acetamide;
(123) 4-((4-(4-(4-((3S,4R)-4-(dimethylamino)-1-(2-fluoro-6-methylbenzyl)pyrrolidin-3-yl)phenyl)piperazin-1-yl)-4-oxobutyl)amino)-2-(2,6-dioxopiperidin-3-yl)isoindolin-1,3-dione;
(124) 5-((4-(4-(4-((3S,4R)-4-(dimethylamino)-1-(2-fluoro-6-methylbenzyl)pyrrolidin-3-yl)phenyl)piperazin-1-yl)-4-oxobutyl)amino)-2-(2,6-dioxopiperidin-3-yl)isoindolin-1,3-dione;
(125) 4-((8-(4-(4-((3S,4R)-4-(dimethylamino)-1-(2-fluoro-6-methylbenzyl)pyrrolidin-3-yl)phenyl)piperazin-1-yl)-8-oxooctyl)amino)-2-(2,6-dioxopiperidin-3-yl)isoindolin-1,3-dione;
(126) 5-((8-(4-(4-((3S,4R)-4-(dimethylamino)-1-(2-fluoro-6-methylbenzyl)pyrrolidin-3-yl)phenyl)piperazin-1-yl)-8-oxooctyl)amino)-2-(2,6-dioxopiperidin-3-yl)isoindolin-1,3-dione;
(127) 2-(2,6-dioxopiperidin-3-yl)-5-((2-(4-((4-(4-(5-((furan-2-ylmethyl)amino)-[1,2,4]triazolo[4,3-c]pyrimidin-8-yl)-1H-pyrazol-1-yl)piperidin-1-yl)methyl)piperidin-1-yl)-2-oxoethyl)amino)isomdolin-1,3-dione;
(128) 3-(6-((2-(4-((4-(4-(5-((furan-2-ylmethyl)amino)-[1,2,4]triazolo[4,3-c]pyrimidin-8-yl)-1H-pyrazol-1-yl)piperidin-1-yl)methyl)piperidin-1-yl)-2-oxoethyl)amino)-4-oxobenzo[d][1,2,3]triazin-3(4H)-yl)piperidin-2,6-dione;
(129) 2-((2-(2,6-dioxopiperidin-3-yl)-1,3-dioxoisoindolin-5-yl)amino)-N-(2-(2-(2-(4-(4-(5-((furan-2-ylmethyl)amino)-[1,2,4]triazolo[4,3-c]pyrimidin-8-yl)-1H-pyrazol-1-yl)piperidin-1-yl)ethoxy)ethoxy)ethyl)acetamide;
(130) 2-((3-(2,6-dioxopiperidin-3-yl)-4-oxo-3,4-dihydrobenzo[d][1,2,3]triazin-6-yl)amino)-N-(2-(2-(2-(4-(4-(5-((furan-2-ylmethyl)amino)-[1,2,4]triazolo[4,3-clpyrimidin-8-yl)-1H-pyrazol-1-yl)piperidin-1-yl)ethoxy)ethoxy)ethyl)acetamide;
(131) 5-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-(2,6-dioxopiperidin-3-yl)isoindolin-1,3-dione;
(132) 5-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-(2,6-dioxopiperidin-3-yl)isoindolin-1,3-dione;
(133) 5-(4-(3-(4-(4-((3S,4S)-4-(dimethylamino)-1-(2-fluoro-6-methylbenzyl)pyrrolidin-3-yl)phenyl)piperazin-1-yl)-3-oxopropyl)piperidin-1-yl)-2-(2,6-dioxopiperidin-3-yl)isoindolin-1,3-dione;
(134) 5-(4-((4-((4-(4-((3S,4R)-4-(dimethylamino)-1-(2-fluoro-6-methylbenzyl)pyrrolidin-3-yl)phenyl)piperazin-1-yl)methyl)piperidin-1-yl)methyl)piperidin-1-yl)-2-(2,6-dioxopiperidin-3-yl)isoindolin-1,3-dione;
(135) N-(2-(2-(2-(2-(4-(4-((3S,4R)-4-(dimethylamino)-1-(2-fluoro-6-methylbenzyl)pyrrolidm-3-yl)phenyl)piperazm-1-yl)ethoxy)ethoxy)ethoxy)ethyl)-2-((2-(2,6-dioxopiperidin-3-yl)-1,3-dioxoisoindolin-5-yl)amino)acetamide;
(136) 5-((2-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-oxoethyl)amino)-2-(2,6-dioxopiperidin-3-yl)isoindolin-1,3-dione;
(137) 4-((6-(4-(4-((3S,4R)-4-(dimethylamino)-1-(2-fluoro-6-methylbenzyl)pyrrolidin-3-yl)phenyl)piperazin-1-yl)-6-oxohexyl)amino)-2-(2,6-dioxopiperidin-3-yl)isoindolin-1,3-dione;
(138) 5-((6-(4-(4-((3S,4R)-4-(dimethylamino)-1-(2-fluoro-6-methylbenzyl)pyrrolidin-3-yl)phenyl)piperazin-1-yl)-6-oxohexyl)amino)-2-(2,6-dioxopiperidin-3-yl)isoindolin-1,3-dione;
(139) 4-(4-((4-(4-((3S,4R)-4-(dimethylamino)-1-(2-fluoro-6-methylbenzyl)pyrrolidin-3-yl)phenyl)piperazin-1-yl)methyl)piperidin-1-yl)-2-(2,6-dioxopiperidin-3-yl)isoindolin-1,3-dione;
(140) 5-((2-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-oxoethyl)amino)-2-(2,6-dioxopiperidin-3-yl)isoindolin-1,3-dione;
(141) 4-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-(2,6-dioxopiperidin-3-yl)isoindolin-1,3-dione;
(142) 4-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-(2,6-dioxopiperidin-3-yl)isoindolin-1,3-dione;
(143) 4-(2-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-oxoethoxy)-2-(2,6-dioxopiperidin-3-yl)isoindolin-1,3-dione;
(144) 4-((2-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-oxoethyl)amino)-2-(2,6-dioxopiperidin-3-yl)isoindolin-1,3-dione;
(145) 5-(2-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-oxoethoxy)-2-(2,6-dioxopiperidin-3-yl)isoindolin-1,3-dione;
(146) 5-((2-(4-(2-(4-(4-((3S,4R)-4-(dimethylamino)-1-(2-fluoro-6-methylbenzyl)pyrrolidin-3-yl)phenyl)piperazin-1-yl)-2-oxoethyl)piperidin-1-yl)-2-oxoethyl)amino)-2-(2,6-dioxopiperidin-3-yl)isoindolin-1,3-dione;
(147) 5-(2-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-oxoethoxy)-2-(2,6-dioxopiperidin-3-yl)isoindolin-1,3-dione;
(148) 4-(2-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-oxoethoxy)-2-(2,6-dioxopiperidin-3-yl)isoindolin-1,3-dione;
(149) 4-((2-(4-(3-(4-(4-((3S,4R)-4-(dimethylamino)-1-(2-fluoro-6-methylbenzyl)pyrrolidin-3-yl)phenyl)piperazin-1-yl)-3-oxopropyl)piperidin-1-yl)-2-oxoethyl)amino)-2-(2,6-dioxopiperidin-3-yl)isoindolin-1,3-dione;
(150) 5-(2-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-oxoethoxy)-2-(2,6-dioxopiperidin-3-yl)isoindolin-1,3-dione;
(151) 4-(2-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-oxoethoxy)-2-(2,6-dioxopiperidin-3-yl)isoindolin-1,3-dione;
(152) 4-((2-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-oxoethyl)amino)-2-(2,6-dioxopiperidin-3-yl)isoindolin-1,3-dione;
(153) 4-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-(2,6-dioxopiperidin-3-yl)isoindolin-1 ,3-dione;
(154) 5-(4-(2-(4-(4-((3S,4R)-4-(dimethylamino)-1-(2-fluoro-6-methylbenzyl)pyrrolidin-3-yl)phenyl)piperazin-1-yl)ethyl)piperidin-1-yl)-2-(2,6-dioxopiperidin-3-yl)isoindolin-1,3-dione;
(155) 5-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-2-(2,6-dioxopiperidin-3-yl)isoindolin-1,3-dione;
(156) 5-(2-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-2-oxoethoxy)-2-(2,6-dioxopiperidin-3-yl)isoindolin-1,3-dione;
(157) 5-((2-(4-((4-(4-((3S,4R)-4-(dimethylamino)-1-(7-fluoro-2,3-dihydro-1H-mden-1-yl)pyrrolidin-3-yl)phenyl)piperazin-1-yl)methyl)piperidin-1-yl)-2-oxoethyl)amino)-2-(2,6-dioxopiperidin-3-yl)isoindolin-1,3-dione;
(158) 5-(4-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-carbonyl)piperidin-1-yl)-2-(2,6-dioxopiperidin-3-yl)isoindolin-1,3-dione;
(159) 5-((8-(4-(4-((3S,4R)-4-(dimethylamino)-1-(7-fluoro-2,3-dihydro-1H-inden-1-yl)pyrrolidin-3-yl)phenyl)piperazin-1-yl)-8-oxooctyl)amino)-2-(2,6-dioxopiperidin-3-yl)isoindolin-1,3-dione;
(160) N-(8-(4-(4-((3S,4R)-4-(dimethylamino)-1-(7-fluoro-2,3-dihydro-1H-inden-1-yl)pyrrolidin-3-yl)phenyl)piperazin-1-yl)-8-oxooctyl)-2-((2-(2,6-dioxopiperidin-3-yl)-1,3-dioxoisoindolin-5-yl)oxy)acetamide;
(161) N-(8-(4-(4-((3S,4R)-4-(dimethylamino)-1-(7-fluoro-2,3-dihydro-1H-mden-1-yl)pyrrolidin-3-yl)phenyl)piperazin-1-yl)-8-oxooctyl)-2-((2-(2,6-dioxopiperidin-3-y1)-1,3-dioxoisoindolin-5-yl)amino)acetamide;
(162) N-(8-(4-(4-((3S,4R)-4-(dimethylamino)-1-(7-fluoro-2,3-dihydro-1H-inden-1-yl)pyrrolidin-3-yl)phenyl)piperazin-1-yl)-8-oxooctyl)-1-(2-(2,6-dioxopiperidin-3-yl)-1,3-dioxoisoindolin-5-yl)piperidin-4-carboxamide;
(163) 5-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-2-(2,6-dioxopiperidin-3-yl)isoindolin-1,3-dione;
(164) 5-(2-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-2-oxoethoxy)-2-(2,6-dioxopiperidin-3-yl)isoindolin-1,3-dione;
(165) 5-((2-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-2-oxoethyl)amino)-2-(2,6-dioxopiperidin-3-yl)isoindolin-1,3-dione;
(166) 5-(4-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-carbonyl)piperidin-1-yl)-2-(2,6-dioxopiperidin-3-yl)isoindolin-1,3-dione;
(167) 5-(4-(2-(4-(4-((3S,4R)-4-(dimethylamino)-1-(7-fluoro-2,3-dihydro-1H-mden-1-yl)pyrrolidin-3-yl)phenyl)piperazin-1-yl)-2-oxoethyl)piperidin-1-yl)-2-(2,6-dioxopiperidin-3-yl)isoindolin-1,3-dione;
(168) 5-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-2-(2,6-dioxopiperidin-3-yl)isoindolin-1,3-dione;
(169) 5-(2-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-2-oxoethoxy)-2-(2,6-dioxopiperidin-3-yl)isoindolin-1,3-dione;
(170) 5-((2-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-2-oxoethyl)amino)-2-(2,6-dioxopiperidin-3-yl)isoindolin-1,3-dione;
(171) 5-(4-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-carbonyl)piperidin-1-yl)-2-(2,6-dioxopiperidin-3-yl)isoindolin-1,3-dione;
(172) 3-(6-((8-(4-(4-((3S,4R)-4-(dimethylamino)-1(7-fluoro-2,3-dihydro-1H-inden-1-yl)pyrrolidin-3-yl)phenyl)piperazin-1-yl)-8-oxooctyl)amino)-4-oxobenzo[d][1,2,3]triazin-3(4H)-yl)piperidin-2,6-dione;
(173) 3-(6-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-4-oxobenzo[d][1,2,3]triazin-3(4H)-yl)piperidin-2,6-dione;
(174) 3-(6-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)-2-oxoethyl)piperidin-1-yl)-4-oxobenzo[d] [1,2,3]triazin-3(4H)-yl)piperidin-2,6-dione;
(175) 3-(6-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-4-oxobenzo[d][1,2,3]triazin-3(4H)-yl)piperidin-2,6-dione;
(176) 3-(6-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-4-oxobenzo[d][1,2,3]triazin-3(4H)-yl)piperidin-2,6-dione;
(177) 3-(6-((2-(4-(2-(4-(4-((3S,4R)-4-(dimethylamino)-1-(7-fluoro-2,3-dihydro-1H-inden-1-yl)pyrrolidin-3-yl)phenyl)piperazin-1-yl)ethyl)piperidin-1-yl)-2-oxoethyl)amino)-4-oxobenzo[d][1,2,3]triazin-3(4H)-yl)piperidin-2,6-dione;
(178) 3-(6-((2-(4-(3-(4-(4-((3S,4R)-4-(dimethylamino)-1-(7-fluoro-2,3-dihydro-1H-inden-1-yl)pyrrolidin-3-yl)phenyl)piperazin-1-yl)-3-oxopropyl)piperidin-1-yl)-2-oxoethyl)amino)-4-oxobenzo[d][1,2,3]triazin-3(4H)-yl)piperidin-2,6-dione;
(179) 3-(6-((2-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-2-oxoethyl)amino)-4-oxobenzo[d][1,2,3]triazin-3(4H)-yl)piperidin-2,6-dione; and
(180) 3-(6-(2-(4-((4-(4-((3S,4R)-4-(dimethylamino)-1-(7-fluoro-2,3-dihydro-1H-inden-1-yl)pyrrolidin-3-yl)phenyl)piperazin-1-yl)methyl)piperidin-1-yl)-2-oxoethoxy)-4-oxobenzo[d][1,2,3]triazin-3(4H)-yl)piperidin-2,6-dione.

9. A method for preparing a compound represented by chemical formula 1 of claim 1, comprising, as shown in scheme 1 below,
preparing a compound represented by the chemical formula ULB-L-H from the compound represented by ULB-X; and
preparing a compound of ULB-L-PTM represented by chemical formula 1 from the compound represented by ULB-L-H prepared above: 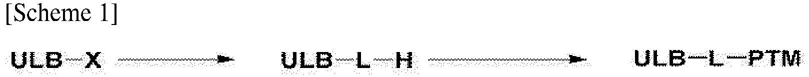
In scheme 1,
ULB, L, and PTM are as defined in claim 1, and
X is H or F.

10. A method for preparing a compound represented by chemical formula 1 of claim 1, comprising, as shown in scheme 2 below,
preparing a compound represented by the chemical formula PTM-L-H from the compound represented by PTM-X; and
preparing a compound of ULB-L-PTM represented by chemical formula 1 from the compound represented by PTM-L-H prepared above. 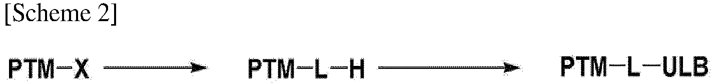
In scheme 2,
ULB, L, and PTM are as defined in claim 1, and
X is H.

11. A pharmaceutical composition for preventing or treating a disease or condition related with EED (embryonic ectoderm development), EZH2 (Enhancer of zeste homolog 2), or PRC2 (polycomb repressive complex 2), comprising the compound of claim 1, a stereoisomer thereof, or a pharmaceutically acceptable salt thereof as an active ingredient.

12. The pharmaceutical composition of claim 11, wherein
the disease or condition is at least one selected from the group consisting of diffuse large B-cell lymphoma, follicular lymphoma, lymphoma, leukemia, multiple myeloma, mesothelioma, gastric cancer, rhabdomyosarcoma, hepatocellular carcinoma, prostate cancer, breast carcinoma, bile duct and gallbladder cancer, bladder cancer, neuroblastoma, schwannoma, glioma, brain tumors including glioblastoma and astrocytoma, cervical cancer, colon cancer, melanoma, endometrial cancer, esophageal cancer, head and neck cancer, lung cancer, nasopharyngeal carcinoma, ovarian cancer, pancreatic cancer, renal cell carcinoma, rectal cancer, thyroid cancer, parathyroid tumor, uterine tumors, and soft tissue sarcoma.

13. A pharmaceutical composition for preventing or treating a cancer, comprising the compound of claim 1, a stereoisomer thereof, or a pharmaceutically acceptable salt thereof as an active ingredient.

14. A health functional food composition for preventing or improving a disease or condition related with EED (embryonic ectoderm development), EZH2 (Enhancer of zeste homolog 2), or PRC2 (polycomb repressive complex 2), comprising the compound of claim 1, a stereoisomer thereof, or a pharmaceutically acceptable salt thereof as an active ingredient.
